# EUROPEAN PATENT SPECIFICATION

(11) **EP 3 897 644 B1**
(45) Date of publication and mention of the grant of the patent: **12.08.2026**
(21) Application number: 19901393.9
(22) Date of filing: 20.12.2019
(51) Int. Cl.: A61K 31/504, A61K 38/12, C07K 5/02, C07D 487/18, C07D 498/18, C07D 498/22

(54) **COMPOUNDS THAT PARTICIPATE IN COOPERATIVE BINDING AND USES THEREOF**
VERBINDUNGEN MIT BETEILIGUNG AN EINER KOOPERATIVEN BINDUNG UND VERWENDUNGEN DAVON
COMPOSÉS PARTICIPANT À UNE LIAISON COOPÉRATIVE ET UTILISATIONS ASSOCIÉES

(30) Priority: 21.12.2018 US 201862783816 P; 30.08.2019 US 201962894493 P; 04.11.2019 US 201962930489 P
(43) Date of publication of application: 27.10.2021
(73) Proprietor: Revolution Medicines, Inc., Redwood City, CA 94063 (US)
(72) Inventor: JIN, Meizhong, Wellesley, MA 02481 (US); PERL, Nicholas, Somerville, MA 02143 (US); KOHLMANN, Anna, Winchester, MA 01890 (US); YIN, Ning, Lexington, MA 02420 (US); LOWE, Jason T., Bridgewater, MA 02333 (US); AHN, Jae Young, Somerville, MA 02144 (US); MULVIHILL, Mark Joseph, Sudbury, MA 01776 (US); KOLTUN, Elena S., Redwood City, CA 94063 (US); GILL, Adrian L., Redwood City, CA 94063 (US)
(74) Representative: Hoffmann Eitle
(86) International application number: PCT/US2019/068100
(87) International publication number: WO 2020/132597

(56) References cited:
- US-A1- 2007 265 333
- US-A1- 2016 296 528
- US-A1- 2017 190 734
- B. C. DOAK ET. AL.: "Cyclophilin Succumbs to a Macrocyclic Chameleon", JOURNAL OF MEDICINAL CHEMISTRY, vol. 61, no. 21, 25 October 2018 (2018-10-25), pages 9469 - 9472, XP055789944, DOI: 10.1021/acs.jmedchem.8b01555
- UPADHYAYA, P ET AL.: "Direct Ras Inhibitors Identified from a Structurally Rigidified Bicyclic Peptide Library", TETRAHEDRON, vol. 70, no. 42, 2014, pages 7714 - 7720, XP029056151, DOI: 10.1016/j.tet.2014.05.113
- DATABASE PUBCHEM 7 October 2017 (2017-10-07), ANONYMOUS: "COMPOUND SUMMARY for CID 130196149", XP055722000, retrieved from NCBI Database accession no. CID 130196149

## Description

### Background

The vast majority of small molecule drugs act by binding a functionally important pocket on a target protein, thereby modulating the activity of that protein. For example, the cholesterol-lowering drugs statins bind the enzyme active site of HMG-CoA reductase, thus preventing the enzyme from engaging with its substrates. The fact that many such drug/target interacting pairs are known may have misled some into believing that a small molecule modulator could be discovered for most, if not all, proteins provided a reasonable amount of time, effort, and resources. This is far from the case. Current estimates are that only about 10% of all human proteins are targetable by small molecules. The other 90% are currently considered refractory or intractable toward above-mentioned small molecule drug discovery. Such targets are commonly referred to as "undruggable." These undruggable targets include a vast and largely untapped reservoir of medically important human proteins. Thus, there exists a great deal of interest in discovering new molecular modalities capable of modulating the function of such undruggable targets.

It has been well established in literature that RAS proteins (KRAS, HRAS and NRAS) play an essential role in various human cancers and are therefore appropriate targets for anticancer therapy. Dysregulation of RAS proteins by activating mutations, overexpression or upstream activation is common in human tumors, and activating mutations in RAS are found in approximately 30% of human cancer. Of the RAS proteins, KRAS is the most frequently mutated and is therefore an important target for cancer therapy. Despite extensive small molecule drug discovery efforts against RAS during the last several decades, a drug directly targeting RAS is still not available for clinical use.

Covalent drugs bond covalently to their biological target. Covalent drugs have a long history in medicine and will continue to impact drug discovery and human health into the future. Biological targets with nucleophilic reactive groups such as -SH, -OH, -NH₂, -COOH and others are potentially amenable to a covalent drug discovery approach.

Doak, B.C. Kihlberg, J. (Journal of Medicinal Chemistry 2018 61 (21), 9469-9472) state that targets that have large and groove-shaped binding sites, such as cyclophilin, are difficult to drug with small molecules; macrocycles of natural product origin can be ideal starting points for such targets as illustrated by the transformation of sanglifehrin A into an orally bioavailable potential candidate drug.

### Summary

The present dislcosure features compounds (e.g., macrocyclic compounds) of Formula I capable of modulating biological processes, for example through binding to a presenter protein that is a member of the cyclophilin A ("CYPA") family and a target protein that is a mutated RAS protein in which the mutation replaces an amino acid in the wild-type amino acid sequence with a cysteine, e.g., KRAS G12C, KRAS G13C, NRAS G12C, NRAS G13C, HRAS G12C and HRAS G13C. In some embodiments, provided compounds may be useful in the treatment of diseases and disorders in which the above-described RAS mutants play a role, such as cancer.

In an aspect, the disclosure features a compound of structural formula (I): 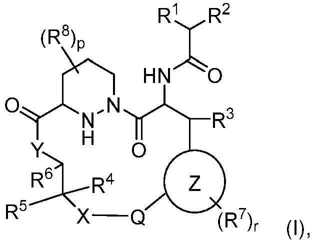 or a pharmaceutically acceptable salt, an enantiomer, a stereoisomer, or a tautomer thereof, where:
Q is a bicyclic arylene, a bicyclic heteroarylene, or a bicyclic heterocyclylene, where a first ring in Q is bonded to X, and a second ring in Q is bonded to Z, and where Q is optionally substituted;
X is a bond; a straight chain C₁-C₃ alkylene optionally substituted with 1 to 3 substituents independently selected from fluoro, -CN, -C₁-C₃ alkyl, and -O-C₁-C₃ alkyl; -O-; -S(O)₀-₂-; *-CH₂-O-; *-CH₂-S(O)₀-₂-; *-O-CH₂-; or *-CH₂-S(O)₀-₂-, where "*" represents a portion of X bound to -C(R⁴)(R⁵)-;
Y is -O-, -NH-, or -N(C₁-C₃ alkyl)-;
ring Z is phenyl or a 6-membered heteroaryl;
R¹ is optionally substituted C₁-C₆ alkyl, -(CH₂)₀-₁-(C₃-C₆ optionally substituted cycloalkyl), -(CH₂)₀-₁-(optionally substituted aryl), or optionally substituted heterocyclyl;
R² is: 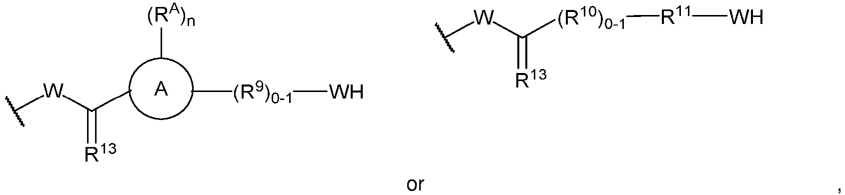 where:
   ring A is a 4-8 membered cycloalkyl or a 4-8 membered heterocyclyl;
   W is -N(R¹²)-, -O-, or -C(R^{12a})(R^{12b})-;
   each R^{A} is each independently fluoro; chloro; -CN; -OH; -NH₂; -C₁-C₃ alkyl optionally substituted with CN, OH, NH₂ or -O-C₁-C₃ alkyl; -O-C₁-C₃ alkyl; or -NH-C₁-C₃ alkyl;
   R⁹, if present, is -N(C₀-C₅ alkylene-H)-, -N(C(O)-(C₀-C₅ alkylene-H)-, -C(C₀-C₃ alkylene-H)(C₀-C₅ alkylene-H)-, or -C(C₀-C₃ alkylene-H)(C(O)-C₀-C₅ alkylene-H)-, where each alkylene portion of R⁹ is optionally substituted with one or more substituent, where each substituent is, independently, selected from halo, -CN, -OH, -C₁-C₃ alkyl, and -O-C₁-C₃ alkyl;
   R¹⁰, if present, is C₁-C₄ alkylene optionally substituted with one or more substituent, where each substituent is, independently, selected from halo, -CN, -OH, -C₁-C₃ alkyl, and -O-C₁-C₃ alkyl;
   R¹¹ is -N(C₀-C₅ alkylene-H)-, -N(C(O)-(C₀-C₅ alkylene-H)-, -C(C₀-C₃ alkylene-H)(C₀-C₅ alkylene-H)-, -C(C₀-C₃ alkylene-H)(C(O)-C₀-C₅ alkylene-H)-, or a saturated, nitrogen-containing heterocyclyl, where each alkylene portion of R¹¹ is optionally substituted with one or more substituent, where each substituent is, independently, selected from halo, -CN, -OH, -C₁-C₃ alkyl, and -O-C₁-C₃ alkyl;
   R¹² is hydrogen or -C₁-C₃ alkyl, or
   R¹² is taken together with one R^{A}, the atoms to which they are respectively attached and any intervening atoms to form an optionally substituted, 5-8 membered heterocyclyl that is fused or spiro-fused to ring A, or
   R¹² is taken together with any methylene unit in R¹⁰, or any methylene unit in R¹¹, the atoms to which they are respectively attached and any intervening atoms to form an optionally substituted, 5-8 membered heterocyclyl;
   each of R^{12a} and R^{12b} are independently hydrogen, or -C₁-C₃ alkyl, or R^{12a} and R^{12b} are taken together with the carbon atom to which they are bound to form a 3-6 membered cycloalkyl ring;
   R¹³ is O, S, N-CN, or N-O-C₁-C₃ alkyl; and
   WH is 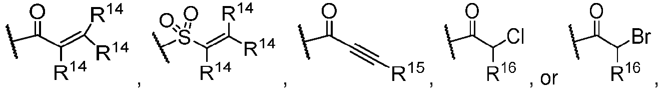
   each R¹⁴ is, independently, hydrogen, -CN, or -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or an optionally substituted 4-7 membered saturated heterocyclyl;
   R¹⁵ is -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or an optionally substituted 4-7 membered saturated heterocyclyl;
   R¹⁶ is hydrogen, -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or an optionally substituted 4-7 membered saturated heterocyclyl; or
   R¹⁴ is taken together with either of R⁹ or R¹¹, the atoms to which they are attached and any intervening atoms to form an optionally substituted 5-8 membered ring system; or
   R¹⁶ is taken together with either of R⁹ or R¹¹, the atoms to which they are attached and any intervening atoms to form an optionally substituted 5-8 membered ring system;
   R³ is hydrogen, halogen, C₁-C₃ alkyl, or C₁-C₃ hydroxyalkyl;
   R⁴ is hydrogen, halogen, or optionally substituted C₁-C₃ alkyl;
   R⁵ is hydrogen, halogen, -OH, -CN, -O-(optionally substituted C₁-C₃ alkyl), optionally substituted C₁-C₃ alkyl, optionally substituted C₂-C₆ alkenyl, optionally substituted C₂-C₆ alkynyl, -(CH₂)₀-₁-aryl, -(CH₂)₀-₁-heteroaryl, -(CH₂)₀-₁-cycloalkyl, or -(CH₂)₀-₁-heterocyclyl; or
   R⁴ and R⁵ are taken together to form =CH₂, an optionally substituted C₃-C₆ cycloalkyl, or a 3-7 membered saturated heterocyclyl; or
   R⁵ is taken together with a ring atom in Q, the carbon atom to which R⁴ is bound and X to form a 4-9 membered saturated or unsaturated heterocyclyl that is fused to Q;
   R⁶ is hydrogen or -CH₃;
   each R⁷ is independently halo, C₁-C₃ alkyl, C₁-C₃ haloalkyl, C₁-C₃ hydroxyalkyl, -OH, -O-C₁-C₃ alkyl, -O-C₁-C₃ haloalkyl, -NRⁿ¹Rⁿ², -NRⁿ¹ORⁿ², -ONRⁿ¹Rⁿ², or -NRⁿ¹NRⁿ²Rⁿ³;
   Rⁿ¹ is H, C₁-C₃ alkyl, C₁-C₃ heteroalkyl, C₁-C₃ haloalkyl, C₁-C₃ hydroxyalkyl, or C₁-C₃ aminoalkyl, wherein one methylene unit of Rⁿ¹ is optionally substituted with 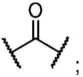
   Rⁿ² is H, C₁-C₃ alkyl, C₁-C₃ heteroalkyl, C₁-C₃ haloalkyl, C₁-C₃ hydroxyalkyl, or C₁-C₃ aminoalkyl, wherein one methylene unit of Rⁿ² is optionally substituted with 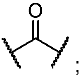
   Rⁿ³ is H, C₁-C₃ alkyl, C₁-C₃ heteroalkyl, C₁-C₃ haloalkyl, C₁-C₃ hydroxyalkyl, or C₁-C₃ aminoalkyl, wherein one methylene unit of Rⁿ³ is optionally substituted with 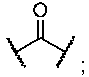
   each R⁸ is independently halo, C₁-C₃ alkyl, or C₁-C₃ haloalkyl;
   n is 0, 1, 2, 3, 4, 5, or 6;
   p is 0, 1, 2, or 3; and
   r is 0, 1, 2, 3, or 4.

In some embodiments Y is -O-. In some embodiments, Y is -NH-. In some embodiments, Y is - N(C₁-C₃ alkyl)-.

In some embodiments, WH is 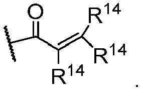 In some embodiments, WH is 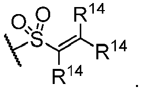 In some embodiments, WH is 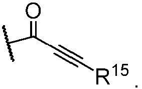 In some embodiments, WH is 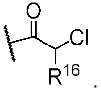 In some embodiments, WH is

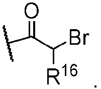

In some embodiments, Z is phenyl or pyridyl. In some embodiments, Z is phenyl. In some embodiments, Z is 3-hydroxyphen-1,5-diyl. In some embodiments, Z is 6-membered hyeteroaryl. In some embodiments, Z is pyridyl.

In some embodiments, n is 0. In some embodiments, n is 1. In some embodiments, n is 2. In some embodiments, n is 3. In some embodiments, n is 4. In some embodiments, n is 5. In some embodiments, n is 6.

In some embodiments, p is 0. In some embodiments, p is 1. In some embodiments, p is 2. In some embodiments, p is 3.

In some embodiments, r is 0. In some embodiments, r is 1. In some embodiments, r is 2. In some embodiments, r is 3. In some embodiments, r is 4.

In some embodiments, R³ is H. In some embodiments, R³ is halogen. In some embodiments, R³ is C₁-C₃ alkyl. In some embodiments, R³ is C₁-C₃ hydroxyalkyl.

In some embodiments, X is -CH₂-. In some embodiments, X is a bond.

In some embodiments, the compound has the structure of formula (Ia): 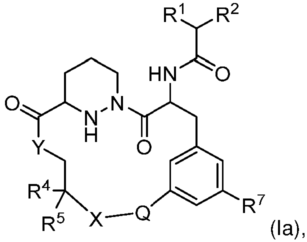 or a pharmaceutically acceptable salt, an enantiomer, a stereoisomer, or a tautomer thereof,
where:
X is a bond, -O-, -CH₂-, -CH(CH₃)-, *-CH₂-O-, or -CH₂-CH₂-, where "*" represents a portion of X bound to C(R⁴)(R⁵);
Y is -O- or -NH-;
R¹ is -C₁-C₄ alkyl, -(CH₂)₀-₁-(C₃-C₆ cycloalkyl), or -C₄-C₆ cycloalkyl;
R² is: 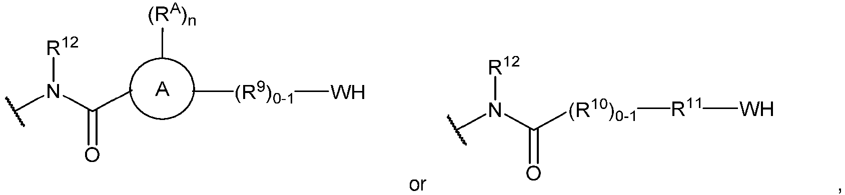 where:
   ring A is a 4-8 membered cycloalkyl or a 4-8 membered saturated heterocyclyl;
   each R^{A} is each independently fluoro; chloro; -CN; -OH; -NH₂; -C₁-C₃ alkyl optionally substituted with CN, OH, NH₂ or -O-C₁-C₃ alkyl; -O-C₁-C₃ alkyl; or -NH-C₁-C₃ alkyl;
   n is 0, 1, 2, 3, 4, 5, or 6;
   R⁹, if present, is -N(C₀-C₅ alkylene-H)-, -N(C(O)-(C₀-C₅ alkylene-H)-, -C(C₀-C₃ alkylene-H)(C₀-C₅ alkylene-H)-, or -C(C₀-C₃ alkylene-H)(C(O)-C₀-C₅ alkylene-H)-, where each alkylene portion of R⁹ is optionally substituted with one or more substituent independently selected from halo, -CN, -OH, -C₁-C₃ alkyl, and -O-C₁-C₃ alkyl;
   R¹⁰, if present, is C₁-C₄ alkylene optionally substituted with one or more substituent independently selected from halo, -CN, -OH, -C₁-C₃ alkyl, and -O-C₁-C₃ alkyl;
   R¹¹ is -N(C₀-C₅ alkylene-H)-, -N(C(O)-(C₀-C₅ alkylene-H)-, -C(C₀-C₃ alkylene-H)(C₀-C₅ alkylene-H)-, or -C(C₀-C₃ alkylene-H)(C(O)-C₀-C₅ alkylene-H)-, where each alkylene portion of R¹¹ is optionally substituted with one or more substituent independently selected from halo, -CN, -OH, -C₁-C₃ alkyl, and -O-C₁-C₃ alkyl;
   R¹² is hydrogen or -C₁-C₃ alkyl, or
   R¹² is taken together with one R^{A}, the atoms to which they are respectively attached and any intervening atoms to form an optionally substituted, 5-8 membered heterocyclyl that is fused to ring A, or
   R¹² is taken together with any methylene unit in R¹⁰, or any methylene unit in R¹¹, the atoms to which they are respectively attached and any intervening atoms to form an optionally substituted, 5-8 membered heterocyclyl;
   WH is 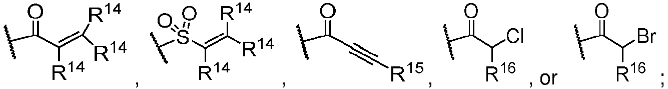
   each R¹⁴ is independently hydrogen, -CN, -C₁-C₃ alkyl, -C₁-C₃ hydroxyalkyl, -O-C₁-C₃ alkyl;
   R¹⁵ is -C₁-C₃ alkyl, -C₁-C₃ hydroxyalkyl, or -C₁-C₃ alkylene-O-C₁-C₃ alkyl;
   R¹⁶ is hydrogen, -C₁-C₃ alkyl, -C₁-C₃ hydroxyalkyl, or -C₁-C₃ alkylene-O-C₁-C₃ alkyl; or
   R¹⁴ is taken together with either of R⁹ or R¹¹, the atoms to which they are attached and any intervening atoms to form an optionally substituted 5-8 membered ring system, or
   R¹⁶ is taken together with either of R⁹ or R¹¹, the atoms to which they are attached and any intervening atoms to form an optionally substituted 5-8 membered ring system;
   R⁴ is hydrogen, halo, or C₁-C₃ alkyl;
   R⁵ is hydrogen, halo, -OH, C₁- C₃ alkyl, C₁-C₃ hydroxyalkyl, C₁-C₃ alkylene-O-C₁-C₃ alkyl, C₁-C₃ haloalkyl, -(CH₂)₀-₁-C₃-C₆ cycloalkyl, C₁-C₃ cyanoalkyl, or -(CH₂)₀-₁-aryl (benzyl), or
   R⁴ and R⁵ are taken together to form =CH₂, or a C₃-C₆ cycloalkyl, or
   R⁵ is taken together with a ring atom of Q, the carbon atom to which it is bound and X to form a 5-7 membered saturated heterocyclyl;
   R⁷ is -OH, -NH₂, or C₁-C₃ haloalkyl;
   Q is a bicyclic arylene, a bicyclic heteroarylene, or a bicyclic heterocyclylene, where:
      a first ring in Q is bonded to X, and a second ring in Q is bonded Z; and
      Q is optionally substituted with one or more independently selected substituents selected from =O; -CN; -C₁-C₅ alkyl optionally substituted with one or more independently selected halo, CN, OH, -O-(C₁-C₃ alkyl), -C(O)-(C₁-C₃ alkyl), -O-(C₂-C₃ alkynyl), -(C₃-C₆ cycloalkyl), or a 4-7 membered saturated heterocyclyl; -O-(C₁-C₃ alkyl) optionally substituted with one or more independently selected halo; C₂-C₅ alkenyl optionally substituted with one or more independently selected -CN, or -OH; C₂-C₃ alkynyl; -S(O)₂-C₁-C₃ alkyl; -(CH₂)₀-₁-C₃-C₆ cycloalkyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heteroaryl optionally substituted with one or more independently selected halo, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heterocyclyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-aryl optionally substituted with one or more independently selected halo, -CN, -C₁-C₃ alkyl optionally substituted with -CN or -O-C₁-C₃ alkyl, -C(O)-saturated heterocyclyl, -O-saturated heterocyclyl, O-cycloalkyl, or -O-aryl; -C(O)-NH-(C₁-C₃ alkyl); -C(O)-N(C₁-C₃ alkyl)₂; C₂-C₃ alkenylene=N-O-(C₁-C₃ alkyl) optionally substituted with C₃-C₆ cycloalkyl; or
      two substituents on the same or adjacent ring atoms of Q are taken together to form a 5-7 membered monocyclic ring or a 6-12 membered bicyclic ring optionally substituted with one or more independently selected halo, =O, -CN, C₁-C₃ alkyl, or -O-C₁-C₃ alkyl; and fused to Q.

In some embodiments, the compound has the structure of formula (Ib): 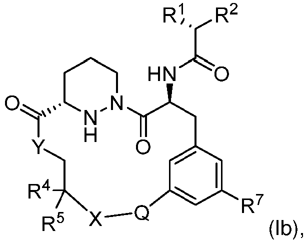 or a pharmaceutically acceptable salt, an enantiomer, a stereoisomer, or a tautomer thereof.

In some embodiments, the compound has the structure of formula (Ic): 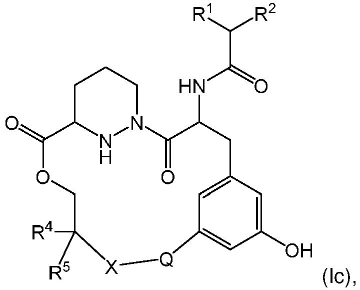 or a pharmaceutically acceptable salt, an enantiomer, a stereoisomer, or a tautomer thereof.

In some embodiments, Q is a 5,6 bicyclic heteroarylene, a 5,6 bicyclic heterocyclylene, a 6,6 bicyclic heteroarylene, or a 6,6 bicyclic heterocyclylene; and where Q is optionally substituted. In some embodiments, Q is a 5,6 bicyclic heteroarylene, wherein Q is optionally substituted. In some embodiments, Q is a 5,6 bicyclic heterocyclylene, wherein Q is optionally substituted. In some embodiments, Q is a 6,6 bicyclic heteroarylene, wherein Q is optionally substituted. In some embodiments, Q is a 6,6 bicyclic heterocyclylene, wherein Q is optionally substituted.

In some embodiments, Q is selected from the group consisting of: 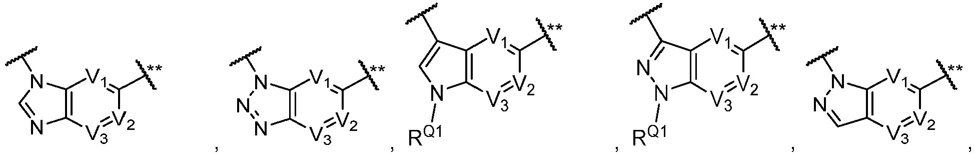 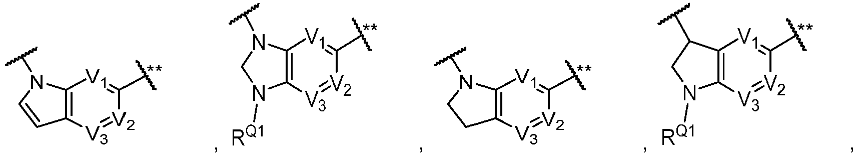 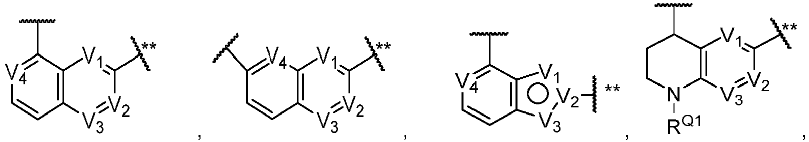 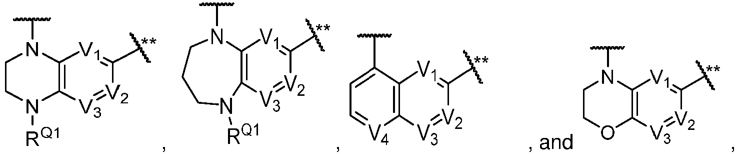 wherein:
each of V₁, V₂, V₃ and V₄ is independently C, CH, or N;
R^{Q1} is -S(O)₂-R^{Q11}, - C(O)-R^{Q11}, -S(O)₂-N(R^{Q11})R^{Q12}, -C(O)-N(R^{Q11})R^{Q12}, C₁-C₁₀alkyl, C₃-C₁₀ cycloalkyl, a 4-14 membered heterocyclyl, aryl, or heteroaryl, where the alkyl, cycloalkyl, heterocyclyl, aryl, or heteroaryl are optionally substituted; or
R^{Q1} is taken together with the nitrogen atom to which it is attached and an adjacent ring atom to form an optionally substituted 4-8 membered ring, which is optionally further fused to a 5-6 membered ring;
each of R^{Q11} and R^{Q12} is independently C₁-C₁₀ alkyl, C₃-C₁₀cycloalkyl, a 4-14 membered heterocyclyl, aryl, or heteroaryl, where each of R^{Q11} and R^{Q12} is optionally substituted; or
R^{Q11} and R^{Q12} are taken together with the nitrogen atom to which they are both attached to form an optionally substituted 4-8 membered ring, where the ring formed by taking R^{Q11} and R^{Q12} together is optionally fused to another 5-6 membered ring.

In some embodiments, Q is optionally additionally substituted with 1 to 4 substituents independently selected from =O; halo; -OH; -CN; -C₁-C₅ alkyl optionally substituted with one or more independently selected halo, CN, OH, -O-(C₁-C₃ alkyl), -C(O)-(C₁-C₃ alkyl), -O-C(O)-N(C₁-C₃ alkyl)₂, -O-(C₂-C₃ alkynyl), -(C₃-C₆ cycloalkyl), a 5-6 membered heteroaryl optionally substituted with one or more C₁-C₃ alkyl, or a 4-7 membered saturated heterocyclyl; -O-(C₁-C₃ alkyl) optionally substituted with one or more independently selected halo; -C₂-C₅ alkenyl optionally substituted with one or more independently selected -CN, or -OH; C₂-C₃ alkynyl optionally substituted with a heteroaryl; -S(O)₂-C₁-C₃ alkyl; -(CH₂)₀-₁-C₃-C₆ cycloalkyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heteroaryl optionally substituted with one or more independently selected halo, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heterocyclyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-aryl optionally substituted with one or more independently selected halo, -CN, -C₁-C₃ alkyl optionally substituted with -CN, -C(O)-O-C₁-C₃ alkyl, -C₁-C₃ alkylene-O-C₁-C₃ alkyl, -O-C₁-C₃ alkyl, NO₂, -C(O)-saturated heterocyclyl, -CH₂-saturated heterocyclyl, -O-saturated heterocyclyl, O-cycloalkyl, or -O-aryl; -CH₂-O-heteroaryl, -C(O)-NH-(C₁-C₃ alkyl); -C(O)-N(C₁-C₃ alkyl)₂; C₂-C₃ alkenylene=N-O-(C₁-C₃ alkyl) optionally substituted with C₃-C₆ cycloalkyl; or
two substituents on Q are taken together to form a 5-7 membered monocyclic ring or a 6-12 membered bicyclic ring optionally substituted with one or more independently selected halo, =O, -CN, C₁-C₃ alkyl, or -O-C₁-C₃ alkyl, and fused to Q; and
"**" represents a portion of Q that is bound to ring Z.

In some embodiments, Q is 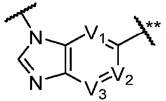 In some embodiments, Q is 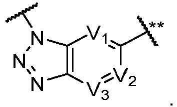 In some embodiments, Q is 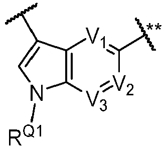 In some embodiments, Q is 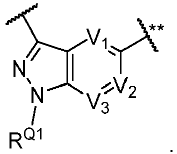 In some embodiments, Q is 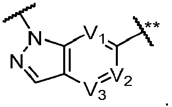 In some embodiments, Q is 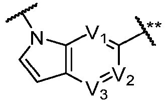 In some embodiments, Q is 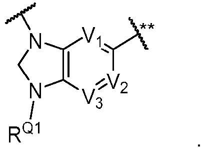 In some embodiments, Q is 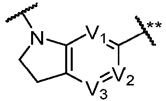 In some embodiments, Q is 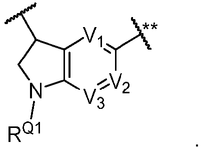 In some embodiments, Q is 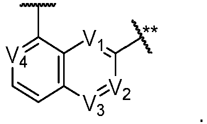 In some embodiments, Q is 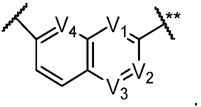 In some embodiments, Q is 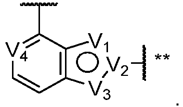 In some embodiments, Q is 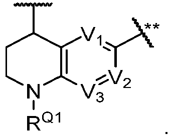 In some embodiments, Q is 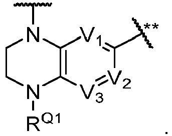 In some embodiments, Q is 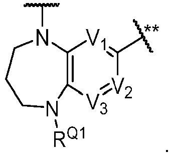 In some embodiments, Q is 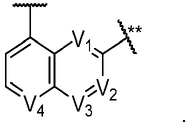 In some embodiments, Q is

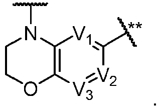

In some embodiments, Q is optionally additionally substituted with 1 to 4 substituents independently selected from chloro,
fluoro, -CN, -CH₃, -CF₃, -CHF₂, -CH₂CH₃, -CH₂-CN, -(CH₂)₂-CN, -OCH₃, -CH₂-O-CH₃, -(CH₂)₂-O-CH₃, -C H₂-O-CH₂-CN, -CH(CN)-CH₃, -C(O)-N(CH₃)₂, -C(O)-NH-CH₃, -C(O)-CH₃, -S(O)₂CH₃, -C(CH₃)=N-O-CH(C H₃)₂, -C(CH₃)=N-O-CH₃, -C≡C-CH₃, -C≡CH, -CH=CH-CN, -CH₂-O-CH₂-C≡CH, -C(CH₃)(CN)CH₂CN, -CH₂-O-C(O)-N(CH₃)₂, 1-(cyclopentyl)-1-cyanoethan-1-yl, 1-(tetrahydrofuran-3-yl)-1-cyanoethan-1-yl, 1-(tetrahydropyran-4-yl)-1-cyanoethan-1-yl, 1,3-dimethoxy-2-cyanopropan-2-yl, 1,4-dimethylpyrazol-5-yl, 1-cyanocyclobutyl, 1-cyanocyclopropyl, 1-cyanocylopentyl, 1-methyl-1,2,3,6-tetrahydropyridin-4-yl, 1-methylpiperidin-4-yl, 1-methylpyrazol-3-yl, 1-methylpyrazol-5-yl, (1-methylpyrazol-4-yl)cyanomethyl, 1-oxoindolin-5-yl, 1-oxoisoindolin-4-yl, 1-oxoisoindolin-6-yl, 2-(2-methoxyethan-1-yl)phenyl, 3-(1,1-dioxothiomorpholin-1-ylmethyl)phenyl, 2-(tetrahydropyran-4-yloxy)phenyl, 2,2-difluoro-benzo[d][1,3]dioxol-4-yl, 2-chlorophenyl, 2-cyano-2-tetrahydrofuran-3-ylpropanyl, 2-cyano-3-chlorophenyl, 2-cyano-3-fluorophenyl, 2-cyano-3-methoxyphenyl, 2-cyano-4-fluorophenyl, 2-cyano-4-chlorophenyl, 2-cyano-4-methoxybutan-2-yl, 2-cyano-5-chlorophenyl, 2-cyano-5-fluorophenyl, 2-cyano-5-methoxyphenyl, 2-cyano-5-(methoxymethyl)phenyl, 2-cyano-6-chlorophenyl, 2-cyano-6-fluorophenyl, 2-cyano-6-bromophenyl, 2-cyano-6-(methoxymethyl)phenyl, 2-cyano-6-(tetrahydropyran-4-yloxy)phenyl, 2-cyanomethylphenyl, 2-cyanophenyl, 2-cyanopropan-2-yl, 2-cyclopentylphenyl, 2-difluoromethoxyphenyl, 2-fluorophenyl, 2-methoxy-6-cyanophenyl, 2-methoxyphenyl, 2-methoxycarbonylphenyl, 2-(methoxymethyl)phenyl, 2-nitrophenyl, 2-oxopyrrolidin-1-yl, 2-phenoxyphenyl, 3-(2-methoxyethan-1-yl)phenyl, 3-methoxycarbonylphenyl, 3,5-difluoro-4-(pyrrolidin-1-ylcarbonyl)phenyl, 3-cyano-2-methylpropan-2-yl, 3-cyanomethylphenyl, 3-cyanopentan-3-yl , 3-cyanophenyl, 3-hydroxy-2-methylbutan-2-yl, 3-hydroxy-3-methyl-but-1-yne-1-yl, 3-methoxy-2-methylbutan-2-yl, 3-methoxyphenyl, 3-methoxymethyl-5-methylisoxazol-4-yl, 3-oxo-2-methylbutan-2-yl, 3-(tetrahydropyran-4-yl)-2-cyanopropan-2-yl, 4-cyanophenyl, 4-cyanotetrahydropyran-4-yl, 4-methoxyphenyl, benzo[d][1,3]dioxol-4-yl, benzo[d]oxazol-7-yl, benzo[d]thiazol-2-yl, benzo[d]thiazol-4-yl, benzo[d]thiazol-5-yl, benzo[d]thiazol-6-yl, benzo[d]thiazol-7-yl, cyclobutyl, cyclopropyl, cyclopropylcyanomethyl, morpholin-4-ylmethyl,
N-methoxycyclopropanecarbimidoyl, phenyl, pyrazol-1-ylmethyl, pyridin-2-yl, pyridin-2-ylmethyl, pyridin-2-yloxymethyl, pyridin-3-yl, pyridin-3-yl-ethynyl, pyridin-3-ylmethyl, pyridin-4-ylmethyl, pyridin-4-yl-ethynyl, tetrahydrofuran-3-ylmethyl, tetrahydrofuran-3-ylcyanomethyl, tetrahydropyridin-4-yl, tetrahydropyran-4-ylmethyl, 2-(tetrahydropyran-4-yl)ethan-1-yl, tetrahydropyran-4-ylcyanomethyl, or tetrahydropyran-4-yl, or
two substituents attached to the same carbon atom are taken together to form =O, 2,3-dihydrobenzofuran-3,3-diyl, 2,3-dihydrofuro[2,3-b]pyridin-3,3-diyl, tetrahydropyran-3,3-diyl, 6,7-dihydro-5H-cyclopenta[c]pyridin-6,6-diyl, or tetrahydropyran-4,4-diyl, or
two substituents attached to adjacent carbon atoms are taken together to form 4-cyanobenzene-1,2-diyl, 3-cyanobenzene-1,2-diyl, 5-methyl-5-cyanotetrahydropyran-3,4-diyl, 3-cyanocyclohexan-1,2-diyl, 3-methoxybenzene-1,2-diyl, benzene-1,2-diyl, 3-oxocyclohexyl-1,2-diyl, 3-cyanocyclopentan-1,2-diyl, or pyridin-3,4-diyl.

In some embodiments, Q is selected from the group consisting of: 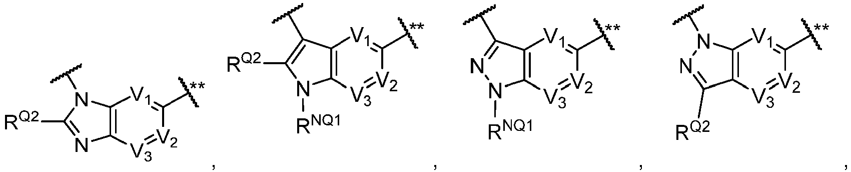 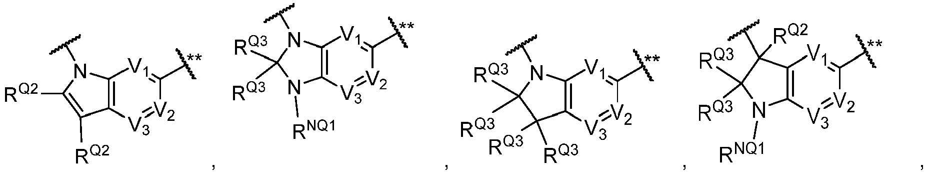 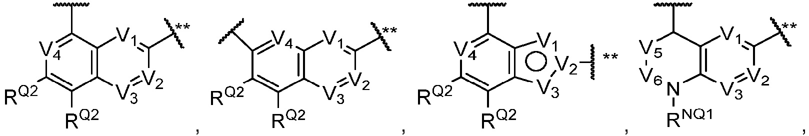 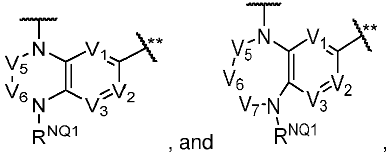 wherein:
each of V₁, V₂, V₃ and V₄ is independently CH, N, C(F), C(CH₃), C(OH), C(OCH₃), or C(CN);
each of V₅, V₆, and V₇ is independently, C(R^{17a})(R^{17b}), or C(=O), where each of R^{17a} and R^{17b} is independently selected from hydrogen, halo, -C₁-C₃ alkyl, -C₁-C₃ haloalkyl, -O-C₁-C₃ alkyl, -O-C₁-C₃ haloalkyl, and no more than two of V₅, V₆, and V₇ is C(=O);
R^{NQ1} is hydrogen, optionally
   substituted -S(O)₂-R^{Q11}, - C(O)-R^{Q11}, -S(O)₂-N(R^{Q11})R^{Q12}, -C(O)-N(R^{Q11})R^{Q12}, C₁-C₁₀alkyl, C₃-C₁₀ cycloalkyl, a 4-14 membered heterocyclyl, aryl, or heteroaryl, where the alkyl, cycloalkyl, heterocyclyl, aryl, or heteroaryl are optionally substituted;
each R^{Q2} is independently hydrogen, CN, optionally
   substituted -S(O)₂-R^{Q11}, - C(O)-R^{Q11}, -S(O)₂-N(R^{Q11})R^{Q12}, -C(O)-N(R^{Q11})R^{Q12}, C₁-C₁₀alkyl, C₃-C₁₀ cycloalkyl, a 4-14 membered heterocyclyl, aryl, or heteroaryl, where the alkyl, cycloalkyl, heterocyclyl, aryl, or heteroaryl are optionally substituted; or
R^{NQ1} and one R^{Q2} are taken together with the atoms to which they are bound to form an optionally substituted 4-8 membered ring, where the ring formed by taking R^{NQ1} and one R^{Q2} together is optionally further fused to a 5-6 membered ring;
each R^{Q3} is independently hydrogen, CN, optionally
   substituted -S(O)₂-R^{Q11}, - C(O)-R^{Q11}, -S(O)₂-N(R^{Q11})R^{Q12}, -C(O)-N(R^{Q11})R^{Q12}, C₁-C₁₀ alkyl, C₃-C₁₀ cycloalkyl, a 4-14 membered heterocyclyl, aryl, or heteroaryl, where the alkyl, cycloalkyl, heterocyclyl, aryl, or heteroaryl are optionally substituted, or
two R^{Q3} bound to the same atom are taken together to form =CH, =O, =S, or =NR^{V4}; or
two R^{Q3} bound to the same atom are taken together with the atom to which they are bound to form an optionally substituted 4-8 membered ring, where the ring formed by taking each R^{Q3} together is optionally further fused to a 5-6 membered ring; or
R^{NQ1} and one R^{Q3} are taken together with the atoms to which they are bound to form an optionally substituted 4-8 membered ring, where the ring formed by taking R^{NQ1} and R^{Q3} together is optionally further fused to a 5-6 membered ring;
each of R^{Q11} and R^{Q12} is independently C₁-C₁₀ alkyl, C₃-C₁₀cycloalkyl, a 4-14 membered heterocyclyl, aryl, or heteroaryl, where each of R^{Q11} and R^{Q12} is optionally substituted; or
R^{Q11} and R^{Q12} are taken together with the atoms to which they are attached to form an optionally substituted 4-8 membered ring, where the ring formed by taking R^{Q11} and R^{Q12} together is optionally fused to another 5-6 membered ring; and
"**" represents a portion of Q that is bound to ring Z.

In some embodiments, Q is 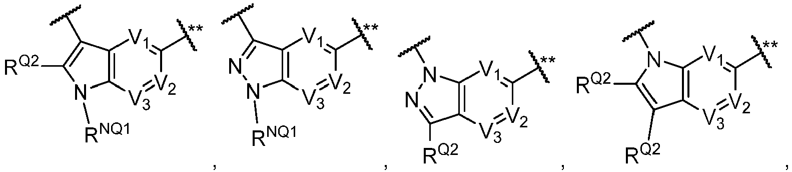 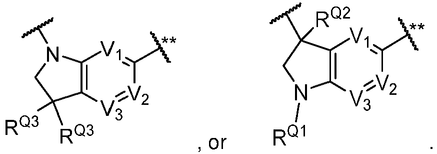 In some embodiments, Q is 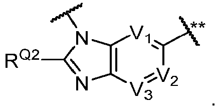 In some embodiments, Q is 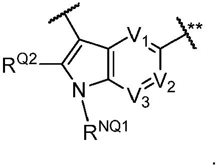 In some embodiments, Q is 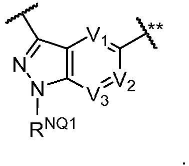 In some embodiments, Q is 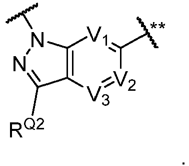 In some embodiments, Q is 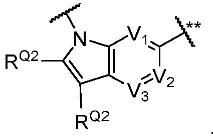 In some embodiments, Q is 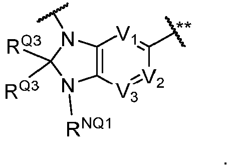 In some embodiments, Q is 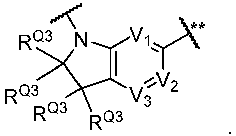 In some embodiments, Q is 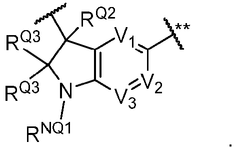 In some embodiments, Q is 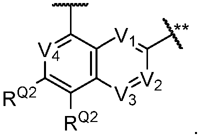 In some embodiments, Q is 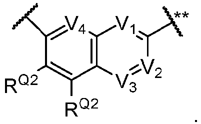 In some embodiments, Q is 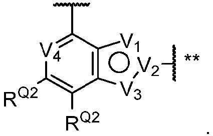 In some embodiments, Q is 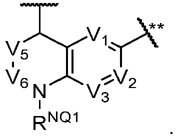 In some embodiments, Q is 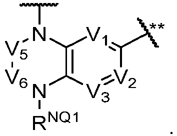 In some embodiments, Q is

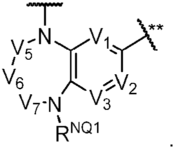

In some embodiments, Q is selected from the group consisting of: 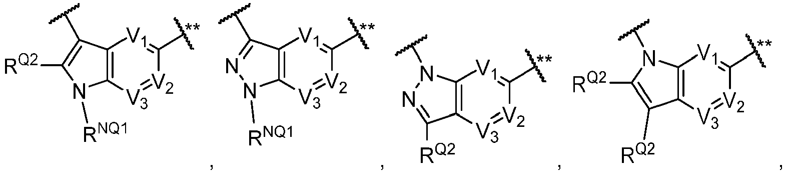 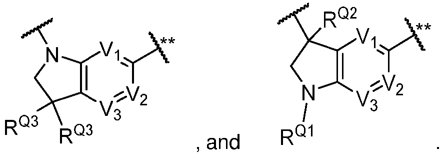 In some embodiments, Q is 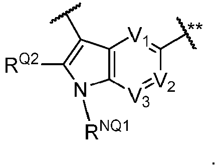 In some embodiments, Q is 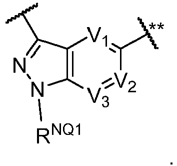 In some embodiments, Q is 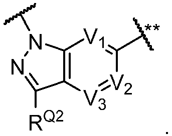 In some embodiments, Q is 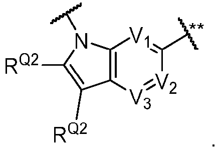 In some embodiments, Q is 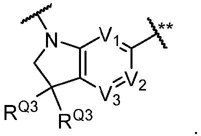 In some embodiments, Q is

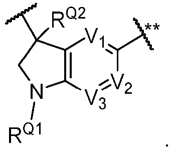

In some embodiments, the compound has the structure of formula (Id): 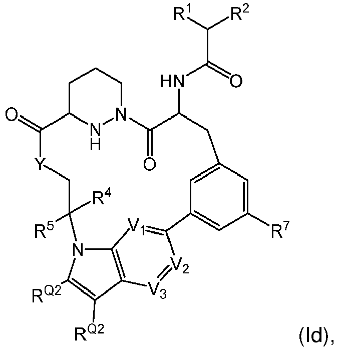 or a pharmaceutically acceptable salt, an enantiomer, a stereoisomer, or a tautomer thereof.

In some embodiments, the compound has the structure of formula (Ie): 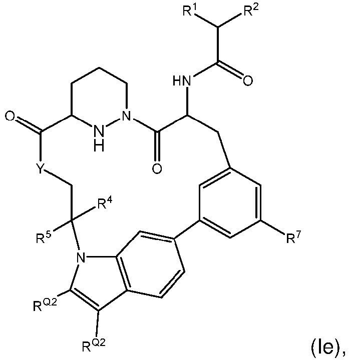 or a pharmaceutically acceptable salt, an enantiomer, a stereoisomer, or a tautomer thereof.

In some embodiments, the compound has the structure of formula (Ig): 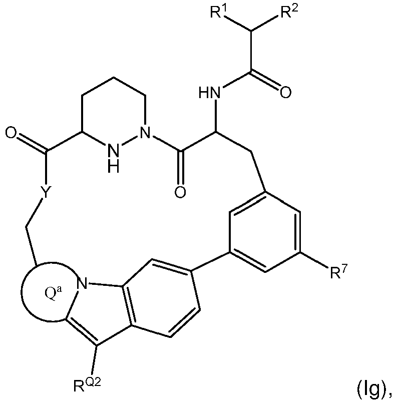 or a pharmaceutically acceptable salt, an enantiomer, a stereoisomer, or a tautomer thereof, where Q^{a} is a 4-9 membered saturated heterocyclyl.

In some embodiments, the compound has the structure of formula (Ij): 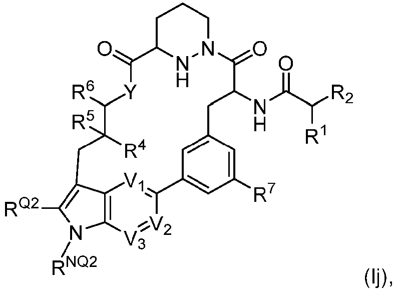 or a pharmaceutically acceptable salt, an enantiomer, a stereoisomer, or a tautomer thereof, where Q^{a} is a 4-9 membered saturated heterocyclyl.

In some embodiments, the compound has the structure of formula (Ik): 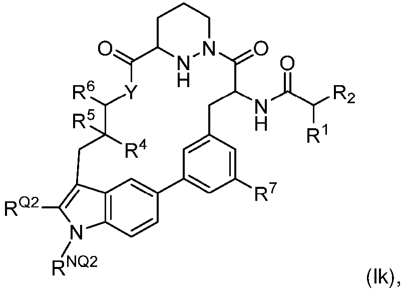 or a pharmaceutically acceptable salt, an enantiomer, a stereoisomer, or a tautomer thereof, where Q^{a} is a 4-9 membered saturated heterocyclyl.

In some embodiments, the compound has the structure of formula (Ik'): 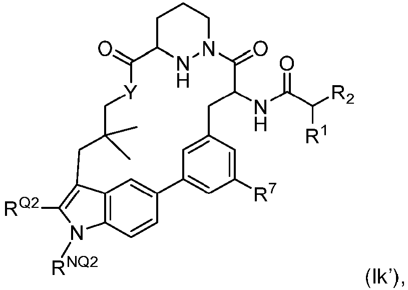 or a pharmaceutically acceptable salt, an enantiomer, a stereoisomer, or a tautomer thereof, where Q^{a} is a 4-9 membered saturated heterocyclyl.

In some embodiments, R⁹ is absent and ring A is a 4-8 membered heterocyclyl; or R¹¹ is -N(C₀-C₅ alkylene-H)-, or -N(C(O)-(C₀-C₅ alkylene-H)-, where each alkylene portion of R¹¹ is optionally substituted with one or more substituent independently selected from halo, -CN, -OH, -C₁-C₃ alkyl, and -O-C₁-C₃ alkyl.

In some embodiments, W is -N(R¹²)-; and R¹³ is =O.

In some embodiments, the compound has the structure of formula (IL): 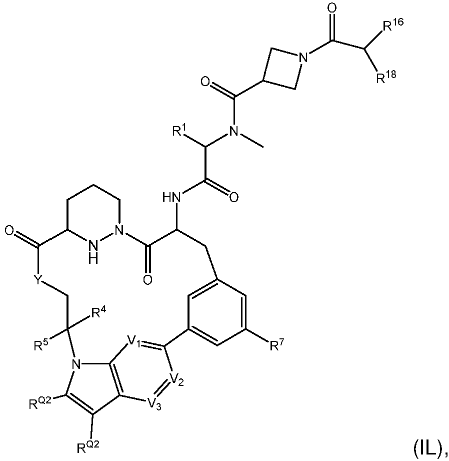 or a pharmaceutically acceptable salt, an enantiomer, a stereoisomer, or a tautomer thereof, where R¹⁸ is Br or Cl.

In some embodiments, the compound has the structure of formula (Im): 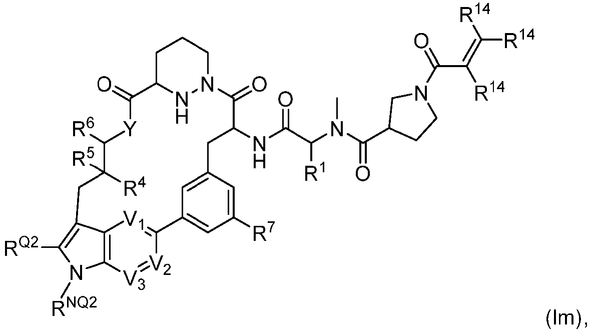 or a pharmaceutically acceptable salt, an enantiomer, a stereoisomer, or a tautomer thereof, wherein R¹⁴ is H.

In some embodiments, Q is selected from the group consisting of: 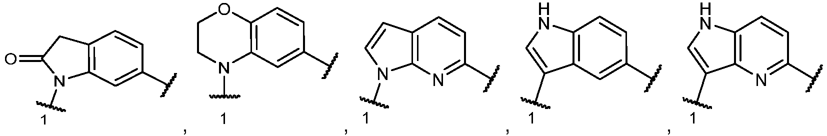 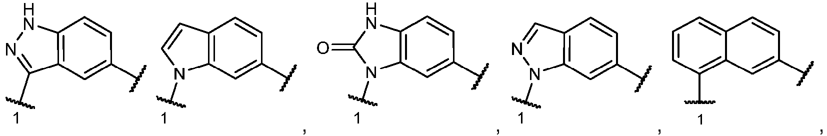 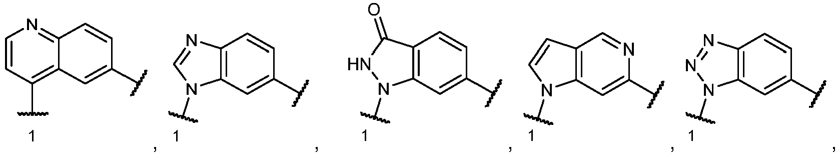 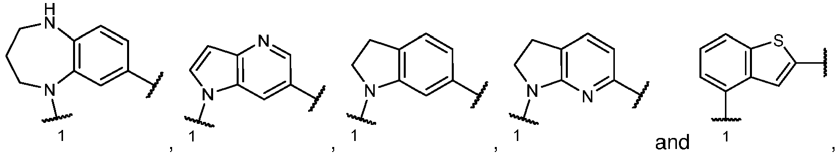 wherein:
"1" indicates a portion of Q bound to X; and Q is further optionally substituted. In some embodiments, Q is 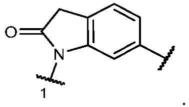 In some embodiments, Q is 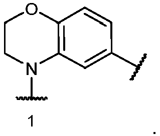 In some embodiments, Q is 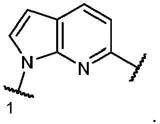 In some embodiments, Q is 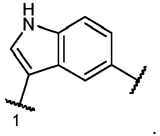 In some embodiments, Q is 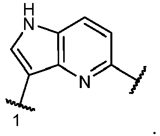 In some embodiments, Q is 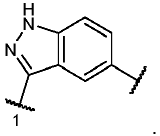 In some embodiments, Q is 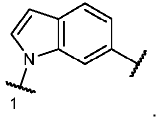 In some embodiments, Q is 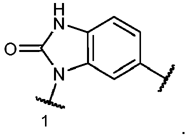 In some embodiments, Q is 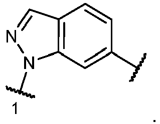 In some embodiments, Q is 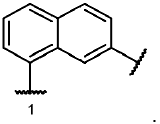 In some embodiments, Q is 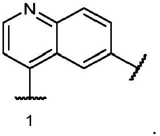 In some embodiments, Q is 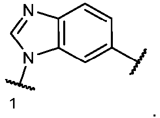 In some embodiments, Q is . In some embodiments, Q is 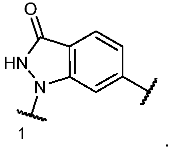 In some embodiments, Q is 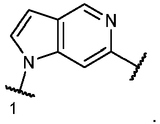 In some embodiments, Q is 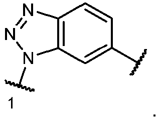 In some embodiments, Q is 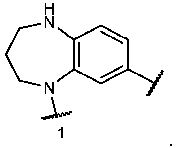 In some embodiments, Q is 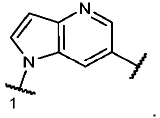 In some embodiments, Q is 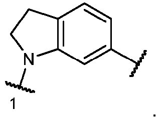 In some embodiments, Q is 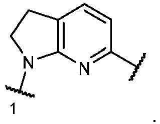 In some embodiments, Q is

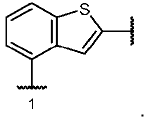

In some embodiments, Q is selected from the group consisting of: 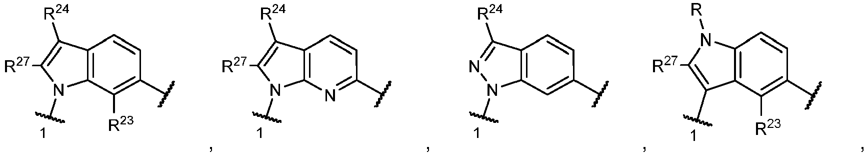 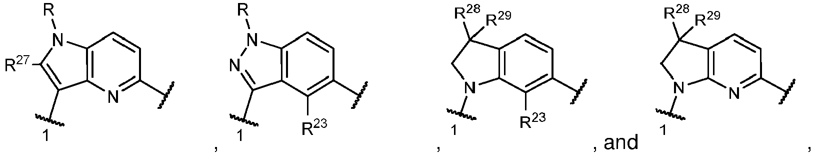 wherein:
R is -CH₂CH₃, -CH₂CH-OCH₃, -CH₂CHF₂, -CH₂-CN, CH₂(CH₃)₂-CN, -C(CH₃)₂-CH₂CN, -CH₂CH₂-CN, cyclohexyl, cyclobutyl, cyclopropyl, pyridin-4-yl, tetrahydropyran-4-yl, tetrahydropyran-4-ylmethyl, oxetan-3-ylmethyl, 2-cyano-5-methoxyphenyl, 2-cyano-5-methoxymethylphenyl, 2-cyano-6-(methoxymethyl)phenyl, 2-cyano-6-bromophenyl, 2-methoxyethan-1-yl, 2-cyanopropan-2-yl, 2-tetrahydropyran-4-ylethan-1-yl, 3-cyanopentan-3-yl, 2-cyano-4-methoxybutan-2-yl, or R is 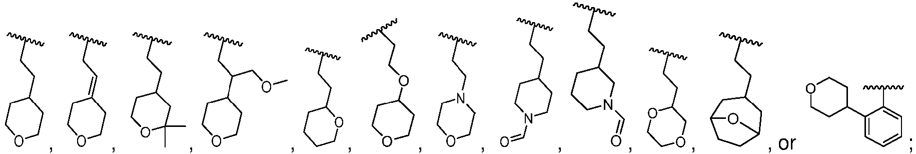
R²³ is hydrogen or fluoro;
R²⁴ is hydrogen,
   chloro, -CN, -CH₃, -CH₂CH₃, -CHF₂, -CF₃, -CH₂-CN, -CH(CN)-CH₃, -C(CH₃)₂-CN, -C(CH₂CH₃)₂-CN, -CH₂-CH₂-CN, -C(CH₃)=N-O-CH(CH₃)₂, -C(CH₃)=N-O-CH₃, -C(O)-N(CH₃)₂, -C(O)-NH-CH₃, -OCH₃, -CH₂-O-CH₃ , -C≡CH, -C≡C-CH₃, -S(O)₂CH₃, 1-(cyclopentyl)-1-cyanoethan-1-yl, 1-(tetrahydropyran-4-yl)-1-cyanoethan-1-yl, 1-(tetrahydrofuran-3-yl)-1-cyanoethan-1-yl, 1,3-dimethoxy-2-cyanopropan-2-yl, 1,4-dimethylpyrazol-5-yl, 1-cyanocyclobutyl, 1-cyanocyclopropyl, 1-cyanocylopentyl, 1-methyl-1,2,3,6-tetrahydropyridin-4-yl, 1-methylpyrazol-3-yl, 1-methylpyrazol-4-ylcyanomethyl, 1-methylpiperidin-4-yl, 1-methylpyrazol-5-yl, 1-oxoindolin-5-yl, 1-oxoisoindolin-4-yl, 1-oxoisoindolin-6-yl, 2-(2-methoxyethan-1-yl)phenyl, 2-(methoxymethyl)phenyl, 2-(tetrahydropyran-4-yloxy)phenyl, 2,2-difluoro-benzo[d][1,3]dioxol-4-yl, 2,3-dicyanopropan-2-yl, 2-chlorophenyl, 2-cyano-3-(tetrahydropyran-4-yl)propan-2-yl, 2-cyano-3-chlorophenyl, 2-cyano-3-fluorophenyl, 2-cyano-3-methoxyphenyl, 2-cyano-4-fluorophenyl, 2-cyano-4-chlorophenyl, 2-cyano-5-chlorophenyl, 2-cyano-5-fluorophenyl, 2-cyano-5-methoxyphenyl, 2-cyano-6-chlorophenyl, 2-cyano-6-fluorophenyl, 2-cyano-6-(tetrahydropyran-4-yloxy)phenyl, 2-cyanomethylphenyl, 2-cyanophenyl, 2-cyanopropan-2-yl, 2-cyclopentylphenyl, 2-difluoromethoxyphenyl, 2-fluorophenyl, 2-methoxy-6-cyanophenyl, 2-methoxyphenyl, 2-methoxycarbonylphenyl, 2-nitrophenyl, 2-oxopyrrolidin-1-yl, 2-phenoxyphenyl, 3-(1,1-dioxothiomorpholin-4-ylmethyl)phenyl, 3-(2-methoxyethan-1-yl)phenyl, 3,5-difluoro-4-(pyrrolidin-1-ylcarbonyl)phenyl, 3-cyano-2-methylpropan-2-yl, 3-cyanomethylphenyl, 3-cyanopentan-3-yl , 3-cyanophenyl, 3-hydroxy-2-methylbutan-2-yl, 3-hydroxy-3-methyl-but-1-yne-1-yl, 3-methoxy-2-methylbutan-2-yl, 3-methoxymethyl-5-methylisoxazol-4-yl, 3-methoxyphenyl, 3-methoxycarbonylphenyl, 3-oxo-2-methylbutan-2-yl, 4-cyanophenyl, 4-cyanotetrahydropyran-4-yl, 4-methoxyphenyl, benzo[d][1,3]dioxol-4-yl, benzo[d]oxazol-7-yl, benzo[d]thiazol-2-yl, benzo[d]thiazol-4-yl, benzo[d]thiazol-5-yl, benzo[d]thiazol-6-yl, benzo[d]thiazol-7-yl, cyclobutyl, cyclopropyl, cyclopropylcyanomethyl, N-methoxycyclopropanecarbimidoyl, phenyl, pyridin-2-ylmethyl, pyridin-3-yl, pyridin-3-ylmethyl, pyridin-4-ylmethyl, tetrahydrofuran-3-ylmethyl, tetrahydrofuran-3-ylcyanomethyl, tetrahydropyran-4-yl, or tetrahydropyran-4-ylcyanomethyl;
R²⁷ is hydrogen, -CH₃, -CHF₂, -CH₂CH₃, -CH₂-O-CH₃, - CH₂CN, -CN, -CH₂-O-CH₂-CN, -C(O)-N(CH₃)₂, -C(O)-NH-CH₃, -CH₂-O-CH₂-C≡CH, 2-methoxyphenyl, 3-methoxyphenyl, 2,2-difluorobenzo[d][1,3]dioxol-4-yl, 2-cyanophenyl, 3-cyanophenyl, phenyl, 2-benzyl methyl ether, 2-(2-methoxyethyl) benzene, 2-(2-difluoromethoxyethyl)benzene, 2-(2-dimethylmethoxyethyl)benzene, pyridin-3-yl, pyridin-2-yl, pyridin-3-ylmethyl, or tetrahydropyridin-4-yl, or
R²⁴ and R²⁷ are taken together to form 4-cyanobenzene-1,2-diyl, 3-cyanobenzene-1,2-diyl, 5-methyl-5-cyanotetrahydropyran-3,4-diyl, 3-cyanocyclohexan-1,2-diyl, 3-methoxybenzene-1,2-diyl, benzene-1,2-diyl, 3-oxocyclohexyl-1,2-diyl, 3-cyanocyclopentan-1,2-diyl, or pyridin-3,4-diyl;
R²⁸ is hydrogen, -CH₃, or -CH₂-O-CH₃; and
R29 is hydrogen, acetyl,
   CN, -CH2-CN, -CH2-CH2-CN, -CH2-O-CH3, -CH=CH-CN, -CH2-O-C(O)-N(CH3)2, morpholin-4-ylmethyl, pyrazol-1-ylmethyl, pyridin-3-yl, pyridin-3-ylethynyl, pyridin-2-yloxymethyl, or 2-cyanopropan-2-yl, or R²⁸ and R²⁹ are taken together to form 2,3-dihydrobenzofuran-3,3-diyl,
   2,3-dihydrofuro[2,3-b]pyridin-3,3-diyl, tetrahydropyran-3,3-diyl, 6,7-dihydro-5H-cyclopenta[c]pyridin-6-yl, tetrahydropyran-4,4-diyl, or 4-methoxycyclohexane. In some embodiments, Q is 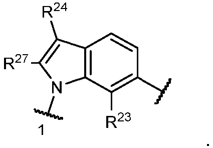 In some embodiments, Q is 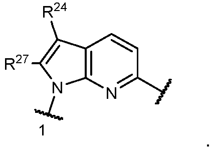 In some embodiments, Q is 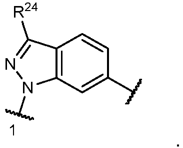 In some embodiments, Q is 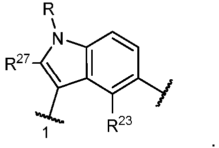 In some embodiments, Q is 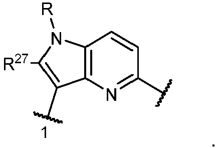 In some embodiments, Q is 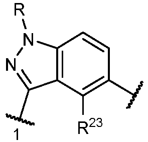 In some embodiments, Q is 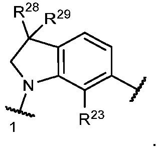 In some embodiments, Q is

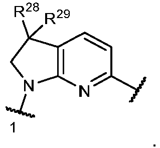

In some embodiments, R¹ is -CH₃, -CH₂CH₃, -(CH₂)₂CH₃, -CH(CH₃)₂, -CH(CH₃)CH₂CH₃, cyclopropylmethyl, cyclobutylmethyl, cyclopentylmethyl, cyclohexylmethyl, cyclopropyl, cyclobutyl, cyclopentyl, cyclohexyl, phenyl, 4-methoxybenzyl, or tetrahydropyran-4-yl.

In some embodiments, R⁹ is absent and ring A is a saturated, nitrogen-containing heterocyclyl.

In some embodiments, the portion of R² represented by: 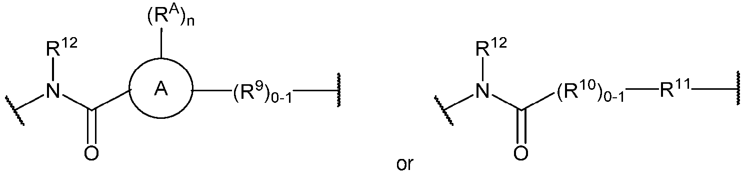 is selected from the group consisting of: 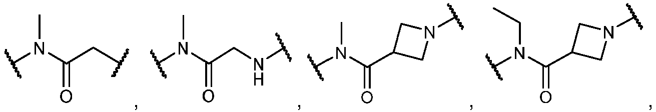 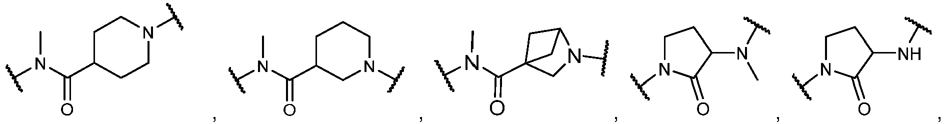 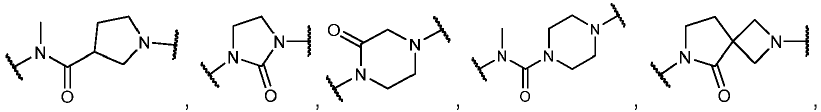 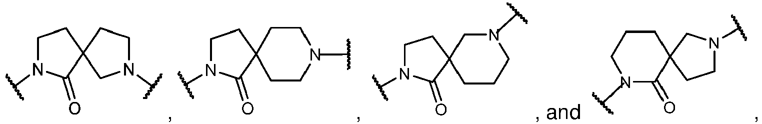 where each ring system in R² is optionally substituted with up to 4 substituents independently selected from fluoro; chloro; -CN; -OH; -NH₂; -C₁-C₃ alkyl optionally substituted with CN, OH, NH₂ or -O-C₁-C₃ alkyl; -O-C₁-C₃ alkyl; and -NH-C₁-C₃ alkyl.

In some embodiments, the portion or R² is represented by 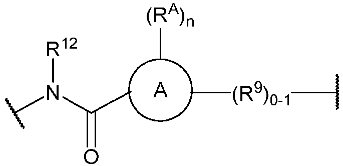 In some embodiments, the portion of R² is represented by 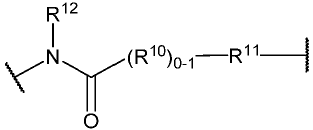 In some embodiments, R² is 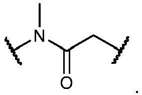 In some embodiments, R² is 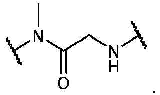 In some embodiments, R² is 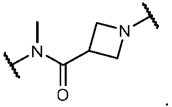 In some embodiments, R² is 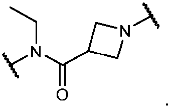 In some embodiments, R² is 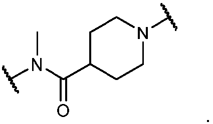 In some embodiments, R² is 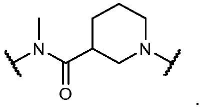 In some embodiments, R² is 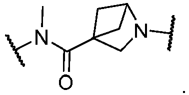 In some embodiments, R² is 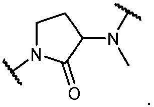 In some embodiments, R² is 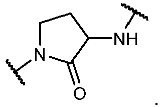 In some embodiments, R² is 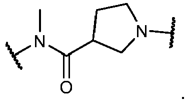 In some embodiments, R² is 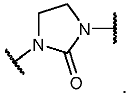 In some embodiments, R² is 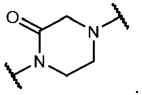 In some embodiments, R² is 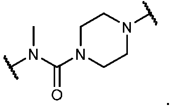 In some embodiments, R² is 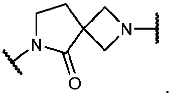 In some embodiments, R² is 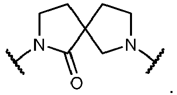 In some embodiments, R² is 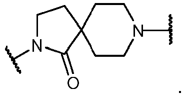 In some embodiments, R² is 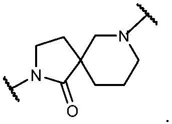 In some embodiments, R² is

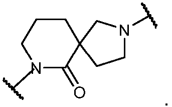

In some embodiments, the portion of R² represented by WH is -C(O)-C≡C-CH₃, -C(O)-CH=CH₂, -S(O)₂-CH=CH₂, -C(O)-CH₂Cl, -C(O)-CH(CH₃)Cl, or -C(O)-CH(Cl)-CH₂-O-CH₃, or
the portion of R² represented by -R¹¹-WH, when R¹¹ is taken together with one R¹⁴ is 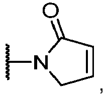 or

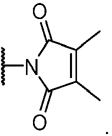

In some embodiments, R² is selected from the group consisting of:
1-(2-chloro-3-methoxypropanoyl)azetidin-3-yl-N-methylcarboxamido,
1-(2-chloroacetyl)azetidin-3-ylcarboxamido, 1-(2-chloroacetyl)azetidin-3-yl-N-ethylcarboxamido,
1-(2-chloroacetyl)azetidin-3-yl-N-methylcarboxamido,
1-(2-chloroacetyl)piperidin-3-yl-N-methylcarboxamido,
1-(2-chloroacetyl)piperidin-4-yl-N-methylcarboxamido,
1-(2-chloroacetyl)pyrrolidin-3-yl-N-methylcarboxamido,
1-(2-chloropropanoyl)-piperidin-4-yl-N-methylcarboxamido,
1-(2-chloropropanoyl)-3-fluoroazetidin-3-yl-N-methylcarboxamido,
1-(2-chloropropanoyl)azetidin-3-yl-N-methylcarboxamido,
1-(2-chloropropanoyl)pyrrolidin-3-yl-N-methylcarboxamido,
1-(but-2-ynoyl)-4-fluoropiperidin-4-ylcarbonylmethylamino,
1-(but-2-ynoyl)azetidin-2-yl-N-methylcarboxamido, 1-(but-2-ynoyl)azetidin-3-yl-N-methylcarboxamido,
1-(but-2-ynoyl)-piperidin-3-ylcarbonylmethylamino, 1-(but-2-ynoyl)-piperidin-4-ylcarbonylmethylamino,
1-(but-2-ynoyl)pyrrolidin-2-ylcarbonyl-N-methylamino,
1-(but-2-ynoyl)pyrrolidin-3-ylcarbonyl-N-methylamino, 1-acryloyl-2-oxo-imidazolidin-3-yl,
1-acryloyl-3-fluoroazetidin-3-yl-N-methylcarboxamido,
1-acryloyl-3-fluoropyrrolidin-3-yl-N-methylcarboxamido,
1-acryloyl-4-fluoropiperidin-4-ylcarbonylmethylamino, 1-acryloylazetidin-2-yl-N-methylcarboxamido,
1-acryloylazetidin-3-yl-N-methylcarboxamido, 1-acryloyl-piperidin-3-ylcarbonylmethylamino,
1-acryloyl-piperidin-4-ylcarbonylmethylamino, 1-acryloylpyrrolidin-2-yl-N-methylcarboxamido,
1-acryloylpyrrolidin-3-yl-N-methylcarboxamido, 1-oxo-7-(2-chloroacetyl)-2,7-diazaspiro[4.3]octan-2-yl,
1-oxo-7-(2-chloroacetyl)-2,7-diazaspiro[4.4]nonan-2-yl,
1-oxo-2-(2-chloroacetyl)-2,7-diazaspiro[4.5]decan-7-yl,
1-oxo-7-(2-chloroacetyl)-2,7-diazaspiro[4.5]decan-2-yl,
1-oxo-7-(2-chloropropanoyl)-2,7-diazaspiro[4.3]octan-2-yl,
1-oxo-7-(but-2-ynoyl)-2,7-diazaspiro[4.4]nonan-2-yl, 1-oxo-7-acryloyl-2,7-diazaspiro[4.3]octan-2-yl,
1-oxo-7-acryloyl-2,7-diazaspiro[4.4]nonan-2-yl, 1-oxo-7-acryloyl-2,7-diazaspiro[4.5]decan-2-yl,
1-oxo-8-(2-chloroacetyl)-2,8-diazaspiro[4.5]decan-2-yl,
1-oxo-8-(but-2-ynoyl)-2,8-diazaspiro[4.5]decan-2-yl, 1-oxo-8-acryloyl-2,8-diazaspiro[4.5]decan-2-yl,
1-vinylsulfonyl-2-oxoimidazolidin-3-yl, 1-vinylsulfonylazetidin-3-yl-N-methylcarboxamido,
2-(1-acryloylpiperidin-4-yl)-N-methylacetamido, 2-(but-2-ynoyl)-5-oxo-2,6-diazaspiro[3.4]octan-6-yl,
2,5-dioxo-3,4-dimethyl-2,5-dihydropyrrol-1-yl-N-methylacetamido,
2-acryloyl-2-azabicyclo[2.1.1]hexan-4-yl-N-methylcarboxamido,
2-chloroacetamidomethyl-N-methylcarboxamido, 2-oxo-2,5-dihydro-1H-pyrrol-1-yl-N-methylacetamido,
2-oxo-3-(2-chloroacetamido)pyrrolidin-1-yl, 2-oxo-3-(N-methyl-2-chloroacetamido)pyrrolidin-1-yl,
2-oxo-3-(N-methylacrylamido)pyrrolidin-1-yl, 2-oxo-3-acrylamidopyrrolidin-1-yl,
2-oxo-4-(2-chloroacetyl)piperazin-1-yl, 2-oxo-4-acryloylpiperazin-1-yl, 2-oxo-4-vinylsulfonylpiperazin-1-yl,
2-oxocyclopent-3-en-1-yl-N-methylacetamido,
3-(4-(dimethylamino)but-2-enamido)phenyl-N-methylcarboxamido,
4-(but-2-ynoyl)-piperazin-1-yl-N-methylcarboxamido, 4-acryloylpiperazin-1-yl-N-methylcarboxamido,
6-oxo-2-(2-chloroacetyl)-2,7-diazaspiro[4.5]decan-7-yl, and
6-oxo-2-acryloyl-2,7-diazaspiro[4.5]decan-7-yl.

In some embodiments, R⁴ is hydrogen, fluoro, or -CH₃; and R⁵ is hydrogen, fluoro, chloro, -OH, -CH₃, -CH₂CH₃, -CH(CH₃)₂, -CH₂OH, -CH₂OCH₃, -CH₂F, -CHF₂, CH₂CN, -CH₂-cyclopropyl, cyclopropyl, pyridyl, phenyl, or -CH₂-phenyl, where any phenyl portion of R⁵ is optionally substituted with up to 4 substituents independently selected from halo, -CN, and -O-C₁-C₃ alkyl;R⁴ and R⁵ are taken together to form =CH₂ or cyclopropyl, or cyclobutyl, or cyclopentyl, or cyclohexyl; or R⁵ is taken together with the carbon atom to which it is bound, a ring atom of Q, and X to form oxazepane.

In some embodiments, R⁷ is -OH, -NH₂, or -CHF₂. In some embodiments, R⁷ is -OH.

In some embodiments, the compound has the structure of any of compounds 1-418, or 1-461, a pharmaceutically acceptable salt, an enantiomer, a stereoisomer, or a tautomer thereof.

In an aspect, the disclosure features a pharmaceutical composition including any compound of the present invention or a pharmaceutically acceptable salt, enantiomer, stereoisomer, or tautomer thereof, and a pharmaceutically acceptable carrier.

In an aspect, the disclosure features a complex including a presenter protein, a RAS protein, and any compound of the present invention or a pharmaceutically acceptable salt, enantiomer, stereoisomer, or tautomer thereof, or any of the pharmaceutical compositions comprising such a compound as described herein.

In some embodiments, the RAS protein is KRAS. In some embodiments, the RAS protein is NRAS. In some embodiments, the RAS protein is HRAS. In some embodiments the RAS protein is KRAS G12C. In some embodiments, the RAS protein is KRAS G13C. In some embodiments, the RAS protein is NRAS G12C. In some embodiments, the RAS protein is NRAS G13C. In some embodiments, the RAS protein is HRAS G12C. In some embodiments, the RAS protein is HRAS G13C.

In some embodiments, the presenter protein is a cyclophilin. In some embodiments, the presenter protein is CYPA, CYPB, CYPC, CYP40, CYPE, CYPD, NKTR, SRCyp, CYPH, CWC27, CYPL1, CYP60, CYPJ, PPIL4, PPIL6, RANBP2, or PPWD1. In some embodiments, the presenter protein is CYPA.

In an aspect, the disclosure features a method of producing a complex, the method including contacting a presenter protein and a KRAS G12C protein with a compound of the present invention under conditions suitable to permit complex formation. In some embodiments, the disclosure features a method of producing a complex, the method including contacting a presenter protein and a KRAS G13C protein, an NRAS G12C protein, an NRAS G13C protein, an HRAS G12C protein or an HRAS G13C protein, with a compound of the present invention under conditions suitable to permit complex formation. In some embodiments, the presenter protein is a cyclophilin protein. In some embodiments, the presenter protein is PP1A, CYPA, CYPB, CYPC, CYP40, CYPE, CYPD, NKTR, SRCyp, CYPH, CWC27, CYPL1, CYP60, CYPJ, PPIL4, PPIL6, RANBP2, or PPWD1. In some embodiments, the presenter protein is CYPA.

In an aspect, the disclosure features a method of producing a complex, the method including contacting a presenter protein and a KRAS G12C protein with a compound of the present invention under conditions suitable to permit complex formation if the compound is capable of forming a complex with the presenter protein and the KRAS G12C protein. In an aspect, the disclosure features a method of producing a complex, the method including contacting a presenter protein and a KRAS G13C protein, an NRAS G12C protein, an NRAS G13C protein, an HRAS G12C protein or an HRAS G13C protein, with a compound of the present invention under conditions suitable to permit complex formation if the compound is capable of forming a complex with the presenter protein and the RAS protein. In some embodiments, the presenter protein is a cyclophilin protein. In some embodiments, the presenter protein is PP1A, CYPA, CYPB, CYPC, CYP40, CYPE, CYPD, NKTR, SRCyp, CYPH, CWC27, CYPL1, CYP60, CYPJ, PPIL4, PPIL6, RANBP2, or PPWD1. In some embodiments, the presenter protein is CYPA.

In an aspect, the disclosure features a method of treating cancer in a subject in need thereof, the method including administering to the subject an effective amount of a compound of the present invention or any of pharmaceutical compositions comprising such a compound.

In some embodiments, the cancer is pancreactic cancer, colorectal cancer, non-small cell lung cancer, or small cell lung cancer.

In an aspect, the disclosure features a method of inhibiting a KRAS G12C protein in a cell, the method including contacting the cell with an effective amount of a compound of the present invention or any pharmaceutical composition comprising such a compound. In an aspect, the disclosure features a method of inhibiting a KRAS G13C protein, an NRAS G12C protein, an NRAS G13C protein, an HRAS G12C protein or an HRAS G13C protein in a cell, the method including contacting the cell with an effective amount of a compound of the present invention or any pharmaceutical composition comprising such a compound. In some embodiments, the cell is a cancer cell.

In an aspect, the disclosure features a method of treating a KRAS G12C protein-related disorder in a subject in need thereof, the method including administering to the subject an effective amount of a compound of the present invention or any pharmaceutical composition comprising such a compound. In an aspect, the disclosure features a method of treating a KRAS G13C protein-related disorder, an NRAS G12C protein-related disorder, an NRAS G13C protein-related disorder, an HRAS G12C protein-related disorder, or an HRAS G13C protein-related disorder, in a subject in need thereof, the method including administering to the subject an effective amount of a compound of the present invention or any pharmaceutical composition comprising such a compound.

In some embodiments, the cell is a cancer cell. In some embodiments, the cancer is pancreatic cancer, colorectal cancer, non-small cell lung cancer, or small cell lung cancer. In some embodiments, the cancer is pancreatic cancer, colorectal cancer, non-small cell lung cancer, small cell lung cancer, acute myeloid leukemia, multiple myeloma, thyroid gland adenocarcinoma, a myelodysplastic syndrome, or squamous cell lung carcinoma.

In an aspect, the disclosure features a method of inhibiting RAF-RAS binding in a cell, the method including contacting the cell with an effective amount of a compound of the present invention or any pharmaceutical composition comprising such a compound. In some embodiments, the cell is a cancer cell. In some embodiments, the cancer is pancreatic cancer, colorectal cancer, non-small cell lung cancer, or small cell lung cancer. In some embodiments, the cancer is pancreatic cancer, colorectal cancer, non-small cell lung cancer, small cell lung cancer, acute myeloid leukemia, multiple myeloma, thyroid gland adenocarcinoma, a myelodysplastic syndrome, or squamous cell lung carcinoma

In an aspect, the disclosure features the use of a compound of the present invention, any pharmaceutical composition comprising such a compound, or any of the complexes described herein for treating cancer in a subject in need thereof.

In an aspect, the disclosure features the use of any a compound of the present invention, any pharmaceutical composition comprising such a compound, or any of the complexes described herein for treating a KRAS G12C protein-related disorder in a subject in need thereof. In an aspect, the disclosure features the use of a compound of the present invention, any pharmaceutical composition comprising such a compound, or any of the complexes described herein for treating a KRAS G13C protein-related disorder, an NRAS G12C protein-related disorder, an NRAS G13C protein-related disorder, an HRAS G12C protein-related disorder, or an HRAS G13C protein-related disorder, in a subject in need thereof.

In some embodiments, a method may further include administering an additional therapeutic agent (e.g., an anti-cancer agent). In some embodiments, the additional therapeutic agent is a HER2 inhibitor, an EGFR inhibitor, a second Ras inhibitor, a SHP2 inhibitor, a SOS1 inhibitor, a Raf inhibitor, a MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, or a combination thereof.

In some embodiments, the additional therapeutic agent is a SHP2 inhibitor. SHP2 is a non-receptor protein tyrosine phosphatase encoded by the PTPN11 gene that contributes to multiple cellular functions including proliferation, differentiation, cell cycle maintenance and migration. SHP2 has two N-terminal Src homology 2 domains (N-SH2 and C-SH2), a catalytic domain (PTP), and a C-terminal tail. The two SH2 domains control the subcellular localization and functional regulation of SHP2. The molecule exists in an inactive, self-inhibited conformation stabilized by a binding network involving residues from both the N-SH2 and PTP domains. Stimulation by, for example, cytokines or growth factors acting through receptor tyrosine kinases (RTKs) leads to exposure of the catalytic site resulting in enzymatic activation of SHP2.

SHP2 is involved in signaling through the RAS-mitogen-activated protein kinase (MAPK), the JAK-STAT or the phosphoinositol 3-kinase-AKT pathways. Mutations in the PTPN11 gene and subsequently in SHP2 have been identified in several human developmental diseases, such as Noonan Syndrome and Leopard Syndrome, as well as human cancers, such as juvenile myelomonocytic leukemia, neuroblastoma, melanoma, acute myeloid leukemia and cancers of the breast, lung and colon. Some of these mutations destabilize the auto-inhibited conformation of SHP2 and promote autoactivation or enhanced growth factor driven activation of SHP2. SHP2, therefore, represents a highly attractive target for the development of novel therapies for the treatment of various diseases including cancer. A SHP2 inhibitor (e.g., RMC-4550 or SHP099) in combination with a RAS pathway inhibitor (e.g., a MEK inhibitor) have been shown to inhibit the proliferation of multiple cancer cell lines in vitro (e.g., pancreas, lung, ovarian and breast cancer). Thus, combination therapy involving a SHP2 inhibitor with a RAS pathway inhibitor could be a general strategy for preventing tumor resistance in a wide range of malignancies.

Non-limiting examples of such SHP2 inhibitors that are known in the art, include: Chen et al. Mol Pharmacol. 2006, 70, 562; Sarver et al., J. Med. Chem. 2017, 62, 1793; Xie et al., J. Med. Chem. 2017, 60, 113734; and Igbe et al., Oncotarget, 2017, 8, 113734; and PCT applications: WO2015107493; WO2015107494; WO201507495; WO2016203404; WO2016203405; WO2016203406; WO2011022440; WO2017156397; WO2017079723; WO2017211303; WO2012041524; WO2017211303; WO2019051084; WO2017211303; US20160030594; US20110281942; WO2010011666; WO2014113584; WO2014176488; WO2017100279; WO2019051469; US8637684; WO2007117699; WO2015003094; WO2005094314; WO2008124815; WO2009049098; WO2009135000; WO2016191328; WO2016196591; WO2017078499; WO2017210134; WO2018013597; WO2018129402; WO2018130928; WO20181309928; WO2018136264; WO2018136265; WO2018160731; WO2018172984; and WO2010121212, each of which is incorporated herein by reference.

In some embodiments, a SHP2 inhibitor binds in the active site. In some embodiments, a SHP2 inhibitor is a mixed-type irreversible inhibitor. In some embodiments, a SHP2 inhibitor binds an allosteric site e.g., a non-covalent allosteric inhibitor. In some embodiments, a SHP2 inhibitor is a covalent SHP2 inhibitor, such as an inhibitor that targets the cysteine residue (C333) that lies outside the phosphatase's active site. In some embodiments a SHP2 inhibitor is a reversible inhibitor. In some embodiments, a SHP2 inhibitor is an irreversible inhibitor. In some embodiments, the SHP2 inhibitor is SHP099. In some embodiments, the SHP2 inhibitor is TNO155. In some embodiments, the SHP2 inhibitor is RMC-4550. In some embodiments, the SHP2 inhibitor is RCM-4630. In some embodiments, the SHP2 inhibitor is JAB-3068.

### Chemical Terms

Those skilled in the art will appreciate that certain compounds described herein can exist in one or more different isomeric (e.g., stereoisomers, geometric isomers, tautomers) and/or isotopic (e.g., in which one or more atoms has been substituted with a different isotope of the atom, such as hydrogen substituted for deuterium) forms. Unless otherwise indicated or clear from context, a depicted structure can be understood to represent any such isomeric or isotopic form, individually or in combination.

Compounds described herein can be asymmetric (e.g., having one or more stereocenters). All stereoisomers, such as enantiomers and diastereomers, are intended unless otherwise indicated. Compounds of the present disclosure that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present disclosure. Cis and trans geometric isomers of the compounds of the present disclosure are described and may be isolated as a mixture of isomers or as separated isomeric forms.

In some embodiments, one or more compounds depicted herein may exist in different tautomeric forms. As will be clear from context, unless explicitly excluded, references to such compounds encompass all such tautomeric forms. In some embodiments, tautomeric forms result from the swapping of a single bond with an adjacent double bond and the concomitant migration of a proton. In certain embodiments, a tautomeric form may be a prototropic tautomer, which is an isomeric protonation states having the same empirical formula and total charge as a reference form. Examples of moieties with prototropic tautomeric forms are ketone - enol pairs, amide - imidic acid pairs, lactam - lactim pairs, amide - imidic acid pairs, enamine - imine pairs, and annular forms where a proton can occupy two or more positions of a heterocyclic system, such as, 1H- and 3H-imidazole, 1H-, 2H- and 4H- 1,2,4-triazole, 1H- and 2H- isoindole, and 1H- and 2H-pyrazole. In some embodiments, tautomeric forms can be in equilibrium or sterically locked into one form by appropriate substitution. In certain embodiments, tautomeric forms result from acetal interconversion, e.g., the interconversion illustrated in the scheme below:

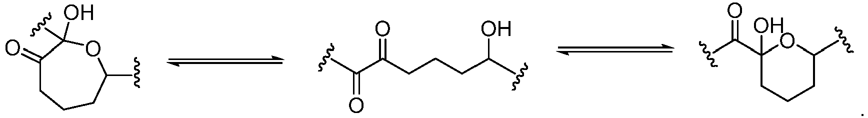

Those skilled in the art will appreciate that, in some embodiments, isotopes of compounds described herein may be prepared and/or utilized in accordance with the present invention. "Isotopes" refers to atoms having the same atomic number but different mass numbers resulting from a different number of neutrons in the nuclei. For example, isotopes of hydrogen include tritium and deuterium. In some embodiments, an isotopic substitution (e.g., substitution of hydrogen with deuterium) may alter the physicochemical properties of the molecules, such as metabolism, the distribution of metabolites, and/or the rate of racemization of a chiral center.

As is known in the art, many chemical entities (in particular many organic molecules and/or many small molecules) can adopt a variety of different solid forms such as, for example, amorphous forms and/or crystalline forms (e.g., polymorphs, hydrates, solvates, etc). In some embodiments, such entities may be utilized in any form, including in any solid form. In some embodiments, such entities are utilized in a particular form, for example in a particular solid form.

In some embodiments, compounds described and/or depicted herein may be provided and/or utilized in salt form.

In certain embodiments, compounds described and/or depicted herein may be provided and/or utilized in hydrate or solvate form.

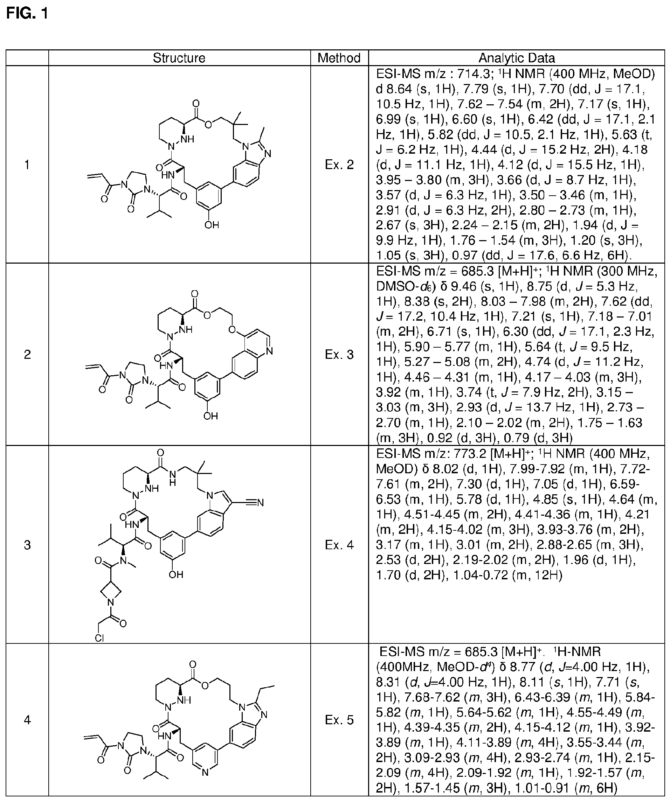

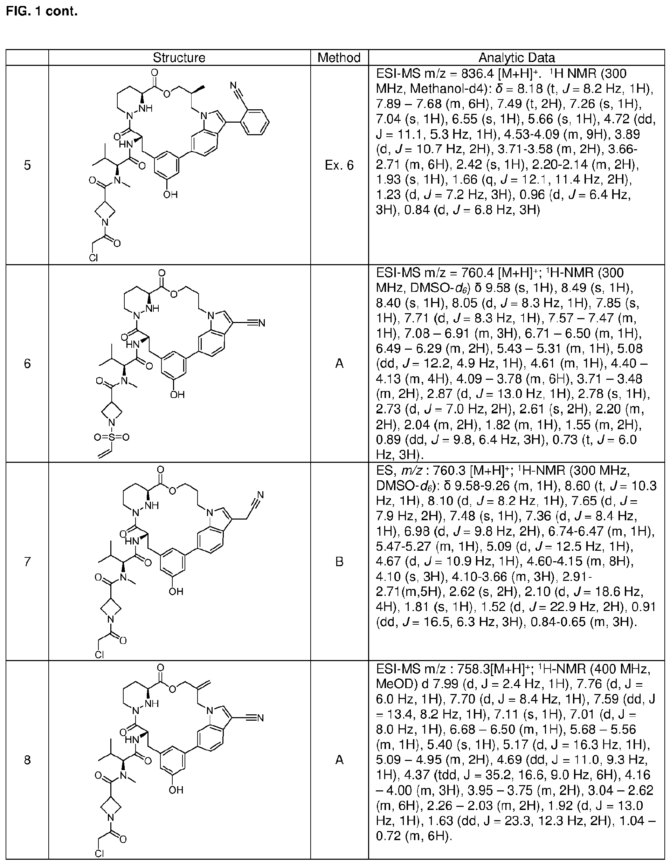

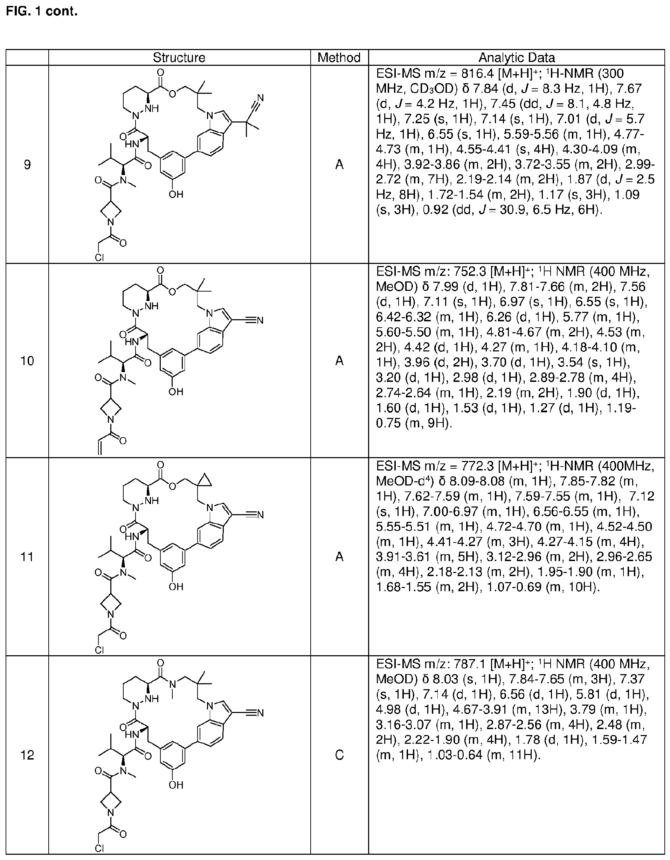

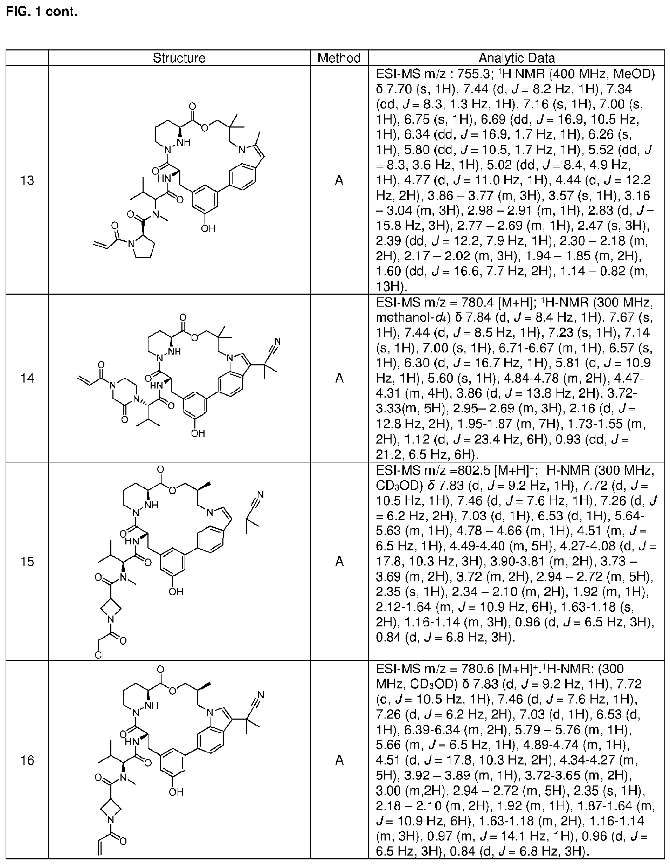

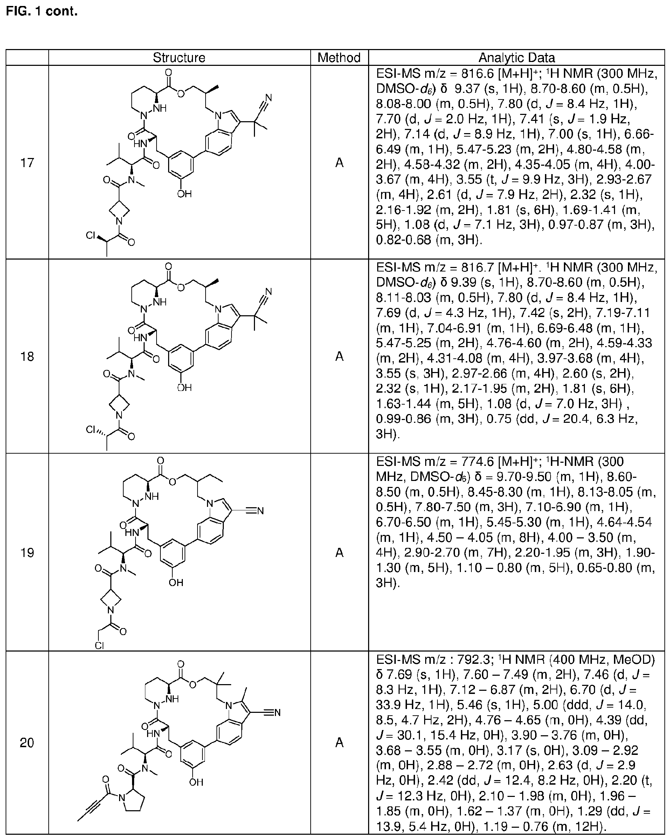

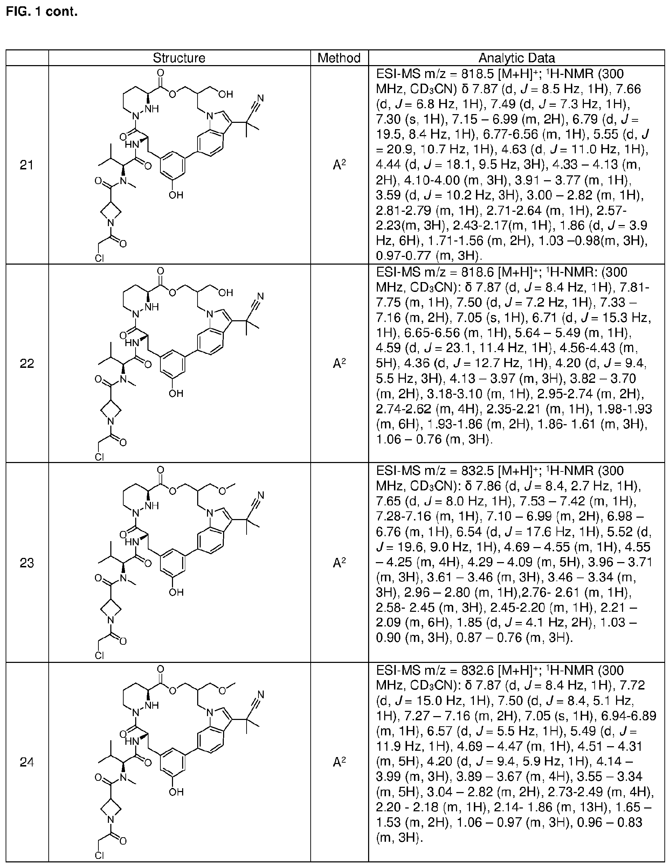

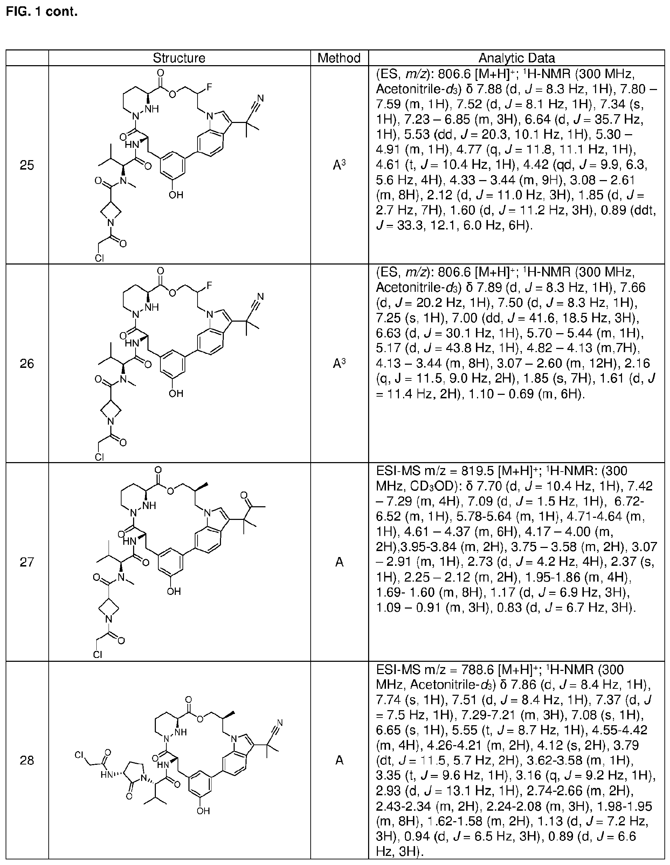

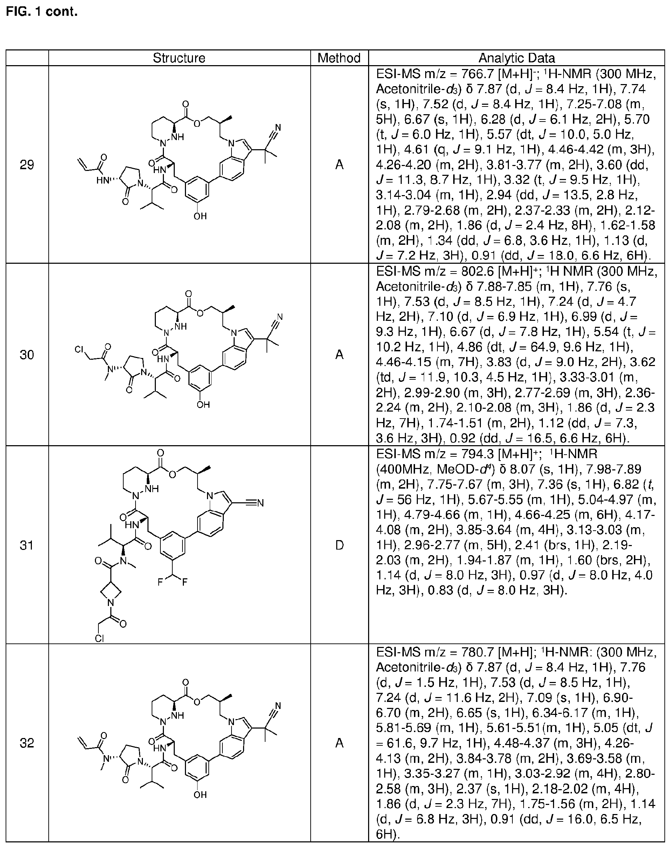

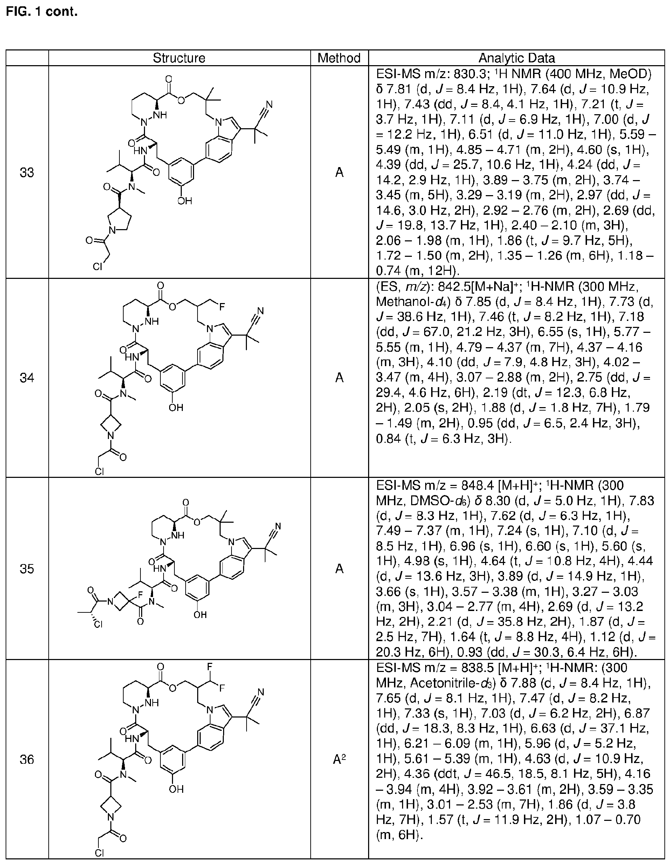

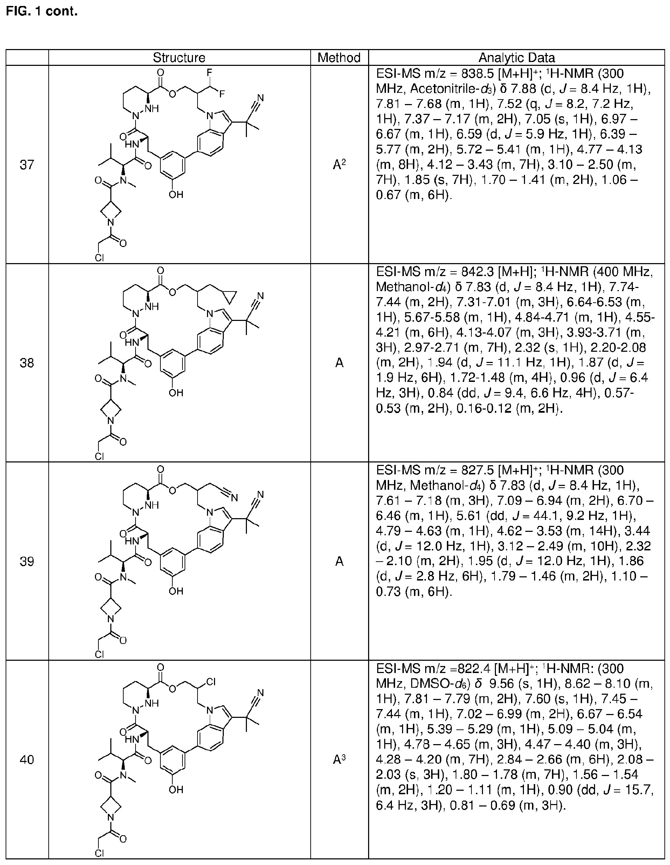

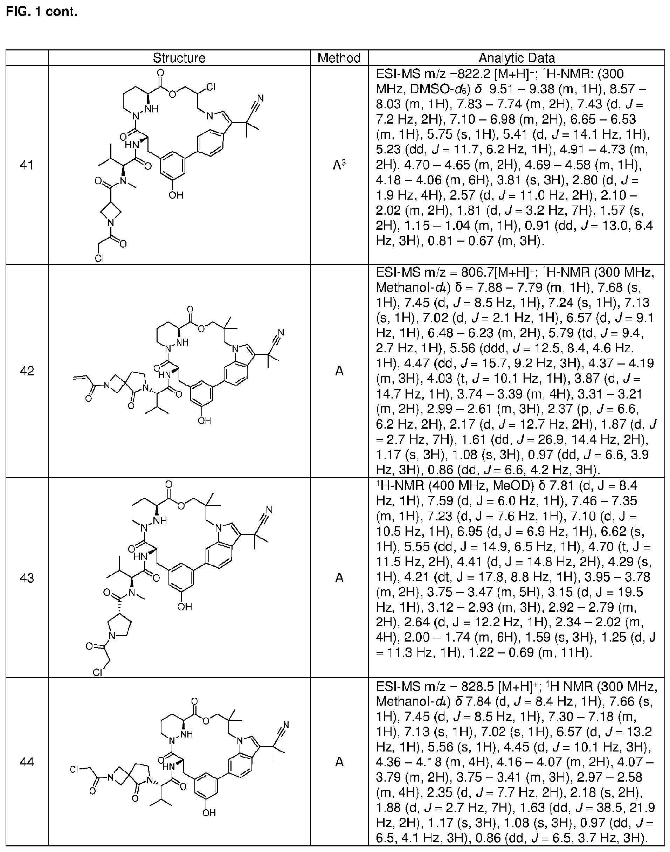

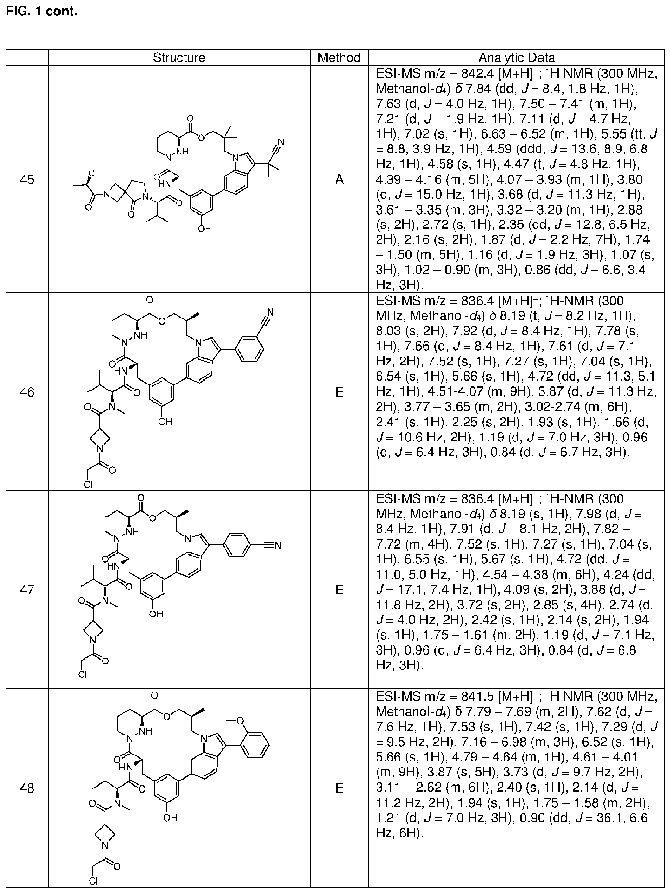

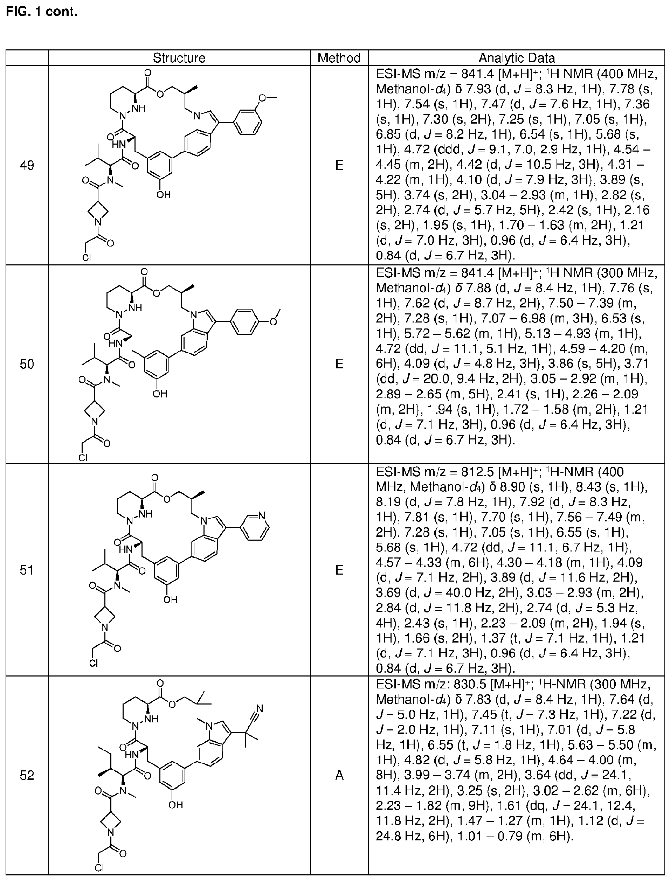

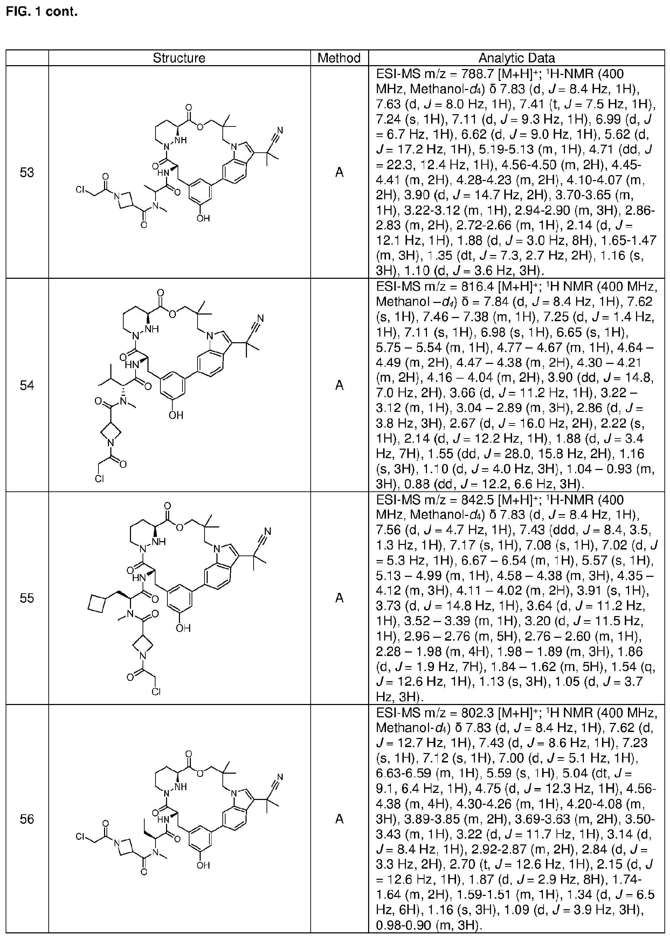

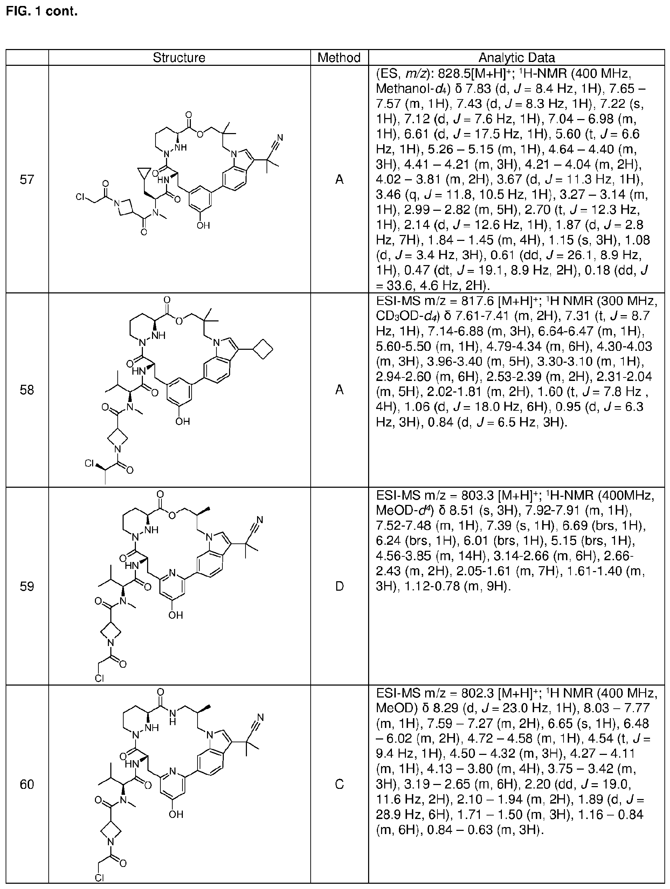

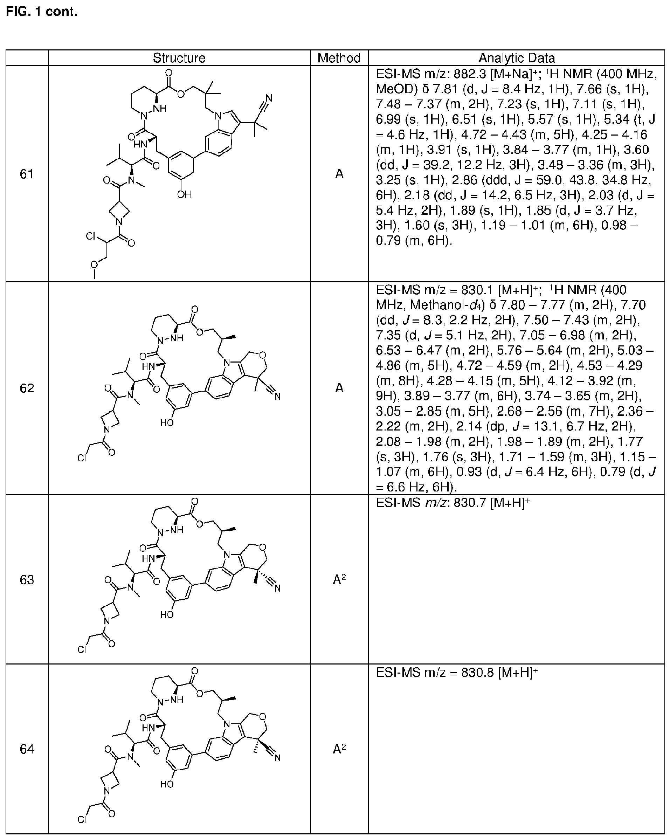

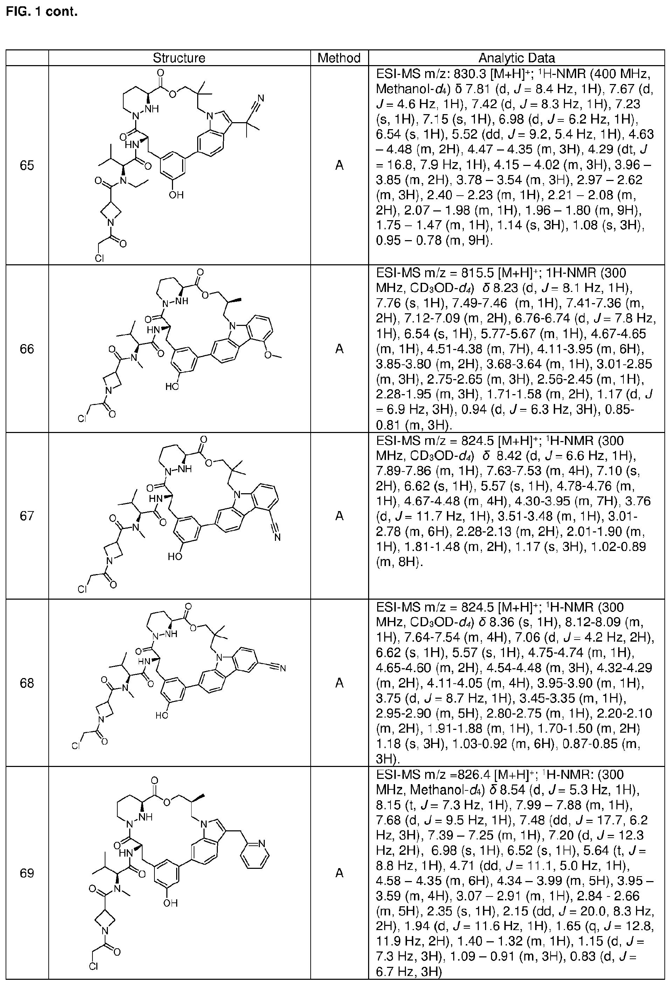

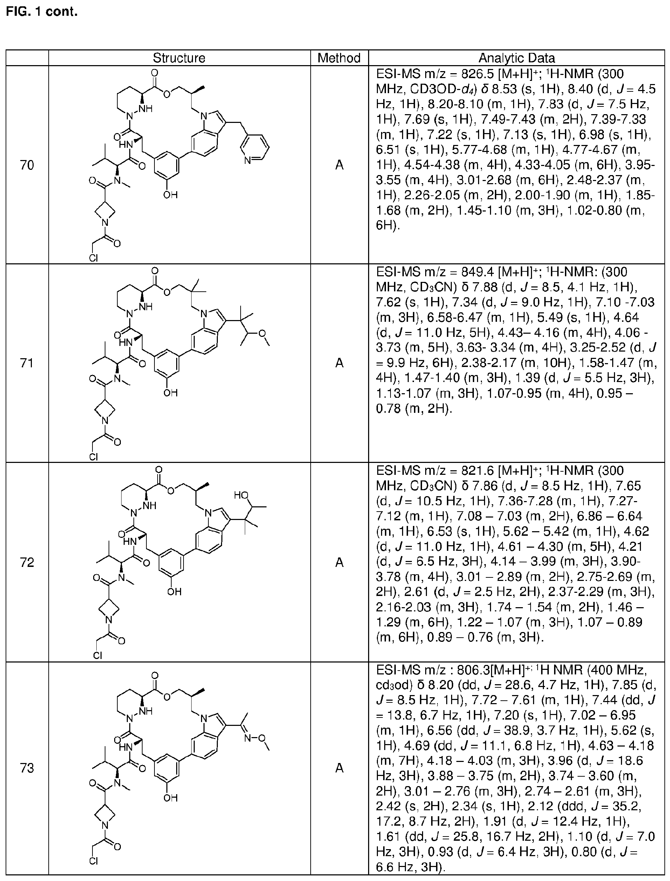

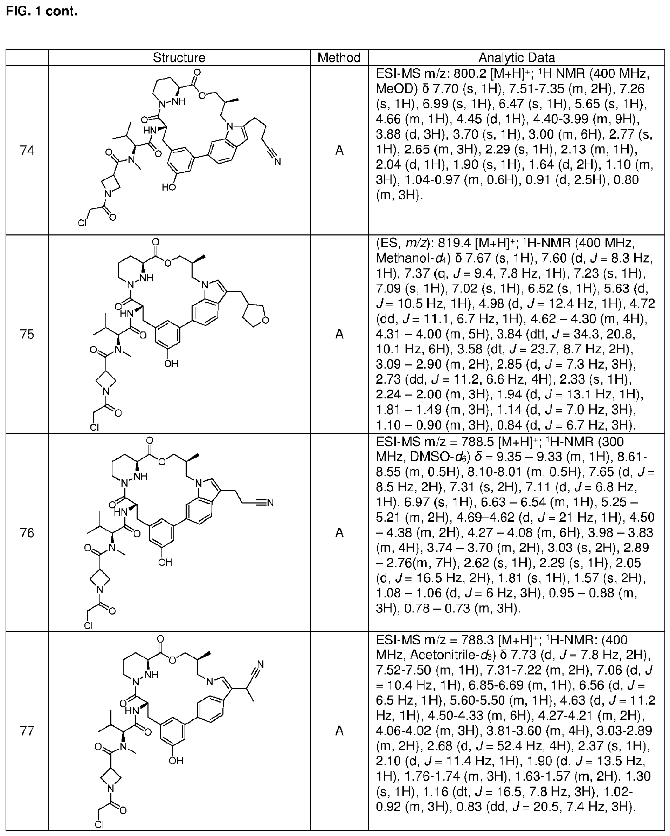

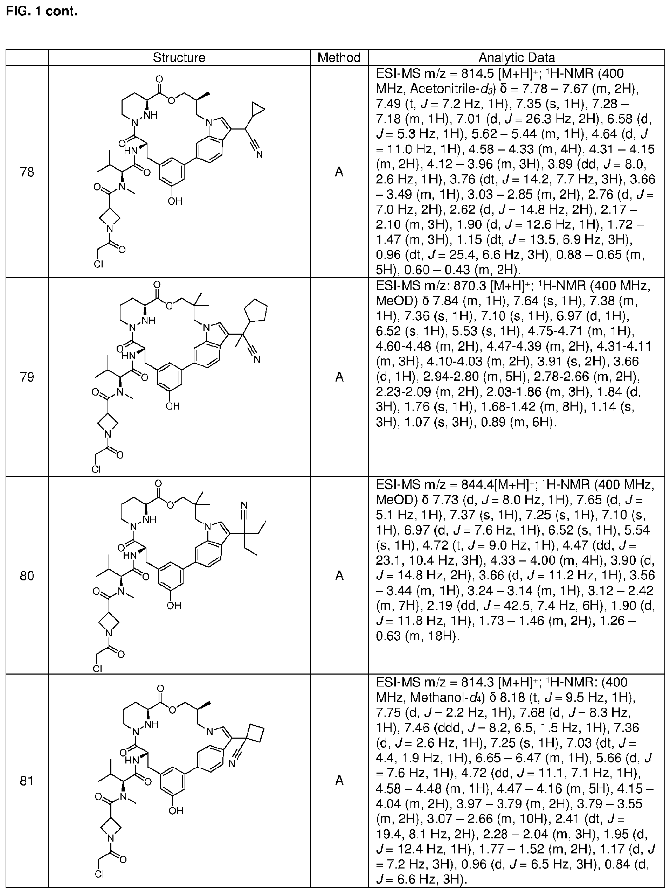

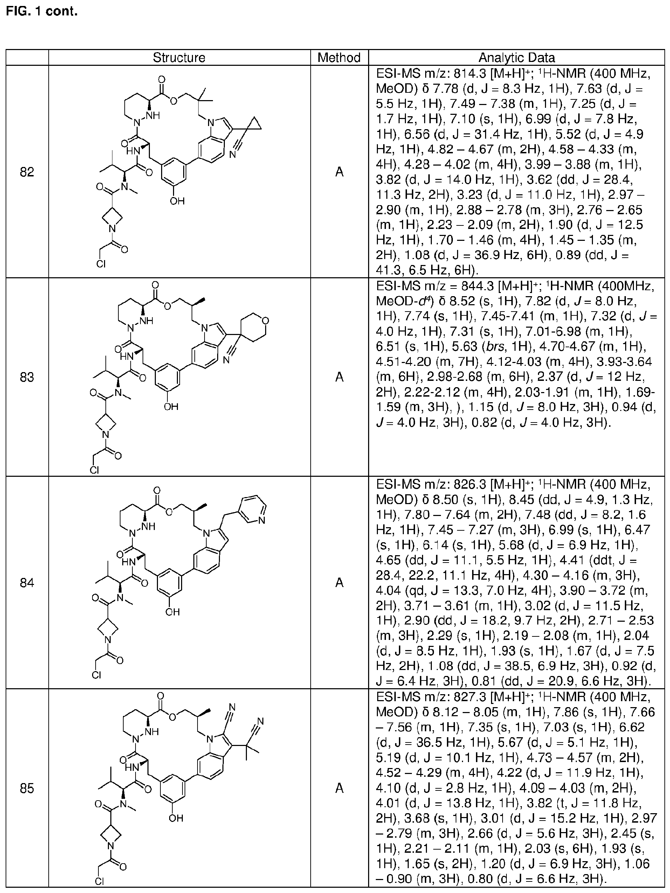

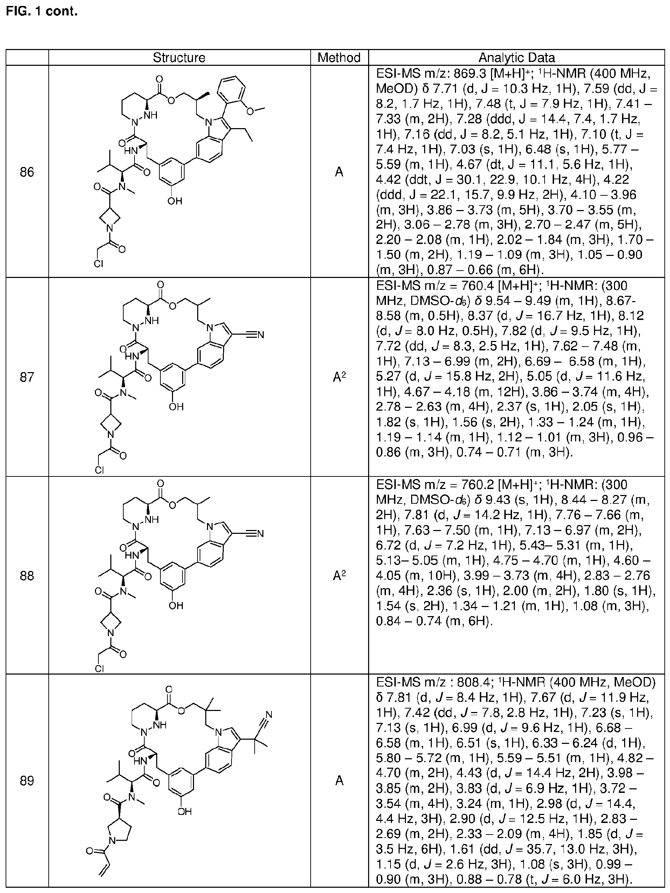

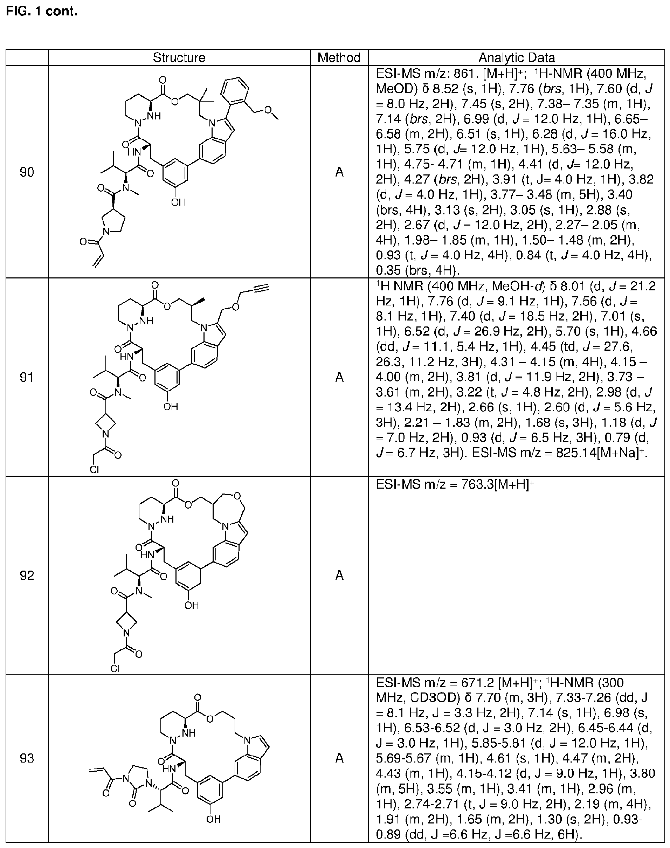

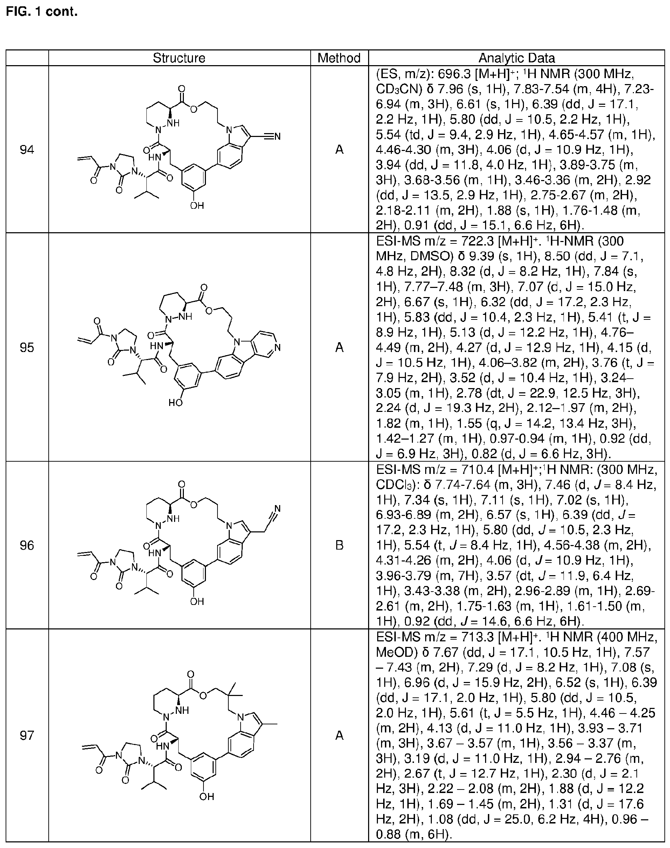

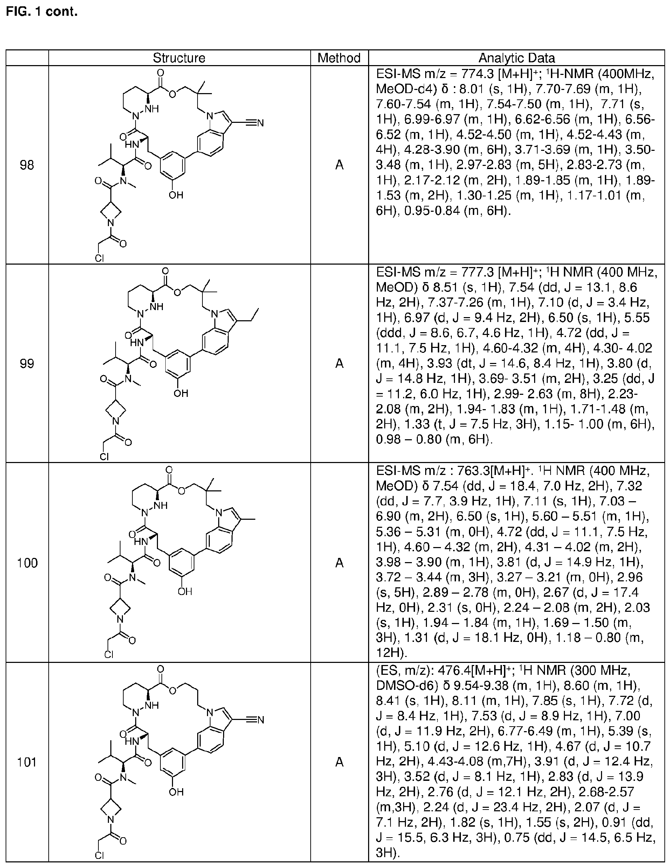

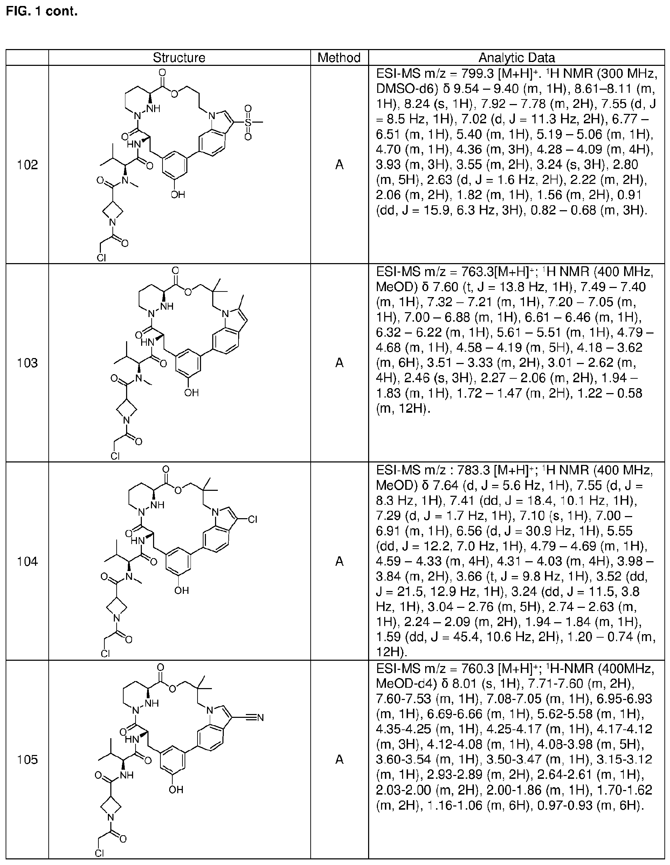

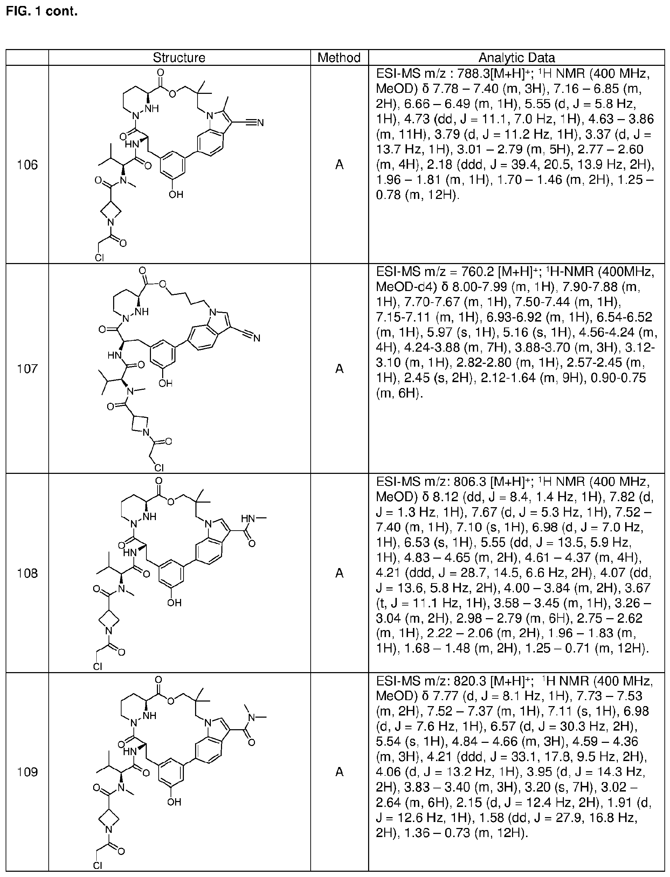

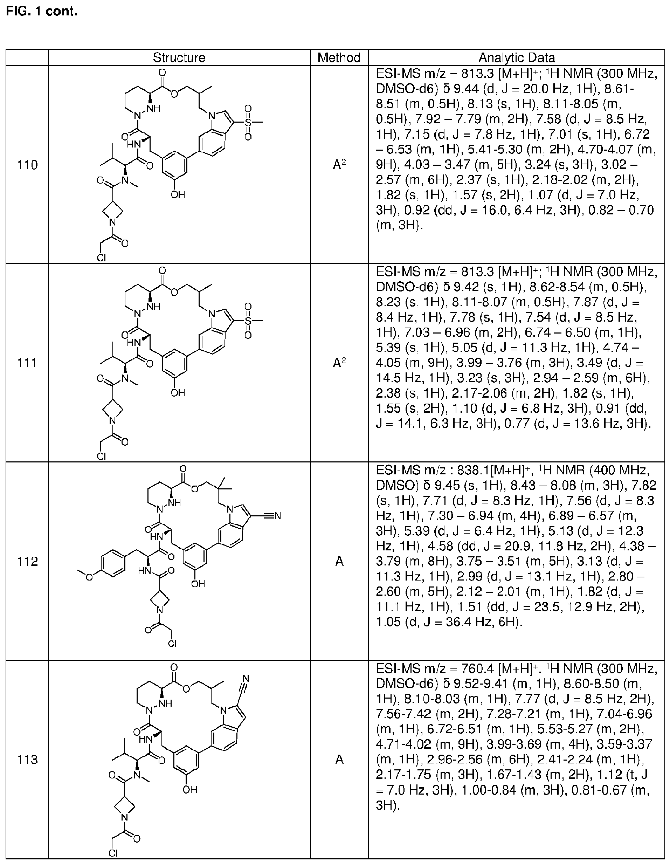

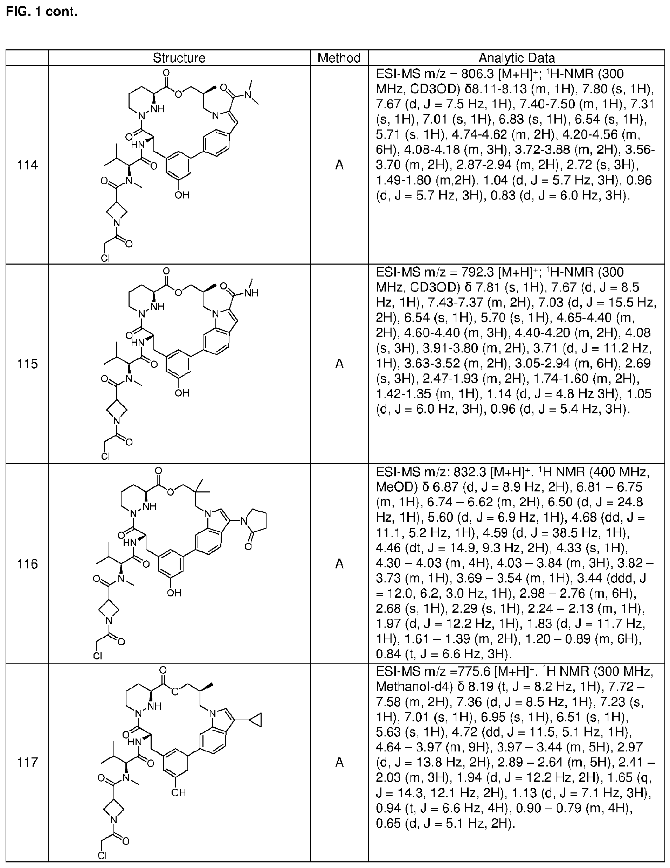

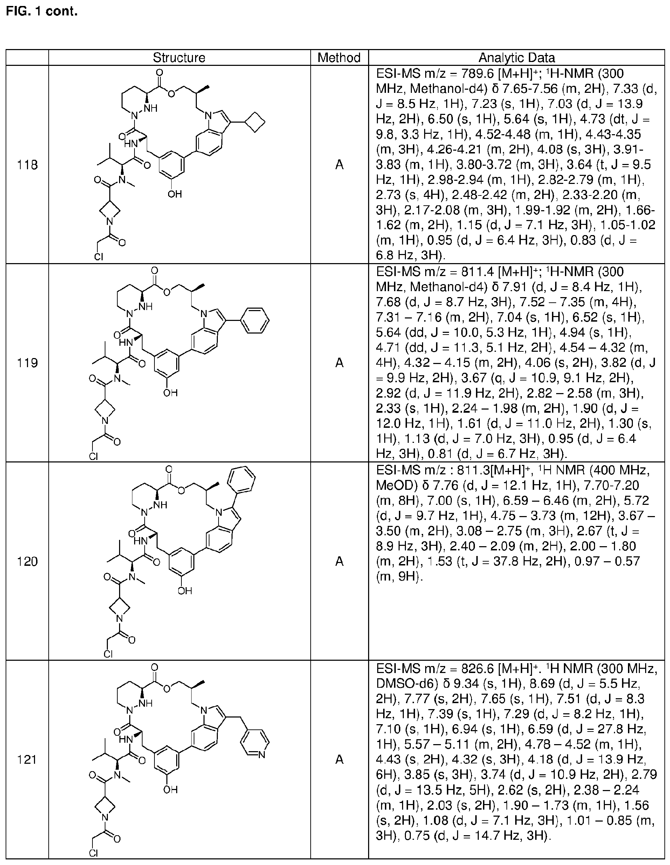

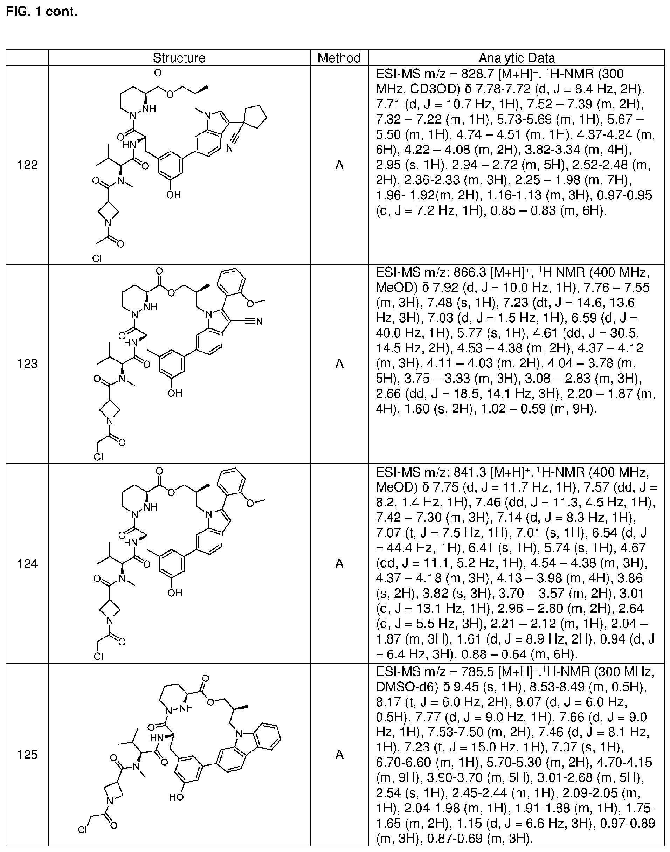

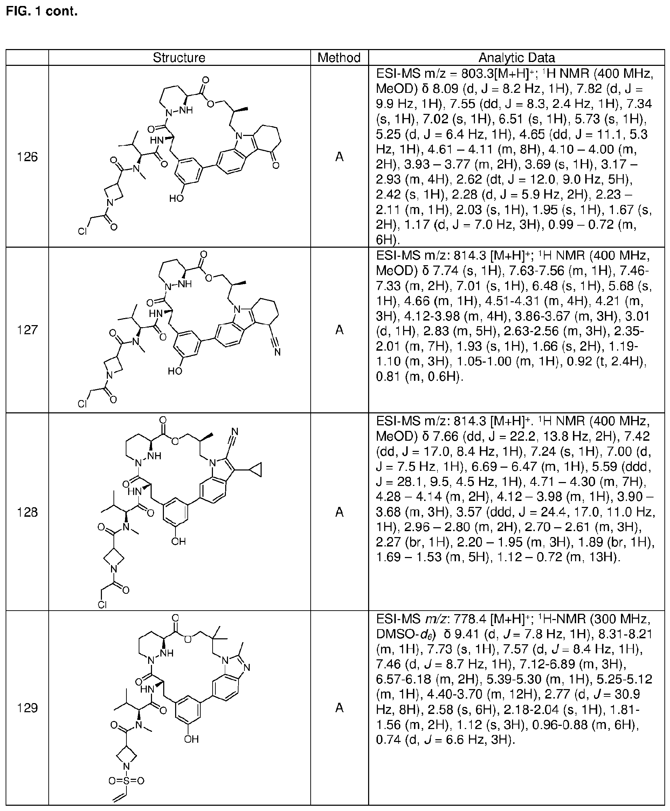

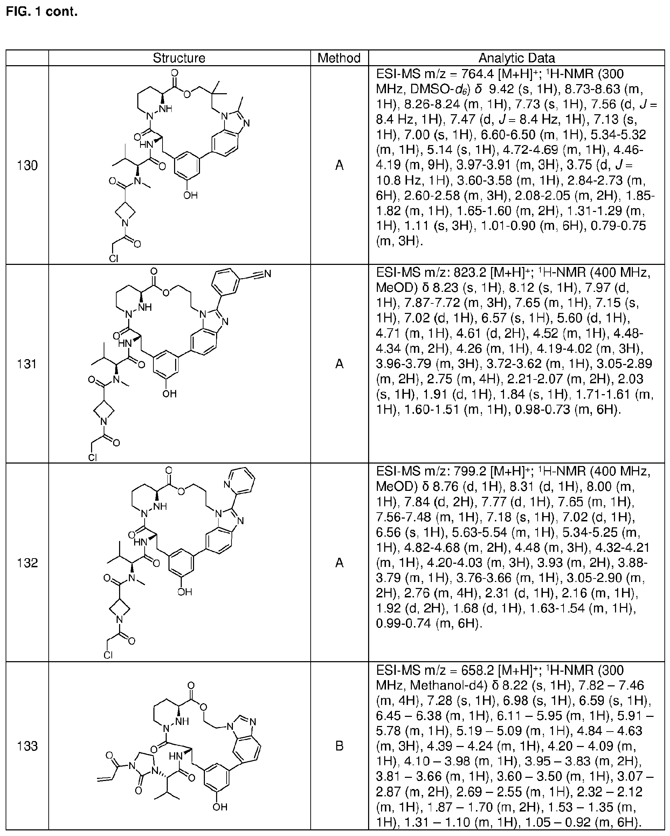

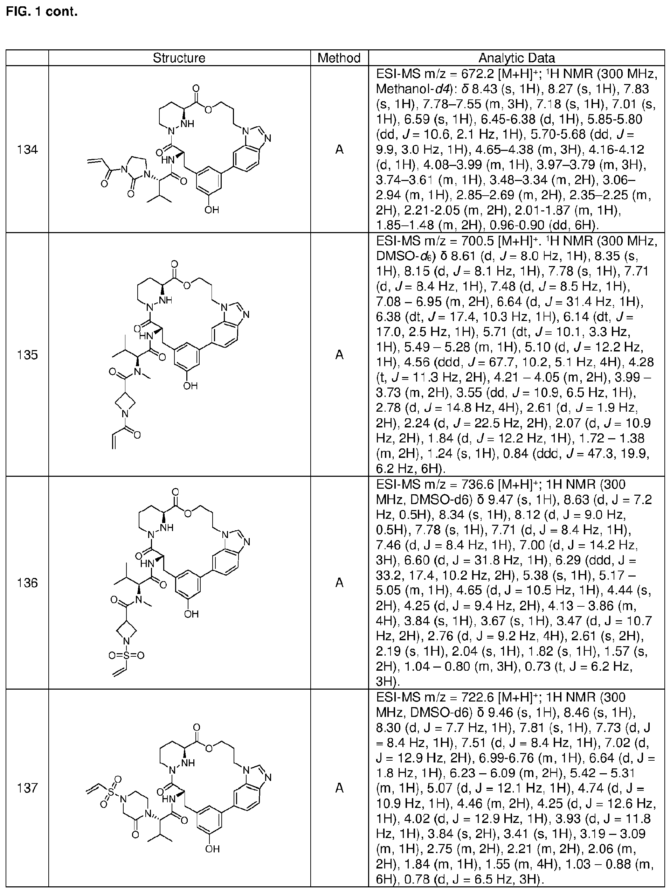

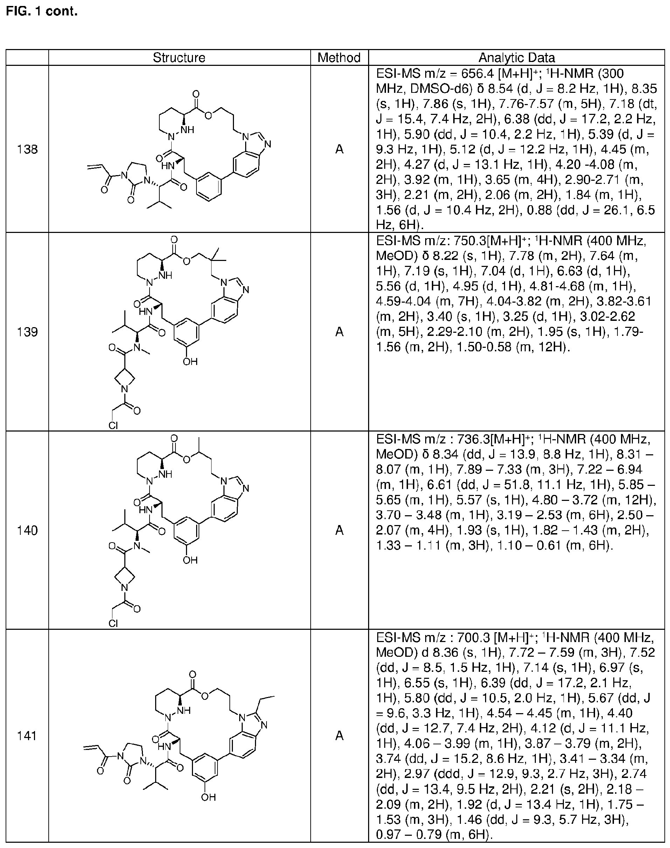

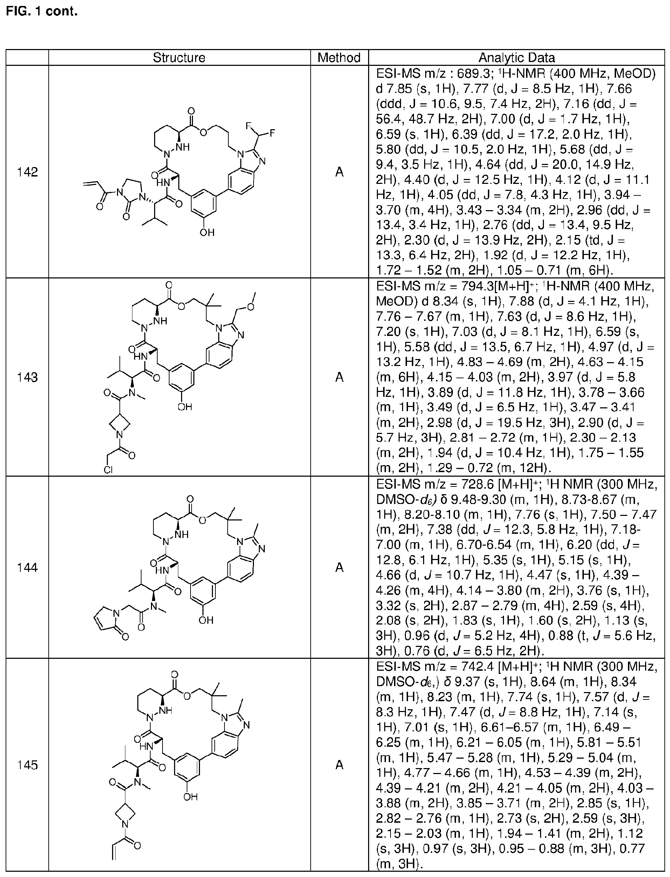

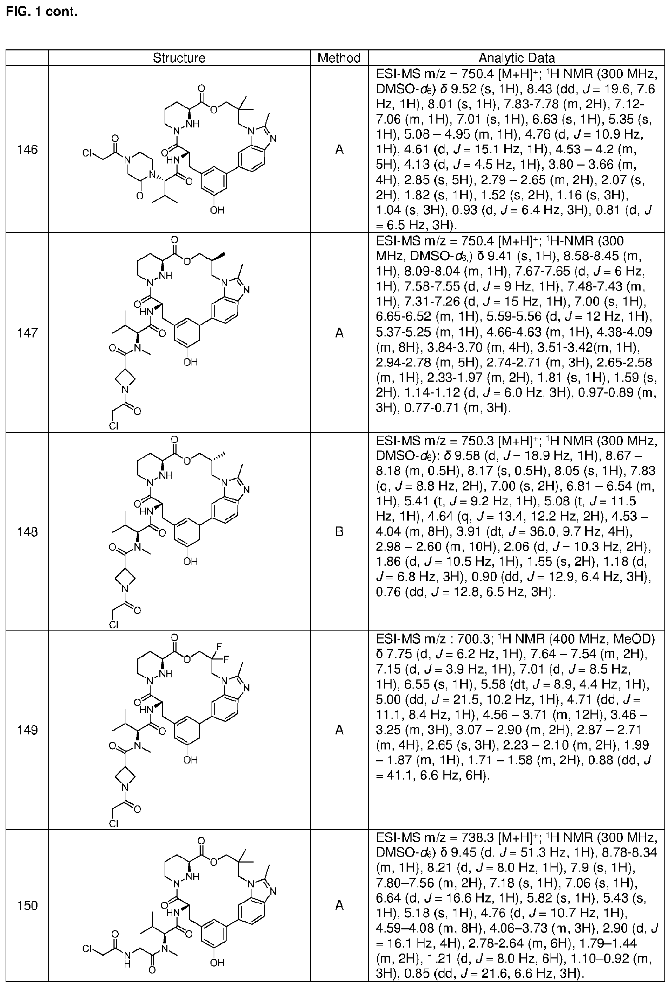

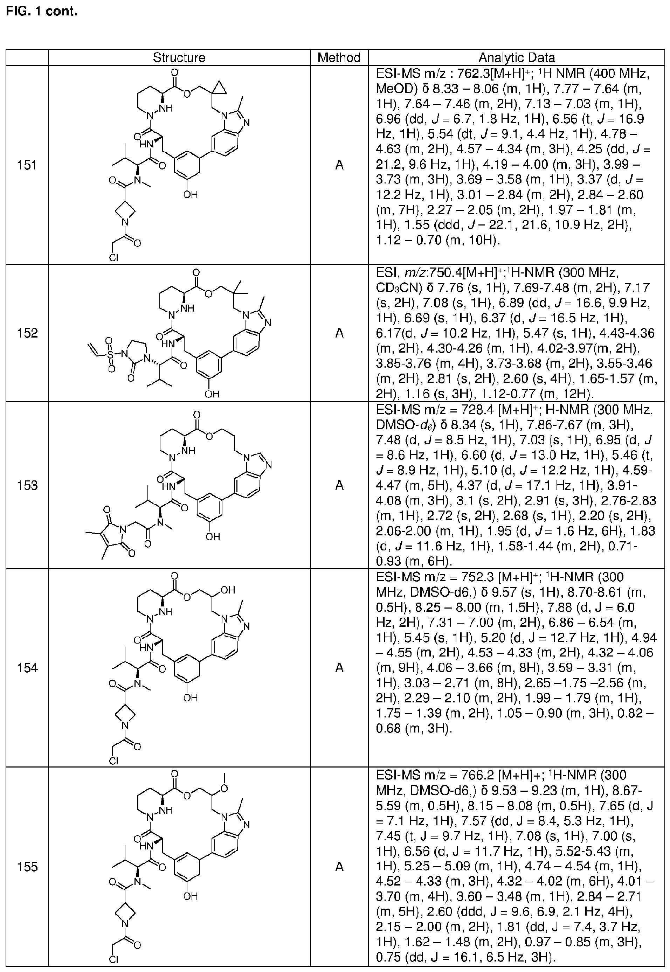

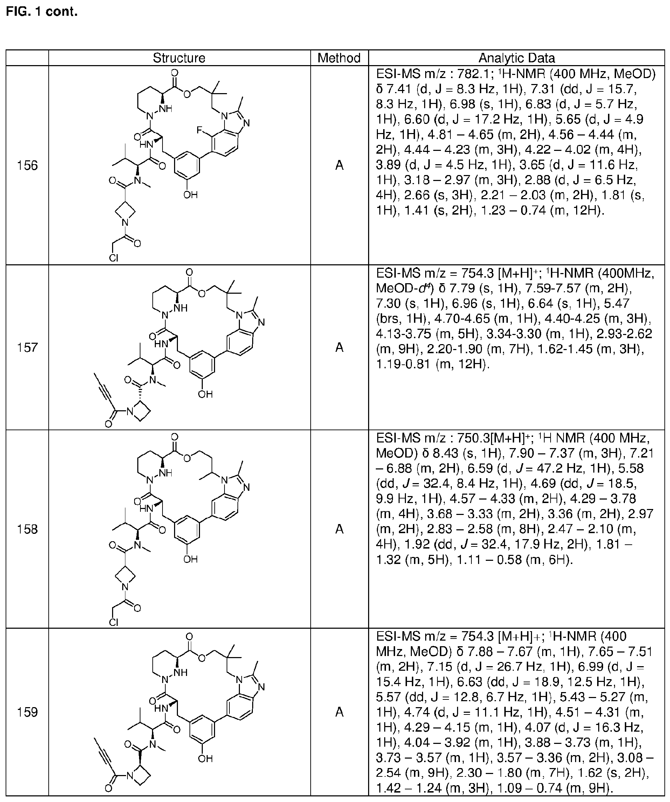

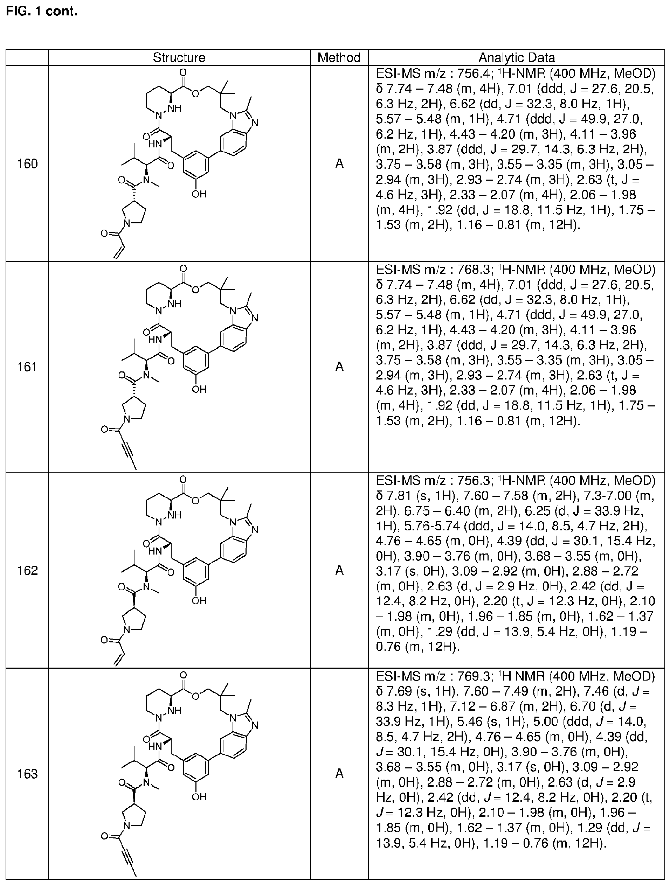

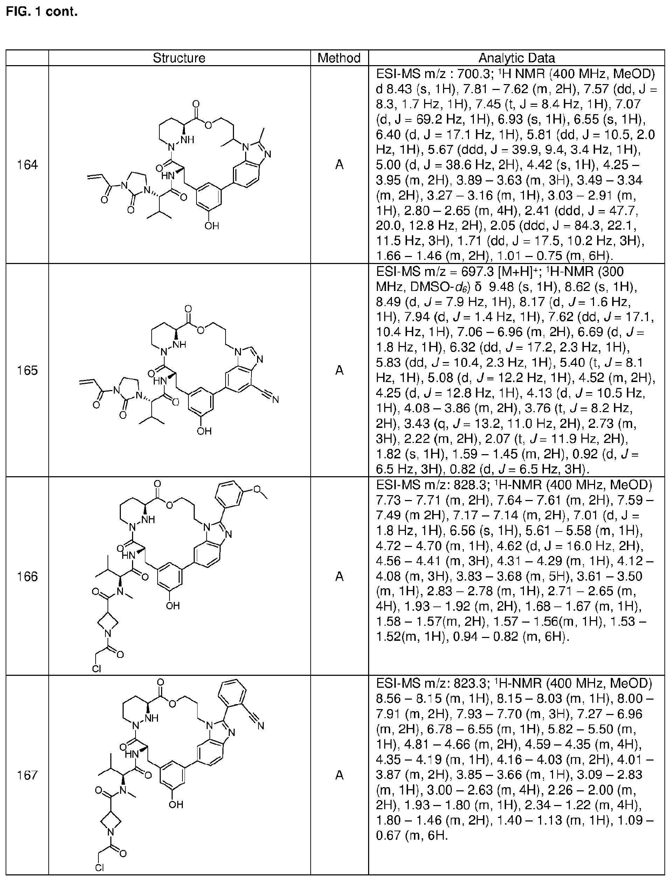

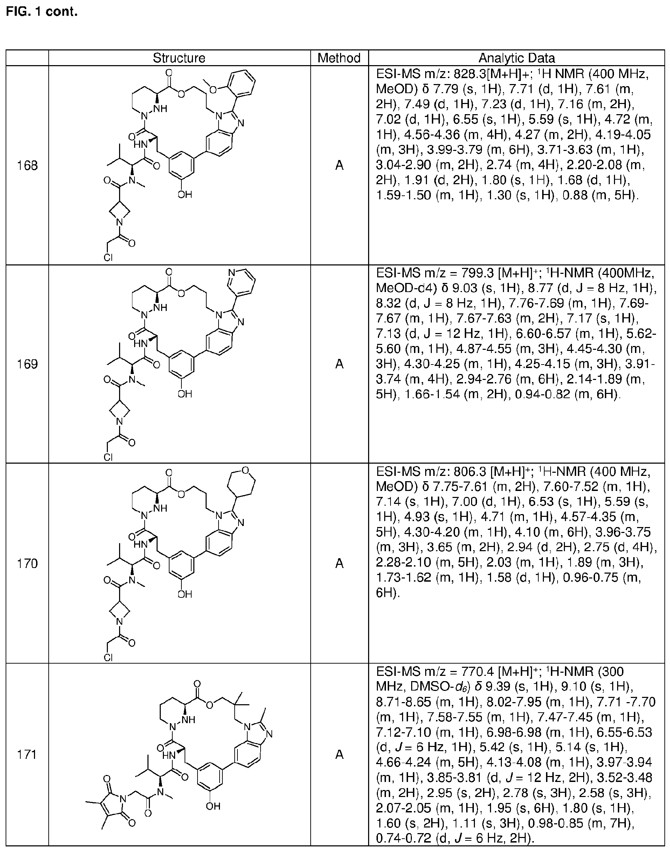

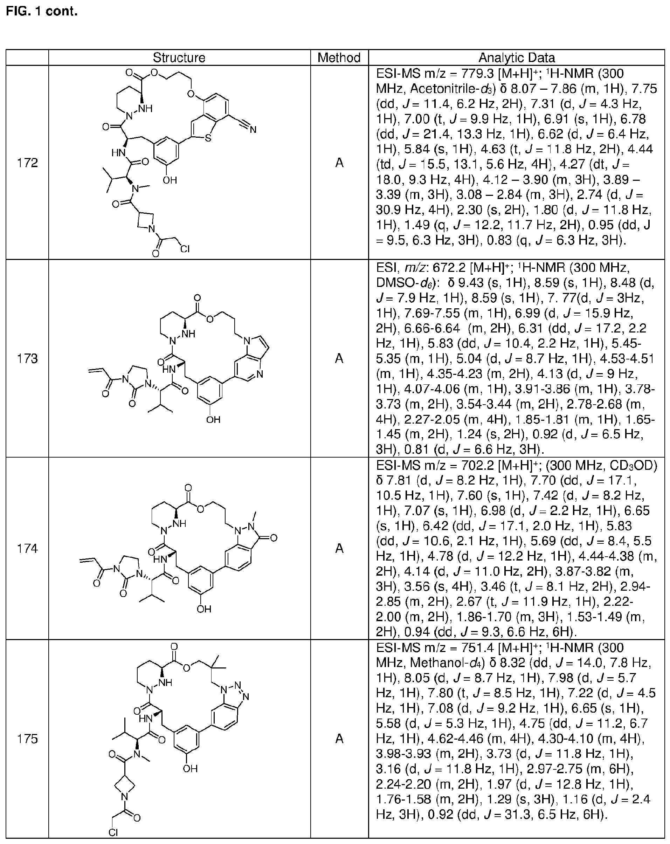

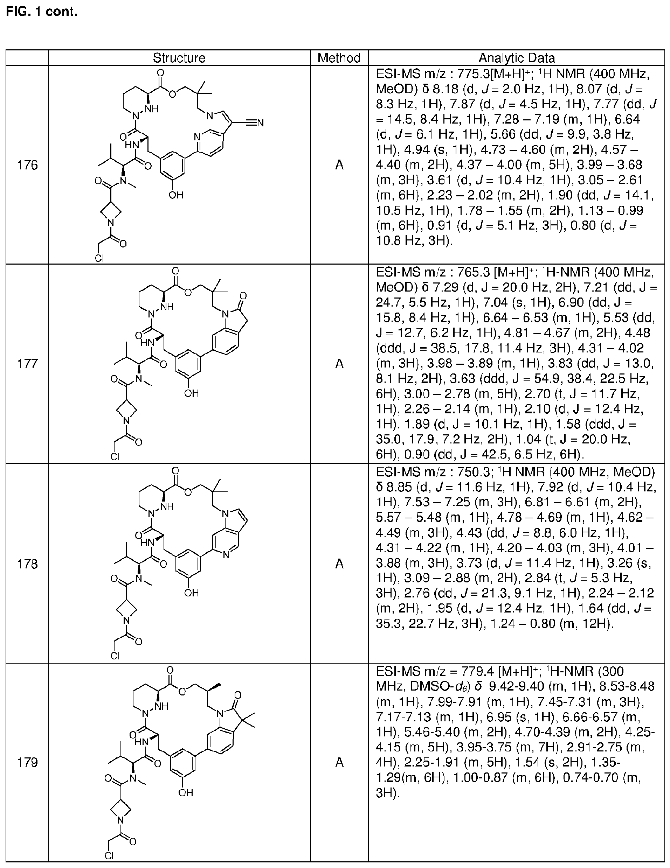

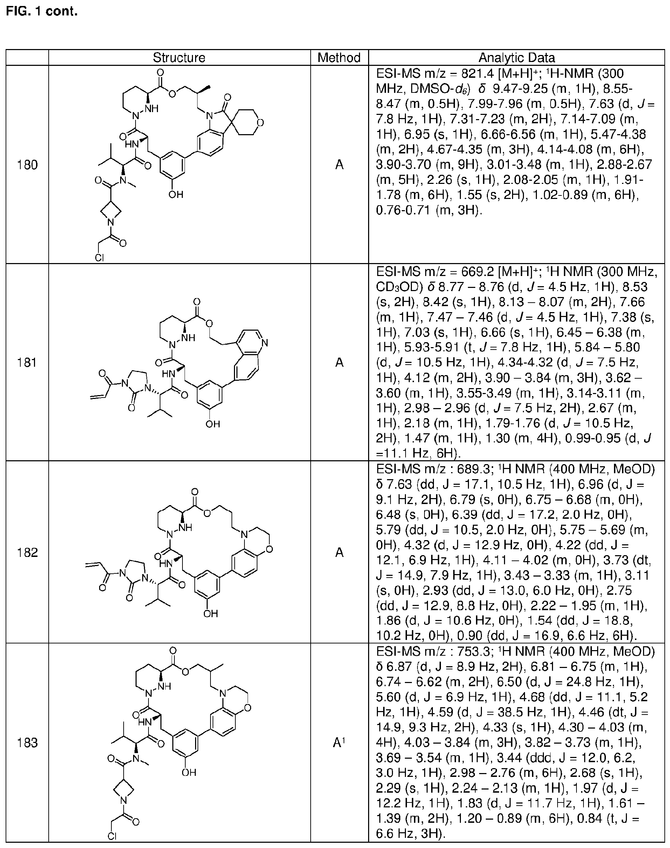

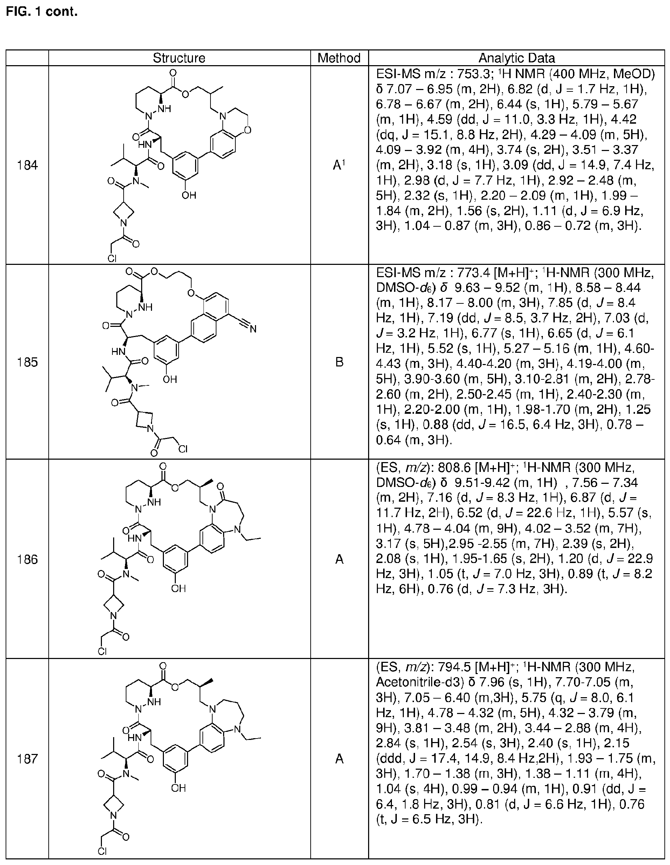

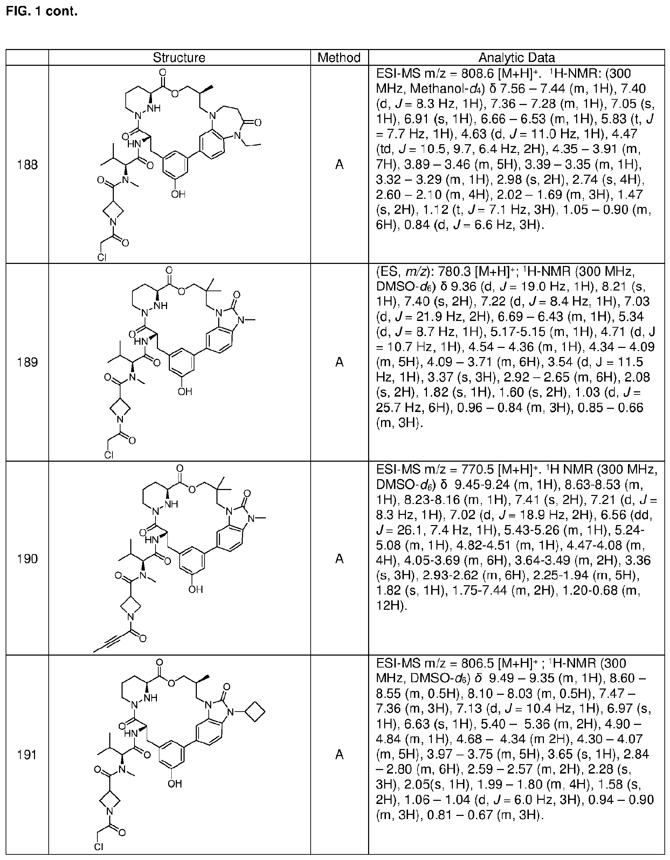

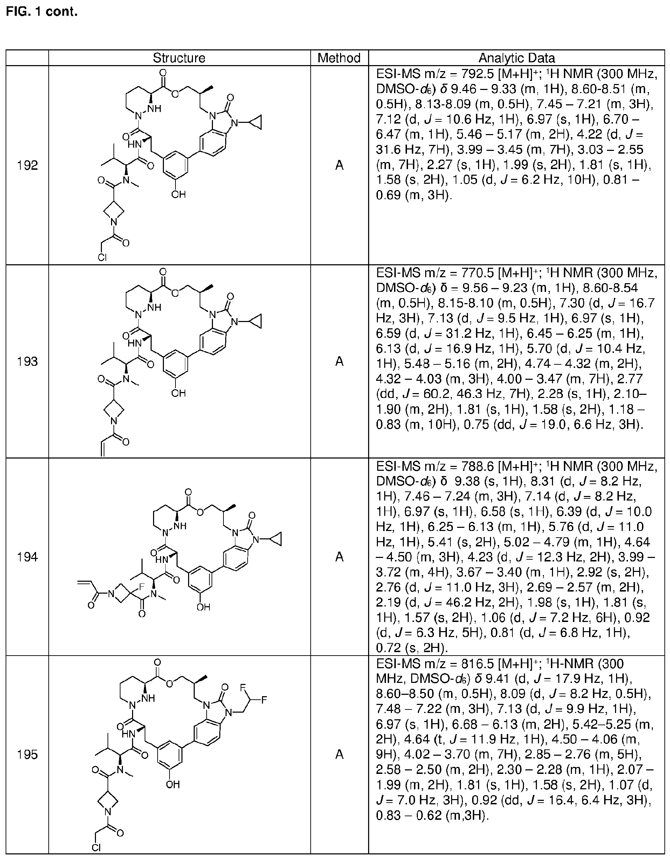

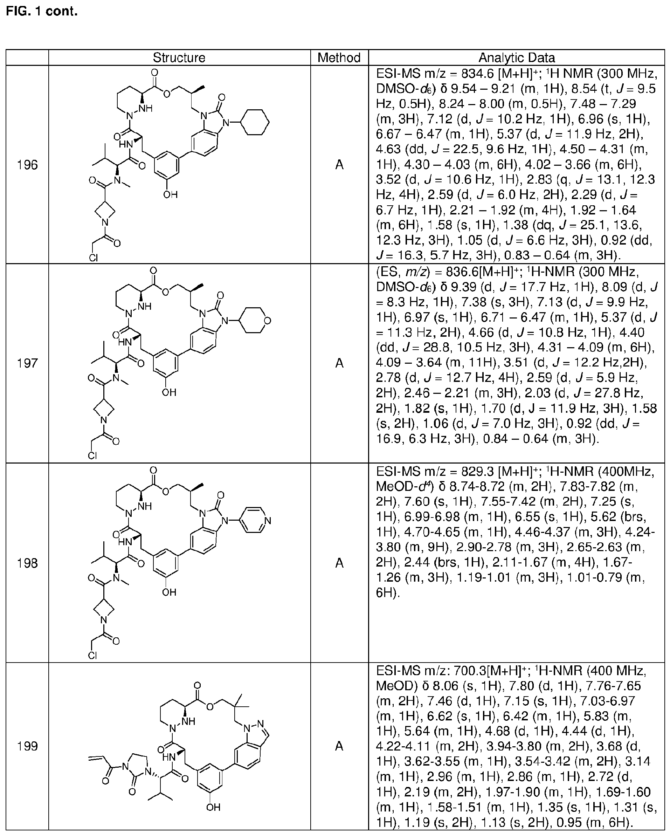

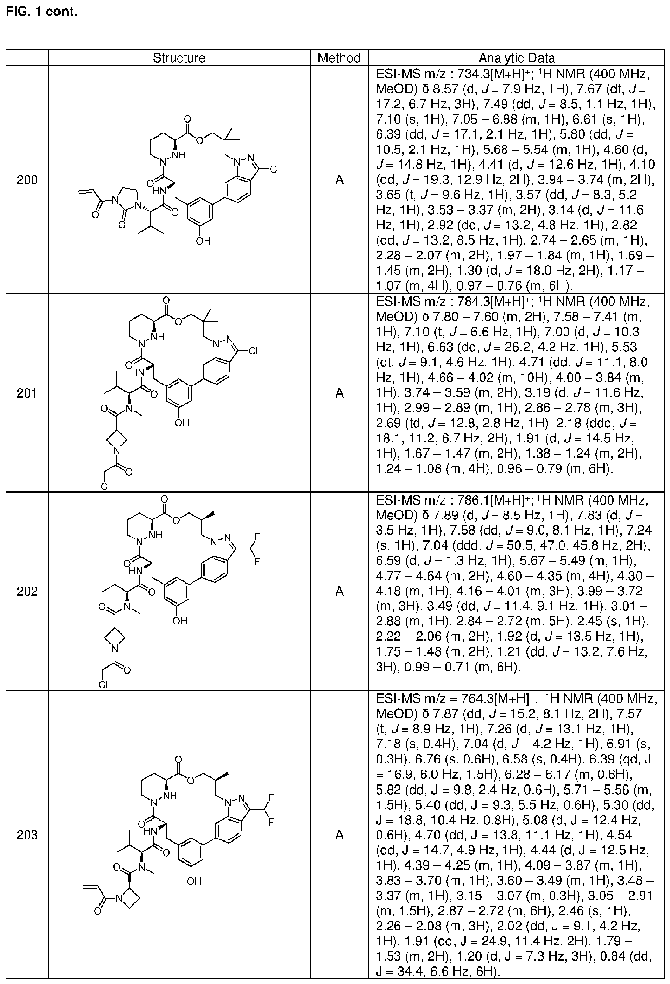

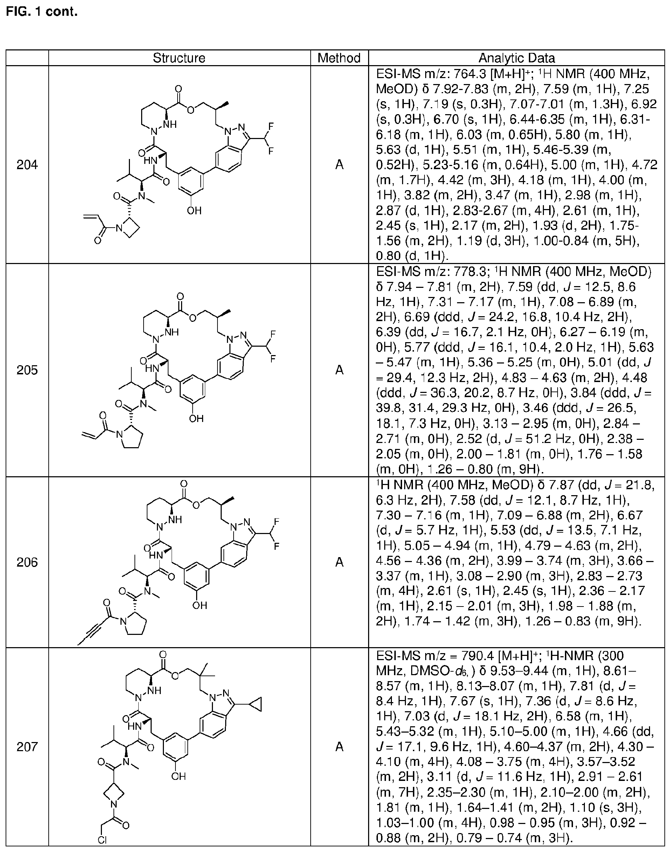

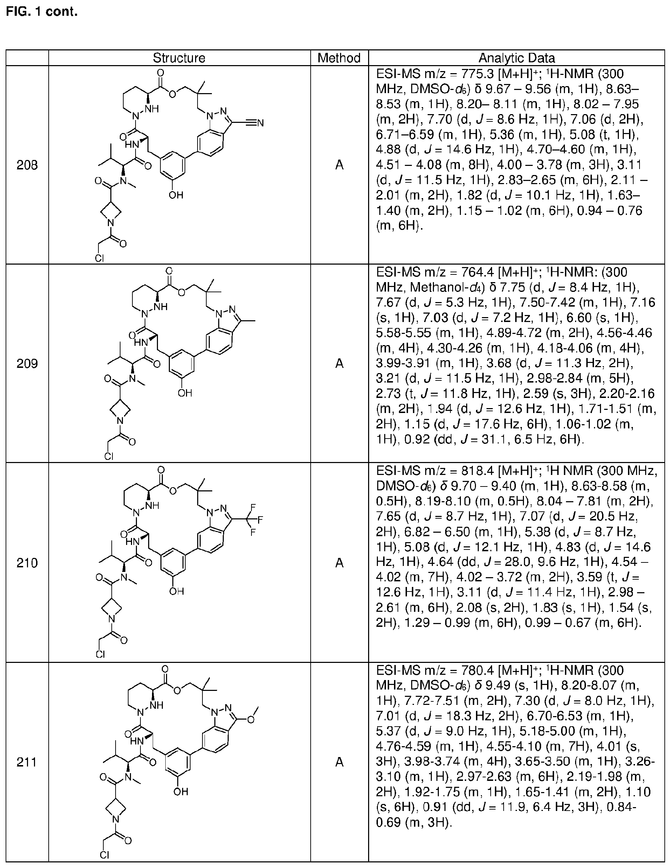

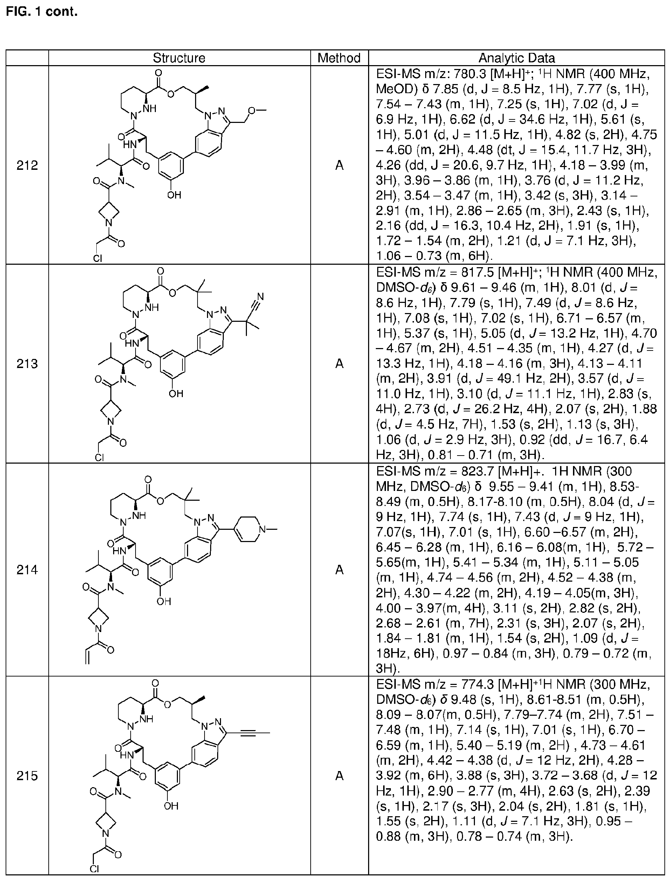

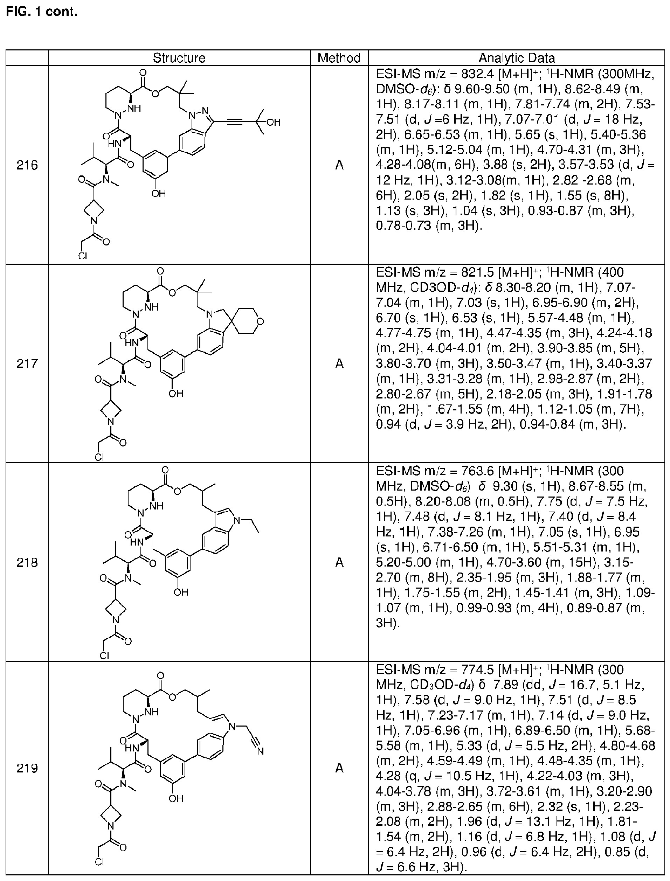

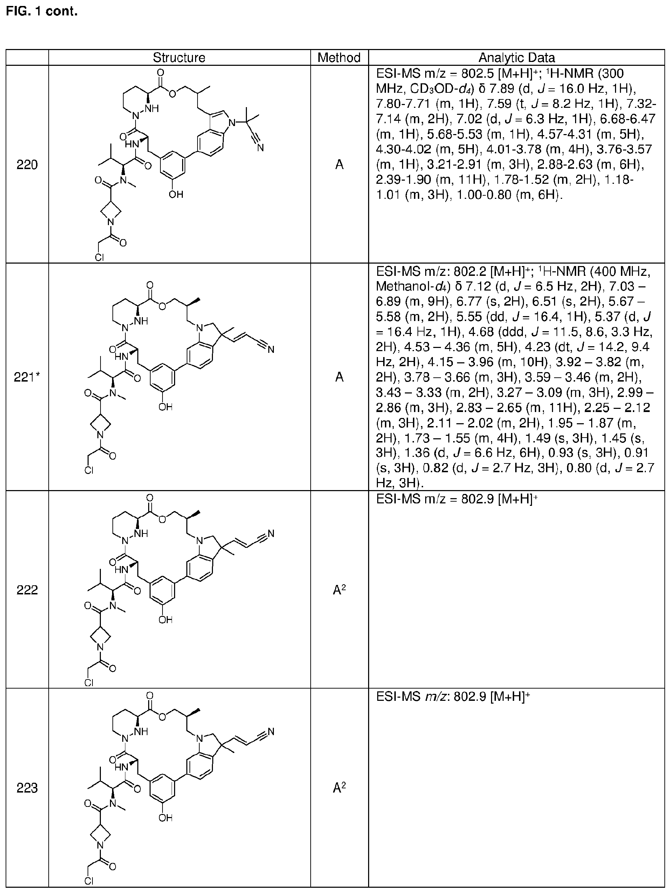

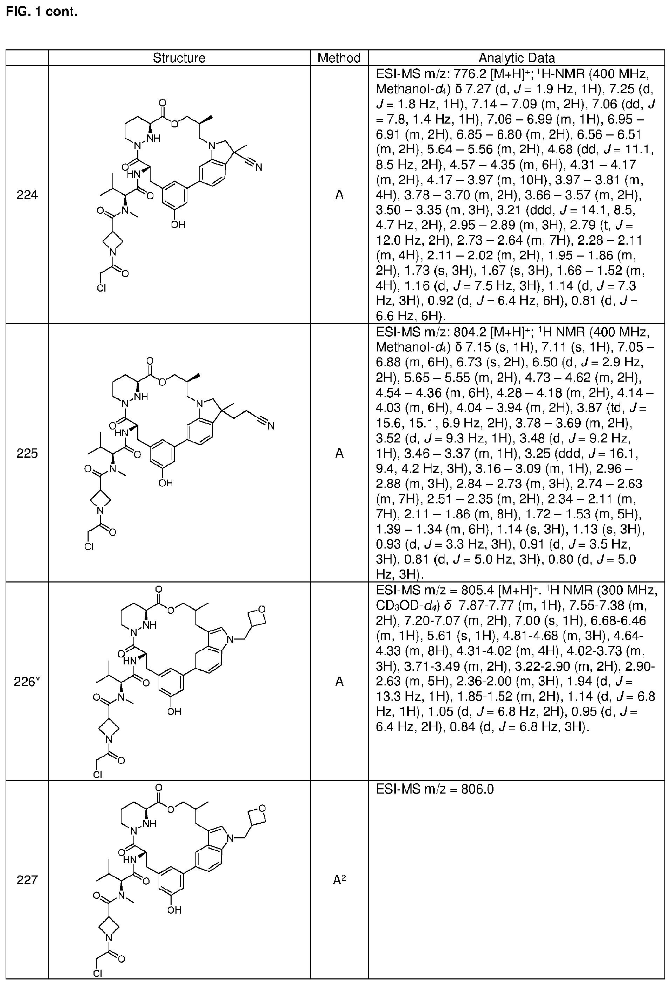

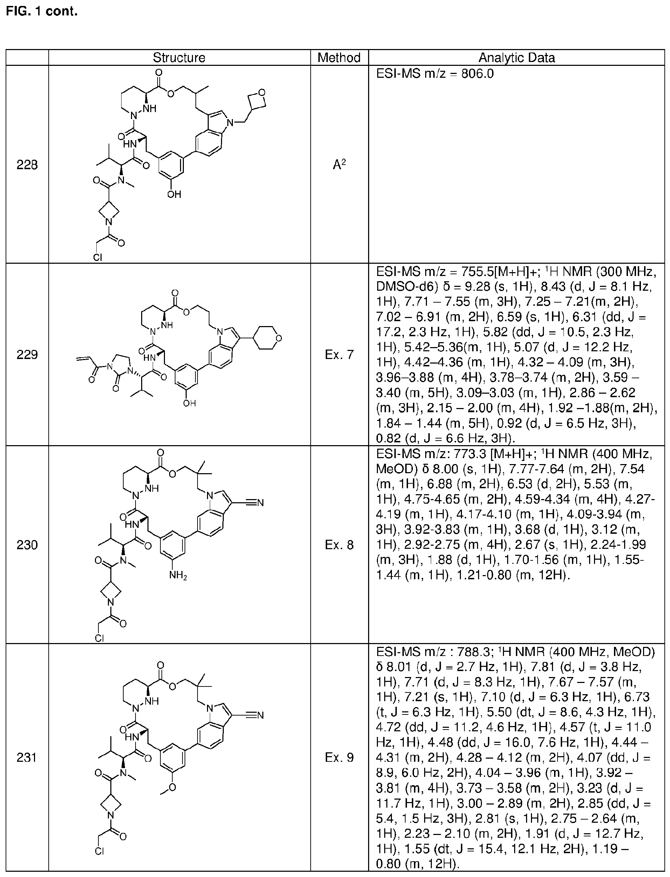

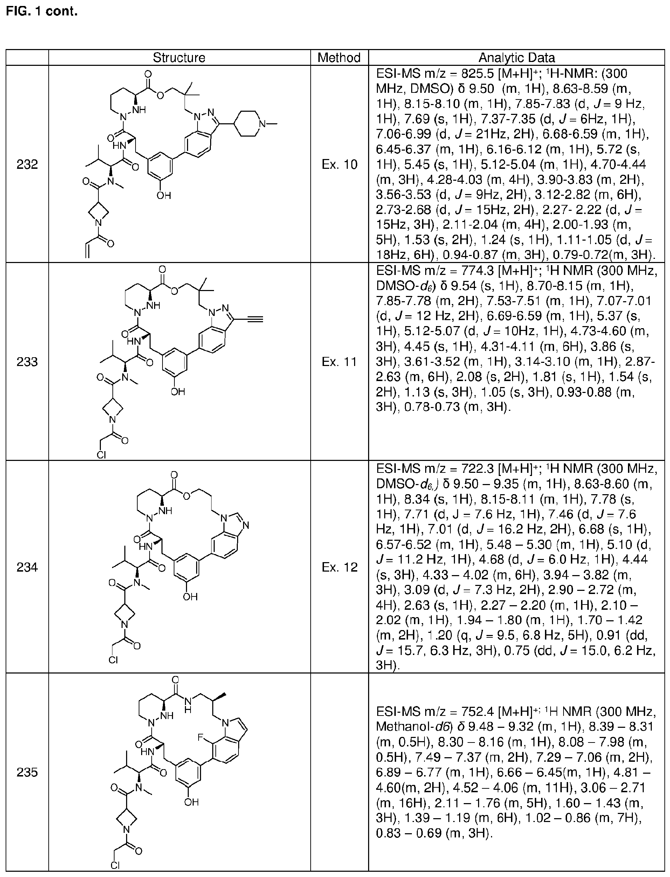

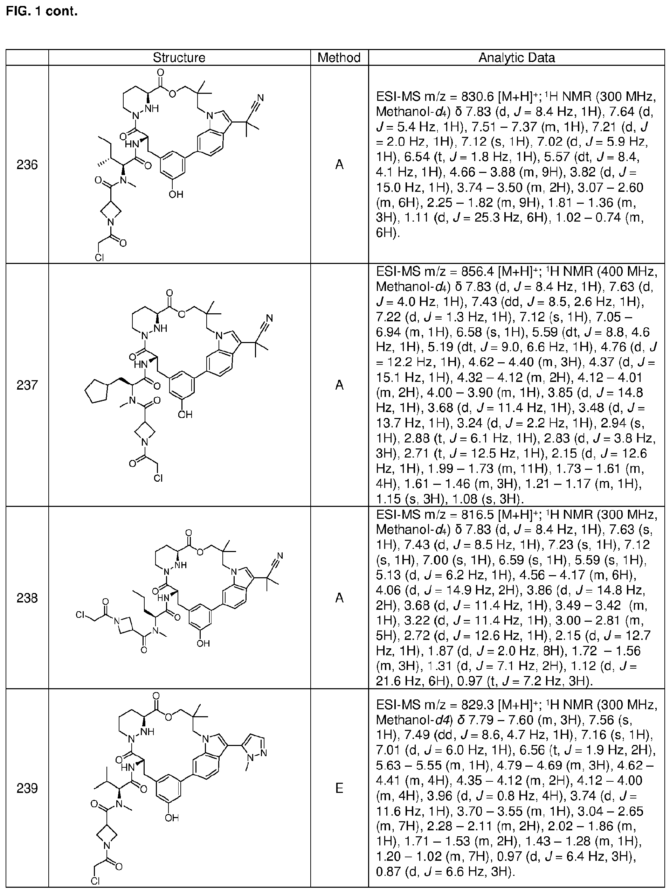

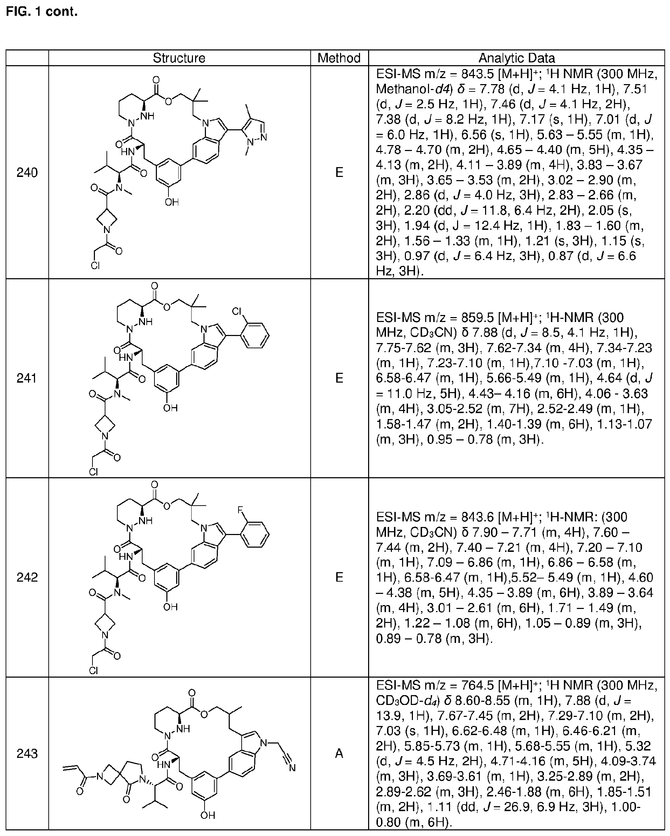

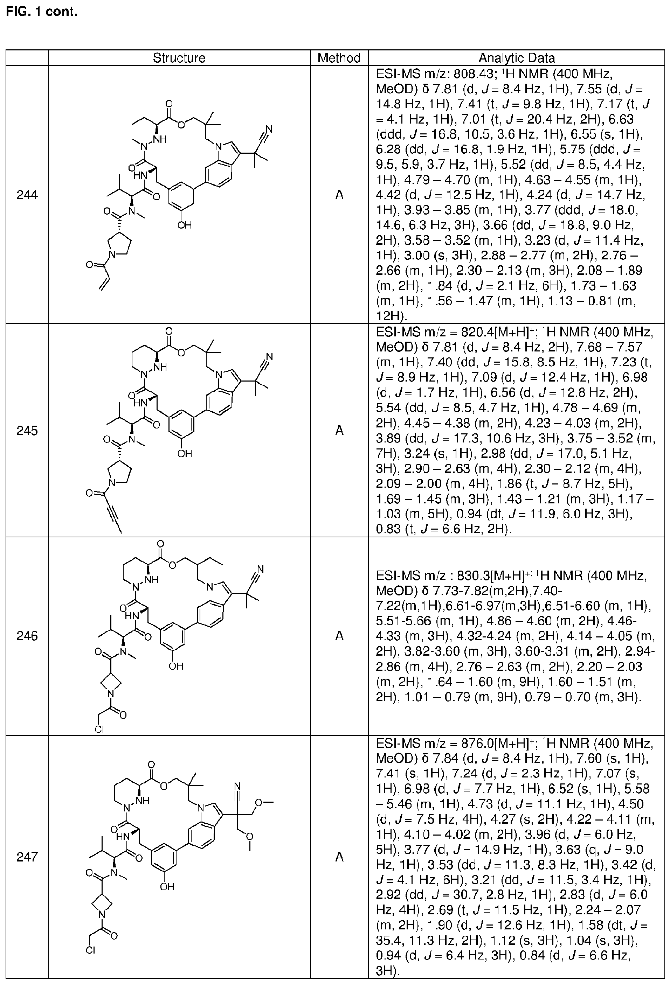

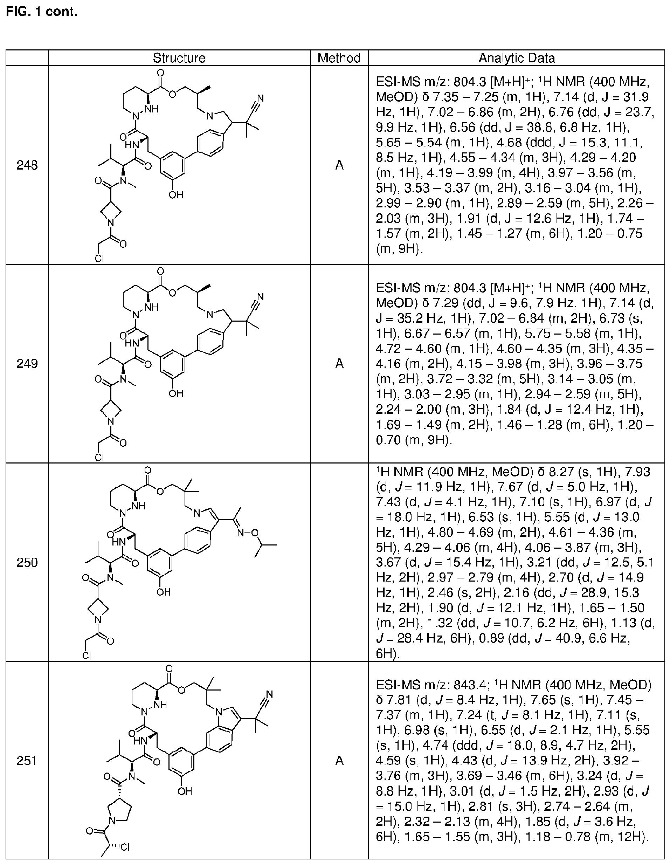

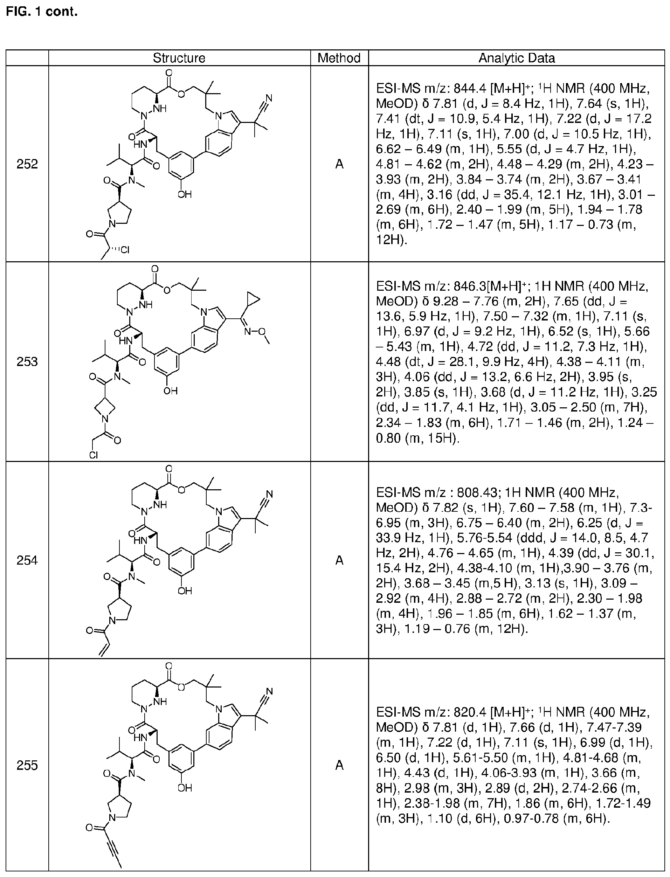

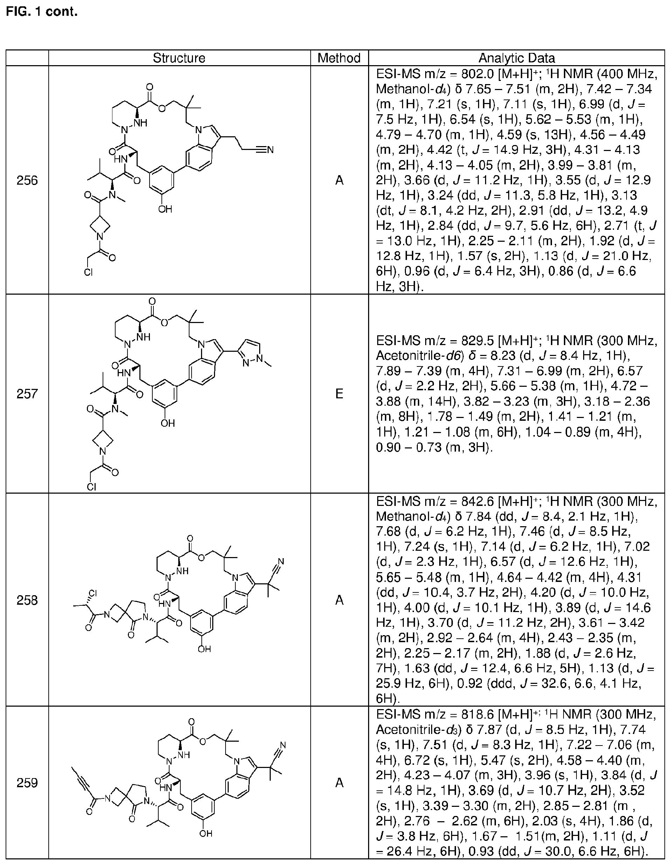

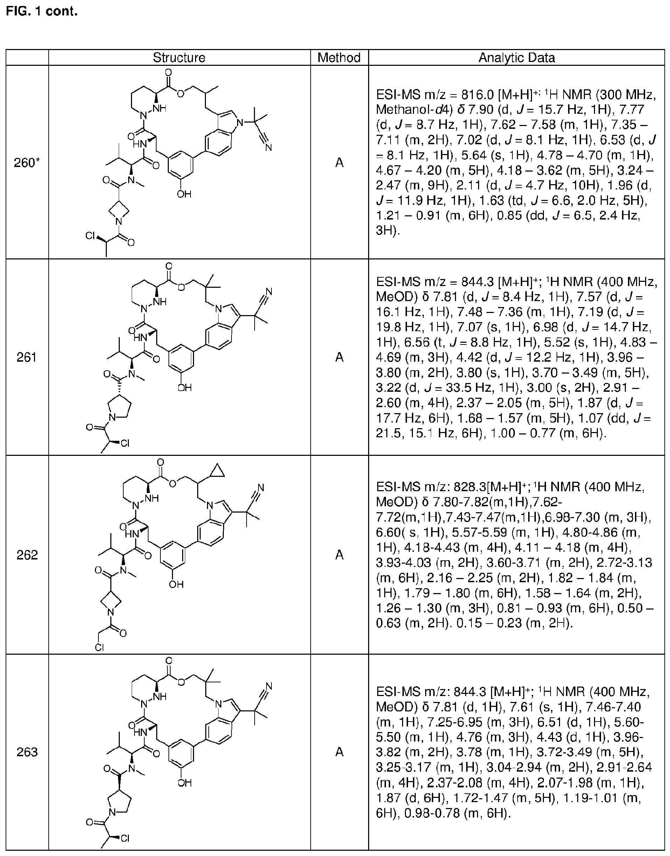

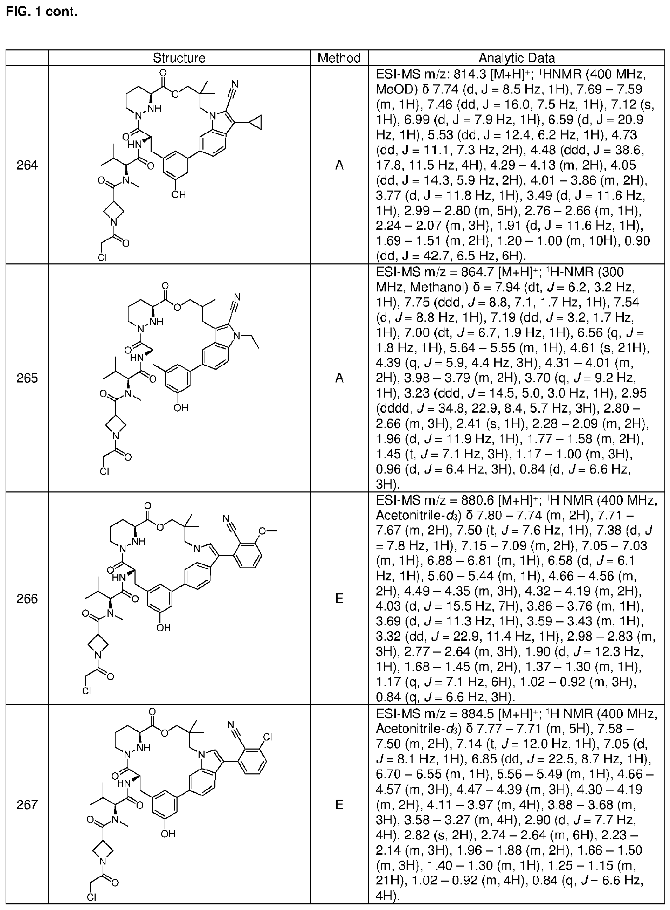

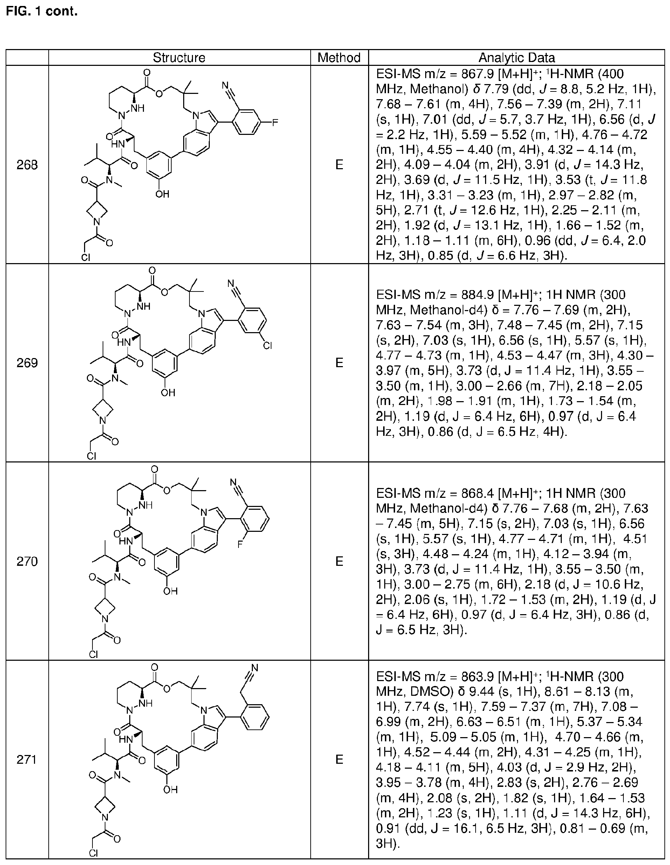

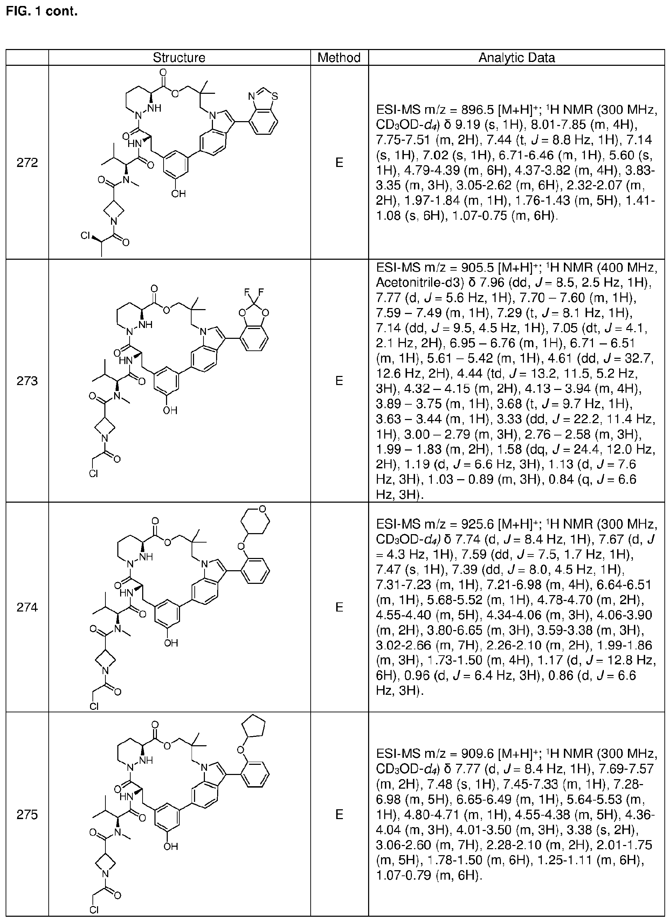

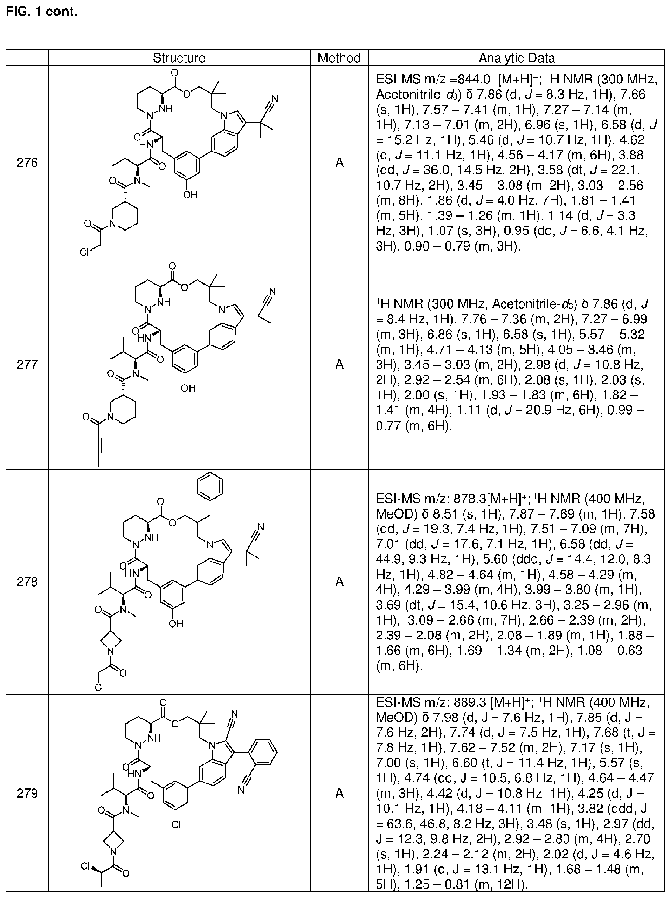

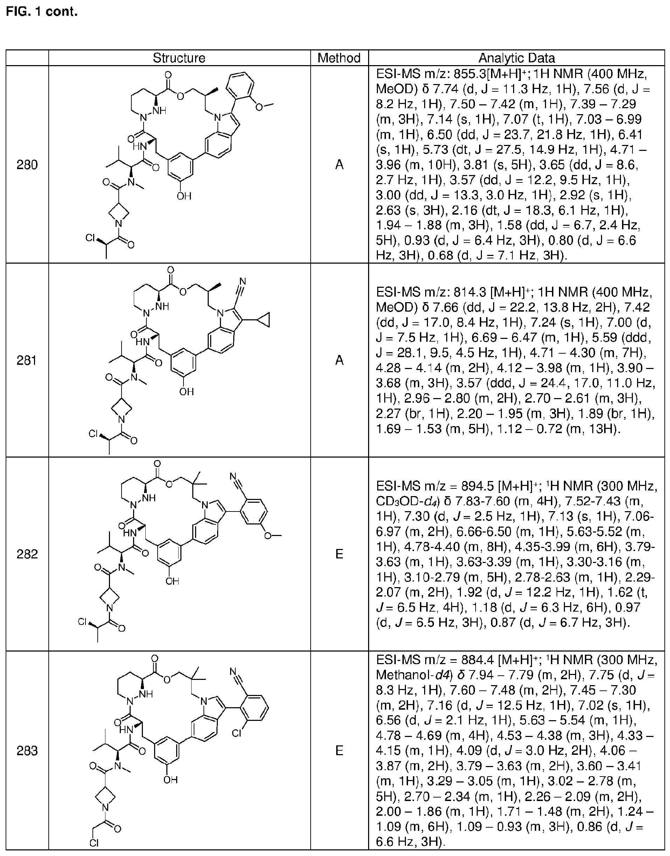

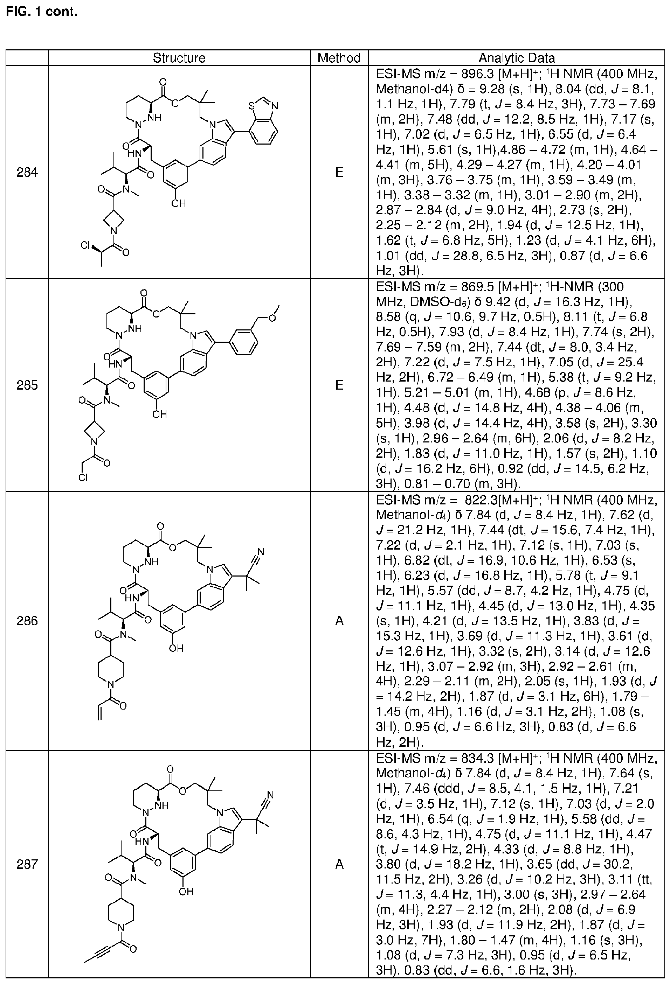

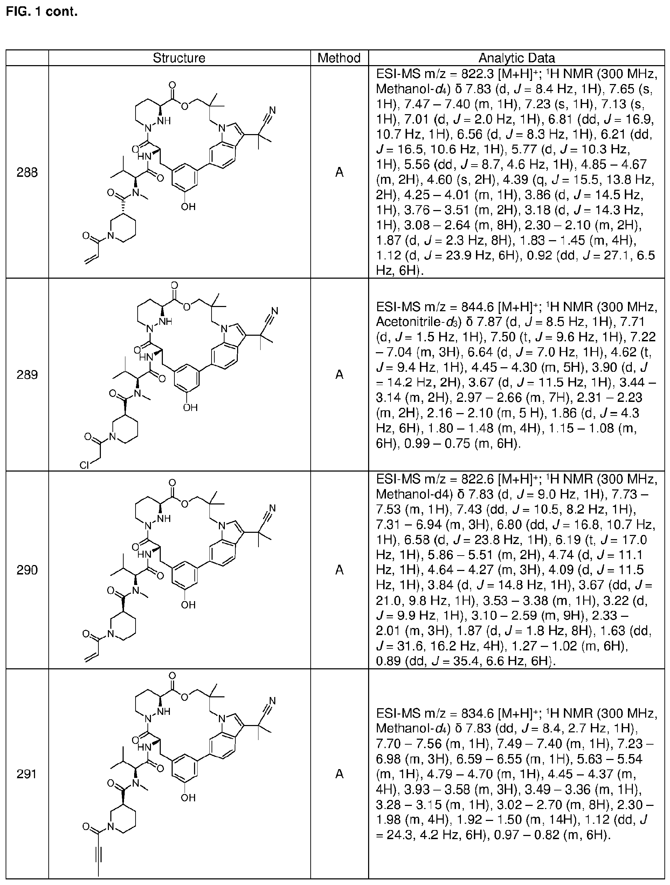

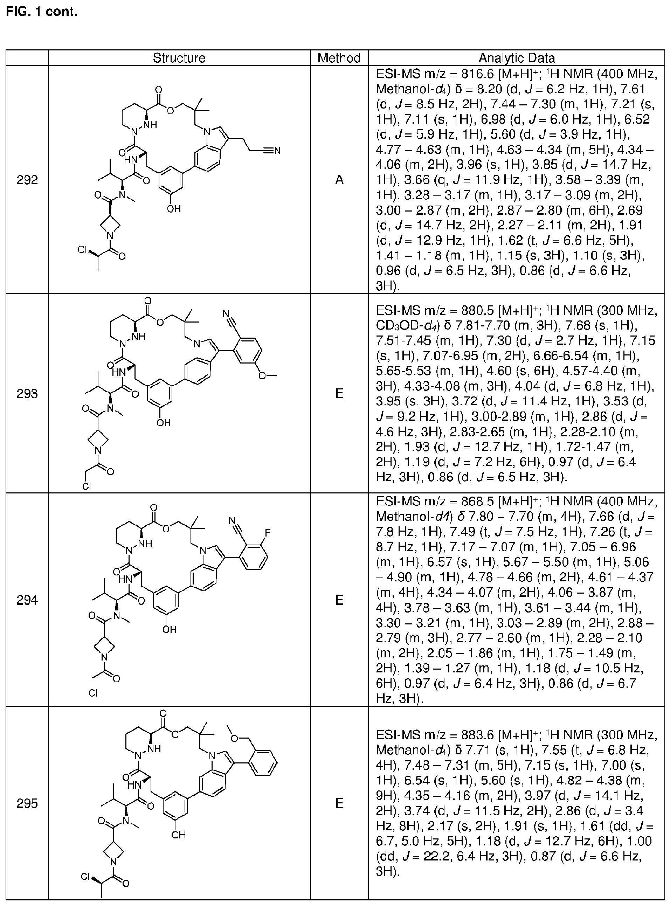

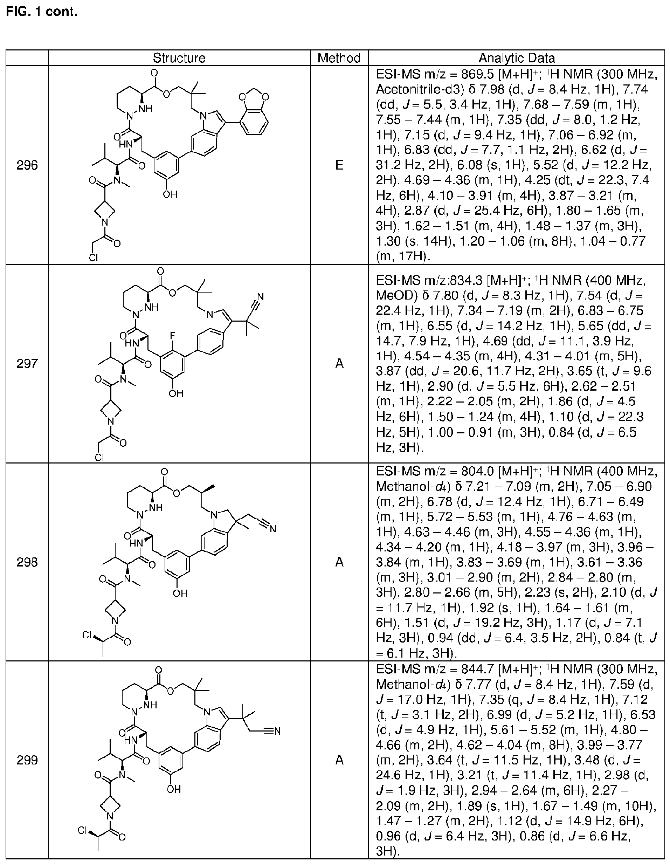

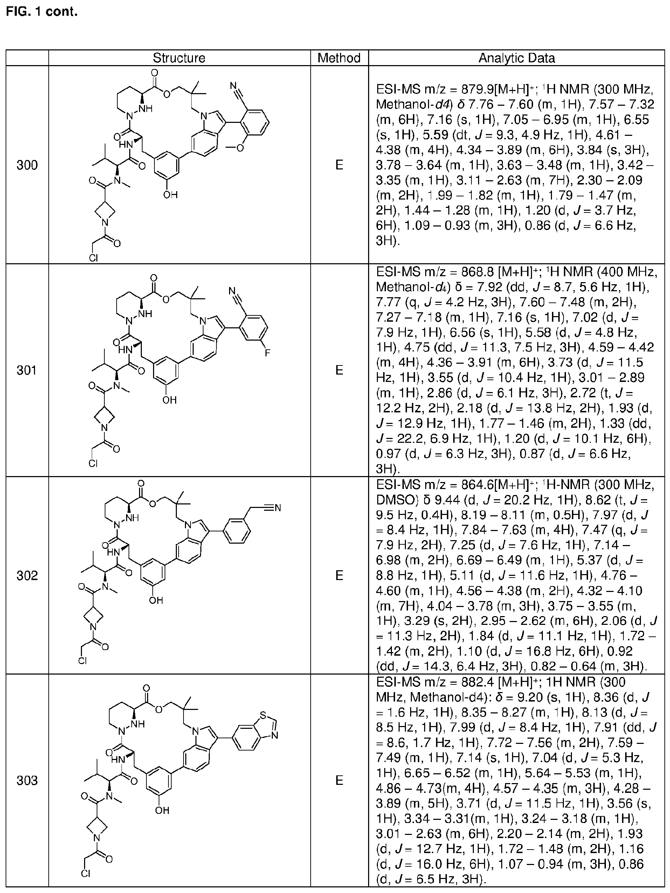

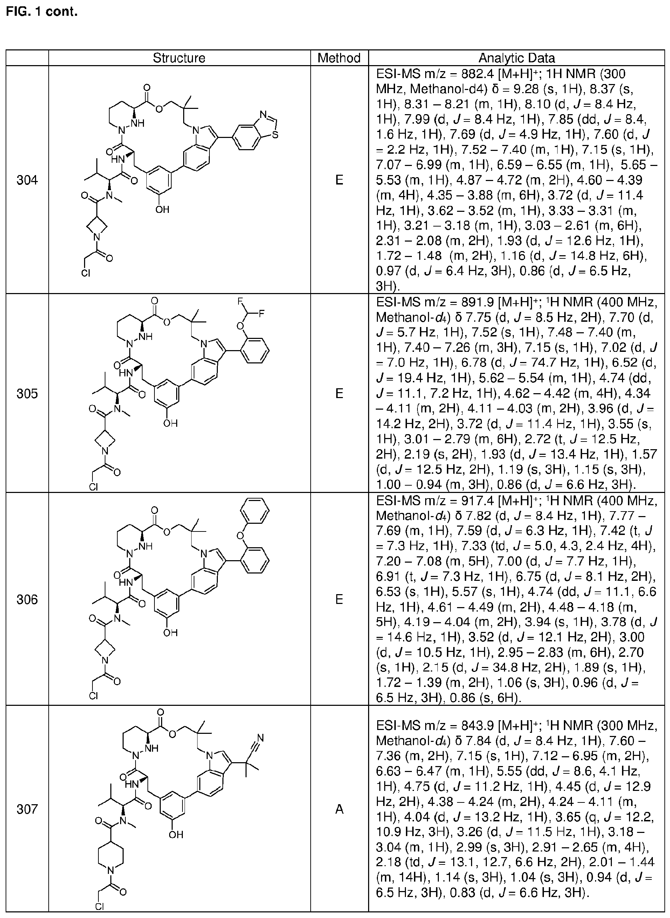

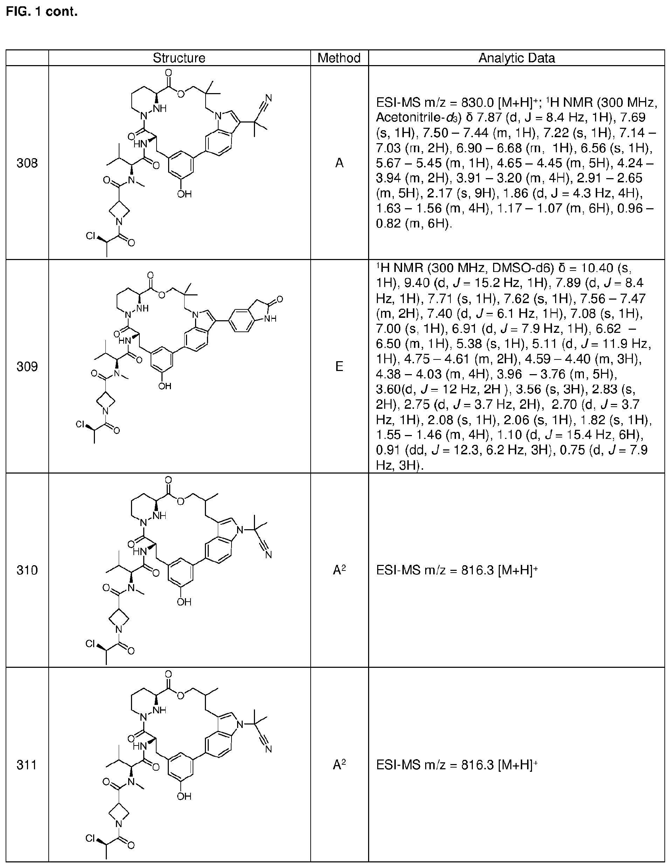

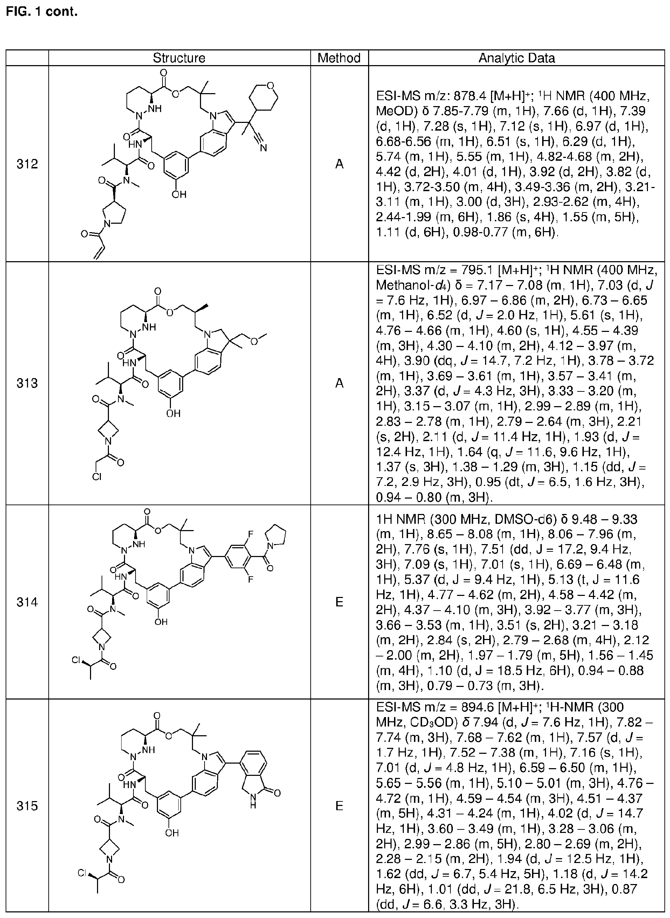

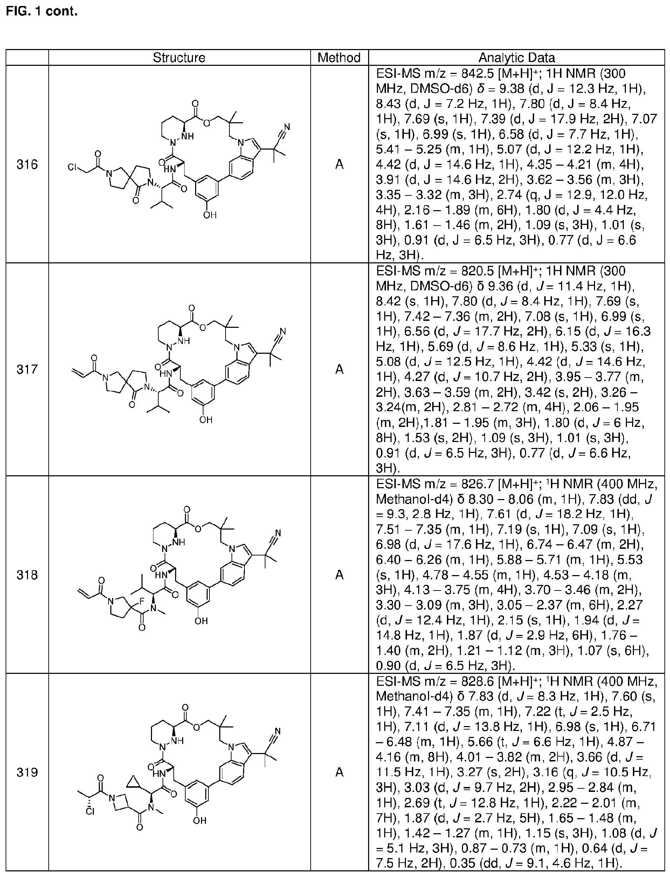

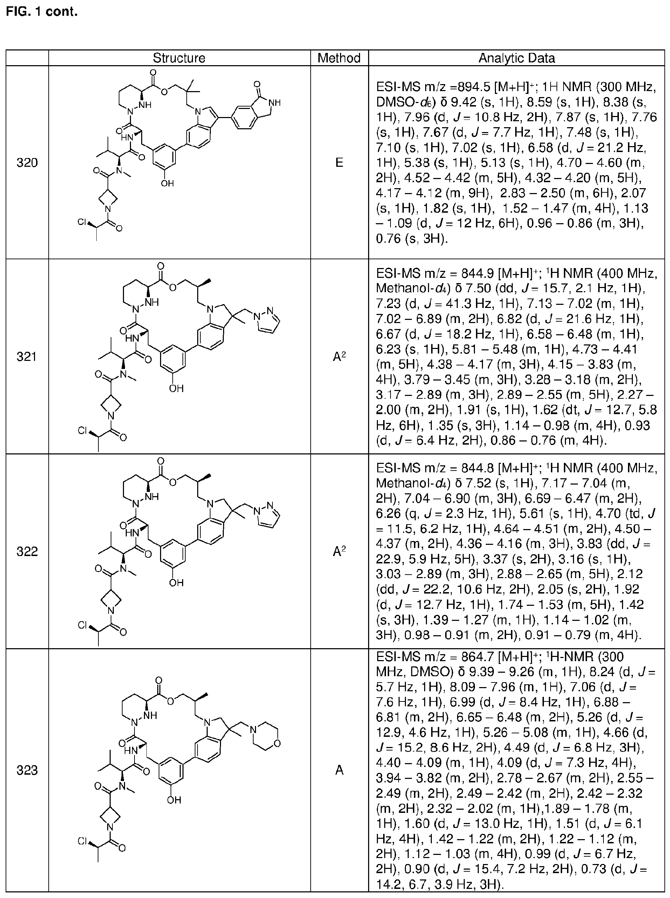

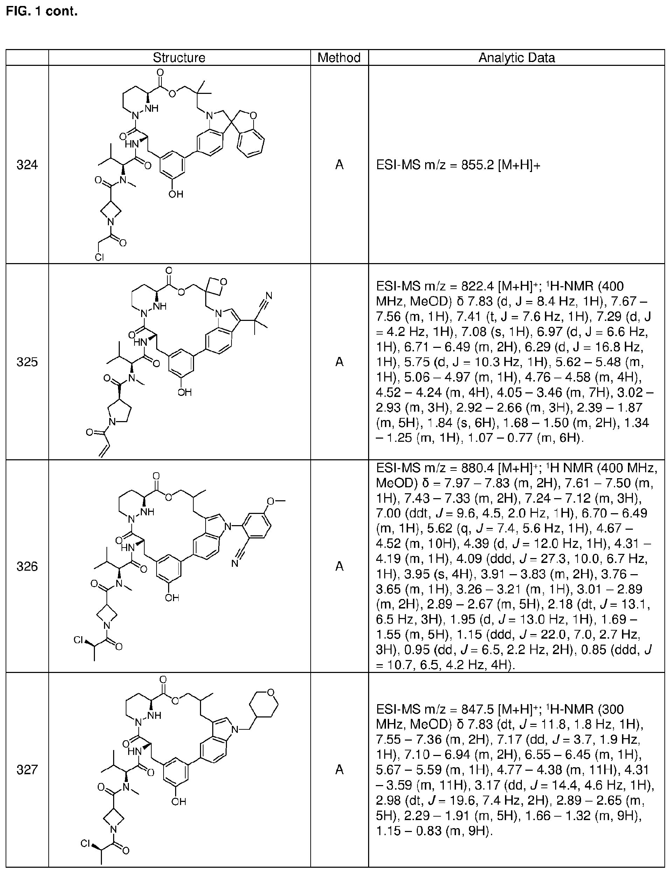

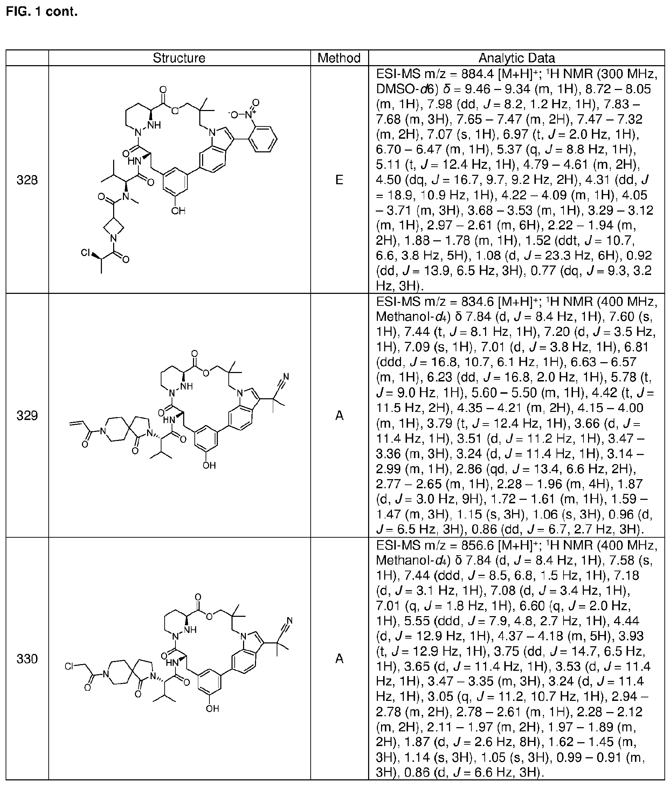

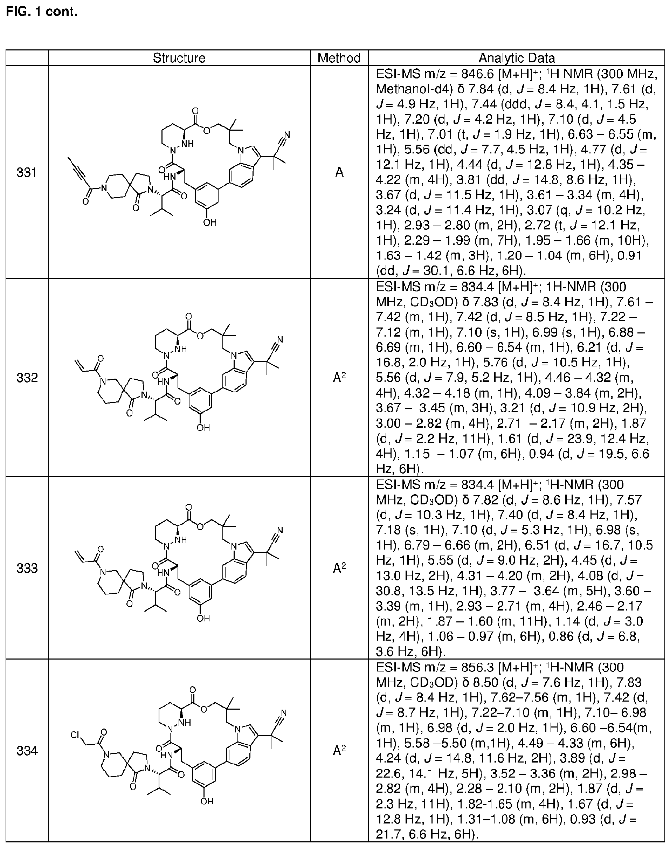

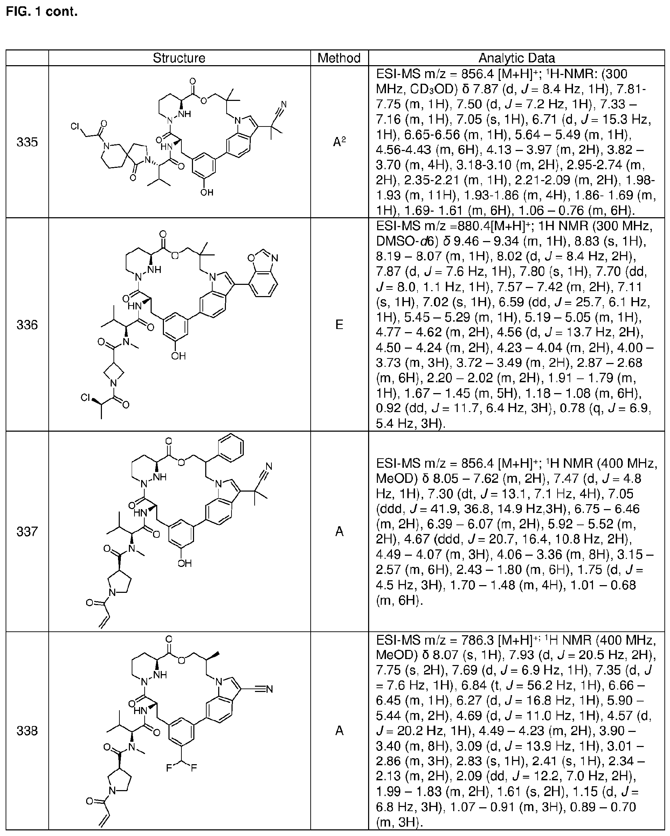

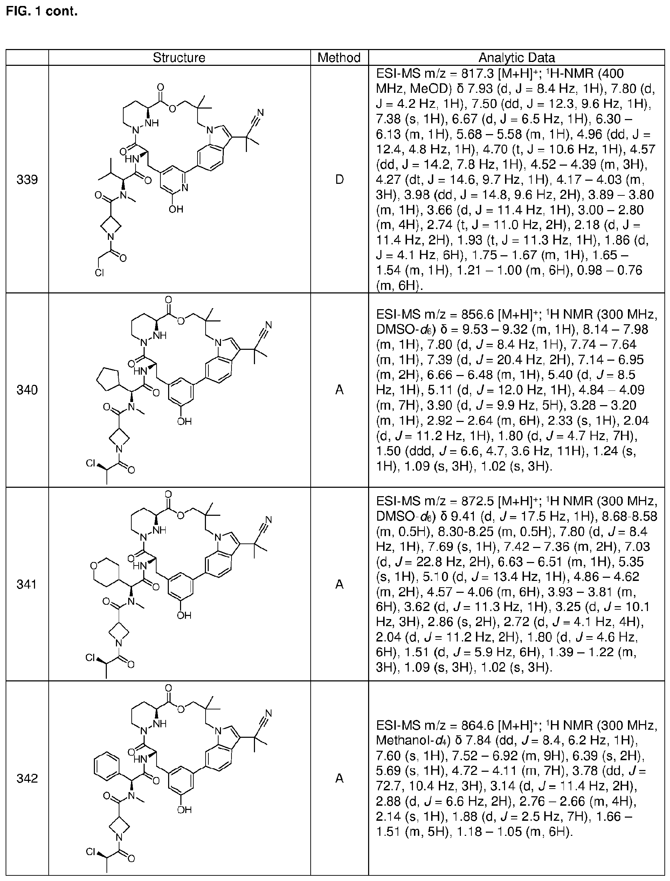

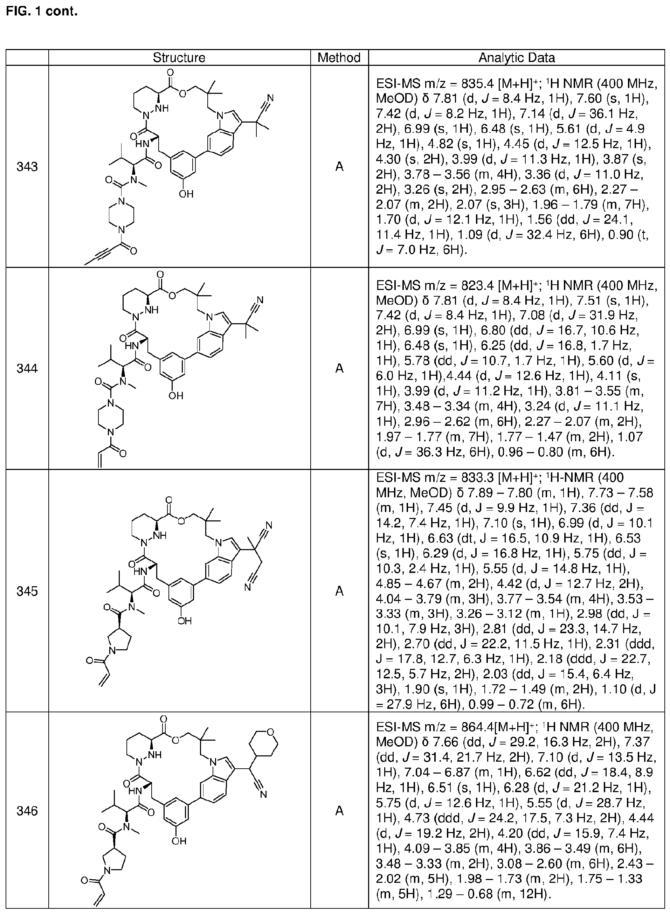

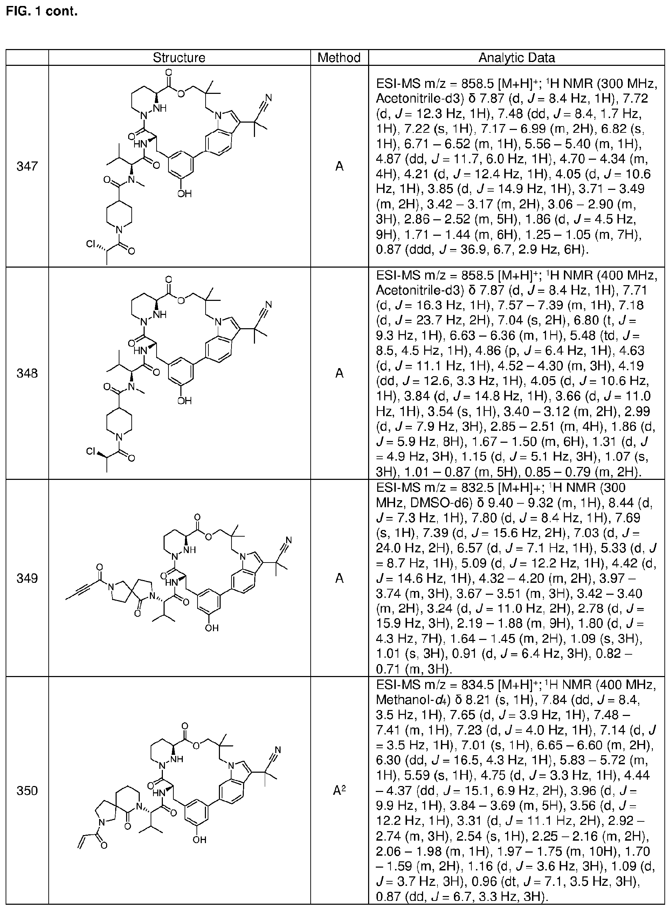

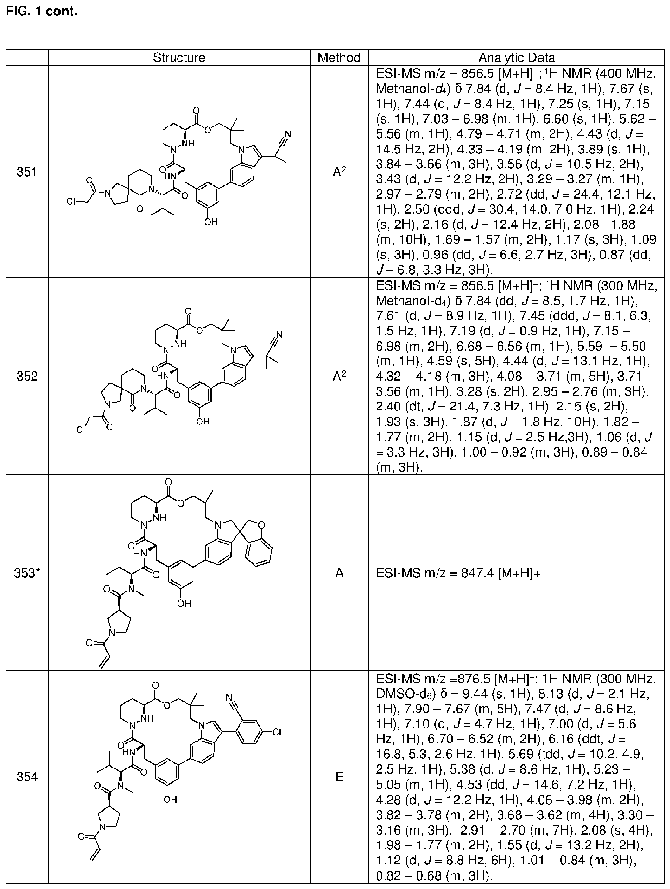

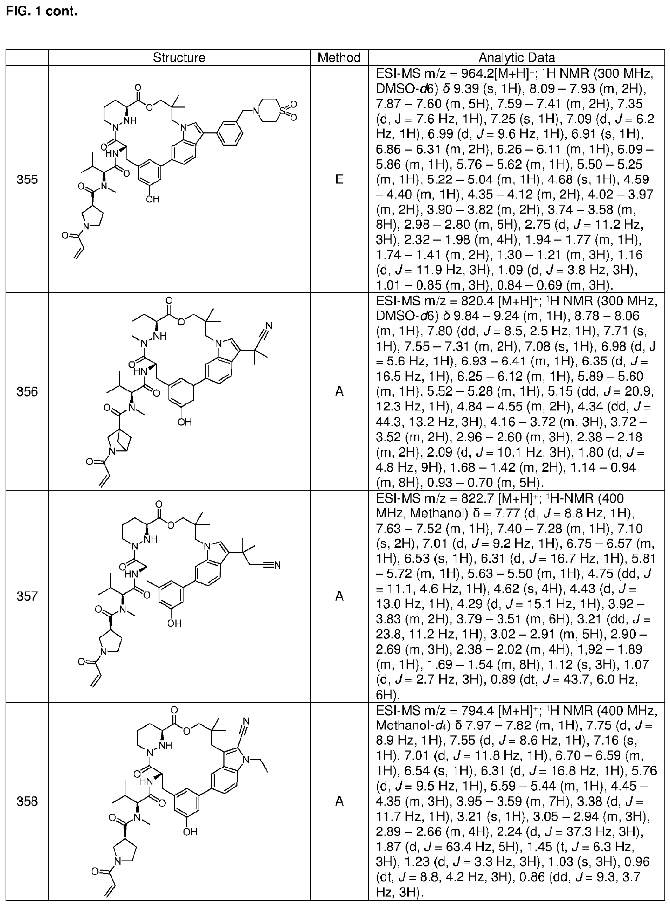

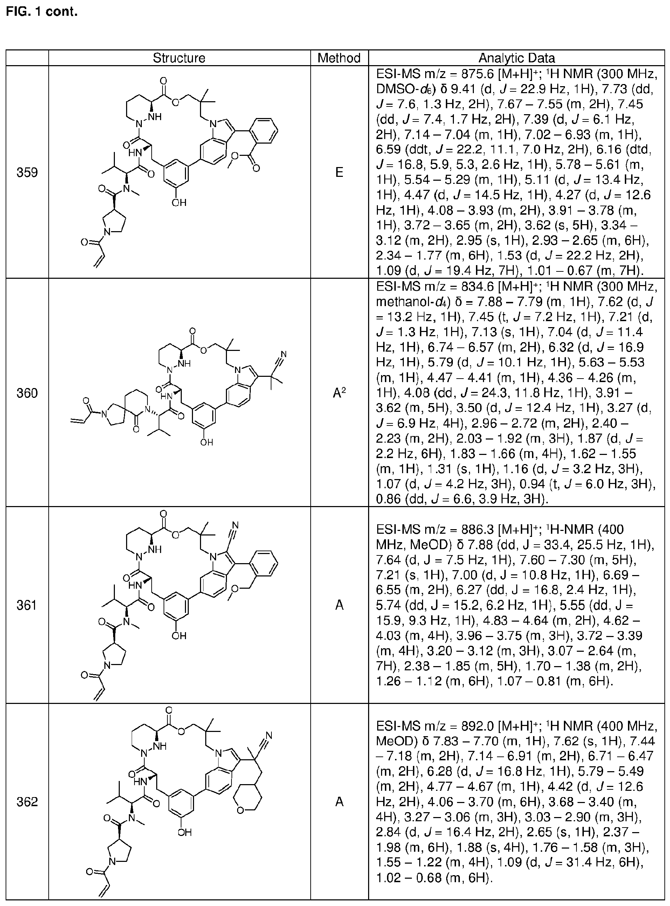

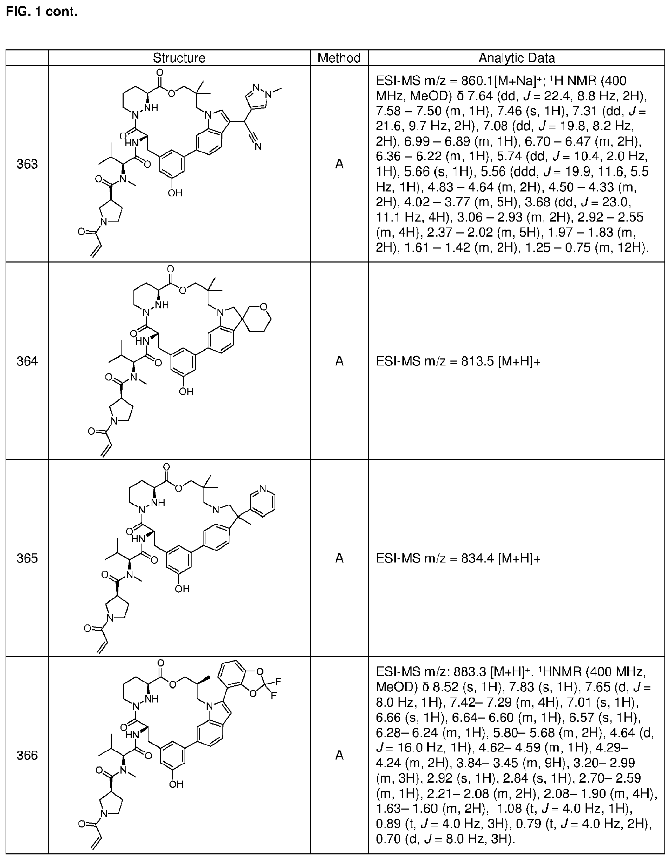

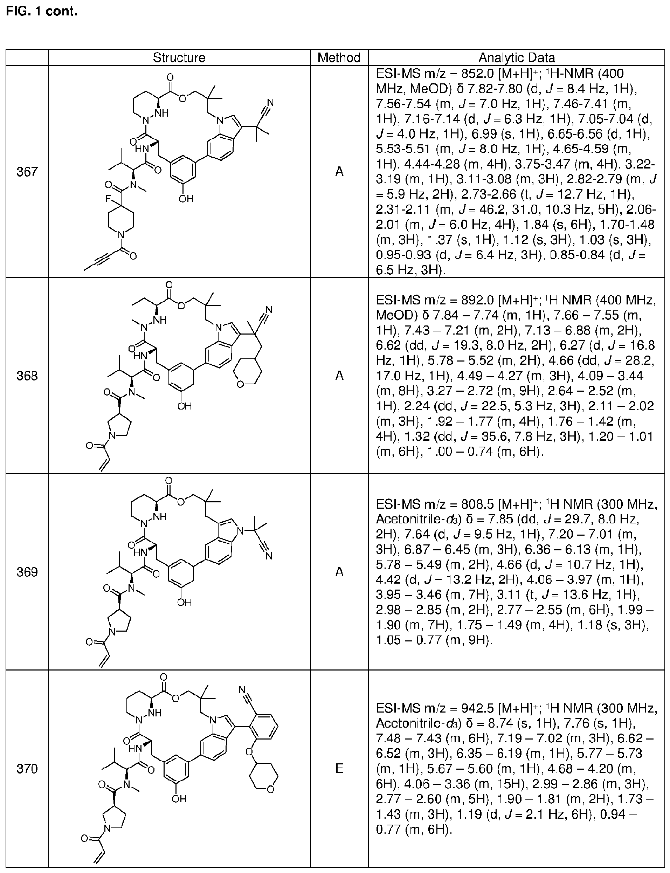

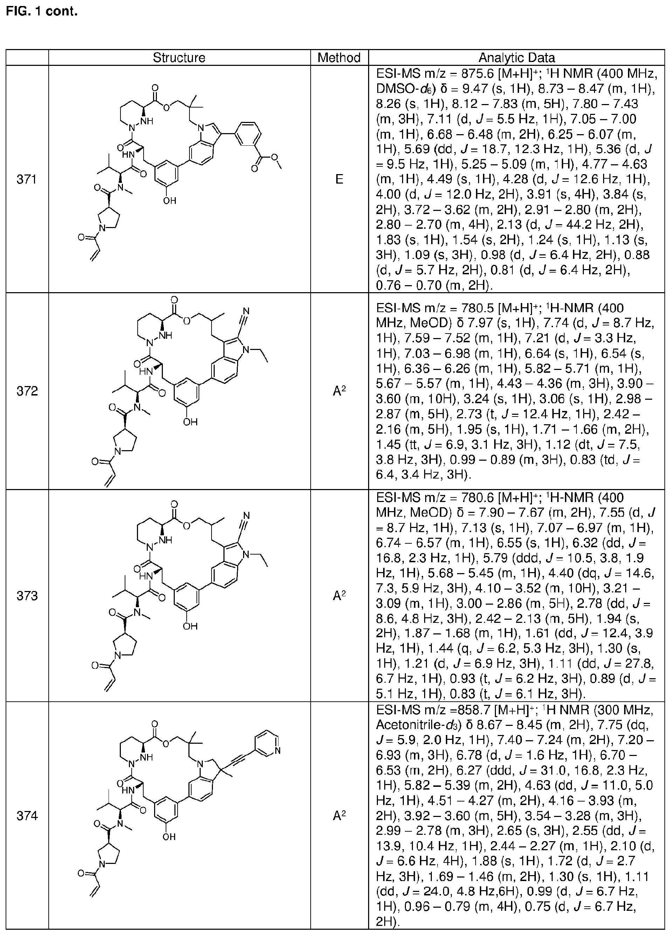

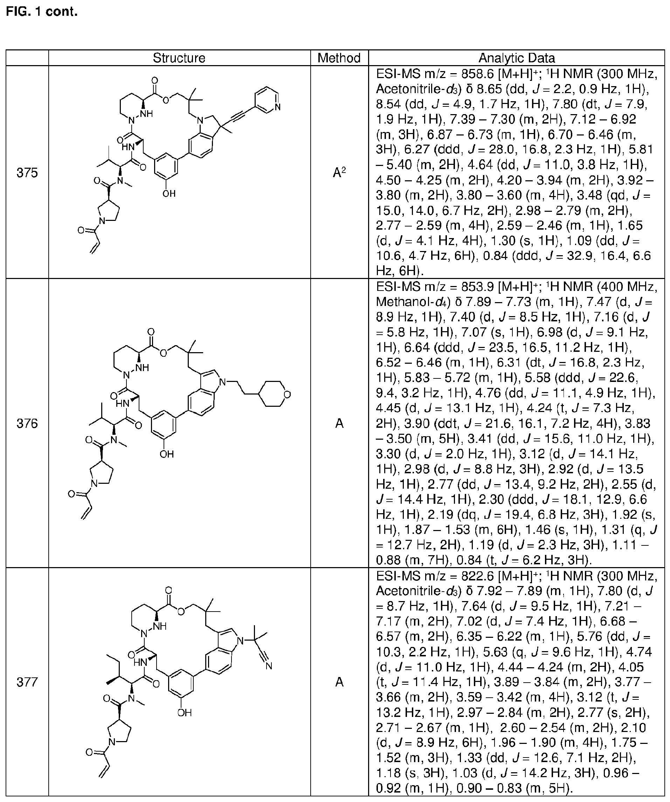

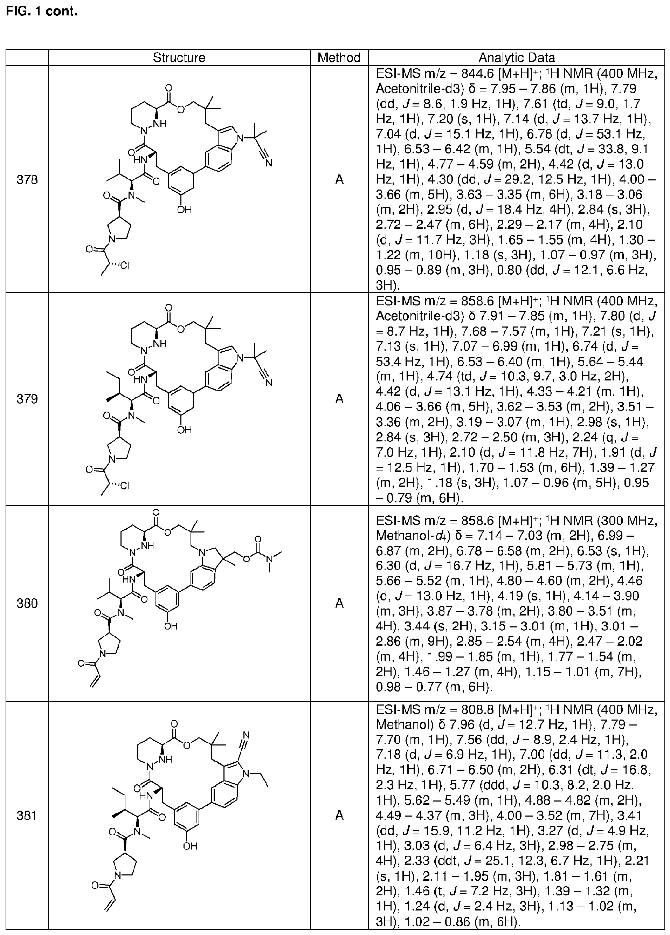

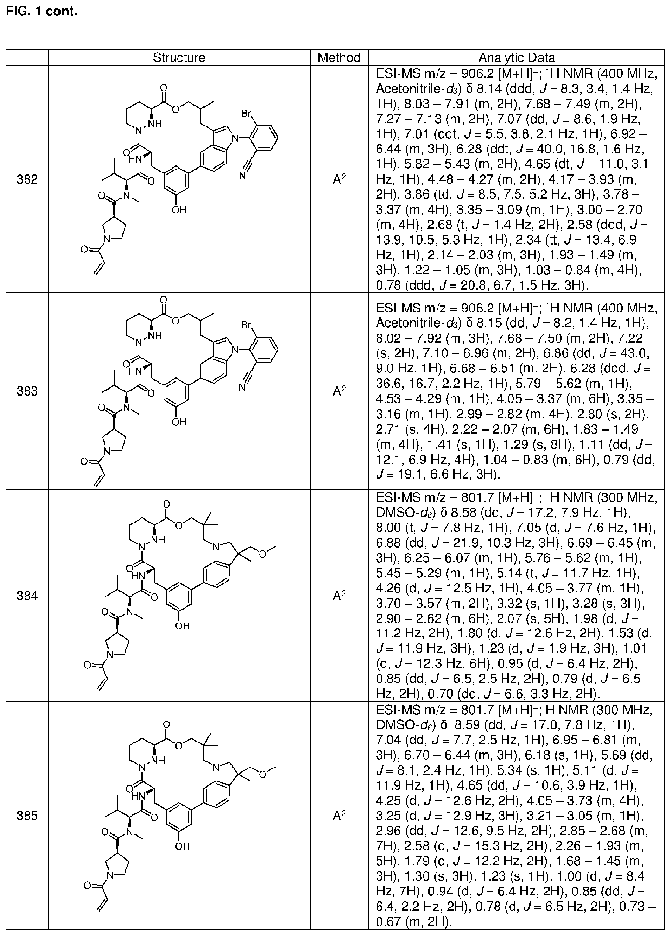

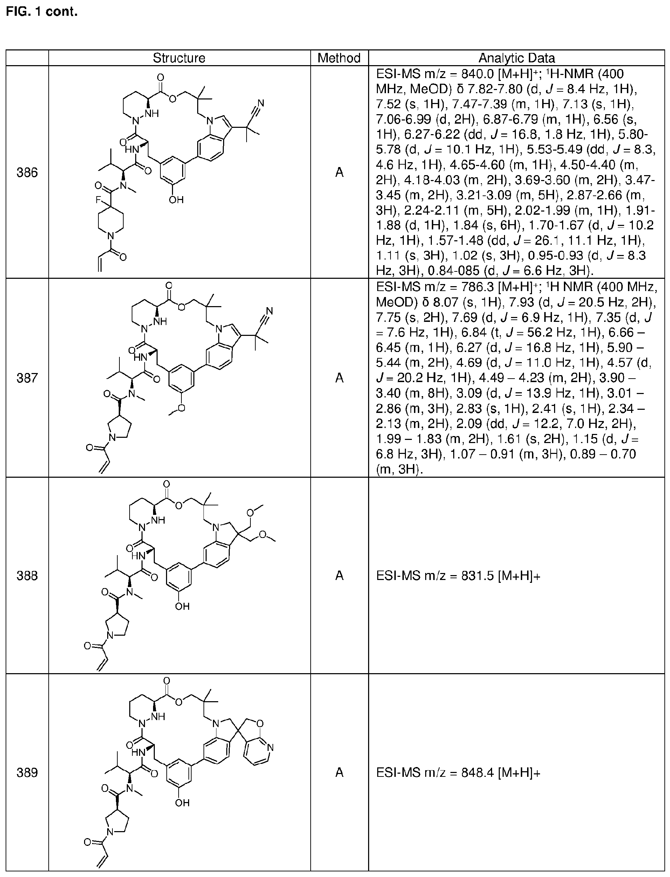

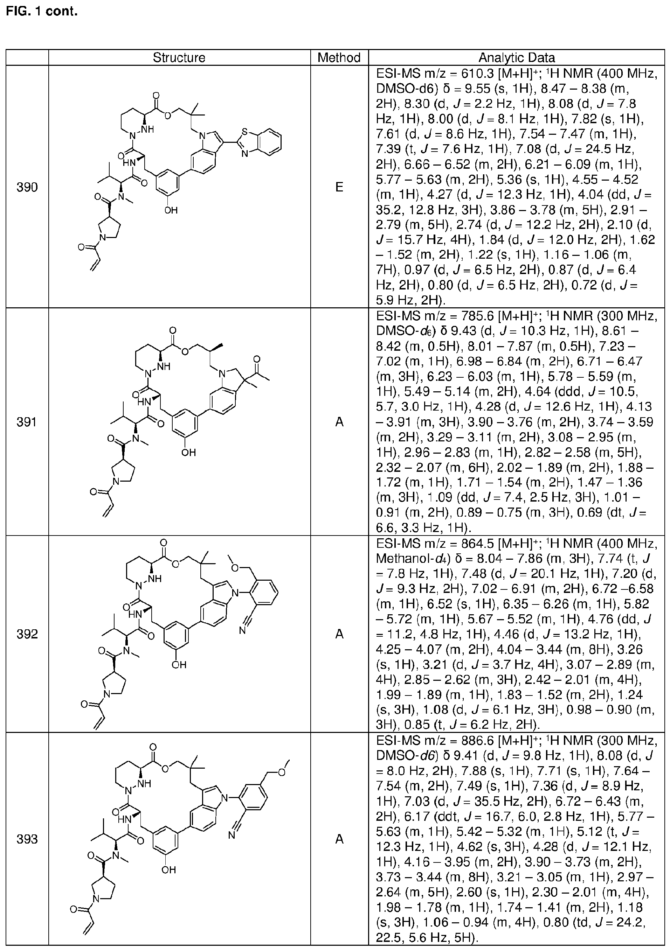

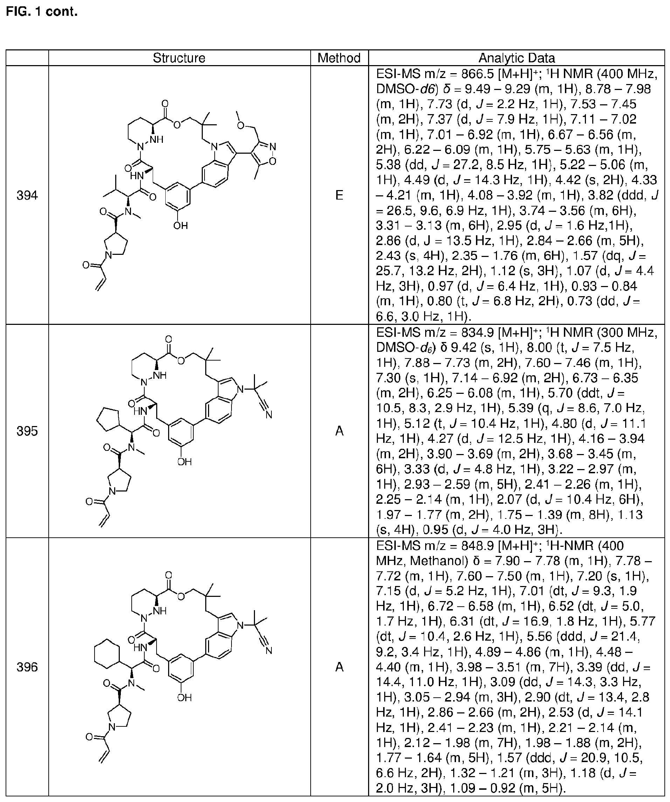

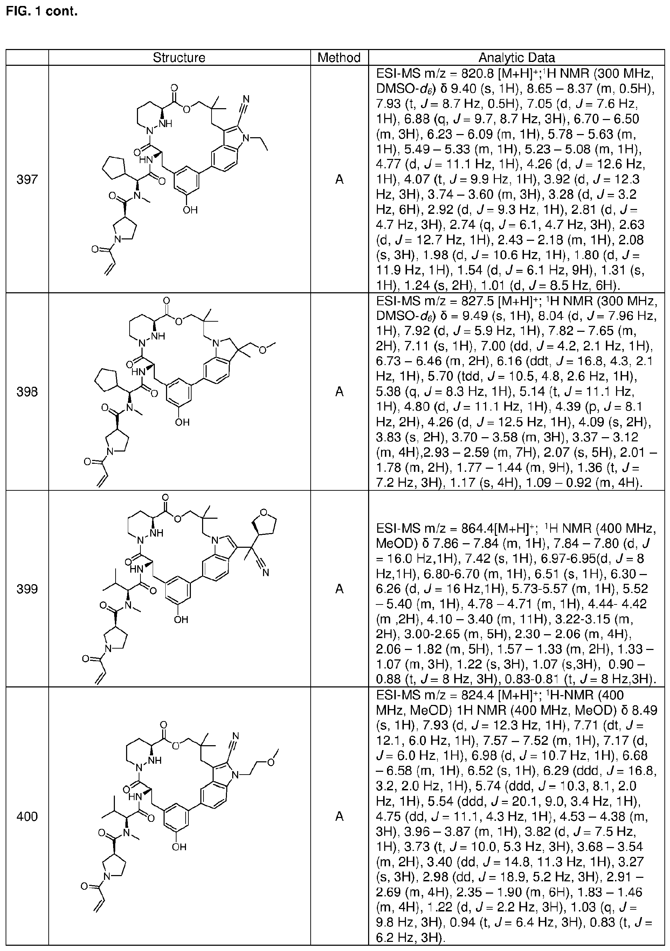

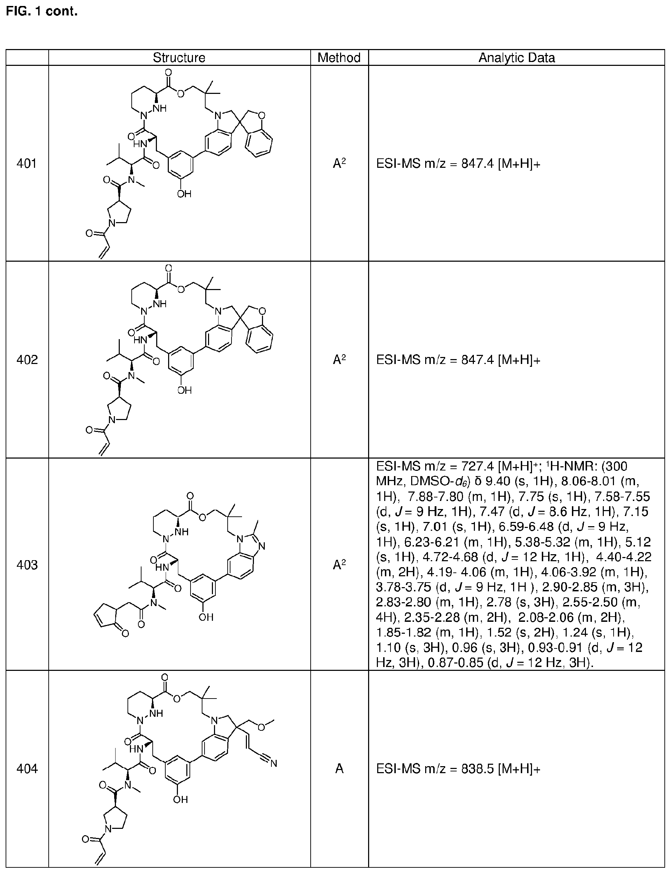

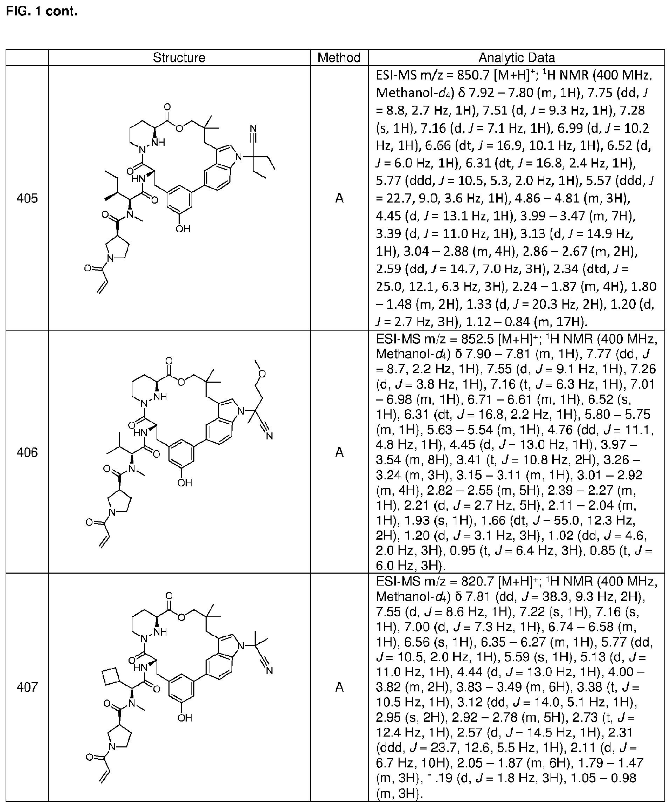

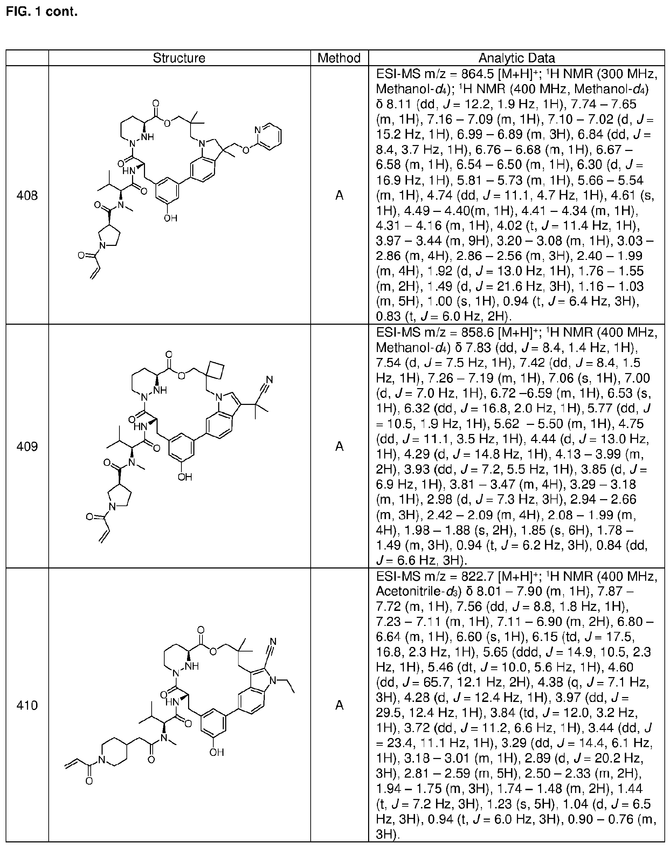

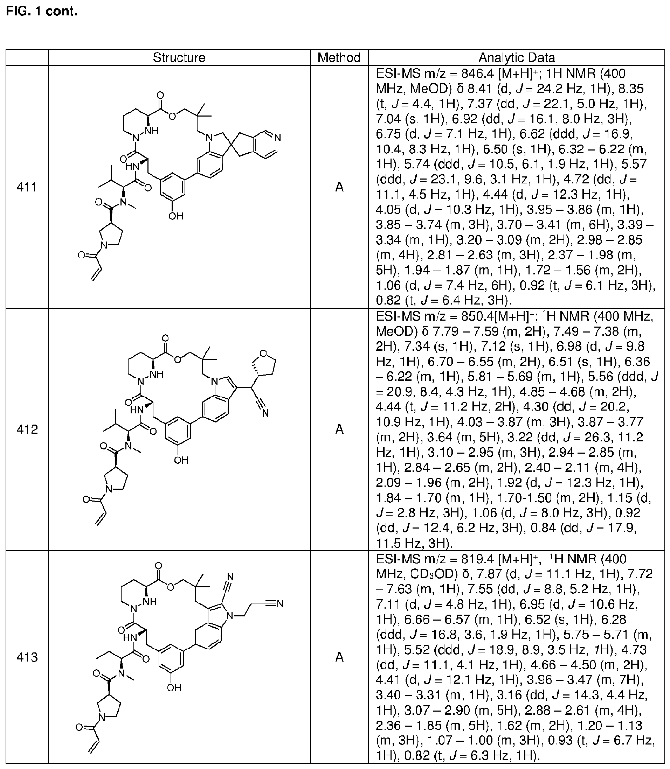

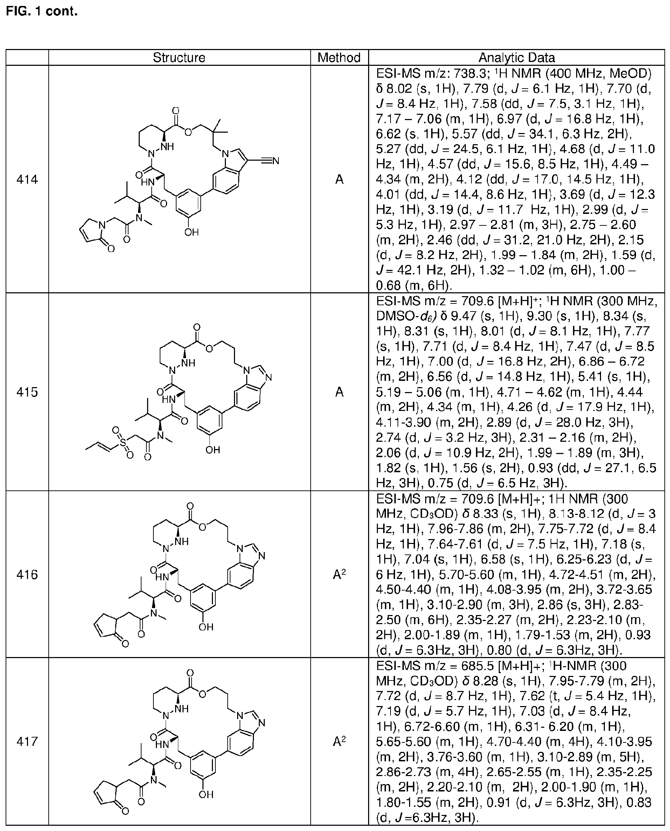

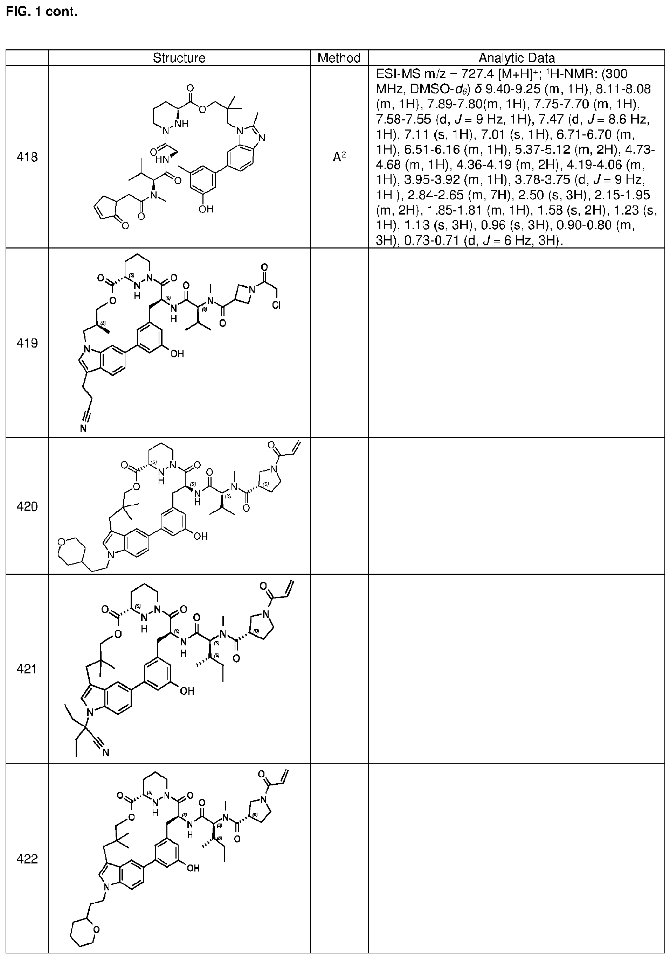

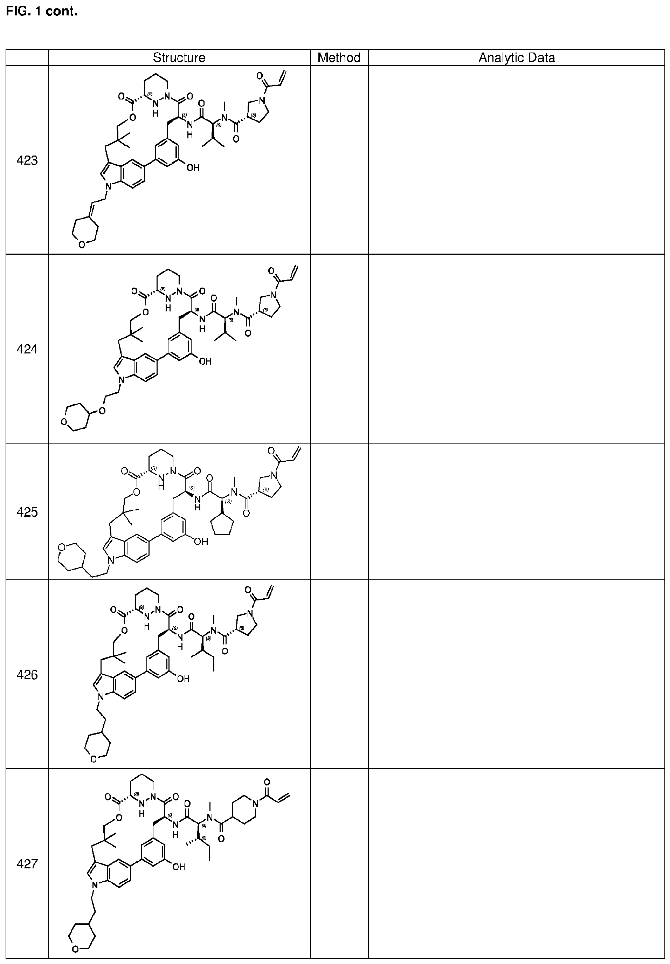

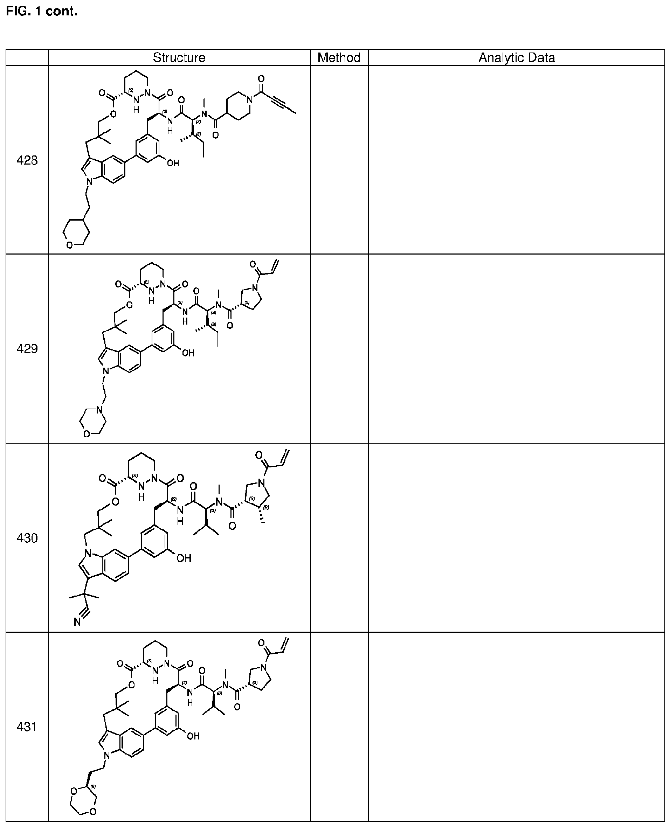

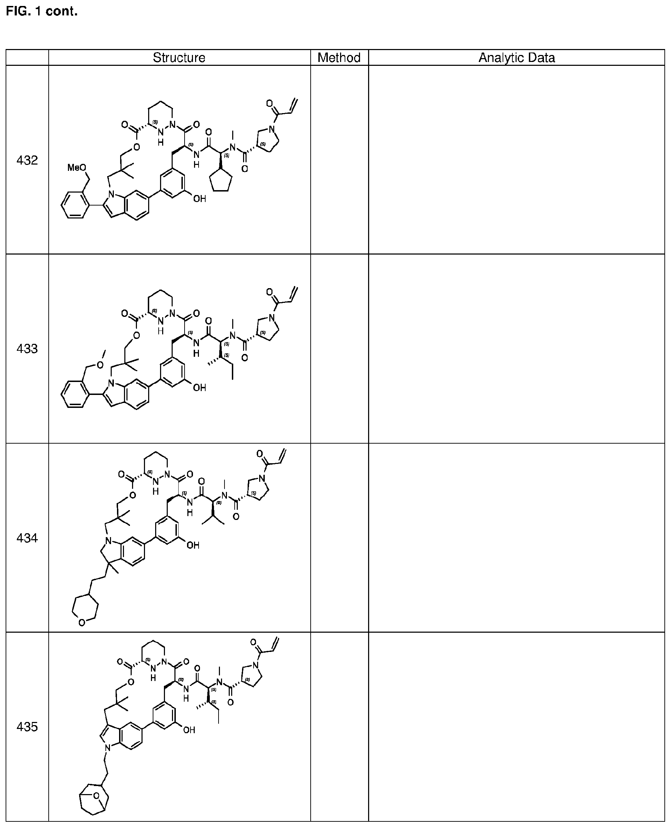

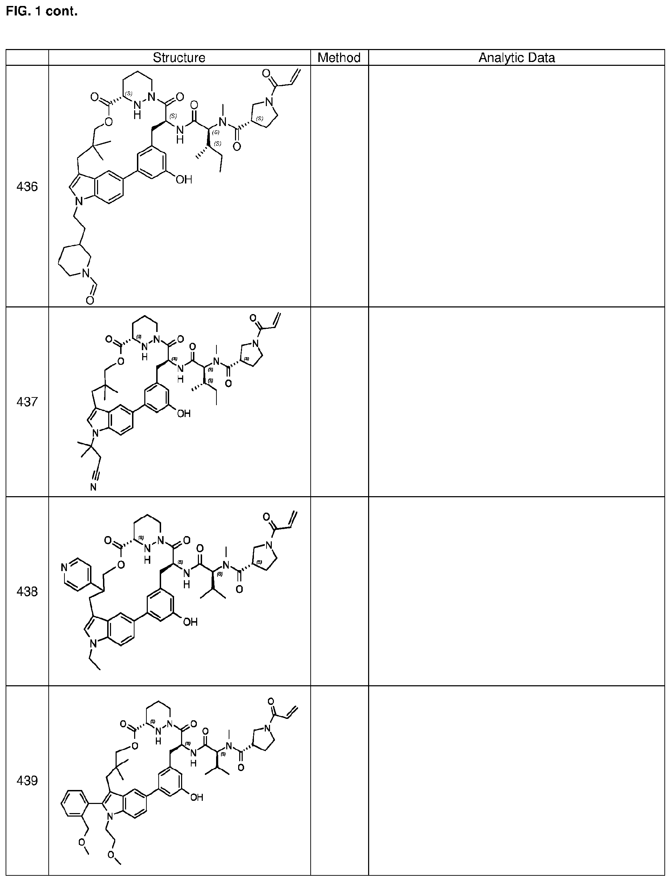

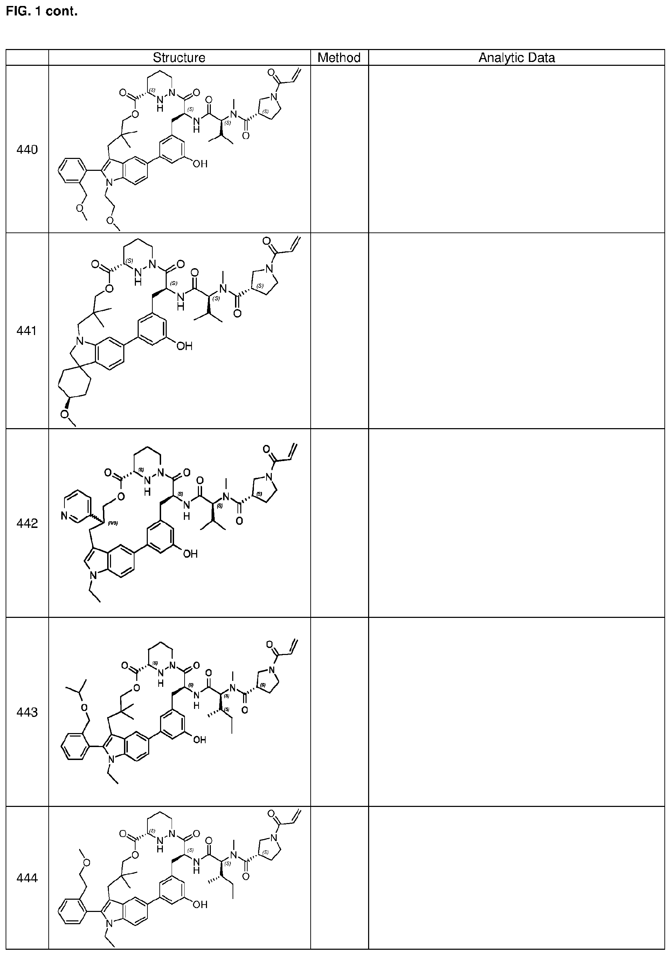

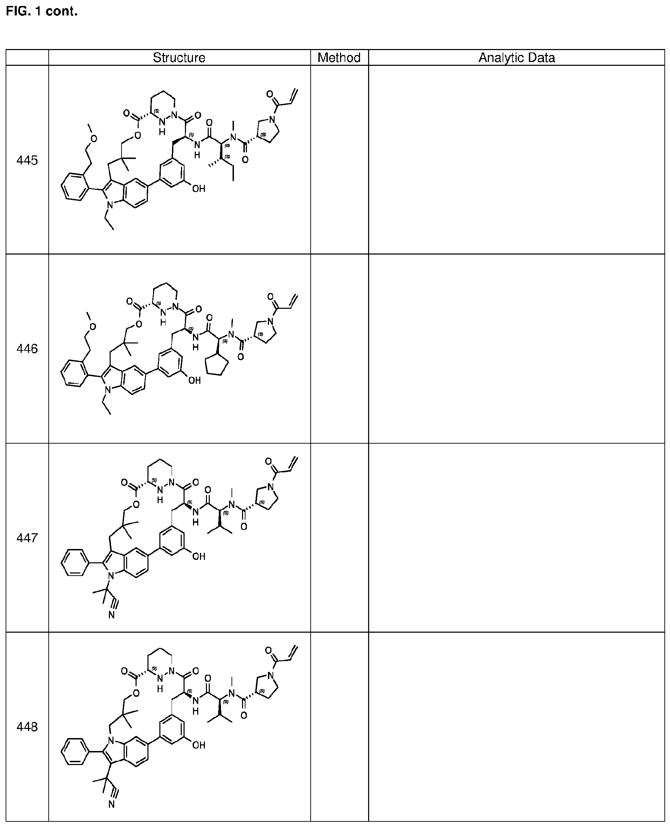

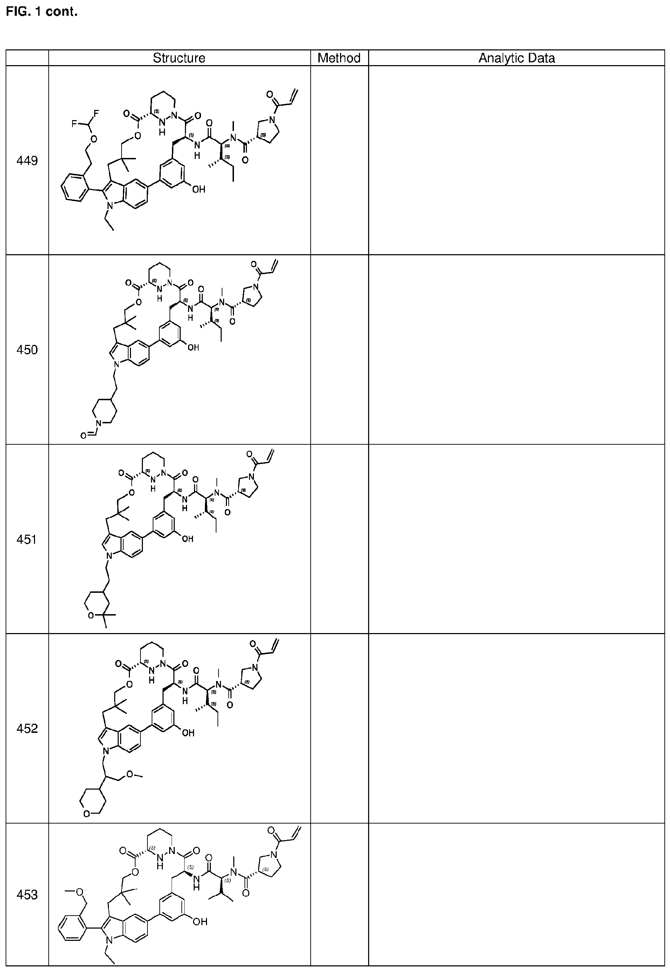

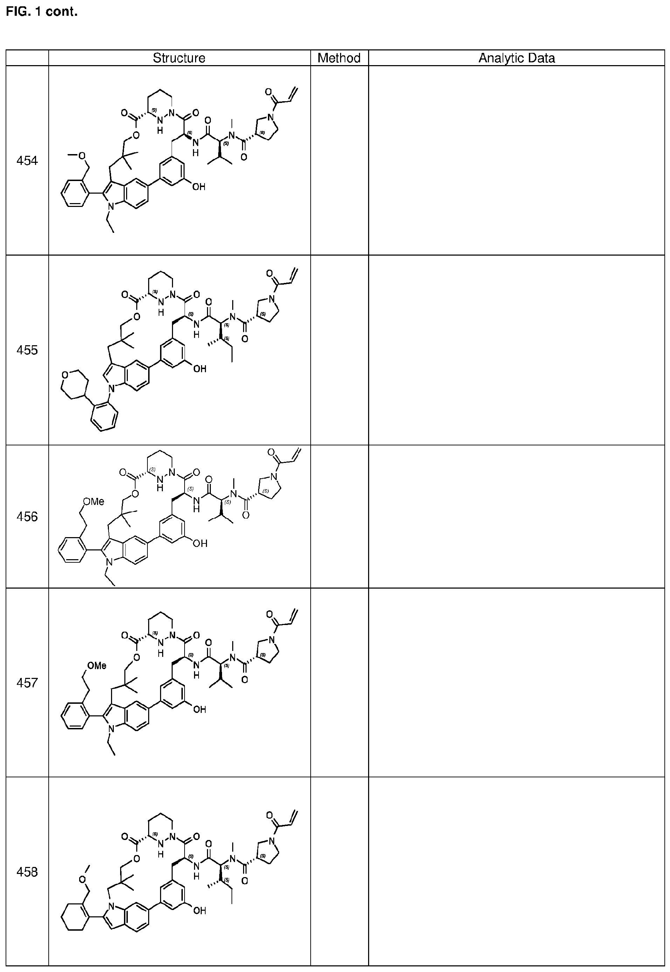

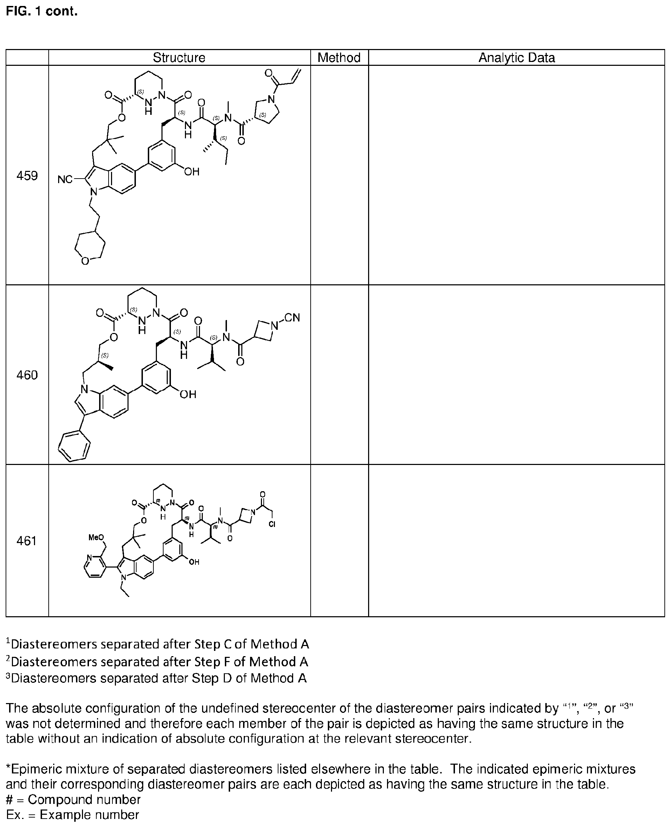

The term "a compound of the present invention" or "compounds of the present invention" or the like, is intended to encompass the salt (e.g., a pharmaceutically acceptable salt), hydrate, and solvate forms of such a compound as well as an enantiomer, stereoisomer, or tautomer thereof. In some embodiments, a "compound of the present invention" or the like, may refer to the compound and a pharmaceutically acceptable salt thereof. Non-limiting, exemplary compounds of the present invention are found in FIG. 1.

At various places in the present specification, substituents of compounds of the present disclosure are disclosed in groups or in ranges. It is specifically intended that the present disclosure include each and every individual subcombination of the members of such groups and ranges. For example, the term "C₁-C₆ alkyl" is specifically intended to individually disclose methyl, ethyl, C₃ alkyl, C₄ alkyl, C₅ alkyl, and C₆ alkyl. Furthermore, where a compound includes a plurality of positions at which substitutes are disclosed in groups or in ranges, unless otherwise indicated, the present disclosure is intended to cover individual compounds and groups of compounds (e.g., genera and subgenera) containing each and every individual subcombination of members at each position.

Herein a phrase of the form "optionally substituted X" (e.g., optionally substituted alkyl) is intended to be equivalent to "X, wherein X is optionally substituted" (e.g., "alkyl, wherein said alkyl is optionally substituted"). It is not intended to mean that the feature "X" (e.g., alkyl) *per* se is optional. As described herein, certain compounds of interest may contain one or more "optionally substituted" moieties. In general, the term "substituted", whether preceded by the term "optionally" or not, means that one or more hydrogens of the designated moiety are replaced with a suitable substituent, e.g., any of the substituents or groups described herein. Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by the present disclosure are preferably those that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group may be independently deuterium;
halogen; -(CH₂)₀-₄R°; -(CH₂)₀-₄OR°; -O(CH₂)₀-₄R°; -O-(CH₂)₀-₄C(O)OR°; -(CH₂)₀-₄CH(OR°)₂; -(CH₂)₀-₄SR°; -(CH₂)₀-₄Ph, which may be substituted with R°; -(CH₂)₀-₄O(CH₂)₀-₁Ph which may be substituted with R°; -CH=CHPh, which may be substituted with R°; -(CH₂)₀-₄O(CH₂)₀-₁-pyridyl which may be substituted with R°; 4-8 membered saturated or unsaturated heterocyclyl (e.g., pyridyl); 3-8 membered saturated or unsaturated cycloalkyl (e.g., cyclopropyl, cyclobutyl, or
cyclopentyl); -NO₂; -CN; -N₃; -(CH₂)₀-₄N(R°)₂; -(CH₂)₀-₄N(R°)C(O)R°; -N(R°)C(S)R°; -(CH₂)₀-₄N(R°)C(O)N R°₂; -N(R°)C(S)NR°₂; -(CH₂)₀-₄N(R°)C(O)OR°; - N(R°)N(R°)C(O)R°; -N(R°)N(R°)C(O)NR°₂; -N(R°)N(R°)C (O)OR°; -(CH₂)₀-₄C(O)R°; -C(S)R°; -(CH₂)₀-₄C(O)OR°; -(CH₂)₀-₄-C(O)-N(R°)₂;
-(CH₂)₀-₄-C(O)-N(R°)-S(O)₂-R°; -C(NCN)NR°₂; -(CH₂)₀-₄C(O)SR°; -(CH₂)₀-₄C(O)OSiR°₃;
-(CH₂)₀-₄OC(O)R°; -OC(O)(CH₂)₀-₄SR°; -SC(S)SR°; -(CH₂)₀-₄SC(O)R°; -(CH₂)₀-₄C(O)NR°₂; -C(S)NR°₂; -C( S)SR°; -(CH₂)₀-₄OC(O)NR°₂; -C(O)N(OR°)R°; -C(O)C(O)R°; -C(O)CH₂C(O)R°; -C(NOR°)R°; -(CH₂)₀-₄SS R°; -(CH₂)₀-₄S(O)₂R°; -(CH₂)₀-₄S(O)₂OR°; -(CH₂)₀-₄OS(O)₂R°; -S(O)₂NR°₂; -(CH₂)₀-₄S(O)R°; -N(R°)S(O)₂N R°₂; -N(R°)S(O)₂R°; -N(OR°)R°; -C(NOR°)NR°₂; -C(NH)NR°₂; -P(O)₂R°; -P(O)R°₂; -P(O)(OR°)₂; -OP(O)R° ₂; -OP(O)(OR°)₂; -OP(O)(OR°)R°, -SiR°₃; -(C₁-₄ straight or branched alkylene)O-N(R°)₂; or -(C₁-₄ straight or branched alkylene)C(O)O-N(R°)₂, wherein each R° may be substituted as defined below and is independently hydrogen, -C₁-₆ aliphatic, -CH₂Ph, -O(CH₂)₀-₁Ph, -CH₂-(5-6 membered heteroaryl ring), or a 3-6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), may be, independently, halogen, -(CH₂)₀-₂R^{•}, -(haloR^{•}), -(CH₂)₀-₂OH, -(CH₂)₀-₂OR^{•}, -(CH₂)₀-₂CH(OR^{•})₂; -O(haloR^{•}), -CN, -N₃, -(C H₂)₀-₂C(O)R^{•}, -(CH₂)₀-₂C(O)OH, -(CH₂)₀-₂C(O)OR^{•}, -(CH₂)₀-₂SR^{•}, -(CH₂)₀-₂SH, -(CH₂)₀-₂NH₂, -(CH₂)₀-₂NH R^{•}, -(CH₂)₀-₂NR^{•}₂, -NO₂, -SiR^{•}₃, -OSiR^{•}₃, -C(O)SR^{•}, -(C₁-₄ straight or branched alkylene)C(O)OR^{•}, or -SSR^{•} wherein each R^{•} is unsubstituted or where preceded by "halo" is substituted only with one or more halogens, and is independently selected from C₁-₄ aliphatic, -CH₂Ph, -O(CH₂)₀-₁ Ph, or a 5-6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, =NNR*₂, =NNHC(O)R', =NNHC(O)OR*, =NNHS(O)₂R*, =NR*, =NOR*, -O(C(R*₂))₂-₃O-, or -S(C(R*₂))₂-₃S-, wherein each independent occurrence of R* is selected from hydrogen, C₁-₆ aliphatic which may be substituted as defined below, or an unsubstituted 5-6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR*₂)₂-₃O-, wherein each independent occurrence of R* is selected from hydrogen, C₁-₆ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R* include halogen, -R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁-₄ aliphatic, -CH₂Ph, -O(CH₂)₀-₁Ph, or a 5-6-membered saturated, partially unsaturated, or aryl ring having 0-4 heteroatoms independently selected from nitrogen, oxygen, or sulfur.

Suitable substituents on a substitutable nitrogen of an "optionally substituted" group include -R^{†}, -NR^{†}₂, -C(O)R^{†}, -C(O)OR^{†}, -C(O)C(O)R^{†}, -C(O)CH₂C(O)R^{†}, -S(O)₂R^{†}, -S(O)₂NR^{†}₂, -C(S)NR^{†}₂, -C(NH)NR^{†}₂, or -N(R^{†})S(O)₂R^{†}; wherein each R^{†} is independently hydrogen, C₁-₆ aliphatic which may be substituted as defined below, unsubstituted -OPh, or an unsubstituted 3-6-membered saturated, partially unsaturated, or aryl ring having 0-4 heteroatoms independently selected from nitrogen, oxygen, or sulfur, or, notwithstanding the definition above, two independent occurrences of R^{†}, taken together with their intervening atom(s) form an unsubstituted 3-12-membered saturated, partially unsaturated, or aryl mono- or bicyclic ring having 0-4 heteroatoms independently selected from nitrogen, oxygen, or sulfur.

Suitable substituents on an aliphatic group of R^{†} are independently halogen, -R^{•}, -(haloR^{•}), -OH, -OR^{•}, -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁-₄ aliphatic, -CH₂Ph, -O(CH₂)₀-₁Ph, or a 5-6-membered saturated, partially unsaturated, or aryl ring having 0-4 heteroatoms independently selected from nitrogen, oxygen, or sulfur. Suitable divalent substituents on a saturated carbon atom of R^{†} include =O and =S.

The term "alkyl," as used herein, refers to saturated hydrocarbon groups containing from 1 to 20 (e.g., from 1 to 10 or from 1 to 6) carbons. In some embodiments, an alkyl group is unbranched (i.e., is linear); in some embodiments, an alkyl group is branched. Alkyl groups are exemplified by, but not limited to, methyl, ethyl, *n-* and *iso*-propyl, *n-, sec-, iso-* and *tert*-butyl, and neopentyl.

The term "alkylene" as used herein, represent a saturated divalent hydrocarbon group derived from a straight or branched chain saturated hydrocarbon by the removal of two hydrogen atoms, and is exemplified by methylene, ethylene, isopropylene, and the like. The term "Cₓ-C_{y} alkylene" represents alkylene groups having between x and y carbons. Exemplary values for x are 1, 2, 3, 4, 5, and 6, and exemplary values for y are 2, 3, 4, 5, 6, 7, 8, 9, 10, 12, 14, 16, 18, or 20 (e.g., C₁-C₆, C₁-C₁₀, C₂-C₂₀, C₂-C₆, C₂-C₁₀, or C₂-C₂₀ alkylene). In some embodiments, the alkylene can be further substituted with 1, 2, 3, or 4 substituent groups as defined herein for an alkyl group.

The term "alkenyl," as used herein, represents monovalent straight or branched chain groups of, unless otherwise specified, from 2 to 20 carbons (e.g., from 2 to 6 or from 2 to 10 carbons) containing one or more carbon-carbon double bonds and is exemplified by ethenyl, 1-propenyl, 2-propenyl, 2-methyl-1-propenyl, 1-butenyl, 2-butenyl, and the like. Alkenyls include both cis and trans isomers. The term "alkenylene," as used hereing represents a divalent straight or branched chain groups of, unless otherwise specified, from 2 to 20 carbons (e.g., from 2 to 6 or from 2 to 10 carbons) containing one or more carbon-carbon double bonds.

The term "alkynyl," as used herein, represents monovalent straight or branched chain groups from 2 to 20 carbon atoms (e.g., from 2 to 4, from 2 to 6, or from 2 to 10 carbons) containing a carbon-carbon triple bond and is exemplified by ethynyl, 1-propynyl, and the like.

The term "amino," as used herein, represents -N(R^{†})₂.

The term "amino acid," as described herein, refers to a molecule having a side chain, an amino group, and an acid group (e.g., a carboxy group of -CO₂H or a sulfo group of -SO₃H), wherein the amino acid is attached to the parent molecular group by the side chain, amino group, or acid group (e.g., the side chain). As used herein, the term "amino acid" in its broadest sense, refers to any compound and/or substance that can be incorporated into a polypeptide chain, e.g., through formation of one or more peptide bonds. In some embodiments, an amino acid has the general structure H₂N-C(H)(R)-COOH. In some embodiments, an amino acid is a naturally-occurring amino acid. In some embodiments, an amino acid is a synthetic amino acid; in some embodiments, an amino acid is a D-amino acid; in some embodiments, an amino acid is an L-amino acid. "Standard amino acid" refers to any of the twenty standard L-amino acids commonly found in naturally occurring peptides. "Nonstandard amino acid" refers to any amino acid, other than the standard amino acids, regardless of whether it is prepared synthetically or obtained from a natural source. In some embodiments, an amino acid, including a carboxy- and/or amino-terminal amino acid in a polypeptide, can contain a structural modification as compared with the general structure above. For example, in some embodiments, an amino acid may be modified by methylation, amidation, acetylation, and/or substitution as compared with the general structure. In some embodiments, such modification may, for example, alter the circulating half life of a polypeptide containing the modified amino acid as compared with one containing an otherwise identical unmodified amino acid. In some embodiments, such modification does not significantly alter a relevant activity of a polypeptide containing the modified amino acid, as compared with one containing an otherwise identical unmodified amino acid. As will be clear from context, in some embodiments, the term "amino acid" is used to refer to a free amino acid; in some embodiments it is used to refer to an amino acid residue of a polypeptide. In some embodiments, the amino acid is attached to the parent molecular group by a carbonyl group, where the side chain or amino group is attached to the carbonyl group. In some embodiments, the amino acid is an α-amino acid. In certain embodiments, the amino acid is a β-amino acid. In some embodiments, the amino acid is a γ-amino acid. Exemplary side chains include an optionally substituted alkyl, aryl, heterocyclyl, alkaryl, alkheterocyclyl, aminoalkyl, carbamoylalkyl, and carboxyalkyl. Exemplary amino acids include alanine, arginine, asparagine, aspartic acid, cysteine, glutamic acid, glutamine, glycine, histidine, optionally substituted hydroxylnorvaline, isoleucine, leucine, lysine, methionine, norvaline, ornithine, phenylalanine, proline, pyrrolysine, selenocysteine, serine, taurine, threonine, tryptophan, tyrosine, and valine.

The term "aryl," as used herein, represents a monovalent mono-, bicyclic, or multicyclic ring system formed by carbon atoms, wherein each ring is aromatic. Examples of aryl groups are phenyl, naphthyl, phenanthrenyl, and anthracenyl. An aryl ring can be attached to its pendant group at any heteroatom or carbon ring atom that results in a stable structure and any of the ring atoms can be optionally substituted unless otherwise specified.

The term "C₀" as used herein, represents a bond. For example, part of the term -N(C(O)-(C₀-C₅ alkylene-H)- includes -N(C(O)-(C₀ alkylene-H)-, which is also represented by -N(C(O)-H)-.

The terms "carbocyclic" and "carbocyclyl," as used herein, refer to a monovalent, optionally substituted C₃-C₁₂ monocyclic, bicyclic, or tricyclic ring structure in which all of the rings are formed by carbon atoms and at least one ring is non-aromatic. Carbocyclic structures include cycloalkyl, cycloalkenyl, and cycloalkynyl groups. Examples of carbocyclyl groups are cyclohexyl, cyclohexenyl, cyclooctynyl, 1,2-dihydronaphthyl (e.g., 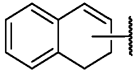 ), 1,2,3,4-tetrahydronaphthyl (e.g., 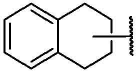 ), fluorenyl (e.g, 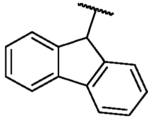 ), indenyl (e.g., 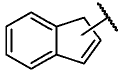 ), indanyl (e.g., 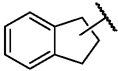 ), decalinyl, and the like. A carbocyclic ring can be attached to its pendant group at any heteroatom or carbon ring atom that results in a stable structure and any of the ring atoms can be optionally substituted unless otherwise specified.

The term "carbonyl," as used herein, represents a C(O) group, which can also be represented as C=O.

The term "carboxy," as used herein, means -CO₂H or the unprotonated counterpart.

The term "cyano," as used herein, represents a -CN group.

The term "cycloalkyl," as used herein, represents a monovalent saturated cyclic hydrocarbon group from three to eight carbons, unless otherwise specified, and is exemplified by cyclopropyl, cyclobutyl, cyclopentyl, cyclohexyl, cycloheptyl, bicycle heptyl, and the like.

The term "diyl," when used in the name of a chemical compound represents a divalent radical.

The term "diastereomer," as used herein, means stereoisomers that are not mirror images of one another and are non-superimposable on one another.

The term "enantiomer," as used herein, means each individual optically active form of a compound of the invention, having an optical purity or enantiomeric excess (as determined by methods standard in the art) of at least 80% (i.e., at least 90% of one enantiomer and at most 10% of the other enantiomer), preferably at least 90% and more preferably at least 98%.

The term "halo," as used herein, represents a halogen selected from bromine, chlorine, iodine, or fluorine.

The term "heteroaryl," as used herein, represents a monovalent, monocyclic or polycyclic ring structure that contains at least onefully aromatic ring : i.e., they contain 4*n*+2 pi electrons within the monocyclic or polycyclic ring system and contains at least one ring heteroatom selected from N, O, or S in that aromatic ring. Exemplary unsubstituted heteroaryl groups are of 1 to 12 (e.g., 1 to 11, 1 to 10, 1 to 9, 2 to 12, 2 to 11, 2 to 10, or 2 to 9) carbons. The term "heteroaryl" includes bicyclic, tricyclic, and tetracyclic groups in which any of the above heteroaromatic rings is fused to one or more, aryl or carbocyclic rings, e.g., a phenyll ring, or a cyclohexane ring. Examples of heteroaryl groups include, but are not limited to, pyridyl, pyrazolyl, benzooxazolyl, benzoimidazolyl, benzothiazolyl, imidazolyl, thiazolyl, quinolinyl (e.g., 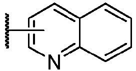 and 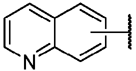 ), tetrahydroquinolinyl (e.g., 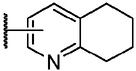 ), 4-azaindolyl (e.g., 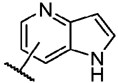 and 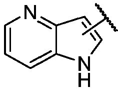 ), and the like. A heteroaryl ring can be attached to its pendant group at any heteroatom or carbon ring atom that results in a stable structure and any of the ring atoms can be optionally substituted unless otherwise specified. In some embodiment, the heteroaryl is substituted with 1, 2, 3, or 4 substituents groups.

The term "heteroarylene," as used herein, represents a divalent heteroaromatic ring system monocyclic or polycyclic ring structure that contains at least one fully aromatic ring and contains at least one ring heteroatom selected from N, O, or S in that aromatic ring. The term "heteroarylene" includes bivalent bicyclic, tricyclic, and tetracyclic groups in which any of the above heteroaromatic ring is fused to one or more, aryl or carbocyclic rings. A heteroarylene ring can be attached to its pendant groups at any heteroatom or carbon ring atom that results in a stable structure and any of the ring atoms can be optionally substituted unless otherwise specified. In some embodiment, the heteroarylene is substituted with 1, 2, 3, or 4 substituents groups.

The term "heterocyclyl," as used herein, represents a monovalent monocyclic, bicyclic or polycyclic ring system wherein at least one ring is non-aromatic and wherein the non-aromatic ring contains one, two, three, or four heteroatoms independently selected from the group consisting of nitrogen, oxygen, and sulfur. The 5-membered ring has zero to two double bonds, and the 6- and 7-membered rings have zero to three double bonds. Exemplary unsubstituted heterocyclyl groups are of 1 to 12 (e.g., 1 to 11, 1 to 10, 1 to 9, 2 to 12, 2 to 11, 2 to 10, or 2 to 9) carbons. The term "heterocyclyl" also represents a heterocyclic compound having a bridged multicyclic structure in which one or more carbons and/or heteroatoms bridges two non-adjacent members of a monocyclic ring, e.g., a quinuclidinyl group. The term "heterocyclyl" includes bicyclic, tricyclic, and tetracyclic groups in which any of the above heterocyclic rings is fused to one or more aromatic, carbocyclic, heteroaromatic, or heterocyclic rings, e.g., an aryl ring, a cyclohexane ring, a cyclohexene ring, a cyclopentane ring, a cyclopentene ring, a pyridine ring, or a pyrrolidine ring. Examples of heterocyclyl groups are pyrrolidinyl, piperidinyl, 1,2,3,4-tetrahydroquinolinyl (e.g., 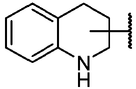 ), decahydroquinolinyl (e.g., 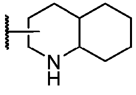 ), dihydropyrrolopyridine (e.g., 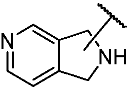 ), decahydronapthyridinyl (e.g., 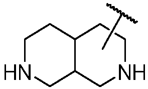 ), and the like. A heterocyclic ring can be attached to its pendant group at any heteroatom or carbon ring atom that results in a stable structure and any of the ring atoms can be optionally substituted unless otherwise specified.

The term "heterocyclylene,"as used herein represents a divalent monocyclic, bicyclic or polycyclic ring system wherein at least one ring is non-aromatic and wherein the non-aromatic ring contains one, two, three, or four heteroatoms independently selected from the group consisting of nitrogen, oxygen, and sulfur. The term "heterocyclylene" includes bicyclic, tricyclic, and tetracyclic groups in which any of the above heterocyclic rings is fused to one or more aromatic, carbocyclic, heteroaromatic, or heterocyclic rings. A heterocyclylene ring can be attached to its pendant groups at any heteroatom or carbon ring atom that results in a stable structure and any of the ring atoms can be optionally substituted unless otherwise specified.

The term "haloyalkyl," as used herein, represents an alkyl moiety substituted on one or more carbon atoms with one or more of the same of different halo moieities.

The term "hydroxyalkyl," as used herein, represents an alkyl moiety substituted on one or more carbon atoms with one or more -OH moieties.

The term "isomer," as used herein, means any tautomer, stereoisomer, enantiomer, or diastereomer of any compound of the invention. It is recognized that the compounds of the invention can have one or more chiral centers and/or double bonds and, therefore, exist as stereoisomers, such as double-bond isomers (i.e., geometric E/Z isomers) or diastereomers (e.g., enantiomers (i.e., (+) or (-)) or cis/trans isomers). According to the invention, the chemical structures depicted herein, and therefore the compounds of the invention, encompass all of the corresponding stereoisomers, that is, both the stereomerically pure form (e.g., geometrically pure, enantiomerically pure, or diastereomerically pure) and enantiomeric and stereoisomeric mixtures, e.g., racemates. Enantiomeric and stereoisomeric mixtures of compounds of the invention can typically be resolved into their component enantiomers or stereoisomers by well-known methods, such as chiral-phase gas chromatography, chiral-phase high performance liquid chromatography, crystallizing the compound as a chiral salt complex, or crystallizing the compound in a chiral solvent. Enantiomers and stereoisomers can also be obtained from stereomerically or enantiomerically pure intermediates, reagents, and catalysts by well-known asymmetric synthetic methods.

The term "methylene unit," as used herein represents a divalent -CH₂- moiety.

The term "nitro," as used herein, represents a -NO₂ group.

The term "oxo" as used herein, represents =O.

The term "saturated, nitrogen-containing heterocyclyl," as used herein represents a heterocyclyl moiety containing no double bonds in the ring and containing at least one nitrogen atom. Examples of a "saturated, nitrogen-containing heterocyclyl" include azetidinyl, pyrrolidinyl, piperidinyl, piperazinyl, morpholinyl, thiomorpholinyl and the like.

The term "spirocyclyl," as used herein, represents a C₂-C₇ alkylene diradical, both ends of which are bonded to the same carbon atom of the parent group to form a spirocyclic group, and also a C₁-C₆ heteroalkylene diradical, both ends of which are bonded to the same atom. The heteroalkylene radical forming the spirocyclyl group can containing one, two, three, or four heteroatoms independently selected from the group consisting of nitrogen, oxygen, and sulfur. In some embodiments, the spirocyclyl group includes one to seven carbons, excluding the carbon atom to which the diradical is attached. The spirocyclyl groups of the invention may be optionally substituted with 1, 2, 3, or 4 substituents provided herein as optional substituents for cycloalkyl and/or heterocyclyl groups.

The term "stereoisomer," as used herein, refers to all possible different isomeric as well as conformational forms which a compound may possess (e.g., a compound of any formula described herein), in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

The term "sulfonyl," as used herein, represents an -S(O)₂- group.

### Definitions

In this application, unless otherwise clear from context, (i) the term "a" may be understood to mean "at least one"; (ii) the term "or" may be understood to mean "and/or"; (iii) the terms "comprising" and "including" may be understood to encompass itemized components or steps whether presented by themselves or together with one or more additional components or steps; and (iv) the terms "about" and "approximately" may be understood to permit standard variation as would be understood by those of ordinary skill in the art; and (v) where ranges are provided, endpoints are included.

As used herein, the term "adjacent" in the context of describing adjacent atoms refers to bivalent atoms that are directly connected by a covalent bond.

As used herein, the term "administration" refers to the administration of a composition (e.g., a compound, a complex or a preparation that includes a compound or complex as described herein) to a subject or system. Administration to an animal subject (e.g., to a human) may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal and vitreal.

As is known in the art, "affinity" is a measure of the tightness with which a particular ligand binds to its partner. Affinities can be measured in different ways. In some embodiments, affinity is measured by a quantitative assay. In some such embodiments, binding partner concentration may be fixed to be in excess of ligand concentration so as to mimic physiological conditions. Alternatively or additionally, in some embodiments, binding partner concentration and/or ligand concentration may be varied. In some such embodiments, affinity may be compared to a reference under comparable conditions (e.g., concentrations).

As used herein, the term "animal" refers to any member of the animal kingdom. In some embodiments, "animal" refers to humans, at any stage of development. In some embodiments, "animal" refers to non-human animals, at any stage of development. In some embodiments, the non-human animal is a mammal (e.g., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, and/or worms. In some embodiments, an animal may be a transgenic animal, genetically-engineered animal, and/or a clone.

As used herein, the term "antagonist" refers to a compound that i) inhibits, decreases or reduces the effects of a target protein (e.g., a eukaryotic target protein such as a mammalian target protein or a fungal target protein or a prokaryotic target protein such as a bacterial target protein); and/or ii) inhibits, decreases, reduces, or delays one or more biological events. An antagonist may be direct (in which case it exerts its influence directly upon its target) or indirect (in which case it exerts its influence by other than binding to its target; e.g., by interacting with a regulator of the target protein (e.g., a eukaryotic target protein such as a mammalian target protein or a fungal target protein or a prokaryotic target protein such as a bacterial target protein), for example so that level or activity of the target protein is altered).

As used herein, the terms "approximately" and "about" are each intended to encompass normal statistical variation as would be understood by those of ordinary skill in the art as appropriate to the relevant context. In certain embodiments, the terms "approximately" or "about" each refer to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of a stated value, unless otherwise stated or otherwise evident from the context (e.g., where such number would exceed 100% of a possible value).

Two events or entities are "associated" with one another, as that term is used herein, if the presence, level and/or form of one is correlated with that of the other. For example, a particular entity (e.g., polypeptide) is considered to be associated with a particular disease, disorder, or condition, if its presence, level and/or form correlates with incidence of and/or susceptibility of the disease, disorder, or condition (e.g., across a relevant population). In some embodiments, two or more entities are physically "associated" with one another if they interact, directly or indirectly, so that they are and remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

It will be understood that the term "binding" as used herein, typically refers to association (e.g., non-covalent or covalent) between or among two or more entities. "Direct" binding involves physical contact between entities or moieties; indirect binding involves physical interaction by way of physical contact with one or more intermediate entities. Binding between two or more entities can typically be assessed in any of a variety of contexts - including where interacting entities or moieties are studied in isolation or in the context of more complex systems (e.g., while covalently or otherwise associated with a carrier entity and/or in a biological system or cell).

The affinity of a molecule X for its partner Y can generally be represented by the dissociation constant (K_{D}). Affinity can be measured by common methods known in the art, including those described herein. Specific illustrative and exemplary embodiments for measuring binding affinity are described below. The term "K_{D}," as used herein, is intended to refer to the dissociation equilibrium constant of a particular compound-protein or complex-protein interaction. Typically, the compounds of the invention bind to presenter proteins with a dissociation equilibrium constant (K_{D}) of less than about 10-⁶ M, such as less than approximately 10-⁷ M, 10-⁸ M, 10-⁹ M, or 10-¹⁰ M or even lower, e.g., when determined by surface plasmon resonance (SPR) technology using the presenter protein as the analyte and the compound as the ligand. The presenter protein/compound complexes of the invention bind to target proteins (e.g., a eukaryotic target protein such as a mammalian target protein or a fungal target protein or a prokaryotic target protein such as a bacterial target protein) with a dissociation equilibrium constant (K_{D}) of less than about 10-⁶ M, such as less than approximately 10-⁷ M, 10-⁸ M, 10-⁹ M, or 10-¹⁰ M or even lower, e.g., when determined by surface plasmon resonance (SPR) technology using the target protein as the analyte and the complex as the ligand.

As used herein, the term "combination therapy" refers to those situations in which a subject is simultaneously exposed to two or more therapeutic regimens (e.g., two or more compounds such as compounds of this invention). In some embodiments, two or more compounds may be administered simultaneously; in some embodiments, such compounds may be administered sequentially; in some embodiments, such compounds are administered in overlapping dosing regimens.

The term "comparable," as used herein, refers to two or more compounds, entities, situations, sets of conditions, etc that may not be identical to one another but that are sufficiently similar to permit comparison therebetween so that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such compounds, entities, situations, sets of conditions, etc to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

As used herein, the term "complex" refers to a group of two or more compounds and/or proteins which are bound together through a binding interaction (e.g., a non-covalent interaction, such as a hydrophobic effect interaction, an electrostatic interaction, a van der Waals interaction, or π-effect interaction). Examples of complexes are "presenter protein/compound complex" which include a compound of the invention bound to a presenter protein.

As used herein, the term "corresponding to" is often used to designate a structural element or moiety in a compound of interest that shares a position (e.g., in three-dimensional space or relative to another element or moiety) with one present in an appropriate reference compound. For example, in some embodiments, the term is used to refer to position/identity of a residue in a polymer, such as an amino acid residue in a polypeptide or a nucleotide residue in a nucleic acid. Those of ordinary skill will appreciate that, for purposes of simplicity, residues in such a polymer are often designated using a canonical numbering system based on a reference related polymer, so that a residue in a first polymer "corresponding to" a residue at position 190 in the reference polymer, for example, need not actually be the 190^{th} residue in the first polymer but rather corresponds to the residue found at the 190^{th} position in the reference polymer; those of ordinary skill in the art readily appreciate how to identify "corresponding" amino acids, including through use of one or more commercially-available algorithms specifically designed for polymer sequence comparisons.

Many methodologies described herein include a step of "determining." Those of ordinary skill in the art, reading the present specification, will appreciate that such "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference.

As used herein, the term "dosage form" refers to a physically discrete unit of an active compound (e.g., a therapeutic or diagnostic agent) for administration to a subject. Each unit contains a predetermined quantity of active agent. In some embodiments, such quantity is a unit dosage amount (or a whole fraction thereof) appropriate for administration in accordance with a dosing regimen that has been determined to correlate with a desired or beneficial outcome when administered to a relevant population (i.e., with a therapeutic dosing regimen). Those of ordinary skill in the art appreciate that the total amount of a therapeutic composition or compound administered to a particular subject is determined by one or more attending physicians and may involve administration of multiple dosage forms.

As used herein, the term "dosing regimen" refers to a set of unit doses (typically more than one) that are administered individually to a subject, typically separated by periods of time. In some embodiments, a given therapeutic compound has a recommended dosing regimen, which may involve one or more doses. In some embodiments, a dosing regimen comprises a plurality of doses each of which are separated from one another by a time period of the same length; in some embodiments, a dosing regimen comprises a plurality of doses and at least two different time periods separating individual doses. In some embodiments, all doses within a dosing regimen are of the same unit dose amount. In some embodiments, different doses within a dosing regimen are of different amounts. In some embodiments, a dosing regimen comprises a first dose in a first dose amount, followed by one or more additional doses in a second dose amount different from the first dose amount. In some embodiments, a dosing regimen comprises a first dose in a first dose amount, followed by one or more additional doses in a second dose amount same as the first dose amount. In some embodiments, a dosing regimen is correlated with a desired or beneficial outcome when administered across a relevant population (i.e., is a therapeutic dosing regimen).

The term "macrocyclic compound," as used herein, refers to a small molecule compound containing a ring with nine or more ring atoms. In some embodiments, a macrocyclic compound is a small molecule in which greater than 25% (e.g., greater than 30%, greater than 35%, greater than 40%, greater than 45%) of the non-hydrogen atoms in the small molecule are included in a single or fused ring structure.

The term "modulator" is used to refer to an entity whose presence or level in a system in which an activity of interest is observed correlates with a change in level and/or nature of that activity as compared with that observed under otherwise comparable conditions when the modulator is absent. In some embodiments, a modulator is an activator, in that activity is increased in its presence as compared with that observed under otherwise comparable conditions when the modulator is absent. In some embodiments, a modulator is an antagonist or inhibitor, in that activity is reduced in its presence as compared with otherwise comparable conditions when the modulator is absent. In some embodiments, a modulator interacts directly with a target entity whose activity is of interest. In some embodiments, a modulator interacts indirectly (i.e., directly with an intermediate compound that interacts with the target entity) with a target entity whose activity is of interest. In some embodiments, a modulator affects level of a target entity of interest; alternatively or additionally, in some embodiments, a modulator affects activity of a target entity of interest without affecting level of the target entity. In some embodiments, a modulator affects both level and activity of a target entity of interest, so that an observed difference in activity is not entirely explained by or commensurate with an observed difference in level. In some embodiments, a modulator is an allosteric modulator such as an allosteric agonist.

As used herein, the term "mutant RAS protein" means a RAS protein (e.g., KRAS, NRAS, HRAS) that comprises at least one mutation in which a non-cysteine amino acid in the corresponding wild-type RAS protein is mutated to a cysteine.

As used herein, the term "pharmaceutical composition" refers to an active compound, formulated together with one or more pharmaceutically acceptable carriers. In some embodiments, active compound is present in unit dose amount appropriate for administration in a therapeutic regimen that shows a statistically significant probability of achieving a predetermined therapeutic effect when administered to a relevant population. In some embodiments, pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

A "pharmaceutically acceptable excipient," as used herein, refers any inactive ingredient (for example, a vehicle capable of suspending or dissolving the active compound) having the properties of being nontoxic and non-inflammatory in a subject. Typical excipients include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspensing or dispersing agents, sweeteners, or waters of hydration. Excipients include, but are not limited to: butylated optionally substituted hydroxyltoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, optionally substituted hydroxylpropyl cellulose, optionally substituted hydroxylpropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, stearic acid, sucrose, talc, titanium dioxide, vitamin A, vitamin E, vitamin C, and xylitol. Those of ordinary skill in the art are familiar with a variety of agents and materials useful as excipients.

The term "pharmaceutically acceptable salt," as use herein, refers to those salts of the compounds described here that are, within the scope of sound medical judgment, suitable for use in contact with the tissues of humans and animals without undue toxicity, irritation, allergic response and the like and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting the free base group with a suitable organic acid.

The compounds of the invention may have ionizable groups so as to be capable of preparation as pharmaceutically acceptable salts. These salts may be acid addition salts involving inorganic or organic acids or the salts may, in the case of acidic forms of the compounds of the invention be prepared from inorganic or organic bases. Frequently, the compounds are prepared or used as pharmaceutically acceptable salts prepared as addition products of pharmaceutically acceptable acids or bases. Suitable pharmaceutically acceptable acids and bases are well-known in the art, such as hydrochloric, sulphuric, hydrobromic, acetic, lactic, citric, or tartaric acids for forming acid addition salts, and potassium hydroxide, sodium hydroxide, ammonium hydroxide, caffeine, various amines, and the like for forming basic salts. Methods for preparation of the appropriate salts are well-established in the art.

Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-optionally substituted hydroxyl-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts and the like. Representative alkali or alkaline earth metal salts include sodium, lithium, potassium, calcium, magnesium and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine and the like.

The term "presenter protein" refers to a protein that binds to a small molecule to form a complex that binds to and modulates the activity of a target protein (e.g., a eukaryotic target protein such as a mammalian target protein or a fungal target protein or a prokaryotic target protein such as a bacterial target protein). In some embodiments, the presenter protein is a relatively abundant protein (e.g., the presenter protein is sufficiently abundant that participation in a ternary complex does not substantially impact the biological role of the presenter protein in a cell and/or viability or other attiributes of the cell). In certain embodiments, the presenter protein is a protein that has chaperone activity within a cell. In some embodiments, the presenter protein is a protein that has multiple natural interaction partners within a cell. In certain embodiments, the presenter protein is one which is known to bind a small molecule to form a binary complex that is known to or suspected of binding to and modulating the biological activity of a target protein.

The term "pure" means substantially pure or free of unwanted components (e.g., other compounds and/or other components of a cell lysate), material defilement, admixture or imperfection.

The term "reference" is often used herein to describe a standard or control compound, individual, population, sample, sequence or value against which a compound, individual, population, sample, sequence or value of interest is compared. In some embodiments, a reference compound, individual, population, sample, sequence or value is tested and/or determined substantially simultaneously with the testing or determination of the compound, individual, population, sample, sequence or value of interest. In some embodiments, a reference compound, individual, population, sample, sequence or value is a historical reference, optionally embodied in a tangible medium. Typically, as would be understood by those skilled in the art, a reference compound, individual, population, sample, sequence or value is determined or characterized under conditions comparable to those utilized to determine or characterize the compound, individual, population, sample, sequence or value of interest.

The term "small molecule" means a low molecular weight organic and/or inorganic compound. In general, a "small molecule" is a molecule that is less than about 5 kilodaltons (kD) in size. In some embodiments, a small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, a small molecule is not a polymer. In some embodiments, a small molecule does not include a polymeric moiety. In some embodiments, a small molecule is not a protein or polypeptide (e.g., is not an oligopeptide or peptide). In some embodiments, a small molecule is not a polynucleotide (e.g., is not an oligonucleotide). In some embodiments, a small molecule is not a polysaccharide. In some embodiments, a small molecule does not comprise a polysaccharide (e.g., is not a glycoprotein, proteoglycan, glycolipid, *etc*.). In some embodiments, a small molecule is not a lipid. In some embodiments, a small molecule is a modulating compound. In some embodiments, a small molecule is biologically active. In some embodiments, a small molecule is detectable (e.g., comprises at least one detectable moiety). In some embodiments, a small molecule is a therapeutic.

Those of ordinary skill in the art, reading the present disclosure, will appreciate that certain small molecule compounds described herein may be provided and/or utilized in any of a variety of forms such as, for example, salt forms, protected forms, pro-drug forms, ester forms, isomeric forms (e.g., optical and/or structural isomers), isotopic forms, etc. In some embodiments, reference to a particular compound may relate to a specific form of that compound. In some embodiments, reference to a particular compound may relate to that compound in any form. In some embodiments, where a compound is one that exists or is found in nature, that compound may be provided and/or utilized in accordance in the present invention in a form different from that in which it exists or is found in nature. Those of ordinary skill in the art will appreciate that a compound preparation including a different level, amount, or ratio of one or more individual forms than a reference preparation or source (e.g., a natural source) of the compound may be considered to be a different form of the compound as described herein. Thus, in some embodiments, for example, a preparation of a single stereoisomer of a compound may be considered to be a different form of the compound than a racemic mixture of the compound; a particular salt of a compound may be considered to be a different form from another salt form of the compound; a preparation containing one conformational isomer ((Z) or (E)) of a double bond may be considered to be a different form from one containing the other conformational isomer ((E) or (Z)) of the double bond; a preparation in which one or more atoms is a different isotope than is present in a reference preparation may be considered to be a different form; etc.

As used herein, the terms "specific binding" or "specific for" or "specific to" refer to an interaction between a binding agent and a target entity. As will be understood by those of ordinary skill, an interaction is considered to be "specific" if it is favored in the presence of alternative interactions, for example, binding with a K_{D} of less than 10 µM (e.g., less than 5 µM, less than 1 µM, less than 500 nM, less than 200 nM, less than 100 nM, less than 75 nM, less than 50 nM, less than 25 nM, less than 10 nM). In many embodiments, specific interaction is dependent upon the presence of a particular structural feature of the target entity (e.g., an epitope, a cleft, a binding site). It is to be understood that specificity need not be absolute. In some embodiments, specificity may be evaluated relative to that of the binding agent for one or more other potential target entities (e.g., competitors). In some embodiments, specificity is evaluated relative to that of a reference specific binding agent. In some embodiments, specificity is evaluated relative to that of a reference non-specific binding agent.

The term "specific" when used with reference to a compound having an activity, is understood by those skilled in the art to mean that the compound discriminates between potential target entities or states. For example, in some embodiments, a compound is said to bind "specifically" to its target if it binds preferentially with that target in the presence of one or more competing alternative targets. In many embodiments, specific interaction is dependent upon the presence of a particular structural feature of the target entity (e.g., an epitope, a cleft, a binding site). It is to be understood that specificity need not be absolute. In some embodiments, specificity may be evaluated relative to that of the binding agent for one or more other potential target entities (e.g., competitors). In some embodiments, speicifcity is evaluated relative to that of a reference specific binding agent. In some embodiments specificity is evaluated relative to that of a reference non-specific binding agent. In some embodiments, the agent or entity does not detectably bind to the competing alternative target under conditions of binding to its target entity. In some embodiments, binding agent binds with higher on-rate, lower off-rate, increased affinity, decreased dissociation, and/or increased stability to its target entity as compared with the competing alternative target(s).

A "therapeutic regimen" refers to a dosing regimen whose administration across a relevant population is correlated with a desired or beneficial therapeutic outcome.

The term "therapeutically effective amount" means an amount that is sufficient, when administered to a population suffering from or susceptible to a disease, disorder, and/or condition in accordance with a therapeutic dosing regimen, to treat the disease, disorder, and/or condition. In some embodiments, a therapeutically effective amount is one that reduces the incidence and/or severity of, and/or delays onset of, one or more symptoms of the disease, disorder, and/or condition. Those of ordinary skill in the art will appreciate that the term "therapeutically effective amount" does not in fact require successful treatment be achieved in a particular individual. Rather, a therapeutically effective amount may be that amount that provides a particular desired pharmacological response in a significant number of subjects when administered to patients in need of such treatment. It is specifically understood that particular subjects may, in fact, be "refractory" to a "therapeutically effective amount." To give but one example, a refractory subject may have a low bioavailability such that clinical efficacy is not obtainable. In some embodiments, reference to a therapeutically effective amount may be a reference to an amount as measured in one or more specific tissues (e.g., a tissue affected by the disease, disorder or condition) or fluids (e.g., blood, saliva, serum, sweart, tears, urine, etc). Those of ordinary skill in the art will appreciate that, in some embodiments, a therapeutically effective amount may be formulated and/or administered in a single dose. In some embodiments, a therapeutically effective amount may be formulated and/or administered in a plurality of doses, for example, as part of a dosing regimen.

The term "treatment" (also "treat" or "treating"), in its broadest sense, refers to any administration of a substance (e.g., provided compositions) that partially or completely alleviates, ameliorates, relives, inhibits, delays onset of, reduces severity of, and/or reduces incidence of one or more symptoms, features, and/or causes of a particular disease, disorder, and/or condition. In some embodiments, such treatment may be administered to a subject who does not exhibit signs of the relevant disease, disorder and/or condition and/or of a subject who exhibits only early signs of the disease, disorder, and/or condition. Alternatively or additionally, in some embodiments, treatment may be administered to a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

The term "variant" refers to an entity that shows significant structural identity with a reference entity but differs structurally from the reference entity in the presence or level of one or more chemical moieties as compared with the reference entity. In many embodiments, a variant also differs functionally from its reference entity. In general, whether a particular entity is properly considered to be a "variant" of a reference entity is based on its degree of structural identity with the reference entity. As will be appreciated by those skilled in the art, any biological or chemical reference entity has certain characteristic structural elements. A variant, by definition, is a distinct chemical entity that shares one or more such characteristic structural elements. To give but a few examples, a small molecule may have a characteristic core structural element (e.g., a hexahydropyridazine core) and/or one or more characteristic pendent moieties so that a variant of the small molecule is one that shares the core structural element and the characteristic pendent moieties but differs in other pendent moieties and/or in types of bonds present (single vs double, E vs Z, etc) within the core, a polypeptide may have a characteristic sequence element comprised of a plurality of amino acids having designated positions relative to one another in linear or three-dimensional space and/or contributing to a particular biological function, a nucleic acid may have a characteristic sequence element comprised of a plurality of nucleotide residues having designated positions relative to on another in linear or three-dimensional space. For example, a variant polypeptide may differ from a reference polypeptide as a result of one or more differences in amino acid sequence and/or one or more differences in chemical moieties (e.g., carbohydrates, lipids, etc) covalently attached to the polypeptide backbone. In some embodiments, a variant polypeptide shows an overall sequence identity with a reference polypeptide that is at least 85%, 86%, 87%, 88%, 89%, 90%, 91%, 92%, 93%, 94%, 95%, 96%, 97%, or 99%. Alternatively or additionally, in some embodiments, a variant polypeptide does not share at least one characteristic sequence element with a reference polypeptide. In some embodiments, the reference polypeptide has one or more biological activities. In some embodiments, a variant polypeptide shares one or more of the biological activities of the reference polypeptide. In some embodiments, a variant polypeptide lacks one or more of the biological activities of the reference polypeptide. In some embodiments, a variant polypeptide shows a reduced level of one or more biological activities as compared with the reference polypeptide. In many embodiments, a polypeptide of interest is considered to be a "variant" of a parent or reference polypeptide if the polypeptide of interest has an amino acid sequence that is identical to that of the parent but for a small number of sequence alterations at particular positions. Typically, fewer than 20%, 15%, 10%, 9%, 8%, 7%, 6%, 5%, 4%, 3%, 2% of the residues in the variant are substituted as compared with the parent. In some embodiments, a variant has 10, 9, 8, 7, 6, 5, 4, 3, 2, or 1 substituted residue as compared with a parent. Often, a variant has a very small number (e.g., fewer than 5, 4, 3, 2, or 1) number of substituted functional residues (i.e., residues that participate in a particular biological activity). Furthermore, a variant typically has not more than 5, 4, 3, 2, or 1 additions or deletions, and often has no additions or deletions, as compared with the parent. Moreover, any additions or deletions are typically fewer than about 25, about 20, about 19, about 18, about 17, about 16, about 15, about 14, about 13, about 10, about 9, about 8, about 7, about 6, and commonly are fewer than about 5, about 4, about 3, or about 2 residues. In some embodiments, the parent or reference polypeptide is one found in nature. As will be understood by those of ordinary skill in the art, a plurality of variants of a particular polypeptide of interest may commonly be found in nature.

The term "wild-type" refers to an entity having a structure and/or activity as found in nature in a "normal" (as contrasted with mutant, diseased, altered, etc) state or context. Those of ordinary skill in the art will appreciate that wild-type genes and polypeptides often exist in multiple different forms (e.g., alleles).

### Brief Description of the Figures

**FIG. 1** illustrates compounds 1-418 of the invention, the general scheme by which they were made or the specific example that describes their synthesis, and their mass spectrometry and/or NMR values. Compounds 419-461 in FIG. 1 are additional compounds of the invention, prepared using similar methodologies.

### Detailed Description

### Compounds

The present disclosure features compounds of formula (I): 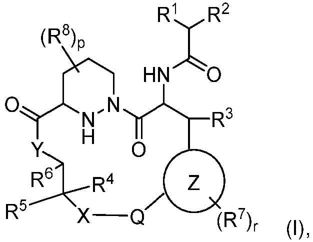 a pharmaceutically acceptable salt, an enantiomer, a stereoisomer, or a tautomer thereof.

The present disclosure features complexes including a presenter protein, a compound of the present invention (e.g., a compound of formula (I) or any of compounds 1-461), or a pharmaceutically acceptable salt, enantiomer, stereoisomer, or tautomer thereof, and a target protein.

The present disclsoure features compounds (e.g., a compound of formula (I) or any of compounds 1-461) capable of modulating biological processes, for example through binding to a presenter protein (e.g., a member of the cyclophilin family) and a target protein (e.g. a member of the RAS family). In some embodiments, the target and/or presenter proteins are intracellular proteins. In some embodiments, the target and/or presenter proteins are mammalian proteins. In some embodiments, provided compounds participate in ternary presenter protein-compound-target protein complexes inside cells, e.g., mammalian cells. In some embodiments, provided compounds may be useful in the treatment of diseases and disorders such as cancer, inflammation, or infections.

### Compound Synthesis

The following general reaction schemes illustrate exemplary methods of making compounds of Formula I, or a pharmaceutically acceptable salt thereof.

Coupling agents useful in these schemes include, but are not limited to dicyclohexylcarbodiimide (DCC), diisopropylcarbodiimide (DIC), ethyl-(*N',N'-*dimethylamino)propylcarbodiimide hydrochloride (EDC), 1-hydroxybenzotriazole (HOBt)/EDC, (benzotriazol-1-yloxy)tris(dimethylamino)phosphonium hexafluorophosphate (BOP), (benzotriazol-1-yloxy)tripyrrolidinophosphonium hexafluorophosphate (PyBOP), bromotripyrrolidinophosphonium hexafluorophosphate (PyBROP), (7-azabenzotriazol-1-yloxy)tripyrrolidinophosphonium hexafluorophosphate (PyAOP), *O*-(benzotriazol-1-yl)-*N,N,N',N*'-tetramethyluronium hexafluorophosphate (HBTU), *O*-(benzotriazol-1-yl)-*N,N,N',N'*-tetramethyluronium tetrafluoroborate (TBTU), *O*-(7-azabenzotriazol-1-yl)-*N,N,N',N'*-tetramethyluronium hexafluorophosphate (HATU), *O*-(7-azabenzotriazol-1-yl)-*N,N,N',N'*-tetramethyluronium tetrafluoroborate (TATU), *O*-(6-chlorobenzotriazol-1-yl)-*N,N,N',N'*-tetramethyluronium hexafluorophosphate(HCTU), carbonyldiimidazole (CDI), (1-cyano-2-ethoxy-2-oxoethylidenaminooxy)dimethylamino-morpholino-carbenium hexafluorophosphate (COMU^{®}), 1-propanephosphonic anhydride (T₃P^{®}), a combination of 2,2'-dipyridyl disulfide and triphenylphosphine, and the like known to those skilled in the art.

Coupling is typically achieved in an organic solvent such as, but not limited to, *N,N-*dimethylformamide (DMF), dichloromethane (DCM), acetonitrile, and tetrahydrofuran (THF) in the presence of base, such as, but not limited to diisopropylethylamine, triethylamine, and *N-*methylmorpholine.

Coupling reactions may be conducted with or without DMAP (in catalytic, stoichiometric, or superstoichiometric amounts, but more specifically catalytic amounts) at temperatures ranging from - 78 °C to about 120 °C, but specifically from from -20 °C to 50 °C, and more specifically from -5 °C to 30 °C.

Cross-coupling reactions useful in synthesizing the compounds of the invention include, but are not limited to, Suzuki coupling, Negishi coupling, Stille coupling, Kumada coupling, and Hiyama coupling.

A cross-coupling reaction generally requires a metal catalyst or a mixture of metal catalysts. Suitable metal catalysts include, but are not limited to, palladium catalysts, copper catalysts, nickel catalysts, iron catalysts, silver catalysts, gold catalysts, or a combination of two or more of these catalysts. Suitable palladium catalysts include, but are not limited to, palladium on carbon (Pd/C), palladium acetate (Pd(OAc)₂), tetrakis(triphenylphosphine)palladium(0) (Pd(PPh₃)₄), bis(triphenylphosphine)palladium(II) dichloride (PdCl₂(PPh₃)₂), [1,1'-bis(diphenylphosphino)ferrocene]palladium(II) dichloride ((dppf)PdCl₂), and tris(dibenzylideneacetone)dipalladium(0) (Pd₂(dba)₃). Suitable copper catalysts include, but are not limited to, CuCl, CuBr, CuI, Cu₂O, CuOTf, Cu(MeCN)₄PF₆, CuTC (copper(I) thiophene-2-carboxylate), Cu(OAc)₂, and Cu(OTf)₂. Suitable nickel catalysts include, but are not limited to, bis(cyclooctadiene)nickel(0), bis(triphenylphosphine)nickel chloride, [1,2-bis(diphenylphosphino)ethane]dichloronickel(II) ((dppe)NiCl₂), [1,1'-bis(diphenylphosphino)ferrocene]dichloronickel(II) ((dppf)NiCl₂), and [1,3-bis(diphenylphosphino)propane]dichloronickel(II) ((1,3-dppp)NiCl₂). Suitable iron catalysts include, but are not limited to, FeCl₂, FeCl₃, Fe(acac)₃, and Fe(OAc)₂. Suitable silver catalysts include, but are not limited to, Ag(OAc), AgOTf, AgPF₆, and AgClO₄. Suitable gold catalysts include, but are not limited to, chloro(triphenylphosphine)gold(I) ((Ph₃P)AuCl), chloro[1,3-bis(2,6-diisopropylphenyl)imidazol-2-ylidene]gold(I), methyl(triphenylphosphine)gold(I), chloro[1,3-bis(2,4,6-trimethylphenyl)imidazol-2-ylidene]gold(I), and chloro(trimethylphosphine)gold(I).

The cross-coupling reaction may be carried out in a suitable solvent at temperatures between - 78 °C and 250 °C, more specifically at 0 °C to 120 °C.

A suitable solvent for a cross-coupling reaction may be, but is not limited to, MeOH, EtOH, isopropanol, *tert*-butanol, H₂O, DMF, DMSO, THF, 1,4-dioxane, 1,2-dimethoxyethane, or a mixture of two or more of these solvents.

The cross-coupling reaction may be performed under conventional heating or in a microwave reactor. Certain cross-coupling reactions are carried out under nitrogen or argon atmosphere. Other cross-coupling reactions may require the presence of air or oxygen. Additionally, base may be necessary for some cross-coupling reactions. Suitable bases include, but are not limited to, AgO, K₂CO₃, tBuOK, tBuONa, Cs₂CO₃, and K₃PO₄.

Reactive groups for Suzuki cross-coupling reactions (referred to B¹ and B² in the below schemes) are typically (1) a boronic acid, bororic ester or a trifluoroborate salt moiety, such as but not limited to -B(OH)₂, -B(OMe)₂, -B(OEt)₂, -B(OPr-*i*)₂, -B(pinacolato), and -BF₃K; (2) a halogen or a sulfonic ester group, such as but not limited to Cl, Br, I, -O₃SCF₃, -O₃SC₆H₄Me-*p*, and -O₃SC₆H₅.

Various protecting groups (PG) are used in these schemes. Suitable amine protecting group including, but not limited to, *tert*-butyloxycarbonyl (Boc), carbobenzyloxy (Cbz), *p*-methoxybenzyl carbonyl (Moz), allyloxycarbonyl (Alloc), 9-fluorenylmethyloxycarbonyl (Fmoc), *p*-methoxybenzyl (PMB), 3,4-dimethoxybenzyl (DMPM), benzoyl (Bz), acetyl (Ac), methanesulfonyl (Ms), trifluoromethanesulfonyl (Tf), *p*-toluenesulfonyl (Ts), and 4-nitrobenzenesulfonyl (Nosyl). In certain embodiments, the amine protecting group is *tert*-butyloxycarbonyl (Boc). Suitable alcohol protecting groups include, but are not limited to, silyl groups (including, but not limited to, -SiMe₃, -SiEt₃, -Si(*iso*-Pr)₃, -SiMe₂(*tert*-Bu), -SiPh₂(*tert*-Bu), -SEM (2-(trimethylsilyl)ethoxymethyl)), ether groups (including, but not limited to, -MOM (methoxymethyl), -MEM (2-methoxyethoxymethyl), -BOM (benzyloxymethyl), -PMBM (*p*-methoxybenzyloxymethyl), and -THP (tetrahydropyranyl)), and ester groups (including, but not limited to, acetate (Ac), formate, pivaloate (Pv), and benzoate). In certain embodiments, the alcohol protecting group is acetyl. Some protecting groups are an alkyl or any aryl group, such as but not limited to methyl, ethyl, *n*-propyl, isopropyl, *tert*-butyl, benzyl, *p*-methoxybenzyl, allyl, phenyl, and *p*-nitrophenyl. In some embodiments, the alkyl or aryl protecting group is methyl.

The removal of a protecting group may be carried out under basic or acidic conditions, depending upon the nature of the protecting group. Which conditions are applicable to specific protecting groups is well-known in the art. Suitable bases for protecting group removal include, but are not limited to, LiOH, NaOH, KOH, CsOH, Li₂CO₃, Na₂CO₃, K₂CO₃, Cs₂CO₃, and CsF. Suitable acids for protecting group removal include, but are not limited to, HCl, HBr, HI, H₂SO₄, HNO₃, and CF₃CO₂H. Certain protecting groups may also be removed by using conditions or reagents, such as trimethyltin hydroxide, ceric ammonium nitrate, and oxalyl chloride.

Protecting group removal is typically out in a suitable solvent at temperatures between -78 °C and about 150 °C, specifically at 0 °C to 120 °C, more specifically at 0 °C to 25 °C. A suitable solvent for such reaction includes, but is not limited to, MeOH, EtOH, isopropanol, tert-butanol, H₂O, dichloromethane, ethyl acetate, DMF, DMSO, THF, 1,4-dioxane, and 1,2-dimethoxyethane.

Variables such as Q, X, ring Z, R¹, R², R³, R⁴, R⁵, R⁶, R⁷, R⁸, p and r have the meaning set forth in Formula I.

Dipeptide A-1 may be prepared as shown in Scheme 1 below where the structural variables are as described above.

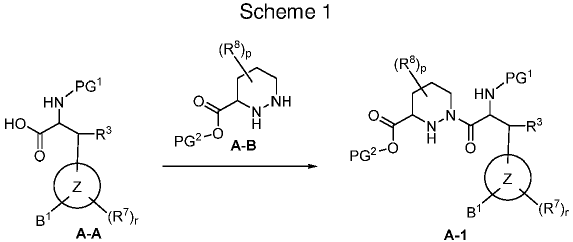

In a typical procedure, intermediate A-A is reacted with intermediate A-B in the presence of a coupling agent.

### Method A

Method A may be used to prepare compounds of Formula I as shown in Scheme 2 below. The structural variables of Formula I are as defined above.

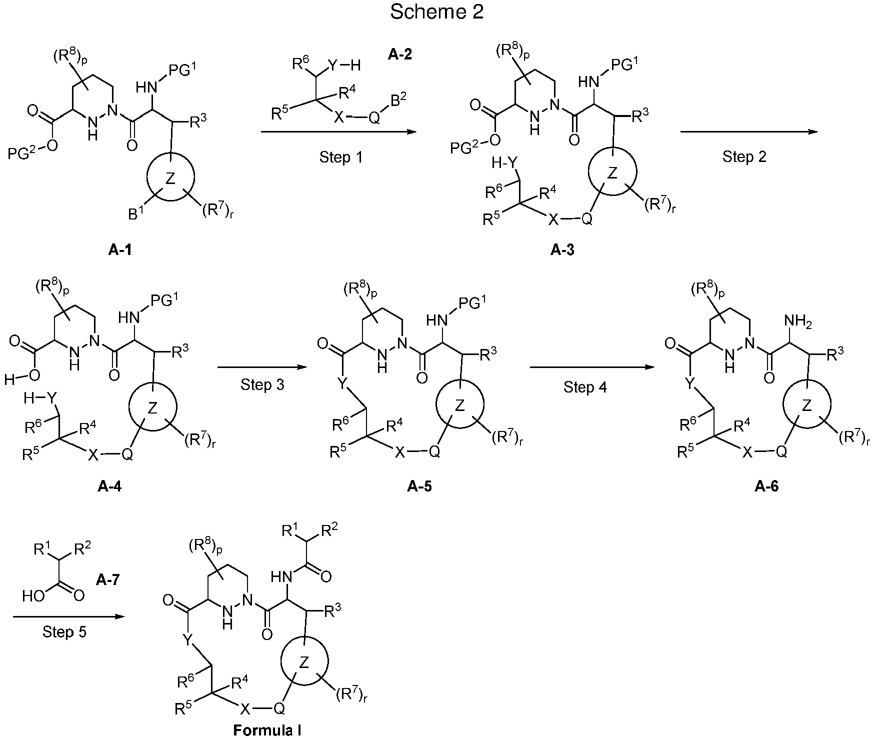

*Step* 1: Intermediate A-3 is synthesized from intermediates A-1 and A-2 via a cross-coupling reaction. In some embodiments, the cross-coupling reaction is a Suzuki coupling reaction. PG' is a suitable amine protecting group. In certain embodiments, PG¹ is tert-butyloxycarbonyl (Boc).

*Step 2:* PG² is an alkyl or aryl protecting group. In some embodiments, PG² is methyl.

Intermediate A-4 may be synthesized from A-3 using a deprotecting reaction that hydrolyzes the PG²-containing ester into its corresponding acid.

*Step 3:* The macrocyclization of intermediate A-4 gives intermediate A-5 is achieved by a cross-coupling reaction.

Alternatively, the cyclization reaction may be carried out by converting the acid group (-CO₂H) in the precursor to the corresponding acid chloride (-COCl) using a chlorinating reagent (including, but not limited to, thionyl chloride, PCl₃, PCl₅, and oxalyl chloride with catalytic DMF) in a suitable solvent at temperatures between -78 °C and 120 °C, preferably at 0 °C. A suitable solvent includes, but is not limited to, DMF, dichloromethane, tetrahydrofuran, 1,2-dimethoxyethane, acetonitrile, ethyl acetate, toluene, and 1,4-dioxane. Following formation of the acid chloride, the solvent may be removed under reduced pressure and replaced with an alternative solvent including, but not limited to, *N,N-*dimethylformamide, dichloromethane, 1,2-dimethoxyethane, acetonitrile, tetrahydrofuran, and 1,2-dichloroethane. Addition of base (including, but not limited to, pyridine, diisopropylethylamine, triethylamine, *N*-methylmorpholine, and the like) is followed to form the cyclized product. The reaction temperatures range from -78 °C to 120 °C, preferably between -20 °C and 50 °C.

*Step 4:* Intermediate A6 is synthesized from intermediate A-5 by removing PG¹.

*Step 5:* A compound of Formula I is synthesized from intermediates A-6 and A-7 via an amide formation reaction using a cross-coupling reaction. T

Alternatively, those skilled in the art will appreciate that a compound of Formula I may be synthesized by converting intermediate A-7 to its acyl chloride or acyl fluoride or activated ester or anhydride, and reacting with intermediate A-6. Examples of these types of reactions are available in the literature, such as Compendium of Organic Synthetic Methods, Vol. I-VI (Wiley-Interscience); or the Comprehensive Organic Transformations, by R.C. Larock (Wiley-Interscience).

### Method B

Method B may alternatively be used to synthesize compounds of Formula I as shown in Scheme 3 below. The structural variables of Formula I are as defined above.

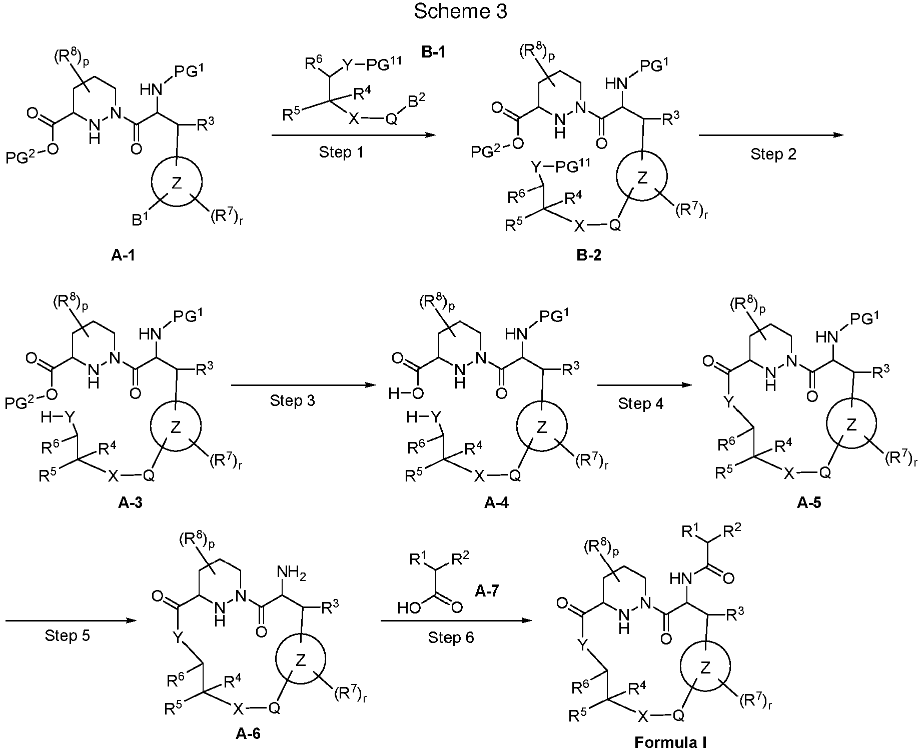

*Step 1:* PG¹¹ is a suitable alcohol (when Y = O) or amine (when Y = NH or N(C₁-C₃ alkyl)) protecting group.

Intermediate B-2 is synthesized from intermediates A-1 and B-1 via a cross-coupling reaction.

*Step 2:* Intermediate A-3 may be synthesized from Intermediate B-2 by removing the PG¹¹ group from the alcoholic oxygen atom to which it is attached or from an amino nitrogen atom to which it is attached.

*Steps 3-6:* The conversion of Intermediate A-3 to Formula I is detailed in the description of Method A.

### Method C

Method C may alternatively be used to synthesize compounds of Formula I as shown in Scheme 4 below. The structural variables of Formula I are as defined above.

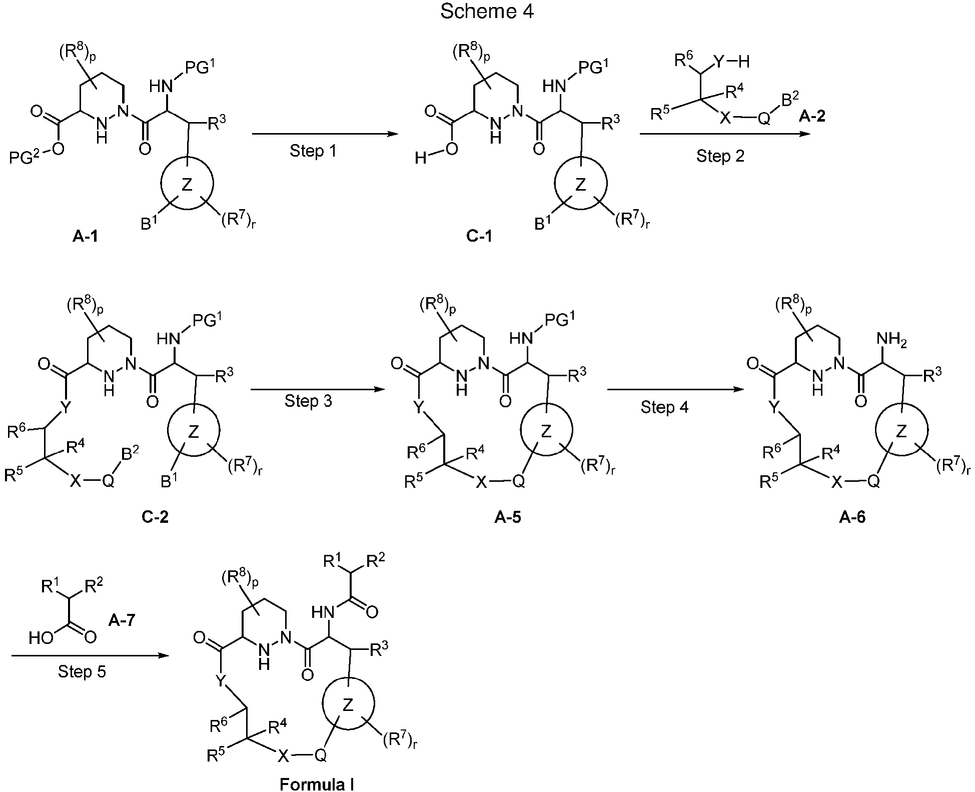

*Step 1*: Intermediate C-1 may be synthesized from A-1 using a deprotecting reaction that hydrolyzes the PG²-containing ester to its corresponding acid.

*Step 2:* Intermediate C-2 may be synthesized from C-1 via amide formation using a cross-coupling reaction.

Alternatively, those skilled in the art will appreciate that a compound of Formula I may be synthesized by converting intermediate A-7 to its acyl chloride, acyl fluoride, activated ester or anhydride, and reacting with intermediate A-2. Examples of these types of reactions are available in literature, such as Compendium of Organic Synthetic Methods, Vol. I-VI (Wiley-Interscience); or the Comprehensive Organic Transformations, by R.C. Larock (Wiley-Interscience).

*Step 3:* Macrocycle A-5 may be synthesized from Intermediate C-2 using a cross-coupling reaction. In some embodiments, the cross-coupling reaction is a Suzuki coupling reaction.

*Steps 4-5*: The conversion of macrocycle A-5 to Formula I is detailed in the description of Method A.

### Proteins

### Presenter Proteins

Presenter proteins can bind a compound of the invention to form a complex, which can bind to and modulate the activity of a mutant RAS target protein. The presenter protein is a member of the cyclophilin A family (e.g., CYPA, CYPB, CYPC, CYP40, CYPE, CYPD, NKTR, SRCyp, CYPH, CWC27, CYPL1, CYP60, CYPJ, PPIL4, PPIL6, RANBP2, PPWD1, PPIAL4A, PPIAL4B, PPIAL4C, PPIAL4D, or PPIAL4G).

The "cyclophilin family" is a family of proteins that bind to cyclosporine. Genes that encode proteins in this family include PPIA, PPIB, PPIC, PPID, PPIE, PPIF, PPIG, PPIH, SDCCAG-10, PPIL1, PPIL2, PPIL3, PPIL4, P270, PPWD1, and COAS-2. Exemplary cyclophilins include CYPA, CYPB, CYPC, CYP40, CYPE, CYPD, NKTR, SRCyp, CYPH, CWC27, CYPL1, CYP60, CYPJ, PPIL4, PPIL6, RANBP2, PPWD1, PPIAL4A, PPIAL4B, PPIAL4C, PPIAL4D, and PPIAL4G.

Representative presenter proteins are encoded by the genes or homologs thereof listed in Table 1; in some embodiments, a reference presenter protein is encoded by a gene set forth in Table 1. Also, those of ordinary skill in the art, referring to Table 1, can readily identify sequences that are characteristic of presenter proteins generally, and/or of particular subsets of presenter proteins.

**Table 1. Genes that Encode Selected Presenter Proteins**

| **Gene Name** | **Uniprot Accession Number** |
|---|---|
| PPIA | Q567Q0 |
| PPIB | P23284 |
| PPIC | P45877 |
| PPID | Q08752 |
| PPIE | Q9UNP9 |
| PPIG | Q13427 |
| PPIH | O43447 |
| PPIL1 | Q9Y3C6 |
| PPIL2 | Q13356 |
| PPIL3 | Q9H2H8 |
| PPIL4 | Q8WUA2 |
| PPIL5 | Q32Q17 |
| PPIL6 | Q8IXY8 |
| PPWD1 | Q96BP3 |

### Target Proteins

A target protein (e.g., a eukaryotic target protein such as a mammalian target protein) is a protein which mediates a disease condition or a symptom of a disease condition. As such, a desirable therapeutic effect can be achieved by modulating (inhibiting or increasing) its activity. Target proteins useful in the complexes and methods of the invention include those which do not naturally associate with a presenter protein, e.g., those which have an affinity for a presenter protein in the absence of a binary complex with a compound of the invention of greater than 1 µM, preferably greater than 5 µM, and more preferably greater than 10 µM. Alternatively, target proteins which do not naturally associate with a presenter protein are those which have an affinity for a compound of the invention in the absence of a binary complex greater than 1 µM, preferably greater than 5 µM, and more preferably greater than 10 µM. In yet another alternative, target proteins which do not naturally associate with a presenter protein are those which are other than calcineurin or mTOR.

Target proteins can be naturally occurring, e.g., wild type. Alternatively, a target protein can vary from the wild type protein but still retain biological function, e.g., as a mutant, a splice variant or a biologically active fragment.

In some embodiments, the target protein is a RAS family protein.

In some embodiments, the target protein is a KRAS protein. In some embodiments, the KRAS protein is a KRAS G12C protein. In some embodiments, the KRAS protein is a KRAS G13C protein.

In some embodiments, the target protein is an NRAS protein. In some embodiments, the NRAS protein is an NRAS G12C protein. In some embodiments, the NRAS protein is an NRAS G13C protein.

In some embodiments, the target protein is an HRAS protein. In some embodiments, the HRAS protein is an HRAS G12C protein. In some embodiments, the HRAS protein is an HRAS G13C protein.

### Complexes

### Presenter protein/compound complexes

In one aspect, the invention provides a complex comprising a compound of the invention, a CYPA family member presented protein and a mutant RAS protein.

In a related aspect, this disclosure features a method of producing the above-described complex, the method including contacting a CYPA family member presenter protein and a mutant RAS protein with a compound of the present invention, any pharmaceutical composition comprising such a compound, under conditions suitable to permit complex formation.

In some embodiments of either of the above two aspects, the mutated RAS protein is KRAS G12C, NRAS G12C, or HRAS G12C. In some embodiments, the mutated RAS protein is KRAS G13C, NRAS G13C, or HRAS G13C. In some embodiments, the mutated RAS protein is KRAS G12C.

In some embodiments of either of the above two aspects, the presenter protein is CYPA, CYPB, CYPC, CYP40, CYPE, CYPD, NKTR, SRCyp, CYPH, CWC27, CYPL1, CYP60, CYPJ, PPIL4, PPIL6, RANBP2, or PPWD1. In some embodiments, the presenter protein is CYPA.

In some embodiments, a presenter protein/compound/target protein complex of the invention inhibits a naturally occurring interaction between a target protein and a ligand, such as a protein or a small molecule that specifically binds to the target protein.

In some embodiments, a presenter protein/compound/target protein complex of the invention inhibits the binding of BRAF to the mutant RAS (e.g., KRAS G12C, KRAS G13C, NRAS G12C, NRAS G13C, HRAS G12C, or HRAS G13C).

### Kits

In some embodiments, the present invention relates to a kit for conveniently and effectively carrying out the methods in accordance with the present invention. In general, the pharmaceutical pack or kit comprises one or more containers filled with one or more of the ingredients of the pharmaceutical compositions of the invention. Such kits are especially suited for the delivery of solid oral forms such as tablets or capsules. Such a kit preferably includes a number of unit dosages, and may also include a card having the dosages oriented in the order of their intended use. If desired, for instance if the subject suffers from Alzheimer's disease, a memory aid can be provided, for example in the form of numbers, letters, or other markings or with a calendar insert, designating the days in the treatment schedule in which the dosages can be administered. Alternatively, placebo dosages, or calcium dietary supplements, either in a form similar to or distinct from the dosages of the pharmaceutical compositions, can be included to provide a kit in which a dosage is taken every day. Optionally associated with such container(s) can be a notice in the form prescribed by a governmental agency regulating the manufacture, use or sale of pharmaceutical products, which notice reflects approval by the agency of manufacture, use or sale for human administration.

### Pharmaceutical Compositions

For use as treatment of human and animal subjects, the compounds of the invention can be formulated as pharmaceutical or veterinary compositions. Depending on the subject to be treated, the mode of administration, and the type of treatment desired-e.g., prevention, prophylaxis, or therapy-the compounds are formulated in ways consonant with these parameters. A summary of such techniques is found in Remington: The Science and Practice of Pharmacy, 21st Edition, Lippincott Williams & Wilkins, (2005); and Encyclopedia of Pharmaceutical Technology, eds. J. Swarbrick and J. C. Boylan, 1988-1999, Marcel Dekker, New York, each of which is incorporated herein by reference.

Compounds described herein may be present in amounts totaling 1-95% by weight of the total weight of the composition. The composition may be provided in a dosage form that is suitable for intraarticular, oral, parenteral (e.g., intravenous, intramuscular), rectal, cutaneous, subcutaneous, topical, transdermal, sublingual, nasal, vaginal, intravesicular, intraurethral, intrathecal, epidural, aural, or ocular administration, or by injection, inhalation, or direct contact with the nasal, genitourinary, reproductive or oral mucosa. Thus, the pharmaceutical composition may be in the form of, e.g., tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, preparations suitable for iontophoretic delivery, or aerosols. The compositions may be formulated according to conventional pharmaceutical practice.

In general, for use in treatment, compounds described herein may be used alone, or in combination with one or more other active agents. An example of other pharmaceuticals to combine with the compounds described herein would include pharmaceuticals for the treatment of the same indication. Another example of a potential pharmaceutical to combine with compounds described herein would include pharmaceuticals for the treatment of different yet associated or related symptoms or indications. Depending on the mode of administration, compounds will be formulated into suitable compositions to permit facile delivery. Each compound of a combination therapy may be formulated in a variety of ways that are known in the art. For example, the first and second agents of the combination therapy may be formulated together or separately. Desirably, the first and second agents are formulated together for the simultaneous or near simultaneous administration of the agents.

Compounds of the invention may be prepared and used as pharmaceutical compositions comprising an effective amount of a compound described herein and a pharmaceutically acceptable carrier or excipient, as is well known in the art. In some embodiments, a composition includes at least two different pharmaceutically acceptable excipients or carriers.

Formulations may be prepared in a manner suitable for systemic administration or topical or local administration. Systemic formulations include those designed for injection (e.g., intramuscular, intravenous or subcutaneous injection) or may be prepared for transdermal, transmucosal, or oral administration. A formulation will generally include a diluent as well as, in some cases, adjuvants, buffers, preservatives and the like. Compounds can be administered also in liposomal compositions or as microemulsions.

For injection, formulations can be prepared in conventional forms as liquid solutions or suspensions or as solid forms suitable for solution or suspension in liquid prior to injection or as emulsions. Suitable excipients include, for example, water, saline, dextrose, glycerol and the like. Such compositions may also contain amounts of nontoxic auxiliary substances such as wetting or emulsifying agents, pH buffering agents and the like, such as, for example, sodium acetate, sorbitan monolaurate, and so forth.

Various sustained release systems for drugs have also been devised. See, for example, U.S. patent No. 5,624,677, which is herein incorporated by reference.

Systemic administration may also include relatively noninvasive methods such as the use of suppositories, transdermal patches, transmucosal delivery and intranasal administration. Oral administration is also suitable for compounds of the invention. Suitable forms include syrups, capsules, and tablets, as is understood in the art.

Each compound of a combination therapy, as described herein, may be formulated in a variety of ways that are known in the art. For example, the first and second agents of the combination therapy may be formulated together or separately.

The individually or separately formulated agents can be packaged together as a kit. Non-limiting examples include, but are not limited to, kits that contain, e.g., two pills, a pill and a powder, a suppository and a liquid in a vial, two topical creams, etc. The kit can include optional components that aid in the administration of the unit dose to subjects, such as vials for reconstituting powder forms, syringes for injection, customized IV delivery systems, inhalers, etc. Additionally, the unit dose kit can contain instructions for preparation and administration of the compositions. The kit may be manufactured as a single use unit dose for one subject, multiple uses for a particular subject (at a constant dose or in which the individual compounds may vary in potency as therapy progresses); or the kit may contain multiple doses suitable for administration to multiple subjects ("bulk packaging"). The kit components may be assembled in cartons, blister packs, bottles, tubes, and the like.

Formulations for oral use include tablets containing the active ingredient(s) in a mixture with non-toxic pharmaceutically acceptable excipients. These excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, optionally substituted hydroxylpropyl methylcellulose, ethylcellulose, polyvinylpyrrolidone, or polyethylene glycol); and lubricating agents, glidants, and antiadhesives (e.g., magnesium stearate, zinc stearate, stearic acid, silicas, hydrogenated vegetable oils, or talc). Other pharmaceutically acceptable excipients can be colorants, flavoring agents, plasticizers, humectants, buffering agents, and the like.

Two or more compounds may be mixed together in a tablet, capsule, or other vehicle, or may be partitioned. In one example, the first compound is contained on the inside of the tablet, and the second compound is on the outside, such that a substantial portion of the second compound is released prior to the release of the first compound.

Formulations for oral use may also be provided as chewable tablets, or as hard gelatin capsules wherein the active ingredient is mixed with an inert solid diluent (e.g., potato starch, lactose, microcrystalline cellulose, calcium carbonate, calcium phosphate or kaolin), or as soft gelatin capsules wherein the active ingredient is mixed with water or an oil medium, for example, peanut oil, liquid paraffin, or olive oil. Powders, granulates, and pellets may be prepared using the ingredients mentioned above under tablets and capsules in a conventional manner using, e.g., a mixer, a fluid bed apparatus or a spray drying equipment.

Dissolution or diffusion controlled release can be achieved by appropriate coating of a tablet, capsule, pellet, or granulate formulation of compounds, or by incorporating the compound into an appropriate matrix. A controlled release coating may include one or more of the coating substances mentioned above and/or, e.g., shellac, beeswax, glycowax, castor wax, carnauba wax, stearyl alcohol, glyceryl monostearate, glyceryl distearate, glycerol palmitostearate, ethylcellulose, acrylic resins, dl-polylactic acid, cellulose acetate butyrate, polyvinyl chloride, polyvinyl acetate, vinyl pyrrolidone, polyethylene, polymethacrylate, methylmethacrylate, 2-optionally substituted hydroxylmethacrylate, methacrylate hydrogels, 1,3 butylene glycol, ethylene glycol methacrylate, and/or polyethylene glycols. In a controlled release matrix formulation, the matrix material may also include, e.g., hydrated methylcellulose, carnauba wax and stearyl alcohol, carbopol 934, silicone, glyceryl tristearate, methyl acrylate-methyl methacrylate, polyvinyl chloride, polyethylene, and/or halogenated fluorocarbon.

The liquid forms in which the compounds and compositions of the present invention can be incorporated for administration orally include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Generally, when administered to a human, the oral dosage of any of the compounds of the combination of the invention will depend on the nature of the compound, and can readily be determined by one skilled in the art. Typically, such dosage is normally about 0.001 mg to 2000 mg per day, desirably about 1 mg to 1000 mg per day, and more desirably about 5 mg to 500 mg per day. Dosages up to 200 mg per day may be necessary.

Administration of each drug in a combination therapy, as described herein, can, independently, be one to four times daily for one day to one year, and may even be for the life of the subject. Chronic, long-term administration may be indicated.

The following examples are intended to illustrate the synthesis of a representative number of compounds and the use of these compounds for the ternary complex formation between CYPA and KRAS G12C. Accordingly, the examples are intended to illustrate but not to limit the invention. Additional compounds not specifically exemplified may be synthesized using conventional methods in combination with the methods described herein. Moreover, other RAS proteins, such as KRAS G13C, NRAS G12C, NRAS G13C, HRAS G12C or HRAS G13C, may be employed for ternary complex formation.

### Methods of Treatment

The references to the methods of treatment by therapy or surgery or in vivo diagnosis methods in this description are to be interpreted as references to compounds, pharmaceutical compositions and medicaments of the present invention for use in those methods.

In an aspect, the invention discloses a method of treating a disease or disorder that is characterized by aberrant RAS activity due to a RAS mutant. In some embodiments, the disease or disorder is a cancer. In some embodiments, the cancer is pancreatic cancer, colorectal cancer, non-small cell lung cancer, small cell lung cancer, acute myeloid leukemia, multiple myeloma, thyroid gland adenocarcinoma, a myelodysplastic syndrome, or squamous cell lung carcinoma. In some embodiments, the aberrant RAS activity is due to a RAS G12C mutation. In some embodiments, the aberrant RAS activity is due to a RAS G13C mutation. In some embodiments, the aberrant RAS activity is due to a KRAS G12C mutation. In some embodiments, the aberrant RAS activity is due to a KRAS G13C mutation. In some embodiments, the aberrant RAS activity is due to an HRAS G12C mutation. In some embodiments, the aberrant RAS activity is due to an HRAS G13C mutation. In some embodiments, the aberrant RAS activity is due to an HRAS G12C mutation. In some embodiments, the aberrant RAS activity is due to an HRAS G13C mutation.

In an aspect, the invention discloses a method of treating a disease or disorder that is characterized by aberrant or unwanted BRAF-RAS binding, the method including contacting the cell with a compound of the present invention, any pharmaceutical composition comprising such a compound. In some embodiments, the disease is characterized by aberrant or unwanted binding between BRAF and a mutant RAS protein. In some embodiments, the disease or disorder is a cancer. In some embodiments, the cancer is pancreatic cancer, colorectal cancer, non-small cell lung cancer, small cell lung cancer, acute myeloid leukemia, multiple myeloma, thyroid gland adenocarcinoma, a myelodysplastic syndrome, or squamous cell lung carcinoma. In some embodiments, the aberrant RAS activity is due to a RAS G12C mutation. In some embodiments, the aberrant RAS activity is due to a RAS G13C mutation. In some embodiments, the aberrant RAS activity is due to a KRAS G12C mutation. In some embodiments, the aberrant RAS activity is due to a KRAS G13C mutation. In some embodiments, the aberrant RAS activity is due to an NRAS G12C mutation. In some embodiments, the aberrant RAS activity is due to an NRAS G13C mutation. In some embodiments, the aberrant RAS activity is due to an HRAS G12C mutation. In some embodiments, the aberrant RAS activity is due to an HRAS G13C mutation.

In an aspect, the invention discloses a method of treating a disease or disorder that is characterized by aberrant or unwanted pERK expression, the method including contacting the cell with an effective amount of a compound of the present invention, any pharmaceutical composition comprising such a compound. In some embodiments, the aberrant or unwanted pERK expression is driven by a mutant RAS protein. In some embodiments, the disease or disorder is a cancer. In some embodiments, the cancer is pancreatic cancer, colorectal cancer, non-small cell lung cancer, small cell lung cancer, acute myeloid leukemia, multiple myeloma, thyroid gland adenocarcinoma, a myelodysplastic syndrome, or squamous cell lung carcinoma. In some embodiments, the mutant RAS that drives pERK expression has a G12C mutation. In some embodiments, the mutant RAS that drives pERK expression has a G13C embodiments, the mutant RAS activity is due to a KRAS G13C mutation. In some embodiments, the mutant RAS activity is due to an NRAS G12C mutation. In some embodiments, the mutant RAS that drives pERK expression is due to an NRAS G13C mutation. In some embodiments, the mutant RAS activity is due to a KRAS G12C mutation. In some embodiments, the mutant RAS that drives pERK expression is due to a KRAS G13C mutation.

In some embodiments, the compounds of the present invention or pharmaceutically acceptable salts thereof, pharmaceutical compositions comprising such compounds or salts, and methods provided herein may be used for the treatment of a wide variety of cancers including tumors such as lung, prostate, breast, brain, skin, cervical carcinomas, testicular carcinomas, etc. More particularly, cancers that may be treated by the compounds or salts thereof, pharmaceutical compositions comprising such compounds or salts, and methods of the invention include, but are not limited to tumor types such as astrocytic, breast, cervical, colorectal, endometrial, esophageal, gastric, head and neck, hepatocellular, laryngeal, lung, oral, ovarian, prostate and thyroid carcinomas and sarcomas. Other cancers include, for example:
Cardiac, for example: sarcoma (angiosarcoma, fibrosarcoma, rhabdomyosarcoma, liposarcoma), myxoma, rhabdomyoma, fibroma, lipoma and teratoma;
Lung, for example: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma;
Gastrointestinal, for example: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma);
Genitourinary tract, for example: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma);
Liver, for example: hepatoma (hepatocellular carcinoma), cholangiocarcinoma, hepatoblastoma, angiosarcoma, hepatocellular adenoma, hemangioma;
Biliary tract, for example: gall bladder carcinoma, ampuliary carcinoma, cholangiocarcinoma;
Bone, for example: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors;
Nervous system, for example: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, neurofibromatosis type 1, meningioma, glioma, sarcoma);
Gynecological, for example: uterus (endometrial carcinoma, uterine carcinoma, uterine corpus endometrial carcinoma), cervix (cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma);
Hematologic, for example: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma);
Skin, for example: malignant melanoma, basal cell carcinoma, squamous cell carcinoma, Kaposi's sarcoma, moles dysplastic nevi, lipoma, angioma, dermatofibroma, keloids, psoriasis; and
Adrenal glands, for example: neuroblastoma.

Also provided is a method of inhibiting a Ras protein in a cell, the method comprising contacting the cell with an effective amount of a compound of the present invention, or a pharmaceutically acceptable salt thereof. A method of inhibiting RAF-Ras binding, the method comprising contacting the cell with an effective amount of a compound of the present invention, or a pharmaceutically acceptable salt thereof, is also provided. The cell may be a cancer cell. The cancer cell may be of any type of cancer described herein.

### Combination Therapies

It will be appreciated that the compounds and pharmaceutical compositions of the present invention can be formulated and employed in combination therapies, that is, the compounds and pharmaceutical compositions can be formulated with or administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder, or they may achieve different effects (e.g., control of any adverse effects).

In some embodiments of the methods described herein, the methods may further include an additional therapeutic agent. For example, the methods of the invention may include a compound of the invention used alone or in combination with one or more additional therapies (e.g., non-drug treatments or therapeutic agents). The dosages of one or more of the additional therapies (e.g., non-drug treatments or therapeutic agents) may be reduced from standard dosages when administered alone. For example, doses may be determined empirically from drug combinations and permutations or may be deduced by isobolographic analysis (e.g., Black et al., Neurology 65:S3-S6 (2005)).

A compound of the present invention may be administered before, after, or concurrently with one or more of such additional therapies. When combined, dosages of a compound of the invention and dosages of the one or more additional therapies (e.g., non-drug treatment or therapeutic agent) provide a therapeutic effect (e.g., synergistic or additive therapeutic effect). A compound of the present invention and an additional therapy, such as an anti-cancer agent, may be administered together, such as in a unitary pharmaceutical composition, or separately and, when administered separately, this may occur simultaneously or sequentially. Such sequential administration may be close or remote in time.

In some embodiments, the additional therapy is the administration of side-effect limiting agents (e.g., agents intended to lessen the occurrence or severity of side effects of treatment. For example, in some embodiments, the compounds of the present invention can also be used in combination with a therapeutic agent that treats nausea. Examples of agents that can be used to treat nausea include: dronabinol, granisetron, metoclopramide, ondansetron, and prochlorperazine, or pharmaceutically acceptable salts thereof.

In some embodiments, the one or more additional therapies includes a non-drug treatment (e.g., surgery or radiation therapy). In some embodiments, the one or more additional therapies includes a therapeutic agent (e.g., a compound or biologic that is an anti-angiogenic agent, signal transduction inhibitor, antiproliferative agent, glycolysis inhibitor, or autophagy inhibitor). In some embodiments, the one or more additional therapies includes a non-drug treatment (e.g., surgery or radiation therapy) and a therapeutic agent (e.g., a compound or biologic that is an anti-angiogenic agent, signal transduction inhibitor, antiproliferative agent, glycolysis inhibitor, or autophagy inhibitor). In other embodiments, the one or more additional therapies includes two therapeutic agents. In still other embodiments, the one or more additional therapies includes three therapeutic agents. In some embodiments, the one or more additional therapies includes four or more therapeutic agents.

### Non-drug therapies

Examples of non-drug treatments include, but are not limited to, radiation therapy, cryotherapy, hyperthermia, surgery (e.g., surgical excision of tumor tissue), and T cell adoptive transfer (ACT) therapy.

In some embodiments, the compounds of the invention may be used as an adjuvant therapy after surgery. In some embodiments, the compounds of the invention may be used as a neo-adjuvant therapy prior to surgery.

Radiation therapy may be used for inhibiting abnormal cell growth or treating a hyperproliferative disorder, such as cancer, in a subject (e.g., mammal (e.g., human)). Techniques for administering radiation therapy are known in the art. Radiation therapy can be administered through one of several methods, or a combination of methods, including, without limitation, external-beam therapy, internal radiation therapy, implant radiation, stereotactic radiosurgery, systemic radiation therapy, radiotherapy and permanent or temporary interstitial brachy therapy. The term "brachy therapy," as used herein, refers to radiation therapy delivered by a spatially confined radioactive material inserted into the body at or near a tumor or other proliferative tissue disease site. The term is intended, without limitation, to include exposure to radioactive isotopes (e.g., At-211, I-131, I-125, Y-90, Re-186, Re-188, Sm-153, Bi-212, P-32, and radioactive isotopes of Lu). Suitable radiation sources for use as a cell conditioner of the present invention include both solids and liquids. By way of non-limiting example, the radiation source can be a radionuclide, such as I-125, I-131, Yb-169, Ir-192 as a solid source, 1-125 as a solid source, or other radionuclides that emit photons, beta particles, gamma radiation, or other therapeutic rays. The radioactive material can also be a fluid made from any solution of radionuclide(s), e.g., a solution of I-125 or 1-131, or a radioactive fluid can be produced using a slurry of a suitable fluid containing small particles of solid radionuclides, such as Au-198, or Y-90. Moreover, the radionuclide(s) can be embodied in a gel or radioactive micro spheres.

In some embodiments, the compounds of the present invention can render abnormal cells more sensitive to treatment with radiation for purposes of killing or inhibiting the growth of such cells. Accordingly, this invention further relates to a method for sensitizing abnormal cells in a mammal to treatment with radiation which comprises administering to the mammal an amount of a compound of the present invention, which amount is effective to sensitize abnormal cells to treatment with radiation. The amount of the compound in this method can be determined according to the means for ascertaining effective amounts of such compounds described herein. In some embodiments, the compounds of the present invention may be used as an adjuvant therapy after radiation therapy or as a neo-adjuvant therapy prior to radiation therapy.

In some embodiments, the non-drug treatment is a T cell adoptive transfer (ACT) therapy. In some embodiments, the T cell is an activated T cell. The T cell may be modified to express a chimeric antigen receptor (CAR). CAR modified T (CAR-T) cells can be generated by any method known in the art. For example, the CAR-T cells can be generated by introducing a suitable expression vector encoding the CAR to a T cell. Prior to expansion and genetic modification of the T cells, a source of T cells is obtained from a subject. T cells can be obtained from a number of sources, including peripheral blood mononuclear cells, bone marrow, lymph node tissue, cord blood, thymus tissue, tissue from a site of infection, ascites, pleural effusion, spleen tissue, and tumors. In certain embodiments of the present invention, any number of T cell lines available in the art may be used. In some embodiments, the T cell is an autologous T cell. Whether prior to or after genetic modification of the T cells to express a desirable protein (e.g., a CAR), the T cells can be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 7,572,631 ; 5,883,223; 6,905,874; 6,797,514; and 6,867,041.

### Therapeutic agents

A therapeutic agent may be a compound used in the treatment of cancer or symptoms associated therewith.

For example, a therapeutic agent may be a steroid. Accordingly, in some embodiments, the one or more additional therapies includes a steroid. Suitable steroids may include, but are not limited to, 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, desoximetasone, dexamethasone, diflorasone, diflucortolone, difuprednate, enoxolone, fluazacort, fiucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, hydrocortisone, loteprednol etabonate, mazipredone, medrysone, meprednisone, methylprednisolone, mometasone furoate, paramethasone, prednicarbate, prednisolone, prednisolone 25-diethylaminoacetate, prednisolone sodium phosphate, prednisone, prednival, prednylidene, rimexolone, tixocortol, triamcinolone, triamcinolone acetonide, triamcinolone benetonide, triamcinolone hexacetonide, and salts or derivatives thereof.

Further examples of therapeutic agents that may be used in combination therapy with a compound of the present invention include compounds described in the following patents: U.S. Patent Nos. 6,258,812, 6,630,500, 6,515,004, 6,713,485, 5,521,184, 5,770,599, 5,747,498, 5,990,141, 6,235,764, and 8,623,885, and International Patent Applications WO01/37820, WO01/32651, WO02/68406, WO02/66470, WO02/55501, WO04/05279, WO04/07481, WO04/07458, WO04/09784, WO02/59110, WO99/45009, WO00/59509, WO99/61422, WO00/12089, and WO00/02871.

A therapeutic agent may be a biologic (e.g., cytokine (e.g., interferon or an interleukin such as IL-2)) used in treatment of cancer or symptoms associated therewith. In some embodiments, the biologic is an immunoglobulin-based biologic, e.g., a monoclonal antibody (e.g., a humanized antibody, a fully human antibody, an Fc fusion protein, or a functional fragment thereof) that agonizes a target to stimulate an anti-cancer response or antagonizes an antigen important for cancer. Also included are antibody-drug conjugates.

A therapeutic agent may be a T-cell checkpoint inhibitor. In one embodiment, the checkpoint inhibitor is an inhibitory antibody (e.g., a monospecific antibody such as a monoclonal antibody). The antibody may be, e.g., humanized or fully human. In some embodiments, the checkpoint inhibitor is a fusion protein, e.g., an Fc-receptor fusion protein. In some embodiments, the checkpoint inhibitor is an agent, such as an antibody, that interacts with a checkpoint protein. In some embodiments, the checkpoint inhibitor is an agent, such as an antibody, that interacts with the ligand of a checkpoint protein. In some embodiments, the checkpoint inhibitor is an inhibitor (e.g., an inhibitory antibody or small molecule inhibitor) of CTLA-4 (e.g., an anti-CTLA-4 antibody or fusion a protein). In some embodiments, the checkpoint inhibitor is an inhibitor or antagonist (e.g., an inhibitory antibody or small molecule inhibitor) of PD-1. In some embodiments, the checkpoint inhibitor is an inhibitor or antagonist (e.g., an inhibitory antibody or small molecule inhibitor) of PDL-1. In some embodiments, the checkpoint inhibitor is an inhibitor or antagonist (e.g., an inhibitory antibody or Fc fusion or small molecule inhibitor) of PDL-2 (e.g., a PDL-2/Ig fusion protein). In some embodiments, the checkpoint inhibitor is an inhibitor or antagonist (e.g., an inhibitory antibody or small molecule inhibitor) of B7-H3, B7-H4, BTLA, HVEM, TIM3, GAL9, LAG3, VISTA, KIR, 2B4, CD160, CGEN-15049, CHK 1, CHK2, A2aR, B-7 family ligands, or a combination thereof. In some embodiments, the checkpoint inhibitor is pembrolizumab, nivolumab, PDR001 (NVS), REGN2810 (Sanofi/Regeneron), a PD-L1 antibody such as, e.g., avelumab, durvalumab, atezolizumab, pidilizumab, JNJ-63723283 (JNJ), BGB-A317 (BeiGene & Celgene) or a checkpoint inhibitor disclosed in Preusser, M. et al. (2015) Nat. Rev. Neurol., including, without limitation, ipilimumab, tremelimumab, nivolumab, pembrolizumab, AMP224, AMP514/ MEDI0680, BMS936559, MEDI4736, MPDL3280A, MSB0010718C, BMS986016, IMP321, lirilumab, IPH2101, 1-7F9, and KW-6002.

A therapeutic agent may be an anti-TIGIT antibody, such as MBSA43, BMS-986207, MK-7684, COM902, AB154, MTIG7192A or OMP-313M32 (etigilimab).

A therapeutic agent may be an agent that treats cancer or symptoms associated therewith (e.g., a cytotoxic agent, non-peptide small molecules, or other compound useful in the treatment of cancer or symptoms associated therewith, collectively, an "anti-cancer agent"). Anti-cancer agents can be, e.g., chemotherapeutics or targeted therapy agents.

Anti-cancer agents include mitotic inhibitors, intercalating antibiotics, growth factor inhibitors, cell cycle inhibitors, enzymes, topoisomerase inhibitors, biological response modifiers, alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Further anti-cancer agents include leucovorin (LV), irenotecan, oxaliplatin, capecitabine, paclitaxel, and doxetaxel. In some embodiments, the one or more additional therapies includes two or more anti-cancer agents. The two or more anti-cancer agents can be used in a cocktail to be administered in combination or administered separately. Suitable dosing regimens of combination anti-cancer agents are known in the art and described in, for example, Saltz et al., Proc. Am. Soc. Clin. Oncol. 18:233a (1999), and Douillard et al., Lancet 355(9209):1041-1047 (2000).

Other non-limiting examples of anti-cancer agents include Gleevec^{®} (Imatinib Mesylate); Kyprolis^{®} (carfilzomib); Velcade^{®} (bortezomib); Casodex (bicalutamide); Iressa^{®} (gefitinib); alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; sarcodictyin A; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimustine; antibiotics such as the enediyne antibiotics (e.g., calicheamicin, such as calicheamicin gammall and calicheamicin omegall (see, e.g., Agnew, Chem. Intl. Ed Engl. 33:183-186 (1994)); dynemicin such as dynemicin A; bisphosphonates such as clodronate; an esperamicin; neocarzinostatin chromophore and related chromoprotein enediyne antiobiotic chromophores, aclacinomysins, actinomycin, authramycin, azaserine, bleomycins, cactinomycin, calicheamicin, carabicin, caminomycin, carminomycin, carzinophilin, chromomycins, dactinomycin, daunorubicin, detorubicin, 6-diazo- 5-oxo-L-norleucine, adriamycin (doxorubicin), morpholino-doxorubicin, cyanomorpholino-doxorubicin, 2-pyrrolino-doxorubicin, deoxydoxorubicin, epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5- FU); folic acid analogues such as denopterin, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenishers such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone such as epothilone B; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes such as T- 2 toxin, verracurin A, roridin A and anguidine; urethane; vindesine; dacarbazine; mannomustine; mitobronitol; mitolactol; pipobroman; gacytosine; arabinoside ("Ara-C"); cyclophosphamide; thiotepa; taxoids, e.g., Taxol^{®} (paclitaxel), Abraxane^{®} (cremophor-free, albumin-engineered nanoparticle formulation of paclitaxel), and Taxotere^{®} (doxetaxel); chloranbucil; tamoxifen (Nolvadex^{™}); raloxifene; aromatase inhibiting 4(5)-imidazoles; 4-hydroxytamoxifen; trioxifene; keoxifene; LY 117018; onapristone; toremifene (Fareston^{®}); flutamide, nilutamide, bicalutamide, leuprolide, goserelin; chlorambucil; Gemzar^{®} gemcitabine; 6-thioguanine; mercaptopurine; platinum coordination complexes such as cisplatin, oxaliplatin and carboplatin; vinblastine; platinum; etoposide (VP-16); ifosfamide; mitoxantrone; vincristine; Navelbine^{®} (vinorelbine); novantrone; teniposide; edatrexate; daunomycin; aminopterin; ibandronate; irinotecan (e.g., CPT-11); topoisomerase inhibitor RFS 2000; difluoromethylornithine (DMFO); retinoids such as retinoic acid; esperamicins; capecitabine (e.g., Xeloda^{®}); and pharmaceutically acceptable salts of any of the above.

Additional non-limiting examples of anti-cancer agents include trastuzumab (Herceptin^{®}), bevacizumab (Avastin^{®}), cetuximab (Erbitux^{®}), rituximab (Rituxan^{®}), Taxol^{®}, Arimidex^{®}, ABVD, avicine, abagovomab, acridine carboxamide, adecatumumab, 17-N-allylamino-17-demethoxygeldanamycin, alpharadin, alvocidib, 3-aminopyridine-2-carboxaldehyde thiosemicarbazone, amonafide, anthracenedione, anti-CD22 immunotoxins, antineoplastics (e.g., cell-cycle nonspecific antineoplastic agents, and other antineoplastics described herein), antitumorigenic herbs, apaziquone, atiprimod, azathioprine, belotecan, bendamustine, BIBW 2992, biricodar, brostallicin, bryostatin, buthionine sulfoximine, CBV (chemotherapy), calyculin, dichloroacetic acid, discodermolide, elsamitrucin, enocitabine, eribulin, exatecan, exisulind, ferruginol, forodesine, fosfestrol, ICE chemotherapy regimen, IT-101, imexon, imiquimod, indolocarbazole, irofulven, laniquidar, larotaxel, lenalidomide, lucanthone, lurtotecan, mafosfamide, mitozolomide, nafoxidine, nedaplatin, olaparib, ortataxel, PAC-1, pawpaw, pixantrone, proteasome inhibitors, rebeccamycin, resiquimod, rubitecan, SN-38, salinosporamide A, sapacitabine, Stanford V, swainsonine, talaporfin, tariquidar, tegafur-uracil, temodar, tesetaxel, triplatin tetranitrate, tris(2-chloroethyl)amine, troxacitabine, uramustine, vadimezan, vinflunine, ZD6126, and zosuquidar.

Further non-limiting examples of anti-cancer agents include natural products such as vinca alkaloids (e.g., vinblastine, vincristine, and vinorelbine), epidipodophyllotoxins (e.g., etoposide and teniposide), antibiotics (e.g., dactinomycin (actinomycin D), daunorubicin, and idarubicin), anthracyclines, mitoxantrone, bleomycins, plicamycin (mithramycin), mitomycin, enzymes (e.g., L-asparaginase which systemically metabolizes L-asparagine and deprives cells which do not have the capacity to synthesize their own asparagine), antiplatelet agents, antiproliferative/antimitotic alkylating agents such as nitrogen mustards (e.g., mechlorethamine, cyclophosphamide and analogs, melphalan, and chlorambucil), ethylenimines and methylmelamines (e.g., hexaamethylmelaamine and thiotepa), CDK inhibitors (e.g., a CDK 4/6 inhibitor such as palbociclib; seliciclib, UCN-01, P1446A-05, PD-0332991, dinaciclib, P27-00, AT-7519, RGB286638, and SCH727965), alkyl sulfonates (e.g., busulfan), nitrosoureas (e.g., carmustine (BCNU) and analogs, and streptozocin), trazenes-dacarbazinine (DTIC), antiproliferative/antimitotic antimetabolites such as folic acid analogs, pyrimidine analogs (e.g., fluorouracil, floxuridine, and cytarabine), purine analogs and related inhibitors (e.g., mercaptopurine, thioguanine, pentostatin, and 2-chlorodeoxyadenosine), aromatase inhibitors (e.g., anastrozole, exemestane, and letrozole), and platinum coordination complexes (e.g., cisplatin and carboplatin), procarbazine, hydroxyurea, mitotane, aminoglutethimide, histone deacetylase (HDAC) inhibitors (e.g., trichostatin, sodium butyrate, apicidan, suberoyl anilide hydroamic acid, vorinostat, LBH 589, romidepsin, ACY-1215, and panobinostat), mTOR inhibitors (e.g., vistusertib, temsirolimus, everolimus, ridaforolimus, and sirolimus), KSP(Eg5) inhibitors (e.g., Array 520), DNA binding agents (e.g., Zalypsis^{®}), PI3K inhibitors such as PI3K delta inhibitor (e.g., GS-1101 and TGR-1202), PI3K delta and gamma inhibitor (e.g., CAL-130), copanlisib, alpelisib and idelalisib; multi-kinase inhibitor (e.g., TG02 and sorafenib), hormones (e.g., estrogen) and hormone agonists such as leutinizing hormone releasing hormone (LHRH) agonists (e.g., goserelin, leuprolide and triptorelin), BAFF-neutralizing antibody (e.g., LY2127399), IKK inhibitors, p38MAPK inhibitors, anti-IL-6 (e.g., CNT0328), telomerase inhibitors (e.g., GRN 163L), aurora kinase inhibitors (e.g., MLN8237), cell surface monoclonal antibodies (e.g., anti-CD38 (HUMAX-CD38), anti-CSI (e.g., elotuzumab), HSP90 inhibitors (e.g., 17 AAG and KOS 953), P13K / Akt inhibitors (e.g., perifosine), Akt inhibitors (e.g., GSK-2141795), PKC inhibitors (e.g., enzastaurin), FTIs (e.g., Zarnestra^{™}), anti-CD138 (e.g., BT062), Torcl/2 specific kinase inhibitors (e.g., INK128), ER/UPR targeting agents (e.g., MKC-3946), cFMS inhibitors (e.g., ARRY-382), JAK1/2 inhibitors (e.g., CYT387), PARP inhibitors (e.g., olaparib and veliparib (ABT-888)), and BCL-2 antagonists.

In some embodiments, an anti-cancer agent is selected from mechlorethamine, camptothecin, ifosfamide, tamoxifen, raloxifene, gemcitabine, Navelbine^{®}, sorafenib, or any analog or derivative variant of the foregoing.

In some embodiments, the anti-cancer agent is a HER2 inhibitor. Non-limiting examples of HER2 inhibitors include monoclonal antibodies such as trastuzumab (Herceptin^{®}) and pertuzumab (Perjeta^{®}); small molecule tyrosine kinase inhibitors such as afatinib, gefitinib (Iressa^{®}), erlotinib (Tarceva^{®}), pilitinib, CP-654577, CP-724714, canertinib (CI 1033), HKI-272, lapatinib (GW-572016; Tykerb^{®}), PKI-166, AEE788, BMS-599626, HKI-357, BIBW 2992, ARRY-334543, JNJ-26483327, and JNJ-26483327.

In some embodiments, an anti-cancer agent is an ALK inhibitor. Non-limiting examples of ALK inhibitors include ceritinib, TAE-684 (NVP-TAE694), PF02341066 (crizotinib or 1066), alectinib; brigatinib; entrectinib; ensartinib (X-396); lorlatinib; ASP3026; CEP-37440; 4SC-203; TL-398; PLB1003; TSR-011; CT-707; TPX-0005, and AP26113. Additional examples of ALK kinase inhibitors are described in examples 3-39 of WO05016894.

In some embodiments, an anti-cancer agent is an inhibitor of a member downstream of a Receptor Tyrosine Kinase (RTK)/Growth Factor Receptor (e.g., a SHP2 inhibitor (e.g., SHP099, TNO155, RMC-4550, RMC-4630, JAB-3068), a SOS1 inhibitor (e.g., BI-1701963, BI-3406), a Raf inhibitor, a MEK inhibitor, an ERK inhibitor, a PI3K inhibitor, a PTEN inhibitor, an AKT inhibitor, or an mTOR inhibitor (e.g., mTORC1 inhibitor or mTORC2 inhibitor). In some embodiments, the anti-cancer agent is JAB-3312. In some embodiments, an anti-cancer agent is an additional Ras inhibitor (e.g., AMG 510, MRTX1257, MRTX849, JNJ4699157, LY3499446, ARS-3248, or ARS-1620), or a Ras vaccine, or another therapeutic modality designed to directly or indirectly decrease the oncogenic activity of Ras.

In some embodiments, a therapeutic agent that may be combined with a compound of the present invention is an inhibitor of the MAP kinase (MAPK) pathway (or "MAPK inhibitor"). MAPK inhibitors include, but are not limited to, one or more MAPK inhibitor described in Cancers (Basel) 2015 Sep; 7(3): 1758-1784. For example, the MAPK inhibitor may be selected from one or more of trametinib, binimetinib, selumetinib, cobimetinib, LErafAON (NeoPharm), ISIS 5132; vemurafenib, pimasertib, TAK733, RO4987655 (CH4987655); CI-1040; PD-0325901; CH5126766; MAP855; AZD6244; refametinib (RDEA 119/BAY 86-9766); GDC-0973/XL581; AZD8330 (ARRY-424704/ARRY-704); RO5126766 (Roche, described in PLoS One. 2014 Nov 25;9(11)); and GSK1120212 (or JTP-74057, described in Clin Cancer Res. 2011 Mar 1;17(5):989-1000). The MAPK inhibitor may be PLX8394, LXH254, GDC-5573, or LY3009120.

In some embodiments, an anti-cancer agent is a disrupter or inhibitor of the RAS-RAF-ERK or PI3K-AKT-TOR or PI3K-AKT signaling pathways. The PI3K/AKT inhibitor may include, but is not limited to, one or more PI3K/AKT inhibitor described in Cancers (Basel) 2015 Sep; 7(3): 1758-1784. For example, the PI3K/AKT inhibitor may be selected from one or more of NVP-BEZ235; BGT226; XL765/SAR245409; SF1126; GDC-0980; PI-103; PF-04691502; PKI-587; GSK2126458.

In some embodiments, an anti-cancer agent is a PD-1 or PD-L1 antagonist.

In some embodiments, additional therapeutic agents include ALK inhibitors, HER family inhibitors, EGFR inhibitors, IGF-1R inhibitors, MEK inhibitors, PI3K inhibitors, AKT inhibitors, TOR inhibitors, MCL-1 inhibitors, BCL-2 inhibitors, SHP2 inhibitors, proteasome inhibitors, and immune therapies. In some embodiments, a therapeutic agent may be a pan-RTK inhibitor, such as afatinib.

IGF-1R inhibitors include linsitinib, or a pharmaceutically acceptable salt thereof.

EGFR inhibitors include, but are not limited to, small molecule antagonists, antibody inhibitors, or specific antisense nucleotide or siRNA. Useful antibody inhibitors of EGFR include cetuximab (Erbitux^{®}), panitumumab (Vectibix^{®}), zalutumumab, nimotuzumab, and matuzumab. Further antibody-based EGFR inhibitors include any anti-EGFR antibody or antibody fragment that can partially or completely block EGFR activation by its natural ligand. Non-limiting examples of antibody-based EGFR inhibitors include those described in Modjtahedi et al., Br. J. Cancer 1993, 67:247-253; Teramoto et al., Cancer 1996, 77:639-645; Goldstein et al., Clin. Cancer Res. 1995, 1:1311-1318; Huang et al., 1999, Cancer Res. 15:59(8):1935-40; and Yang et al., Cancer Res.1999, 59:1236-1243. The EGFR inhibitor can be monoclonal antibody Mab E7.6.3 (Yang, 1999 supra), or Mab C225 (ATCC Accession No. HB-8508), or an antibody or antibody fragment having the binding specificity thereof.

Small molecule antagonists of EGFR include gefitinib (Iressa^{®}), erlotinib (Tarceva^{®}), and lapatinib (TykerB^{®}). See, e.g., Yan et al., Pharmacogenetics and Pharmacogenomics In Oncology Therapeutic Antibody Development, BioTechniques 2005, 39(4):565-8; and Paez et al., EGFR Mutations In Lung Cancer Correlation With Clinical Response To Gefitinib Therapy, Science 2004, 304(5676):1497-500. Further non-limiting examples of small molecule EGFR inhibitors include any of the EGFR inhibitors described in the following patent publications, and all pharmaceutically acceptable salts of such EGFR inhibitors: EP 0520722; EP 0566226; WO96/33980; U.S. Pat. No. 5,747,498; WO96/30347; EP 0787772; WO97/30034; WO97/30044; WO97/38994; WO97/49688; EP 837063; WO98/02434; WO97/38983; WO95/19774; WO95/19970; WO97/13771; WO98/02437; WO98/02438; WO97/32881; DE 19629652; WO98/33798; WO97/32880; WO97/32880; EP 682027; WO97/02266; WO97/27199; WO98/07726; WO97/34895; WO96/31510; WO98/14449; WO98/14450; WO98/14451 ; WO95/09847; WO97/19065; WO98/17662; U.S. Pat. No. 5,789,427; U.S. Pat. No. 5,650,415; U.S. Pat. No. 5,656,643; WO99/35146; WO99/35132; WO99/07701; and WO92/20642. Additional non-limiting examples of small molecule EGFR inhibitors include any of the EGFR inhibitors described in Traxler et al., Exp. Opin. Ther. Patents 1998, 8(12):1599-1625. In some embodiments, the therapeutic agent is lapatinib, neratinib, or afatinib.

MEK inhibitors include, but are not limited to, pimasertib, selumetinib, cobimetinib (Cotellic^{®}), trametinib (Mekinist^{®}), and binimetinib (Mektovi^{®}). In some embodiments, a MEK inhibitor targets a MEK mutation that is a Class I MEK1 mutation selected from D67N; P124L; P124S; and L177V. In some embodiments, the MEK mutation is a Class II MEK1 mutation selected from ΔE51-Q58; ΔF53-Q58; E203K; L177M; C121 S; F53L; K57E; Q56P; and K57N.

PI3K inhibitors include, but are not limited to, wortmannin; 17-hydroxywortmannin analogs described in WO06/044453; 4-[2-(1H-Indazol-4-yl)-6-[[4-(methylsulfonyl)piperazin-1-yl]methyl]thieno[3,2-d]pyrimidin-4-yl]morpholine (also known as pictilisib or GDC-0941 and described in WO09/036082 and WO09/055730); 2-methyl-2-[4-[3-methyl-2-oxo-8-(quinolin-3-yl)-2,3-dihydroimidazo[4,5-c]quinolin-1-yl]phenyl]propionitrile (also known as BEZ 235 or NVP-BEZ 235, and described in WO06/122806); (S)-I-(4-((2-(2-aminopyrimidin-5-yl)-7-methyl-4-morpholinothieno[3,2-d]pyrimidin-6-yl)methyl)piperazin-1-yl)-2-hydroxypropan-1-one (described in WO08/070740); LY294002 (2-(4-morpholinyl)-8-phenyl-4H-I-benzopyran-4-one (available from Axon Medchem); PI 103 hydrochloride (3-[4-(4-morpholinylpyrido-[3',2':4,5]furo[3,2-d]pyrimidin-2-yl] phenol hydrochloride (available from Axon Medchem); PIK 75 (2-methyl-5-nitro-2-[(6-bromoimidazo[1,2-a]pyridin-3-yl)methylene]-1-methylhydrazide-benzenesulfonic acid, monohydrochloride) (available from Axon Medchem); PIK 90 (N-(7,8-dimethoxy-2,3-dihydro-imidazo[1,2-c]quinazolin-5-yl)-nicotinamide (available from Axon Medchem); AS-252424 (5-[I-[5-(4-fluoro-2-hydroxyphenyl)-furan-2-yl]-meth-(Z)-ylidene]-thiazolidine-2,4-dione (available from Axon Medchem); TGX-221 (7-methyl-2-(4-morpholinyl)-9-[1-(phenylamino)ethyl]-4H-pyrido-[1,2-a]pyrirnidin-4-one (available from Axon Medchem); XL-765; and XL-147. Other PI3K inhibitors include demethoxyviridin, perifosine, CAL101, PX-866, BEZ235, SF1126, INK1117, IPI-145, BKM120, XL147, XL765, Palomid 529, GSK1059615, ZSTK474, PWT33597, IC87114, TGI 00-115, CAL263, PI-103, GNE-477, CUDC-907, and AEZS-136.

AKT inhibitors include, but are not limited to, Akt-1-1 (inhibits Aktl) (Barnett et al., Biochem. J. 2005, 385(Pt. 2): 399-408); Akt-1-1,2 (inhibits Akl and 2) (Barnett et al., Biochem. J. 2005, 385(Pt. 2): 399-408); API-59CJ-Ome (e.g., Jin et al., Br. J. Cancer 2004, 91:1808-12); 1-H-imidazo[4,5-c]pyridinyl compounds (e.g., WO 05/011700); indole-3-carbinol and derivatives thereof (e.g., U.S. Pat. No. 6,656,963; Sarkar and Li J Nutr. 2004, 134(12 Suppl):3493S-3498S); perifosine (e.g., interferes with Akt membrane localization; Dasmahapatra et al. Clin. Cancer Res. 2004, 10(15):5242-52); phosphatidylinositol ether lipid analogues (e.g., Gills and Dennis Expert. Opin. Investig. Drugs 2004, 13:787-97); and triciribine (TCN or API-2 or NCI identifier: NSC 154020; Yang et al., Cancer Res. 2004, 64:4394-9).

mTOR inhibitors include, but are not limited to, ATP-competitive mTORC1/mTORC2 inhibitors, e.g., PI-103, PP242, PP30; Torin 1; FKBP12 enhancers; 4H-1-benzopyran-4-one derivatives; and rapamycin (also known as sirolimus) and derivatives thereof, including: temsirolimus (Torisel^{®}); everolimus (Afinitor^{®}; WO94/09010); ridaforolimus (also known as deforolimus or AP23573); rapalogs, e.g., as disclosed in WO98/02441 and WO01/14387, e.g. AP23464 and AP23841; 40-(2-hydroxyethyl)rapamycin; 40-[3-hydroxy(hydroxymethyl)methylpropanoate]-rapamycin (also known as CC1779); 40-epi-(tetrazolyt)-rapamycin (also called ABT578); 32-deoxorapamycin; 16-pentynyloxy-32(S)-dihydrorapanycin; derivatives disclosed in WO05/005434; derivatives disclosed in U.S. Patent Nos. 5,258,389, 5,118,677, 5,118,678, 5,100,883, 5,151,413, 5,120,842, and 5,256,790, and in WO94/090101, WO92/05179, WO93/111130, WO94/02136, WO94/02485, WO95/14023, WO94/02136, WO95/16691, WO96/41807, WO96/41807, and WO2018204416; and phosphorus-containing rapamycin derivatives (e.g., WO05/016252). In some embodiments, the mTOR inhibitor is a bisteric inhibitor, such as RMC-5552.

BRAF inhibitors that may be used in combination with compounds of the invention include, for example, vemurafenib, dabrafenib, and encorafenib. A BRAF may comprise a Class 3 BRAF mutation. In some embodiments, the Class 3 BRAF mutation is selected from one or more of the following amino acid substitutions in human BRAF: D287H; P367R; V459L; G466V; G466E; G466A; S467L; G469E; N581S; N581I; D594N; D594G; D594A; D594H; F595L; G596D; G596R and A762E.

MCL-1 inhibitors include, but are not limited to, AMG-176, MIK665, and S63845. The myeloid cell leukemia-1 (MCL-1) protein is one of the key anti-apoptotic members of the B-cell lymphoma-2 (BCL-2) protein family. Over-expression of MCL-1 has been closely related to tumor progression as well as to resistance, not only to traditional chemotherapies but also to targeted therapeutics including BCL-2 inhibitors such as ABT-263.

In some embodiments, the additional therapeutic agent is selected from the group consisting of a HER2 family inhibitor, a SHP2 inhibitor, CDK4/6 inhibitor, an mTOR inhibitor, a SOS1 inhibitor, or a PD-L1 inhibitor. See, e.g., Hallin et al., Cancer Discovery, DOI: 10.1158/2159-8290 (October 28, 2019) and Canon et al., Nature, 575:217(2019).

Proteasome inhibitors include, but are not limited to, carfilzomib (Kyprolis^{®}), bortezomib (Velcade^{®}), and oprozomib.

Immune therapies include, but are not limited to, monoclonal antibodies, immunomodulatory imides (IMiDs), GITR agonists, genetically engineered T-cells (e.g., CAR-T cells), bispecific antibodies (e.g., BiTEs), and anti-PD-1, anti-PDL-1, anti-CTLA4, anti-LAGI, and anti-OX40 agents).

Immunomodulatory agents (IMiDs) are a class of immunomodulatory drugs (drugs that adjust immune responses) containing an imide group. The IMiD class includes thalidomide and its analogues (lenalidomide, pomalidomide, and apremilast).

Exemplary anti-PD-1 antibodies and methods for their use are described by Goldberg et al., Blood 2007, 110(1):186-192; Thompson et al., Clin. Cancer Res. 2007, 13(6):1757-1761; and WO06/121168 A1), as well as described elsewhere herein.

GITR agonists include, but are not limited to, GITR fusion proteins and anti-GITR antibodies (e.g., bivalent anti-GITR antibodies), such as, a GITR fusion protein described in U.S. Pat. No. 6,111,090, , U.S. Pat. No. 8,586,023, WO2010/003118 and WO2011/090754; or an anti-GITR antibody described, e.g., in U.S. Pat. No. 7,025,962, EP 1947183, U.S. Pat. No. 7,812,135, U.S. Pat. No. 8,388,967, U.S. Pat. No. 8,591,886, U.S. Pat. No. 7,618,632, EP 1866339, and WO2011/028683, WO2013/039954, WO05/007190, WO07/133822, WO05/055808, WO99/40196, WO01/03720, WO99/20758, WO06/083289, WO05/115451, and WO2011/051726.

Another example of a therapeutic agent that may be used in combination with the compounds of the invention is an anti-angiogenic agent. Anti-angiogenic agents are inclusive of, but not limited to, in vitro synthetically prepared chemical compositions, antibodies, antigen binding regions, radionuclides, and combinations and conjugates thereof. An anti-angiogenic agent can be an agonist, antagonist, allosteric modulator, toxin or, more generally, may act to inhibit or stimulate its target (e.g., receptor or enzyme activation or inhibition), and thereby promote cell death or arrest cell growth. In some embodiments, the one or more additional therapies include an anti-angiogenic agent.

Anti-angiogenic agents can be MMP-2 (matrix-metalloproteinase 2) inhibitors, MMP-9 (matrix-metalloprotienase 9) inhibitors, and COX-II (cyclooxygenase 11) inhibitors. Non-limiting examples of anti-angiogenic agents include rapamycin, temsirolimus (CCI-779), everolimus (RAD001), sorafenib, sunitinib, and bevacizumab. Examples of useful COX-II inhibitors include alecoxib, valdecoxib, and rofecoxib. Examples of useful matrix metalloproteinase inhibitors are described in WO96/33172, WO96/27583, WO98/07697, WO98/03516, WO98/34918, WO98/34915, WO98/33768, WO98/30566, WO90/05719, WO99/52910, WO99/52889, WO99/29667, WO99007675, EP0606046, EP0780386, EP1786785, EP1181017, EP0818442, EP1004578, and US20090012085, and U.S. Patent Nos. 5,863,949 and 5,861,510. Preferred MMP-2 and MMP-9 inhibitors are those that have little or no activity inhibiting MMP-1. More preferred, are those that selectively inhibit MMP-2 or AMP-9 relative to the other matrix-metalloproteinases (i.e., MAP-1, MMP-3, MMP-4, MMP-5, MMP-6, MMP- 7, MMP- 8, MMP-10, MMP-11, MMP-12, and MMP-13). Some specific examples of MMP inhibitors are AG-3340, RO 32-3555, and RS 13-0830.

Further exemplary anti-angiogenic agents include KDR (kinase domain receptor) inhibitory agents (e.g., antibodies and antigen binding regions that specifically bind to the kinase domain receptor), anti-VEGF agents (e.g., antibodies or antigen binding regions that specifically bind VEGF (such as bevacizumab), or soluble VEGF receptors or a ligand binding region thereof) such as VEGF-TRAP^{™}, and anti-VEGF receptor agents (e.g., antibodies or antigen binding regions that specifically bind thereto), EGFR inhibitory agents (e.g., antibodies or antigen binding regions that specifically bind thereto) such as Vectibix^{®} (panitumumab), erlotinib (Tarceva^{®}), anti-Angl and anti-Ang2 agents (e.g., antibodies or antigen binding regions specifically binding thereto or to their receptors, e.g., Tie2/Tek), and anti-Tie2 kinase inhibitory agents (e.g., antibodies or antigen binding regions that specifically bind thereto). Other anti-angiogenic agents include Campath, IL-8, B-FGF, Tek antagonists (US2003/0162712; US6,413,932), anti-TWEAK agents (e.g., specifically binding antibodies or antigen binding regions, or soluble TWEAK receptor antagonists; see US6,727,225), ADAM distintegrin domain to antagonize the binding of integrin to its ligands (US 2002/0042368), specifically binding anti-eph receptor and/or anti-ephrin antibodies or antigen binding regions (U.S. Patent Nos. 5,981,245; 5,728,813; 5,969,110; 6,596,852; 6,232,447; 6,057,124 and patent family members thereof), and anti-PDGF-BB antagonists (e.g., specifically binding antibodies or antigen binding regions) as well as antibodies or antigen binding regions specifically binding to PDGF-BB ligands, and PDGFR kinase inhibitory agents (e.g., antibodies or antigen binding regions that specifically bind thereto). Additional anti-angiogenic agents include: SD-7784 (Pfizer, USA); cilengitide (Merck KGaA, Germany, EPO 0770622); pegaptanib octasodium, (Gilead Sciences, USA); Alphastatin, (BioActa, UK); M-PGA, (Celgene, USA, US 5712291); ilomastat, (Arriva, USA, US5892112); emaxanib, (Pfizer, USA, US 5792783); vatalanib, (Novartis, Switzerland); 2-methoxyestradiol (EntreMed, USA); TLC ELL-12 (Elan, Ireland); anecortave acetate (Alcon, USA); alpha-D148 Mab (Amgen, USA); CEP-7055 (Cephalon, USA); anti-Vn Mab (Crucell, Netherlands), DACantiangiogenic (ConjuChem, Canada); Angiocidin (InKine Pharmaceutical, USA); KM-2550 (Kyowa Hakko, Japan); SU-0879 (Pfizer, USA); CGP-79787 (Novartis, Switzerland, EP 0970070); ARGENT technology (Ariad, USA); YIGSR-Stealth (Johnson & Johnson, USA); fibrinogen-E fragment (BioActa, UK); angiogenic inhibitor (Trigen, UK); TBC-1635 (Encysive Pharmaceuticals, USA); SC-236 (Pfizer, USA); ABT-567 (Abbott, USA); Metastatin (EntreMed, USA); maspin (Sosei, Japan); 2-methoxyestradiol (Oncology Sciences Corporation, USA); ER-68203-00 (IV AX, USA); BeneFin (Lane Labs, USA); Tz-93 (Tsumura, Japan); TAN-1120 (Takeda, Japan); FR-111142 (Fujisawa, Japan, JP 02233610); platelet factor 4 (RepliGen, USA, EP 407122); vascular endothelial growth factor antagonist (Borean, Denmark); bevacizumab (pINN) (Genentech, USA); angiogenic inhibitors (SUGEN, USA); XL 784 (Exelixis, USA); XL 647 (Exelixis, USA); MAb, alpha5beta3 integrin, second generation (Applied Molecular Evolution, USA and Medlmmune, USA); enzastaurin hydrochloride (Lilly, USA); CEP 7055 (Cephalon, USA and Sanofi-Synthelabo, France); BC 1 (Genoa Institute of Cancer Research, Italy); rBPI 21 and BPI-derived antiangiogenic (XOMA, USA); PI 88 (Progen, Australia); cilengitide (Merck KGaA, German; Munich Technical University, Germany, Scripps Clinic and Research Foundation, USA); AVE 8062 (Ajinomoto, Japan); AS 1404 (Cancer Research Laboratory, New Zealand); SG 292, (Telios, USA); Endostatin (Boston Childrens Hospital, USA); ATN 161 (Attenuon, USA); 2-methoxyestradiol (Boston Childrens Hospital, USA); ZD 6474, (AstraZeneca, UK); ZD 6126, (Angiogene Pharmaceuticals, UK); PPI 2458, (Praecis, USA); AZD 9935, (AstraZeneca, UK); AZD 2171, (AstraZeneca, UK); vatalanib (pINN), (Novartis, Switzerland and Schering AG, Germany); tissue factor pathway inhibitors, (EntreMed, USA); pegaptanib (Pinn), (Gilead Sciences, USA); xanthorrhizol, (Yonsei University, South Korea); vaccine, gene-based, VEGF-2, (Scripps Clinic and Research Foundation, USA); SPV5.2, (Supratek, Canada); SDX 103, (University of California at San Diego, USA); PX 478, (ProlX, USA); METASTATIN, (EntreMed, USA); troponin I, (Harvard University, USA); SU 6668, (SUGEN, USA); OXI 4503, (OXiGENE, USA); o-guanidines, (Dimensional Pharmaceuticals, USA); motuporamine C, (British Columbia University, Canada); CDP 791, (Celltech Group, UK); atiprimod (pINN), (GlaxoSmithKline, UK); E 7820, (Eisai, Japan); CYC 381, (Harvard University, USA); AE 941, (Aeterna, Canada); vaccine, angiogenic, (EntreMed, USA); urokinase plasminogen activator inhibitor, (Dendreon, USA); oglufanide (pINN), (Melmotte, USA); HIF-lalfa inhibitors, (Xenova, UK); CEP 5214, (Cephalon, USA); BAY RES 2622, (Bayer, Germany); Angiocidin, (InKine, USA); A6, (Angstrom, USA); KR 31372, (Korea Research Institute of Chemical Technology, South Korea); GW 2286, (GlaxoSmithKline, UK); EHT 0101, (ExonHit, France); CP 868596, (Pfizer, USA); CP 564959, (OSI, USA); CP 547632, (Pfizer, USA); 786034, (GlaxoSmithKline, UK); KRN 633, (Kirin Brewery, Japan); drug delivery system, intraocular, 2-methoxyestradiol; anginex (Maastricht University, Netherlands, and Minnesota University, USA); ABT 510 (Abbott, USA); AAL 993 (Novartis, Switzerland); VEGI (ProteomTech, USA); tumor necrosis factor-alpha inhibitors; SU 11248 (Pfizer, USA and SUGEN USA); ABT 518, (Abbott, USA); YH16 (Yantai Rongchang, China); S-3APG (Boston Childrens Hospital, USA and EntreMed, USA); MAb, KDR (ImClone Systems, USA); MAb, alpha5 beta (Protein Design, USA); KDR kinase inhibitor (Celltech Group, UK, and Johnson & Johnson, USA); GFB 116 (South Florida University, USA and Yale University, USA); CS 706 (Sankyo, Japan); combretastatin A4 prodrug (Arizona State University, USA); chondroitinase AC (IBEX, Canada); BAY RES 2690 (Bayer, Germany); AGM 1470 (Harvard University, USA, Takeda, Japan, and TAP, USA); AG 13925 (Agouron, USA); Tetrathiomolybdate (University of Michigan, USA); GCS 100 (Wayne State University, USA) CV 247 (Ivy Medical, UK); CKD 732 (Chong Kun Dang, South Korea); irsogladine, (Nippon Shinyaku, Japan); RG 13577 (Aventis, France); WX 360 (Wilex, Germany); squalamine, (Genaera, USA); RPI 4610 (Sirna, USA); heparanase inhibitors (InSight, Israel); KL 3106 (Kolon, South Korea); Honokiol (Emory University, USA); ZK CDK (Schering AG, Germany); ZK Angio (Schering AG, Germany); ZK 229561 (Novartis, Switzerland, and Schering AG, Germany); XMP 300 (XOMA, USA); VGA 1102 (Taisho, Japan); VE-cadherin-2 antagonists(ImClone Systems, USA); Vasostatin (National Institutes of Health, USA); Flk-1 (ImClone Systems, USA); TZ 93 (Tsumura, Japan); TumStatin (Beth Israel Hospital, USA); truncated soluble FLT 1 (vascular endothelial growth factor receptor 1) (Merck & Co, USA); Tie-2 ligands (Regeneron, USA); and thrombospondin 1 inhibitor (Allegheny Health, Education and Research Foundation, USA).

Further examples of therapeutic agents that may be used in combination with compounds of the invention include agents (e.g., antibodies, antigen binding regions, or soluble receptors) that specifically bind and inhibit the activity of growth factors, such as antagonists of hepatocyte growth factor (HGF, also known as Scatter Factor), and antibodies or antigen binding regions that specifically bind its receptor, c-Met.

Another example of a therapeutic agent that may be used in combination with compounds of the invention is an autophagy inhibitor. Autophagy inhibitors include, but are not limited to chloroquine, 3-methyladenine, hydroxychloroquine (Plaquenil^{™}), bafilomycin A1, 5-amino-4-imidazole carboxamide riboside (AICAR), okadaic acid, autophagy-suppressive algal toxins which inhibit protein phosphatases of type 2A or type 1, analogues of cAMP, and drugs which elevate cAMP levels such as adenosine, LY204002, N6-mercaptopurine riboside, and vinblastine. In addition, antisense or siRNA that inhibits expression of proteins including but not limited to ATG5 (which are implicated in autophagy), may also be used. In some embodiments, the one or more additional therapies include an autophagy inhibitor.

Another example of a therapeutic agent that may be used in combination with compounds of the invention is an anti-neoplastic agent. In some embodiments, the one or more additional therapies include an anti-neoplastic agent. Non-limiting examples of anti-neoplastic agents include acemannan, aclarubicin, aldesleukin, alemtuzumab, alitretinoin, altretamine, amifostine, aminolevulinic acid, amrubicin, amsacrine, anagrelide, anastrozole, ancer, ancestim, arglabin, arsenic trioxide, BAM-002 (Novelos), bexarotene, bicalutamide, broxuridine, capecitabine, celmoleukin, cetrorelix, cladribine, clotrimazole, cytarabine ocfosfate, DA 3030 (Dong-A), daclizumab, denileukin diftitox, deslorelin, dexrazoxane, dilazep, docetaxel, docosanol, doxercalciferol, doxifluridine, doxorubicin, bromocriptine, carmustine, cytarabine, fluorouracil, HIT diclofenac, interferon alfa, daunorubicin, doxorubicin, tretinoin, edelfosine, edrecolomab, eflornithine, emitefur, epirubicin, epoetin beta, etoposide phosphate, exemestane, exisulind, fadrozole, filgrastim, finasteride, fludarabine phosphate, formestane, fotemustine, gallium nitrate, gemcitabine, gemtuzumab zogamicin, gimeracil/oteracil/tegafur combination, glycopine, goserelin, heptaplatin, human chorionic gonadotropin, human fetal alpha fetoprotein, ibandronic acid, idarubicin, (imiquimod, interferon alfa, interferon alfa, natural, interferon alfa-2, interferon alfa-2a, interferon alfa-2b, interferon alfa-NI, interferon alfa-n3, interferon alfacon-1, interferon alpha, natural, interferon beta, interferon beta-la, interferon beta-Ib, interferon gamma, natural interferon gamma- la, interferon gamma-Ib, interleukin-1 beta, iobenguane, irinotecan, irsogladine, lanreotide, LC 9018 (Yakult), leflunomide, lenograstim, lentinan sulfate, letrozole, leukocyte alpha interferon, leuprorelin, levamisole + fluorouracil, liarozole, lobaplatin, lonidamine, lovastatin, masoprocol, melarsoprol, metoclopramide, mifepristone, miltefosine, mirimostim, mismatched double stranded RNA, mitoguazone, mitolactol, mitoxantrone, molgramostim, nafarelin, naloxone + pentazocine, nartograstim, nedaplatin, nilutamide, noscapine, novel erythropoiesis stimulating protein, NSC 631570 octreotide, oprelvekin, osaterone, oxaliplatin, paclitaxel, pamidronic acid, pegaspargase, peginterferon alfa-2b, pentosan polysulfate sodium, pentostatin, picibanil, pirarubicin, rabbit antithymocyte polyclonal antibody, polyethylene glycol interferon alfa-2a, porfimer sodium, raloxifene, raltitrexed, rasburiembodiment, rhenium Re 186 etidronate, RII retinamide, rituximab, romurtide, samarium (153 Sm) lexidronam, sargramostim, sizofiran, sobuzoxane, sonermin, strontium-89 chloride, suramin, tasonermin, tazarotene, tegafur, temoporfin, temozolomide, teniposide, tetrachlorodecaoxide, thalidomide, thymalfasin, thyrotropin alfa, topotecan, toremifene, tositumomab-iodine 131, trastuzumab, treosulfan, tretinoin, trilostane, trimetrexate, triptorelin, tumor necrosis factor alpha, natural, ubenimex, bladder cancer vaccine, Maruyama vaccine, melanoma lysate vaccine, valrubicin, verteporfin, vinorelbine, virulizin, zinostatin stimalamer, or zoledronic acid; abarelix; AE 941 (Aeterna), ambamustine, antisense oligonucleotide, bcl-2 (Genta), APC 8015 (Dendreon), decitabine, dexaminoglutethimide, diaziquone, EL 532 (Elan), EM 800 (Endorecherche), eniluracil, etanidazole, fenretinide, filgrastim SD01 (Amgen), fulvestrant, galocitabine, gastrin 17 immunogen, HLA-B7 gene therapy (Vical), granulocyte macrophage colony stimulating factor, histamine dihydrochloride, ibritumomab tiuxetan, ilomastat, IM 862 (Cytran), interleukin-2, iproxifene, LDI 200 (Milkhaus), leridistim, lintuzumab, CA 125 MAb (Biomira), cancer MAb (Japan Pharmaceutical Development), HER-2 and Fc MAb (Medarex), idiotypic 105AD7 MAb (CRC Technology), idiotypic CEA MAb (Trilex), LYM-1-iodine 131 MAb (Techni clone), polymorphic epithelial mucin-yttrium 90 MAb (Antisoma), marimastat, menogaril, mitumomab, motexafin gadolinium, MX 6 (Galderma), nelarabine, nolatrexed, P 30 protein, pegvisomant, pemetrexed, porfiromycin, prinomastat, RL 0903 (Shire), rubitecan, satraplatin, sodium phenylacetate, sparfosic acid, SRL 172 (SR Pharma), SU 5416 (SUGEN), TA 077 (Tanabe), tetrathiomolybdate, thaliblastine, thrombopoietin, tin ethyl etiopurpurin, tirapazamine, cancer vaccine (Biomira), melanoma vaccine (New York University), melanoma vaccine (Sloan Kettering Institute), melanoma oncolysate vaccine (New York Medical College), viral melanoma cell lysates vaccine (Royal Newcastle Hospital), or valspodar.

Additional examples of therapeutic agents that may be used in combination with compounds of the invention include ipilimumab (Yervoy^{®}); tremelimumab; galiximab; nivolumab, also known as BMS-936558 (Opdivo^{®}); pembrolizumab (Keytruda^{®}); avelumab (Bavencio^{®}); AMP224; BMS-936559; MPDL3280A, also known as RG7446; MEDI-570; AMG557; MGA271; IMP321; BMS-663513; PF-05082566; CDX-1127; anti-OX40 (Providence Health Services); huMAbOX40L; atacicept; CP-870893; lucatumumab; dacetuzumab; muromonab-CD3; ipilumumab; MEDI4736 (Imfinzi^{®}); MSB0010718C; AMP 224; adalimumab (Humira^{®}); ado-trastuzumab emtansine (Kadcyla^{®}); aflibercept (Eylea^{®}); alemtuzumab (Campath^{®}); basiliximab (Simulect^{®}); belimumab (Benlysta^{®}); basiliximab (Simulect^{®}); belimumab (Benlysta^{®}); brentuximab vedotin (Adcetris^{®}); canakinumab (Ilaris^{®}); certolizumab pegol (Cimzia^{®}); daclizumab (Zenapax^{®}); daratumumab (Darzalex^{®}); denosumab (Prolia^{®}); eculizumab (Soliris^{®}); efalizumab (Raptiva^{®}); gemtuzumab ozogamicin (Mylotarg^{®}); golimumab (Simponi^{®}); ibritumomab tiuxetan (Zevalin^{®}); infliximab (Remicade^{®}); motavizumab (Numax^{®}); natalizumab (Tysabri^{®}); obinutuzumab (Gazyva^{®}); ofatumumab (Arzerra^{®}); omalizumab (Xolair^{®}); palivizumab (Synagis^{®}); pertuzumab (Perjeta^{®}); pertuzumab (Perjeta^{®}); ranibizumab (Lucentis^{®}); raxibacumab (Abthrax^{®}); tocilizumab (Actemra^{®}); tositumomab; tositumomab-i-131; tositumomab and tositumomab-i-131 (Bexxar^{®}); ustekinumab (Stelara^{®}); AMG 102; AMG 386; AMG 479; AMG 655; AMG 706; AMG 745; and AMG 951.

The compounds described herein can be used in combination with the agents disclosed herein or other suitable agents, depending on the condition being treated. Hence, in some embodiments the one or more compounds of the disclosure will be co-administered with other therapies as described herein. When used in combination therapy, the compounds described herein may be administered with the second agent simultaneously or separately. This administration in combination can include simultaneous administration of the two agents in the same dosage form, simultaneous administration in separate dosage forms, and separate administration. That is, a compound described herein and any of the agents described herein can be formulated together in the same dosage form and administered simultaneously. Alternatively, a compound of the invention and any of the therapies described herein can be simultaneously administered, wherein both the agents are present in separate formulations. In another alternative, a compound of the present disclosure can be administered and followed by any of the therapies described herein, or vice versa. In some embodiments of the separate administration protocol, a compound of the invention and any of the therapies described herein are administered a few minutes apart, or a few hours apart, or a few days apart.

In some embodiments of any of the methods described herein, the first therapy (e.g., a compound of the invention) and one or more additional therapies are administered simultaneously or sequentially, in either order. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours, up to 24 hours, or up to 1-7, 1-14, 1-21 or 1-30 days before or after the one or more additional therapies.

### Examples

### Materials and Methods

In some aspects, the invention includes the intermediates, examples, and synthetic methods described herein in all of their embodiments.

The compounds of the **Formula I** may be prepared by the methods described below, together with synthetic methods known in the art of organic chemistry, or modifications and derivatizations that are familiar to those of ordinary skill in the art. The starting materials used herein are commercially available or may be prepared by routine methods known in the art, e.g., methods disclosed in standard reference books such as the Compendium of Organic Synthetic Methods, Vol. I-VI (Wiley-Interscience); or the Comprehensive Organic Transformations, by R.C. Larock (Wiley-Interscience). Preferred methods include, but are not limited to, those described below.

During any of the following synthetic sequences it may be necessary and/or desirable to protect sensitive or reactive groups on any of the molecules concerned. This can be achieved by means of conventional protecting groups, such as those described in T.W. Greene, Protective Groups in Organic Chemistry, John Wiley & Sons (1981); T.W. Greene and P.G.M. Wuts, Protective Groups in Organic Chemistry, John Wiley & Sons (1991), T.W. Greene and P.G.M. Wuts, Protective Groups in Organic Chemistry, John Wiley & Sons (1999); T.W. Greene and P.G.M. Wuts, Protective Groups in Organic Chemistry, John Wiley & Sons (2006); and T.W. Greene and P.G.M. Wuts, Protective Groups in Organic Chemistry, John Wiley & Sons (2014), which are hereby incorporated by reference in their entireties.

Compounds of **Formula I,** or their pharmaceutical acceptable salts, and the intermediates used in the synthesis of the compounds of this invention can be prepared according to the reaction schemes discussed hereinbelow and the general skill in the art.

Unless otherwise indicated, the substituents in the schemes are defined as above. Isolation and purification of the products is accomplished by standard procedures, which are known to a chemist of ordinary skill.

When a general or exemplary synthetic procedure is referred to, one skilled in the art can readily determine the appropriate reagents, if not indicated, extrapolating from the general or exemplary procedures. Some of the general procedures are given as examples for preparing specific compounds. One skilled in the art can readily adapt such procedures to the synthesis of other compounds. Representation of an unsubstituted position in structures shown or referred to in the general procedures is for convenience and does not preclude substitution as described elsewhere herein. For specific groups that can be present, either as R groups in the general procedures or as optional substituents not shown, refer to the descriptions in the remainder of this document, including the claims, summary and detailed description.

The process to produce compounds of the present invention is preferably carried out at about atmospheric pressure although higher or lower pressures can be used if desired. Substantially equimolar amounts of reactants are preferably used although higher or lower amounts may also be used.

Unless otherwise noted, all materials and reagents were obtained from commercial suppliers and used without further purification. Reactions were monitored by thin layer chromatography (TLC) on silica gel 60 F254 (0.2 mm) precoated aluminum foil or glass-backed and visualized using UV light or appropriate TLC stains. Flash chromatography was performed using either an Agela Technologies CombiFlash with CHEETAH Purification System or an ISCO CombiFlash Rf 200 Organic Purification System. Preparative TLC was performed on Xinnuo Silica Gel 10-40 µm size 20x20 cm plates with a thickness of 1000 pm or equivalent.

¹H NMR (300 or 400 MHz) spectra were recorded on Bruker or Varian instruments at room temperature with TMS or the residual solvent peak as the internal standard. The line positions or multiples are given in (δ) and the coupling constants (J) are given as absolute values in Hertz (Hz). The multiplicities in ¹H NMR spectra are abbreviated as follows: (singlet), d (doublet), t (triplet), q (quartet), quint (quintet), m (multiplet), mc (centered multip- let), br or broad (broadened).

NMR data are generally collected in deuterated solvents such as DMSO-*d*₆, CD3OD, CDCl3 or Acetonitrile-*d*₃, although the deuterated status of the solvent may or may not be explicitly shown in NMR data section.

Preparative HPLC purifications were performed on a Waters^{®} Mass-Directed Purification System equipped with 2545 or 2525 Binary Gradient Module, 2767 Sample Manager, a Column Fluidics Organizer (CFO), 2489 Photodiode Array Detector, a 515 pump for makeup flow, a reagent manager, a 515 pump for at- column-dilution, Zspray^{™} single-quadrupole Mass Detector equipped with a Z-spray electrospray interface, controlled by MassLynx^{™} Version 4.1 with FractionLynx^{™} software. The mobile phases were water and acetonitrile with 0.1% formic acid or 0.01 M NH₄HCO₃ unless otherwise noted. The flow rate was 25 mL/min. After the columns, a 1: 1000 LC packings flow splitter allowed transfer of a small portion of the eluent into the UV detector and, subsequently, a 10% portion into the ZQ MS. The electrospray source was set at 3.0 kV capillary voltage, 30 V cone voltage, 110° C source temperature, 350° C desolvation temperature, 600 L/h desolvation gas flow, and 60 L/h cone gas flow. For the analyzer, the multiplier was set at 550 for preparative tune method.

Analytical LCMS data was collected on LCMS01, LCMS02, UPLC01, or UPLC02 instruments with a mobile phase of acetonitrile (B) and HPLC grade water (A) with either 0.05% formic acid or 0.05% TFA in HPLC grade water (B) unless otherwise noted.

LCMS01 is a Shimadzu LC-20ADXR HPLC equipped with a SPD-M20A detector and LCMS-2020 for ionization. The system uses the following conditions for either 5 or 3 minute run time.

5 minute run: Ascentis Express C18 column, 2 µm, 3.0x50 mm. The flow rate is 1.5 mL/min, the run time is 5 min, and the gradient profiles are 0.01 min 5% B, 3.00 min 100% B, 4.60 min 100% B, 4.90 min 5% B, 5.00 min 0% B. The LCMS-2020 instrument utilized electrospray ionization in positive (ES+) or negative (ES-) mode.

3 minute run: Ascentis Express C18 column, 2 µm, 3.0x50 mm. The flow rate is 1.5 mL/min, the run time is 3 min, and the gradient profiles are 0.01 min 5% B, 2.00 min 100% B, 2.70 min 100% B, 2.75 min 5% B, 3.00 min 0% B.

Agilent LCMS is an Agilent 1260 HPLC equipped with 6120/6125 single-quadrupole Mass detector, ESI for ionization. The system uses the following conditions for 2.5 min run time.

Conditions: Waters CORTECS C18+ column, 2.7µm, 4.6x30 mm. The flow rate is 1.8 mL/min, the run time is 2.5 min, and the gradient profiles are 0.00 min 5% B, 1.00 min 95% B, 2.0

min 95% B, 2.1 min 5% B, 2.5 min 5% B. Premier XE MS utilized electrospray ionization in positive (ES+) or negative (ES-) modes.

UPLC01 is an Agilent Technologies 1260 Infinity II attached to a DAD (G4212-60008) detector. Waters T3 column, 4.6x100 mm was heated to 60° C with detection at 254 nm and at 220 nm and electrospray ionization in positive mode was used. Table 2 below lists the mobile phase gradient (solvent A: 0.05% TFA in water; solvent B: 0.05% TFA in acetonitrile) and flow rate for the analytical UPLC program.

**Table 2**

| **Time (mm)** | **A %** | **B %** | **Flow Rate mL/mm** |
|---|---|---|---|
| 0.00 | 95.0 | 5.0 | 1.00 |
| 8.00 | 5.0 | 95.0 | 1.00 |
| 10.00 | 5.0 | 95.0 | 1.00 |
| 11.00 | 95.0 | 5.0 | 1.00 |
| 13.00 | 95.0 | 5.0 | 1.00 |

UPLC02 is an ACQUITY sample manager attached to PDA detector. ACQUITYUPLC^{®} BEH CI8 1.7 pm 2.1x50 mm was heated to 45° C. with detection at 254/214 nm. Table 3 below lists the mobile phase gradient (solvent A: 0.05% TFA in water; solvent B: 0.05% TFA in acetonitrile) and flow rate for the analytical UPLC program.

**Table 3**

| **Time (mm)** | **A%** | **B %** | **Flow Rate (mL/mm)** |
|---|---|---|---|
| 0.00 | 95 | 5 | 0.50 |
| 2.00 | 5 | 95 | 0.50 |
| 2.50 | 5 | 95 | 0.50 |
| 2.70 | 95 | 5 | 0.50 |
| 3.50 | 95 | 5 | 0.50 |

### Example 1 - Synthesis of Intermediates

### A. Methyl (S)-1-((S)-2-((tert-butoxycarbonyl)amino)-3-(3-(4,4,5,5-tetramethyl-1,3,2-dioxaborolan-2-yl)-5-((triisopropylsilyl)oxy)phenyl)propanoyl)hexahydropyridazine-3-carboxylate

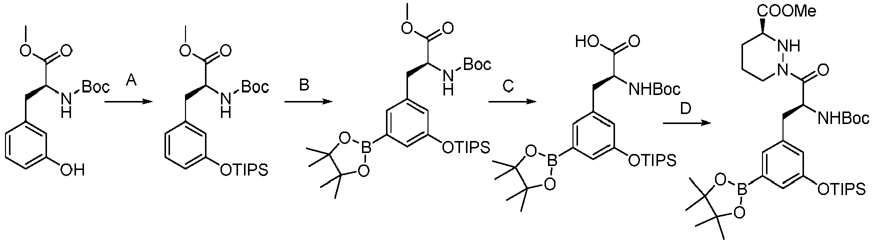

### Step A

To a solution of (*S*)-methyl 2-(*tert*-butoxycarbonylamino)-3-(3-hydroxyphenyl)propanoate (10.0 g, 33.9 mmol) in dichloromethane (100 mL) was added imidazole (4.6 g, 67.8 mmol) and TIPSCI (7.8 g, 40.7 mmol). The mixture was stirred for 16 hours and then diluted with dichloromethane (200 mL) and washed with H₂O (3 x 150 mL). The organic layer was dried over anhydrous sodium sulfate, filtered, and concentrated to give a residue that was purified by silica gel chromatography (0-10% ethyl acetate in petroleum ether) to give methyl (S)-2-((*tert*-butoxycarbonyl)amino)-3-(3-((triisopropylsilyl)oxy)phenyl)propanoate (98% yield) as a colorless oil. ESI-MS *m*/*z =* 474.2 [M+Na]⁺

### Step B

Methyl (S)-2-((*tert*-butoxycarbonyl)amino)-3-(3-((triisopropylsilyl)oxy)phenyl)propanoate (7.5 g, 16.6 mmol), bis(pinacolato)diborane (6.3 g, 24.9 mmol), [Ir(OMe)(COD)]₂ (1.1 g, 1.66 mmol) and *4-tert-*butyl-2-(4-*tert*-butyl-2-pyridyl)pyridine (1.3 g, 4.98 mmol) were combined in flask. After purging with argon, tetrahydrofuran (75 mL) was added. The flask was sealed, heated to 80 °C and stirred for 16 hours. The mixture was concentrated *in vacuo* and then purified by silica gel chromatography (0-20% ethyl acetate in petroleum ether) to give methyl (S)-2-((*tert*-butoxycarbonyl)amino)-3-(3-(4,4,5,5-tetramethyl-1,3,2-dioxaborolan-2-yl)-5-((triisopropylsilyl)oxy)phenyl)propanoate (78% yield) as a white solid. ESI-MS *m*/*z =* 600.4 [M+Na]⁺.

### Step C

To a solution of methyl (S)-2-((*tert*-butoxycarbonyl)amino)-3-(3-(4,4,5,5-tetramethyl-1,3,2-dioxaborolan-2-yl)-5-((triisopropylsilyl)oxy)phenyl)propanoate (4.95 g, 6.88 mmol) in methanol (53 mL) at 0 °C was added lithium hydroxide (840 mg, 34.4 mmol) in water (35 mL). The mixture was stirred at 0 °C for 2 hours and then acidified to pH~5 with aqueous 1 M hydrochloric acid. The resulting solution was extracted with ethyl acetate (2 x 250 mL) and washed with brine (3 x 100 mL). The organic layer was dried over anhydrous sodium sulfate and concentrated to give (S)-2-((*tert*-butoxycarbonyl)amino)-3-(3-(4,4,5,5-tetramethyl-1,3,2-dioxaborolan-2-yl)-5-((triisopropylsilyl)oxy)phenyl)propanoic acid as a white solid that was used in the next step without further purification. ESI-MS *m*/*z =* 581.4 [M+NH₄]⁺.

### Step D

To a solution of the trifluoroacetic acid salt of methyl (*S*)-hexahydropyridazine-3-carboxylate (6.48 g, 45.0 mmol) in dichloromethane (200 mL) at 0 °C was added *N*-methylmorpholine (40.99 g, 405.2 mmol), (S)-2-((*tert*-butoxycarbonyl)amino)-3-(3-(4,4,5,5-tetramethyl-1,3,2-dioxaborolan-2-yl)-5-((triisopropylsilyl)oxy)phenyl)propanoic acid (24.0 g, 42.6 mmol) in dichloromethane (50 mL), HOBt (1.21 g, 9.01 mmol) and EDCI (12.9 g, 67.55 mmol). The mixture was stirred at 20 °C for 16 hours and then diluted with dichloromethane (200 mL) and washed with water (3 x 150 mL). The organic layer was dried over anhydrous sodium sulfate and concentrated to give the crude product that was purified by silica gel chromatography (0-20% ethyl acetate in petroleum ether) to give methyl (S)-1-((S)-2-((*tert-*butoxycarbonyl)amino)-3-(3-(4,4,5,5-tetramethyl-1,3,2-dioxaborolan-2-yl)-5-((triisopropylsilyl)oxy)phenyl)propanoyl)hexahydropyridazine-3-carboxylate (71% yield) as a yellow oil.
ESI-MS *m*/*z* = 690.5 [M+H]⁺

### B. Methyl (S)-1-((S)-2-((tert-butoxycarbonyl)amino)-3-(3-(4,4,5,5-tetramethyl-1,3,2-dioxaborolan-2-yl)-5-((triisopropylsilyl)oxy)phenyl)propanoyl)hexahydropyridazine-3-carboxylate

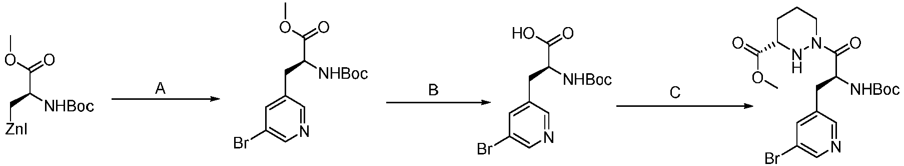

### Step A

A solution of (R)-(2-((*tert*-butoxycarbonyl)amino)-3-methoxy-3-oxopropyl)zinc(II) iodide (20.0 mL, 25 mmol, 1.0 equiv), Pd(PPh₃)₂Cl₂ (1.75 g, 2.5 mmol, 0.1 equiv) and 3-bromo-5-iodopyridine (7.1 g, 25 mmol, 1.0 equiv) in DMF (10 mL) was stirred at 50 °C for 15 hours. The reaction was quenched by addition of ice water (300 mL) and extracted with ethyl acetate (3 x 200 mL). The combined organic layers were dried over anhydrous sodium sulfate, filtered, and concentrated under reduced pressure to give a crude product which was purified by silica gel chromatography (PE to petroleum ether/ethyl acetate=1:1) to give methyl (S)-3-(5-bromopyridin-3-yl)-2-((*tert*-butoxycarbonyl)amino)propanoate (3.1 g, 35 % yield) as a yellow solid. ESI-MS *m*/*z=* 359.1 [M+H]⁺.

### Step B

To a solution of methyl (S)-3-(5-bromopyridin-3-yl)-2-((*tert*-butoxycarbonyl)amino)propanoate (1.8 g, 5.0 mmol, 1.0 equiv) in MeOH (20 mL) was added LiOH (600 mg, 25.0 mmol, 5.0 equiv) in H₂O (5 mL) at 0 °C. The mixture was stirred at 0 °C for 5 hours. The mixture was acidified to pH ~5 with 1 M HCl and extracted with ethyl acetate (100 mL x 2). The organic layer washed with brine (100 mL x 3), dried over anhydrous sodium sulfate and concentrated to give a residue. The crude product (1.73 g crude) was used in the next step directly without further purification. ESI-MS *m*/*z* = 345.0 [M+H]⁺.

### Step C

A solution of (S)-3-(5-bromopyridin-3-yl)-2-((*tert*-butoxycarbonyl)amino)propanoic acid (1.73 g, 5.0 mmol, 1.0 equiv), HATU (2.85 g, 7.5 mmol, 1.5 equiv) and DIPEA (3.23 g, 25 mmol, 5.0 equiv) in DMF (15 mL) was stirred at 0 °C for 30 minutes. Methyl (S)-hexahydropyridazine-3-carboxylate (2.23 g, 6.0 mmol, 1.2 equiv, TFA salt) in DMF (5 mL) was then added dropwise. After 2 hours, the reaction was quenched by the addition of ice water (100 mL) and extracted with ethyl acetate (3 x 100 mL). The combined organic layers were dried over anhydrous sodium sulfate and concentrated under reduced pressure to give a crude product, which was purified by silica gel chromatography (dichloromethane to dichloromethane/MeOH = 20:1) to give methyl (S)-1-((S)-3-(5-bromopyridin-3-yl)-2-((*tert-*butoxycarbonyl)amino)propanoyl)hexahydropyridazine-3-carboxylate (1.51 g, 64% yield) as an oil. ESI-MS *m*/*z=* 471.1 [M+H]⁺.

The following intermediate was synthesized according to the procedure described to make Intermediate B using appropriate building blocks and modified reaction conditions (such as reagents, ratio of reagents, temperature, and reaction time) as needed.

| **Intermediate No.** | **Structure** | **Analytical Data** |
|---|---|---|
| B-1 | 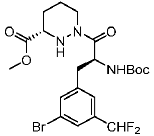 | ESI-MS *m*/*z=* 520.2 [M+H]⁺ |

### C. Methyl (S)-1-((S)-3-(6-bromo-4-((tert-butoxycarbonyl)oxy)pyridin-2-yl)-2-((tert-butoxycarbonyl)amino)propanoyl)hexahydropyridazine-3-carboxylate

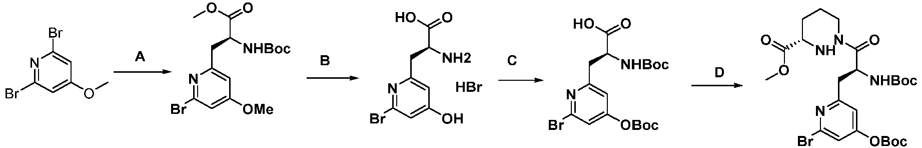

### Step A

A solution of (R)-(2-((*tert*-butoxycarbonyl)amino)-3-methoxy-3-oxopropyl)zinc(II) iodide (20.0 mL, 24 mmol, 2.0 equiv), Pd(PPh₃)₂Cl₂ (1.68 g, 2.4 mmol, 0.2 equiv) and 2,6-dibromo-4-methoxypyridine (3.2 g, 12 mmol, 1.0 equiv) in DMF (10 mL) was stirred at 65 °C for 2 hours. The reaction was quenched by the addition of ice water (300 mL) and extracted with ethyl acetate (3 x 200 mL). The combined organic layers were dried over anhydrous sodium sulfate and concentrated under reduced pressure to give a crude product that was purified by silica gel chromatography (dichloromethane to dichloromethane/MeOH = 40:1) to give methyl (S)-3-(6-bromo-4-methoxypyridin-2-yl)-2-((*tert*-butoxycarbonyl)amino)propanoate (2.4 g, 51% yield) as a yellow oil. ESI-MS *m*/*z=* 389.0 [M+H]⁺.

### Step B

A solution of methyl (S)-3-(6-bromo-4-methoxypyridin-2-yl)-2-((*tert-*butoxycarbonyl)amino)propanoate (2.4 g, 6.17 mmol, 1.0 equiv) in HBr (40% in water) (20 mL) at 130 °C was stirred for 16 hours. The mixture was concentrated to give a crude residue (2.1 g) as a yellow solid that was used in the next step without further purification. ESI-MS *m*/*z =* 261.0 [M+H]⁺.

### Step C

To a stirred solution of (S)-2-amino-3-(6-bromo-4-hydroxypyridin-2-yl)propanoic acid (2.1 g, 6.17 mmol, 1.0 equiv) in THF (100 mL) was added DMAP (753 mg, 6.17 mmol, 1.0 equiv) and TEA (1.2 g, 12.34 mmol, 2.0 equiv) followed by (Boc)₂O (2.69 g, 12.34 mmol, 2.0 equiv). The mixture was stirred for 5 hours and then the solution was concentrated to give a residue. The residue was purified by silica gel chromatography (dichloromethane to dichloromethane/MeOH = 20:1) to give (S)-3-(6-bromo-4-((*tert-*butoxycarbonyl)oxy)pyridin-2-yl)-2-((*tert*-butoxycarbonyl)amino)propanoic acid (2.15 g, 76% yield) as a yellow oil. ESI-MS *m*/*z=* 460.1 [M+H]⁺.

### Step D

A solution of (S)-3-(3-bromo-5-(difluoromethyl)phenyl)-2-((*tert*-butoxycarbonyl)amino)propanoic acid (2.15 g, 4.66 mmol, 1.0 equiv), HATU (2.66 g, 6.99 mmol, 1.5 equiv) and DIEA (3.00 g, 23.3 mmol, 5.0 equiv) in DMF (15 mL) was stirred at 5 °C for 30 minutes. Methyl (S)-hexahydropyridazine-3-carboxylate (1.44 g, 5.6 mmol, 1.2 equiv, TFA salt) in DMF (5 mL) was added dropwise. After 2 hours, the reaction was quenched by the addition of ice water (100 mL) and extracted with ethyl acetate (3 x 100 mL). The combined organic layers were dried over anhydrous sodium sulfate, filtered, and concentrated under reduced pressure to give a crude product, which was purified by silica gel chromatography (dichloromethane to dichloromethane/MeOH = 40:1) to give methyl (S)-1-((S)-3-(6-bromo-4-((*tert-*butoxycarbonyl)oxy)pyridin-2-yl)-2-((*tert*-butoxycarbonyl)amino)propanoyl)hexahydropyridazine-3-carboxylate (2.05 g, 75 % yield) as a yellow oil. ESI-MS *m*/*z=* 587.1 [M+H]⁺.

### D. 2-(6-bromo-1-(3-hydroxy-2,2-dimethylpropyl)-1H-indol-3-yl)-2-methylpropanenitrile

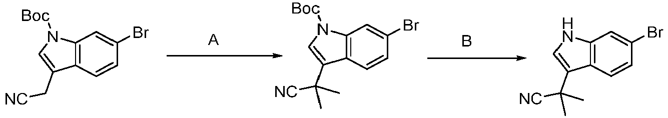

### Step A

A solution of *tert*-butyl 6-bromo-3-(cyanomethyl)-1H-indole-1-carboxylate (1.3 g, 3.88 mmol, 1.0 equiv) in THF (25 mL) was added LiHMDS (9.7 mL, 9.7 mmol, 2.5 equiv) at -78 °C. This was followed by the addition of Mel (1.38 g, 9.72 mmol, 2.51 equiv) dropwise at -78 °C. The resulting mixture was slowly warmed to room temperature and then stirred for 16 hours. The reaction was quenched by the addition of saturated aqueous NH₄Cl (10 mL). The resulting mixture was diluted with water (200 mL) and then extracted with ethyl acetate (3 x 100 mL). The combined organic layers were washed with brine (100 mL) and dried over anhydrous sodium sulfate. After filtration, the filtrate was concentrated under reduced pressure and the crude product was purified by silica gel chromatography, eluting with petroleum ether/ dichloromethane (5:1) to afford *tert*-butyl 6-bromo-3-(1-cyano-1-methylethyl)-1H-indole-1-carboxylate (1.2 g, 81%) as a white solid. ¹H NMR (300 MHz, CDCl₃) δ 8.41 (s, 1H), 7.69 (d, *J =* 8.5 Hz, 1H), 7.51-7.39 (m, 2H), 1.85 (s, 6H), 1.70 (s, 9H).

### Step B

To a stirred solution of *tert*-butyl 6-bromo-3-(1-cyano-1-methylethyl)-1H-indole-1-carboxylate (1.1 g, 3.03 mmol, 1.0 equiv) in dichloromethane (20 mL) was added TFA (10 mL, 134.63 mmol, 44.5 equiv) dropwise at 0 °C. The resulting mixture was stirred for 2 hours at 0 °C. The mixture was then concentrated under vacuum. The resulting mixture was diluted with water (200 mL). The mixture was basified to pH 8 with saturated aqueous NaHCO₃. The resulting solution was extracted with ethyl acetate (3 x 200 mL). The combined organic layers were washed with brine (100 mL) and dried over anhydrous sodium sulfate. After filtration, the filtrate was concentrated under reduced pressure. The crude 2-(6-bromo-1H-indol-3-yl)-2-methylpropanenitrile (750 mg, 89% yield) was used in the next step directly without further purification. ESI-MS *m*/*z =* 263.1 [M+H]⁺.

The following intermediates were synthesized according to the procedure described to make Intermediate D using appropriate building blocks and modified reaction conditions (such as reagents, ratio of reagents, temperature, and reaction time) as needed.

| **Intermediate No.** | **Structure** | **Analytical Data** |
|---|---|---|
| D-1 | 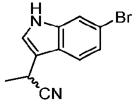 | ESI-MS *m*/*z* = 249.0, 251.0 [M+H]⁺ |
| D-2 | 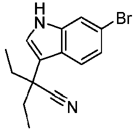 | ESI-MS *m*/*z* = 291.0 [M+H]⁺ |
| D-3 | 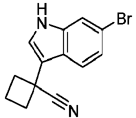 | |
| D-4 | 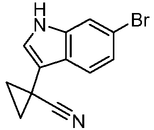 | ESI-MS *m*/*z* = 261.0 [M+H]⁺ |
| D-5 | 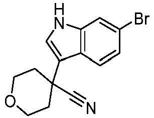 | ESI-MS *m*/*z* = 305.0 [M+H]⁺ |
| D-6 | 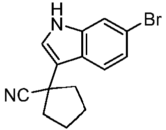 | ESI-MS *m*/*z* = 289.1 [M+H]⁺ |

### E. 3-(6-bromo-1H-indol-3-yl)-3-methylbutan-2-one

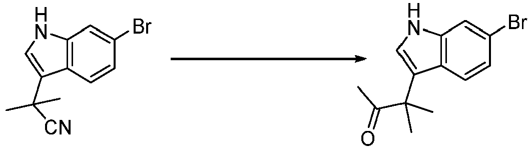

To a stirred solution of 2-(6-bromo-1H-indol-3-yl)-2-methylpropanenitrile (3.5 g, 0.013 mmol, 1.0 equiv) in THF (50 mL) was added MeLi (1 M, 10 equiv, 35 mL) dropwise at 0 °C. The resulting mixture was stirred for 3 hours at 0 °C. To the mixture was added aqueous HCl (1 L) at room temperature. The resulting mixture was stirred for 16 hours at room temperature. The reaction was quenched by the addition of aqueous NaHCO₃ solution (500 mL) at room temperature. The mixture was diluted with water (300 mL) and then extracted with ethyl acetate (2 x 300 mL). The combined organic layers were washed with water (2 x 200 mL) and dried over anhydrous sodium sulfate. After filtration, the filtrate was concentrated under reduced pressure and the residue was purified by silica gel column chromatography, eluting with EA/PE (1:20-1:12) to afford 3-(6-bromo-1H-indol-3-yl)-3-methylbutan-2-one (2 g, 48% yield) as a brown solid. ESI-MS *m*/*z =* 280.1 [M+H]⁺.

### F: 6-bromo-1H-indole-3-carboxamide

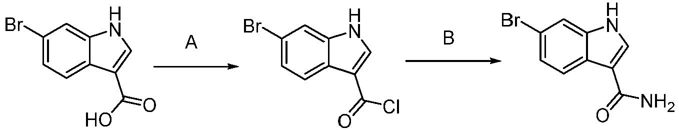

### Step A

To a solution of 6-bromo-1H-indole-3-carboxylic acid (2.88 g, 12.0 mmol, 1.0 equiv) in dichloromethane (10 mL) and DMF (10 mL) at 0 °C was added oxalyl dichloride (4.57 g, 36.0 mmol, 3.0 equiv) dropwise. The mixture was stirred at 0 °C for 2 hours. The mixture was used in the next step directly.

### Step B

To a solution of NH₃·H₂O (8.16 g, 120.0 mmol, 10.0 equiv, 25% NH₃) in H₂O (20 mL) was added 6-bromo-1H-indole-3-carbonyl chloride (reaction solvent from Step A) dropwise at 0 °C. The mixture was stirred at 0 °C for 2 hours. The mixture was poured into water and extracted with ethyl acetate (200 mL). The organic layer washed with brine (3 x 50 mL), dried over anhydrous sodium sulfate and concentrated to give a residue. The residue was purified by silica gel chromatography (dichloromethane to dichloromethane/MeOH = 20:1) to give 6-bromo-1H-indole-3-carboxamide (2.45 g, 85% yield) as a white solid. ESI-MS *m*/*z =* 241.0 [M+H]⁺.

The following intermediates were synthesized according to the procedure described to make Intermediate F using appropriate building blocks and modified reaction conditions (such as reagents, ratio of reagents, temperature, and reaction time) as needed.

| **Intermediate No.** | **Structure** | **Analytical Data** |
|---|---|---|
| F-1 | 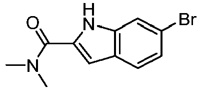 | ESI-MS *m*/*z* = 267.0 [M+H]⁺ |
| F-2 | 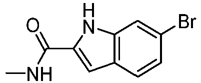 | ESI-MS *m*/*z* = 253.0 [M+H]⁺ |

### G: 6-bromo-3-(methylsulfonyl)-1H-indole

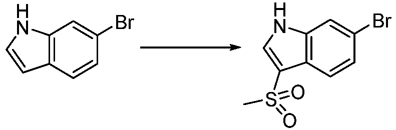

6-Bromo-1H-indole (1.0 g, 5.13 mmol, 1.0 equiv) and *tert*-BuOK (1.15 g, 10.3 mmol, 2.0 equiv) in THF (15 mL) was stirred for 30 minutes at room temperature. A solution of Et₃B (10.3 mL, 10.3 mmol, 2 equiv, 1 M in THF) was added dropwise over the course of 30 minutes. Methanesulfonyl chloride (1.2 g, 10.3 mmol, 2.0 equiv) was added at -15 °C and the solution was maintained at that temperature for 24 hours. The reaction was quenched by the addition of 30 mL of saturated aqueous NH₄Cl. The resulting solution was extracted with ethyl acetate (3 x 30 mL) and dried over anhydrous sodium sulfate, filtered, and concentrated. The crude product was purified by Prep-HPLC (5% MeCN-95% MeCN in water w/ 0.05% FA) to give 6-bromo-3-(methylsulfonyl)-1H-indole (620 mg, 44% yield) of as a light green solid. ESI-MS *m*/*z =* 274.0, 276.0 [M+H]⁺.

### H: 6-bromo-2-methyl-1H-indole-3-carbonitrile

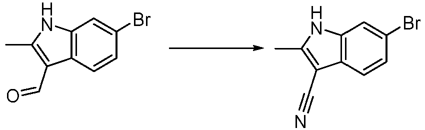

To a mixture of 6-bromo-2-methyl-1*H*-indole-3-carbaldehyde (3.2 g, 13.4 mmol, 1.0 equiv), hydroxylamine hydrochloride (1.0 g, 14.8 mmol, 1.1 equiv), and Et₃N (1.5 g, 14.8 mmol, 1.1 equiv) in DMF (30 mL) was added T₃P^{®} (4.7 g, 14.8 mmol, 1.1 equiv, 50% in ethyl acetate). The mixture was stirred at 100 °C for 3 hours and then poured onto saturated aqueous NaHCO₃ solution (200 mL) and extracted with ethyl acetate (3 × 50 mL). The combined organic layers were washed with H₂O (50 mL) and brine (50 mL), dried over sodium sulfate, filtered, and concentrated under reduced pressure to give 6-bromo-2-methyl-1*H-*indole-3-carbonitrile (1.5 g, 45% yield) as a white solid. ESI-MS *m*/*z* = 235.0 [M+H]⁺.

### I: 6-bromo-1-(3-hydroxy-2,2-dimethylpropyl)-1H-pyrrolo[2,3-b]pyridine-3-carbonitrile

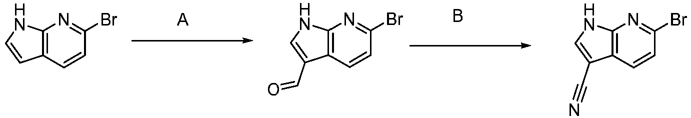

### Step A

To a stirred solution of 6-bromo-1*H*-pyrrolo[2,3-b]pyridine (6.0 g, 30 mmol, 1.0 equiv) in DMF (10 mL) at 0 °C was added phosphorus chloride oxide (90 mmol, 8.4 mL, 3.0 equiv) and the resulting mixture was stirred at room temperature. After being stirred for 1 hour, the reaction mixture was poured into cold saturated aqueous NaHCO₃ solution and stirred for 30 minutes. The reaction mixture was extracted with ethyl acetate (3 x). The combined organic layers were dried over anhydrous sodium sulfate, filtered, and concentrated under reduced pressure to give 6-bromo-1*H*-pyrrolo[2,3-b]pyridine-3-carbaldehyde (6.0 g, 87% yield) as a white solid. ESI-MS *m*/*z =* 225.0 [M+H]⁺. ¹H NMR (400 MHz, DMSO-*d₆*) δ 12.91 (s, 1H), 9.94 (s, 1H), 8.51 (s, 1H), 8.34 (d, *J* = 8.2 Hz, 1H), 7.49 - 7.47 (d, *J =* 8.2 Hz, 1H).

### Step B

To a mixture of 6-bromo-1*H*-pyrrolo[2,3-b]pyridine-3-carbaldehyde (2.24 g, 10 mmol, 1.0 equiv), hydroxylamine hydrochloride (764 mg, 11 mmol, 1.1 equiv), and triethylamine (1.11 g, 11 mmol, 1.1 equiv) in DMF (30 mL) was added T₃P^{®} (3.5 g, 11 mmol, 1.1 equiv, 50% solutionn in ethyl acetate). The mixture was stirred at 100 °C for 3 hours. The mixture was cooled and poured into aqueous sodium bicarbonate solution (200 mL) and extracted with ethyl acetate (3 × 50 mL). The combined organic layers were washed with water (50 mL) and brine (50 mL), dried over sodium sulfate, filtered, and concentrated under reduced pressure to give the product of 6-bromo-1*H*-pyrrolo[2,3-b]pyridine-3-carbonitrile (2.0 g, 91% yield) as a white solid. ESI-MS *m*/*z =* 222.1 [M+H]⁺.

### J: 6-bromo-4-hydroxy-1-naphthonitrile

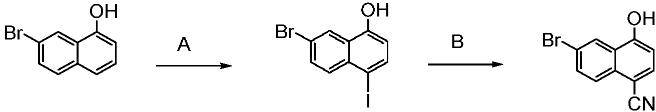

### Step A

A solution of 7-bromonaphthalen-1-ol (100 mg, 0448 mmol, 1 equiv), MeCN (10 mL), *p*TsOH (77.0 mg, 0.45 mmol, 1.0 equiv), and *N-*iodosuccinimide (101.0 mg, 0.45 mmol 1.0 equiv) was stirred for 14 hours at 25 °C. The residue was purified by silica gel chromatography (ethyl acetate/petroleum ether (5:1)) to give 7-bromo-4-iodonaphthalen-1-ol (130 mg 83% yield). ESI-MS *m*/*z =* 347.0 [M-H]⁻.

### Step B

A solution of 7-bromo-4-iodonaphthalen-1-ol (2.20 g, 6.30 mmol, 1.0 equiv), acetonitrile (40 mL), zincdicarbonitrile (1.10 g, 9.46 mmol, 1.5 equiv), and Pd(dba)₂ (220 mg, 0.383 mmol, 0.06 equiv) was stirred for 16 hours at 70 °C. The residue was applied onto a silica gel column and eluted with ethyl acetate/hexane (5:1) to give 6-bromo-4-hydroxynaphthalene-1-carbonitrile (700 mg, 45% yield). ESI-MS *m*/*z* = 246.0 [M-H]⁻.

### K: 8-bromo-5-ethyl-1,3,4,5-tetrahydro-2H-benzo[b][1,4]diazepin-2-one

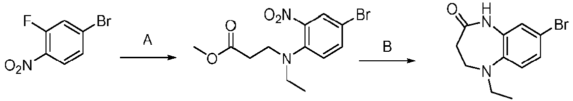

### Step A

To a stirred solution of 4-bromo-2-fluoro-1-nitrobenzene (5.0 g, 22.7 mmol, 1.0 equiv) in DMF (50 mL) was added K₂CO₃ (6.33 g, 45.5 mmol, 2.0 equiv) and methyl 3-(ethylamino)propanoate (3.9 g, 29.7 mmol, 1.3 equiv) dropwise. The resulting mixture was stirred for for 16 hours and then the mixture was diluted with 100 mL water and extracted with ethyl acetate (3 x 50 mL). The combined organic layers were washed with brine (3 x 100 mL) and dried over anhydrous sodium sulfate. After filtration, the filtrate was concentrated under reduced pressure. The residue was purified by silica gel chromatography, eluting with petroleum ether (20:1 to 12:1) to afford methyl 3-((4-bromo-2-nitrophenyl)(ethyl)amino)propanoate (6.53 g, 85% yield) as a red oil. ES *m*/*z* = 333.1 [M+H]⁺.

### Step B

To a stirred solution of 3-((4-bromo-2-nitrophenyl)(ethyl)amino)propanoate (6.52 g, 19.688 mmol, 1 equiv) in methanol (60 mL) was added acetic acid (23.7 g, 394.6 mmol, 20 equiv) and zinc (6.4 g, 99 mmol, 5.0 equiv) in portions at room temperature. The resulting mixture was stirred for 2 hours at room temperature. The precipitated solids were collected by filtration and washed with MeOH (160 mL). The resulting filtrate was stirred overnight at 80 °C. The mixture was neutralized to pH 7 with aqueous saturated NaHCO₃. The precipitated solids were filtered off and washed with ethyl acetate (3 x 10 mL). The resulting mixture was extracted with ethyl acetate (3 x 50 mL). The combined organic layers were washed with brine (2 x 50 mL), dried over anhydrous sodium sulfate, filtered, and the filtrate was concentrated under reduced pressure. The crude residue was purified by silica gel column chromatography, eluting with petroleum ether/ethyl acetate (10:1 to 3:1) to afford 8-bromo-5-ethyl-1,3,4,5-tetrahydro-2H-benzo[b][1,4]diazepin-2-one (2.8 g, 50% yield) as a brown solid. ESI-MS *m*/*z* = 269.0 [M+H]⁺.

### L: 6-bromo-3-(3,6-dihydro-2H-pyran-4-yl)-1H-indole

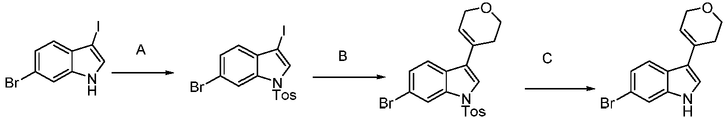

### Step A

To a stirred solution of NaH (60% dispersion in oil, 0.4 g, 16.67 mmol, 1.25 equiv) in DMF (40 mL) was added 6-bromo-3-iodo-1H-indole (4.3 g, 13.36 mmol, 1 equiv) dropwise at 0 °C. The resulting mixture was stirred for 1 hour at 0 °C and then 4-methylbenzene-1-sulfonyl chloride (5.6 g, 29.38 mmol, 2.2 equiv) was added dropwise at 0 °C. The mixture was stirred for an additional 16 hours at room temperature. The reaction was poured into ice water. The aqueous layer was extracted with ethyl acetate (3 x 100 mL). The combined organic layers were washed with brine (3 x 50 mL) and dried over anhydrous sodium sulfate. After filtration, the filtrate was concentrated under reduced pressure and 6-bromo-3-iodo-1-tosyl-1H-indole was used in the next step directly without further purification. ¹H NMR (300 MHz, CDCl₃) δ 8.17 (d, *J=* 1.5 Hz, 1H), 7.80 (d, *J =* 8.4 Hz, 2H), 7.67 (s, 1H), 7.44 (dd, *J= 8.4, 1.6* Hz, 1H), 7.30 (d, *J =* 8.4 Hz, 2H), 7.25 (d, *J =* 8.4 Hz, 1H), 2.40 (s, 3H).

### Step B

A solution of 6-bromo-3-iodo-1-tosyl-1H-indole (3.0 g, 6.30 mmol, 1 equiv), 2-(3,6-dihydro-2H-pyran-4-yl)-4,4,5,5-tetramethyl-1,3,2-dioxaborolane(4.0 g, 18.90 mmol, 3 equiv), Pd(dppf)Cl₂ (0.3 g, 0.41 mmol, 0.07 equiv), and K₂CO₃ (4.4 g, 31.84 mmol, 5.05 equiv) in dioxane (30 mL) and H₂O (6 mL) was stirred for 3 hours at 60 °C. The resulting mixture was extracted with ethyl acetate (3 x 50 mL). The combined organic layers were washed with brine (3 x 30 mL) and dried over anhydrous sodium sulfate. After filtration, the filtrate was concentrated under reduced pressure. The residue was purified by silica gel column chromatography, eluting with petroleum ether/ethyl acetate (10:1) to afford 6-bromo-3-(3,6-dihydro-2H-pyran-4-yl)-1-tosyl-1H-indole (2.1 g, 77% yield) as a yellow solid. ¹H NMR (300 MHz, CDCl₃) δ 8.21 (d, *J=* 1.7 Hz, 1H), 7.79 (d, *J =* 8.4 Hz, 2H), 7.64 (d, *J =* 8.6 Hz, 1H), 7.49 (s, 1H), 7.42-7.37 (m, 1H), 7.30 (s, 1H), 7.27 (s, 1H), 6.22 (s, 1H), 4.37 (q, *J =* 2.6 Hz, 2H), 3.98 (t, *J* = 5.5 Hz, 2H), 2.53 (dd, *J*= 4.8, 2.2 Hz, 2H), 2.39 (s, 3H).

### Step C

A solution of 6-bromo-3-(3,6-dihydro-2H-pyran-4-yl)-1-tosyl-1H-indole (2.1 g, 4.86 mmol, 1 equiv) and KOH (2.7 g, 48.12 mmol, 9.91 equiv) in MeOH (40 mL) and H₂O (10 mL) was stirred for 3 hours at 65 °C. The resulting mixture was extracted with ethyl acetate (3 x 50 mL) and the combined organic layers were washed with brine (3 x 10 mL) and dried over anhydrous sodium sulfate. After filtration, the filtrate was concentrated under reduced pressure to give 6-bromo-3-(3,6-dihydro-2H-pyran-4-yl)-1H-indole that was used in the next step directly without further purification. ESI-MS *m*/*z =* 278.0 [M+H]⁺.

The following intermediate was synthesized according to the procedure described to make Intermediate L using appropriate building blocks and modified reaction conditions (such as reagents, ratio of reagents, temperature, and reaction time) as needed.

| **Intermediate No.** | **Structure** | **Analytical Data** |
|---|---|---|
| L-1 | 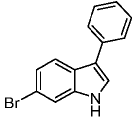 | ESI-MS *m*/*z* = 272.2 [M+H]⁺ |

### M: 6-bromo-2-(2-(methoxymethyl)phenyl)-1H-indole

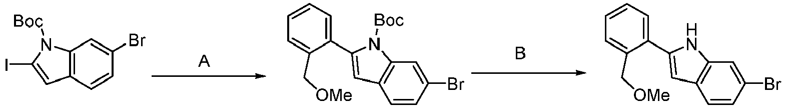

### Step A

A solution of (2-(methoxymethyl)phenyl)boronic acid (1.66 g, 10.0 mmol, 1.0 equiv), *tert*-butyl 6-bromo-2-iodo-1H-indole-1-carboxylate (4.2 g, 10.0 mmol, 1.0 equiv), Pd(dppf)Cl₂•CH₂Cl₂ (408 mg, 0.5 mmol, 0.05 equiv), and K₂CO₃ (4.14 g, 30 mmol, 3.0 equiv) in dioxane (20 mL) and water (4 mL) was stirred at 80 °C for 5 hours. After concentration, the residue was purified by silica gel chromatography (petroleum ether) to afford *tert*-butyl 6-bromo-2-(2-(methoxymethyl)phenyl)-1H-indole-1-carboxylate (2.95 g, 71% yield). ESI-MS *m*/*z* : 438.0 [M+Na]⁺.

### Step B

To a stirred solution of *tert*-butyl 6-bromo-2-(2-(methoxymethyl)phenyl)-1H-indole-1-carboxylate (2.95 g, 7.1 mmol, 1.0 equiv) in dichloromethane (10 mL) at 0 °C was added TFA (10 mL) dropwise. The resulting mixture was stirred for 1.5 hours at 15 °C and then concentrated under vacuum. The resulting mixture was diluted with water (100 mL). The mixture was basified to pH 8 with saturated Na₂CO₃. The resulting mixture was extracted with ethyl acetate (3 x 100 mL). The combined organic layers were washed with brine (200 mL) and dried over anhydrous sodium sulfate. After filtration, the filtrate was concentrated under reduced pressure. The crude product was purified by flash column on silica gel (petroleum ether/ethyl acetate (10:1)) to give 6-bromo-2-(2-(methoxymethyl)phenyl)-1H-indole (1.25 g, 55% yield) as a white solid. ESI-MS *m*/*z* : 316.0 [M+H]⁺.

### N: 6-bromo-3-cyclopropyl-1H-indole

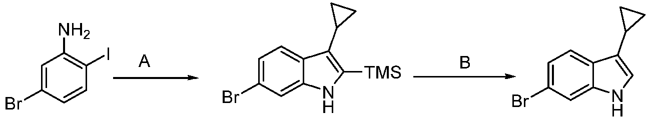

### Step A

A solution of 5-bromo-2-iodoaniline (5.0 g, 16.8 mmol, 1.0 equiv), Na₂CO₃ (4.5 g, 42.5 mmol, 2.5 equiv), Pd(PPh₃)₂Cl₂ (1.3 g, 2.0 mmol, 0.1 equiv ), and (cyclopropylethynyl)trimethylsilane (3.9 g, 28.3 mmol, 1.7 equiv) was stirred at 80 °C for 15 hours. The resulting mixture was diluted with ethyl acetate and washed with brine (3 x 40 mL). The organic phase was dried over anhydrous sodium sulfate and concentrated. The residue was applied onto a silica gel column with petroleum ether /ethyl acetate (20:1) to give 3.6 g (70% yield) of 6-bromo-3-cyclopropyl-2-(trimethylsilyl)-1H-indole as a yellow oil. ¹H NMR (300 MHz, DMSO-*d*₆) δ 10.66 (s, 1H), 7.58 - 7.38 (m, 2H), 7.05 (dd, *J =* 8.5, 1.8 Hz, 1H), 1.88 (tt, *J =* 8.4, 5.2 Hz, 1H), 0.98 - 0.84 (m, 2H), 0.72 - 0.59 (m, 2H), 0.39 (s, 9H).

### Step B

To a solution of 6-bromo-3-cyclopropyl-2-(trimethylsilyl)-1H-indole (1.8 g, 5.9 mmol, 1 equiv) in THF (18 mL) was added TBAF/THF (1 M). The resulting solution was stirred for 1 hour at 70 °C. After concentration, the residue was purified by silica gel chromatography (petroleum ether /ethyl acetate 1:1) to give 1.30 g (94% yield) of 6-bromo-3-cyclopropyl-1H-indole as a yellow solid. ¹H NMR (300 MHz, DMSO-*d*₆) δ 10.89 (s, 1H), 7.71 - 7.31 (m, 2H), 7.21 - 6.96 (m, 2H), 1.90 (ddd, *J =* 13.4, 8.5, 5.0 Hz, 1H), 0.95 - 0.73 (m, 2H), 0.58 (h, *J =* 3.7 Hz, 2H).

### O: 6-bromo-3-cyclobutyl-1H-indole

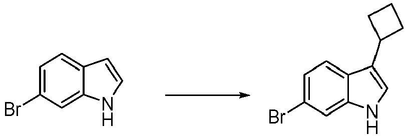

To a stirred solution 6-bromo-1H-indole (4.0 g, 20.40 mmol, 1 equiv) in toluene (20 mL) was added cyclobutanone (1.5 g, 21.40 mmol, 1.05 equiv) in portions. This solution was then added over the course of 30 minutes to a stirred solution of 2,2,2-trichloroacetic acid (5.0 g, 30.60 mmol, 1.50 equiv) and Et₃SiH (7.1 g, 61.06 mmol, 2.99 equiv) in toluene (20 mL) at 70 °C. The resulting mixture was stirred for an additional 16 hours at 70 °C at which point the mixture was concentrated under vacuum. The residue was basified to about pH 8 with 10% aqueous Na₂CO₃. The mixture was then extracted with ethyl acetate (3 x 200 mL). The combined organic layers were washed with water (100 mL) and saturated NaCl (100 mL) and dried over anhydrous sodium sulfate. After filtration, the filtrate was concentrated under reduced pressure. The residue was purified by silica gel chromatography, eluting with petroleum ether/dichloromethane (20:1) to afford 6-bromo-3-cyclobutyl-1H-indole(1.9 g, 32% yield) as a light-yellow solid. ESI-MS *m*/*z =* 250.3 [M+H].

### P: 2-bromo-5-methoxy-9H-carbazole

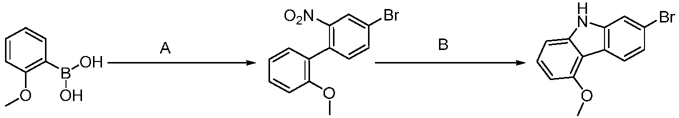

### Step A

A solution of (2-methoxyphenyl)boronic acid (1.0 g, 6.58 mmol, 1.0 equiv), 4-bromo-1-iodo-2-nitrobenzene (2.59 g, 7.90 mmol, 1.2 equiv), Pd(PPh₃)₂Cl₂ (100 mg, 0.142 mmol, 0.02 equiv), and K₂CO₃ (4.55 g, 32.9 mmol, 5.00 equiv) in dioxane (10 mL) and water (2 mL) was stirred for 15 hours at 60 °C. The mixture was concentrated under reduced pressure. The residue was purified by silica gel chromatography, eluting with petroleum ether/ethyl acetate (3:1) to afford 4-bromo-2'-methoxy-2-nitro-1,1'-biphenyl (1.3 g, 64% yield) as a yellow solid.

### Step B

A solution of 4-bromo-2'-methoxy-2-nitro-1,1'-biphenyl (1.2 g, 3.89 mmol, 1.0 equiv), PPh₃ (3.58 g, 13.63 mmol, 3.5 equiv), and 1,2-dichlorobenzene (10 mL). The reaction mixture was irradiated with microwave radiation for 12 hours at 180 °C. The crude product was purified by silica gel chromatography (petroleum ether/ethyl acetate, 100:1 to 10:1) to give 2-bromo-5-methoxy-9H-carbazole (890 mg, 83% yield) as a yellow solid. ESI-MS *m*/*z* = 276.1 [M+H]⁺.

The following intermediates were synthesized according to the procedure described to make Intermediate P using appropriate building blocks and modified reaction conditions (such as reagents, ratio of reagents, temperature, and reaction time) as needed.

| **Intermediate No.** | **Structure** |
|---|---|
| P-1 | 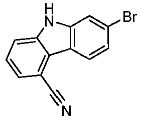 |
| P-2 | 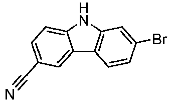 |

### Q: 6-bromo-3-(pyridin-2-ylmethyl)-1H-indole

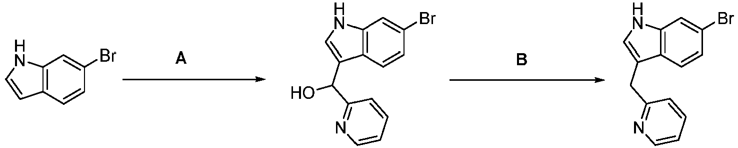

### Step A

To a stirred solution of 6-bromo-1H-indole (1.0 g, 5.10 mmol, 1.0 equiv) and pyridine-2-carbaldehyde (546 mg, 5.10 mmol, 1.0 equiv) in MeOH (10 mL) at 0 °C was added NaOH (224 mg, 5.61 mmol, 1.1 equiv) in portions. The resulting mixture was stirred for 1 hour at 0 °C and stirred for another 5 hours at room temperature. The resulting mixture was concentrated under reduced pressure and the mixture was diluted with water (30 mL). The aqueous layer was extracted with ethyl acetate (3 x 30 mL). The combined organic layers were dried over anhydrous sodium sulfate. After filtration, the filtrate was concentrated under reduced pressure. The crude 6-bromo-1H-indol-3-yl)(pyridin-2-yl)methanol (1.5 g) was used in the next step without further purification.

### Step B

A solution of crude (6-bromo-1H-indol-3-yl)(pyridin-2-yl)methanol (1.5 g, 4.948 mmol, 1.0 equiv) in dichloromethane (20 mL) was treated with TFA (6.2 g, 54.4 mmol, 11 equiv) followed by Et₃SiH (633 mg, 5.44 mmol, 1.10 equiv). The resulting solution was stirred for 2 hours at room temperature. The mixture was then concentrated under vacuum and 40 mL of water was added. The resulting solution was extracted with ethyl acetate (3 x 40 mL) and dried over anhydrous sodium sulfate. After filtering and concentrating *in vacuo,* the residue was purified by silica gel chromatography eluting with ethyl acetate/petroleum ether (1:4) to give 6-bromo-3-[(pyridin-2-yl)methyl]-1H-indole (1.1 g, 77% yield, 2 steps). ESI-MS *m*/*z* = 287.0 [M+H]⁺.

The following intermediates were synthesized according to the procedure described to make Intermediate Q using appropriate building blocks and modified reaction conditions (such as reagents, ratio of reagents, temperature, and reaction time) as needed.

| **Intermediate No.** | **Structure** | **Analytical Data** |
|---|---|---|
| Q-1 | 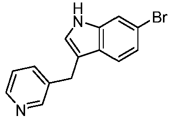 | ESI-MS *m*/*z* = 287.0 [M+H]⁺ |
| Q-2 | 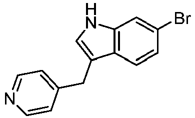 | ESI-MS *m*/*z* = 287.0 [M+H] ⁺ |

### R: 6-bromo-3-((tetrahydrofuran-3-yl)methyl)-1H-indole

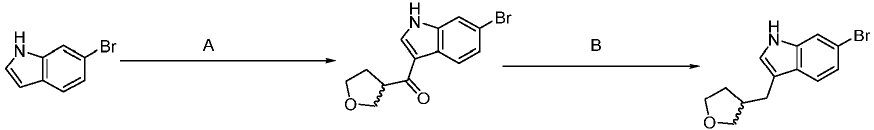

### Step A

To a solution of oxolane-3-carboxylic acid (4.39 g, 37.807 mmol, 1.21 equiv) in dichloromethane (45 mL) 0 °C was added oxalyl chloride (9.5 g, 74.847 mmol, 2.39 equiv) and N,N-dimethylformamide (0.150 mL) dropwise. The resulting mixture was stirred for 2 hours at 0 °C to 25 °C under an argon atmosphere and was then concentrated under vacuum. To a stirred solution of 6-bromo-1H-indole (6.14 g, 31.3 mmol, 1.0 equiv) in dichloromethane (70 mL) at 0 °C was added tetrachlorostannane (37.3 mL) dropwise. The resulting mixture was stirred for 10 minutes at 0 °C and then tetrahydrofuran-3-carbonyl chloride and nitromethane (3.37 mL) were dropwise at 0 °C. The resulting mixture was stirred for 15 hours at 0 °C to 25 °C. The reaction was quenched by the addition of ice water. The precipitated solids were collected by filtration and washed with ethyl acetate (3 x 10 mL). The filtrate was extracted with ethyl acetate (3 x 50 mL). The combined organic layers were washed with brine (1 x 100 mL) and dried over anhydrous sodium sulfate. After filtration, the filtrate was concentrated under reduced pressure. The residue was purified by silica gel chromatography, eluting with petroleum ether/ethyl acetate (10:1 to 2:1) to afford 6-bromo-3-(oxolane-3-carbonyl)-1H-indole (6.13 g, 52% yield) as a brown solid. ESI-MS *m*/*z* = 294.0 [M+H]⁺.

### Step B

To a stirred solution of 6-bromo-3-(oxolane-3-carbonyl)-1H-indole (6.0 g, 20.398 mmol, 1.0 equiv) was added 1 N BH₃ in THF (60 mL) dropwise. The mixture was stirred for 2 hours at room temperature. The mixture was quenched with MeOH (20 mL) at 0 °C. Water (100 mL) was added and the mixture was extracted with ethyl acetate (3 x 50 mL). The combined organic layers were washed with brine (100 mL) and dried over anhydrous sodium sulfate. After filtration, the filtrate was concentrated under reduced pressure. The residue was purified by silica gel chromatography, eluting with petroleum ether/ethyl acetate (20:1 to 6:1) to afford 6-bromo-3-[(oxolan-3-yl)methyl]-1H-indole (2.7 g, 44% yield ) as a brown oil. ESI-MS *m*/*z =* 282.0 [M+H]⁺.

### S: 3-(6-bromo-1H-indol-3-yl)propanenitrile

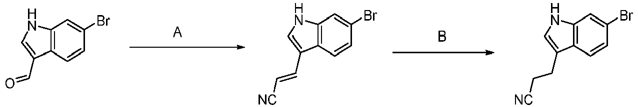

### Step A

A solution of 6-bromo-1H-indole-3-carbaldehyde (5.0 g, 22.3 mmol, 1 equiv) in THF (50 mL) at 0 °C was treated with NaH (60%, 535 mg, 22.3 mmol, 1.0 equiv) and maintained at that temperature for 30 minutes. Diethyl (cyanomethyl)phosphonate (7.91 g, 44.632 mmol, 2.0 equiv) was added dropwise and then the reaction mixture stirred overnight at room temperature. Water was added and the organics were removed under vacuum. The resulting aqueous layer was extracted with ethyl acetate (3 x 250 mL). The combined organic layers were washed with water (3 x 250 mL) and dried over anhydrous sodium sulfate. After filtration, the filtrate was concentrated under reduced pressure. The residue was purified by silica gel chromatography, eluting with petroleum ether/ethyl acetate (1:1) to afford (E)-3-(6-bromo-1H-indol-3-yl)acrylonitrile (2.5 g, 45% yield) as a yellow solid. ESI-MS *m*/*z* = 245.0 [M-H]⁻.

### Step B

To a solution of (E)-3-(6-bromo-1H-indol-3-yl)acrylonitrile (2.5 g, 10.1 mmol, 1.0 equiv) in THF (15 mL) and EtOH (15 mL) was added Pd/C (10%, 500 mg, 4.7 mmol, 0.46 equiv) and the reaction was stirred under a hydrogen atmosphere for 48 hours. The mixture was filtered and the filter cake was washed with EtOH (3 x 30 mL). The filtrate was concentrated under reduced pressure and the residue was purified by reverse phase chromatography (water/MeCN 45-50% with 0.1%FA) to afford 3-(6-bromo-1H-indol-3-yl)propanenitrile (1.1 g, 44% yield) as a dark yellow oil. ESI-MS *m*/*z =* 247.0 [M-H]⁻.

### T: 6-bromo-2-(pyridin-3-ylmethyl)-1H-indole

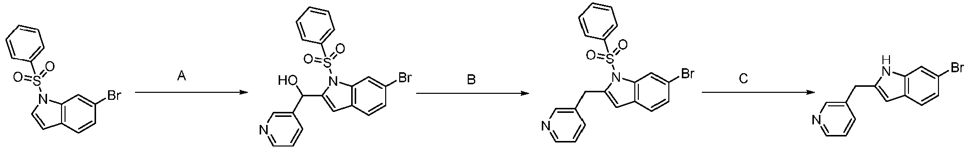

### Step A

6-bromo-1-(phenylsulfonyl)-1 H-indole (10.0 g, 29.8 mmol, 1.0 equiv) in THF (300 mL) at -78 °C was treated with LDA (2M in THF, 22.4 mL, 44.8 mmol, 1.5 equiv). The mixture was stirred at -78 °C for 0.5 hours and then pyridine-3-carbaldehyde (3.8 g, 35.8 mmol, 1.2 equiv) was added. The reaction mixture was stirred at -78 °C for 3 hours and then water (50 mL) was added. After adding additional water (1 L), the mixture was extracted with ethyl acetate (3 x 500 mL). The combined organics were washed with brine (500 mL) and dried over anhydrous sodium sulfate. After filtration, the filtrate was concentrated under reduced pressure. The crude product was purified by silica gel chromatography (petroleum ether/ethyl acetate = 1:2) to provide (6-bromo-1-(phenylsulfonyl)-1H-indol-2-yl)(pyridin-3-yl)methanol (10.8 g, 81% yield) as a yellow solid. ESI -MS m/z: 443.0 [M+H]⁺. ¹H NMR (400 MHz, DMSO-*d*₆) δ 8.58 (d, *J* = 1.9 Hz, 1H), 8.50 (dd, *J* = 4.8, 1.6 Hz, 1H), 8.15 - 8.08 (m, 1H), 7.90 - 7.80 (m, 2H), 7.73 - 7.65 (m, 2H), 7.63 - 7.52 (m, 3H), 7.43 (dd, *J* = 8.3, 1.7 Hz, 1H), 7.36 (dd, *J =* 7.8, 4.8 Hz, 1H), 6.75 (s, 1H), 6.42 (q, *J =* 5.5 Hz, 2H).

### Step B

To a solution of (6-bromo-1-(phenylsulfonyl)-1H-indol-2-yl)(pyridin-3-yl)methanol (10.2 g, 23.0 mmol, 1.0 equiv) in TFA (50 mL) was added Et₃SiH (50 mL). After stirring for 10 hours at 80 °C, the reaction solution was concentrated to dryness to give a residue. The residue was purified by silica gel chromatography (petroleum ether/ethyl acetate = 3:1) to give 6-bromo-1-(phenylsulfonyl)-1 H-indole (9.3 g, 95% yield) as a yellow solid. ESI-MS *m*/*z* = 427.0 [M+H]⁺. ¹H NMR (400 MHz, DMSO-*d*₆) δ 8.76 (d, *J* = 1.8 Hz, 1H), 8.71 (dd, *J* = 5.3, 1.3 Hz, 1H), 8.17 - 8.13 (m, 1H), 8.13 - 8.09 (m, 1H), 7.92 - 7.85 (m, 2H), 7.73 (ddd, *J =* 10.3, 5.0, 3.1 Hz, 2H), 7.65 - 7.56 (m, 2H), 7.49 (d, *J* = 8.3 Hz, 1H), 7.43 (dd, *J =* 8.3, 1.7 Hz, 1H), 6.52 (d, *J =* 0.6 Hz, 1H), 4.55 (s, 2H).

### Step C

To a solution of 6-bromo-1-(phenylsulfonyl)-1 H-indole (9.0 g, 21.1 mmol, 1.0 equiv) in MeOH (300 mL) and water (90 mL) was added KOH (23.6 g, 42.2 mmol, 2.0 equiv). After stirring for 16 hours at 90 °C, the reaction solution was concentrated to dryness to give a residue that was purified by silica gel chromatography (petroleum ether/ethyl acetate = 1:1) to give 6-bromo-2-(pyridin-3-ylmethyl)-1H-indole (4.98 g, 82% yield) as a yellow solid. ESI-MS *m*/*z* = 287.0 [M+H]⁺.

### U: 2-(6-bromo-1-(3-hydroxy-2,2-dimethylpropyl)-1H-indazol-3-yl)-2-methylpropanenitrile

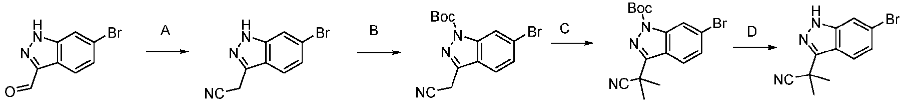

To a stirred solution of 6-bromo-1H-indazole-3-carbaldehyde (2.24 g, 9.95 mmol, 1.0 equiv) in formamide (50 mL) and MeOH (50 mL) was added NaBH₄ (1883 mg, 49.77 mmol, 5.0 equiv) portionwise. The resulting mixture was stirred for 2 hours at room temperature and then KCN (3.241 g, 49.7 mmol, 5.00 equiv) was added in portions. The resulting mixture was stirred for 16 hours at 60 °C and was then concentrated under reduced pressure. The residue was diluted with water (100 mL) and extracted with ethyl acetate (3 x 100 mL). The combined organic layers were washed with brine (3 x 100 mL), dried over anhydrous sodium sulfate, filtered, and the filtrate was concentrated under reduced pressure. The residue was purified by silica gel column chromatography, eluting with petroleum ether/ethyl acetate to afford 2-(6-bromo-1H-indazol-3-yl)acetonitrile (900 mg, 38% yield) as a yellow solid. ¹H NMR (400 MHz, DMSO-*d*₆) δ = 13.25 (s, 1H), 7.91 - 7.68 (m, 2H), 7.32 (dd, *J =* 8.7, 1.5 Hz, 1H), 4.40 (s, 2H).

### Step B

To a stirred solution/mixture of 2-(6-bromo-1H-indazol-3-yl)acetonitrile (1.1 g, 4.660 mmol, 1.0 equiv), TEA (0.71 g, 6.989 mmol, 1.5 equiv) and DMAP (57 mg, 0.466 mmol, 0.1 equiv) in dichloromethane (20 mL) was added Boc₂O (1.12 g, 5.13 mmol, 1.10 equiv) in portions at 0 °C. The resulting mixture was stirred for 3 hours at room temperature and then extracted with dichloromethane (3 x 30 mL). The combined organic layers were washed with brine (3 x 20 mL), dried over anhydrous sodium sulfate, filtered, and the filtrate was concentrated under reduced pressure. The residue was purified by Prep-TLC (petroleum ether/ethyl acetate (5:1)) to afford tert-butyl 6-bromo-3-(cyanomethyl)-1H-indazole-1-carboxylate (1.3 g, 83% yield) as a yellow solid. ¹H NMR (300 MHz, CDCl₃) δ = 8.43 (s, 1H), 7.75 (d, *J =* 8.5 Hz, 1H), 7.54 (dd, *J =* 8.6, 1.6 Hz, 1H), 4.11 (s, 2H), 1.75 (s, 9H).

### Step C

To a stirred solution of tert-butyl 6-bromo-3-(cyanomethyl)-1H-indazole-1-carboxylate (2.4 g, 7.139 mmol, 1 equiv) in THF(50 mL) was added LiHMDS (21 mL) dropwise at -78 °C under an argon atmosphere. The resulting mixture was stirred for 1 hour at -78 °C and then Mel (3.04 g, 21.418 mmol, 3.00 equiv) was added dropwise over 30 minutes at -78 °C. The resulting mixture was stirred for additional 16 hours at room temperature. The reaction was quenched with saturated aqueous NH₄Cl at 0 °C. The aqueous layer was extracted with ethyl acetate (3 x 100 mL). The combined organic layers were washed with brine (3 x 100 mL), dried over anhydrous sodium sulfate, filtered, and the filtrate was concentrated under reduced pressure. The residue was purified by reverse phase chromatography (MeCN in water (0.1% FA), 0% to 100%) to afford *tert*-butyl 6-bromo-3-(2-cyanopropan-2-yl)-1H-indazole-1-carboxylate (800 mg, crude) as a yellow solid.

### Step D

To a stirred solution of *tert*-butyl 6-bromo-3-(2-cyanopropan-2-yl)-1H-indazole-1-carboxylate (800 mg, 2.2 mmol, 1 equiv) in dichloromethane (12 mL) was added TFA (6 mL) in portions at 0 °C. The resulting mixture was stirred for 2 hours at 0 °C and then concentrated under vacuum. The residue was purified by reverse phase chromatography (MeCN in water (0.1% FA), 0% to 69 % gradient) to afford 2-(6-bromo-1H-indazol-3-yl)-2-methylpropanenitrile (500 mg, 86% yield) as a white solid. ESI-MS *m*/*z* = 264.4 [M+H]⁺

### V: (2-(bromomethyl)butoxy)(tert-butyl)diphenylsilane

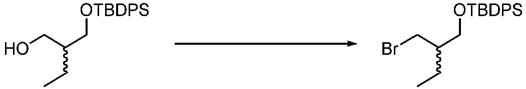

A solution of 2-(((*tert*-butyldiphenylsilyl)oxy)methyl)butan-1-ol (2.7 g, 7.9 mmol, 1 equiv) in dichloromethane (30 mL) was treated with PPh₃ (3.1 g, 12 mmol, 1.5 equiv). After cooling to 0 °C, CBr₄ (3.9 g, 12 mmol, 1.5 equiv) was added. The resulting solution was stirred for 1 hour at room temperature and then concentrated. The residue was purified by silica gel chromatography with petroleum ether to give (2-(bromomethyl)butoxy)(tert-butyl)diphenylsilane (2.7 g, 85% yield) as a light yellow oil. ¹H-NMR (300 MHz, DMSO-*d*₆) δ 7.68 - 7.58 (m, 4H), 7.54 - 7.38 (m, 6H), 3.66 (dqd, *J =* 16.5, 10.0, 5.4 Hz, 4H), 1.78 (hept, *J =* 6.0 Hz, 1H), 1.36 (dq, *J* = 14.1, 7.2 Hz, 2H), 1.01 (s, 9H), 0.84 (td, *J =* 7.6, 7.1, 1.8 Hz, 5H).

### W: 3-((tert-butyldiphenylsilyl)oxy)-2-fluoropropyl methanesulfonate

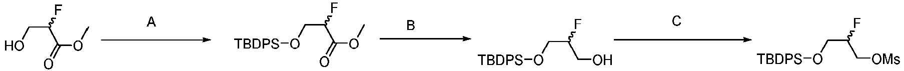

### Step A

A solution of methyl 2-fluoro-3-hydroxypropanoate (5.0 g, 41 mmol, 1.0 equiv) in dichloromethane (100 mL) was treated with imidazole (5.576 g, 82 mmol, 2.0 equiv) and TBDPS-Cl (12.33 g, 45 mmol, 1.1 equiv) at 0 °C. The solution was stirred for 2 hours at room temperature and then ice water (100 mL) was added. The solution was extracted with dichloromethane (2 x 100 mL) and the organic layers were combined and washed with brine (2 x 100 mL). The mixture was dried over anhydrous sodium sulfate. Purification by silica gel chromatography eluting with ethyl acetate/petroleum ether (1:20 to 1:5) gave methyl 3-(*tert*-butyldiphenylsilyloxy)-2-fluoropropanoate (16 g) as a white solid.

### Step B

A solution of methyl 3-(*tert*-butyldiphenylsilyloxy)-2-fluoropropanoate (8 g, 22.2 mmol, 1.0 equiv) in THF (100 mL) at 0 °C was treated with LiBH₄ (1.95 g, 88.8 mmol, 4.0 equiv). The solution was stirred for 15 hours at room temperature and then quenched with ice water (100 mL). After extraction with ethyl acetate (3 x 100 mL), the organic layers were combined. The solution was dried over anhydrous sodium sulfate. The residue was purified by silica gel chromatography eluting with ethyl acetate/petroleum ether (1:10-1:3) to give 3-((*tert*-butyldiphenylsilyl)oxy)-2-fluoropropan-1-ol (7.0 g, 95% yield) as a colorless oil. ¹H-NMR (300 MHz, DMSO-*d*₆) δ7.74-7.59 (m, 4H), 7.54-7.35 (m, 6H), 4.94 (t, *J =* 5.6 Hz, 1H), 4.74- 4.59 (m, 1H), 4.56-4.43 (m, 2H), 3.62 (m, 5.3 Hz, 2H), 1.01 (s, 9H).

### Step C

A solution of 3-((*tert*-butyldiphenylsilyl)oxy)-2-fluoropropan-1-ol (2 g, 6.024 mmol, 1 equiv) in dichloromethane (20 mL) at 0 °C was treated with Et₃N (1.22 g, 12.048 mmol, 2.0 equiv), DMAP (73 mg, 0.602 mmol, 0.05 equiv), and methanesulfonyl chloride (0.89 g, 7.831 mmol, 1.3 equiv). The solution was stirred for 3 hours at room temperature and then the crude product was purified by silica gel chromatography eluting with ethyl acetate/petroleum ether (1:10 to 1:3) to give 3-((*tert-*butyldiphenylsilyl)oxy)-2-fluoropropyl methanesulfonate (2.6 g, crude) as a yellow oil. ESI-MS *m*/*z =* 433.2 [M+Na]⁺.

### X: 3-bromo-2-(cyclopropylmethyl)propoxy)(tert-butyl)diphenylsilane

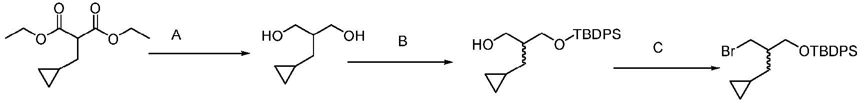

### Step A

To a stirred solution of 1,3-diethyl 2-(cyclopropylmethyl)propanedioate (2.2 g, 10.268 mmol, 1 equiv) in THF (40 mL) was added LiBH₄ (1.36 g, 62.43 mmol, 6.08 equiv) portionwise at 0 °C. The resulting mixture was stirred for 14 hours at 50 °C and then diluted with water (200 mL). After extraction with ethyl acetate (3 x 100 mL), the combined organic layers were washed with brine (20 mL) and dried over anhydrous sodium sulfate. After filtration, the filtrate was concentrated under reduced pressure. The crude product 2-(cyclopropylmethyl)propane-1,3-diol (1.3 g, crude) was used in the next step directly without further purification. ESI-MS *m*/*z =* 131.2 [M+H]⁺.

### Step B

To a stirred solution of 2-(cyclopropylmethyl)propane-1,3-diol (1.3 g, 9.99 mmol, 1 equiv) in THF (30 mL) was added NaH (480 mg, 12 mmol, 1.2 equiv, 60% dispersion in mineral oil) portionwise at 0 °C. The mixture was stirred for 1 hour at 0 °C and then TBDPSCI (2.87 g, 10.442 mmol, 1.05 equiv) was added dropwise over 15 minutes. The resulting mixture was stirred for an additional 1 hour at 0 °C and then concentrated under reduced pressure. The residue was diluted with water (200 mL) and then extracted with ethyl acetate (3 x 100 mL). The combined organic layers were washed with brine (50 mL) and dried over anhydrous sodium sulfate. After filtration, the filtrate was concentrated under reduced pressure. The residue was purified by silica gel chromatography, eluting with petroleum ether/EA (5:1) to afford 3-[(*tert*-butyldiphenylsilyl)oxy]-2-(cyclopropylmethyl)propan-1-ol (3.4 g, 65% yield) as a light-yellow oil. ESI-MS *m*/*z =* 369.2 [M+H]⁺.

### Step C

To a stirred solution of 3-[(*tert*-butyldiphenylsilyl)oxy]-2-(cyclopropylmethyl)propan-1-ol (3.4 g, 9.22 mmol, 1 equiv) in dichloromethane (40 mL) was added PPh₃ (9.7 g, 37.0 mmol, 4.0 equiv) and NBS (2.5 g, 14.1 mmol, 1.5 equiv) portionwise at 0 °C. The resulting mixture was stirred for 14 hours at room temperature and then concentrated under reduced pressure. The resulting mixture was filtered and the filter cake was washed with petroleum ether (3 x 100 mL). The filtrate was concentrated under reduced pressure and the residue was purified by silica gel chromatography, eluting with 100% petroleum ether to afford [3-bromo-2-(cyclopropylmethyl)propoxy](*tert*-butyl)diphenylsilane (2.3 g, 55% yield) as a colorless oil. ESI-MS *m*/*z =* 431.1 [M+H]⁺.

### Y: (3-bromo-2-(cyclopropylmethyl)propoxy)(tert-butyl)diphenylsilane

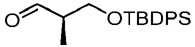

To a stirred solution of oxalyl chloride (580 mg, 4.570 mmol, 1.5 equiv) in dichloromethane (10.0 mL) was added DMSO (714 mg, 9.1 mmol, 3.0 equiv) dropwise at -78 °C. The resulting mixture was stirred for 30 minutes at -78 °C under a nitrogen atmosphere. To the above mixture was added (2S)-3-[(*tert*-butyldiphenylsilyl)oxy]-2-methylpropan-1-ol (1.00 g, 3.044 mmol, 1.0 equiv) dropwise over 10 min at -78 °C. The resulting mixture was stirred for an additional 30 minutes at -78 °C. To the above mixture was added TEA (1.23 g, 12.155 mmol, 3.99 equiv) dropwise over 10 min at -78 °C. The resulting mixture was stirred for an additional 30 minutes at -78 °C and then warmed to room temperature. The mixture was diluted with water (100 mL). The resulting mixture was extracted with dichloromethane (3 x 50 mL). The combined organic layers were washed with brine (50 mL) and dried over anhydrous sodium sulfate. After filtration, the filtrate was concentrated under reduced pressure. The residue was purified by silica gel column chromatography, eluting with petroleum ether/ethyl acetate (5:1) to afford (2R)-3-[(*tert-*butyldiphenylsilyl)oxy]-2-methylpropanal (930 mg, 84% yield) as a colorless oil. ¹H-NMR (300 MHz, DMSO-*d*₆) δ 9.69 (d, *J* = 1.3 Hz, 1H), 7.64 - 7.57 (m, 4H), 7.49 - 7.41 (m, 6H), 4.02 - 3.78 (m, 2H), 2.63 (qddd, *J =* 7.0, 5.8, 4.6, 1.4 Hz, 1H), 1.04 (d, *J =* 7.0 Hz, 3H), 0.98 (s, 9H).

### Z: 6-bromo-1-((1-(hydroxymethyl)cyclopropyl)methyl)-1H-indole-3-carbonitrile

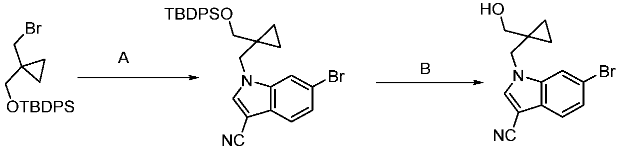

### Step A

A solution of 6-bromo-1H-indole-3-carboxamide (1.2 g, 5.0 mmol, 1.0 equiv), K₂CO₃ (1.38 g, 10.0 mmol, 2.0 equiv), KI (0.83 g, 5.0 mmol, 1.0 equiv), and ((1-(bromomethyl)cyclopropyl)methoxy)(*tert-*butyl)diphenylsilane (2.2 g, 5.5 mmol, 1.1 equiv) in DMSO (15 mL) was stirred at 150 °C overnight. The reaction solvent was cooled to 15 °C and ice water (100 mL) was added. The resulting solution was extracted with ethyl acetate (3 x 100 mL). The combined organic layers were dried over anhydrous sodium sulfate, filtered, and concentrated under reduced pressure to give a crude product that was purified by silica gel chromatography (100% dichloromethane to dichloromethane/MeOH = 20:1) to give 6-bromo-1-((1-(((*tert*-butyldiphenylsilyl)oxy)methyl)cyclopropyl)methyl)-1H-indole-3-carbonitrile (2.18 g, 80% yield) as a clear oil.

### Step B

To a solution of 6-bromo-1-((1-(((*tert*-butyldiphenylsilyl)oxy)methyl)cyclopropyl)methyl)-1H-indole-3-carbonitrile (2.18 g, 4.0 mmol, 1.0 equiv) in THF (20 mL) was added TBAF (8.0 mL, 1 M in THF) dropwise at 0 °C. The mixture was stirred at 0 °C for 2 hours. The mixture was poured into water and extracted with ethyl acetate (200 mL). The organic phase was washed with brine (50 mL x 3), dried over anhydrous sodium sulfate, and concentrated to give a crude residue that was purified by silica gel chromatography (petroleum ether to petroleum ether/ethyl acetate = 3:1) to give 6-bromo-1-((1-(hydroxymethyl)cyclopropyl)methyl)-1H-indole-3-carbonitrile (1.05 g, 86% yield) as a white solid. ESI-MS *m*/*z* = 307.0 [M+H]⁺.

The following compounds were synthesized according to the procedure described to make Intermediate Z using appropriate building blocks and modified reaction conditions (such as reagents, ratio of reagents, temperature, and reaction time) as needed.

| **Intermediate No.** | **Structure** | **Analytical Data** |
|---|---|---|
| Z-1 | 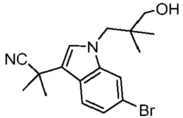 | ESI-MS *m*/*z* = 349.1 [M+H]⁺; ¹H-NMR (300 MHz, DMSO-*d*₆) δ 7.89 (d, *J* = 1.7 Hz, 1H), 7.68 (d, *J =* 8.5 Hz, 1H), 7.32 (s, 1H), 7.23 (dd, *J =* 8.5, 1.7 Hz, 1H), 4.94 (t, *J* = 4.9 Hz, 1H), 4.01 (s, 2H), 3.11 (d, *J =* 4*.*9 Hz, 2H), 1.76 (s, 6H), 0.84 (s, *J* = 6H). |
| Z-2 | 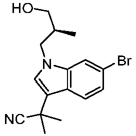 | ESI-MS *m*/*z* = 335.3, 337.3 [M+H]⁺ |
| Z-3 | 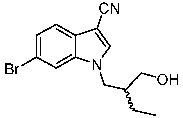 | ¹H-NMR (300 MHz, DMSO-*d*₆) δ 8.31 (s, 1H), 7.98 (d, *J* = 1.7 Hz, 1H), 7.61 (d, *J =* 8.4 Hz, 1H), 7.40 (dd, *J =* 8.5, 1.7 Hz, 1H), 4.69 (t, *J =* 4.9 Hz, 1H), 4.36 - 4.09 (m, 2H), 3.28 (d, *J =* 9.9 Hz, 2H), 1.86 (t, *J =* 6.3 Hz, 1H), 1.39 - 1.13 (m, 2H), 0.85 (t, *J* = 7.4 Hz, 3H). |
| Z-4 | 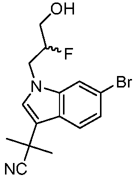 | ES-MS *m*/*z* = 339.1 [M+H]⁺ |
| Z-5 | 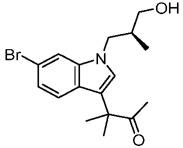 | ESI-MS *m*/*z* = 352.0[M+H]⁺ |
| Z-6 | 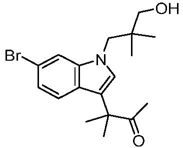 | |
| Z-7 | 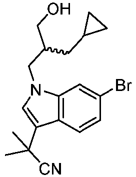 | ESI-MS *m*/*z* = 375.0 [M+H]⁺ |
| Z-8 | 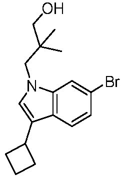 | ESI-MS *m*/*z* = 336.1 [M+H]⁺ |
| Z-9 | 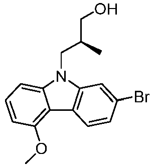 | ESI-MS *m*/*z* = 348.1 [M+H]⁺ |
| Z-10 | 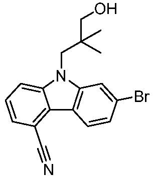 | ESI-MS *m*/*z* = 357.1 [M+H]⁺ |
| Z-11 | 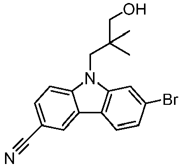 | |
| Z-12 | 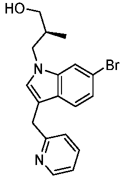 | ESI-MS *m*/*z* = 359.0 [M+H]⁺ |
| Z-13 | 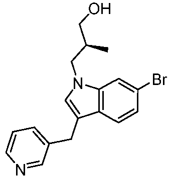 | ESI-MS *m*/*z* = 359.1 [M+H]⁺ |
| Z-14 | 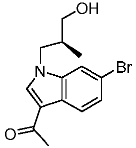 | ESI-MS *m*/*z* = 310.0 [M+H]⁺ |
| Z-15 | 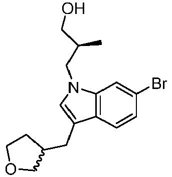 | (ESI *m*/*z):* 352.2 [M+H]⁺; ¹H-NMR (300 MHz, DMSO-*d*₆) δ 7.67 (d, *J* = 1.8 Hz, 1H), 7.50 (d, *J =* 8.4 Hz, 1H), 7.19 (s, 1H), 7.12 (dd, *J =* 8.4, 1.7 Hz, 1H), 4.65 (t, *J =* 5.2 Hz, 1H), 4.14 (dd, *J =* 14.2, 6.4 Hz, 1H), 3.92 (dd, *J =* 14.1, 7.5 Hz, 1H), 3.71 (ddt, *J* = 23.4, 15.2, 7.8 Hz, 3H), 3.37 (d, *J =* 8.2 Hz, 2H), 3.28 - 3.18 (m, 2H), 2.71 (d, *J =* 7.5 Hz, 2H), 2.56 (d, *J* = 7.6 Hz, 1H), 1.98 (ddt, *J* = 18.1, 12.8, 6.2 Hz, 2H), 1.56 (dq, *J* = 13.8, 7.1 Hz, 1H), 0.78 (d, *J* = 6.8 Hz, 3H). |
| Z-16 | 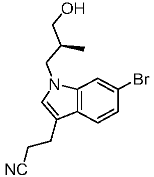 | ESI-MS *m*/*z* = 321.1 [M+H]⁺ |
| Z-17 | 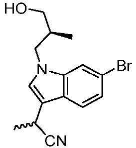 | |
| Z-18 | 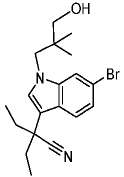 | ESI-MS *m*/*z* = 377.1 [M+H]⁺ |
| Z-19 | 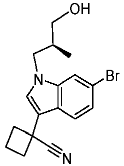 | ¹H-NMR (300 MHz, CDCl₃) δ 7.57 (dd, *J =* 5.1, 3.3 Hz, 2H), 7.31 - 7.22 (m, 1H), 7.07 (s, 1H), 4.21 (dd, *J* = 14.4, 6.9 Hz, 1H), 3.93 (dd, *J* = 14.4, 7.1 Hz, 1H), 3.52 (dd, *J =* 5.3, 3.5 Hz, 2H), 2.90 (dddt, *J =* 11.3, 9.3, 5.1, 2.3 Hz, 2H), 2.77 - 2.60 (m, 2H), 2.42 (dtt, J= 11.7, 9.1, 7.5 Hz, 1H), 2.30 - 2.09 (m, 2H), 0.98 (d, J = 6.9 Hz, 3H). |
| Z-20 | 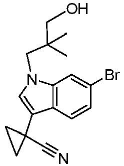 | ESI-MS *m*/*z* = 347.1 [M+H]⁺; ¹H-NMR (400 MHz, DMSO-*d*₆) δ 7.88 (d, *J* = 1.5 Hz, 1H), 7.62 (d, *J =* 8.4 Hz, 1H), 7.37 (s, 1H), 7.25 (dd, *J =* 8.4, 1.7 Hz, 1H), 4.93 (t, *J* = 4.9 Hz, 1H), 3.97 (s, 2H), 3.09 (d, *J =* 5.0 Hz, 2H), 1.65 (q, *J =* 4.5 Hz, 2H), 1.35 (q, *J* = 4.8 Hz, 2H), 0.82 (s, 6H). |
| Z-21 | 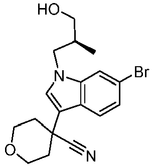 | ESI-MS *m*/*z* = 377.0 [M+H]⁺ |
| Z-22 | 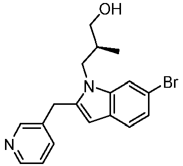 | ESI-MS m/z = 359.1 [M+H]⁺ |
| Z-23 | 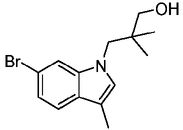 | ESI-MS *m*/*z* = 296.1 [M+H]⁺ |
| Z-24 | 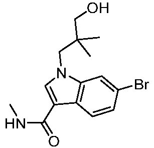 | ESI-MS *m*/*z* = 339.1 [M+H]⁺. |
| Z-25 | 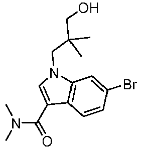 | ESI-MS *m*/*z* = 353.1[M+H]⁺ |
| Z-26 | 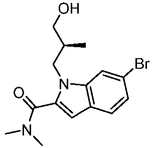 | ESI-MS *m*/*z* = 341.2 [M+H]⁺ |
| Z-27 | 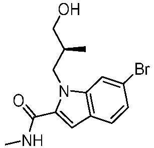 | ESI-MS *m*/*z* = 325.1 [M+H]⁺ |
| Z-28 | 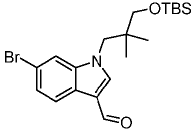 | ESI-MS *m*/*z* = 424.2 [M+H]⁺ |
| Z-29 | 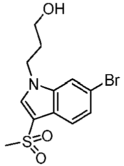 | ESI-MS *m*/*z* = 332.0 [M+H]⁺ |
| Z-30 | 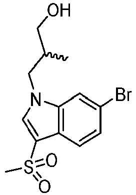 | ESI-MS *m*/*z* = 346.0 [M+H]⁺ |
| Z-31 | 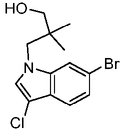 | ESI-MS *m*/*z* = 316.0 [M+H]⁺ |
| Z-32 | 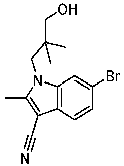 | ESI-MS *m*/*z* = 353.1 [M+Na]⁺; ¹H-NMR (400 MHz, CDCl₃) δ 7.75 (d, *J =* 3.4 Hz, 1H), 7.49 (d, *J =* 8.4 Hz, 1H), 7.33 (dd, *J =* 8.4, 1.6 Hz, 1H), 4.07 (s, 2H), 3.38 (s, 2H), 2.62 (s, 3H), 1.73 (s, 1H), 0.97 (s, 6H) |
| Z-33 | 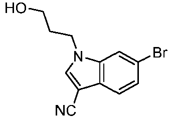 | ESI-MS *m*/*z* = 279.1 [M+H]⁺ |
| Z-34 | 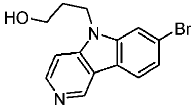 | ESI-MS *m*/*z* = 304.9 [M+H]⁺ |
| Z-35 | 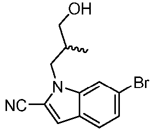 | ¹H-NMR (300 MHz, CDCl₃): δ = 7.66 (s, 1H), 7.54 (d, *J* = 8.6 Hz, 1H), 7.32 (dd, *J* = 8.5, 1.6 Hz, 1H), 7.16 (d, *J =* 0.9 Hz, 1H), 4.45 (dd, *J =* 14.7, 7.0 Hz, 1H), 4.11 (dd, *J =* 14.7, 7.5 Hz, 1H), 3.58 (dd, *J =* 5.3, 2.6 Hz, 2H), 2.42-2.28 (m, 1H), 1.02 (d, *J =* 6.9 Hz, 3H). |
| Z-36 | 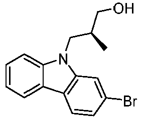 | ESI-MS *m*/*z* = 318.1 [M+H]⁺. |
| Z-37 | 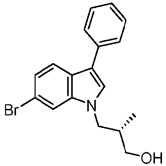 | ¹H-NMR (300 MHz, Methanol-*d*₄) δ 7.78 (d, *J* = 8.6 Hz, 1H), 7.69 (d, *J* = 1.7 Hz, 1H), 7.67 - 7.58 (m, 2H), 7.49 - 7.38 (m, 3H), 7.25 (tt, *J* = 8.5, 1.5 Hz, 2H), 4.31 (dd, *J =* 14.3, 6.5 Hz, 1H), 4.03 (dd, *J =* 14.3, 7.7 Hz, 1H), 3.54 - 3.39 (m, 2H), 2.23 (dq, *J* = 13.0, 6.5 Hz, 1H), 0.95 (d, *J =* 6.9 Hz, 3H). |
| Z-38 | 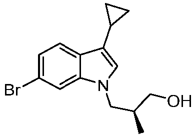 | ¹H-NMR (300 MHz, DMSO-*d*₆) δ 7.65 (d, *J* = 1.7 Hz, 1H), 7.54 (d, *J =* 8.4 Hz, 1H), 7.12 (dd, *J* = 8.4, 1.6 Hz, 1H), 7.06 (s, 1H), 4.65 (t, *J* = 5.1 Hz, 1H), 4.09 (dd, *J* = 14.1, 6.5 Hz, 1H), 3.87(dd, *J* = 14.2, 7.5 Hz, 1H), 3.28 - 3.13 (m, 2H), 1.90 (td, *J* = 8.2, 4.0 Hz, 1H), 0.92 - 0.82 (m, 2H), 0.78 (d, *J* = 6.7 Hz, 3H), 0.61 - 0.52 (m, 2H). |
| Z-39 | 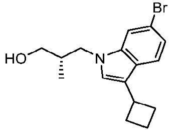 | ESI-MS *m*/*z* = 322.3 [M+H] |
| Z-40 | 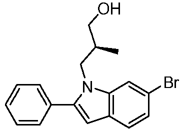 | ESI-MS *m*/*z* = 344.0[M+H]⁺ |
| Z-41 | 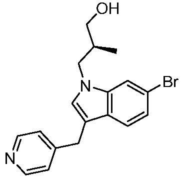 | ESI-MS *m*/*z* = 359.1 [M+H] |
| Z-42 | 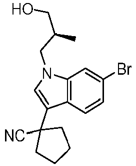 | ESI-MS *m*/*z* = 361.2 [M+H]⁺ |
| Z-43 | 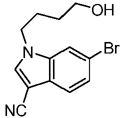 | ESI-MS *m*/*z* = 293.0 [M+H]⁺ |
| Z-44 | 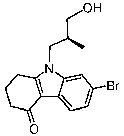 | |
| Z-45 | 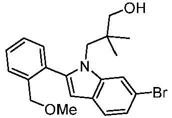 | ESI-MS *m*/*z* = 402.1 [M+H]⁺ |
| Z-46 | 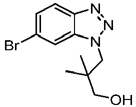 | ESI-MS *m*/*z* = 284.1, 286.1 [M+H]⁺. |
| Z-47 | 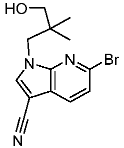 | ESI-MS *m*/*z* = 308.0[M+H]⁺ |
| Z-48 | 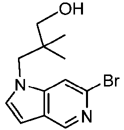 | ESI-MS *m*/*z* = 283.1 [M+H]⁺ |
| Z-49 | 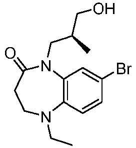 | ESI-MS *m*/*z* = 341.2 [M+H]⁺; ¹H-NMR (300 MHz, DMSO-*d*₆) δ 7.55 (d, *J =* 2.3 Hz, 1H), 7.38 (dd, *J =* 8.6, 2.3 Hz, 1H), 7.07 (d, *J =* 8.7 Hz, 1H), 4.31 (s, 1H), 3.20 (ddt, *J =* 31.3, 10.9, 5.7 Hz, 4H), 3.07 (s, 2H), 2.26 (d, *J =* 7.4 Hz, 2H), 1.55 (s, 1H), 1.02 (t, *J* = 7.1 Hz, 3H), 0.74 (d, *J* = 6.8 Hz, 3H) |
| Z-50 | 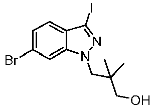 | ESI-MS *m*/*z* = 408.8 [M+H]⁺ |

### AA: 4-(3-hydroxypropoxy)-2-iodobenzo[b]thiophene-7-carbonitrile

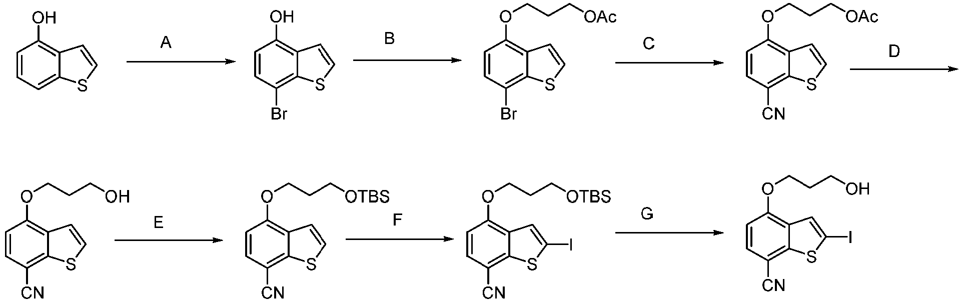

### Step A

To a stirred solution of 1-benzothiophen-4-ol (2.0 g, 13.32 mmol, 1.0 equiv) in acetonitrile (20 mL) at 0 °C was added N-bromosuccinimide (2.5 g, 14.05 mmol, 1.05 equiv) portionwise. The resulting mixture was stirred for 16 hours at room temperature. The resulting residue was purified by silica gel chromatography (ethyl acetate/petroleum ether (1:4)) to afford 7-bromo-1-benzothiophen-4-ol (2.0 g, 59 % yield) as a light yellow solid. ¹H-NMR (300 MHz, CDCl₃) δ 7.58 (d, *J =* 5.5 Hz, 1H), 7.46 (d, *J =* 5.5 Hz, 1H), 7.35 (d, *J =* 8.2 Hz, 1H), 6.68 (d, *J =* 8.2 Hz, 1H).

### Step B

A solution of 7-bromo-1-benzothiophen-4-ol (2.0 g, 8.73 mmol, 1 equiv), 3-bromopropyl acetate (1.89 g, 10.44 mmol, 1.2 equiv), and Cs₂CO₃ (4.29 g, 13.17 mmol, 1.51 equiv) in DMF (20 mL) was stirred for 16 hours at room temperature. The mixture was diluted with water (200 mL) and extracted with ethyl acetate (3 x 150 mL). The combined organic layers were washed with brine and dried over anhydrous sodium sulfate. After filtration, the filtrate was concentrated under reduced pressure and the residue was purified by silica gel column chromatography (ethyl acetate/petroleum ether (1:4)) to afford 3-[(7-bromo-1-benzothiophen-4-yl)oxy]propyl acetate (2.5 g, 78% yield) as a light yellow liquid. ¹H-NMR (300 MHz, CDCl₃)) δ 7.60 (d, *J =* 5.4 Hz, 1H), 7.46 - 7.36 (m, 2H), 6.68 (d, *J =* 8.3 Hz, 1H), 4.34 (t, *J =* 6.3 Hz, 2H), 4.21 (t, *J =* 6.1 Hz, 2H), 2.24 (h, *J =* 6.6 Hz, 2H), 2.08 (s, 3H).

### Step C

A solution of 3-[(7-bromo-1-benzothiophen-4-yl)oxy]propyl acetate (2.5 g, 7.59 mmol, 1.0 equiv), N,N-dimethylformamide (25 mL), Zn(CN)₂ (1.55 g, 15.07 mmol, 1.98 equiv), and Pd(PPh₃)₄ (1.76 g, 1.52 mmol, 0.2 equiv) was stirred for 16 hours at 130 °C. The resulting mixture was diluted with water (250 mL) and extracted with ethyl acetate (3 x 150 mL). The combined organic layers were washed with brine, dried over anhydrous sodium sulfate, filtered, and concentrated under reduced pressure. The residue was purified by silica gel column chromatography (ethyl acetate / petroleum ether 1:4) to afford 3-[(7-cyano-1-benzothiophen-4-yl)oxy]propyl acetate(1.6 g, 69% yield) as a yellow solid. ESI-MS *m*/*z =* 276.1 [M+H]⁺.

### Step D

To a stirred solution of 3-[(7-cyano-1-benzothiophen-4-yl)oxy]propyl acetate (1.6 g, 5.81 mmol, 1 equiv) in THF (16 mL) was added LiOH (698 mg, 29.2 mmol, 5.0 equiv) portionwise at 0 °C. The resulting mixture was warmed to room temperature and stirred for 16 hours at room temperature. The mixture was diluted with water (100 mL) and extracted with ethyl acetate (3 x 70 mL). The combined organic layers were washed with brine, dried over anhydrous sodium sulfate, filtered, and the filtrate was concentrated under reduced pressure to afford 4-(3-hydroxypropoxy)-1-benzothiophene-7-carbonitrile (1.5 grams, 99% yield) as a purple solid. ¹H-NMR (300 MHz, Methanol-*d*₄) δ 7.76 (d, *J =* 8.2 Hz, 1H), 7.67 (d, *J =* 5.5 Hz, 1H), 7.58 (d, *J =* 5.5 Hz, 1H), 7.03 (d, *J =* 8.3 Hz, 1H), 4.36 (t, *J =* 6.2 Hz, 2H), 3.83 (t, *J =* 6.2 Hz, 2H), 2.13 (p, *J =* 6.2 Hz, 2H).

### Step E

To a stirred solution of 4-(3-hydroxypropoxy)-1-benzothiophene-7-carbonitrile (1.5 g, 6.43 mmol, 1 equiv) in tetrahydrofuran (15 mL) at 0 °C was added NaH (60% dispersion in mineral oil, 387 mg, 9.68 mmol, 1.50 equiv) portionwise. The resulting mixture was stirred for 30 minutes at 0 °C at which point TBSCI (1.16 grams, 7.70 mmol, 1.20 equiv) was added portionwise. The resulting mixture was stirred for an additional 4 hours at room temperature. The mixture was then neutralized to pH 7.0 with saturated aqueous ammonium chloride. The resulting mixture was extracted with ethyl acetate and the combined organic layers were washed with brine and dried over anhydrous sodium sulfate. After filtration, the filtrate was concentrated under reduced pressure and the residue was purified by silica gel chromatography (ethyl acetate/petroleum ether (1:4)) to afford 4-[3-[(tert-butyldimethylsilyl)oxy]propoxy]-1-benzothiophene-7-carbonitrile (2.1 g, 75% yield) as a light yellow liquid. ¹H-NMR (300 MHz, CDCl₃) δ 7.68 (d, *J =* 8.2 Hz, 1H), 7.56 (d, *J =* 5.4 Hz, 1H), 7.48 (d, *J =* 5.5 Hz, 1H), 6.85 (d, *J =* 8.3 Hz, 1H), 4.31 (t, *J =* 6.2 Hz, 2H), 3.88 (t, *J* = 5.9 Hz, 2H), 2.12 (p, *J =* 6.1 Hz, 2H), 0.91 (s, 9H), 0.06 (s, 6H).

### Step F

To a stirred solution of 4-[3-[(tert-butyldimethylsilyl)oxy]propoxy]-1-benzothiophene-7-carbonitrile (800 mg, 2.30 mmol, 1 equiv) in THF was added lithium diisopropylamine in THF (1 M, 3.4 mL, 3.4 mmol, 1.50 equiv) dropwise at -60 °C. The resulting mixture was stirred for 1 hour at -30 °C and then N-iodosuccinimide (778 mg, 3.46 mmol, 1.50 equiv) was added in portions at -60 °C. The resulting mixture was stirred for an additional 16 hours at room temperature. The reaction was quenched by the addition of saturated aqueous ammonium chloride (200 mL). The resulting mixture was extracted with ethyl acetate (3 x 100mL). The combined organic layers were washed with water (1x 100 mL), dried over anhydrous sodium sulfate, filtered, and the filtrate was concentrated under reduced pressure. The residue was purified by silica gel column chromatography (ethyl acetate/petroleum ether (1:1)) to afford 4-[3-[(*tert-*butyldimethylsilyl)oxy]propoxy]-2-iodo-1-benzothiophene-7-carbonitrile (800 mg, 62% yield) as a yellow solid. ¹H-NMR (300 MHz, CDCl₃) δ 7.75 (d, *J =* 1.6 Hz, 1H), 7.60 (d, *J =* 8.3 Hz, 1H), 6.80 (d, *J =* 8.4 Hz, 1H), 4.29 (t, *J* = 6.3 Hz, 2H), 3.86 (t, *J* = 5.9 Hz, 2H), 2.11 (q, *J* = 7.4, 6.8 Hz, 2H), 0.91 (d, *J* = 1.8 Hz, 9H), 0.07 (s, *J =* 1.7 Hz, 6H).

### Step G

To a stirred solution of 4-[3-[(tert-butyldimethylsilyl)oxy]propoxy]-2-iodo-1-benzothiophene-7-carbonitrile (800 mg, 1.69 mmol, 1 equiv) in THF (10 mL) at 0 °C was added TBAF (1.0 M in THF, 2 mL) dropwise. After completion of the reaction as monitored by LC-MS, the mixture was concentrated under reduced pressure. The residue was purified by silica gel chromatography (ethyl acetate / petroleum ether 1:1) to afford 4-(3-hydroxypropoxy)-2-iodo-1-benzothiophene-7-carbonitrile (500 mg, 74.14% yield) as a yellow solid. ¹H-NMR (300 MHz, CDCl₃) δ 7.73 (s, 1H), 7.56 (t, *J =* 8.1 Hz, 1H), 6.79 (d, *J =* 8.4 Hz, 1H), 4.33 (t, *J =* 6.3 Hz, 2H), 4.02 - 3.84 (m, 2H), 2.17 (p, *J =* 6.2 Hz, 2H).

### AB: 3-(6-bromo-2-methyl-1H-benzo(djimidazol-1-yl)-2,2-dimethylpropan-1-ol

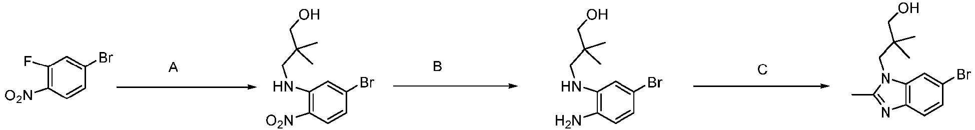

### Step A

To a stirred solution of 4-bromo-2-fluoro-1-nitrobenzene (1.0 g, 45.4 mmol, 1.0 equiv) at 0 °C in DMSO (5.0 mL) was added K₂CO₃ (1.25 g, 90.8 mmol, 2.0 equiv) followed by 3-amino-2,2-dimethylpropan-1-ol (0.70 g, 68.1 mmol, 1.5 equiv). The mixture was stirred for 2 hours at 25 °C. The solution was diluted with ethyl acetate (50 mL) and water (50 mL). The layers was separated and the organic layer was washed with water (2 × 30 mL), brine (20 mL), dried over anhydrous sodium sulfate, filtered, and the solvent was removed under reduced pressure to give 3-((5-bromo-2-nitrophenyl)amino)-2,2-dimethylpropan-1-ol (1.3 g, 95% yield) as a yellow solid. ESI-MS *m*/*z* = 303.0 [M+H]⁺. ¹H NMR (400 MHz, DMSO-*d*₆) d 8.59 (t, *J =* 4.7 Hz, 1H), 7.98 (d, *J =* 9.1 Hz, 1H), 7.31 (s, 1H), 6.80 (d, *J =* 9.1 Hz, 1H), 5.02 (t, *J* = 4.9 Hz, 1H), 3.28 (d, *J =* 4.9 Hz, 2H), 3.21 (d, *J =* 5.2 Hz, 2H), 0.93 (s, 6H).

### Step B

To a stirred suspension of 3-((5-bromo-2-nitrophenyl)amino)-2,2-dimethylpropan-1-ol (1.3 g, 4.3 mmol, 1.0 equiv) and iron powder (1.2 g, 21.5 mmol, 5.0 equiv) in ethanol (10 mL) and water (10 mL) was added NH₄Cl (690 mg, 12.9 mmol, 3.0 equiv). After stirring at 70 °C for 1 hour, the mixture was filtered. The filtrate was concentrated under reduced pressure to provide crude 3-((2-amino-5-bromophenyl)amino)-2,2-dimethylpropan-1-ol (1.2 grams) as a brown oil. ESI-MS *m*/*z* = 273.1 [M+H]⁺.

### Step C

A solution of 3-((2-amino-5-bromophenyl)amino)-2,2-dimethylpropan-1-ol (6.0 g, 22.0 mmol, 1.2 eq), 1,1,1-trimethoxyethane (20 mL) and concentrated hydrochloric acid (3.0 mL) was stirred at 25 °C for 16 hours. After concentration, the residue was purified by silica gel chromatography (petroleum/ethyl acetate = 3/1 to 1/1) to afford the 3-(6-bromo-2-methyl-1H-benzo[d]imidazol-1-yl)-2,2-dimethylpropan-1-ol (4.1 g, 63% yield) as an off-white solid. ESI-MS *m*/*z* = 297.1 [M+H]⁺. ¹H NMR (400 MHz, DMSO-*d*₆) d 7.89 (d, *J =* 1.7 Hz, 1H), 7.44 (d, *J =* 8.5 Hz, 1H), 7.25 (dd, *J =* 8.5, 1.8 Hz, 1H), 5.01 (t, *J =* 5.0 Hz, 1H), 4.04 (s, 2H), 3.15 (d, *J =* 5.0 Hz, 2H), 2.54 (s, 3H), 0.86 (s, 6H).

The following intermediates were synthesized according to the procedure described to make Intermediate AB using appropriate building blocks and modified reaction conditions (such as reagents, ratio of reagents, temperature, and reaction time) as needed.

| **Intermediate No.** | **Structure** | **Analytical Data** |
|---|---|---|
| AB-1 | 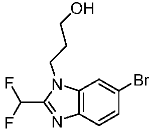 | ESI-MS *m*/*z* = 304.0 [M+H]⁺ |
| AB-2 | 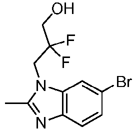 | ESI-MS *m*/*z* = 305.0 [M+H]⁺ |
| AB-3 | 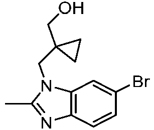 | ESI-MS *m*/*z* = 295.1 [M+H]⁺. |
| AB-4 | 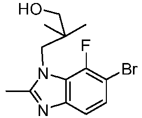 | ESI-MS *m*/*z* = 315.0 [M+H]⁺ |
| AB-5 | 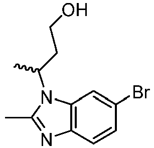 | ESI-MS *m*/*z* = 283.0 [M+H]⁺ |
| AB-6 | 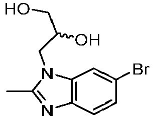 | ESI-MS *m*/*z* = 285.1 [M+H]⁺ |

### AC: 3-(6-bromo-2-(methoxymethyl)-1H-benzo[d]imidazol-1-yl)-2,2-dimethylpropan-1-ol

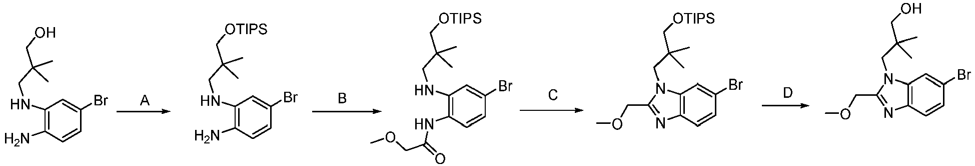

### Step A

To a stirred solution of 3-((2-amino-5-bromophenyl)amino)-2,2-dimethylpropan-1-ol (10.0 g, 37 mmol, 1.0 equiv), imidazole (12.6 g, 185 mmol, 5.0 equiv) and DMAP (22.0 g, 183 mmol, 5.0 equiv) in dichloromethane (250 mL) was added TIPSCI (35.0 g, 183 mmol, 5.0 equiv). The mixture was stirred for 48 hours and then the solution was poured into water (500 mL) and extracted with ethyl acetate (300 mL x 3). The combined organic layers were washed with water (300 mL x 2) and brine (300 mL), dried over sodium sulfate and purified by silica gel chromatography (cyclohexane/ethyl acetate: 1:3-2:1) to give 5-bromo-N¹-(2,2-dimethyl-3-((triisopropylsilyl)oxy)propyl)benzene-1,2-diamine (2.5 g, 37% yield) as a black oil. ESI-MS *m*/*z =* 429.2 [M+H]⁺.

### Step B

To a stirred solution of 5-bromo-N1-(2,2-dimethyl-3-((triisopropylsilyl)oxy)propyl)benzene-1,2-diamine and (10.0 g, 9.3 mmol, 1.0 equiv) and 2-methoxyacetic acid (922 mg, 10.2 mmol, 1.1 equiv) in DMF (50 mL) was added DIPEA (6.0 g, 46.5 mmol, 5.0 equiv) followed by HATU (5.3 g, 13.9 mmol, 1.5 equiv). The resulting solution was stirred for 1 hour and then the solution was diluted with ethyl acetate (20 mL) and water (20 mL). The layers was separated and the organic layer was washed with water (3 × 20 mL), brine (10 mL), dried over anhydrous sodium sulfate, filtered, and the solvent was removed under reduced pressure to give the *N*-(4-bromo-2-((2,2-dimethyl-3-((triisopropylsilyl)oxy)propyl)amino)phenyl)-2-methoxyacetamide (12.0 g) as an oil. The crude product was used in the next step without further purification. ESI-MS *m*/*z* = 501.3[M+H]⁺.

### Step C

A solution of N-(4-bromo-2-((2,2-dimethyl-3-((triisopropylsilyl)oxy)propyl)amino)-phenyl)-2-methoxyacetamide (10.5 g, 20.9 mmol, 1.0 equiv) in AcOH (110 mL) was stirred for 16 hours at 75 °C. After concentration, the crude product was purified by silica gel chromatography (petroleum ether:ethyl acetate (3:1)) to afford 6-bromo-1-(2,2-dimethyl-3-((triisopropylsilyl)oxy)propyl)-2-(methoxymethyl)-1H-benzo[d]imidazole (3.4 g, 57% yield) as a brown oil. ESI-MS *m*/*z* = 483.2 [M+H]⁺.

### Step D

A solution of 6-bromo-1-(2,2-dimethyl-3-((triisopropylsilyl)oxy)propyl)-2-(methoxymethyl)-1H-benzo[d]imidazole (3.4 g, 7.0 mmol, 1.0 eq) in HCl/MeOH (10 M, 18 mL) was stirred for 1 hour. After concentration, the crude product was washed with ether (20 mL) and then the product was filtered to give 3-(6-bromo-2-(methoxymethyl)-1H-benzo[d]imidazol-1-yl)-2,2-dimethylpropan-1-ol as a brown solid (1.5 g, 65% yield). ESI-MS *m*/*z* = 327.1[M+H]⁺.¹H NMR(400 MHz, DMSO-*d*₆) δ 7.99 (d, *J* = 1.7 Hz, 1H), 7.56 (d, *J =* 8.5 Hz, 1H), 7.32 (dd, *J =* 8.5, 1.8 Hz, 1H), 5.05 (t, *J =* 5.0 Hz, 1H), 4.72 (s, 2H), 4.16 (s, 2H), 3.31 (s, 3H), 3.14 (d, *J =* 5.0 Hz, 2H), 0.86 (s, 6H).

The following intermediate was synthesized according to the procedures described for intermediate AC using appropriate building blocks and modified reaction conditions (such as reagent ratio, temperature, coupling conditions, and reaction time) as needed.

| **Intermediate No.** | **Structure** | **Analytical Data** |
|---|---|---|
| AC-1 | 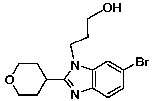 | ESI-MS *m*/*z* = 339.0 [M+H]+ |

### AD: 3-(6-bromo-2-methyl-1H-benzo[d]imidazol-1-yl)butan-1-ol

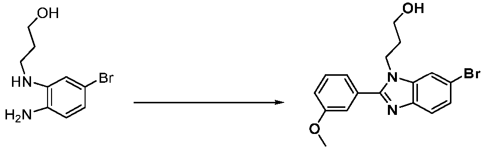

A solution of 3-((2-amino-5-bromophenyl) amino) propan-1-ol (3.0 g, 12.3 mmol, 1.0 equiv) and 3-methoxybenzaldehyde (1.7 g, 12.3 mmol, 1.0 equiv) was stirred in DMSO (15 mL) at 40 °C for 16 hours. Water (10 mL) was added and the solution was extracted with ethyl acetate (20 mL x 3). The combined organic layers were concentrated under reduced pressure to give a residue that was purified by silica gel chromatography (petroleum ether/ethyl acetate (3/1 to 1/1)) to afford the product of 3-(6-bromo-2-(3-methoxyphenyl)-1H-benzo[d]imidazol-1-yl)propan-1-ol (2.5 g, 57% yield) as a colorless oil. ESI-MS *m*/*z =* 361.0 [M+H]⁺.

The following Intermediates were synthesized according to the procedures described for the synthesis of intermediate AD using appropriate building blocks and modified reaction conditions (such as reagent ratio, temperature, coupling conditions, and reaction time) as needed

| **Intermediate No.** | **Structure** | **Analytical Data** |
|---|---|---|
| AD-1 | 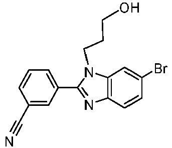 | ESI-MS *m*/*z* = 356.1 [M+H]+ |
| AD-2 | 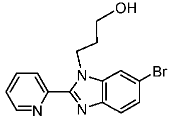 | ESI-MS *m*/*z* = 332.0 [M+H]+ |
| AD-3 | 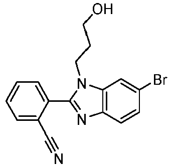 | ESI-MS *m*/*z* = 356.0 [M+H]+. |
| AD-4 | 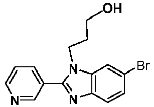 | ESI-MS *m*/*z* = 356.0 [M+H]+. |
| AD-5 | 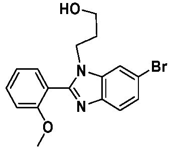 | ESI-MS *m*/*z* = 361.0 [M+H]+. |

### AE: (S)-2-(4-(2-chloroacetyl)-2-oxopiperazin-1-yl)-3-methylbutanoic acid

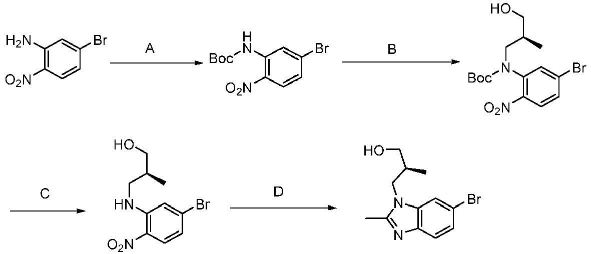

### Step A

A solution of 5-bromo-2-nitroaniline (1 g, 4.61 mmol, 1 equiv) in DMF (12 mL) at 0 °C was treated with NaH (60%, 222 mg, 9.25 mmol, 2.01 equiv). After 30 minutes, di-tert-butyl dicarbonate (1.2 g, 5.53 mmol, 1.2 equiv) was added. The resulting solution was stirred for 2 hours at 0 °C and then water was added. The solution was extracted with ethyl acetate (3 x 250 mL) and the organic layers were combined and dried over anhydrous sodium sulfate, filtered, and concentrated. The residue was purified by silica gel chromatography with ethyl acetate/petroleum ether (1:2) to give tert-butyl N-(5-bromo-2-nitrophenyl)carbamate (1.4 g, 96% yield) as a yellow solid. ESI-MS *m*/*z* = 316.9 [M+H]⁺.

### Step B

A solution of *tert*-butyl N-(5-bromo-2-nitrophenyl)carbamate (600 mg, 1.89 mmol, 1 equiv), (2*R*)-3-bromo-2-methylpropyl acetate (443 mg, 2.27 mmol, 1.2 equiv), MeCN (10 mL), KI (31 mg, 0.19 mmol, 0.1 equiv), and Cs₂CO₃ (1232.8 mg, 3.78 mmol, 2.0 equiv) was stirred for 15 hours at 65 °C. The solids were filtered off and the filtrate was concentrated. The residue was applied onto a silica gel column with ethyl acetate/petroleum ether (1:5) to give (2*S*)-3-[(5-bromo-2-nitrophenyl)[(*tert*-butoxy)carbonyl]amino]-2-methylpropyl acetate (580 mg, 64%) as a yellow oil.

### Step C

A solution of (2*S*)-3-[(5-bromo-2-nitrophenyl)[(*tert*-butoxy)carbonyl]amino]-2-methylpropyl acetate (580 mg, 1.34 mmol, 1 equiv), dichloromethane (6 mL) and TFA (3 mL) was stirred for 1 hour at 0 °C. The resulting mixture was concentrated to give (2S)-3-[(5-bromo-2-nitrophenyl)amino]-2-methylpropyl acetate (600 mg) as a red oil. ESI-MS *m*/*z =* 289.1 [M+H]⁺.

### Step D

A solution of (2*S*)-3-[(5-bromo-2-nitrophenyl)amino]-2-methylpropyl acetate (600 mg, 1.81 mmol, 1 equiv), CH₃COOH (3 mL), H₂O (3 mL, 166.53 mmol, 91.91 equiv), and zinc (592.3 mg, 9.06 mmol, 5 equiv) was stirred for 1 hour at 0 °C. After warming to room temperature and stirring for an additional 2 hours at 110 °C, the solution was neutralized to about pH 7 with aqueous Na₂CO₃. The resulting solution was extracted with ethyl acetate (3 x 80 mL), washed with brine, dried over anhydrous sodium sulfate, filtered, and concentrated. The residue was applied onto a silica gel column with ethyl acetate/petroleum ether (1:2) to give (2S)-3-(6-bromo-2-methyl-1H-1,3-benzodiazol-1-yl)-2-methylpropan-1-ol (260 mg, 51% yield) as a black oil. ESI-MS *m*/*z =* 283.1 [M+H]⁺.

### AF: (R)-3-(6-bromo-2-methyl-1H-benzo[d]imidazol-1-yl)-2-methylpropyl acetate

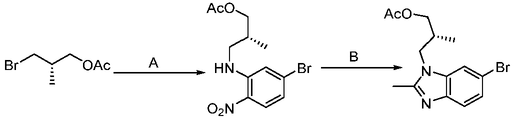

### Step A

To a stirred solution of *tert*-butyl (5-bromo-2-nitrophenyl)carbamate (630 mg, 1.99 mmol, 1.0 equiv), Cs₂CO₃ (1.3 g, 3.99 mmol, 1.0 equiv), and KI (67.4 mg, 0.41 mmol, 0.18 equiv) in MeCN (10 mL) was added (S)-3-bromo-2-methylpropyl acetate (440 mg, 2.26 mmol, 1.2 equiv) at room temperature. The resulting mixture was stirred for 3 hours at 65 °C and then concentrated under vacuum. The residue was purified by silica gel chromatography, eluting with petroleum ether/ethyl acetate (3:1) to afford (R)-3-((5-bromo-2-nitrophenyl)amino)-2-methylpropyl acetate (400 mg, 54% yield) as a light yellow oil. ESI-MS *m*/*z* = 331.0 [M+H]⁺.

### Step B

To a stirred solution of (*R*)-3-((5-bromo-2-nitrophenyl)amino)-2-methylpropyl acetate (400 mg, 1.21 mmol, 1 equiv) in acetic acid (5 mL) and water (5 mL) was added zinc (380 mg, 5.81 mmol, 4.81 equiv) at room temperature. The resulting mixture was stirred for 2 hours at 110 °C and then diluted with water (100 mL). The mixture was neutralized to pH 7 with aqueous saturated sodium bicarbonate. The resulting solution was extracted with ethyl acetate (2 x 100 mL). The combined organic layers were washed with brine (2 x 100 mL) and dried over anhydrous sodium sulfate. After filtration, the filtrate was concentrated under reduced pressure. The residue was purified by silica gel column chromatography, eluting with petroleum ether/ethyl acetate (1:1) to afford (*R*)-3-(6-bromo-2-methyl-1H-benzo[d]imidazol-1-yl)-2-methylpropyl acetate (280 mg, 64% yield) as an off-white solid. ESI-MS *m*/*z* = 325.1 [M+H]⁺.

### AG: 3-(6-bromo-2-methyl-1H-benzo[d]imidazol-1-yl)butan-1-ol

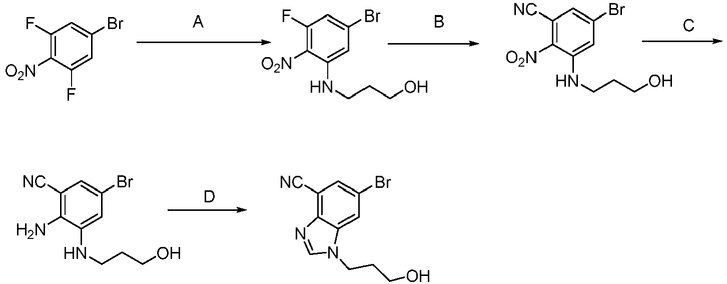

### Step A

A solution of 5-bromo-1,3-difluoro-2-nitrobenzene (5.0 g, 21 mmol, 1.0 equiv), 3-aminopropan-1-ol (1.6 g, 21 mmol, 2 equiv) and K₂CO₃ (8.7 g, 63 mmol, 3.0 equiv) in DMF (70 mL) was stirred for 3 hours at room temperature. The resulting solution was diluted with 100 mL of H₂O. The solution was extracted with ethyl acetate (3 x 100 mL). The organics were washed with brine (3 x 100 mL). The mixture was dried over anhydrous sodium sulfate and the residue was applied onto a silica gel column eluting with ethyl acetate/petroleum ether (3:2) to give 5.5 g (89% yield) of 3-((5-bromo-3-fluoro-2-nitrophenyl)amino)propan-1-ol as a yellow solid. ESI-MS *m*/*z* = 293.0 [M+H]⁺.

### Step B

3-[(5-bromo-3-fluoro-2-nitrophenyl)amino]propan-1-ol (2.5 g, 8.56 mmol, 1.0 equiv) and tetraethylammonium cyanide (1.6 g, 10.3 mmol, 1.2 equiv) were stirred in MeCN (30 mL) for 20 minutes at 55 °C. The resulting mixture was concentrated and the residue was applied onto a silica gel column eluting with ethyl acetate/petroleum ether (1:1) to give 2.6 g of 5-bromo-3-((3-hydroxypropyl)amino)-2-nitrobenzonitrile as a yellow solid. ESI-MS *m*/*z* = 300.0 [M+H]⁺.

### Step C

A solution of 5-bromo-3-[(3-hydroxypropyl)amino]-2-nitrobenzonitrile (900 mg, 3mmol, 1.0 equiv) and zinc (960 mg 15mmol, 5.0 equiv) in AcOH (9 mL) and H₂O (9 mL) was stirred for 1 hour at room temperature. The solution was basified to pH 8 with aqueous NaHCO₃. The resulting solution was extracted with ethyl acetate (3 x 20 mL), dried over anhydrous sodium sulfate and concentrated under vacuum to give 720 mg (89% yield) of 2-amino-5-bromo-3-((3-hydroxypropyl)amino)benzonitrile as brown oil and was used without further purification. ESI-MS *m*/*z =* 270.0 [M+H]⁺.

### Step D

A solution of 2-amino-5-bromo-3-[(3-hydroxypropyl)amino]benzonitrile (600 mg, 2.23mmol, 1.0 equiv) in formic acid (2 mL) and aqueous HCl (9 mL) was stirred for 2 hours at 110 °C. The solution was basified to pH 8 with aqueous Na₂CO₃. The resulting solution was extracted with ethyl acetate (3 x 20 mL), dried over anhydrous sodium sulfate, and concentrated under vacuum. The residue was applied onto a silica gel column eluting with ethyl acetate/petroleum ether (7:3) to give 376 mg (60% yield) of 6-bromo-1-(3-hydroxypropyl)-1H-benzo[d]imidazole-4-carbonitrile as a brown solid. ESI-MS *m*/*z* = 280.0 [M+H]⁺.

### AH: 3-(6-bromo-3-(3,6-dihydro-2H-pyran-4-yl)-1H-indol-1-yl)propyl acetate

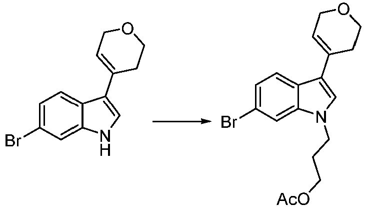

A solution of 6-bromo-3-(3,6-dihydro-2H-pyran-4-yl)-1H-indole (1.4 g, 4.89 mmol, 1.0 equiv), 3-bromopropyl acetate (1.2 g, 6.36 mmol, 1.3 equiv), and Cs₂CO₃ (3.2 g, 9.82 mmol, 2.01 equiv) in DMF (20 mL) was stirred for 16 hours at room temperature. The reaction was poured into water (100 mL) and then extracted with ethyl acetate (3 x 100 mL). The combined organic layers were washed with brine (5 x 50 mL) and dried over anhydrous sodium sulfate. After filtration, the filtrate was concentrated under reduced pressure. The residue was purified by prep-TLC (petroleum ether/ethyl acetate 4:1) to afford 3-(6-bromo-3-(3,6-dihydro-2H-pyran-4-yl)-1H-indol-1-yl)propyl acetate (1.7 g, 92% yield) as a yellow oil. ESI-MS *m*/*z* = 380.1 [M+H]⁺.

The following intermediates were synthesized according to the procedure described to make Intermediate AH using appropriate building blocks and modified reaction conditions (such as reagents, ratio of reagents, temperature, and reaction time) as needed.

| **Intermediate No.** | **Structure** | **Analytical Data** |
|---|---|---|
| AH-1 | 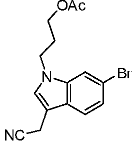 | ESI-MS *m*/*z* = 335.0, 337.0 [M+H]+ |
| AH-2 | 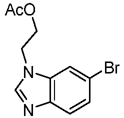 | ¹H NMR (300 MHz, DMSO-*d*₆) δ 7.93 (s, 1H), 7.70 (d, *J =* 8.4 Hz, 1H), 7.61 (d, *J =* 1.5 Hz, 1H), 7.42 (dd, *J =* 8.4, 1.5 Hz, 1H), 4.43-4.41 (m, 4H), 2.06 (s, 3H). |
| AH-3 | 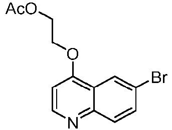 | ESI-MS *m*/*z* = 310.0 [M+H]+ |
| AH-4 | 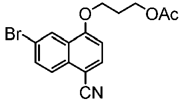 | |

### AI: 3-(6-bromo-1H-indazol-1-yl)-2,2-dimethylpropan-1-ol

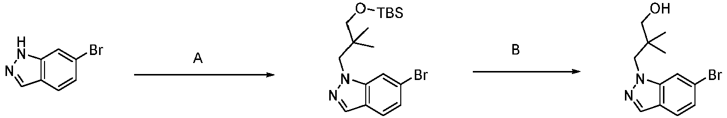

### Step A

A mixture of (3-bromo-2,2-dimethylpropoxy)(*tert*-butyl)dimethylsilane (3.7 g, 18.9 mmol, 1.0 equiv), 6-bromo-1H-indazole (7.9 mg, 28.3 mmol, 1.5 equiv), K₂CO₃ (5.2 mg, 37.8 mmol, 2.0 equiv) and KI (6.3 g, 37.8 mmol, 2.0 equiv) in DMF (20 mL) was stirred at 150 °C for 24 hours. The mixture was diluted with ethyl acetate (100 mL), then washed with water (2 x 50 mL) and brine (80 mL). The organic phase was collected, dried over sodium sulfate, filtered and concentrated to give a residue. The residue was purified by silica gel chromatography (ethyl acetate/petroleum ether (1:10)) to give 6-bromo-1-(3-((*tert*-butyldimethylsilyl)oxy)-2,2-dimethylpropyl)-1H-indazole (6.3 g, 84% yield) as a brown oil. ESI-MS *m*/*z* = 397.2[M+H]⁺.

### Step B

TBAF (8.8 g, 33.8 mmol, 2.0 eq, 1.0 M in THF) was add to a solution of 6-bromo-1-(3-((tertbutyldimethylsilyl)oxy)-2,2-dimethylpropyl)-1H-indazole (6.7 g, 16.9 mmol, 1.0 equiv) in THF (40 mL), and then the mixture was stirred at 20 °C for 6 hours. The mixture was diluted with EA (100 mL), then washed with water (20 mL x 6) and brine (80 mL). The organic phase was collected, dried over sodium sulfate, filtered and concentrated to give a residue that was purified by silica gel chromatography (ethyl acetate/petroleum ether (1:5)) to give *3*-(6-bromo-1H-indazol-1-yl)-2,2-dimethylpropan-1-ol (2.1 g, 44% yield) as a brown oil. ESI-MS *m*/*z* = 283.1 [M+H]⁺. ¹H-NMR (400 MHz, DMSO-*d*₆) δ 8.10 (d, 1H), 8.03 (s, 1H), 7.70 (s, 1H), 7.24 (m, 1H), 4.79 (s, 1H), 4.24 (s, 2H), 3.16 (d, 2H), 0.84 (s, 6H).

The following intermediates were synthesized according to Intermediate AI described above using appropriate building blocks and modified reaction conditions (such as ratio of reagents, temperature, and reaction time) as needed.

| **Intermediate No.** | **Structure** | **Analytical Data** |
|---|---|---|
| A-1 | 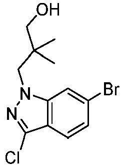 | ESI-MS *m*/*z* = 317.0[M+H]+ |
| A1-2 | 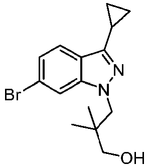 | ESI-MS *m*/*z* = 323.1 [M+H]+. |
| A1-3 | 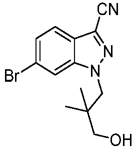 | ESI-MS *m*/*z* = 308.1 [M+H]+. |
| A1-4 | 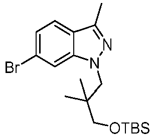 | ESI-MS *m*/*z* = 411.2 [M+H]+. |
| A1-5 | 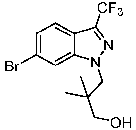 | ESI-MS *m*/*z* = 351.0 [M+H]+ |
| Al-6 | 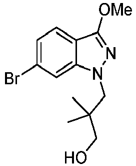 | H NMR (300 MHz, CDCl₃) δ 7.55-7.36 (m, 2H), 7.12 (s, 1H), 4.03 (s, 3H), 3.96 (s, 2H), 3.28 (s, 2H), 0.96 (s, 6H) |
| AI-7 | 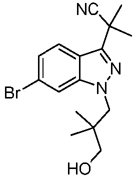 | ESI-MS *m*/*z* = 350.2 [M+H]+ ¹H-NMR (400 MHz, DMSO-*d*₆) δ = 8.08 (s, 1H), 7.89 (d, *J =* 8.7 Hz, 1H), 7.34 (dd, *J =* 8.7, 1.6 Hz, 1H), 4.80 - 4.75 (m, 1H), 4.23 (s, 2H), 3.16 (d, *J =* 5.2 Hz, 2H), 1.84 (d, *J =* 1.4 Hz, 6H), 0.85 (d, *J =* 1.4 Hz, 6H). |

### AJ: (S)-3-(6-bromo-3-(difluoromethyl)-1H-indazol-1-yl)-2-methylpropan-1-ol

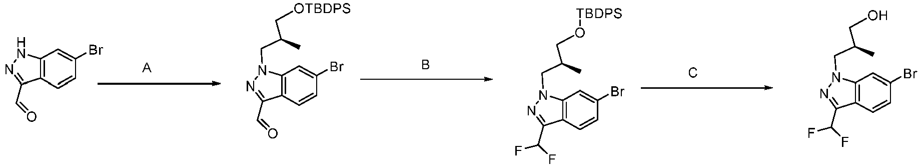

### Step A

To a 100 mL flask was added 6-bromo-1*H*-indazole-3-carbaldehyde (2.25 g, 10 mmol, 1 equiv) in DMSO (30 mL) was added (*R*)-(3-bromo-2-methylpropoxy)(*tert*-butyl)diphenylsilane (5.0 g, 13 mmol, 1.3 equiv), KI (1.66 g, 10 mmol, 1 equiv), and K₂CO₃ (2.76 g, 20 mmol, 2 equiv). The mixture was stirred at 150 °C for 16 hours. The mixture was cooled and poured into water (150 mL) and extracted with ethyl acetate (3 × 25 mL). The combined organic layers were washed with H₂O (50 mL), brine (3 x 25 mL), then dried over sodium sulfate and filtered. After concentration, the residue was purified by silica gel chromatography (petroleum ether) to afford (*S*)-6-bromo-1-(3-((*tert*-butyldiphenylsilyl)oxy)-2-methylpropyl)-1*H*-indazole-3-carbaldehyde (4.3 g, 80% yield) as a colorless oil. ¹H NMR (400 MHz, CDCl₃) δ 10.18 (s, 1H), 8.17 (d, *J =* 8.6 Hz, 1H), 7.75 (s, 1H), 7.63 (ddd, *J =* 15.5, 7.9, 1.3 Hz, 4H), 7.52 - 7.29 (m, 7H), 4.65 (dd, *J =* 13.8, 6.5 Hz, 1H), 4.28 (dd, *J =* 13.8, 7.3 Hz, 1H), 3.61 - 3.49 (m, 2H), 2.40 (tp, *J =* 13.4, 6.8 Hz, 1H), 1.12 (s, 9H), 0.95 (d, *J =* 6.9 Hz, 3H).

### Step B

To a solution of (*S*)-6-bromo-1-(3-((tert-butyldiphenylsilyl)oxy)-2-methylpropyl)-1*H*-indazole-3-carbaldehyde (3.6 g, 6.74 mmol, 1.0 equiv) in dichloromethane (30 mL) was added DAST (15 mL) at 20 °C. The solution was stirred at room temperature for 16 hours. The reaction was poured into ice water and extracted with ethyl acetate (3 x 30 mL). The organic layers were washed with water, brine, and dried over sodium sulfate. After filtration and concentration, the residue was purified by silica gel chromatography (petroleum/ethyl acetate (10:1)) to afford (*S*)-6-bromo-1-(3-((*tert*-butyldiphenylsilyl)oxy)-2-methylpropyl)-3-(difluoromethyl)-1*H*-indazole (2.1 g, 56% yield) as a colorless oil. ESI-MS *m*/*z* = 557.1 [M+H]⁺.

### Step C

To a solution of (*S*)-6-bromo-1-(3-((*tert*-butyldiphenylsilyl)oxy)-2-methylpropyl)-3-(difluoromethyl)-1*H*-indazole (2.1 g, 3.78 mmol, 1.0 equiv) in THF (15 mL) was added TBAF (1 M in THF, 3.8 mL, 3.8 mmol, 1.0 equiv) at 20 °C. The solution was stirred at room temperature for 0.5 hours. After concentration, the crude product was diluted with ethyl acetate (20 mL) and washed with water (5 x 5 mL). The organic layer was dried over anhydrous sodium sulfate and filtered. After concentration, the residue was purified by column chromatography (petroleum/ethyl acetate (1:1)) to afford 3-(6-bromo-3-(difluoromethyl)-1*H*-indazol-1-yl)-2-methylpropan-1-ol (1.0 g, 84% yield) as a white solid.

### AK: (S)-3-(6-bromo-3-(methoxymethyl)-1H-indazol-1-yl)-2-methylpropan-1-ol

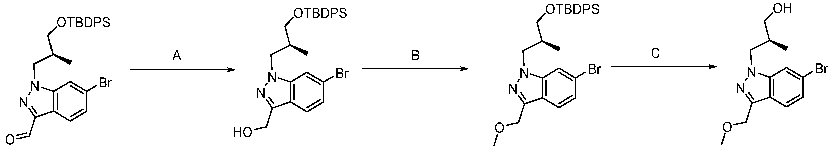

### Step A

To a stirred solution of (*S*)-6-bromo-1-(3-((*tert*-butyldiphenylsilyl)oxy)-2-methylpropyl)-1*H-*indazole-3-carbaldehyde (5.0 g, 9.3 mmol, 1.0 equiv) in MeOH (25 mL) and THF (50 mL) was added NaBH₄ (700 mg, 18.7 mmol, 2.0 equiv) at 15 °C. The mixture was stirred at 15 °C for 2 hours. The solution was poured into water (300 mL) and extracted with ethyl acetate (150 mL x 3). The combined organic layers were washed with water (200 mL x 2) and brine (200 mL x 1), dried over sodium sulfate and concentrated to give a residue. The residue was purified by silica gel chromatography (petroleum ether / ethyl acetate (5:1)) to give (*S*)-(6-bromo-1-(3-((*tert*-butyldiphenylsilyl)oxy)-2-methylpropyl)-1H-indazol-3-yl)methanol (4.3 g, 86% yield) as an oil. ESI-MS *m*/*z* = 537.3 [M+H]⁺.

### Step B

(*S*)-(6-bromo-1-(3-((*tert*-butyldiphenylsilyl)oxy)-2-methylpropyl)-1H-indazol-3-yl)methanol (500 mg, 0.93 mmol, 1.0 equiv) was dissolved in THF (10 mL) at 0 °C, and NaH (60% dispersion in oil, 74 mg, 1.86 mmol, 2.0 equiv) was added in portions. The mixture was stirred at 0 °C for 0.5 hours, then Mel (264 mg, 1.86 mmol, 2.0 equiv) was added. The reaction mixture was stirred at 0 °C to 15 °C for 16 hours, and then the reaction mixture was poured into 50 mL of ice water. The solution was extracted with ethyl acetate (30 mL x 3). The organic phase was concentrated to give a residue. The residue was purified by silica gel chromatography (petroleum ether/ethyl acetate (10:1)) to give (*S*)-6-bromo-1-(3-((*tert-*butyldiphenylsilyl)oxy)-2-methylpropyl)-3-(methoxymethyl)-1H-indazole (400 mg, 78% yield) as an oil. ESI-MS *m*/*z* = 551.3 [M+H]⁺. ¹H-NMR (400 MHz, CDCl₃) δ 7.70 - 7.57 (m, 6H), 7.45 - 7.31 (m, 6H), 7.27 - 7.23 (m, 1H), 4.77 (s, 2H), 4.50 (dd, *J =* 14.0, 6.6 Hz, 1H), 4.19 - 4.13 (m, 1H), 3.51 (qd, *J =* 10.3, 5.1 Hz, 2H), 3.37 (s, 3H), 2.34 (dt, *J =* 12.1, 6.2 Hz, 1H), 1.11 (s, 9H), 0.91 (d, *J =* 6.9 Hz, 3H).

### Step C

To a stirred solution of (*S*)-6-bromo-1-(3-((*tert*-butyldiphenylsilyl)oxy)-2-methylpropyl)-3-(methoxymethyl)-1H-indazole (4.5 g, 8.2 mmol, 1.0 equiv) in THF (30 mL) was added TBAF (1 M in THF, 16.4 mL, 16.4 mmol, 2.0 equiv) at 0 °C. The resulting mixture was stirred for 2 hours at 0 °C. After concentration, the crude product was diluted with ethyl acetate (250 mL) and washed with water (30 mL x 5). The organic layer was concentrated under reduced pressure to give a residue that was purified by flash column on silica gel (petroleum/ethyl acetate = 3:1) to give (*S*)-3-(6-bromo-3-(methoxymethyl)-1H-indazol-1-yl)-2-methylpropan-1-ol (2.5 g, 88% yield) as an oil. ESI-MS *m*/*z =* 313.0 [M+H]⁺.

### AL: 3-(6-bromo-3-(1-methyl-1,2,3,6-tetrahydropyridin-4-yl)-1H-indazol-1-yl)-2,2-dimethylpropan-1-ol

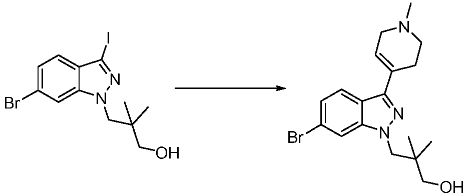

Into a 40 mL vial was placed 3-(6-bromo-3-iodo-1H-indazol-1-yl)-2,2-dimethylpropan-1-ol (700 mg, 1.71 mmol, 1 equiv), 1-methyl-4-(4,4,5,5-tetramethyl-1,3,2-dioxaborolan-2-yl)-1,2,3,6-tetrahydropyridine (458.2 mg, 2.05 mmol, 1.2 equiv), K₂CO₃ (591.2 mg, 4.28 mmol, 2.5 equiv), Pd(dppf)Cl₂ (150 mg, 0.21 mmol, 0.12 equiv), and dioxane/H₂O (10 mL). The resulting solution was stirred for 2 hours at 60 °C. The solids were filtered off and the resulting mixture was concentrated. The residue purified by silica gel chromatography with dichloromethane/methanol (15:1) to give 3-(6-bromo-3-(1-methyl-1,2,3,6-tetrahydropyridin-4-yl)-1H-indazol-1-yl)-2,2-dimethylpropan-1-ol (634 mg, 93% yield) as a dark yellow oil. ESI-MS *m*/*z =* 378.0 [M+H]⁺.

### AM: (S)-3-(6-bromo-3-(prop-1-yn-1-yl)-1H-indazol-1-yl)-2-methylpropan-1-ol

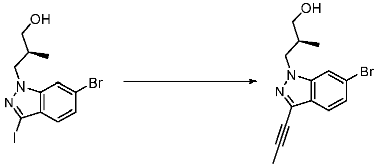

To a stirred solution of (2*S*)-3-(6-bromo-3-iodo-1H-indazol-1-yl)-2-methylpropan-1-ol (1.5 g, 3.80 mmol, 1 equiv) and tributyl(prop-1-yn-1-yl)stannane (1.38 g, 4.18 mmol, 1.1 equiv) in THF was added Pd(PPh₃)₄ (439 mg, 0.38 mmol, 0.1 equiv) and LiCl (483 mg, 11.4 mmol, 3.0 equiv) in portions. The resulting mixture was stirred for 2 hours and concentrated under vacuum. The residue was purified by silica gel chromatography, eluting with petroleum ether/ethyl acetate (3:1) to afford (2S)-3-[6-bromo-3-(prop-1-yn-1-yl)-1H-indazol-1-yl]-2-methylpropan-1-ol (680 mg, 53% yield) as a yellow oil. ESI-MS *m*/*z* = 307.1 [M+H]⁺.

### AN: 4-(6-bromo-1-(3-hydroxy-2,2-dimethylpropyl)-1H-indazol-3-yl)-2-methylbut-3-yn-2-ol

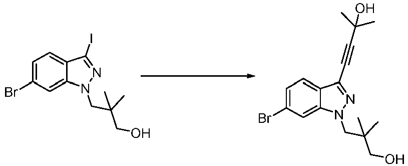

A solution of 3-(6-bromo-3-iodo-1H-indazol-1-yl)-2,2-dimethylpropan-1-ol (470 mg, 1.15 mmol, 1 equiv) in THF (16 mL) at 0 °C was treated with Et₃N (4 mL), 2-methylbut-3-yn-2-ol (116 mg, 1.38 mmol, 1.20 equiv), Cul (23 mg, 0.11 mmol, 0.10 equiv), and PdCl₂(PPh₃)₂ (120.6 mg, 0.17 mmol, 0.15 equiv). The solution was stirred for 2 hours at 0 °C and then concentrated. The residue was diluted with 20 mL of water and then extracted with ethyl acetate (2 x 40 mL). The organics were washed with 30 mL of water. The organics were dried over sodium sulfate, filtered, and the solvent was removed *in vacuo.* The residue was purified by silica gel chromatography eluting with ethyl acetate/petroleum ether (1:1) to give 4-(6-bromo-1-(3-hydroxy-2,2-dimethylpropyl)-1H-indazol-3-yl)-2-methylbut-3-yn-2-ol (88% yield) as a dark yellow oil. ESI-MS *m*/*z =* 365.1 [M+H]⁺.

### AO: 3-(6-bromo-3-((trimethylsilyl)ethynyl)-1H-indazol-1-yl)-2,2-dimethylpropan-1-ol

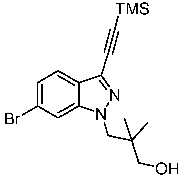

The title compound was synthesized using a procedure similar to the one described for the synthesis of 4-(6-bromo-1-(3-hydroxy-2,2-dimethylpropyl)-1H-indazol-3-yl)-2-methylbut-3-yn-2-ol to give the desired product in 83% yield. ESI-MS *m*/*z =* 379.1 [M+H]⁺.

### AP: 6-bromo-1-(2-(hydroxymethyl)allyl)-1H-indole-3-carbonitrile

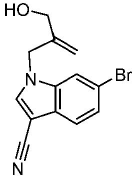

To a stirred solution of 6-bromo-1H-indole-3-carbonitrile (1.0 g, 4.5 mmol, 1.0 equiv) in DMF (30 mL) was added 2-(bromomethyl)prop-2-en-1-ol (910 mg, 6.0 mmol, 1.5 equiv) followed by K₂CO₃ (1.2 g, 9.0 mmol, 2.0 equiv). The mixture was stirred at 70 °C for 16 hours. The solution was then poured into water (200 mL), extracted with ethyl acetate (100 mL x 3). The combined organic layer was washed with water (2 x 100 mL) and brine (100 mL) and dried over sodium sulfate and concentrated to give a crude residue that was purified by silica gel chromatography (petroleum ether /ethyl acetate (5:1)) to give 6-bromo-1-(2-(hydroxymethyl)allyl)-1H-indole-3-carbonitrile (1.1 g, 78% yield) as an oil. ESI-MS *m*/*z =* 291.0 [M+H]⁺.

The following intermediates were synthesized according to the procedure described to make Intermediate AP using appropriate building blocks and modified reaction conditions (such as reagents, ratio of reagents, temperature, and reaction time) as needed.

| **Intermediate No.** | **Structure** | **Analytical Data** |
|---|---|---|
| AP-1 | 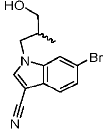 | |
| AP-2 | 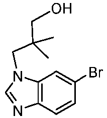 | ESI-MS *m*/*z* = 283.1 [M+H]+ |
| AP-3 | 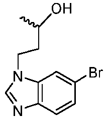 | ESI-MS *m*/*z* = 269.1 [M+H]+ |
| AP-4 | 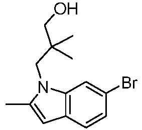 | ESI-MS *m*/*z* = 296.1[M+H]+ |
| AP-5 | 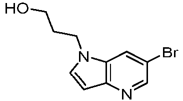 | ESI-MS *m*/*z =* 255.2; 257.2 [M+H]+; ¹H-NMR (300 MHz, DMSO-*d*₆) δ 8.38 (s, 1H), 8.24 (s, 1H), 7.70 (s, 1H), 6.59 (s, 1H), 4.64-4.61 (m, 1H), 4.28-4.23 (m, 2H), 3.31 (m, 2H), 1.93-1.84 (m, 2H). |
| AP-6 | 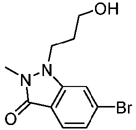 | ESI-MS *m*/*z* = 285.0 |

### AQ: (S)-6-bromo-1-(3-hydroxy-2-methylpropyl)-3,3-dimethylindolin-2-one

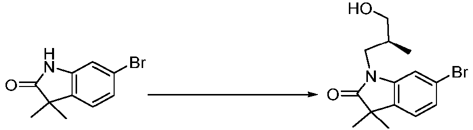

A solution of 6-bromo-3,3-dimethyl-2,3-dihydro-1H-indol-2-one (800 mg, 3.33 mmol, 1.0 equiv), DMF (10 mL), (2*R*)-3-bromo-2-methylpropan-1-ol (560.8 mg, 3.67 mmol, 1.1 equiv) and Cs₂CO₃ (3.26 grams, 10.00 mmol, 3.0 equiv) was stirred for 15 hours at room temperature. The residue was purified by silica gel chromatography (petroleum ether/ethyl acetate (2:1)) to afford (*S*)-6-bromo-1-(3-hydroxy-2-methylpropyl)-3,3-dimethylindolin-2-one (850 mg, 82% yield) as a yellow oil.

The following intermediate were synthesized according to Intermediate AQ described above using appropriate building blocks and modified reaction conditions (such as ratio of reagents, temperature, and reaction time) as needed.

| **Intermediate No.** | **Structure** | **Analytical Data** |
|---|---|---|
| AQ-1 | 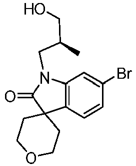 | ESI-MS *m*/*z* = 354.1 [M+H]+ |

### AR: 2-(6-bromoquinolin-4-yl)ethan-1-ol

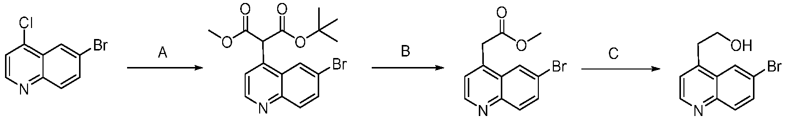

### Step A

To a solution of *tert*-butyl methyl malonate (6.5 g, 0.0374 mol, 3.0 equiv) in DMF was added sodium hydride (1.0 g, 0.0436 mol, 3.5 equiv). The mixture was stirred for 1 hour at 80 °C and then cooled to room temperature and 6-bromo-4-chloroquinoline (3 g, 0.0124 mol, 1.0 equiv) was added. The reaction was stirred for 15 hours at 100 °C. The reaction was treated with NaHSO₄ (10% aqueous) and then extracted with ethyl acetate (4 x 50 mL), washed with brine, and dried over sodium sulfate. After filtration, the solution was concentrated and purified by silica gel chromatography (petroleum ether/ethyl acetate (1:4)) to give 1-(*tert*-butyl) 3-methyl 2-(6-bromoquinolin-4-yl)malonate (2.7 g, 57% yield) as a yellow oil. ESI-MS *m*/*z =* 380.1 [M+H]⁺.

### Step B

To a solution of 1-(*tert*-butyl) 3-methyl 2-(6-bromoquinolin-4-yl)malonate (2.7 g, 0.0071 mol, 1.0 equiv) in dichloromethane was added TFA (10 ml) at 0 °C. The mixture was stirred for 6 hours at room temperature. Water was added and the solution was neutralized to pH 7. After extraction with ethyl acetate (x 3), the organics were washed with brine, dried over sodium sulfate, and filtered. Finally, the organic phase was concentrated and purified by silica gel chromatography (petroleum ether/ethyl acetate (1:4)) to give methyl 2-(6-bromoquinolin-4-yl)acetate (600 mg, 30% yield) as a yellow solid. ESI-MS *m*/*z* = 280.1 [M+H]⁺.

### Step C

To a solution of methyl 2-(6-bromoquinolin-4-yl)acetate (600 mg, 0.0021 mol, 1.0 equiv) in THF at 0 °C was added lithium aluminum hydride (163 mg, 0.0042 mol, 2.0 equiv) portionwise. The mixture was stirred overnight at room temperature and then water was added the solution was extracted with ethyl acetate. The organic phase was concentrated and purified by Prep-HPLC to afford 2-(6-bromoquinolin-4-yl)ethan-1-ol (220 mg, 41% yield) as a yellow oil. ESI-MS *m*/*z* = 252.0 [M+H]⁺.

### AS: 3-(6-bromo-2,3-dihydro-4H-benzo[b][1,4]oxazin-4-yl)propan-1-ol

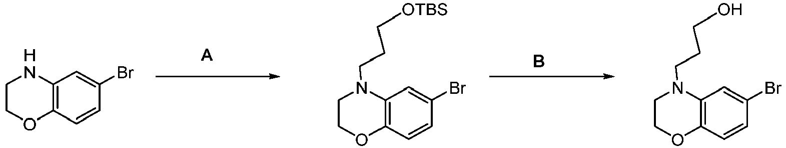

### Step A

To a stirred solution of 6-bromo-3,4-dihydro-2H-benzo[b][1,4]oxazine (9.0 g, 42.0 mmol, 1.0 equiv) in DMF (90.0 mL) was added NaH (60% dispersion, 2.5 g, 62.5 mmol, 1.5 equiv) at 0 °C. The mixture was stirred for 1 hour and then (3-bromopropoxy)(*tert*-butyl)dimethylsilane (16.0 g, 62.5 mmol, 1.5 equiv) was added. The resulting mixture was stirred for 16 hours at 20 °C. The solution was diluted with ethyl acetate (700 mL) and water (700 mL). The layers were separated and the organic layer was washed with water (3 × 300 mL), brine (200 mL), dried over anhydrous sodium sulfate, filtered, and the solvent was removed under reduced pressure to give a crude residue. The residue was purified by silica gel chromatography (petroleum ether) to give the 6-bromo-4-(3-((*tert*-butyldimethylsilyl)oxy)propyl)-3,4-dihydro-2H-benzo[b][1,4]oxazine (9.5 g, 59% yield). ESI-MS *m*/*z =* 386.1 [M+H]⁺. ¹H NMR (400 MHz, CDCl₃) δ 6.78 (d, *J =* 2.1 Hz, 1H), 6.65 (d, *J =* 2.1 Hz, 1H), 6.61 (d, *J =* 8.4 Hz, 1H), 4.20 (dd, *J =* 9.0, 4.5 Hz, 2H), 3.72 - 3.65 (m, 4H), 3.38 - 3.30 (m, 4H), 0.92 (d, *J =* 3.1 Hz, 9H), 0.07 (d, *J =* 3.2 Hz, 6H).

### Step B

To a stirred solution of 6-bromo-4-(3-((*tert*-butyldimethylsilyl)oxy)propyl)-3,4-dihydro-2H-benzo[b][1,4]oxazine (500 mg, 1.29 mmol, 1.0 equiv) in MeOH (0.5 mL) was added HCl/MeOH (10 N, 5.0 mL) at 20 °C. The resulting mixture was stirred for 0.5 hours at 20 °C. The solution was concentrated under reduced pressure to give a residue that was diluted with ethyl acetate (20 mL) and washed with aqueous sodium bicarbonate solution (20 mL). The organic phase was dried over anhydrous sodium sulfate, then concentrated to give 3-(6-bromo-2,3-dihydro-4H-benzo[b][1,4]oxazin-4-yl)propan-1-ol (250 mg, 71%). ESI-MS *m*/*z* = 272.0 [M+H]⁺.

### AT: 3-(6-bromo-2,3-dihydro-4H-benzo[b][1,4]oxazin-4-yl)-2-methylpropan-1-ol

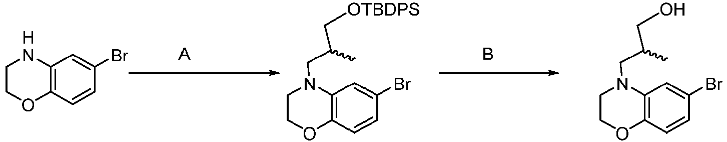

### Step A

To a stirred solution of 6-bromo-3,4-dihydro-2H-benzo[b][1,4]oxazine (7.0 g, 32.7 mmol, 1.0 equiv) in DCE (100 mL) was added 3-((*tert*-butyldiphenylsilyl)oxy)-2-methylpropanal (10.6 g, 32.7 mmol, 1.0 equiv) and NaBH₃CN (3.1 g, 49.0 mmol, 1.5 equiv) followed by AcOH (2.9 g, 49.0 mmol, 1.5 equiv) at 10 °C. The mixture was stirred for 16 hours at 60 °C and then diluted with ethyl acetate (500 mL) and water (500 mL). The layers was separated and the organic layer was washed with water (3 × 300 mL), brine (200 mL), dried over anhydrous sodium sulfate, filtered, and the solvent was removed under reduced pressure to give a residue that was purified by flash column on silica gel (petroleum ether) to give 6-bromo-4-(3-((*tert*-butyldiphenylsilyl)oxy)-2-methylpropyl)-3,4-dihydro-2H-benzo[b][1,4]oxazine (5.8 g, 34% yield) as an oil. ESI-MS *m*/*z* = 523.9 [M+H]⁺. ¹H NMR (400 MHz, CDCl₃) δ 7.70 - 7.62 (m, 4H), 7.47 - 7.35 (m, 6H), 6.75 (d, *J =* 2.1 Hz, 1H), 6.66 (dd, *J =* 8.4, 2.1 Hz, 1H), 6.61 (d, *J =* 8.4 Hz, 1H), 4.10 - 3.98 (m, 2H), 3.62 (dd, *J =* 10.1, 4.6 Hz, 1H), 3.53 (dd, *J =* 10.1, 5.5 Hz, 1H), 3.41 (dd, *J =* 14.5, 7.1 Hz, 1H), 3.27 (td, *J =* 5.3, 3.4 Hz, 2H), 2.97 (dd, *J =* 14.4, 7.4 Hz, 1H), 2.11 (dd, *J =* 12.0, 5.2 Hz, 1H), 1.10 (s, 9H), 0.97 (d, *J =* 6.8 Hz, 3H).

### Step B

To a stirred solution of 6-bromo-4-(3-((*tert*-butyldiphenylsilyl)oxy)-2-methylpropyl)-3,4-dihydro-2H-benzo[b][1,4]oxazine (5.8 g, 11.0 mmol, 1.0 equiv) in THF (50 mL) was added TBAF (1 M in THF, 28.0 mL, 28.0 mmol, 2.5 equiv) at 0 °C. The resulting mixture was stirred for 2 hours at 0 °C. After concentration *in vacuo,* the crude product was diluted with ethyl acetate (50 mL) and washed with water (10 mL x 5). The organic phase was concentrated under reduced pressure to give a residue that was purified by silica gel chromatography (petroleum ether / ethyl acetate = 5:2) to give 3-(6-bromo-2,3-dihydro-4H-benzo[b][1,4]oxazin-4-yl)-2-methylpropan-1-ol (3.2 g, 100% yield) as a white solid. ESI-MS *m*/*z =* 286.0 [M+H]⁺. ¹H NMR (400 MHz, CDCl₃) δ 6.80 (d, *J = 2.2* Hz, 1H), 6.69 (dd, *J =* 8.4, 2.1 Hz, 1H), 6.63 (d, *J =* 8.4 Hz, 1H), 4.18 (dd, *J =* 6.8, 2.5 Hz, 2H), 3.68 - 3.56 (m, 2H), 3.37 (dd, *J =* 9.2, 4.7 Hz, 2H), 3.28 (dd, *J =* 14.5, 8.1 Hz, 1H), 3.05 (dd, *J =* 14.5, 6.5 Hz, 1H), 2.14 (ddd, *J =* 13.4, 6.7, 1.3 Hz, 1H), 0.99 (d, *J =* 6.9 Hz, 3H).

### AU: (S)-8-bromo-5-ethyl-1-(3-hydroxy-2-methylpropyl)-1,3,4,5-tetrahydro-2H-benzo[b][1,4]diazepin-2-one

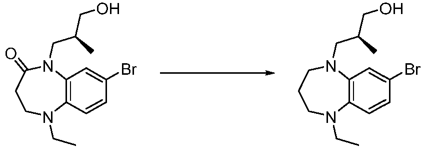

To a stirred solution of (*S*)-8-bromo-5-ethyl-1-(3-hydroxy-2-methylpropyl)-1,3,4,5-tetrahydro-2H-benzo[b][1,4]diazepin-2-one (2.1 g, 6.154 mmol, 1.0 equiv) in THF was added BH₃•THF (1 N, 25 mL) dropwise at 25 °C under an atmosphere of argon. The resulting mixture was stirred for 1 hour at room temperature and then quenched with 1 mL MeOH. The mixture was neutralized to pH 7 with saturated aqueous NaHCO₃. The resulting mixture was extracted with ethyl acetate (3 x 50 mL). The combined organic layers were washed with brine (1 x 100 mL), dried over anhydrous sodium sulfate, filtered, and the filtrate was concentrated under reduced pressure. The residue was purified by silica gel column chromatography, eluting with petroleum ether/ethyl acetate (10:1 to 5:1) to afford (S)-8-bromo-5-ethyl-1-(3-hydroxy-2-methylpropyl)-1,3,4,5-tetrahydro-2H-benzo[b][1,4]diazepin-2-one (1.6 g ,75% yield) as a colorless oil. ESI-MS *m*/*z =* 329.1 [M+H]⁺. ¹H-NMR (300 MHz, DMSO-*d*₆) δ 6.90 - 6.81 (m, 2H), 6.65 (d, *J =* 8.4 Hz, 1H), 4.48 (dd, *J =* 5.8, 4.7 Hz, 1H), 4.08 - 3.96 (m, 1H), 3.38 (dt, *J =* 10.4, 5.2 Hz, 1H), 3.28 (dd, *J =* 10.5, 5.6 Hz, 1H), 3.21 - 3.00 (m, 7H), 2.84 (dd, *J =* 13.1, 7.4 Hz, 1H), 2.36 (s, 1H), 1.87 (q, *J* = 6.5 Hz, 1H), 1.69 (p, *J =* 6.0 Hz, 2H), 1.08 (t, *J =* 7.0 Hz, 3H), 0.85 (d, *J =* 6.7 Hz, 3H).

### A V: (S)-7-bromo-1-ethyl-5-(3-hydroxy-2-methylpropyl)-1,3,4,5-tetrahydro-2H-benzo[b][1,4]diazepin-2-one

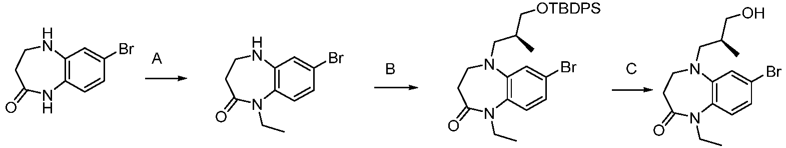

### Step A

To a stirred solution of 7-bromo-2,3,4,5-tetrahydro-1H-1,5-benzodiazepin-2-one (4 g, 16.6 mmol, 1 equiv) in DMF (40 mL) was added NaH (60% dispersion, 0.80 g, 19.9 mmol, 1.2 equiv) in portions at -15 °C under a nitrogen atmosphere. The resulting mixture was stirred for 20 min at -15 °C, at which point iodoethane (2.85 g, 18.2 mmol, 1.1 equiv) was added dropwise at -15 °C. The resulting mixture was stirred for an additional 2 hours at room temperature. The solution was quenched with saturated aqueous NH₄Cl and the resulting mixture was extracted with ethyl acetate (3 x 150 mL). The combined organic layers were washed with brine (1 x 100 mL) and dried over anhydrous sodium sulfate. After filtration, the filtrate was concentrated under reduced pressure to afford 7-bromo-1-ethyl-2,3,4,5-tetrahydro-1H-1,5-benzodiazepin-2-one (3.5 g, 71% yield) as a yellow solid. ¹H-NMR (300 MHz, DMSO-*d*₆) δ 7.31 - 7.11 (m, 2H), 7.05 (dq, *J =* 8.7, 2.0 Hz, 1H), 5.52 - 5.31 (m, 1H), 3.72 (q, *J =* 7.2 Hz, 2H), 3.55 (tt, *J =* 6.2*,* 2.7 Hz, 2H), 2.34 (t, *J =* 6.6 Hz, 2H), 0.98 (ddd, *J =* 9.1*,* 6.5, 2.3 Hz, 3H).

### Step B

A solution of AcOH (80 mL), 7-bromo-1-ethyl-2,3,4,5-tetrahydro-1H-1,5-benzodiazepin-2-one (800 mg, 2.972 mmol, 1 equiv) and (2*R*)-3-[(*tert*-butyldiphenylsilyl)oxy]-2-methylpropanal (1.946 g, 5.960 mmol, 2.01 equiv) was stirred for 10 minutes at room temperature. To the mixture was added NaBH₃CN (282 mg, 4.487 mmol, 1.51 equiv) in portions at room temperature. The resulting mixture was stirred for an additional 16 hours. The resulting mixture was diluted with saturated aqueous NaHCO₃ and then extracted with ethyl acetetate (3 x 200 mL). The combined organic layers were washed with brine (150 mL) and dried over anhydrous sodium sulfate. After filtration, the filtrate was concentrated under reduced pressure to afford 7-bromo-5-[(2*S*)-3-[(*tert*-butyldiphenylsilyl)oxy]-2-methylpropyl]-1-ethyl-2,3,4,5-tetrahydro-1H-1,5-benzodiazepin-2-one (2.3 g, crude) as a yellow oil. The crude product was used in the next step directly without further purification. ESI-MS *m*/*z* = 579.1 [M+H]⁺.

### Step C

To a stirred solution of 7-bromo-5-[(2*S*)-3-[(*tert*-butyldiphenylsilyl)oxy]-2-methylpropyl]-1-ethyl-2,3,4,5-tetrahydro-1H-1,5-benzodiazepin-2-one (2.3 g, 3.968 mmol, 1.0 equiv) in THF (30 mL) was added TBAF (1 M in THF, 10 mL, 10.0 mmol, 2.52 equiv) dropwise at room temperature. The mixture was stirred for 16 hours at room temperature and then concentrated under reduced pressure. The resulting residue was diluted with water (200 mL) and extracted with ethyl acetate (3 x 100 mL). The combined organic layers were washed with brine (1 x 100 mL), dried over anhydrous sodium sulfate, filtered, and concentrated under reduced pressure. The residue was purified by Prep-TLC (petroleum ether/ethyl acetate (1:4)) to afford 7-bromo-1-ethyl-5-[(2S)-3-hydroxy-2-methylpropyl]-2,3,4,5-tetrahydro-1H-1,5-benzodiazepin-2-one (600 mg, 40% yield) as a yellow solid. ¹H-NMR (300 MHz, CDCl₃) δ 7.27 - 7.17 (m, 2H), 7.11 - 7.03 (m, 1H), 3.52 (d, *J =* 5.6 Hz, 4H), 3.16 - 2.77 (m, 2H), 2.40 (t, *J =* 6.8 Hz, 2H), 2.12 - 1.88 (m, *J =* 6.0*,* 5.3 Hz, 1H), 1.41 - 1.18 (m, 2H), 1.11 (t, *J =* 7.1 Hz, 3H), 0.90 (d, *J =* 6.8 Hz, 3H).

### AW: 5-bromo-3-(3-hydroxy-2,2-dimethylpropyl)-1-methyl-1,3-dihydro-2H-benzo[d]imidazol-2-one

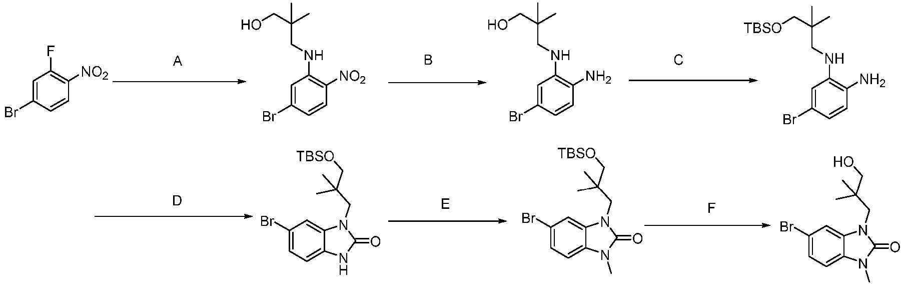

### Step A

To a stirred solution of 4-bromo-2-fluoro-1-nitrobenzene (2 g, 9.1 mmol, 1.0 equiv) in DMF (20 mL) was added K₂CO₃ (2.52 g, 18.23 mmol, 2.0 equiv) and 3-amino-2,2-dimethylpropan-1-ol (1.41 g, 13.7 mmol, 1.5 equiv) in portions at 25 °C. The resulting mixture was stirred overnight and the resulting mixture was diluted with 150 mL water and extracted with ethyl acetate (3 x 50 mL). The combined organic layers were washed with brine (1 x 100 mL) and dried over anhydrous sodium sulfate. After filtration, the filtrate was concentrated under reduced pressure. The residue was purified by silica gel column chromatography, eluting with petroleum ether/ethyl acetate (10:1 to 3:1) to afford 3-[(5-bromo-2-nitrophenyl)amino]-2,2-dimethylpropan-1-ol (2.7 g, 96% yield) as a red solid. ESI-MS *m*/*z =* 303.1 [M+H]⁺.

### Step B

A solution of 3-[(5-bromo-2-nitrophenyl)amino]-2,2-dimethylpropan-1-ol (2.7 g, 8.91 mmol, 1 equiv), water (27 mL) and acetic acid (27 mL) was treated with zinc (2.9 g, 44.36 mmol, 4.98 equiv) at 0 °C. The resulting solution was stirred for 1 hour while warming to room temperature. The solution was neutralized to pH 7 with saturated aqueous sodium bicarbonate. The resulting mixture was extracted with ethyl acetate (3 x 50 mL). The combined organic layers were washed with brine (3 x 50 mL) and saturated sodium chloride (3 x 50 mL). The mixture was dried over anhydrous sodium sulfate and concentrated. The resulting mixture was concentrated under vacuum to give 3-[(2-amino-5-bromophenyl)amino]-2,2-dimethylpropan-1-ol (2.4 g, 91% yield) as a brown solid. ESI-MS *m*/*z* = 273.1 [M+H]⁺.

### Step C

A solution of 3-[(2-amino-5-bromophenyl)amino]-2,2-dimethylpropan-1-ol (2.0 g, 7.32 mmol, 1.0 equiv), N,N-dimethylformamide (20 mL) and imidazole (1.0 g, 14.69 mmol, 2.01 equiv) was treated with *tert*-butyl(chloro)dimethylsilane (1.2 g, 7.96 mmol, 1.1 equiv) dropwise at 25 °C. The resulting solution was stirred for 2 hours at 25 °C. The resulting mixture was diluted with 150 mL water and extracted with ethyl acetate (3 x 25 mL) and the organic layers were combined. The organics were washed with brine (3 x 15 mL). The mixture was dried over anhydrous sodium sulfate and concentrated and the residue was purified by silica gel chromatography eluting with ethyl acetate/petroleum ether (3:8) to give 2.33 g (82% yield) of 5-bromo-N1-[3-[(tert-butyldimethylsilyl)oxy]-2,2-dimethylpropyl]benzene-1,2-diamine as a brown oil. ESI-MS *m*/*z =* 389.1 [M+H]⁺.

### Step D

To a stirred solution of 5-bromo-*N*1-[3-[(*tert*-butyldimethylsilyl)oxy]-2,2-dimethylpropyl]benzene-1,2-diamine(2.98 g, 7.692 mmol, 1 equiv) in THF (20 mL) was added CDI (1.5 g, 9.25 mmol, 1.2 equiv) in portions at 25 °C. The mixture was stirred overnight at 60 °C. The resulting mixture was diluted with 150 mL water and extracted with ethyl acetate (3 x 50 mL). The combined organic layers were washed with brine (1 x 100 mL) and dried over anhydrous sodium sulfate. After filtration, the filtrate was concentrated under reduced pressure. The residue was purified by silica gel column chromatography, eluting with petroleum ether/ethyl acetate (20:1-10:1) to afford 6-bromo-1-[3-[(*tert*-butyldimethylsilyl)oxy]-2,2-dimethylpropyl]-2,3-dihydro-1H-1,3-benzodiazol-2-one (1.27 g, 37% yield) as a yellow solid. ESI-MS *m*/*z =* 415.2 [M+H]⁺.

### Step E

To a stirred solution of 6-bromo-1-[3-[(*tert*-butyldimethylsilyl)oxy]-2,2-dimethylpropyl]-2,3-dihydro-1H-1,3-benzodiazol-2-one (1.27 g, 3.072 mmol, 1.0 equiv) in *N,N*-dimethylformamide (10 mL) was added cesium carbonate (2.0 g, 6.12 mmol, 2.0 equiv) and iodomethane (0.86 g, 6.06 mmol, 1.97 equiv) dropwise at 0 °C. The resulting mixture was stirred overnight after warming to room temperature. The resulting mixture was diluted with 100 mL of water and extracted with ethyl acetate (3 x 50 mL). The combined organic layers were washed with water (2 x 50 mL) and brine (1 x 100 mL), dried over anhydrous sodium sulfate, filtered, and the filtrate was concentrated under reduced pressure. The residue was purified by silica gel chromatography, eluting with petroleum ether/ethyl acetate (20:1 to 6:1) to afford 5-bromo-3-[3-[(*tert*-butyldimethylsilyl)oxy]-2,2-dimethylpropyl]-1-methyl-2,3-dihydro-1H-1,3-benzodiazol-2-one (1.26 g, 92% yield) as a white solid. ESI-MS *m*/*z* = 429.2 [M+H]⁺.

### Step F

To a stirred solution of 5-bromo-3-[3-[(*tert-*butyldimethylsilyl)oxy]-2,2-dimethylpropyl]-1-methyl-2,3-dihydro-1H-1,3-benzodiazol-2-one (1.23 g, 2.877 mmol, 1.0 equiv) in THF (10 mL) was added TBAF (1 N in THF, 3.4 mL) dropwise at 0 °C. The resulting mixture was stirred overnight at room temperature and then concentrated under vacuum. The residue was purified by Prep-TLC (petroleum ether/ethyl acetate (1:2)) to afford 5-bromo-3-(3-hydroxy-2,2-dimethylpropyl)-1-methyl-2,3-dihydro-1H-1,3-benzodiazol-2-one (875 mg, 94% yield) as a white solid. ESI-MS *m*/*z =* 315.1 [M+H]⁺.

### AX: (S)-5-bromo-1-cyclobutyl-3-(3-hydroxy-2-methylpropyl)-1,3-dihydro-2H-benzo[d]imidazol-2-one

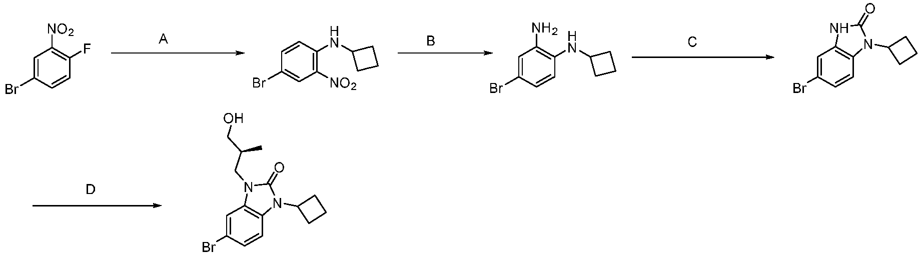

### Step A

A solution of 4-bromo-1-fluoro-2-nitrobenzene (3.00 g, 13.6 mmol, 1.0 equiv), cyclobutanamine (1.16 g, 16.4 mmol, 1.2 equiv) and K₂CO₃ (5.65 g, 40.9 mmol, 3.0 equiv) in DMF (60 mL) was stirred for 2 hours at 25 °C. The reaction cooled to 0 °C and then water was added. The resulting mixture was extracted with ethyl acetate (3 x 250 mL). The combined organic layers were washed with water (3 x 250 mL) and dried over anhydrous sodium sulfate. After filtration, the filtrate was concentrated under reduced pressure. The residue was purified by silica gel column chromatography, eluting with petroleum ether/ethyl acetate (5:1) to give 4-bromo-N-cyclobutyl-2-nitroaniline (3.7 g, 90.07% yield) as a red oil. ESI-MS *m*/*z* = 271.1 [M+H]⁺.

### Step B

A solution of 4-bromo-*N*-cyclobutyl-2-nitroaniline (3.70 g, 13.7 mmol, 1.0 equiv) and zinc (4.46 g, 68.2 mmol, 5.0 equiv) in AcOH (40 ml) and H₂O (40 mL) was stirred for 1 hour at room temperature. The precipitated solids were collected by filtration and washed with AcOH (3 x 10 mL). The filtrate was basified to pH 9 with 1 N NaOH. The solution was extracted with ethyl acetate (3 x 100mL). The combined organic layers were washed with water (3 x 100 mL) and dried over anhydrous sodium sulfate. After filtration, the filtrate was concentrated under reduced pressure to afford 4-bromo-*N*1-cyclobutylbenzene-1,2-diamine (2.7 g, 82% yield) as a red oil. ESI-MS *m*/*z* = 241.3 [M+H]⁺.

### Step C

A solution of 4-bromo-*N*1-cyclobutylbenzene-1,2-diamine (2.70 g, 11.2 mmol, 1.0 equiv) and CDI (3.63 g, 22.4 mmol, 2.0 equiv) in THF (30 ml) was stirred for 15 hours at 60 °C. The resulting mixture was concentrated under reduced pressure. The residue was purified by silica gel column chromatography, eluting with petroleum ether/ethyl acetate (1:1) to afford 5-bromo-1-cyclobutyl-1,3-dihydro-2H-benzo[d]imidazol-2-one (2.4 g, 80% yield) as a brown solid. ES-MS *m*/*z =* 267.0 [M+H]⁺.

### Step D

A solution of 5-bromo-1-cyclobutyl-2,3-dihydro-1*H*-1,3-benzodiazol-2-one (2.40 g, 8.99 mmol, 1.0 equiv), (2*R*)-3-bromo-2-methylpropan-1-ol (2.06 g, 13.5 mmol, 1.5 equiv), and Cs₂CO₃ (5.85 g, 18.0 mmol, 2.0 equiv) in DMF (30 mL) was stirred for 5 hours at room temperature. The resulting mixture was extracted with ethyl acetate (3 x 250 mL). The combined organic layers were washed with water (3 x 250 mL) and dried over anhydrous sodium sulfate. After filtration, the filtrate was concentrated under reduced pressure. The residue was purified by silica gel column chromatography, eluting with petroleum ether/ethyl acetate (2:1) to afford 5-bromo-1-cyclobutyl-3-[(2S)-3-hydroxy-2-methylpropyl]-2,3-dihydro-1*H-*1,3-benzodiazol-2-one (3.0 g, 98% yield) as a brown oil. ESI-MS *m*/*z =* 339.3 [M+H]⁺.

The following intermediates were synthesized according to Intermediate AX described above using appropriate building blocks and modified reaction conditions (such as ratio of reagents, temperature, and reaction time) as needed.

| **Intermediate No.** | **Structure** | **Analytical Data** |
|---|---|---|
| AX-1 | 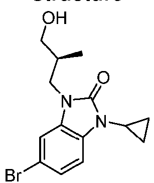 | ESI-MS *m*/*z* = 325.0, 327.0 [M+H]+ |
| AX-2 | 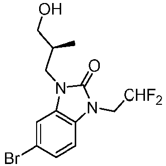 | ESI-MS *m*/*z* = 349.3 [M+H]+ |
| AX-3 | 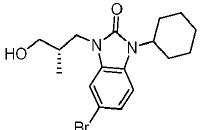 | ESI-MS *m*/*z* = 367.2 [M+H] |
| AX-4 | 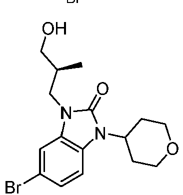 | ESI-MS *m*/*z* = 371.2 [M+H]+ ; ¹H-NMR (300 MHz, DMSO-*d*₆) δ 7.44 (d, *J =* 2.2 Hz, 1H), 7.30 (d, *J =* 8.2 Hz, 1H), 7.20 (dt, *J =* 8.7, 2.1 Hz, 1H), 4.63 (t, *J =* 4.8 Hz, 1H), 4.55 - 4.32 (m, 1H), 4.10 - 3.87 (m, 2H), 3.85 - 3.61 (m, 2H), 3.47 (t, *J =* 11.8 Hz, 2H), 3.30 (t, *J =* 5.5 Hz, 2H), 2.44 - 2.15 (m, 2H), 2.03 (dd, *J =* 12.8, 6.3 Hz, 1H), 1.73 - 1.49 (m, 2H), 0.82 (d, J = 6.7 Hz, 3H). |

### AY: (S)-5-bromo-3-(3-hydroxy-2-methylpropyl)-1-(pyridin-4-yl)-1,3-dihydro-2H-benzo[d]imidazol-2-one

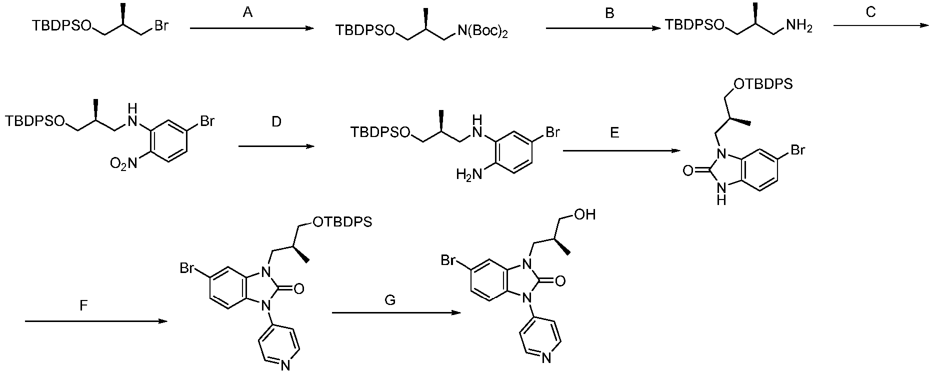

### Step A

A solution of (*R*)-(3-bromo-2-methylpropoxy)(tert-butyl)diphenylsilane (3.75 g, 9.6 mmol, 1.0 equiv), Cs₂CO₃ (6.24 g, 19.2 mmol, 2.0 equiv), and NH(Boc)₂ (2.3 g, 10.6 mmol, 1.1 equiv) in DMF (20 mL) was stirred at 80 °C for 3 hours. The solution was poured into ice water (200 mL) and extracted with ethyl acetate (3 x 100 mL). The combined organic layer was dried over anhydrous sodium sulfate and concentrated under reduced pressure to give a crude product, which was purified by silica gel chromatography (petroleum ether to petroleum ether/ethyl acetate (5:1)) to give the desired product (4.5 g, 85%) as a clear oil. ESI-MS *m*/*z=* 550.3 [M+Na]⁺.

### Step B

To a solution of starting amine (4.5 g, 10.5 mmol, 1.0 equiv) in dichloromethane (12 mL) was added TFA (4 mL) dropwise. The mixture was stirred at for 3 hours and then was concentrated to give (*S*)-3-((*tert*-butyldiphenylsilyl)oxy)-2-methylpropan-1-amine. The crude product was used for the next step directly without further purification. ESI-MS *m*/*z* = 328.3 [M+H]⁺.

### Step C

A solution of 4-bromo-2-fluoro-1-nitrobenzene (2.31 g, 10.5 mmol, 1.0 equiv), K₂CO₃ (2.9 g, 21.0 mmol, 2.0 equiv), and (S)-3-((tert-butyldiphenylsilyl)oxy)-2-methylpropan-1-amine (TFA salt, 4.63 g) was stirred, at which point water (100 mL) was added and the solution was extracted with ethyl acetate (3 x 100 mL). The combined organic layer was dried over anhydrous sodium sulfate and concentrated under reduced pressure to give a crude product, which was purified by silica gel chromatography (petroleum ether to petroleum ether/ethyl acetate (3:1)) to give (*S*)-5-bromo-*N*-(3-((*tert*-butyldiphenylsilyl)oxy)-2-methylpropyl)-2-nitroaniline (3.15 g, 70 %) as a yellow oil. ESI-MS *m*/*z=* 527.2 [M+H]⁺.

### Step D

To a stirred solution of (*S*)-5-bromo-*N*-(3-((*tert*-butyldiphenylsilyl)oxy)-2-methylpropyl)-2-nitroaniline (3.15 g, 5.97 mmol, 1.0 equiv) in EtOH (100 mL) was added iron (3.34 g, 59.7 mmol, 10.0 equiv) and NH₄Cl (3.2 g, 59.7 mmol, 10.0 equiv) as a solution in H₂O (100 mL). The reaction was then stirred at 90 °C for 2 hours. The mixture was cooled to room temperature and then poured into water and extracted with ethyl acetate (100 mL x 3). The organic layer was dried over anhydrous sodium sulfate, and concentrated under vacuum to give the crude (*S*)-5-bromo-*N*1-(3-((*tert*-butyldiphenylsilyl)oxy)-2-methylpropyl}benzene-1,2-diamine (2.55 g, 86% yield), which was used for the next step. ESI-MS *m*/*z=* 497.2 [M+H]⁺.

### Step E

A solution of (*S)-*5-bromo-N1-(3-((*tert*-butyldiphenylsilyl)oxy)-2-methylpropyl)benzene-1,2-diamine (2.35 g, 4.7 mmol, 1.0 equiv) and CDI (2.28 g, 14.1 mmol, 3.0 equiv) in THF (30 mL) was stirred at 70 °C for 16 hours. The reaction mixture was cooled to room temperature and poured into water (100 mL) and extracted with ethyl acetate (3 x 100 mL). The combined organic layers were dried over anhydrous sodium sulfate and concentrated under reduced pressure to give the crude product, which was purified by silica gel chromatography (petroleum ether to petroleum ether/ethyl acetate (1:1)) to give (S)-6-bromo-1-(3-((tert-butyldiphenylsilyl)oxy)-2-methylpropyl)-1,3-dihydro-2H-benzo[d]imidazol-2-one (1.56 g, 63% yield) as a white solid. ESI-MS *m*/*z=* 523.1 [M+H]⁺.

### Step F

A solution of (*S*)-6-bromo-1-(3-((*tert*-butyldiphenylsilyl)oxy)-2-methylpropyl)-1,3-dihydro-2H-benzo[d]imidazol-2-one (1.52 g, 2.9 mmol, 1.0 equiv), Cul (55 mg, 0.29 mmol, 0.1 equiv), 4-iodopyridine (1.19 g, 5.8 mmol, 1.0 equiv), K₂CO₃ (1.2 g, 8.7 mmol, 3.0 equiv), and *N1,N1*-dimethylethane-1,2-diamine (51 mg, 0.58 mmol, 0.2 equiv) in dioxane (20 mL) was stirred at 110 °C for 5 hours. The reaction was quenched by addition of ice water (100 mL) and extracted with ethyl acetate (3 x 100 mL). The combined organic layers were dried over anhydrous sodium sulfate and concentrated under reduced pressure to give a crude product, which was purified by silica gel chromatography (dichloromethane to dichloromethane/MeOH (50:1)) to give (S)-5-bromo-3-(3-((tert-butyldiphenylsilyl)oxy)-2-methylpropyl)-1-(pyridin-4-yl)-1,3-dihydro-2H-benzo[d]imidazol-2-one (1.37 g, 78% yield) as a white solid.

### Step G

To a solution of (*S*)-5-bromo-3-(3-((*tert*-butyldiphenylsilyl)oxy)-2-methylpropyl)-1-(pyridin-4-yl)-1,3-dihydro-2H-benzo[d]imidazol-2-one (1.37 g, 2.28 mmol, 1.0 equiv) in THF (15 mL) was added TBAF (1N in THF, 4.56 mL, 1 M) dropwise at 20 °C. The mixture was stirred at 20 °C for 5 hours and then poured into water and extracted with ethyl acetate (100 mL). The organic layer was washed with brine (50 mL x 3) and dried over anhydrous sodium sulfate, filtered, and concentrated to give a crude residue that was purified by silica gel chromatography (dichloromethane to dichloromethane/MeOH=20:1) to give (S)-5-bromo-3-(3-hydroxy-2-methylpropyl)-1-(pyridin-4-yl)-1,3-dihydro-2H-benzo[d]imidazol-2-one (215 mg, 60.7 % yield) as a white solid. ESI-MS *m*/*z* = 362.0 [M+H]⁺.

### AZ: (6-bromo-1-(3-hydroxy-2,2-dimethylpropyl)-1H-indol-3-yl)pyrrolidin-2-one

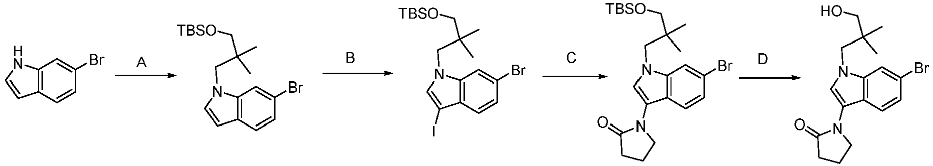

### Step A

To a stirred solution of 6-bromo-1H-indole (5.0 g, 25.6 mmol, 1.0 equiv) in DMSO (100 mL) was added (3-bromo-2,2-dimethylpropoxy)(*tert*-butyl)dimethylsilane (10.7 g, 37.8 mmol, 1.5 equiv) followed by K₂CO₃ (10.7 g, 77.8 mmol, 3.0 equiv) and KI (4.3 g, 25.6 mmol, 1.0 equiv). The mixture was stirred at 120 °C for 16 hours. The solution was poured into water (500 mL) and extracted with ethyl acetate (250 mL x 3). The combined organic layer was washed with water (300 mL x 2) and brine (300 mL x 1), dried over sodium sulfate and concentrated to give a residue. The residue was purified by silica gel chromatography (petroleum ether/ethyl acetate (100:1)) to 6-bromo-1-(3-((*tert*-butyldimethylsilyl)oxy)-2,2-dimethylpropyl)-1*H*-indole (3.4 g, 34% yield) as an oil.

### Step B

6-bromo-1-(3-((*tert*-butyldimethylsilyl)oxy)-2,2-dimethylpropyl)-1*H*-indole (2.9 g, 7.3 mmol, 1.0 equiv) was dissolved in DMF (30 mL) at 0 °C, and NIS (1.6 g, 7.3 mmol, 1.0 equiv) was added in portions. The reaction mixture was stirred at 0 °C for 1 hour, and then the reaction mixture was poured in 200 mL of ice water and Na₂SO₃ (5.0 g). The organic layer was separated, dried, and was concentrated to give a residue. The residue was purified by silica gel chromatography (petroleum ether) to give 6-bromo-1-(3-((*tert*-butyldimethylsilyl)oxy)-2,2-dimethylpropyl)-3-iodo-1H-indole (3.2 g, 84% yield) as a white solid. ¹H NMR (400 MHz, CDCl₃) δ 7.58 (s, 1H), 7.25 (d, *J =* 1.4 Hz, 2H), 7.19 (s, 1H), 3.95 (s, 2H), 3.23 (s, 2H), 1.01 - 0.96 (m, 9H), 0.90 (s, 6H), 0.14 - 0.10 (m, 6H).

### Step C

6-bromo-1-(3-((*tert*-butyldimethylsilyl)oxy)-2,2-dimethylpropyl)-3-iodo-1H-indole (3.2 g, 6.1 mmol, 1.0 equiv) in dioxane (60 mL) was treated with pyrrolidin-2-one (1.0 g, 12.2 mmol, 2.0 equiv), Cul (230 mg, 1.2 mmol, 0.2 equiv), ethylene diamine (72 mg, 1.2 mmol, 0.2 equiv) and Cs₂CO₃ (4.0 mg, 12.2 mmol, 2.0 equiv). The mixture was stirred at 100 °C for 16 hours. After concentration, the residue was purified by silica gel chromatography (Petroleum/ethyl acetate = 3/1) to afford 1-(6-bromo-1-(3-((*tert*butyldimethylsilyl)oxy)-2,2-dimethylpropyl)-1*H*-indol-3-yl)pyrrolidin-2-one (750 mg, 27% yield) as an oil.

### Step D

To a stirred solution of 1-(6-bromo-1-(3-((*tert*-butyldimethylsilyl)oxy)-2,2-dimethylpropyl)-1*H*-indol-3-yl)pyrrolidin-2-one (1.4 g, 2.9 mmol, 1.0 equiv) in THF (18 mL) was added TBAF (1 M in THF, 8.7 mL, 8.7 mmol, 3.0 equiv) at 0 °C. The resulting mixture was stirred for 2 hours at 0 °C. After concentration, the crude product was diluted with ethyl acetate (50 mL) and washed with water (10 mL x 5). The organic phase was concentrated under reduced pressure to give a residue that was purified by silica gel chromatography (petroleum/ethyl acetate (2:1)) to give 1-(6-bromo-1-(3-hydroxy-2,2-dimethylpropyl)-1H-indol-3-yl)pyrrolidin-2-one (790 mg, 75% yield) as an oil. ESI-MS *m*/*z =* 316.0 [M+H]⁺. ¹H NMR (400 MHz, CDCl₃) δ 7.60 (d, *J =* 1.5 Hz, 1H), 7.49 (d, *J =* 8.6 Hz, 1H), 7.41 (s, 1H), 7.17 (dd, *J =* 8.6, 1.6 Hz, 1H), 3.98 (t, *J =* 7.0 Hz, 2H), 3.95 (s, 2H), 3.32 (s, 2H), 2.60 (t, *J =* 8.1 Hz, 2H), 2.29 - 2.19 (m, 2H), 0.96 (s, 6H).

### BA: 6-bromo-1-(3-((tert-butyldimethylsilyl)oxy)-2,2-dimethylpropyl)indolin-2-one

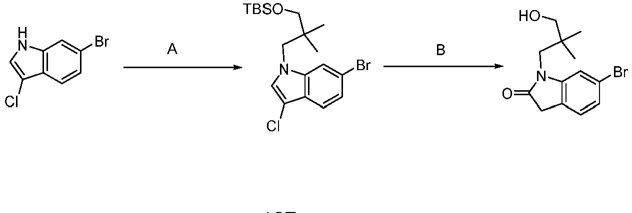

### Step A

To a stirred solution of 6-bromo-3-chloro-1*H*-indole (3.0 g, 13.0 mmol, 1.0 equiv) in DMSO (100 mL) was added 3-bromo-2,2-dimethylpropoxy)(*tert*-butyl)dimethylsilane (8.0 g, 28.6 mmol, 2.2 equiv) followed by K₂CO₃ (5.4 g, 39.1 mmol, 2.0 equiv), and KI (2.2 g, 13.0 mmol, 1.0 equiv). The mixture was stirred at 120 °C for 16 hours. The solution was poured into water (500 mL) and extracted with ethyl acetate (250 mL x 3). The combined organic layers were washed with water (300 mL x 2) and brine (300 mL), dried over sodium sulfate and concentrated to give a residue that was purified by silica gel chromatography (petroleum ether / ethyl acetate 100:1) to give 6-bromo-1-(3-((tert-butyldimethylsilyl)oxy)-2,2-dimethylpropyl)-3-chloro-1H-indole (5.1 g, 89% yield) as an oil.

### Step B

A solution of 6-bromo-1-(3-((*tert*-butyldimethylsilyl)oxy)-2,2-dimethylpropyl)-3-chloro-1H-indole (4.0 g, 8.7 mmol, 1.0 equiv) in THF (50 mL) was treated with aqueous 6N HCl (50 mL). The reaction mixture was stirred at 70 °C for 16 hours. The mixture was poured into water (200 mL) and extracted with ethyl acetate (100 mL x 3), and the organic layers were concentrated to dryness to give a residue. The residue was purified by silica gel chromatography (petroleum/ethyl acetate (10:1)) to afford 6-bromo-1-(3-hydroxy-2,2-dimethylpropyl)indolin-2-one (2.0 g, 64% yield) as a yellow solid. ESI-MS *m*/*z =* 298.0 [M+H]⁺; ¹H-NMR (400 MHz, DMSO-*d*₆) δ 7.44 (d, *J =* 1.2 Hz, 1H), 7.23 - 7.12 (m, 2H), 4.78 (s, 1H), 3.56 (s, 2H), 3.50 (s, 2H), 3.12 (s, 2H), 0.84 (d, *J =* 11.8 Hz, 6H).

### BB: Synthesis of 2-(6-bromo-1-(3-hydroxy-2-(hydroxymethyl)propyl)-1H-indol-3-yl)-2-methylpropanenitrile

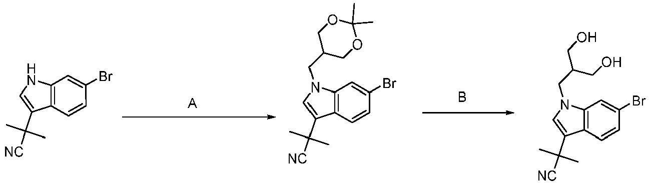

### Step A

Into a 100 mL tube was added 2-(6-bromo-1H-indol-3-yl)-2-methylpropanenitrile (3.2 g, 12.2 mmol, 1.0 equiv), DMF (40 mL), (2,2-dimethyl-1,3-dioxan-5-yl)methyl methanesulfonate (4.1 g, 18.282 mmol, 1.50 equiv) and Cs₂CO₃ (11.9 g, 36.5 mmol, 3.0 equiv). The resulting mixture was stirred for 16 hours at 50 °C and then diluted with water (400 mL). The resulting mixture was extracted with ethyl acetate (3 x 300 mL). The combined organic layers were washed with water (2 x 200 mL) and brine (50 mL) and dried over anhydrous sodium sulfate. After filtration, the filtrate was concentrated under reduced pressure. The residue was purified by silica gel chromatography, eluting with petroleum ether/ethyl acetate (4:1) to afford 2-(6-bromo-1-((2,2-dimethyl-1,3-dioxan-5-yl)methyl)-1H-indol-3-yl)-2-methylpropanenitrile (3.3 g, 62% yield) as a light-yellow solid. ESI-MS *m*/*z* = 391.1 [M+H]⁺.

### Step B

To a stirred solution of 2-(6-bromo-1-((2,2-dimethyl-1,3-dioxan-5-yl)methyl)-1H-indol-3-yl)-2-methylpropanenitrile (3.3 g, 8.433 mmol, 1 equiv) in THF (30 mL) was added concentrated HCl (6 mL) dropwise at 0 °C. The resulting mixture was stirred for 3 hours at room temperature. The resulting mixture was concentrated under vacuum and then neutralized to pH 7 with saturated aqueous NaHCO₃. The resulting mixture was extracted with ethyl acetate (3 x 150 mL). The combined organic layers were washed with brine (100 mL) and dried over anhydrous sodium sulfate. After filtration, the filtrate was concentrated under reduced pressure. The crude product 2-(6-bromo-1-(3-hydroxy-2-(hydroxymethyl)propyl)-1H-indol-3-yl)-2-methylpropanenitrile (2.8 g, 85% yield) was used in the next step directly without further purification. ESI-MS *m*/*z* = 351.0 [M+H]⁺.

### BC: 2-(6-bromo-1-(3-hydroxy-2-(methoxymethyl)propyl)-1H-indol-3-yl)-2-methylpropanenitrile

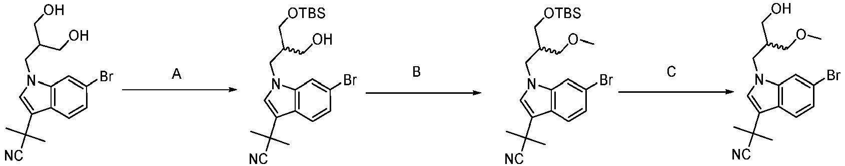

### Step A

A solution of 2-[6-bromo-1-[3-hydroxy-2-(hydroxymethyl)propyl]-1H-indol-3-yl]-2-methylpropanenitrile (7.0 g, 19.929 mmol, 1.0 equiv) in THF (70 mL) was treated with NaH (957 mg, 23.9 mmol, 1.2 equiv, 60% dispersion) at 0 °C. The resulting mixture was maintained at that temperature for 1 hour and then TBSCI (3.15 g, 20.9 mmol, 1.05 equiv) was added. The resulting solution was stirred for 2 hours at 0 °C and then the reaction was quenched with ice water. The resulting mixture was extracted with ethyl acetate (3 x 50 mL). The combined organic layers were washed with brine (3 x 30 mL) and dried over anhydrous sodium sulfate. After filtration, the filtrate was concentrated and the residue was purified by silica gel chromatography (15% to 30% ethyl acetate in petroleum ether) to give 2-(6-bromo-1-(3-(*(tert-*butyldimethylsilyl)oxy)-2-(hydroxymethyl)propyl)-1H-indol-3-yl)-2-methylpropanenitrile (6.0 g, 64% yield) of 2-[6-bromo-1-(2-[[(*tert*-butyldimethylsilyl)oxy]methyl]-3-hydroxypropyl)-1H-indol-3-yl]-2-methylpropanenitrile as an orange oil. ESI-MS *m*/*z* = 465.2, 467.2 [M+H]⁺

### Step B

To a stirred solution of 2-(6-bromo-1-(3-((*tert*-butyldimethylsilyloxy)-2-(hydroxymethyl)propyl)-1H-indol-3-yl)-2-methylpropanenitrile (900 mg, 1.933 mmol, 1 equiv) in THF (10 mL) was added NaH (60% in oil, 69.6 mg, 2.9 mmol, 1.5 equiv) in portions at 0 °C. The resulting mixture was stirred for 2 hours at 0 °C. To the mixture was added iodomethane (1.372 g, 9.667 mmol, 5 equiv) dropwise over 30 minutes at 0 °C. The resulting mixture was stirred for an additional 16 hours at room temperature. The reaction was quenched with water at 0 °C and then further diluted with water (200 mL). The resulting mixture was extracted with ethyl acetate (2 x 200 mL) and the combined organic layers were washed with water (2 x 100 mL) and dried over anhydrous sodium sulfate. After filtration, the filtrate was concentrated under reduced pressure to give 2-(6-bromo-1-(3-((*tert*-butyldimethylsilyl)oxy)-2-(methoxymethyl)propyl)-1H-indol-3-yl)-2-methylpropanenitrile (700 mg, crude) that was used in the next step directly without further purification.

### Step C

To a stirred solution of 2-(6-bromo-1-(3-((*tert*-butyldimethylsilyl)oxy)-2-(methoxymethyl)propyl)-1H-indol-3-yl)-2-methylpropanenitrile (700 mg, 1.460 mmol, 1 equiv) in THF (10 mL) at 0 °C was added TBAF (1.75 mL) dropwise. The resulting mixture was stirred for 1 hour at 0 °C. The residue was purified by prep-TLC (EA/PE=1:2) to afford 2-(6-bromo-1-(3-hydroxy-2-(methoxymethyl)propyl)-1H-indol-3-yl)-2-methylpropanenitrile (437 mg, 74% yield) as a yellow oil. ESI-MS *m*/*z =* 365.1 [M+H]⁺.

### BD: 3-(6-bromo-2-methyl-1H-benzo[d]imidazol-1-yl)-2-methoxypropan-1-ol

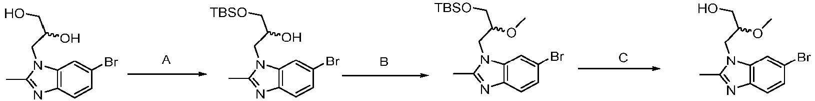

### Step A

A solution of 3-(6-bromo-2-methyl-1H-benzo[d]imidazol-1-yl)propane-1,2-diol (1.4 g, 4.91 mmol, 1.0 equiv), TBSCI (1.48 g, 9.820 mmol, 2.0 equiv), and imidazole (1.34 g, 19.6 mmol, 4.0 equiv) in DMF (10 mL) was stirred for 2 hours at room temperature. The reaction was then quenched by the addition of 100 mL of water. The resulting solution was extracted with ethyl acetate (3 x 100 mL), dried over anhydrous sodium sulfate and concentrated. The residue was purified by silica gel chromatography with ethyl acetate/petroleum ether (1:1) to give 1-(6-bromo-2-methyl-1H-benzo[d]imidazol-1-yl)-3-((*tert-*butyldimethylsilyl)oxy)propan-2-ol (1.0 g, 44% yield) as a yellow solid. ESI-MS *m*/*z* = 399.2 [M+H]⁺.

### Step B

A solution of 1-(6-bromo-2-methyl-1H-benzo[d]imidazol-1-yl)-3-((*tert-*butyldimethylsilyl)oxy)propan-2-ol (1.0 g, 2.504 mmol, 1.0 equiv) in THF (10 mL) at -5 °C was treated with NaH (0.07 g, 2.754 mmol, 1.1 equiv, 60% dispersion in mineral oil) and maintained at that temperature for 30 min. Mel (0.39 g, 2.748 mmol, 1.10 equiv) was added to the reaction solution and stirred for 30 minutes at -5 °C. The reaction was then quenched by the addition of 100 mL of water. The resulting solution was extracted with ethyl acetate (3 x 100 mL) and dried over anhydrous sodium sulfate and concentrated. The residue was purified by silica gel chromatography with ethyl acetate/petroleum ether (1:2) to give 500 mg (38% yield) of 6-bromo-1-(3-((*tert*-butyldimethylsilyl)oxy)-2-methoxypropyl)-2-methyl-¹H-benzo[d]imidazole as a yellow solid. ESI-MS *m*/*z* = 413.1 [M+H]⁺.

### Step C

A solution of 6-bromo-1-(3-((*tert*-butyldimethylsilyl)oxy)-2-methoxypropyl)-2-methyl-1H-benzo[d]imidazole (500 mg, 1.209 mmol, 1 equiv), and TBAF (1 mL, 1.0 M in THF) in THF (5 mL) was stirred for 1 hour at room temperature. The resulting mixture was concentrated and the residue was purified by silica gel chromatography with ethyl acetate/petroleum ether (1:1) to give 300 mg (64% yield) of 3-(6-bromo-2-methyl-1H-benzo[d]imidazol-1-yl)-2-methoxypropan-1-ol as a white solid. ESI-MS *m*/*z* = 299.0 [M+H]⁺.

### BE: 2-(6-bromo-1-(3-fluoro-2-(hydroxymethyl)propyl)-1H-indol-3-yl)-2-methylpropanenitrile

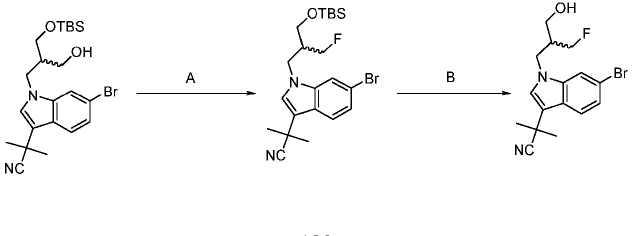

### Step A

To a stirred solution of 2-(6-bromo-1-(3-((*tert*-butyldimethylsilyl)oxy)-2-(hydroxymethyl)propyl)-1H-indol-3-yl)-2-methylpropanenitrile (1.7 g, 3.652 mmol, 1 equiv) in dichloromethane (20 mL) was added sodium carbonate (1.5 g, 14.019 mmol, 3.84 equiv) and DAST (2.3 g, 14.269 mmol, 3.91 equiv) in portions at 0 °C. The resulting mixture was warmed to room temperature and stirred for 15 hours. The reaction was quenched by the addition of 100 mL of ice water. The resulting mixture was extracted with ethyl acetate (3 x 50 mL) and the combined organic layers were washed with brine (100 mL) and dried over anhydrous sodium sulfate. After filtration, the filtrate was concentrated under reduced pressure. The residue was purified by silica gel chromatography, eluting with petroleum ether/ethyl acetate (20:1 to 13:1) to afford 2-(6-bromo-1-(3-((*tert*-butyldimethylsilyl)oxy)-2-(fluoromethyl)propyl)-1H-indol-3-yl)-2-methylpropanenitrile (699 mg, 39% yield) as a yellow oil. ESI-MS *m*/*z =* 469.0 [M+H]⁺.

### Step B

To a stirred solution of 2-(6-bromo-1-(3-((*tert-*butyldimethylsilyl)oxy)-2-(fluoromethyl)propyl)-1H-indol-3-yl)-2-methylpropanenitrile (752 mg, 1.609 mmol, 1.0 equiv) in THF (10 mL ) was added TBAF (1N in THF, 1.9 mL) dropwise at 0 °C. The resulting mixture was stirred for 1 hour at 0 °C to 25 °C. The residue was concentrated under reduced pressure and purified by prep-TLC (petroleum ether/ethyl acetate 1:1) to afford 2-(6-bromo-1-(3-fluoro-2-(hydroxymethyl)propyl)-1H-indol-3-yl)-2-methylpropanenitrile (522 mg, 90% yield) as a white solid. ESI-MS *m*/*z =* 355.1 [M+H]⁺. ¹H-NMR (300 MHz, DMSO-*d*₆) δ 7.79 (d, *J =* 1.7 Hz, 1H), 7.70 (d, *J =* 8.6 Hz, 1H), 7.41 (s, 1H), 7.27 (dd, *J =* 8.6, 1.7 Hz, 1H), 4.86 (t, *J =* 5.1 Hz, 1H), 4.51 (qd, *J =* 9.3*,* 4.8 Hz, 1H), 4.35 (qd, *J =* 9.3, 4.9 Hz, 1H), 4.20 (dd, *J* = 7.2, 2.7 Hz, 2H), 3.40 (d, *J =* 7.5 Hz, 2H), 2.40 - 2.19 (m, 1H), 1.76 (s, 6H).

### BF: 2-(6-bromo-1-(3,3-difluoro-2-(hydroxymethyl)propyl)-1H-indol-3-yl)-2-methylpropanenitrile

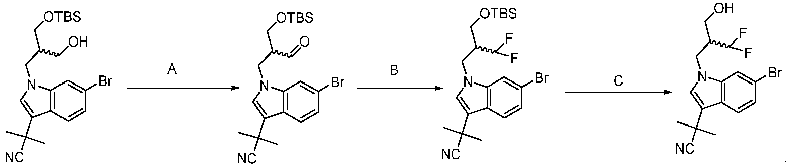

### Step A

A solution of 2-[6-bromo-1-(2-[[(*tert*-butyldimethylsilyl)oxy]methyl]-3-hydroxypropyl)-1H-indol-3-yl]-2-methylpropanenitrile (2.0 g, 4.30 mmol, 1 equiv), dichloromethane (20 mL) and Dess-Martin periodinane (2.73 g, 6.45 mmol, 1.5 equiv) was stirred for 2 hours at room temperature. The reaction was quenched with aqueous NaHCO₃. The resulting mixture was filtered and the filter cake was washed with ethyl acetate. The resulting mixture was extracted with ethyl acetate (3 x 150 mL) and the combined organic layers were washed with brine and dried over anhydrous sodium sulfate. After filtration, the filtrate was concentrated under reduced pressure. The residue was purified by silica gel chromatography, eluting with petroleum ether/ethyl acetate (2:1) to afford 2-[6-bromo-1-(2-[[(*tert-*butyldimethylsilyl)oxy]methyl]-3-oxopropyl)-1H-indol-3-yl]-2-methylpropanenitrile (1.5 g, 68% yield) as a yellow oil. ESI-MS *m*/*z =* 463.1, 465.1 [M+H]⁺.

### Step B

A solution of 2-[6-bromo-1-(2-[[(*tert*-butyldimethylsilyl)oxy]methyl]-3-oxopropyl)-1H-indol-3-yl]-2-methylpropanenitrile (1.5 g, 3.236 mmol, 1 equiv), dichloromethane (15 mL), Na₂CO₃ (1.37 g, 12.945 mmol, 4 equiv) and DAST (3.13 g, 19.418 mmol, 6 equiv) was stirred for 16 hours. The mixture was diluted with water (150 mL) and extracted with ethyl acetate (3 x 100 mL). The combined organic layers were washed with brine and dried over anhydrous sodium sulfate. After filtration, the filtrate was concentrated under reduced pressure to afford 2-(6-bromo-1-[3-[(tert-butyldimethylsilyl)oxy]-2-(difluoromethyl)propyl]-1H-indol-3-yl)-2-methylpropanenitrile (1.8 g) as a yellow oil. The crude product was used in the next step directly without further purification. ESI-MS *m*/*z =* 485.0 [M+H]⁺.

### Step C

To a stirred solution of 2-(6-bromo-1-[3-[(*tert*-butyldimethylsilyl)oxy]-2-(difluoromethyl)propyl]-1H-indol-3-yl)-2-methylpropanenitrile (1.8 g, 3.708 mmol, 1 equiv) in THF (20 mL) was added TBAF (11 mL) dropwise at 0 °C. The resulting mixture was stirred for 1 hour at room temperature and then concentrated under reduced pressure. The residue was purified by prep-TLC (PE/ethyl acetate 1:1) to afford 2-[6-bromo-1-[2-(difluoromethyl)-3-hydroxypropyl]-1H-indol-3-yl]-2-methylpropanenitrile (600 mg, 39% yield) as a yellow solid. ESI-MS *m*/*z =* 371.0 [M+H]⁺.

### BG: 4-(6-bromo-3-(2-cyanopropan-2-yl)-1H-indol-1-yl)-3-(hydroxymethyl)butanenitrile

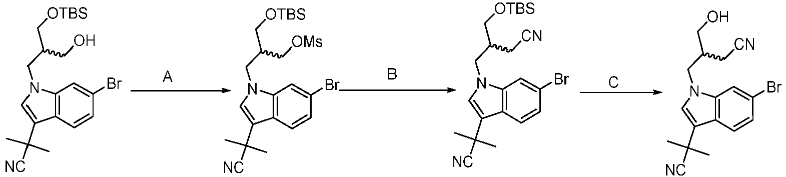

### Step A

To a stirred solution of 2-[6-bromo-1-(2-[[(*tert*-butyldimethylsilyl)oxy]methyl]-3-hydroxypropyl)-1H-indol-3-yl]-2-methylpropanenitrile (1.5 g, 3.222 mmol, 1 equiv) in dichloromethane (20 mL) was added TEA (855 mg, 8.45 mmol, 2.62 equiv) and MsCl (480 mg, 4.190 mmol, 1.30 equiv) dropwise at 0 °C. The resulting mixture was stirred for 30 minutes at 0 °C. The reaction was quenched with water and the resulting mixture was extracted with ethyl acetate. The combined organic layers were washed brine and dried over anhydrous sodium sulfate. After filtration, the filtrate was concentrated under reduced pressure and the residue was purified by prep-TLC (PE/ethyl acetate 2:1) to afford 2-[[6-bromo-3-(1-cyano-1-methylethyl)-1H-indol-1-yl]methyl]-3-[(tert-butyldimethylsilyl)oxy]propyl methanesulfonate (1.5 g, 77% yield) as a yellow oil. ESI-MS *m*/*z =* 565.1, [M+Na]⁺.

### Step B

A solution of 2-[[6-bromo-3-(1-cyano-1-methylethyl)-1H-indol-1-yl]methyl]-3-[(*tert-*butyldimethylsilyl)oxy]propyl methanesulfonate (1.5 g, 2.759 mmol, 1 equiv), DMF (15 mL), H₂O (1.5 mL) and KCN (900 mg, 13.822 mmol, 5.01 equiv) was stirred for 5 hours at 50 °C and then extracted with ethyl acetate. The combined organic layers were washed with brine and dried over anhydrous sodium sulfate. After filtration, the filtrate was concentrated under reduced pressure to afford 4-[6-bromo-3-(1-cyano-1-methylethyl)-1H-indol-1-yl]-3-[[(*tert*-butyldimethylsilyl)oxy]methyl]butanenitrile(1.6 g) as a yellow oil that was carried forward without further purification. ESI-MS *m*/*z* = 474.2 [M+H]⁺.

### Step C

A solution of 4-[6-bromo-3-(1-cyano-1-methylethyl)-1H-indol-1-yl]-3-[[(tertbutyldimethylsilyl)oxy]methyl]butanenitrile (1.6 g, 3.372 mmol, 1 equiv), THF (20 mL) and TBAF (1.0 M in THF, 6.74 mmol, 2 equiv) at room temperature. The resulting mixture was stirred for 1 hour at room temperature and was then concentrated under reduced pressure. The residue was purified by prep-TLC (PE/ethyl acetate 1:2) to afford 3-[[6-bromo-3-(1-cyano-1-methylethyl)-1H-indol-1-yl]methyl]-4-hydroxybutanenitrile (700 mg,58% yield) as a yellow oil. ESI-MS *m*/*z =* 360.1 [M+H]⁺.

### BH: 3-(6-bromo-3-(2-cyanopropan-2-yl)-1H-indol-1-yl)-2-chloropropyl acetate

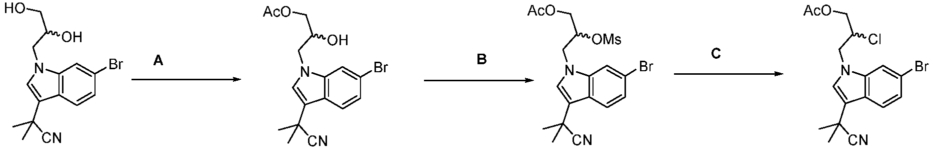

### Step A

To a stirred mixture of 2-(6-bromo-1H-indol-3-yl)-2-methylpropanenitrile (1.4 g, 5.34 mmol, 1.0 equiv) in DMF (20 mL) was added (2,2-dimethyl-1,3-dioxolan-4-yl)methyl methanesulfonate (2.3 g, 8.01 mmol, 1.5 equiv), Cs₂CO₃ (4.35 g, 13.35 mmol, 2.5 equiv), and KI (88.6 mg, 0.534 mmol, 0.1 equiv). The reaction was stirred at 45 °C for 48 hours. The resulting mixture was diluted with water (100 mL) and extracted with ethyl acetate (3 x 100 mL). The combined organic layers were dried over sodium sulfate and concentrated under reduced pressure. The residue was purified by silica gel chromatography, eluting with petroleum ether/ethyl acetate (8:1) to afford 2-(6-bromo-1-((2,2-dimethyl-1,3-dioxolan-4-yl)methyl)-1H-indol-3-yl)-2-methylpropanenitrile (1.7 g, 84% yield) as an yellow oil. ESI-MS *m*/*z =* 377.3 [M+H]⁺.

### Step B

To a stirred mixture of 2-(6-bromo-1-((2,2-dimethyl-1,3-dioxolan-4-yl)methyl)-1H-indol-3-yl)-2-methylpropanenitrile (1.7 g, 4.50 mmol, 1.0 equiv) in THF (10 mL) and water (10 mL) was added TsOH (1.78 g, 10.37 mmol, 2.3 equiv). The reaction was stirred at 30 °C for 15 hours. The resulting mixture was diluted with water (100 mL) and extracted with ethyl acetate (3 x 100 mL). The combined organic layers were dried over sodium sulfate and concentrated under reduced pressure. The residue was purified by silica gel chromatography, eluting with petroleum ether/ethyl acetate (1:1) to afford 2-(6-bromo-1-(2,3-dihydroxypropyl)-1H-indol-3-yl)-2-methylpropanenitrile (1.4 g, 92% yield) as an yellow oil. ESI-MS *m*/*z =* 337.1 [M+H]⁺.

### Step C

A solution of 3-[6-bromo-3-(1-cyano-1-methylethyl)-1H-indol-1-yl]-2-(methanesulfonyloxy)propyl acetate (120 mg, 0.262 mmol, 1 equiv) and LiCl (111 mg, 2.62 mmol, 10 equiv) in DMF (2 mL) was stirred for 4 hours at 80 °C. The reaction was quenched by the addition of 20 mL of water and the resulting solution was extracted with ethyl acetate (3 x 20 mL). The organics were dried over anhydrous sodium sulfate, filtered, and concentrated. The residue was purified by silica gel chromatography with ethyl acetate/petroleum ether (1:4) to give 3-[6-bromo-3-(1-cyano-1-methylethyl)-1H-indol-1-yl]-2-chloropropyl acetate (100 mg, 96% yield) as a yellow solid. ESI-MS *m*/*z =* 397.1/399.1 [M+H]⁺.

### BI: 1-(3-amino-2,2-dimethylpropyl)-6-bromo-1H-indole-3-carbonitrile

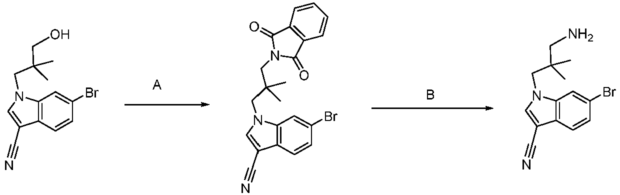

### Step A

DIAD (6.6 g, 32.7 mmol, 2.0 equiv) was added to a solution of 6-bromo-1-(3-hydroxy-2,2-dimethylpropyl)-1H-indole-3-carbonitrile (5.0 g, 16.3 mmol, 1.0 equiv), isoindoline-1,3-dione, and PPh₃ (8.6 g, 32.7 mmol, 2.0 equiv) in THF (100 mL) at 0 °C. The resulting solution was stirred 16 hours at room temperature. The mixture was diluted with ethyl acetate (200 mL) and then washed with water (100 mL x 2) and brine (150 mL). The organic phase was dried over sodium sulfate, filtered, and concentrated to give a residue. The residue was purified by silica gel chromatography (ethyl acetate/petroleum ether = 1/5) to give a mixture of 6-bromo-1-(3-(1,3-dioxoisoindolin-2-yl)-2,2-dimethylpropyl)-1H-indole-3-carbonitrile and triphenylphosphine oxide (12.0 g) as a brown oil. ESI-MS *m*/*z* = 436.0 [M+H]⁺.

### Step B

A mixture of 6-bromo-1-(3-(1,3-dioxoisoindolin-2-yl)-2,2-dimethylpropyl)-1H-indole-3-carbonitrile (5.0 g, 11.5 mmol, 1.0 equiv) and hydrazide-hydrate (5.75 g, 115.0 mmol, 10.0 equiv) in EtOH (150 mL) was stirred at 85 °C for 6 hours. The mixture was diluted with ethyl acetate (100 mL) and washed with water (50 mL x 2) and brine (80 mL). The organic phase was collected, dried over sodium sulfate, filtered and concentrated to give a crude residue that was purified by silica gel chromatography (ethyl acetate/petroleum ether (2:1)) to give 1-(3-amino-2,2-dimethylpropyl)-6-bromo-1H-indole-3-carbonitrile (3.0 g, 60% yield) as a brown solid. ESI-MS *m*/*z* = 306.0 [M+H]⁺.

### BJ: 6-bromo-1-(2,2-dimethyl-3-(methylamino)propyl)-1H-indole-3-carbonitrile

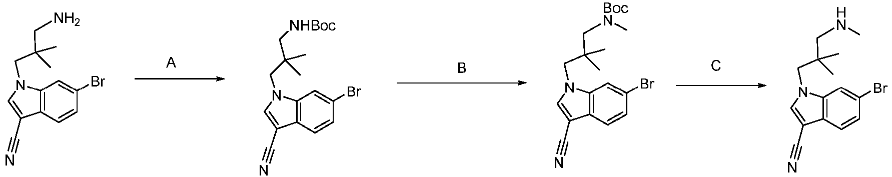

### Step A

A mixture of 1-(3-amino-2,2-dimethylpropyl)-6-bromo-1H-indole-3-carbonitrile (500 mg, 1.64 mmol, 1.0 equiv), Boc₂O (429 mg, 1.968 mmol, 1.2 equiv), and Et₃N (331 mg, 3.28 mmol, 2.0 equiv) in dichloromethane (20 mL) was stirred at room temperature for 16 hours. The mixture was diluted with ethyl acetate (50 mL) and then washed with saturated NaHCO₃ (35 mL), water (50 mL x 2), and brine (50 mL). The organic phase was collected, dried over sodium sulfate, filtered, and concentrated to give a residue. The residue was purified by silica gel chromatography (ethyl acetate/petroleum ether (1:5)) to give *tert*-butyl (3-(6-bromo-3-cyano-1H-indol-1-yl)-2,2-dimethylpropyl)carbamate (550 mg, 80% yield) as an off-white solid. ESI-MS *m*/*z* = 406.1 [M+H]⁺.

### Step B

NaH (63 mg, 60% dispersion in mineral oil, 2.62 mmol, 2.0 equiv) was added to a solution of *tert-*butyl (3-(6-bromo-3-cyano-1H-indol-1-yl)-2,2-dimethylpropyl)carbamate (530 mg, 1.31 mmol, 1.0 equiv) in DMF (10 mL) at 0 °C. The mixture was stirred 0.5 hours at 15 °C and then iodomethane (223 mg, 1.57 mmol, 1.2 equiv) was added at 0 °C and the mixture was stirred 16 hours at room temperature. Water (100 uL) was added to the reaction mixture and then the mixture was diluted with ethyl acetate (100 mL) and washed with water (100 mL x 2) and brine (150 mL). The organic phase was collected, dried over sodium sulfate, filtered and concentrated to give a residue. The residue was purified by silica gel chromatography (ethyl acetate/petroleum ether (1:5)) to give tert-butyl (3-(6-bromo-3-cyano-1H-indol-1-yl)-2,2-dimethylpropyl)(methyl)carbamate (400 mg, 73% yield) as an off-white solid. ESI-MS *m*/*z* = 420.1 [M+H]⁺.

### Step C

*tert*-Butyl (3-(6-bromo-3-cyano-1H-indol-1-yl)-2,2-dimethylpropyl)(methyl)carbamate (400 mg, 0.95 mmol, 1.0 equiv) was dissolved in methanolic HCl (4 M, 10 mL) and stirred at room temperature for 16 hours. The mixture was concentrated and the residue was purified by silica gel chromatography (ethyl acetate/petroleum ether (2:1)) to give 6-bromo-1-(2,2-dimethyl-3-(methylamino)propyl)-1H-indole-3-carbonitrile (300 mg, 99% yield) as an off-white solid. ESI-MS *m*/*z* = 320.1 [M+H]⁺.

### BK: 1-(3-hydroxy-2,2-dimethylpropyl)-6-(4,4,5,5-tetramethyl-1,3,2-dioxaborolan-2-yl)-1H-indole-3-carbonitrile

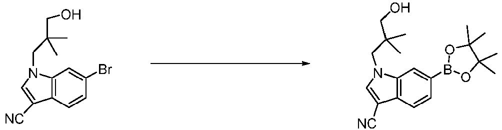

To a solution of 6-bromo-1-(3-hydroxy-2,2-dimethylpropyl)-1*H*-indole-3-carbonitrile (2.0 g, 6.6 mmol, 1.0 equiv), 4,4,4',4',5,5,5',5'-octamethyl-2,2'-bi(1,3,2-dioxaborolane) (2.48 g, 9.8 mmol, 1.5 eq), Pd(dppf)Cl₂ (600 mg, 1.98 mmol, 0.3 equiv), and potassium acetate (1.9 g, 19.8 mmol, 3.0 equiv) in dioxane (100 mL) was stirred at 90 °C for 2 hours. After concentration, the mixture was diluted with water (50 mL) and extracted with ethyl acetate (30 mL × 3). The layers were separated and the organic layer was washed with brine (30 mL), dried over anhydrous sodium sulfate, filtered, and the solvent was removed under reduced pressure. The residue was purified by silica gel chromatography (petroleum/ethyl acetate (5:1 to 3:1)) to afford 1-(3-hydroxy-2,2-dimethylpropyl)-6-(4,4,5,5-tetramethyl-1,3,2-dioxaborolan-2-yl)-1*H*-indole-3-carbonitrile (2.0 g, 87% yield) as a light yellow solid. ESI-MS *m*/*z* = 355.2 [M+H]⁺.

The following intermediates were synthesized according to the procedure described to make Intermediate BK using appropriate building blocks and modified reaction conditions (such as reagents, ratio of reagents, temperature, and reaction time) as needed.

| **Intermediate No.** | **Structure** | **Analytical Data** |
|---|---|---|
| BK-1 | 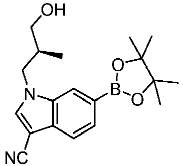 | ESI-MS *m*/*z* = 341.2 [M+H]+ |
| BK-2 | 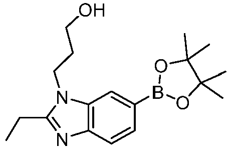 | ESI-MS *m*/*z* = 331.3 [M+H]+ |
| BK-3 | 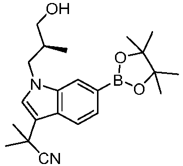 | ESI-MS *m*/*z* = 383.3 [M+H]+ |

### BL: 7-bromo-9-[(2S)-3-hydroxy-2-methyl-propyl]-4-methyl-1,3-dihydropyrano[3,4-b]indole-4-carbonitrile

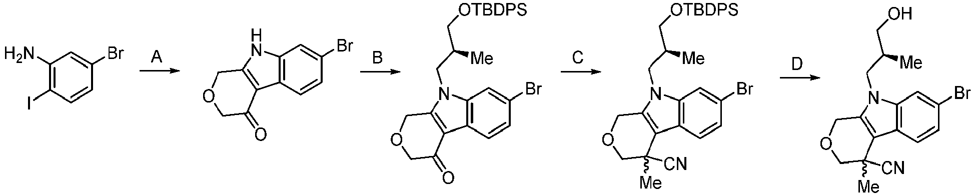

### Step A

To a solution of tetrahydropyran-3,5-dione (400 mg, 3.51 mmol, 1.0 equiv) and 5-bromo-2-iodoaniline (1149 mg, 3.86 mmol, 1.1 equiv) in toluene (11.7mL) was added PTSA monohydrate (67 mg, 0.35 mmol, 0.1 equiv) and the reaction mixture was stirred for 8 hours at reflux using a Dean-Stark trap). Then, the mixture was cooled to room temperature and quenched with 1N NaOH and extracted with ethyl acetate, washed with brine, dried over magnesium sulfate, and concentrated *in vacuo.* The crude mixture was used for the next step without further purification.

To a solution of 3-(5-bromo-2-iodo-anilino)-2H-pyran-5-one (1300 mg, 3.3 mmol, 1 equiv) in DMSO (13.2mL) was added L-proline (76 mg, 0.66 mmol, 0.2 equiv), KOH (740 mg, 13.2 mmol, 4 equiv), and Cul (63 mg, 0.33 mmol, 0.1 equiv), and the reaction mixture was stirred at 90 °C for 14 hours. The reaction mixture was cooled to room temperature and water/ethyl acetate was added. 1 N aqueous HCl was slowly added and the organic phase was combined, washed with brine, and dried over magnesium sulfate. The crude mixture was purified by silica gel chromatography (ethyl acetate/hexanes) to give 7-bromo-1,9-dihydropyrano[3,4-b]indol-4-one (640 mg, 73% yield over 2 steps). ESI-MS *m*/*z =* 266.0 [M+H]⁺. ¹H NMR (400 MHz, DMSO-*d*₆) δ 12.09 (s, 1H), 7.82 (d, *J* = 8.3 Hz, 1H), 7.70 (d, *J* = 1.8 Hz, 1H), 7.34 (dd, *J =* 8.3, 1.8 Hz, 1H), 4.99 (s, 2H), 4.15 (s, 2H).

### Step B

To a solution of 7-bromo-1,9-dihydropyrano[3,4-b]indol-4-one (600 mg, 2.25 mmol, 1 equiv) and [(2R)-3-bromo-2-methyl-propoxy]-*tert*-butyl-diphenyl-silane (1.324 g, 3.38 mmol, 1.5 equiv) in DMF (35 mL) was added potassium carbonate (935 mg, 6.76 mmol, 3 equiv) and NaI (34 mg, 0.23 mmol, 0.1 equiv) at room temperature and the reaction mixture was stirred for 16 hours at 70 °C. The reaction was cooled and quenched with water and diluted with ethyl acetate. The separated organic layers were washed with brine. The organics were separated and dried over magnesium sulfate before concentration to dryness. The crude product was then purified by flash column chromatography eluting with ethyl acetate and hexanes to give 7-bromo-9-[(2*S*)-3-[*tert*-butyl(diphenyl)silyl]oxy-2-methyl-propyl]-1H-pyrano[3,4-b]indol-4-one (900 mg, 69% yield). ESI-MS *m*/*z =* 575.7 [M+H]⁺. ¹H NMR (400 MHz, CDCl₃) 8.05 (d, *J =* 8.4 Hz, 1H), 7.66 - 7.62 (m, 4H), 7.55 (d, *J =* 1.7 Hz, 1H), 7.49 - 7.36 (m, 7H), 4.92 (s, 2H), 4.31 (dd, *J =* 14.5, 6.2 Hz, 1H), 4.27 - 4.16 (m, 2H), 3.76 (dd, *J =* 14.5, 8.5 Hz, 1H), 3.61 (dd, *J =* 10.5, 4.0 Hz, 1H), 3.51 (dd, *J =* 10.6, 6.5 Hz, 1H), 2.29 - 2.12 (m, 1H), 1.13 (s, 9H), 0.87 (d, *J =* 6.8 Hz, 3H).

### Step C

To a solution of 7-bromo-9-[(2S)-3-[*tert*-butyl(diphenyl)silyl]oxy-2-methyl-propyl]-1H-pyrano[3,4-b]indol-4-one (305 mg, 0.53 mmol, 1 equiv) in THF (7.5 mL) was added MeMgBr (3 M in ether, 0.44 mL, 1.32 mmol, 2.5 equiv) at 0 °C and the reaction mixture was stirred for 1 hours at 0 °C. The reaction was quenched with ammonium chloride (aqueous) and diluted with ethyl acetate. Separated organic layers were washed with brine. The organics were then dried over magnesium sulfate before concentration to dryness. The crude mixture was quickly used for the next step without further purification.

### Step D

To a solution of 7-bromo-9-[(2*S*)-3-[*tert*-butyl(diphenyl)silyl]oxy-2-methyl-propyl]-4-methyl-1,3-dihydropyrano[3,4-b]indol-4-ol (300 mg, 0.51 mmol, 1 equiv) in dichloromethane (8.4 mL) was added TMSCN (0.25 mL, 2.02 mmol, 4 equiv) and BF₃·OEt₂ (0.16 mL, 1.27 mmol, 2.5 equiv) sequentially at -78 °C and the reaction mixture was stirred for 90 minutes. The reaction was quenched with aqueous sodium bicarbonate at -78 °C and diluted with dichloromethane. Separated organic layers were washed with saturated brine solution. The organics were then separated and dried (magnesium sulfate) before concentration to dryness. The crude was then purified by flash column chromatography eluting with ethyl acetate and hexanes to give 7-bromo-9-[(2S)-3-[*tert*-butyl(diphenyl)silyljoxy-2-methylpropyl]-4-methyl-1,3-dihydropyrano[3,4-b]indole-4-carbonitrile (284 mg, 93% over 2 steps). ¹H NMR (400 MHz, CDCl₃) δ 7.64 (m, 8H), 7.58 (d, *J =* 8.5 Hz, 2), 7.54 - 7.49 (m, 2H), 7.48 - 7.36 (m, 12H), 7.28 (dd, *J* = 8.6, 1.4 Hz, 2H), 4.84 - 4.70 (m, 2H), 4.19 (dd, *J =* 6.0, 2.8 Hz, 1H), 4.15 (dd, *J =* 6.4, 3.0 Hz, 1H), 4.10 (d, *J =* 11.0 Hz, 1H), 4.04 (d, *J* = 11.1 Hz, 1H), 3.83 (d, *J =* 11.1 Hz, 1H), 3.79 (d, *J =* 11.1 Hz, 1H), 3.69 - 3.61 (m, 2H), 3.60 - 3.54 (m, 2H), 3.53 - 3.43 (m, 2H), 2.19 - 2.08 (m, 2H), 1.75 (s, 3H), 1.73 (s, 3H), 0.86 (d, *J =* 3.7 Hz, 3H), 0.84 (d, *J =* 3.7 Hz, 3H).

### Step D

To a solution of 7-bromo-9-[(2S)-3-[*tert*-butyl(diphenyl)silyl]oxy-2-methyl-propyl]-4-methyl-1,3-dihydropyrano[3,4-b]indole-4-carbonitrile (284 mg, 0.47 mmol, 1 equiv) in THF (7.8 mL) was added TBAF (1 M in THF, 0.57 mL, 0.57 mmol, 1.2 equiv) at room temperature and the reaction mixture was stirred for 1 hour at room temperature. The reaction was quenched with aqueous ammonium chloride and diluted with ethyl acetate. Separated organic layers were washed with saturated brine solution. The organics were then separated and dried over magnesium sulfate before concentration to dryness. The crude was then purified by flash column chromatography eluting with ethyl acetate and hexanes. The desired fractions were concentrated to dryness *in vacuo* to give 7-bromo-9-[(2S)-3-hydroxy-2-methyl-propyl]-4-methyl-1,3-dihydropyrano[3,4-b]indole-4-carbonitrile (167 mg, 97%). ESI-MS *m*/*z* = 363.0 [M+H]⁺. ¹H NMR (500 MHz, CDCl₃) δ 7.61 (d, *J =* 8.3 Hz, 2H), 7.54 (d, *J =* 1.7 Hz, 1H), 7.53 (d, *J =* 1.6 Hz, 1H), 7.30 (dd, *J =* 8.4, 1.7 Hz, 2H), 4.98 - 4.88 (m, 2H), 4.88 - 4.77 (m, 2H), 4.20 - 4.11 (m, 2H), 4.12 - 4.02 (m, 2H), 3.93 - 3.85 (m, 2H), 3.79 - 3.68 (m, 2H), 3.57 - 3.40 (m, 2H), 2.31 - 2.15 (m, 2H), 1.78 (s, 3H), 1.78 (s, 3H), 1.02 (d, *J =* 3.6 Hz, 3H), 1.00 (d, *J =* 3.5 Hz, 3H).

### BM: 6-bromo-4-((S)-3-hydroxy-2-methylpropyl)-1,2,3,4-tetrahydrocyclopenta[b]indole-1-carbonitrile

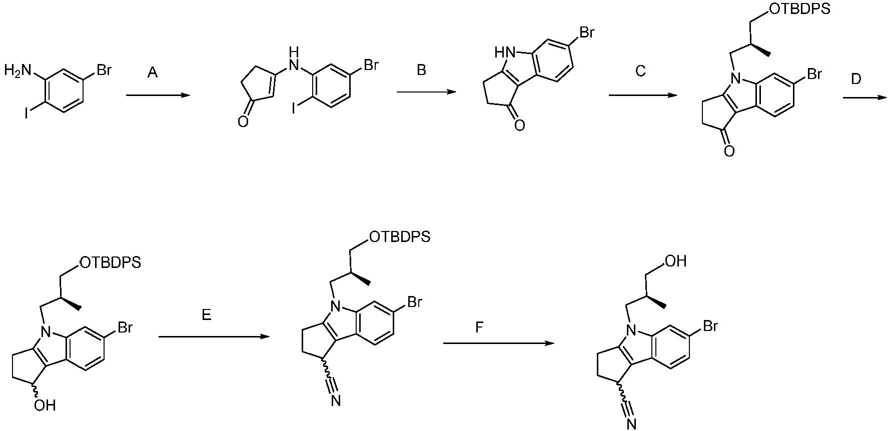

### Step A

A mixture of 5-bromo-2-iodoaniline (5.0 g, 16.8 mmol, 1.0 equiv) and cyclopentane-1,3-dione (1.65 g, 16.8 mmol, 1.0 equiv) in toluene (50 mL) was stirred at 120 °C for 16 hours. The mixture was concentrated and the residue was purified by silica gel chromatography (dichloromethane /MeOH=40/1) to give 3-((5-bromo-2-iodophenyl)amino)cyclopent-2-en-1-one (4.3 g, 68% yield) as a yellow solid. ESI-MS *m*/*z* = 377.9 [M+H]⁺.

### Step B

A mixture of 3-((5-bromo-2-iodophenyl)amino)cyclopent-2-en-1-one (1.0 g, 2.56 mmol, 1.0 equiv), Cul (100 mg, 0.529 mmol, 0.2 equiv), KOH (594 mg, 10.6 mmol, 4.0 equiv), and L-proline (122 mg, 1.06 mmol, 0.4 equiv) in DMSO (100 mL) was stirred at 90 °C for 18 hours. The mixture was diluted with ethyl acetate (1.5 L) and washed with water (500 mL x 2) and brine (500 mL). The organic phase was dried over sodium sulfate, filtered and concentrated to give a residue that was used to next step without further purification. ESI-MS *m*/*z* = 250.1 [M+H]⁺

### Step C

A mixture of 6-bromo-3,4-dihydrocyclopenta[b]indol-1(2*H*)-one (500 mg, 2 mmol, 1.0 equiv), K₂CO₃ (828 mg, 6 mmol, 3.0 equiv), KI (332 mg, 2 mmol, 1.0 equiv), and (*R*)-(3-bromo-2-methylpropoxy)(*tert*-butyl)diphenylsilane (782 mg, 2 mmol, 1.0 equiv) in DMSO (7 mL) was stirred at 110 °C for 16 hours. The mixture was diluted with ethyl acetate (50 mL), then washed with water (50 mL x 2) and brine (50 mL). The organic phase was collected, dried over sodium sulfate, filtered and concentrated to give a residue. The residue was purified by silica gel chromatography (ethyl acetate/Petroleum ether=1/30) to give (*S*)-6-bromo-4-(3-((*tert*-butyldiphenylsilyl)oxy)-2-methylpropyl)-3,4-dihydrocyclopenta[b]indol-1(2H)-one (700 mg, 63% yield) as a yellow oil. ESI-MS *m*/*z* = 560.2 [M+H]⁺.

### Step D

LAH (1 M in THF, 3.1 mL, 3.0 equiv) was added to a solution of (*S*)-6-bromo-4-(3-((*tert-*butyldiphenylsilyl)oxy)-2-methylpropyl)-3,4-dihydrocyclopenta[b]indol-1(2H)-one (700 mg, 1.25 mmol, 1.0 equiv) in THF (10 mL) at 0 °C. The resulting solution was stirred for 0.5 hours at 0-5 °C. Sodium sulfates•10H₂O was added to the reaction mixture, and the mixture was diluted with ethyl acetate (50 mL) and filtered through celite. The filtrate was concentrated to give a crude residue that was used without further purification. ESI-MS *m*/*z* = 544.1 [M-H₂O+H]⁺.

### Step E

A solution of TMSCN (123 mg, 1.24 mmol, 2.0 equiv) in dichloromethane (8 mL) was added to InBr₃ (22 mg, 0.062 mmol, 0.1 equiv) under N₂ and the mixture was stirred at 15 °C for 0.5 hours. A solution of 6-bromo-4-((*S*)-3-((*tert*-butyldiphenylsilyl)oxy)-2-methylpropyl)-1,2,3,4-tetrahydrocyclopenta[b]indol-1-ol (350 mg, 0.62 mmol, 1.0 equiv) in dichloromethane (2 mL) was then added at 0 °C to 5 °C and the final mixture was stirred at 15 °C for 1 hour. Saturated aqueous NaHCO₃ (20 mL) was added to the reaction mixture and the organic phase was collected and washed with water (20 mL x 2) and brine (20 mL). The organics were dried over sodium sulfate, filtered and concentrated to give a residue that was purified by silica gel chromatography (ethyl acetate/Petroleum ether (1:10)) to give 6-bromo-4-((*S*)-3-((*tert*-butyldiphenylsilyl)oxy)-2-methylpropyl)-1,2,3,4-tetrahydrocyclopenta[b]indole-1-carbonitrile (200 mg, 28% yield) as a light yellow solid. ESI-MS *m*/*z =* 593.2 [M+Na]⁺.

### Step F

TBAF (1 M in THF, 0.88 mL, 2.0 equiv) was added to a solution of 6-bromo-4-((*S*)-3-((*tert-*butyldiphenylsilyl)oxy)-2-methylpropyl)-1,2,3,4-tetrahydrocyclopenta[b]indole-1-carbonitrile (250 mg, 0.44 mmol, 1.0 equiv) in THF (5 mL). The resulting solution was stirred for 1 hour at 20 °C. The mixture was diluted with ethyl acetate (20 mL) and washed with water (20 mL x 6) and brine (20 mL). The organic layer was collected, dried over sodium sulfate, filtered and concentrated to give a residue. The residue was purified by silica gel chromatography (ethyl acetate/Petroleum ether (1:1)) to give 6-bromo-4-((S)-3-hydroxy-2-methylpropyl)-1,2,3,4-tetrahydrocyclopenta[b]indole-1-carbonitrile (100 mg, 94% yield) as an off-white solid. ESI-MS *m*/*z* = 333.1 [M+H]⁺.

The following intermediate was synthesized according to the procedure described to make Intermediate BM using appropriate building blocks and modified reaction conditions (such as reagents, ratio of reagents, temperature, and reaction time) as needed.

| **Intermediate No.** | **Structure** | **Analytical Data** |
|---|---|---|
| BM-1 | 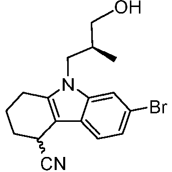 | ESI-MS *m*/*z =* 374.0 [M+H]+. ¹H NMR (400 MHz, DMSO-*d*₆) δ 7.74 (s, 1H), 7.55 - 7.42 (m, 2H), 7.38 - 7.29 (m, 1H), 7.16 (d, *J =* 8.3 Hz, 2H), 7.07 (t, *J =* 7.4 Hz, 1H), 6.39 (s, 1H), 4.50 (t, *J =* 5.1 Hz, 1H), 4.11 (dd, *J =* 14.5, 6.1 Hz, 1H), 3.76 (s, 3H), 3.74 - 3.66 (m, 1H), 3.05 (t, *J* = 5.4 Hz, 2H), 1.79 (dd, *J =* 13.3, 6.7 Hz, 1H), 0.45 (d, *J* = 6.7 Hz, 3H). |

### BN: 3-(6-bromo-3-(3-methoxy-2-methylbutan-2-yl)-1H-indol-1-yl)-2,2-dimethylpropan-1-ol

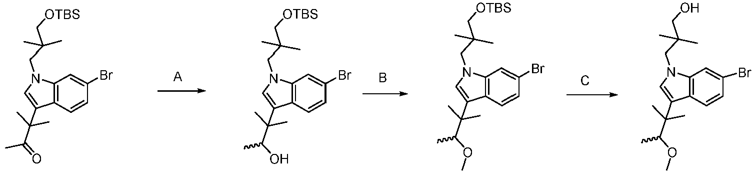

### Step A

To a stirred solution of 3-(6-bromo-1-(3-((*tert*-butyldimethylsilyl)oxy)-2,2-dimethylpropyl)-1H-indol-3-yl)-3-methylbutan-2-one (900 mg, 1.87 mmol, 1.0 equiv) in MeOH (10 mL) at 0 °C was added NaBH₄ (354 mg, 9.36 mmol, 5.0 equiv) in portions. The resulting mixture was stirred for 4 hours at room temperature. The resulting mixture was diluted with water (200 mL) and extracted with ethyl acetate (2 x 200 mL). The combined organic layers were washed with water (2 x 100 mL) and dried over anhydrous sodium sulfate. After filtration, the filtrate was concentrated under reduced pressure. The residue was purified by silica gel column chromatography, eluting with ethyl acetate/petroleum ether (1:12 to 1:7) to afford 3-(6-bromo-1-(3-((*tert*-butyldimethylsilyl)oxy)-2,2-dimethylpropyl)-1H-indol-3-yl)-3-methylbutan-2-ol (900 mg, 90% yield) as a yellow oil. ESI-MS *m*/*z =* 483.2 [M+H]⁺.

### Step B

To a stirred solution of 3-(6-bromo-1-(3-((*tert*-butyldimethylsilyl)oxy)-2,2-dimethylpropyl)-1*H*-indol-3-yl)-3-methylbutan-2-ol (900 mg, 1.483 mmol, 1.0 equiv) in dichloromethane (10 mL) was added tetrafluoroboric acid (240 mg, 1.48 mmol, 1.0 equiv) and TMSCHN₂ (9 mL) dropwise at 0 °C. The resulting mixture was stirred for 5 minutes at 0 °C and then diluted with water (200 mL). The resulting mixture was extracted with ethyl acetate (2 x 200 mL). The combined organic layers were washed with water (2 x 100 mL) and dried over anhydrous sodium sulfate. After filtration, the filtrate was concentrated under reduced pressure. The crude product 6-bromo-1-(3-((*tert*-butyldimethylsilyl)oxy)-2,2-dimethylpropyl)-3-(3-methoxy-2-methylbutan-2-yl)-1H-indole (600 mg, crude) was used in the next step directly without further purification. ESI-MS *m*/*z* = 496.3 [M+H]⁺.

### Step C

To a stirred solution of 6-bromo-1-(3-((*tert*-butyldimethylsilyl)oxy)-2,2-dimethylpropyl)-3-(3-methoxy-2-methylbutan-2-yl)-1H-indole (600 mg, 0.705 mmol, 1.0 equiv) in THF (7 mL) at 0°C was added TBAF (1N in THF, 1.5 mL) dropwise. The resulting mixture was stirred for 1 hour at 0 °C. After removal of solvent, the residue was purified by prep-TLC (petroleum ether/ethyl acetate (2:1)) to afford 3-(6-bromo-3-(3-methoxy-2-methylbutan-2-yl)-1H-indol-1-yl)-2,2-dimethylpropan-1-ol (350 mg, 68% yield) as a yellow oil. ESI-MS *m*/*z =* 382.1 [M+H]⁺.

### BO: 2-(6-bromo-1-((S)-3-hydroxy-2-methylpropyl)-1H-indol-3-yl)-2-cyclopropylacetonitrile

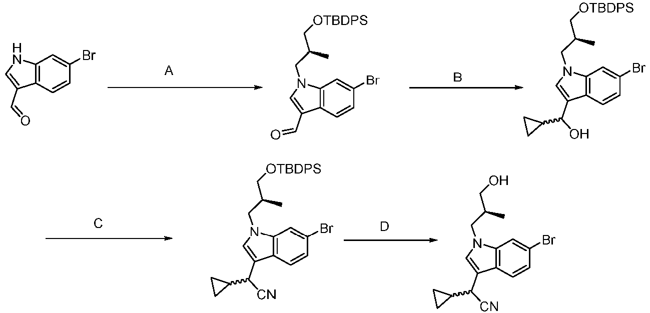

### Step A

To a stirred solution of [(2*R*)-3-bromo-2-methylpropoxy](*tert*-butyl)diphenylsilane (4.67 g, 11.9 mmol, 1.2 equiv) and Cs₂CO₃ (4.86 g, 14.9 mmol, 1.5 equiv) in DMF (50 mL) was added 6-bromo-1H-indole-3-carbaldehyde (2.23 g, 9.9 mmol, 1.0 equiv) dropwise. The mixture was stirred for 3 days at room temperature. Water (500 mL) and ethyl acetate (300 mL) were added. The combined organic layers were washed with brine (3 x 200 mL) and dried over anhydrous sodium sulfate. After filtration, the filtrate was concentrated under reduced pressure. The residue was purified by silica gel chromatography, eluting with petroleum ether/ethyl acetate (5:1) to provide (*S*)-6-bromo-1-(3-((*tert*-butyldiphenylsilyl)oxy)-2-methylpropyl)-1H-indole-3-carbaldehyde (4.8 g, 90% yield) as a yellow oil. ESI-MS *m*/*z* = 534.1 [M+H]⁺.

### Step B

To a stirred solution of (*S*)-6-bromo-1-(3-((*tert*-butyldiphenylsilyl)oxy)-2-methylpropyl)-1H-indole-3-carbaldehyde (2.13 g, 3.99 mmol, 1.0 equiv) in THF (30 mL) was added cyclopropylmagnesiumbromide (1 M, 9.2 mL) dropwise at 0 °C. The resulting mixture was stirred for 2 hours at 0 °C. The reaction was quenched by the addition of saturated aqueous NH₄Cl (30 mL) at 0 °C. Ethyl acetate (30 mL) was added and the organic layer was washed with brine (3 x 100 mL) and dried over anhydrous sodium sulfate. After filtration, the filtrate was concentrated under reduced pressure and the crude product was used in the next step directly without further purification. ESI-MS *m*/*z* = 598.2 [M+Na]⁺.

### Step C

To a stirred solution (6-bromo-1-((*S*)-3-((*tert*-butyldiphenylsilyl)oxy)-2-methylpropyl)-1H-indol-3-yl)(cyclopropyl)methanol (2.3 g, 3.989 mmol, 1.0 equiv) in dichloromethane (20 mL) at -78 °C was added TMSCN (1980 mg, 19.9 mmol, 5.0 equiv) and BF₃•Et₂O (1.415 g, 9.97 mmol, 2.5 equiv) dropwise. The resulting mixture was stirred for 1.5 hours at -78 °C. The reaction was quenched by the addition of saturated aqueous Na₂CO₃ (30 mL) and then diluted with dichloromethane (30 mL). The organic layer was washed with brine (3 x 100 mL) and dried over anhydrous sodium sulfate. After filtration, the filtrate was concentrated under reduced pressure. The residue was purified by silica gel chromatography, eluting with petroleum ether/ethyl acetate (5:1) to afford 2-(6-bromo-1-((*S*)-3-((*tert*-butyldiphenylsilyl)oxy)-2-methylpropyl)-1H-indol-3-yl)-2-cyclopropylacetonitrile (1.5 g, 64% yield) as a yellow oil. ¹H NMR (300 MHz, DMSO-*d*₆) δ 7.76 (s, 1H), 7.58 (t, *J =* 7.0 Hz, 5H), 7.47 - 7.39 (m, 7H), 7.24 (d, *J =* 8.6 Hz, 1H), 4.25 (t, *J =* 10.5 Hz, 1H), 4.08 (t, *J =* 8.8 Hz, 2H), 3.49 (d, *J =* 5.4 Hz,2H), 2.17 (d, *J =* 6.4 Hz, 1H), 1.37 (s, 1H), 1.04 (s, 9H), 0.87 (d, *J =* 6.7 Hz, 3H), 0.63 (s, 1H), 0.54 (s, 1H), 0.47 - 0.30 (m, 2H).

### Step D

To a stirred solution of 2-(6-bromo-1-((*S*)-3-((*tert*-butyldiphenylsilyl)oxy)-2-methylpropyl)-1H-indol-3-yl)-2-cyclopropylacetonitrile (1.0 g, 1.707 mmol, 1 equiv) in THF (10 mL) was added HF-pyridine (1 mL, 40%) dropwise at 0 °C. The resulting mixture was stirred for 16 hours and then the reaction was basified to pH 8 with saturated aqueous NaHCO₃. The mixture was extracted with ethyl acetate (3 x 30 mL) and the combined organic layers were washed with brine (3 x 10 mL) and dried over anhydrous sodium sulfate. After filtration, the filtrate was concentrated under reduced pressure and the residue was purified by Prep-TLC (petroleum ether/ethyl acetate (2:1)) to afford 2-(6-bromo-1-((S)-3-hydroxy-2-methylpropyl)-1H-indol-3-yl)-2-cyclopropylacetonitrile (580 mg, 99% yield) as a yellow oil. ¹H NMR (300 MHz, DMSO-*d*₆) δ= 7.78 (d, *J =* 1.7 Hz, 1H), 7.62 (d, *J =* 8.5 Hz, 1H), 7.45 (s, 1H), 7.23 (dd, *J=* 8.5, 1.7 Hz, 1H), 4.75 - 4.60 (m, 1H), 4.20 (dd, *J =* 14.5, 6.3 Hz, 1H), 4.10 (d*, J =* 8.1 Hz, 1H), 3.99 (dd, *J=* 13.8, 7.1 Hz, 1H), 3.25 (dq, *J =* 10.7, 5.5 Hz, 2H), 2.05 (dt, *J =* 13.2, 6.4 Hz, 1H), 1.51 - 1.39 (m, 1H), 0.81 (d, *J =* 6.7 Hz, 3H), 0.71 - 0.56 (m, 2H), 0.43 (ddt, *J* = 22.4, 9.5, 4.7 Hz, 2H).

### BP: 6-bromo-1-(3-hydroxy-2,2-dimethylpropyl)-1H-indole-3-carbonitrile

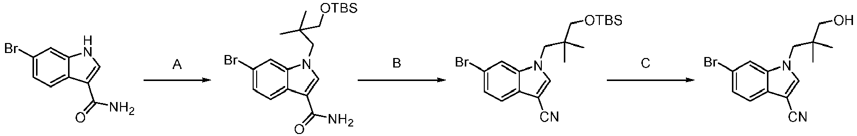

### Step A

A solution of 6-bromo-1H-indole-3-carboxamide (2.39 g, 10.0 mmol, 1.0 equiv), K₂CO₃ (2.76 g, 20.0 mmol, 2.0 equiv), KI (1.66 g, 10.0 mmol, 1.0 equiv), and (3-bromo-2,2-dimethylpropoxy)(*tert-*butyl)dimethylsilane (4.22 g, 15.0 mmol, 1.5 equiv) in DMSO (15 mL) was stirred at 150 °C overnight. After cooling to room temperature, ice water (100 mL) was added and the solution was extracted with ethyl acetate (3 x 100 mL). The combined organic layers were dried over anhydrous sodium sulfate, filtered, and concentrated under reduced pressure to give a crude product, which was purified by silica gel chromatography (dichloromethane to dichloromethane/MeOH (20:1)) to give 6-bromo-1-(3-((*tert-*butyldimethylsilyl)oxy)-2,2-dimethylpropyl)-1H-indole-3-carboxamide (3.25 g, 74 % yield) as an oil. ESI-MS *m*/*z=* 439.1 [M+H]⁺.

### Step B

To a solution of 6-bromo-1-(3-((*tert*-butyldimethylsilyl)oxy)-2,2-dimethylpropyl)-1H-indole-3-carboxamide (3.0 g, 6.83 mmol, 1.0 equiv) in pyridine (30 mL) at 0 °C was added POCl₃ (5.23 g, 34.2 mmol) dropwise. The mixture was stirred at 0 °C for 2 hours. The mixture was poured into water and extracted with ethyl acetate (200 mL). The organic layer was washed with brine (50 mL x 3), dried over anhydrous sodium sulfate, filtered and concentrated to give a residue. The residue was purified by silica gel (petroleum ether to petroleum ether/ethyl acetate (5:1)) to give 6-bromo-1-(3-((*tert-*butyldimethylsilyl)oxy)-2,2-dimethylpropyl)-1H-indole-3-carbonitrile (2.18 g, 76% yield) as a white solid.

### Step C

To a solution of 6-bromo-1-(3-((*tert*-butyldimethylsilyl)oxy)-2,2-dimethylpropyl)-1H-indole-3-carbonitrile (2.11 g, 5.0 mmol, 1.0 equiv) in THF (20 mL) at 0 °C was added TBAF (7.5 mL, 1 M in THF) dropwise. The mixture was stirred at 0 °C for 2 hours. The mixture was poured into water and extracted with ethyl acetate (200 mL). The organic phase was washed with brine (50 mL x 3) and dried over anhydrous sodium sulfate, filtered, and concentrated to give a residue. The residue was purified by silica gel (petroleum ether to petroleum ether/ethyl acetate (3:1)) to give 6-bromo-1-(3-hydroxy-2,2-dimethylpropyl)-1H-indole-3-carbonitrile (1.25 g, 81% yield) as a white solid. ESI-MS *m*/*z* = 309.0 [M+H]⁺.

### BQ: (S)-6-bromo-1-(3-hydroxy-2-methylpropyl)-2-(2-methoxyphenyl)-1H-indole-3-carbonitrile

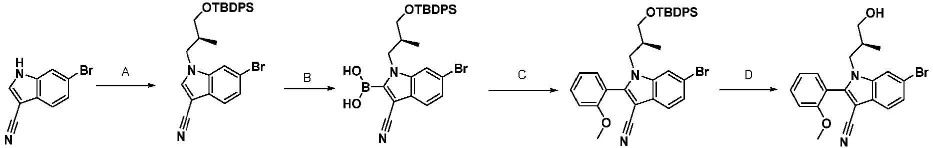

### Step A

A solution of 6-bromo-1H-indole-3-carbonitrile (2.0 g, 9.1 mmol, 1.0 equiv), (*R*)-(3-bromo-2-methylpropoxy)(*tert*-butyl)diphenylsilane (5.3 g, 13.6 mmol, 1.5 equiv), KI (1.5 g, 9.1 mmol, 1.0 equiv), K₂CO₃ (3.8 g, 27.3 mmol, 3.0 equiv), and DMSO (80 mL) was stirred at 130 °C for 16 hours. H₂O (100 mL) was added and the resulting solution was extracted with ethyl acetate (200 mL x 3). The combined organic layers were concentrated under reduced pressure to give a residue. The residue was purified by silica gel chromatography (petroleum ether to petroleum ether/ethyl acetate (95:5)) to afford (*S*)-6-bromo-1-(3-((*tert*-butyldiphenylsilyl) oxy)-2-methylpropyl)-1H-indole-3-carbonitrile (4.6 g, 90% yield) as a white solid. ESI-MS *m*/*z* = 553.2 [M+Na]⁺.

### Step B

To a solution of (*S*)-6-bromo-1-(3-((*tert*-butyldiphenylsilyl)oxy)-2-methylpropyl)-1H-indole-3-carbonitrile (1.0 g, 2.20 mmol, 1.0 equiv) and boron isopropoxide (752 mg, 4.0 mmol, 1.8 equiv) in THF (10 mL) was added LDA (2 M in THF/hexanes, 2.2 mL, 4.4 mmol, 2.0 equiv) dropwise at -78 °C. The mixture was stirred at -78 °C for 0.5 hours. 10 mL ice water was added to the mixture. After warming to room temperature, the layers were separated, and the aqueous phase was extracted with ethyl acetate (20 mL x 3). The combined organic layers were concentrated under reduced pressure to afford 6-bromo-1-(*tert*-butoxycarbonyl)-1*H*-indol-2-yl)boronic acid as a colorless gum. The residue was used for next step without further purification. ESI-MS *m*/*z* = 596.8 [M+Na]⁺.

### Step C

A solution of (6-bromo-1-(*tert*-butoxycarbonyl)-1H-indol-2-yl)boronic acid (2.14 g, 3.70 mmol, 1.0 equiv), 1-iodo-2-methoxybenzene (1.30 g, 5.6 mmol, 1.5 equiv), Pd(dppf)Cl₂ (410 mg, 0.1 equiv), and K₂CO₃ (1.5 g, 11.1 mmol, 3.0 equiv) in toluene (20 mL) and H₂O (12 mL) was stirred at 60 °C for 2 hours. The mixture was separated, and the aqueous phase was extracted with ethyl acetate (20 mL x 3). The combined organic layers were dried and concentrated under reduced pressure to give a residue. The residue was purified by silica gel chromatography (Petroleum/ethyl acetate (3:1 to 1:1)) to afford (S)-6-bromo-1-(3-((*tert*-butyldiphenylsilyl)oxy)-2-methylpropyl)-2-(2-methoxyphenyl)-1*H*-indole-3-carbonitrile (812 mg, 19% yield, 2 steps) as a light-yellow gum. ESI-MS *m*/*z* = 658.9 [M+Na]⁺.

### Step D

To a solution of (*S*)-6-bromo-1-(3-((*tert*-butyldiphenylsilyl)oxy)-2-methylpropyl)-2-(2-methoxyphenyl)-1*H*-indole-3-carbonitrile (1.5 g, 2.36 mmol, 1.0 equiv) in THF (10 mL) was added TBAF (3.5 mL, 1.0 M in THF, 3.5 mmol, 1.5 equiv). The mixture was stirred at 20 °C for 1 hour and then 250 mL of ethyl acetate was poured into the mixture. The resulting solution was then washed with water (10 mL x 8). The organic layer was concentrated under reduced pressure to afford (*S*)-6-bromo-1-(3-hydroxy-2-methylpropyl)-2-(2-methoxyphenyl)-1*H*-indole-3-carbonitrile (1.4 g) as a colorless gum. The crude product was carried on without further purification. ESI-MS *m*/*z* = 398.9 [M+H]⁺.

The following intermediate was synthesized according to the procedure described to make Intermediate BQ using appropriate building blocks and modified reaction conditions (such as reagents, ratio of reagents, temperature, and reaction time) as needed.

| **Intermediate No.** | **Structure** | **Analytical Data** |
|---|---|---|
| BQ-1 | 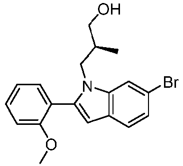 | ESI-MS *m*/*z* = 374.0 [M+H]+. ¹H NMR (400 MHz, DMSO-*d*₆) δ 7.74 (s, 1H), 7.55 - 7.42 (m, 2H), 7.38 - 7.29 (m, 1H), 7.16 (d, *J =* 8.3 Hz, 2H), 7.07 (t, *J =* 7.4 Hz, 1H), 6.39 (s, 1H), 4.50 (t, *J =* 5.1 Hz, 1H), 4.11 (dd, *J =* 14.5, 6.1 Hz, 1H), 3.76 (s, 3H), 3.74 - 3.66 (m, 1H), 3.05 (t, *J* = 5.4 Hz, 2H), 1.79 (dd, *J =* 13.3, 6.7 Hz, 1H), 0.45 (d, *J* = 6.7 Hz, 3H). |

### BR: (S)-3-(6-bromo-3-ethyl-2-(2-methoxyphenyl)-1H-indol-1-yl)-2-methylpropan-1-ol

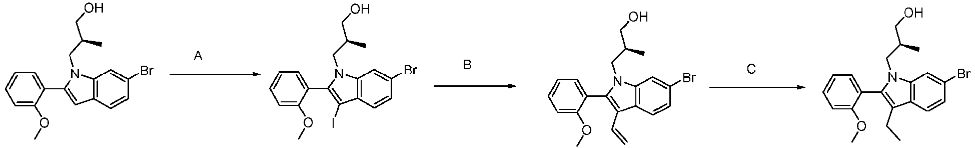

### Step A

A mixture of (*S*)-3-(6-bromo-2-(2-methoxyphenyl)-1H-indol-1-yl)-2-methylpropan-1-ol (1.1 g, 2.9 mmol, 1.0 equiv) and NIS (980 mg, 4.3 mmol, 1.5 equiv) in CH₃CN (50 mL) was stirred for 4 hours at 20 °C. The reaction mixture was concentrated *in vacuo.* The residue was dissolved in ethyl acetate (30 mL) and washed with water (20 mL x 3). The organic layer was dried, filtered, and the solvent removed under reduced pressure. The residue was purified by silica gel chromatography (ethyl acetate/Petroleum ether, 1/3) to yield (*S*)-3-(6-bromo-3-iodo-2-(2-methoxyphenyl)-1H-indol-1-yl)-2-methylpropan-1-ol (1.3 g) as a white solid.

### Step B

To a mixture of (*S*)-3-(6-bromo-3-iodo-2-(2-methoxyphenyl)-1H-indol-1-yl)-2-methylpropan-1-ol (850 mg, 1.7 mmol, 1.0 equiv), K₂CO₃ (705 mg, 5.1 mmol, 3.0 equiv) and potassium vinyltrifluoroborate (455 mg, 3.4 mmol, 2.0 equiv) in toluene/H₂O (5/1, 20 mL) was added Pd(dppf)Cl₂•CH₂Cl₂ (140 mg, 0.1 equiv). After stirring for 3 hours at 110 °C, the reaction mixture was cooled to room temperature and filtered. The filtrate was diluted with ethyl acetate, washed with brine, and concentrated *in vacuo* to yield (*S*)-3-(6-bromo-2-(2-methoxyphenyl)-3-vinyl-1H-indol-1-yl)-2-methylpropan-1-ol (900 mg, crude) as a dark brown solid. The residue was used for the next step without further purification. ESI-MS *m*/*z* = 400.1 [M+H]⁺.

### Step C

A mixture of (*S*)-3-(6-bromo-2-(2-methoxyphenyl)-3-vinyl-1H-indol-1-yl)-2-methylpropan-1-ol (900 mg, crude), diludine (860 mg, 3.4 mmol), and TsOH•H₂O (30 mg) in dichloromethane (30 mL) was stirred for 4 hours at 20 °C. The reaction mixture was diluted with dichloromethane, washed with brine, dried over sodium sulfate, filtered, and concentrated *in vacuo.* The residue was purified by silica gel chromatography (40% ethyl acetate in Petroleum ether) to give (S)-3-(6-bromo-3-ethyl-2-(2-methoxyphenyl)-1H-indol-1-yl)-2-methylpropan-1-ol (410 mg, 60% yield, 2 steps) as a light yellow solid. ESI-MS *m*/*z* = 402.1 [M+H]⁺.

### BS: (S)-6-bromo-3-(2-cyanopropan-2-yl)-1-(3-hydroxy-2-methylpropyl)-1H-indole-2-carbonitrile

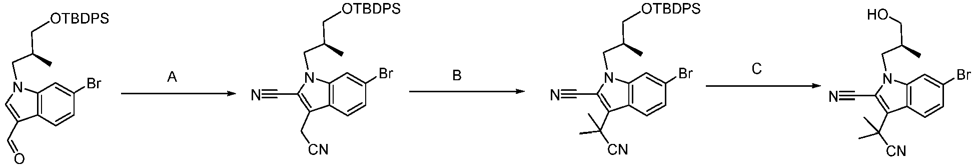

### Step A

To a stirred solution of (*S*)-6-bromo-1-(3-((*tert*-butyldiphenylsilyl)oxy)-2-methylpropyl)-1H-indole-3-carbaldehyde (1.06 g, 2.0 mmol, 1.0 equiv) in DMSO (dry, 30 mL) was added diethyl phosphoryl cyanide (2.0 g, 11.9 mmol, 6.0 equiv) followed by NaCN (0.6 g, 11.9 mmol, 6.0 equiv) at 0 °C. The mixture was stirred at 0 °C to 10 °C for 1 hour. The solution was poured into ice water (200 mL) and extracted with ethyl acetate (100 mL x 3). The combined organic layers were dried and then concentrated to dryness to give a residue. The residue was purified by reverse phase chromatography (90% CH₃CN/water) to give (*S*)-6-bromo-1-(3-((*tert*-butyldiphenylsilyl)oxy)-2-methylpropyl)-3-(cyanomethyl)-1H-indole-2-carbonitrile (810 mg, 75% yield) as an oil. ESI-MS *m*/*z* = 592.2 [M+Na]⁺.

### Step B

(*S*)-6-bromo-1-(3-((*tert-*butyldiphenylsilyl)oxy)-2-methylpropyl)-3-(cyanomethyl)-1H-indole-2-carbonitrile (2.0 g, 3.5 mmol, 1.0 equiv) in THF (50 mL) at -78 °C was treated with NaHMDS (2M in THF, 5.25 mL, 10.5 mmol, 3.0 equiv). The mixture was stirred at -78 °C for 1 hour and then Mel (1.5 g, 10.5 mmol, 3.0 equiv) was added. The reaction mixture was stirred at -78 °C for 30 minutes. The solution was poured into water (1 L) and the solution was extracted with ethyl acetate (300 mL x 3). The combined organic layers were washed with brine (100 mL) and dried over anhydrous sodium sulfate. After filtration, the filtrate was concentrated under reduced pressure. The crude product was purified by silica gel chromatography (petroleum ether/ethyl acetate (20:1)) to give (*S*)-6-bromo-1-(3-((*tert-*butyldiphenylsilyl)oxy)-2-methylpropyl)-3-(2-cyanopropan-2-yl)-1H-indole-2-carbonitrile (2.4 g, 57% yield) as an oil. ESI-MS *m*/*z* = 620.1 [M+Na]⁺. ¹H NMR (400 MHz, CDCl₃) δ 7.96 (d, *J =* 8.8 Hz, 1H), 7.66 (ddd, *J =* 6.4, 3.4, 1.8 Hz, 5H), 7.47 - 7.35 (m, 7H), 4.51 (dd, *J =* 14.7, 5.7 Hz, 1H), 4.06 (dd, *J =* 14.7, 8.7 Hz, 1H), 3.63 (dd, *J =* 10.5, 4.4 Hz, 1H), 3.52 (dd, *J =* 10.5, 6.9 Hz, 1H), 2.33 - 2.23 (m, 1H), 1.98 (d, *J =* 8.1 Hz, 6H), 1.11 (d, *J =* 11.4 Hz, 9H), 0.86 (d, *J =* 6.8 Hz, 3H).

### Step C

To a stirred solution of (*S*)-6-bromo-1-(3-((*tert*-butyldiphenylsilyl)oxy)-2-methylpropyl)-3-(2-cyanopropan-2-yl)-1H-indole-2-carbonitrile (1.6 g, 2.67 mmol, 1.0 equiv) in THF (25 mL) was added AcOH (321 mg, 5.34 mmol, 2.0 equiv) and TBAF (1 M in THF, 5.34 mL, 5.34 mmol, 2.0 equiv) at 0 °C to 5 °C. The resulting mixture was stirred for 6 hours at 0 °C to 5 °C. After concentration, the crude product was diluted with ethyl acetate (50 mL) and washed with water (10 mL x 5). The organic layer was concentrated under reduced pressure to give a residue. The residue was purified by silica gel chromatography (Petroleum ether/ethyl acetate (3:1)) to give (*S*)-6-bromo-3-(2-cyanopropan-2-yl)-1-(3-hydroxy-2-methylpropyl)-1H-indole-2-carbonitrile (870 mg, 90% yield) as a white solid. ESI-MS *m*/*z =* 360.1 [M+H]⁺. ¹H NMR (400 MHz, DMSO-*d₆*) δ 8.03 (d, *J =* 1.6 Hz, 1H), 7.93 (d, *J =* 8.8 Hz, 1H), 7.43 (dd, *J=* 8.8, 1.7 Hz, 1H), 4.76 (s, 1H), 4.38 (dd, *J =* 14.8, 6.5 Hz, 1H), 4.11 (dd, *J =* 14.8, 8.3 Hz, 1H), 3.32 (s, 2H), 2.12 (dd, *J =* 13.7, 6.5 Hz, 1H), 1.94 (s, 6H), 0.82 (d, *J =* 6.8 Hz, 3H).

### BT: 2-(6-bromo-1-(3-hydroxy-2,2-dimethylpropyl)-1H-indol-3-yl)-2-cyclopentylpropanenitrile

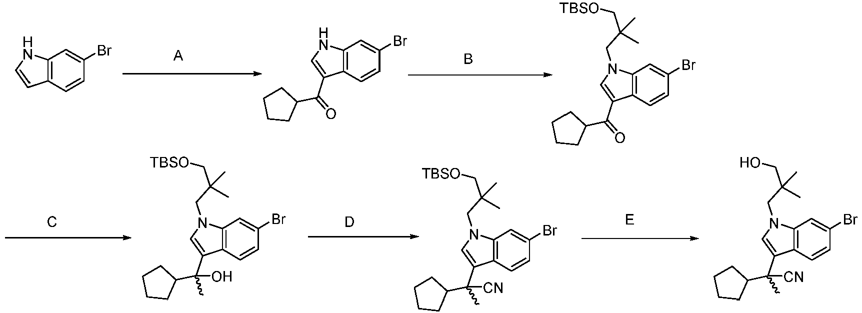

### Step A

SnCl₄ (1 M in dichloromethane, 30.6 mL, 1.2 equiv) was added to a solution of 6-bromo-1H-indole (5.0 g, 25.5 mmol, 1.0 equiv) in dichloromethane (50 mL) at 0 °C. The mixture was stirred at 15 °C for 30 minutes. A solution of cyclopentanecarboxylic acid (2.91 g, 25.5 mmol, 1.0 equiv) in SOCl₂ (0.5 mL) was stirred at 90 °C for 1 hour. The mixture was concentrated to give a residue that was dissolved in dichloromethane (20 mL) and then added to the above mixture, then the final mixture was stirred at 15 °C for 30 min. The reaction was quenched by addition of saturated NaHCO₃ (20 mL) and the solution was then diluted with ethyl acetate (20 mL). The organic phase was separated and washed with water (20 mL x 2) and brine (20 mL) and dried over sodium sulfate. After filtration and concentration, the crude residue was purified by silica gel chromatography (ethyl acetate/Petroleum ether (1:10)) to give (6-bromo-1H-indol-3-yl)(cyclopentyl)methanone (1.98 g, 27% yield) as a brown solid. ESI-MS *m*/*z* = 292.0 [M+H]⁺.

### Step B

A mixture of (6-bromo-1H-indol-3-yl)(cyclopentyl)methanone (2.0 g, 6.85 mmol, 1.0 equiv), Cs₂CO₃ (6.7 g, 20.6 mmol, 3.0 equiv), and (3-bromo-2,2-dimethylpropoxy)(tert-butyl)dimethylsilane (3.85 g, 13.7 mmol, 2.0 equiv) in DMSO (20 mL) was stirred at 100 °C for 16 hours. The mixture was diluted with ethyl acetate (100 mL) and washed with water (100 mL x 2) and brine (100 mL). The organic phase was collected, dried over sodium sulfate, filtered and concentrated to give a residue. The residue was concentrated and purified by silica gel chromatography (ethyl acetate/Petroleum ether (1:20)) to give (6-bromo-1-(3-((*tert*-butyldimethylsilyl)oxy)-2,2-dimethylpropyl)-1*H*-indol-3-yl)(cyclopentyl)methanone (1.4g, 42% yield) as a light yellow solid. ESI-MS *m*/*z* = 492.2 [M+H]⁺.

### Step C

MeMgBr (3M in THF, 1.53 mL, 2.5 equiv) was added to a solution of (6-bromo-1-(3-((tertbutyldimethylsilyl)oxy)-2,2-dimethylpropyl)-1H-indol-3-yl)(cyclopentyl)methanone (900 mg, 1.83 mmol, 1.0 equiv) in THF (20 mL) at 0 °C. The solution was stirred at 0 °C to 5 °C for 1 hour. The reaction was quenched carefully by addition of saturated NH₄Cl (2 mL) at 0 °C. The mixture was diluted with ethyl acetate (25 mL), then washed with brine (15 mL). The organic phase was collected, dried over sodium sulfate, filtered and concentrated to give a residue. The residue was used in the next step without further purification. ESI-MS *m*/*z* = 490.2 [M-H₂O+H]⁺.

### Step D

A solution of TMSCN (363 mg, 3.66 mmol, 2.0 equiv) in dichloromethane (20 mL) was added to InBr₃ (130 mg, 0.2 mmol, 0.2 equiv) under N₂ and the mixture was stirred at 15 °C for 30 minutes. Then a solution of 1-(6-bromo-1-(3-((tert-butyldimethylsilyl)oxy)-2,2-dimethylpropyl)-1H-indol-3-yl)-1-cyclopentylethan-1-ol (930 mg, 1.83 mmol, 1.0 equiv) in dichloromethane (10 mL) was added at 0 °C to 5 °C and the mixture was stirred at 15 °C for 1 hour. Saturated aqueous NaHCO₃ (20 mL) was added to the reaction mixture. The organic phase was collected, washed with water (50 mL x 2) and brine (50 mL), dried over sodium sulfate, filtered, and concentrated to give a residue. The residue was concentrated and purified by silica gel chromatography (ethyl acetate/Petroleum ether (1:30)) to give 2-(6-bromo-1-(3-((*tert*-butyldimethylsilyl)oxy)-2,2-dimethylpropyl)-1H-indol-3-yl)-2-cyclopentylpropanenitrile (300 mg, 34% yield) as a light yellow solid. ESI-MS *m*/*z* = 539.2 [M+Na]⁺.

### Step E

TBAF (1 M in THF, 2.32 mL, 2.0 equiv) was added to a solution of 2-(6-bromo-1-(3-((tertbutyldimethylsilyl)oxy)-2,2-dimethylpropyl)-1H-indol-3-yl)-2-cyclopentylpropanenitrile (600 mg, 1.16 mmol, 1.0 equiv) in THF (20 mL). The resulting solution was stirred for 1 hour at 20 °C. The mixture was diluted with ethyl acetate (20 mL) and then washed with water (20 mL x 3) and brine (20 mL). The organic phase was collected, dried over sodium sulfate, filtered and concentrated to give a residue. The residue was purified by silica gel chromatography (ethyl acetate/Petroleum ether (3:1)) to give 2-(6-bromo-1-(3-hydroxy-2,2-dimethylpropyl)-1H-indol-3-yl)-2-cyclopentylpropanenitrile (380 mg, 97% yield) as an off-white solid. ESI-MS *m*/*z* = 425.1 [M+Na]⁺.

### BU: 3-(6-bromo-3-ethyl-1H-indol-1-yl)-2,2-dimethylpropan-1-ol

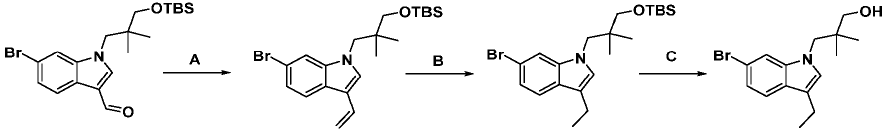

### Step A

To a solution of 6-bromo-1-(3-((*tert*-butyldimethylsilyl)oxy)-2,2-dimethylpropyl)-1H-indole-3-carbaldehyde (2 g, 4.72 mmol, 1.0 equiv) in THF (anhydrous, 15 mL) at -20 °C was added MeMgBr (9.5 mL, 9.5 mmol, 1 M in THF, 2.0 equiv) dropwise. After stirring for 2 hours at this same temperature, aqueous saturated NH₄Cl (20 mL) was added and the resulting solution was extracted with ethyl acetate. The combined organic layers were washed with brine, dried over sodium sulfate, filtered, and concentrated to give crude 1-(6-bromo-1-(3-((*tert*-butyldimethylsilyl)oxy)-2,2-dimethylpropyl)-1H-indol-3-yl)ethan-1-ol (1.99 g, crude) which used in next step directly without further purification.

### Step B

To a mixture of 1-(6-bromo-1-(3-((*tert*-butyldimethylsilyl)oxy)-2,2-dimethylpropyl)-1H-indol-3-yl)ethan-1-ol (1.99 g, crude, 1.0 equiv) and tosic acid hydrate (163 mg, 0.86 mmol, 0.2 equiv) in dichloromethane (20 mL) was added diethyl 2,6-dimethyl-1,4-dihydropyridine-3,5-dicarboxylate (1.09 g, 4.30 mmol, 1.0 equiv). The resulting mixture was stirred for 3 hours at room temperature. The reaction mixture was diluted with dichloromethane and was washed with brine, dried over sodium sulfate, filtered, and concentrated to give the crude product. Purification by silica gel chromatography (ethyl aceate/petroleum ether (1:8)) afforded 6-bromo-1-(3-((tert-butyldimethylsilyl) oxy)-2,2-dimethylpropyl)-3-ethyl-1H-indole (1.22 g, 61% yield two steps).

### Step C

3-(6-Bromo-3-ethyl-1*H*-indol-1-yl)-2,2-dimethylpropan-1-ol was synthesized from 6-bromo-1-(3-((tert-butyldimethylsilyl) oxy)-2,2-dimethylpropyl)-3-ethyl-1H-indole using similar conditions to those described for the synthesis of 6-bromo-1-((1-(hydroxymethyl)cyclopropyl)methyl)-1H-indole-3-carbonitrile. ESI-MS *m*/*z* = 310.1 [M+H]⁺.

### BV: Synthesis of (S)-6-bromo-3-cyclopropyl-1-(3-hydroxy-2-methylpropyl)-1H-indole-2-carbonitrile

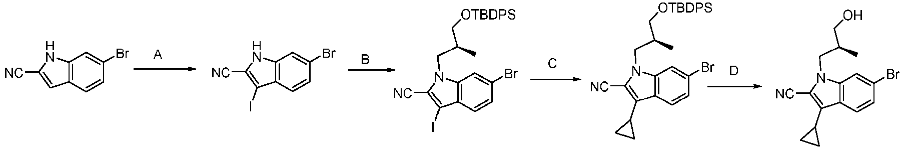

### Step A

To a stirred solution of 6-bromo-1H-indole-2-carbonitrile (3.5 g, 15.8 mmol, 1.0 equiv) in DMF (50 mL) at 0 °C was added NIS (3.8 g, 16.8 mmol, 1.05 equiv) in portions. The resulting mixture was stirred for 1 hour at 20 °C and then poured into water (200 mL) and extracted with ethyl acetate (3 x 100 mL). The combined organic layers were washed with brine (100 mL) and dried over anhydrous sodium sulfate. After filtration, the filtrate was concentrated under reduced pressure. The crude product was purified by silica gel chromatography (Petroleum ether/ethyl acetate (20:1)) to give 6-bromo-3-iodo-1H-indole-2-carbonitrile (5.1 g, 92% yield) as an oil.

### Step B

To a stirred solution of 6-bromo-3-iodo-1H-indole-2-carbonitrile (5.1 g, 14.7 mmol, 1.0 equiv) in DMF (100 mL) was added (*R*)-(3-bromo-2-methylpropoxy)(*tert*-butyl)diphenylsilane (7.2 g, 22.0 mmol, 1.5 equiv) followed by K₂CO₃ (6.1 g, 44.1 mmol, 3.0 equiv). The mixture was stirred at 100 °C for 16 hours and then the solution was poured into water (800 mL) and extracted with ethyl acetate (250 mL x 3). The combined organic layers were washed with water (300 mL x 2) and brine (300 mL x 1) and dried over sodium sulfate and concentrated to give a residue. The residue was purified by silica gel chromatogarphy (petroleum ether/ethyl acetate (100:1)) to give (S)-6-bromo-1-(3-((tert-butyldiphenylsilyl)oxy)-2-methylpropyl)-3-iodo-1H-indole-2-carbonitrile (8.2 g, 84% yield) as an oil. ESI-MS *m*/*z* = 679.0[M+Na]⁺; ¹H NMR (400 MHz, CDCl₃) δ 7.72 - 7.60 (m, 5H), 7.49 - 7.30 (m, 8H), 4.54 (dd, *J =* 14.6, 5.8 Hz, 1H), 4.09 (dd, *J =* 14.6, 8.6 Hz, 1H), 3.61 (dd, *J =* 10.5, 4.3 Hz, 1H), 3.51 (dd, *J =* 10.5, 7.0 Hz, 1H), 2.27 (dd, *J* = 4.2, 2.6 Hz, 1H), 1.12 (s, 9H), 0.85 (t, *J =* 6.0 Hz, 3H).

### Step C

(*S*)-6-Bromo-1-(3-((*tert*-butyldiphenylsilyl}oxy}-2-methylpropyl)-3-iodo-1H-indole-2-carbonitrile (2.0 g, 3.03 mmol, 1.0 equiv), potassium cyclopropyltrifluoroborate (540 mg, 3.64 mmol, 1.2 equiv), Pd(dppf)Cl₂•CH₂Cl₂ (400 mg, 0.49 mmol, 0.16 equiv) and K₂CO₃ (1.25 g, 9.05 mmol, 3.0 equiv) in toluene (80 mL) and water (15 mL) was stirred at 80 °C for 16 hours. After concentration, the residue was purified by C18 reverse phase chromatography (95% CH₃CN/water) to afford (S)-6-bromo-1-(3-((tert-butyldiphenylsilyl)oxy)-2-methylpropyl)-3-cyclopropyl-1H-indole-2-carbonitrile (1.8 g, 51% yield) after combining with a previous batch.

### Step D

To a stirred solution of (S)-6-bromo-1-(3-((tert-butyldiphenylsilyl)oxy)-2-methylpropyl)-3-cyclopropyl-1H-indole-2-carbonitrile (1.7 g, 3.0 mmol, 1.0 equiv) in THF (30 mL) was added TBAF (1 M in THF, 6.0 mL, 6.0 mmol, 2.0 equiv) at 0 °C to 5 °C. The resulting mixture was stirred for 1 hour at 0 °C to 10 °C. After concentration, the crude product was diluted with ethyl acetate (50 mL) and washed with water (10 mL x 5). The organic layer was dried and concentrated under reduced pressure to give a residue. The residue was purified by silica gel chromatography (Petroleum ether/ethyl acetate (10:1)) to give (*S*)-6-bromo-3-cyclopropyl-1-(3-hydroxy-2-methylpropyl)-1H-indole-2-carbonitrile (950 mg, 99% yield) as an oil. ESI-MS *m*/*z* = 333.1 [M+H]⁺. ¹H NMR (400 MHz, DMSO-*d₆*) δ 7.89 (d, *J =* 1.5 Hz, 1H), 7.64 (d, *J* = 8.6 Hz, 1H), 7.28 (dd, *J* = 8.6, 1.7 Hz, 1H), 4.73 (t, *J* = 5.1 Hz, 1H), 4.28 (dd, *J* = 14.7, 6.5 Hz, 1H), 4.01 (dd, *J=* 14.7, 8.3 Hz, 1H), 3.28 (dd, *J =* 9.2, 5.3 Hz, 2H), 2.09 (ddd, *J =* 8.5, 5.3, 3.3 Hz, 2H), 1.10 - 1.02 (m, 2H), 0.92 (ddd, *J =* 6.2, 5.2, 3.7 Hz, 2H), 0.78 (d, *J =* 6.8 Hz, 3H).

### BW: (R)-3-(6-bromo-2-(((3-(trimethylsilyl)prop-2-yn-1-yl)oxy)methyl)-1H-indol-1-yl)-2-methylpropan-1-ol

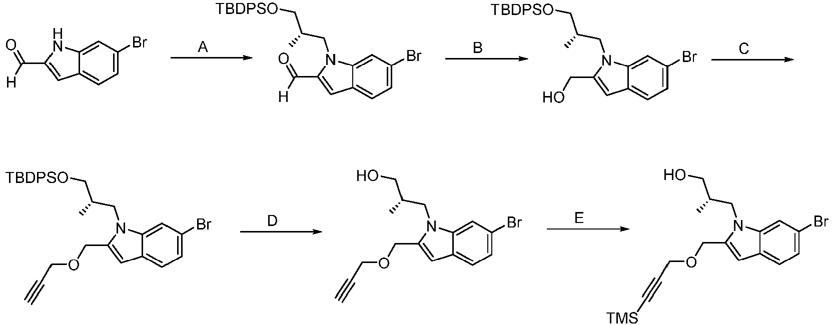

### Step A

To a solution of 6-bromo-1*H*-indole-2-carbaldehyde (0.600 g, 2.25 mmol) and [(2R)-3-bromo-2-methyl-propoxy]-tert-butyl-diphenyl-silane (1.3 g, 3.38 mmol) in DMF (35 mL) was added K₂CO₃ (0.935 g, 6.76 mmol) and Nal (33.8 mg, 0.2300 mmol) and the reaction mixture was stirred for 16 hours at 70 °C. The reaction was quenched with water and diluted with ethyl acetate. Separated organic layers was washed with saturated brine solution. The organics were then separated and dried (magnesium sulfate) before concentration to dryness. The crude product was then purified by flash column chromatography eluting with ethyl acetate and hexanes to give (R)-6-bromo-1-(3-((tert-butyldiphenylsilyl)oxy)-2-methylpropyl)-1H-indole-2-carbaldehyde (0.900 g, 69% yield). ¹H NMR (400 MHz, CDCl₃) δ 9.85 (s, 1H), 7.74 - 7.55 (m, 8H), 7.46 - 7.33 (m, 8H), 7.28 (dd, *J =* 8.6, 1.6 Hz, 1H), 4.67 - 4.44 (m, 3H), 3.66 - 3.45 (m, 3H), 2.23 (ddd, *J =* 8.0, 6.3, 4.6 Hz, 1H), 1.12 (s, 11H), 0.83 (d, *J =* 6.8 Hz, 3H).

### Step B

(R)-6-bromo-1-(3-((*tert*-butyldiphenylsilyl)oxy)-2-methylpropyl)-1H-indole-2-carbaldehyde (0.650 g, 1.2 mmol) was dissolved in methanol (10 mL) and NaBH₄ (0.092 g, 2.4 mmol, 2 equiv)was added in portions. The mixture was stirred for 1 hour at room temperature before being slowly quenched with water and extracted with ethyl acetate. Organics were dried over MgSO₄, filtered and evaporated. Silica gel chromatography eluting with hexanes and ethyl acetate gave (*R*)-(6-bromo-1-(3-((*tert-*butyldiphenylsilyl)oxy)-2-methylpropyl)-1H-indol-2-yl)methanol (0.556 g, 85% yield). ¹H NMR (400 MHz, CDCl₃) δ 7.65 (ddd, *J=* 8.0, 4.9, 1.6 Hz, 4H), 7.47 - 7.31 (m, 6H), 7.19 (dd, *J=* 8.4, 1.7 Hz, 1H), 6.43 (d, *J=* 0.8 Hz, 1H), 4.77 (d, *J =* 1.8 Hz, 2H), 4.40 (d, *J =* 6.3 Hz, 1H), 3.97 (s, 0H), 2.31 (ddd, *J=* 8.4, 6.5, 4.4 Hz, 1H), 1.12 (s, 9H), 0.84 (d, *J =* 6.8 Hz, 3H). ESI-MS *m*/*z* = 536.1 [M+H]⁺.

### Step C

1-(3-((*tert*-butyldiphenylsilyl)oxy)-2-methylpropyl)-1H-indol-2-yl)methanol (0.175 g, 0.783 mmol) was cooled to 0 °C and NaH (0.094 g, 2.35 mmol, 3 equiv) was added in one portion. The reaction stirred for 30 minutes and propargyl bromide (0.131 mL, 1.17 mmol, 1.5 equiv) was then added. The reaction was quenched with aqueous NH₄Cl and extracted with ethyl acetate. The organics were then dried over MgSO₄, filtered and evaporated to get the crude product. Silica gel chromatography with hexanes and ethyl acetate yielded (*R*)-6-bromo-1-(3-((*tert*-butyldiphenylsilyl)oxy)-2-methylpropyl)-2-((prop-2-yn-1-yloxy)methyl)-1H-indole (0.410 g, 91% yield). ¹H NMR (400 MHz, CDCl₃) δ 7.71 - 7.59 (m, 4H), 7.59 - 7.50 (m, 1H), 7.49 - 7.35 (m, 5H), 7.19 (dd, *J =* 8.4, 1.7 Hz, 1H), 6.49 (d, *J =* 0.8 Hz, 1H), 4.71 (s, 2H), 4.38 (dd, *J=* 14.7, 6.2 Hz, 1H), 4.07 (dd, *J =* 2.3, 1.2 Hz, 2H), 3.61 (dd, *J =* 10.3, 4.7 Hz, 1H), 3.52 (dd, *J* = 10.3, 6.2 Hz, 1H), 2.41 (t, *J* = 2.4 Hz, 1H), 2.32 (ddd, *J =* 8.9, 6.8, 5.1 Hz, 1H), 1.12 (s, 9H), 0.84 (d, *J*= 6.8 Hz, 3H). ESI-MS *m*/*z* = 574.1 [M+H]⁺.

### Step D

(*R*)-6-bromo-1-(3-((*tert*-butyldiphenylsilyl)oxy)-2-methylpropyl)-2-((prop-2-yn-1-yloxy)methyl)-1H-indole (0.410 g, 0.7135 mmol) was dissolved in THF (10 mL) and then 1 M TBAF in THF (0.856 mL, 0.856 mmol, 1.2 equiv) was added and the solution stirred for 1 hour. The reaction was quenched with aqueous NH₄Cl and extracted with ethyl acetate. The organics were then dried over MgSO₄, filtered and evaporated to get the crude product. Silica gel chromatography with hexanes and ethyl acetate yielded (*R*)-3-(6-bromo-2-((prop-2-yn-1-yloxy)methyl)-1H-indol-1-yl)-2-methylpropan-1-ol (0.223 g, 92% yield). ¹H NMR (400 MHz, CDCl₃) δ 7.53 - 7.49 (m, 1H), 7.44 (d, *J =* 8.4 Hz, 1H), 7.19 (dd, *J =* 8.4, 1.7 Hz, 1H), 6.52 (d, *J* = 0.9 Hz, 1H), 4.86 - 4.68 (m, 3H), 4.31 - 4.20 (m, 1H), 4.17 (dd, *J =* 5.5, 2.4 Hz, 2H), 3.96 (dd, *J =* 14.7, 6.5 Hz, 1H), 3.44 (dd, *J =* 6.8, 4.4 Hz, 2H), 2.51 (t, *J =* 2.4 Hz, 1H), 2.34 (dddd, *J* = 9.0, 6.7, 4.4, 2.3 Hz, 1H), 1.04 (d, *J =* 7.0 Hz, 4H). ESI-MS *m*/*z* = 336.0 [M+H]⁺.

### Step E

(*R*)-3-(6-bromo-2-((prop-2-yn-1-yloxy)methyl)-1H-indol-1-yl)-2-methylpropan-1-ol (0.188 g, 0.559 mmol) was dissolved in THF (5 mL) and cooled to -78 °C. 1 M LiHMDS in THF (1.17 mL, 1.17 mmol, 2.1 equiv) was slowly added over 15 minutes. The solution was stirred for 30 minutes at -78°C. TMSCI (0.156 mL, 1.23 mmol, 2.2 equiv) was then added dropwise over 5 minutes and the reaction stirred for 1 hour at -78°C. The reaction was quenched with water and warmed to room temperature. 1 M aqueous HCl and ethyl acetate were added and the reaction stirred for 10 minutes. The solution was extracted 3x with ethyl acetate and dried over MgSO4, filtered, and evaporated. Silica gel chromatography with hexanes and ethyl acetate provided (*R*)-3-(6-bromo-2-(((3-(trimethylsilyl)prop-2-yn-1-yl)oxy)methyl)-1H-indol-1-yl)-2-methylpropan-1-ol (0.203 g, 89% yield). ESI-MS *m*/*z* = 408.1 [M+H]⁺.

### BX: (8-bromo-4,5-dihydro-1H,3H-[1,4]oxazepino[4,3-a]indol-4-yl)methanol

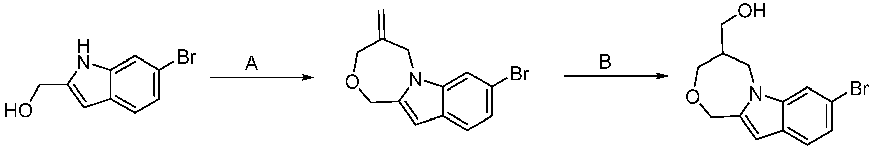

### Step A

(6-bromo-1*H*-indol-2-yl)methanol (4.0 g, 17.7 mmol) in DMF (165 mL) was treated with NaH (60% dispersion, 1.77, g, 44.2 mmol, 2.5 equiv) in one portion. The resulting mixture was stirred for 30 minutes at room temperature and then treated dropwise with dibromo alkene (2.6 mL, 23 mmol, 1.3 equiv). The resulting solution was stirred at room temperature for 2 hours before being quenched by the addition of water and extracted into ethyl acetate. The combined organic layers were washed with water and brine and then dried (MgSO₄), filtered and concentrated *in vacuo.* The crude material was purified over silica gel with hexanes/ethyl acetate affording 8-bromo-4-methylene-4,5-dihydro-1H,3H-[1,4]oxazepino[4,3-a]indole (1.65 g, 34% yield) as a white solid. ¹H NMR (400 MHz, CDCl₃) δ 7.52 - 7.47 (m, 1H), 7.41 (d, *J =* 8.4 Hz, 1H), 7.18 (dd, *J =* 8.4, 1.7 Hz, 1H), 6.34 (t, *J =* 0.8 Hz, 1H), 5.35 (s, 1H), 5.26 (t, *J* = 1.0 Hz, 1H), 4.81 (d, *J =* 0.7 Hz, 2H), 4.73 (d, *J =* 0.7 Hz, 2H), 4.42 (d, *J =* 1.0 Hz, 2H). ESI-MS m/z = 278.0 [M+H]⁺.

### Step B

2 M Borane•Me₂S in THF (2.97 mL, 5.93 mmol, 1 equiv) was added cooling on an ice/methanol bath to a stirred solution of 8-bromo-4-methylene-4,5-dihydro-1H,3H-[1,4]oxazepino[4,3-a]indole (1.65 g, 5.93 mmol) in THF (50 mL) under an atmosphere of argon. The reaction mixture was warmed to 25 °C and stirred at this temperature for 2 hours. Then the reaction mixture was cooled in an ice/methanol bath and slowly treated at this temperature sequentially with 3 N sodium hydroxide (2 mL) and 30% hydrogen peroxide (0.788 mL). The obtained homogenous mixture was stirred overnight, then treated with hexane and dried over potassium carbonate. The organic layer was decanted from the precipitate, which was washed with dichloromethane. The organic layers were evaporated *in vacuo,* and the residue was purified rapidly by chromatography on silica gel to give (8-bromo-4,5-dihydro-1H,3H-[1,4]oxazepino[4,3-a]indol-4-yl)methanol (0.698 g, 40%) as a colorless oil. ¹H NMR (400 MHz, CDCl₃) δ 7.50 (s, 1H), 7.42 (dd, *J =* 8.5, 0.5 Hz, 1H), 7.17 (d, *J =* 6.7 Hz, 1H), 6.40 (s, 1H), 4.82 (d, *J =* 14.3 Hz, 1H), 4.65 - 4.47 (m, 2H), 4.28 (d, *J* = 14.5 Hz, 1H), 4.12 (dd, *J* = 10.4, 2.2 Hz, 1H), 3.98 (d, *J* = 12.5 Hz, 1H), 3.55 (dd, *J =* 10.5, 5.8 Hz, 1H), 3.41 - 3.25 (m, 1H), 2.22 - 2.06 (m, 1H). ESI-MS *m*/*z* = 296.1 [M+H]⁺.

### BY: 3-(6-bromo-2',3',5',6'-tetrahydrospiro[indoline-3,4'-pyran]-1-yl)-2,2-dimethylpropan-1-ol

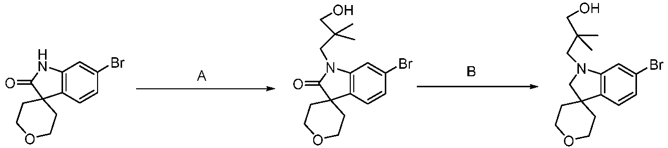

### Step A

A solution of 6-bromo-1,2-dihydrospiro[indole-3,4-oxan]-2-one (800 mg, 2.836 mmol, 1.0 equiv), (3-bromo-2,2-dimethylpropoxy)(*tert*-butyl)dimethylsilane (1.20 g, 4.25 mmol, 1.5 equiv), Cs₂CO₃ (2.21 g , 7.10 mmol, 2.5 equiv), and DMF (8.0 mL) was stirred for 13 hours at 130 °C. The mixture was cooled to room temperature and then TBAF (1.0 M in THF, 8.5 mL) was added dropwise. The resulting mixture was stirred for an additional 2 hours at room temperature and then diluted with ethyl acetate (50 mL). The organic layer was washed with 3 x 40 mL of brine. The organics were dried over sodium sulfate, filtered, and the solvent was removed *in vacuo.* Purification by silica gel column chromatography, eluting with petroleum ether/ethyl acetate (1:2) gave 6-bromo-1-(3-hydroxy-2,2-dimethylpropyl)-2',3',5',6'-tetrahydrospiro[indoline-3,4'-pyran]-2-one (600 mg, 57% yield) as a yellow solid. ESI-MS *m*/*z* = 368.2 [M+H]⁺.

### Step B

A solution of 6-bromo-1-(3-hydroxy-2,2-dimethylpropyl)-2',3',5',6'-tetrahydrospiro[indoline-3,4'-pyran]-2-one (600 mg, 1.63 mmol, 1 equiv), THF (6.0 mL) and BH₃•THF (6.0 mL) was stirred at room temperature for 15 hours. The reaction was quenched by the addition of water (20 mL) at 0 °C and the aqueous layer was extracted with ethyl acetate (3 x 30 mL). T he organics were dried over sodium sulfate, filtered, and the solvent was removed *in vacuo.* The residue was purified by silica gel column chromatography, eluting with petroleum ether/ethyl acetate (1:1) to afford 3-(6-bromo-2',3',5',6'-tetrahydrospiro[indoline-3,4'-pyran]-1-yl)-2,2-dimethylpropan-1-ol (350 mg, 61% yield) as a yellow solid. ESI-MS *m*/*z* = 354.2 [M+H]⁺.

### CA: 3-(5-bromo-1-ethyl-1H-indol-3-yl)-2-methylpropan-1-ol

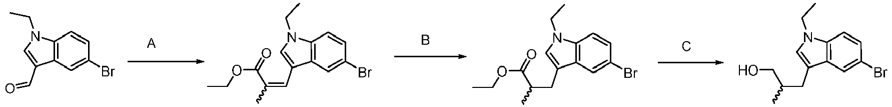

### Step A

Into a 100 mL round-bottom flask was added 5-bromo-1-ethyl-1 H-indole-3-carbaldehyde (5.2 g, 20.63 mmol, 1.0 equiv), methyl 2-(triphenyl-λ-5-phosphanylidene)propanoate (17.96 g, 51.57 mmol, 2.5 equiv) and dichloromethane (50 mL). The resulting mixture was stirred for 15 hours at 35 °C. The resulting mixture was concentrated under vacuum and then the resulting residue was diluted with ethyl acetate (50 mL) and washed with 3 x 50 mL of brine. The organics were dried over sodium sulfate, filtered, and the solvent was removed *in vacuo.* The residue was purified by silica gel chromatography, eluting with petroleum ether/ethyl acetate (2:1) to afford ethyl 3-(5-bromo-1-ethyl-1H-indol-3-yl)-2-methylacrylate (6.1 g, 90% yield) as a yellow solid. ESI-MS *m*/*z* = 336.1 [M+H]⁺.

### Step B

Into a 250 mL round-bottom flask was added ethyl 3-(5-bromo-1-ethyl-1H-indol-3-yl)-2-methylacrylate (5.70 g, 16.95 mmol, 1.0 equiv), 4-methylbenzene-1-sulfonohydrazide (15.79 g, 84.764 mmol, 5.0 equiv) and DMF (50 mL). The mixture was stirred for 15 hours at 110 °C and was then diluted with ethyl acetate (100 mL). The resulting mixture was washed with 3 x 100 mL of brine and the aqueous layer was extracted with ethyl acetate (3 x 50 mL). The organics were dried over sodium sulfate, filtered, and the solvent was removed *in vacuo.* The residue was purified by silica gel column chromatography, eluting with petroleum ether/ethyl acetate (1:2) to afford ethyl 3-(5-bromo-1-ethyl-1H-indol-3-yl)-2-methylpropanoate (3.2 g, 56% yield) as a yellow oil. ESI-MS *m*/*z* = 338.1 [M+H]⁺.

### Step C

Into a 40 mL vial was added ethyl 3-(5-bromo-1-ethyl-1H-indol-3-yl)-2-methylpropanoate (2.00 g, 5.91 mmol, 1.0 equiv), LiBH₄ (515.22 mg, 23.65 mmol, 4.0 equiv) and THF (10 mL). The mixture was stirred for 15 hours at 55 °C. The reaction was quenched with saturated aqueous NH₄Cl after cooling to 0 °C. The resulting mixture was concentrated under vacuum and the resulting aqueous layer was extracted with ethyl acetate (3 x 30 mL). The organics were dried over sodium sulfate, filtered, and the solvent was removed *in vacuo.* The residue was purified by reverse phase chromatography (20-80% MeCN in water) to give 3-(5-bromo-1-ethyl-1H-indol-3-yl)-2-methylpropan-1-ol (1.7 g, 97% yield) as a yellow oil. ESI-MS *m*/*z* = 296.1 [M+H]⁺.

### CB: 2-(5-bromo-3-(3-hydroxy-2-methylpropyl)-1H-indol-1-yl)acetonitrile

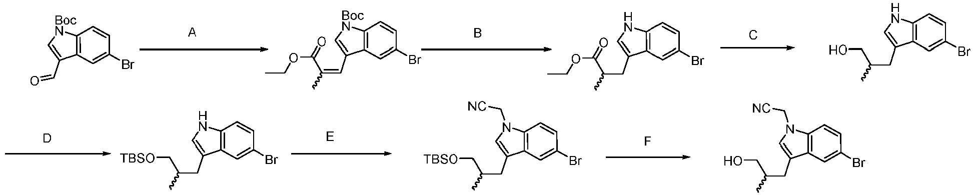

### Step A

A solution of tert-butyl 5-bromo-3-formyl-1H-indole-1-carboxylate (13.4 g, 41.3 mmol, 1.0 equiv) in dichloromethane (140 ml) was treated with ethyl 2-(triphenyl-λ-5-phosphanylidene)propanoate (37.45 g, 103.3 mmol, 2.5 equiv) and was stirred for 24 hours at 35 °C. The solvent was removed and the crude product was purified by silica gel chromatorgraphy (PE/EA= 10% to 20%) to give 16.4 g (97% yield) of tert-butyl 5-bromo-3-(3-ethoxy-2-methyl-3-oxoprop-1-en-1-yl)-1H-indole-1-carboxylate as a yellow solid. ESI-MS *m*/*z* = 408.3 [M+H]⁺.

### Step B

A solution of tert-butyl 5-bromo-3-[(1Z)-3-ethoxy-2-methyl-3-oxoprop-1-en-1-yl]-1H-indole-1-carboxylate (16.4 g, 40.2 mmol, 1.0 equiv), DMF (164 mL) and TsNHNH₂ (74.80 g, 401.7 mmol, 10 equiv) was stirred for 5 days at 110 °C. After cooling, ethyl acetate (200 mL) was added and the solution was washed with water (3 x 100 mL). The organics were dried over sodium sulfate, filtered, and the solvent was removed *in vacuo.* The crude product was purified by silica gel chromatography (petroleum ether/ethyl acetate (10% increasing to 20% within 40 minutes)), affording 6.8 g (55% yield) of ethyl 3-(5-bromo-1H-indol-3-yl)-2-methylpropanoate as a yellow oil. ESI-MS *m*/*z* = 310.1 [M+H]⁺.

### Step C

A solution of ethyl 3-(5-bromo-1H-indol-3-yl)-2-methylpropanoate (5.8 g, 18.7 mmol, 1.0 equiv), THF (60 mL) and LiBH₄ (1.63 g, 74.8 mmol, 4.0 equiv) was stirred for 6 hours at 40 °C. The reaction was quenched by the addition of 30 mL of saturated aqueous NH₄Cl. The resulting solution was extracted with ethyl acetate (3 x 50 mL) and the organic layers were combined. The organics were dried over sodium sulfate, filtered, and the solvent was removed *in vacuo.* The crude product was purified by silica gel chromatography (petroleum ether/ethyl acetate (35% to 55%)) to give 4.85 g (97% yield) of 3-(5-bromo-1H-indol-3-yl)-2-methylpropan-1-ol as a light yellow oil. ESI-MS *m*/*z* = 268.1 [M+H]⁺.

### Step D

Into a 100 mL 3-necked flask was added 3-(5-bromo-1H-indol-3-yl)-2-methylpropan-1-ol (2.0 g, 7.46 mmol, 1 equiv), DMF (20 mL), and 2,6-dimethylpyridine (3.20 g, 29.834 mmol, 4 equiv) at -20 °C. Finally, TBSOTf (5.91 g, 22.375 mmol, 3 equiv) was added dropwise at -20 °C and the resulting solution was stirred for 8 hours at -20 °C. The resulting mixture poured into ethyl acetate (100 mL) and washed with H₂O (3 x 30 mL). The organic layer was concentrated and the crude product was purified by silica gel chromatography (petroleum ether/ethyl acetate (10% to 20%)) to give 2.2 g (77% yield) of 5-bromo-3-(3-((*tert*-butyldimethylsilyl)oxy)-2-methylpropyl)-1H-indole as a yellow oil. ESI-MS *m*/*z* = 382.0 [M+H]⁺.

### Step E

A solution of 5-bromo-3-(3-((tert-butyldimethylsilyl)oxy)-2-methylpropyl)-1H-indole (2.5 g, 6.537 mmol, 1 equiv) in DMF (25 mL) 0 °C was treated with NaH (787 mg, 19.7 mmol, 3.01 equiv, 60% dispersion in mineral oi). After stirring at that temperature for 30 minutes, 2-bromoacetonitrile (1.57 g, 13.1 mmol, 2.00 equiv) was added at 0 °C. The resulting solution was stirred for 30 hours at room temperature. The reaction was quenched with ice water and the mixture was extracted with ethyl acetate (3 x 50 mL). The combined organic layers were concentrated and the crude product was purified by silica gel chromatography (petroleum ether/ethyl acetate (5% to 15%)) to give 968 mg (35% yield) of 2-(5-bromo-3-(3-((tert-butyldimethylsilyl)oxy)-2-methylpropyl)-1H-indol-1-yl)acetonitrile as a yellow oil. ¹H NMR (300 MHz, CDCl₃) δ 7.80 (d, *J* = 1.8 Hz, 1H), 7.40 (dd, *J* = 8.7, 1.9 Hz, 1H), 7.22 (d, *J* = 8.7 Hz, 1H), 6.89 (s, 1H), 4.96 (s, 2H), 4.24-4.08 (m, 1H), 3.56-3.44 (m, 2H), 2.91 (dd, *J =* 14.3, 5.7 Hz, 1H), 2.45 (dd, *J=* 14.3, 8.0 Hz, 1H), 2.07 (s, 1H), 2.03-1.90 (m, 1H), 1.57 (s, 2H), 1.28 (t, *J=* 7.1 Hz, 1H), 0.94 (d, *J=* 11.0 Hz, 14H).

### Step F

A solution of 2-(5-bromo-3-(3-((tert-butyldimethylsilyl)oxy)-2-methylpropyl)-1H-indol-1-yl)acetonitrile (1.0 g, 2.373 mmol, 1 equiv), THF (10 mL), and TBAF (1 M in THF, 4.75 mL) was stirred for 3 hours at room temperature. The solvent was removed *in vacuo* and the crude product was purified by silica gel (petroleum ether/ethyl acetate (30% to 45%)) to give 460 mg (63% yield) of 2-(5-bromo-3-(3-hydroxy-2-methylpropyl)-1H-indol-1-yl)acetonitrile as a yellow oil. ESI-MS *m*/*z* = 307.0 [M+H]⁺.

The following compounds were synthesized according to the procedure described to make Intermediate CB using appropriate building blocks and modified reaction conditions (such as reagents, ratio of reagents, temperature, and reaction time) as needed.

| **Intermediate No.** | **Structure** | **Analytical Data** |
|---|---|---|
| CB-1 | 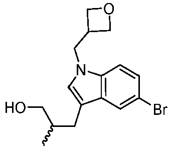 | ESI-MS *m*/*z* = 338.1 [M+H]+ |

### CC: 2-(5-bromo-3-(3-hydroxy-2-methylpropyl)-1H-indol-1-yl)-2-methylpropanenitrile

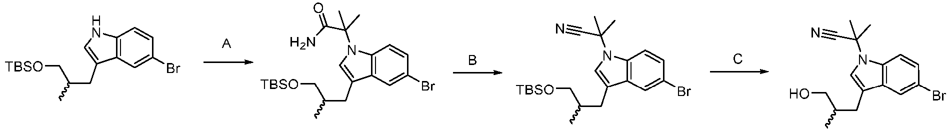

### Step A

A solution of 5-bromo-3-(3-((*tert*-butyldimethylsilyl)oxy)-2-methylpropyl)-1H-indole (2.2 g, 5.75 mmol, 1.0 equiv), 2-bromo-2-methylpropanamide (2.87 g, 17.259 mmol, 3 equiv), PPh₃ (301 mg, 1.151 mmol, 0.2 equiv), K₃PO₄ (2.44 g, 11.506 mmol, 2 equiv), NaOH (230 mg, 5.75 mmol, 1.0 equiv), and CuBr(SMe₂) (237 mg, 1.151 mmol, 0.2 equiv) in toluene (12 mL) was stirred for 15 hours at 55 °C. The resulting mixture was filtered and the filtrate was concentrated under reduced pressure. The crude product was purified by silica gel chromatography (petroleum ether/ethyl acetate (10% to 40%)) to give 2-(5-bromo-3-(3-((*tert*-butyldimethylsilyl)oxy)-2-methylpropyl)-1H-indol-1-yl)-2-methylpropanamide (1.7 g, 63.21% yield) as a light yellow solid. ESI-MS m/z = 489.3 [M+Na]⁺.

### Step B

A solution of 2-(5-bromo-3-(3-((*tert*-butyldimethylsilyl)oxy)-2-methylpropyl)-1H-indol-1-yl)-2-methylpropanamide (1.7 g, 3.64 mmol, 1.0 equiv) in dichloromethane (34 mL) at 0 °C was treated with Et₃N (1.47 g, 14.55 mmol, 4.0 equiv) and then TFAA (1.91 g, 9.090 mmol, 2.5 equiv). The resulting solution was stirred overnight at room temperature and then the solution was diluted with dichloromethane and washed with water. The organics were dried over magnesium sulfate, filtered, and the solvent was removed *in vacuo.* The crude product was purified by silica gel chromatography (petroleum ether/ethyl acetate (4% to 15%)), affording 1.15 g (70% yield) of 2-(5-bromo-3-(3-((tertbutyldimethylsilyl)oxy)-2-methylpropyl)-1H-indol-1-yl)-2-methylpropanenitrile as a light yellow oil. ¹ H NMR (300 MHz, CDCl₃-*d*) δ 7.78 (d, *J =* 1.9 Hz, 1H), 7.59 (d, *J =* 8.8 Hz, 1H), 7.38 (d, *J =* 8.9 Hz, 1H), 6.97 (s, 1H), 3.48 (t, *J* = 5.9 Hz, 2H), 2.89 (dd, *J* = 14.4, 5.9 Hz, 1H), 2.44 (dd, *J* = 14.3, 7.9 Hz, 1H), 2.06 (d, *J* = 1.8 Hz, 6H), 2.00-1.89 (m, 1H), 0.96 (s, 9H), 0.91 (d, *J* = 6.7 Hz, 3H), 0.09 (s, 6H).

### Step C

A solution of 2-(5-bromo-3-(3-((*tert*-butyldimethylsilyl)oxy)-2-methylpropyl)-1H-indol-1-yl)-2-methylpropanenitrile (1.15 g, 2.558 mmol, 1 equiv) in THF (12 mL) at 0 °C was treated with TBAF (1 M in THF, 5.12 mL). The resulting solution was stirred for 4 hours at room temperature. The crude product was purified by silica gel chromatography (petroleum ether/ethyl acetate (10% to 60%)) affording 840 mg (98% yield) of 2-(5-bromo-3-(3-hydroxy-2-methylpropyl)-1H-indol-1-yl)-2-methylpropanenitrile as a yellow oil. ESI-MS *m*/*z* = 335.1 [M+H]⁺.

### CD: (E)-3-(6-bromo-1-((S)-3-hydroxy-2-methylpropyl)-3-methylindolin-3-yl)acrylonitrile

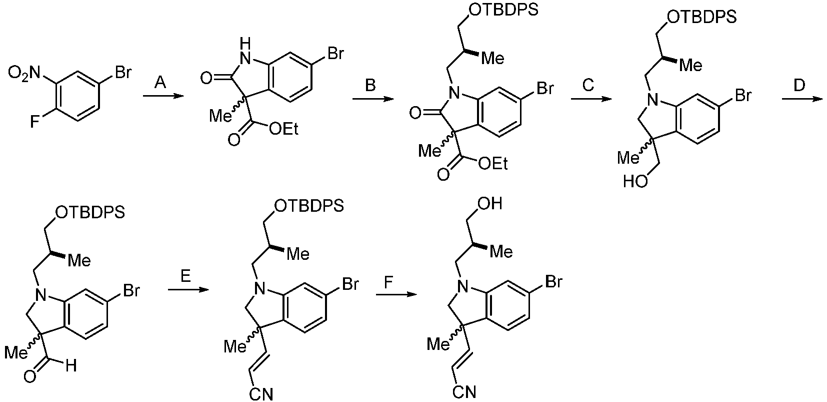

### Step A

To a solution of diethyl propanedioate (4.1 mL, 27.3 mmol, 1.2 equiv) in DMF (32 mL) was added potassium carbonate (4.71 g, 34.09 mmol, 1.5 equiv) and 4-bromo-1-fluoro-2-nitro-benzene (2.8 mL, 22.73 mmol, 1.0 equiv) at room temperature and the reaction mixture was stirred for 20 hours at 70 °C. Then, the reaction was cooled down to 50 °C and additional potassium carbonate (3.14 g, 22.73 mmol, 1 equiv) and iodomethane (4.24 mL, 68.18 mmol, 3 equiv) were added and stirred 1 hour. The reaction was cooled to room temperature and diluted with water and ethyl aceate. Separated organic layers were washed with saturated brine solution. The organics were then separated and dried (magnesium sulfate) before concentration to dryness. The crude mixture was used without further purification. ESI-MS *m*/*z* = 374.0 [M+H]⁺; ¹H NMR (400 MHz, CDCl₃) δ 8.16 (d, *J= 2.2* Hz, 1H), 7.71 (dd, *J =* 8.5, 2.2 Hz, 1H), 7.23 (d, *J* = 8.5 Hz, 1H), 4.29 - 4.12 (m, 4H), 1.98 (s, 3H), 1.23 (t, *J* = 7.1 Hz, 6H).

To a solution of diethyl 2-(4-bromo-2-nitro-phenyl)-2-methyl-propanedioate (8.5 g, 22.7 mmol, 1.0 equiv) in acetic acid (51 mL) was added iron (5.08 g, 90.91 mmol, 4.0 equiv) at room temperature and the reaction mixture was stirred for 1 hour at 95 °C. The crude mixture was cooled to room temperature and filtered through a pad of celite and washed with ethyl acetate, concentrated *in vacuo.* The crude mixture was used without further purification. ESI-MS *m*/*z* = 298.0 [M+H]⁺. ¹H NMR (400 MHz, DMSO-*d*₆) δ 10.80 (s, 1H), 7.16 (s, 2H), 7.02 (s, 1H), 4.09 - 3.99 (m, 2H), 1.48 (s, 3H), 1.04 (t, *J* = 7.0 Hz, 3H).

### Step B

To a solution of ethyl (3R)-6-bromo-3-methyl-2-oxo-indoline-3-carboxylate (1 g, 3.35 mmol, 1.0 equiv) in DMF (22 mL) was added potassium carbonate (1.39 g, 10.06 mmol, 3.0 equiv), [(2R)-3-bromo-2-methyl-propoxy]-tert-butyl-diphenyl-silane (1.84 g, 4.7 mmol, 1.4 equiv), and Nal (50 mg, 0.34 mmol, 0.1 equiv) at room temperature and the reaction mixture was stirred for 2 days at 65 °C. The reaction was cooled down to room temperature and quenched with water and diluted with ethyl acetate. Separated organic layers were washed with brine. The organics were separated and dried (magnesium sulfate) before concentration to dryness. The crude was then purified by flash column chromatography eluting with ethyl acetate and hexanes to give ethyl (3R)-6-bromo-1-[(2S)-3-[tert-butyl(diphenyl)silyl]oxy-2-methylpropyl]-3-methyl-2-oxo-indoline-3-carboxylate (1.2 g, 59% over 3 steps). ESI-MS *m*/*z* = 630.2 [M+Na]⁺. ¹H NMR (400 MHz, CDCl₃) δ 7.72 - 7.62 (m, 8H), 7.48 - 7.32 (m, 12H), 7.23 - 7.16 (m, 2H), 7.17 - 7.11 (m, 2H), 7.12 - 7.06 (m, 2H), 4.19 - 3.97 (m, 4H), 3.87 (dd, *J=* 14.0, 5.3 Hz, 1H), 3.75 (d, *J=* 7.2 Hz, 2H), 3.71 - 3.60 (m, 2H), 3.58 - 3.46 (m, 2H), 2.27 - 2.14 (m, 2H), 1.62 (s, 3H), 1.59 (s, 3H), 1.12 (s, 18H), 1.11 - 1.07 (m, 6H), 0.91 (d, *J =* 6.8 Hz, 3H), 0.87 (d, *J =* 6.8 Hz, 3H).

### Step C

To a solution of ethyl (3R)-6-bromo-1-[(2S)-3-[tert-butyl(diphenyl)silyl]oxy-2-methyl-propyl]-3-methyl-2-oxo-indoline-3-carboxylate (1.05 g, 1.73 mmol, 1.0 equiv) in THF (3.4 mL) was added borane dimethylsulfide (2 M in THF, 12.9 mL, 25.88 mmol, 15 equiv) at room temperature and the reaction mixture was stirred for 3 hours at 60 °C. The reaction mixture was cooled down to room temperature and MeOH was added carefully, dropwise, and stirred until no more gas was observed. The crude mixture was concentrated and purified by silica gel chromatography to give *[(3R)-6-bromo-1-[(2S)-3-[tert-*butyl(diphenyl)silyl]oxy-2-methyl-propyl]-3-methyl-indolin-3-yl]methanol (740 mg, 78% yield). ESI-MS *m*/*z* = 552.3 [M+H]⁺. ¹H NMR (400 MHz, CDCl₃) δ 7.71 - 7.57 (m, 8H), 7.46 - 7.31 (m, 12H), 6.87 - 6.80 (m, 2H), 6.83 - 6.74 (m, 2H), 6.60 (bs, 2H), 3.68 - 3.39 (m, 10H), 3.28 (dd, *J* = 13.5, 7.4 Hz, 1H), 3.12 (dd, *J* = 13.5, 6.6 Hz, 1H), 3.10 - 3.02 (m, 2H), 2.95 (dd, *J =* 13.5, 7.5 Hz, 1H), 2.80 (dd, *J =* 13.5, 6.9 Hz, 1H), 1.26 (s, 6H), 1.08 (s, 18H), 1.00 (d, *J =* 4.3 Hz, 3H), 0.99 (d, *J =* 4.3 Hz, 3H).

### Step D

To a solution of [(3*R*)-6-bromo-1-[(2*S*)-3-[*tert*-butyl(diphenyl)silyl]oxy-2-methyl-propyl]-3-methyl-indolin-3-yl]methanol (710 mg, 1.28 mmol) in dichloromethane (16.0 mL) was added Dess-Martin periodinane (708 mg, 1.67 mmol) at room temperature portionwise and the reaction mixture was stirred for 2 hours. The reaction was quenched with sodium bicarbonate (aqueous) and sodium thiosulfate (aqueous) and diluted with dichloromethane. Separated organic layers were washed with brine. The organics were then separated and dried (magnesium sulfate) before concentration to dryness. The crude was then purified by silica gel column chromatography eluting with ethyl acetate and hexanes to give (3*R*)-6-bromo-1-[(2*S*)-3-[*tert*-butyl(diphenyl)silyl]oxy-2-methyl-propyl]-3-methyl-indoline-3-carbaldehyde (595 mg, 84% yield). ESI-MS *m*/*z* = 550.1 [M+H]⁺.

### Step E

To a solution of 2-diethoxyphosphorylacetonitrile (0.28 mL, 1.73 mmol, 1.6 equiv) in THF (9.1 mL) was added NaH (65 mg, 1.62 mmol, 1.5 equiv, 60% dispersion in mineral oil) at room temperature and the reaction mixture was stirred for 5 minutes. Then (3R)-6-bromo-1-[(2S)-3-[tert-butyl(diphenyl)silyl]oxy-2-methyl-propyl]-3-methyl-indoline-3-carbaldehyde (595 mg, 1.08 mmol, 1 equiv) in THF (9.0812 mL) was added and stirred at room temperature for 30 minutes. The reaction was quenched with ammonium chloride (aq) and diluted with ethyl acetate. Separated organic layers was washed with saturated brine solution. The organics were then separated and dried (magnesium sulfate) before concentration to dryness. The crude was purified by silica gel column chromatography eluting with ethyl acetate and hexanes to give (E)-3-[(3R)-6-bromo-1-[(2S)-3-[*tert*-butyl(diphenyl)silyl]oxy-2-methyl-propyl]-3-methyl-indolin-3-yl]prop-2-enenitrile (430mg, 69.366%). ESI-MS *m*/*z* = 573.2 [M+H]⁺. ¹H NMR (400 MHz, CDCl₃) δ 7.68 - 7.62 (m, 8H), 7.46 - 7.31 (m, 12H), 6.83 - 6.76 (m, 2H), 6.75 - 6.70 (m, 2H), 6.67 (d, *J* = 16.4 Hz, 1H), 6.64 - 6.58 (m, 2H), 5.20 (d, *J =* 16.5 Hz, 1H), 5.15 (d, *J =* 16.5 Hz, 1H), 3.66 - 3.51 (m, 5H), 3.31 - 3.23 (m, 2H), 3.22 (dd, *J =* 13.3, 7.2 Hz, 1H), 3.18 - 3.09 (m, 2H), 2.89 (dd, *J =* 13.6, 7.2 Hz, 1H), 2.80 (dd, *J=* 13.5, 6.7 Hz, 1H), 1.95 (dtq, *J =* 19.7, 13.4, 7.2 Hz, 2H), 1.37 (s, 3H), 1.35 (s, 3H), 1.11 - 1.05 (m, 18H), 0.99 (d, *J =* 3.6 Hz, 3H), 0.97 (d, *J =* 3.5 Hz, 3H).

### Step F

To a solution of (E)-3-[6-bromo-1-[(2S)-3-[tert-butyl(diphenyl)silyl]oxy-2-methyl-propyl]-3-methyl-indolin-3-yl]prop-2-enenitrile (430 mg, 0.75 mmol, 1.0 equiv) in THF (10.7 mL) was added TBAF (1 M in THF, 0.9 mL, 0.9 mmol, 1.2 equiv) at room temperature and the reaction mixture was stirred for 1 hour at room temperature. The reaction was quenched with ammonium chloride (aq) and diluted with ethyl acetate. Separated organic layers was washed with saturated brine solution. The organics were then separated and dried (magnesium sulfate) before concentration to dryness. The crude was purified by silica gel column chromatography eluting with ethyl acetate and hexanes. The desired fractions were concentrated to dryness *in vacuo* to give (*E*)-3-[6-bromo-1-[(2*S*)-3-hydroxy-2-methyl-propyl]-3-methyl-indolin-3-yl]prop-2-enenitrile (250 mg, 99% yield). ESI-MS *m*/*z* = 335.1 [M+H]⁺. ¹H NMR (400 MHz, CDCl₃) δ 6.89 - 6.85 (m, 2H), 6.81 (d, *J =* 16.5 Hz, 1H), 6.80 (d, *J =* 16.5 Hz, 1 H)6.78 (d, *J =* 1.9 Hz, 1H), 6.76 (d, *J* = 1.9 Hz, 1H), 6.75 - 6.69 (m, 2H), 5.28 (d, *J* = 16.5 Hz, 1H), 5.24 (d, *J* = 16.5 Hz, 1H), 3.68 - 3.57 (m, 4H), 3.49 (d, *J =* 9.4 Hz, 1H), 3.40 - 3.31 (m, 2H), 3.22 (d, *J* = 9.4 Hz, 1H), 3.18 (dd, *J* = 13.7, 8.2 Hz, 1H), 3.11 (dd, *J =* 13.6, 7.7 Hz, 1H), 2.96 (dd, *J =* 13.6, 6.6 Hz, 1H), 2.88 (dd, *J =* 13.6, 6.1 Hz, 1H), 2.11 - 1.97 (m, 1H), 1.45 (s, 3H), 1.44 (s, 3H), 0.98 (d, *J =* 6.8 Hz, 6H).

### CE: 6-bromo-1-((S)-3-hydroxy-2-methylpropyl)-3-methylindoline-3-carbonitrile

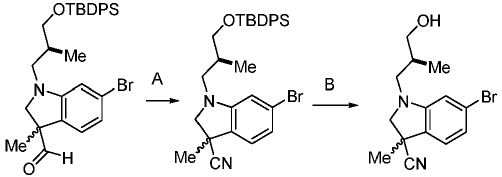

### Step A

To a solution of 6-bromo-1-[(2S)-3-[tert-butyl(diphenyl)silyl]oxy-2-methyl-propyl]-3-methylindoline-3-carbaldehyde (290 mg, 0.53 mmol, 1.0 equiv) in ethanol (5.3 mL) was added NH₂OH•HCl (110 mg, 1.58 mmol, 3.0 equiv) and pyridine (0.21 mL, 2.63 mmol, 5.0 equiv) at room temperature and the reaction mixture was stirred for 1 hour at 50 °C. The reaction was cooled to room temperature and diluted with ethyl acetate and aqueous 1 N HCl was added. Separated organic layers were washed with brine. The organics were then separated and dried (magnesium sulfate) before concentration to dryness. The crude mixture was used for the next step without further purification.

To a solution of (3*E*)-6-bromo-1-[(2S)-3-[*tert*-butyl(diphenyl)silyl]oxy-2-methyl-propyl]-3-methylindoline-3-carbaldehyde oxime (298 mg, 0.53 mmol, 1 equiv) in dichloromethane (3.5 mL) was added di(imidazol-1-yl)methanone (171 mg, 1.05 mmol, 2.0 equiv) at room temperature and the reaction mixture was stirred for 20 hours. The crude mixture was concentrated and directly purified by silica gel chromatography to give 6-bromo-1-[(2S)-3-[*tert*-butyl(diphenyl)silyl]oxy-2-methyl-propyl]-3-methylindoline-3-carbonitrile (255 mg, 88% yield). ESI-MS *m*/*z* = 547.2 [M+H]⁺.

### Step B

To a solution of 6-bromo-1-[(2*S*)-3-[*tert*-butyl(diphenyl)silyl]oxy-2-methyl-propyl]-3-methylindoline-3-carbonitrile (255 mg, 0.47 mmol, 1.0 equiv) in THF (6.5 mL) was added TBAF (1 M in THF, 0.56 mL, 0.56 mmol, 1.2 equiv) at room temperature and the reaction mixture was stirred for 1 hour at room temperature. The reaction was quenched with ammonium chloride (aq) and diluted with ethyl acetate. Separated organic layers was washed with brine. The organics were then separated and dried (magnesium sulfate) before concentration to dryness. The crude was purified by silica gel chromatography eluting with ethyl acetate and hexanes to give 6-bromo-1-[(2S)-3-hydroxy-2-methylpropyl]-3-methyl-indoline-3-carbonitrile (105 mg, 73% yield). ESI-MS *m*/*z* = 309.1 [M+H]⁺.

### CG: tert-butyl ((6³S,4S)-1³-cyano-2⁵-methoxy-10,10-dimethyl-5,7-dioxo-6¹,6²,6³,6⁴,6⁵,6⁶-hexahydro-1¹H-8-oxa-1(6,1)-indola-6(1,3)-pyridazina-2(1,3)-benzenacycloundecaphane-4-yl)carbamate

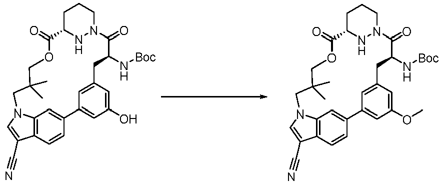

*tert-Butyl* ((6³*S*,4*S*)-1³-cyano-2⁵-hydroxy-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate was synthesized from 6-bromo-1-(3-hydroxy-2,2-dimethylpropyl)-1H-indole-3-carbonitrile using the appropriate intermediates of Example 1 and procedures similar those described in Method A.

To a stirred solution of *tert*-Butyl ((6³*S*,4*S*)-1³-cyano-2⁵-hydroxy-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (300 mg, 0.50 mmol, 1.0 equiv) in MeOH/THF (1:4) (5.0 mL) was added TMS-diazomethane (853 g, 7.5 mmol, 15 equiv) at 0 °C. The mixture was stirred for 24 hours at room temperature. The reaction mixture was concentrated *in vacuo* and diluted with ethyl acetate (50 mL) and water (50 mL). The layers were separated and the organic layer was washed with water (2 × 30 mL), brine (20 mL), dried over anhydrous sodium sulfate, filtered, and the solvent was removed under reduced pressure. The residue was purified by reverse phase chromatography (formic acid in MeCN/Water) to give *tert-butyl* ((6³*S*,4*S*)-1³-cyano-2⁵-methoxy-10,10-dimethyl-5,7-dioxo-6¹,6²,6³,6⁴,6⁵,6⁶-hexahydro-1¹*H-8-*oxa-1(6,1)-indola-6(1,3)-pyridazina-2(1,3)-benzenacycloundecaphane-4-yl)carbamate (180 mg, 60% yield) as a white solid. ESI-MS *m*/*z* = 616.1 [M+H]⁺.

### CH: methyl (S)-1-((S)-3-(3-(1-(3-acetoxypropyl)-3-(tetrahydro-2H-pyran-4-yl)-1H-indol-6-yl)-5-((triisopropylsilyl)oxy)phenyl)-2-((tert-butoxycarbonyl)amino)propanoyl)hexahydropyridazine-3-carboxylate

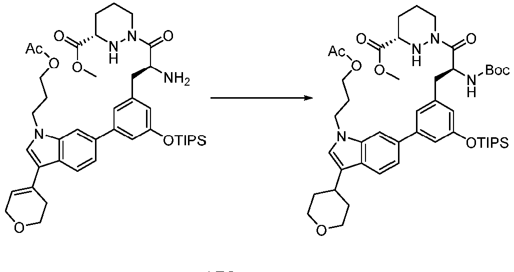

Methyl (*S*)-1-((*S*)-3-(3-(1-(3-acetoxypropyl)-3-(3,6-dihydro-2*H*-pyran-4-yl)-1*H*-indol-6-yl)-5-((triisopropylsilyl)oxy)phenyl)-2-aminopropanoyl)hexahydropyridazine-3-carboxylate was synthesized from 3-(6-bromo-3-(3,6-dihydro-2*H*-pyran-4-yl)-1*H*-indol-1-yl)propyl acetate using conditions similar to those described in Method B.

A solution of methyl (*S*)-1-((*S*)-3-(3-(1-(3-acetoxypropyl)-3-(3,6-dihydro-2H-pyran-4-yl)-1H-indol-6-yl)-5-((triisopropylsilyl)oxy)phenyl)-2-aminopropanoyl)hexahydropyridazine-3-carboxylate (230 mg, 0.227 mmol, 1 equiv) in by MeOH (10 mL) was treated with Pd/C (10% on carbon, 50 mg). The mixture was purged H₂ three times and then stirred under an atmosphere of hydrogen for 15 hours. The solids were filtered off and the solvent was removed *in vacuo* to give methyl (S)-1-((S)-3-(3-(1-(3-acetoxypropyl)-3-(tetrahydro-2H-pyran-4-yl)- 1H-indol-6-yl)-5-((triisopropylsilyl)oxy)phenyl)-2-((tert-butoxycarbonyl)amino)propanoyl)hexahydropyridazine-3-carboxylate (202 mg) that was carried on without further purification. ESI-MS *m*/*z* = 863.6 [M+H]⁺.

### CI: tert-butyl ((6³S,4S,10S)-1³-bromo-10-methyl-5,7-dioxo-2⁵-((triisopropylsilyl)oxy)-6¹,6²,6³,6⁴,6⁵,6⁶-hexahydro-1¹H-8-oxa-1(6,1)-indola-6(1,3)-pyridazina-2(1,3)-benzenacycloundecaphane-4-yl)carbamate

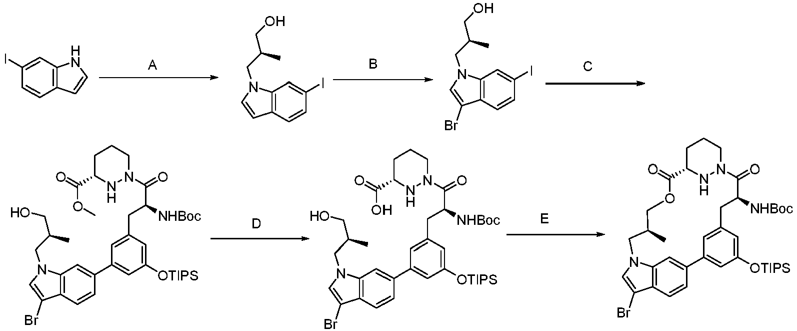

### Step A

A solution of 6-iodo-1*H*-indole (7.00 g, 28.80 mmol, 1.0 equiv), [(2R)-3-bromo-2-methylpropoxy](tert-butyl)diphenylsilane (12.40 g, 31.68 mmol, 1.1 equiv), and Cs₂CO₃ (23.46 g, 72.00 mmol, 2.5 equiv) in DMF was stirred for 14 hours at 80 °C. After concentration of the reaction mixture, the residue was purified by silica gel chromatography, eluting with petroleum ether/ethyl acetate (8:1) to afford (*S*)-3-(6-iodo-1*H*-indol-1-yl)-2-methylpropan-1-ol (4.0 g, 44% yield) as a colorless oil. ESI-MS *m*/*z* = 316.0 [M+H]⁺.

### Step B

To a solution of (*S*)-3-(6-iodo-1*H*-indol-1-yl)-2-methylpropan-1-ol (4.00 g, 12.69 mmol, 1.0 equiv) in DMF (40.0 ml) was added NBS (2.48 g, 13.96 mmol, 1.1 equiv) in DMF (10 mL) dropwise at 0 °C. The solution was maintained at that temperature for 1 hour. Water was added and the solution was extracted with ethyl acetate (3 x 100 mL). After concentration, the residue was purified by silica gel chromatography, eluting with petroleum ether/ethyl acetate (5:1) to afford (*S*)-3-(3-bromo-6-iodo-1*H*-indol-1-yl)-2-methylpropan-1-ol (3.2 g, 60% yield) as a yellow oil. ESI-MS *m*/*z* = 392.0 [M-H]⁻.

### Step C

To a stirred solution of (*S*)-3-(3-bromo-6-iodo-1H-indol-1-yl)-2-methylpropan-1-ol (3.20 g, 4.64 mmol, 1.0 equiv) and (2S)-3-(3-bromo-6-iodo-1H-indol-1-yl)-2-methylpropan-1-ol (1.83 g, 4.64 mmol, 1.0 equiv) in dioxane (25 mL) and H₂O (5.0 mL) was added K₂CO₃ (1.60 g, 11.60 mmol, 2.5 equiv) and Pd(DTBPF)Cl₂ (0.30 g, 0.46 mmol, 0.1 equiv) portionwise. The solution was stirred for 3 hours at 50 °C and then concentrated. The residue was purified by silica gel column chromatography, eluting with petroleum ether/ethyl acetate (4:1) to afford methyl (S)-1-((S)-3-(3-(3-bromo-1-((S)-3-hydroxy-2-methylpropyl)-1*H*-indol-6-yl)-5-((triisopropylsilyl)oxy)phenyl)-2-((*tert-*butoxycarbonyl)amino)propanoyl)hexahydropyridazine-3-carboxylate (2.9 g, 75% yield) as an oil. ESI-MS *m*/*z* = 829.4 [M+H]⁺.

### Step D

To a stirred solution of methyl (*S*)-1-((*S*)-3-(3-(3-bromo-1-((*S*)-3-hydroxy-2-methylpropyl)-1H-indol-6-yl)-5-((triisopropylsilyl)oxy)phenyl)-2-((tert-butoxycarbonyl)amino)propanoyl)hexahydropyridazine-3-carboxylate (2.90 g, 3.49 mmol, 1.0 equiv) in DCE (30 mL) was added trimethylstannanol (3.16 g, 17.47 mmol, 5.0 equiv) dropwise. After stirring for 14 hours at 60 °C, the solution was concentrated and the residue was purified by silica gel chromatography, eluting with petroleum ether/ethyl acetate (2:1) to afford (*S*)-1-((*S*)-3-(3-(3-bromo-1-((*S*)-3-hydroxy-2-methylpropyl)-1*H*-indol-6-yl)-5-((triisopropylsilyl)oxy)phenyl)-2-((tert-butoxycarbonyl)amino)propanoyl)hexahydropyridazine-3-carboxylic acid (2.7 g, 95% yield) as an oil. ESI-MS *m*/*z* = 815.3 [M+H]⁺.

### Step E

To a stirred solution of (*S*)-1-((*S*)-3-(3-(3-bromo-1-((*S*)-3-hydroxy-2-methylpropyl)-1*H*-indol-6-yl)-5-((triisopropylsilyl)oxy)phenyl)-2-((*tert*-butoxycarbonyl)amino)propanoyl)hexahydropyridazine-3-carboxylic acid (4.00 g, 4.90 mmol, 1.0 equiv) and DIEA (15.84 g, 122.56 mmol, 25.0 equiv) in dichloromethane (40 mL) was added HOBT (3.97 g, 29.42 mmol, 6.0 equiv) and EDCI (15.04 g, 78.439 mmol, 16 equiv) in portions at 0 °C. After stirring for 16 hours at room temperature, the solution was concentrated and the residue was purified by silica gel chromatography, eluting with petroleum ether/ethyl acetate (5:1) to afford tert-butyl ((6³*S*,4*S*,10*S*)-1³-bromo-10-methyl-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (2.3 g, 59% yield) as a brown solid. ESI-MS *m*/*z* = 797.3 [M+H]⁺

### CJ: tert-butyl ((6³S,4S,10S)-1³-(3-hydroxy-2-methylbutan-2-yl)-10-methyl-5,7-dioxo-2⁵-((triisopropylsilyl)oxy)-6¹,6²,6³,6⁴,6⁵,6⁶-hexahydro-1¹H-B-oxa-1(6,1)-indola-6(1,3)-pyridazina-2(1,3)-benzenacycloundecaphane-4-yl)carbamate

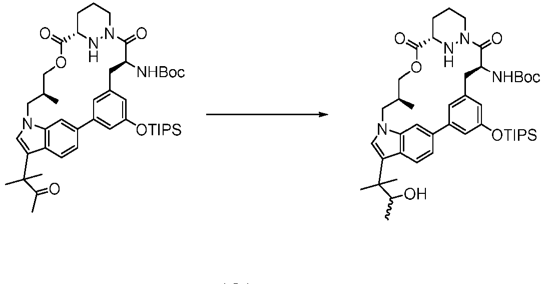

*tert-Butyl* ((6³*S*,4*S*,10*S*)-10-methyl-1³-(2-methyl-3-oxobutan-2-yl)-5,7-dioxo-2⁵. ((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate was synthesized from (S)-3-(6-bromo-1-(3-hydroxy-2-methylpropyl)-1^{H}-indol-3-yl)-3-methylbutan-2-one using the appropriate intermediates of Example 1 and procedures similar those described in Method A.

To a stirred solution of tert-butyl ((6³*S*,4*S*,10*S*)-10-methyl-1³-(2-methyl-3-oxobutan-2-yl)-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (400 mg, 0.498 mmol, 1.0 equiv) in MeOH (10 mL) at 0 °C was added NaBH₄ (75 mg, 1.99 mmol, 4.0 equiv) in portions. The mixture was stirred for 4 hours at room temperature and then water (200 mL) was added. The resulting mixture was extracted with EA (2 x 200 mL). The combined organic layers were washed with water (2 x 100 mL) and dried over anhydrous sodium sulfate. After filtration, the filtrate was concentrated under reduced pressure. The crude product tert-butyl ((6³*S*,4*S*,10*S*)-1³-(3-hydroxy-2-methylbutan-2-yl)-10-methyl-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (350 mg, 70% yield) was used in the next step directly without further purification. ESI-MS *m*/*z* = 805.4 [M+H]⁺.

### CK: tert-butyl-((6³S,4S,10S)-1³-((E)-1-(methoxyimino)ethyl)-10-methyl-5,7-dioxo-2⁵-((triisopropylsilyl)oxy)-6¹,6²,6³,6⁴,6⁵,6⁶-hexahydro-1¹H-B-oxa-1(6,1)-indola-6(1,3)-pyridazina-2(1,3)-benzenacycloundecaphane-4-yl)carbamate

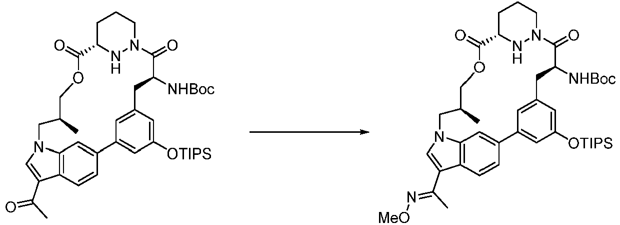

tert-Butyl ((6³*S*,4*S*,10*S*)-1³-acetyl-10-methyl-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate was synthesized from (*S*)-1-(6-bromo-1-(3-hydroxy-2-methylpropyl)-1H-indol-3-yl)ethan-1-one using the appropriate intermediates of Example 1 and procedures similar those described in Method A.

*tert*-Butyl ((6³*S*,4*S*,10*S*)-1³-acetyl-10-methyl-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (350 mg, 0.46 mmol, 1.0 equiv) was dissolved in MeOH (3.0 mL) and O-methylhydroxylamine hydrochloride (385 mg, 4.6 mmol, 10 equiv) was added. The mixture was stirred at 15°C for 10 minutes and then NaHCO₃ (386 mg, 4.6 mmol, 10 equiv) was added in portions. The reaction mixture was stirred for 8 hours. After filtration and concentration, the crude product was purified by silica gel chromatography (Petroleum/ethyl acetate = 1:1) to give *tert-butyl* ((6³*S*,4*S*,10*S*)-1³-((*E*)-1-(methoxyimino)ethyl)-10-methyl-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (330 mg, 90% yield) as a white solid. ESI-MS *m*/*z* = 790.4[M+Na]⁺.

### CL: tert-butyl ((6³S,4S)-2⁵-hydroxy-10,10-dimethyl-1³-(1-methylpiperidin-4-yl)-5,7-dioxo-6¹,6²,6³,6^{4,}6⁵,6⁶-hexahydro-1¹H-8-oxa-1(6,1)-indazola-6(1,3)-pyridazina-2(1,3)-benzenacycloundecaphane-4-yl)carbamate

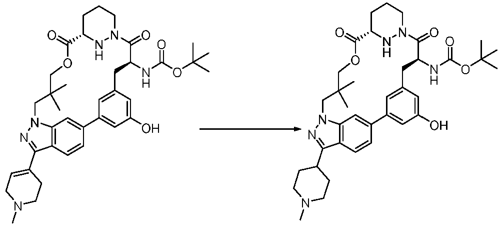

*tert*-Butyl ((6³*S*,4*S*)-2⁵-hydroxy-10,10-dimethyl-1³-(1-methyl-1,2,3,6-tetrahydropyridin-4-yl)-5,7-dioxo-6¹,6²,6³,6⁴,6-¹,6⁶-hexahydro-1¹*H*-8-oxa-1(6,1)-indazola-6(1,3)-pyridazina-2(1,3)-benzenacycloundecaphane-4-yl)carbamate was synthesized from 3-(6-bromo-3-(1-methyl-1,2,3,6-tetrahydropyridin-4-yl)-1H-indazol-1-yl)-2,2-dimethylpropan-1-ol using procedures similar to the ones described for the synthesis of (2*S*)-2-(3-acryloyl-2-oxoimidazolidin-1-yl)-*N*-((6³*S*,4*S*)-2⁵-hydroxy-1²,10,10-trimethyl-5,7-dioxo-6¹ ,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)-3-methylbutanamide.

A solution of *tert*-butyl ((6³*S*,4*S*)-2⁵-hydroxy-10,10-dimethyl-1³-(1-methyl-1,2,3,6-tetrahydropyridin-4-yl)-5,7-dioxo-6¹,6²,6³,6⁴,6⁵,6⁶-hexahydro-1¹*H*-8-oxa-1(6,1)-indazola-6(1,3)-pyridazina-2(1,3)-benzenacycloundecaphane-4-yl)carbamate (78 mg, 0.12 mmol, 1 equiv) and Pd/C (50 mg, 0.47 mmol, 4.05 equiv) in MeOH (1 mL) was purged with H₂ three times. The resulting solution was stirred for 2 hours under an atmosphere of H₂. The solids were filtered off and the resulting mixture was concentrated to give tert-butyl ((6³*S*,4*S*)-2⁵-hydroxy-10,10-dimethyl-1³-(1-methylpiperidin-4-yl)-5,7-dioxo-6¹,6²,6³,6⁴,6⁵,6⁶-hexahydro-1¹*H*-8-oxa-1(6,1)-indazola-6(1,3)-pyridazina-2(1,3)-benzenacycloundecaphane-4-yl)carbamate (55 mg) as a dark yellow oil that was carried on without further purification. ESI-MS *m*/*z* = 675.5 [M+H]⁺.

### CM: tert-butyl ((6³S,4S)-1³-ethynyl-2⁵-hydroxy-10,10-dimethyl-5,7-dioxo-6¹,6²,6³,6⁴,6⁵,6⁶-hexahydro-1¹H-B-oxa-1(6,1)-indazola-6(1,3)-pyridazina-2(1,3)-benzenacycloundecaphane-4-yl)carbamate

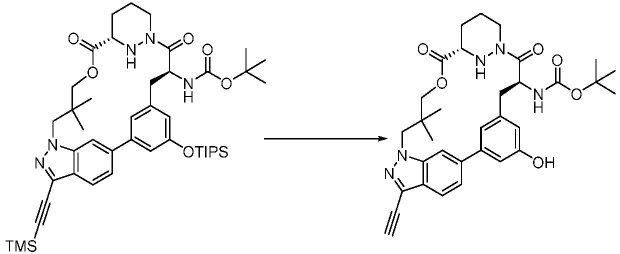

*tert*-Butyl ((6³*S*,4*S*)-10,10-dimethyl-5,7-dioxo-2⁵-((triisopropylsilyl)oxy)-1³-((trimethylsilyl)ethynyl)-6¹,6²,6³,6⁴ ,6⁵,6⁶-hexahydro-1¹*H*-8-oxa-1(6,1)-indazola-6(1,3)-pyridazina-2(1,3)-benzenacycloundecaphane-4-yl)carbamate was synthesized from 3-(6-bromo-3-((trimethylsilyl)ethynyl)-1H-indazol-1-yl)-2,2-dimethylpropan-1-ol using a protocols similar to the ones described for the synthesis of (2S)-2-(3-acryloyl-2-oxoimidazolidin-1-yl)-N-((6³*S*,4*S*)-2⁵-hydroxy-1²,10,10-trimethyl-5,7-dioxo-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)-3-methylbutanamide.

A solution of *tert-butyl* ((6³*S*,4*S*)-1 0,1 0-dimethyl-5, 7 -dioxo-2⁵-( (triisopropylsilyl)oxy)-1³-((trimethylsilyl)ethynyl)-6¹,6²,6³,6⁴,6⁵,6⁶-hexahydro-1¹*H*-8-oxa-1(6,1)-indazola-6(1,3)-pyridazina-2(1,3)-benzenacycloundecaphane-4-yl)carbamate (220 mg, 0.26 mmol, 1.0 equiv) in THF (3 mL) at 0 °C was treated with TBAF (1 M, 0.132 mL, 0.13 mmol, 0.5 equiv). The resulting solution was stirred for 2 hours at 0 °C. The resulting mixture was concentrated. The residue was purified by silica gel chromatography with ethyl acetate/petroleum ether (3/1) to give *tert-butyl* ((6³*S*,4*S*)-1³-ethynyl-2⁵-hydroxy-10,10-dimethyl-5,7-dioxo-6¹,6²,6³,6⁴,6⁵,6⁶-hexahydro-1¹*H*-8-oxa-1(6,1)-indazola-6(1,3)-pyridazina-2(1,3)-benzenacycloundecaphane-4-yl)carbamate (150 mg, 85% yield) of as a light yellow solid. ESI-MS *m*/*z* = 602.3 [M+H]⁺.

### CN: tert-butyl ((6³S,4S,10S)-1³-(2-cyanoethyl)-2⁵-hydroxy-13,10-dimethyl-5,7-dioxo-6¹,6²,6³,6⁴,6⁵,6⁶-hexahydro-8-oxa-1(6,1)-indolina-6(1,3)-pyridazina-2(1,3)-benzenacycloundecaphane-4-yl)carbamate

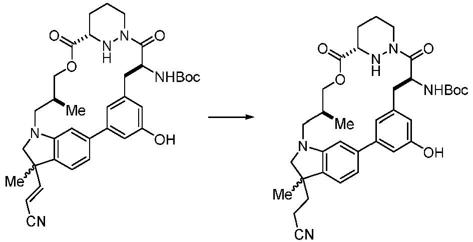

*tert*-Butyl ((6³*S*,4*S*,10*S*)-1³-((E)-2-cyanovinyl)-2⁵-hydroxy-13,10-dimethyl-5,7-dioxo-6¹,6²,6³,6⁴,6⁵,6⁶-hexahydro-8-oxa-1(6,1)-indolina-6(1,3)-pyridazina-2(1,3)-benzenacycloundecaphane-4-yl)carbamate was synthesized from (E)-3-(6-bromo-1-((S)-3-hydroxy-2-methylpropyl)-3-methylindolin-3-yl)acrylonitrile using the appropriate intermediates of Example 1 and procedures similar those described in Method A.

To a solution of *tert-butyl* ((6³*S*,4*S*,10*S*)-1³-((E)-2-cyanovinyl)-2⁵-hydroxy-13,10-dimethyl-5,7-dioxo-6¹,6²,6³,6⁴,6⁵,6⁶-hexahydro-8-oxa-1(6,1)-indolina-6(1,3)-pyridazina-2(1,3)-benzenacycloundecaphane-4-yl)carbamate was synthesized from (*E*)-3-(6-bromo-1-((*S*)-3-hydroxy-2-methylpropyl)-3-methylindolin-3-yl)acrylonitrile (73 mg, 0.12 mmol, 1.0 equiv) in THF (2.3 mL) was added Pd/C (10 wt%, 37 mg, 0.03 mmol, 0.3 equiv) and stirred for 20 hours under 1 atm of H₂. The crude mixture was filtered through a pad of celite and concentrated *in vacuo.* The residue was purified by silica gel chromatography with ethyl acetate and hexanes to give *tert-butyl* ((6³*S*,4*S*,10*S*)-1³-(2-cyanoethyl)-2⁵. hydroxy-13,10-dimethyl-5,7-dioxo-6¹,6²,6³,6⁴,6⁵,6⁶-hexahydro-8-oxa-1(6,1)-indolina-6(1,3)-pyridazina-2(1,3)-benzenacycloundecaphane-4-yl)carbamate (51 mg, 70% yield). ESI-MS *m*/*z* = 654.2 [M+Na]⁺.

### CO: (S)-2-(3-acryloyl-2-oxoimidazolidin-1-yl)-3-methylbutanoic acid

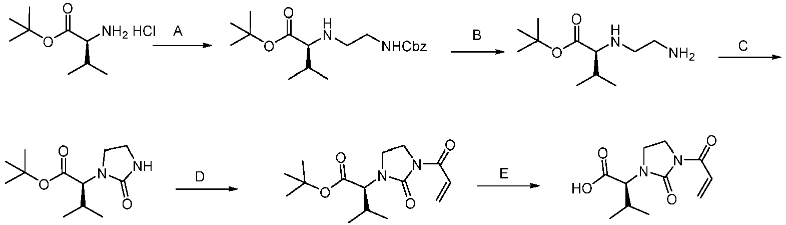

### Step A

To a solution of benzyl N-(2-oxoethyl)carbamate (11.88 g, 1.2 equiv) in methanol (120 mL) was added tert-butyl (2S)-2-amino-3-methylbutanoate hydrochloride (10.70 g, 1 equiv). After cooling to 0 °C sodium cyanoborohydride (9.6 g, 3.0 equiv) was added portionwise while maintaining the reaction temperature between 0 °C and 10 °C. To the resulting mixture was added acetic anhydride (3.1 g, 1.0 equiv). The resulting solution was stirred for 4 hours at 25 °C. The reaction was then quenched by the addition of 200 mL of ice water. The resulting solution was extracted with dichloromethane (3 x 200 mL). The organics were washed with brine (300 mL), dried over anhydrous sodium sulfate and concentrated under vacuum. The crude product was purified by C18 reverse phase chromatography (85-95% acetonitrile in water with 0.1% formic acid) to give desired product (5 g). ESI-MS *m*/*z* = 351.2 [M+H]⁺.

### Step B

To a solution of tert-butyl (2-(((benzyloxy)carbonyl)amino)ethyl)-L-valinate (2.4 g, 6.9 mmol) in methanol (10 mL) was added 10% palladium on carbon (1.2 g) under nitrogen. The suspension was degassed under vacuum and purged with hydrogen three times. The mixture was stirred under hydrogen for 14 hours. The reaction mixture was filtered and concentrated to give the desired product (74% yield) as a white solid, which was used for next step reaction without purification. Calculated MW: 216.2; ESI-MS *m*/*z* = 217 [M+H]⁺.

### Step C

tert-Butyl (2-aminoethyl)-L-valinate (1.1 g, 5.09 mmol) was dissolved in DMF (10 mL) and treated with bis(4-nitrophenyl) carbonate (1.9 g, 1.2 equiv). The resulting solution was stirred for 14 hours at 60 °C. The reaction mixture was cooled to room temperature and 50 mL of ice water was added. The resulting solution was extracted with ethyl acetate (2 x 50 mL) and the organics were washed with water (2 x 100 ml), dried, and concentrated. The residue was purified by silica gel column chromatography (0-10% methanol in dichloromethane) to give the desired product (49% yield) as a yellow solid. ESI-MS m/z = 243.1 [M+H]⁺.

### Step D

To a mixture of tert-butyl (S)-2-(3-acryloyl-2-oxoimidazolidin-1-yl)-3-methylbutanoate (0.05 g, 206 umol) in dichloromethane (1 mL) at 0 °C was added triethylamine (57 uL, 410 umol) followed by acryloyl chloride (20 uL, 250 umol). The mixture was stirred for 1 hour and then diluted with dichloromethane (2 mL), washed with water (2 mL) and brine (4 mL), dried over anhydrous sodium sulfate, filtered and concentrated under reduced pressure at 15 °C to give the crude product as a yellow oil. The crude product was purified by silica gel chromatography (15-25% ethyl acetate in petroleum ether) to give the desired product (83% yield) as a white solid. ¹H NMR (400MHz, CDCl₃) δ 7.61 (dd, *J=10.5,* 17.1 Hz, 1H), 6.49 (dd, *J*=2.0, 17.0 Hz, 1H), 5.79 (dd, J=2.0, 10.4 Hz, 1H), 4.21 (d, J=9.7 Hz, 1H), 3.98 - 3.76 (m, 3H), 3.47 (dt, *J*=6.5, 9.1 Hz, 1H), 2.25 - 2.13 (m, 1H), 1.53 - 1.45 (m, 9H), 1.04 (d, J=6.6 Hz, 3H), 0.96 (d, *J*=6.6 Hz, 3H).

### Step E

To a solution of tert-butyl (S)-3-methyl-2-(2-oxoimidazolidin-1-yl)butanoate (0.05 g, 168 umol) in dichloromethane (0.5 mL) was added trifluoroacetic acid (0.5 mL) at 0 °C. After stirring at room temperature for 2 hours, the reaction mixture was concentrated *in vacuo* to give the desired product (0.041 g) as a yellow solid, which was used without further purification.

### CP: (S)-3-methyl-2-(2-oxo-3-(vinylsulfonyl)imidazolidin-1-yl)butanoic acid

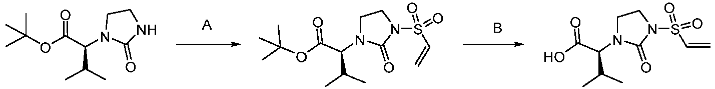

### Step A

To a solution of *tert-*butyl (S)-3-methyl-2-(2-oxoimidazolidin-1-yl)butanoate (80 mg, 0.33 mmol, 1.0 equiv) in dichloromethane (4 mL) was added pyridine (1 mL) and ethenesulfonyl chloride (54 mg, 0.43 mmol, 1.3 equiv) at 0 °C. The reaction solution was stirred at 0 °C for 2 hours and then concentrated under vacuum to give the crude product that was used without purification. ESI-MS *m*/*z* = 333.1 [M+H]⁺.

### Step B

*tert*-Butyl (*S*)-3-methyl-2-(2-oxo-3-(vinylsulfonyl)imidazolidin-1-yl)butanoate (60 mg, 0.18mmol, 1.0 equiv) was treated with a mixture of trifluoroacetic acid (2 mL) and dichloromethane (4 mL) at 0 °C. The reaction solution was stirred at 0 °C for 2 hours. The residue was concentrated under vacuum to give the crude product that was without further purification.

### CQ: (S)-3-methyl-2-(2-oxo-4-(vinylsulfonyl)piperazin-1-yl)butanoic acid

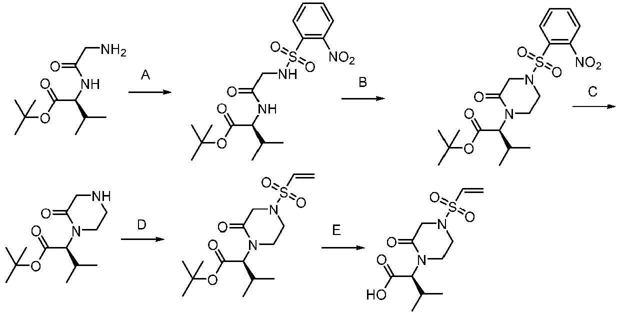

### Step A

*tert*-butyl glycyl-L-valinate (2.3 g, 10.0 mmol, 1.0 equiv) and triethylamine (3.03 g, 30.0 mmol, 3.0 equiv) in dichloromethane (30ml) at 0 °C was added 2-nitrobenzene-1-sulfonyl chloride (2.43 g, 11.0 mmol, 1.1 equiv). The resulting solution was stirred for 2 hours at room temperature. After cooling to 0 °C, the resulting mixture was quenched with ice water (30 mL). The mixture was extracted with dichloromethane (2 x 60 mL). The organic layers were combined and washed with water (2 x 40 mL), dried over anhydrous sodium sulfate, filtered, and the filtrate was concentrated. Purification by silica gel chromatography (20% ethyl acetate in petroleum ether) afforded the desired product (84% yield) as a colorless oil. ESI-MS *m*/*z* = 416.1 [M+H]⁺. ¹H NMR (300 MHz, CDCl₃) δ 8.16-8.13 (m, 1H), 7.96-7.93 (m, 1H), 7.81-7.76 (m, 2H), 6.67 (d, J=6.0 Hz, 1H), 6.26-6.22 (m, 1H), 4.43-4.38 (m, 1H), 3.86 (d, J=6.0 Hz, 2H), 2.19-2.12 (m, 1H), 1.48 (s, 9H), 0.92-0.88 (m, 6H).

### Step B

To a solution of tert-butyl ((2-nitrophenyl)sulfonyl)glycyl-L-valinate (3.8 g, 9.15 mmol, 1.0 equiv) in DMF (50 mL) at 0 °C was added 1,2-dibromoethane (17.0 g, 91.5 mmol, 10.0 equiv) and potassium carbonate (12.6 g, 91.5 mmol, 10.0 equiv). The mixture was stirred at 50 °C for 18 hours and then poured into ice water (60 mL) and extracted with ethyl acetate (2 × 120 mL). The organic layers were combined and washed with water (50 ml x 2), dried, filtered, concentrated, and purified by silica gel chromatography (30% ethyl acetate in petroleum ether) to give the desired product (60% yield) as a yellow oil. ESI-MS *m*/*z* = 442.1 [M+H]⁺.

### Step C

A solution of tert-butyl (S)-3-methyl-2-(4-((2-nitrophenyl)sulfonyl)-2-oxopiperazin-1-yl)butanoate (2.3 g, 5.21 mmol, 1.0 equiv), potassium carbonate (3.6 g, 10.42 mmol, 2.0 equiv), and thiophenol (1.15 g, 10.42 mmol, 5.0 equiv) in DMF (30 mL) was stirred for 4 hours. After filtering off the solids and concentration of the filtrate, purification by silica gel chromatography provided the desired product (90% yield) as a clear oil. ¹H NMR (300 MHz, CDCl₃) δ 4.9(d, *J* = 9.0 Hz, 1H), 3.63(s, 2H), 3.54-3.48(m, 1H), 3.31-3.25(m, 1H), 3.11-3.05(m, 2H), 2.51-2.35(m, 1H), 1.48(s, 9H), 1.15-0.85(m, 6H).

### Step D

To a solution of tert-butyl (S)-3-methyl-2-(2-oxopiperazin-1-yl)butanoate (300 mg, 1.17 mmol, 1.0 equiv) in dichloromethane (10 mL) was added diisopropylethylamine (453 mg, 3.1 mmol, 3.0 equiv) and ethenesulfonyl chloride (221 mg, 1.75 mmol, 1.5 equiv) at 0 °C. The reaction solution was stirred at 0 °C for 2 hours and then the mixture was concentrated *in vacuo.* The residue was purified by reverse phase HPLC (5-95% acetonitrile in water with 0.05% formic acid) to give the desired product (40% yield) as a light yellow solid. ESI-MS *m*/*z* = 347.4 [M+H]⁺. ¹H NMR (300 MHz, CD₃OD) δ 6.79-6.69 (m, 1H), 6.32-6.18 (m, 2H), 4.62 (d, *J* = 12.0 Hz, 1H), 3.90 (d, *J* = 3.0 Hz, 2H), 3.68-3.40 (m, 4H), 2.31-2.23 (m, 1H), 1.49 (s, 9H), 1.07-0.91 (m, 6H).

### Step E

A solution of tert-butyl (*S*)-3-methyl-2-(2-oxo-4-(vinylsulfonyl)piperazin-1-yl)butanoate (50 mg, 0.16 mmol, 1.0 equiv) and trifluoroacetic acid (1 mL) in dichloromethane (3 mL) at 0 °C was stirred for 2 hours. The solution was concentrated under vacuum to give the title compound and was used without further purification.

The following intermediates were synthesized according to the procedure described to make Intermediate CQ using appropriate building blocks and modified reaction conditions (such as reagents, ratio of reagents, temperature, and reaction time) as needed.

| **Intermediate No.** | **Structure** | **Analytical Data** |
|---|---|---|
| CQ-1 | 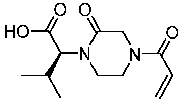 | ESI-MS *m*/*z* = 255.2 [M+H] |
| CQ-2 | 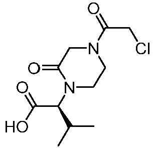 | ESI-MS *m*/*z* = 277.2 [M+H]+ |

### CR: Synthesis of tert-butyl (S)-2-((R)-3-amino-2-oxopyrrolidin-1-yl)-3-methylbutanoate

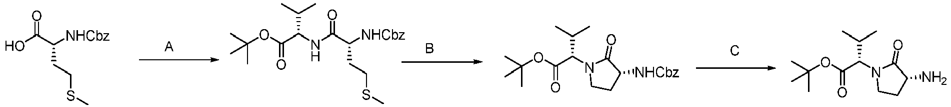

### Step A

To a stirred solution of (2*R*)-2-[[(benzyloxy)carbonyl]amino]-4-(methylsulfanyl)butanoic acid (15 g, 52.940 mmol, 1 equiv) in DMF (200 mL) at 0 °C was added tert-butyl (2S)-2-amino-3-methylbutanoate hydrochloride (12.21 g, 58.2 mmol, 1.10 equiv), DIEA (17.11 g, 132.3 mmol, 2.5 equiv) and HATU (24.16 g, 63.5 mmol, 1.2 equiv). The resulting mixture was stirred for 2 hours at 0 °C and then diluted with water (1 L). The resulting mixture was extracted with ethyl acetate (2 x 500 mL). The combined organic layers were washed with water (2 x 500 mL) and dried over anhydrous sodium sulfate. After filtration, the filtrate was concentrated under reduced pressure to give a residue that was purified by silica gel column chromatography, eluting with EA/PE (1:5-1:4) to afford tert-butyl ((benzyloxy)carbonyl)-D-methionyl-L-valinate (20 g, 78% yield) as a colorless oil. ESI-MS *m*/*z* = 439.3 [M+H]⁺.

### Step B

To a stirred solution of tert-butyl ((benzyloxy)carbonyl)-D-methionyl-L-valinate (20 g, 45.602 mmol, 1 equiv) in acetone (200 mL) was added iodomethane (30 mL). The resulting mixture was stirred for 48 hours at room temperature and then concentrated under reduced pressure. To a mixture of the crude product in acetonitrile (200 mL) was added Cs₂CO₃ (44.57 g, 136.8 mmol, 3.0 equiv). After stirring for 4 hours at 60 °C, water (1 L) was added. The resulting mixture was extracted with ethyl acetate (2 x 500 mL). The combined organic layers were washed with water (2 x 500 mL) and dried over anhydrous sodium sulfate. After filtration, the filtrate was concentrated under reduced pressure. The crude product was purified by reverse phase chromatography (70-75% acetonitrile in water with 0.1% FA) to afford *tert-*butyl (S)-2-((R)-3-(((benzyloxy)carbonyl)amino)-2-oxopyrrolidin-1-yl)-3-methylbutanoate (11 g, 55.60% yield) as a yellow oil. ESI-MS *m*/*z* = 391.3 [M+H]⁺.

### Step C

To a stirred solution of *tert*-butyl (2*S*)-2-[(3*R*)-3-[[(benzyloxy)carbonyl]amino]-2-oxopyrrolidin-1-yl]-3-methylbutanoate (1.5 g, 3.841 mmol, 1 equiv) in ethyl acetate (15 mL) was added Pd/C (10%, 500 mg, 4.7 mmol, 1.22 equiv) in portions. The solution was purged with H₂ and stirred under a hydrogen atmosphere for 16 hours at 40 °C. The resulting mixture was filtered and the filter cake was washed with ethyl acetate (3 x 10 mL). The filtrate was concentrated under reduced pressure to give tert-butyl (2S)-2-[(3R)-3-amino-2-oxopyrrolidin-1-yl]-3-methylbutanoate (800 mg, 73% yield) as a light-yellow oil. ESI-MS *m*/*z* = 257.2 [M+H]⁺.

### CS: tert-butyl (S)-3-methyl-2-((R)-3-(methylamino)-2-oxopyrrolidin-1-yl)butanoate

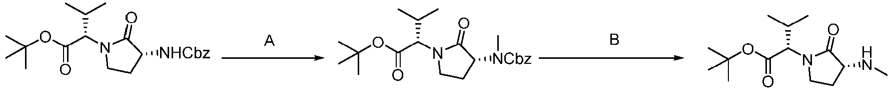

### Step A

To a stirred solution of tert-butyl (S)-2-((R)-3-(((benzyloxy)carbonyl)amino)-2-oxopyrrolidin-1-yl)-3-methylbutanoate (1.5 g, 3.841 mmol, 1 equiv) in THF (15 mL) was added NaH (232 mg, 5.8 mmol, 1.5 equiv, 60%) in several batches at 0 °C. The resulting mixture was stirred for 1 hour at 0°C and then iodomethane (821 mg, 5.784 mmol, 1.51 equiv) was added dropwise. The resulting mixture was stirred for additional 1.5 hours at 0 °C and then aqueous saturated NH₄Cl (10 mL) was added and concentrated under vacuum. The resulting mixture was diluted with water (100 mL) and extracted with ethyl acetate (3 x 200 mL). The combined organic layers were washed with water (2 x 100 mL) and brine (100 mL) and dried over anhydrous sodium sulfate. After filtration, the filtrate was concentrated under reduced pressure to give tert-butyl (S)-2-((R)-3-(((benzyloxy)carbonyl)(methyl)amino)-2-oxopyrrolidin-1-yl)-3-methylbutanoate (1.6 g, 87% yield) as a light-yellow oil. ESI-MS *m*/*z* = 405.3 [M+H]⁺.

### Step B

To a stirred solution of give tert-butyl (S)-2-((R)-3-(((benzyloxy)carbonyl)(methyl)amino)-2-oxopyrrolidin-1-yl)-3-methylbutanoate (800 mg, 1.978 mmol, 1 equiv) in ethyl acetate (15 mL) was added Pd/C (10 mol%, 320 mg, 3.007 mmol, 1.52 equiv) in portions. The solution was purged with hydrogen gas and stirred for 24 hours at 40°C under an atmosphere of hydrogen. The suspension was filtered and the filter cake was washed with ethyl acetate (3 x 10 mL). The filtrate was concentrated under reduced pressure to give tert-butyl (*S*)-3-methyl-2-((*R*)-3-(methylamino)-2-oxopyrrolidin-1-yl)butanoate (460 mg, 73% yield) as a light-yellow oil. ESI-MS *m*/*z* = 271.2 [M+H]⁺.

### CT: tert-butyl N-(azetidine-3-carbonyl)-N-ethyl-L-valinate

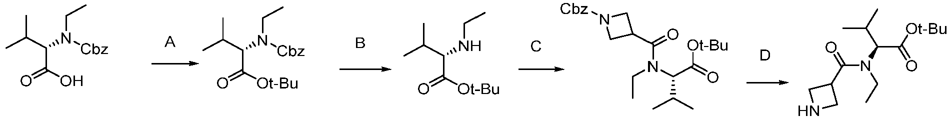

### Step A

A solution of *N*-((benzyloxy)carbonyl)-N-ethyl-L-valine (880 mg, 3.15 mmol, 1.0 equiv) and *tert-*butyl 3,3,3-trichloro-2-iminopropanoate (3.09 g, 12.53 mmol, 4.0 equiv) in THF/dichloromethane (1:4, 10 mL) was stirred for 6 days at room temperature. The crude product was purified by reverse phase chromatography with the following conditions (0% MeCN to 100% MeCN) to afford tert-butyl N-((benzyloxy)carbonyl)-N-ethyl-L-valinate (520 mg, 49% yield) as a yellow oil. ESI-MS *m*/*z* = 336.4 [M+H]⁺.

### Step B

A solution of *tert*-butyl *N*-((benzyloxy)carbonyl)-N-ethyl-L-valinate (500 mg, 1.49 mmol, 1.0 equiv) and Pd/C (10%, 50 mg, 0.47 mmol, 0.3 equiv) in MeOH (5.0 mL) was stirred for 3 hours at room temperature under an atmosphere of hydrogen. The mixture was filtered through celite. The filtrate was concentrated to give tert-butyl ethyl-L-valinate (270 mg, 90% yield) as an oil. ¹H NMR (300 MHz, DMSO-*d*₆) *δ* 2.77 (d, *J* = 6.2 Hz, 1H), 2.65 - 2.52 (m, 1H), 2.38 (t, *J* = 9.2 Hz, 1H), 1.78 (dt, *J* = 13.3, 6.8 Hz, 1H), 1.43 (t, *J =* 1.5 Hz, 9H), 0.99 (t, *J =* 7.1 Hz, 3H), 0.88 (dt, *J = 6.2,* 2.7 Hz, 6H).

### Step C

A solution of *tert*-butyl ethyl-L-valinate (265 mg, 1.32 mmol, 1.0 equiv), DIEA (510 mg, 3.95 mmol, 3.0 equiv), 1-[(benzyloxy)carbonyl]azetidine-3-carboxylic acid (371 mg, 1.58 mmol, 1.2 equiv) and HATU (751 mg, 1.98 mmol, 1.5 equiv) in CH₂Cl₂ (3.0 mL) was stirred for 1 hour at 0 °C. The residue was purified directly by Prep-TLC (PE/ethyl acetate 1:1) to afford benzyl (S)-3-((1-(tert-butoxy)-3-methyl-1-oxobutan-2-yl)(ethyl)carbamoyl)azetidine-1-carboxylate (500 mg, 91% yield) as a yellow oil. ESI-MS *m*/*z* = 419.4 [M+H]⁺.

### Step D

A solution of benzyl *(S)-3-((1-(tert-butoxy)-3-methyl-1-oxobutan-2-yl)(ethyl)carbamoyl)azetidine-1-*carboxylate (460 mg, 1.10 mmol, 1.0 equiv) and Pd/C (10%, 150 mg, 1.41 mmol, 1.28 equiv) in ethyl acetate/MeOH (1:1, 8 mL) was stirred for 3 hours at room temperature under an atmosphere of H₂. The resulting mixture was filtered through celite. The filtrate was concentrated under reduced pressure to give tert-butyl N-(azetidine-3-carbonyl)-N-ethyl-L-valinate (300 mg, 96% yield) as a yellow oil ESI-MS *m*/*z* = 285.2 [M+H]⁺.

### CU: tert-butyl (S)-3-cyclobutyl-2-(methylamino)propanoate

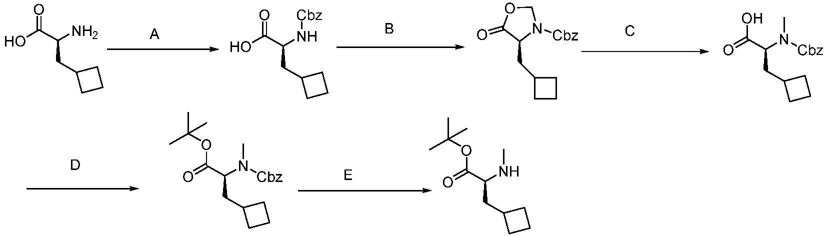

### Step A

To a stirred solution of (2S)-2-amino-3-cyclobutylpropanoic acid (3.0 g, 20.9 mmol, 1 equiv) in THF (30 mL) and H₂O (30 mL) at 0 °C was added sodium bicarbonate (5.28 g, 62.9 mmol, 3.0 equiv) and benzyl 2,5-dioxopyrrolidin-1-yl carbonate (7.83 g, 31.4 mmol, 1.5 equiv) in portions. The mixture was stirred for 16 hours at room temperature and then acidified to pH 5 with aqueous 1N HCl. The resulting mixture was extracted with ethyl acetate (3 x 150 mL). The combined organic layers were washed with brine and dried over anhydrous sodium sulfate. After filtration, the filtrate was concentrated under reduced pressure and the residue was purified by silica gel column chromatography, eluting with petroleum ether/ethyl acetate (2:1) to afford (S)-2-(((benzyloxy)carbonyl)amino)-3-cyclobutylpropanoic acid (5.4 g, 74% yield) as a yellow oil. ESI-MS *m*/*z* = 278.1 [M+H]⁺.

### Step B

A solution of (S)-2-(((benzyloxy)carbonyl)amino)-3-cyclobutylpropanoic acid (5.4 g, 19.5 mmol, 1 equiv), toluene (55 mL), paraformaldehyde (5.84 g, 194.721 mmol, 10.0 equiv) and TsOH (0.34 g, 1.95 mmol, 0.10 equiv) was stirred for 16 hours at 100 °C. The resulting mixture was filtered and the filtrate was concentrated under vacuum. The residue was purified by silica gel chromatography, eluting with petroleum ether/ethyl acetate (4:1) to afford benzyl (*S*)-4-(cyclobutylmethyl)-5-oxooxazolidine-3-carboxylate (2.5 g, 35% yield) as a yellow oil. ¹H-NMR (400 MHz, DMSO-*d*₆) δ 7.45 - 7.35 (m, 5H), 5.45 (d, *J =* 4.1 Hz, 1H), 5.28 (d, *J* = 4.2 Hz, 1H), 5.24 - 5.06 (m, 2H), 4.27 (t, *J* = 5.3 Hz, 1H), 2.32 (h, *J* = 8.3 Hz, 1H), 2.10 - 1.90 (m, 3H), 1.89 - 1.46 (m, 5H).

### Step C

To a stirred solution of benzyl (S)-4-(cyclobutylmethyl)-5-oxooxazolidine-3-carboxylate (2.5 g, 8.641 mmol, 1 equiv) in trichloromethane (30 mL) at 0 °C was added Et₃SiH (6.98 mL) followed by TFA (15 mL) dropwise. The resulting mixture was stirred for 16 hours at room temperature and then concentrated under vacuum. The residue was purified by Prep-TLC (PE/ethyl acetate 2:1) to afford (S)-2-(((benzyloxy)carbonyl)(methyl)amino)-3-cyclobutylpropanoic acid (2.2 g, 79% yield) as a yellow oil. ESI-MS *m*/*z* = 292.2 [M+H]⁺.

### Step D

(*S*)-2-(((Benzyloxy)carbonyl)(methyl)amino)-3-cyclobutylpropanoic acid (2.2 g, 7.551 mmol, 1 equiv) and tert-butyl 3,3,3-trichloro-2-iminopropanoate (14.89 g, 60.409 mmol, 8 equiv) in THF (5 mL) was stirred for 2 days at room temperature. The resulting mixture was concentrated under reduced pressure. The resulting mixture was filtered and the filter cake was washed with dichloromethane (3 x 50 mL). The filtrate was concentrated under reduced pressure and then purified by silica gel chromatography, eluting with petroleum ether/ethyl acetate (4:1) to afford tert-butyl (S)-2-(((benzyloxy)carbonyl)(methyl)amino)-3-cyclobutylpropanoate (2.3 g, 70% yield) as a yellow oil. ESI-MS *m*/*z* = 370.2 [M+Na]⁺

### Step E

A solution of *tert-*butyl (*S*)-2-(((benzyloxy)carbonyl)(methyl)amino)-3-cyclobutylpropanoate (2.3 g, 6.620 mmol, 1 equiv) in toluene (30 mL) was treated with Pd/C (500 mg, 5% on carbon). The solution was purged with hydrogen and the reaction mixture was stirred for 16 hours at room temperature under an atmosphere of hydrogen. The resulting mixture was filtered and the filter cake was washed with ethyl acetate (3 x 100 mL). The filtrate was concentrated under reduced pressure to give *tert-butyl (S)-3-*cyclobutyl-2-(methylamino)propanoate (1.9 g, crude) as a yellow oil. The crude product was used in the next step directly without further purification. ESI-MS *m*/*z* = 214.3 [M+H]⁺.

The following intermediates were synthesized according to the procedure described to make Intermediate CU using appropriate building blocks and modified reaction conditions (such as reagents, ratio of reagents, temperature, and reaction time) as needed.

| **Intermediate No.** | **Structure** | **Analytical Data** |
|---|---|---|
| CU-1 | 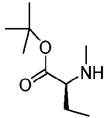 | ESI-MS *m*/*z* = 174.2 [M+H]+ |
| CU-2 | 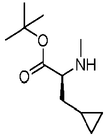 | ESI-MS *m*/*z* = 200.2 [M+H]+ |

### CV: N-(1-(2-chloroacetyl)azetidine-3-carbonyl)-N-methyl-L-valine (PH-SF-42H)

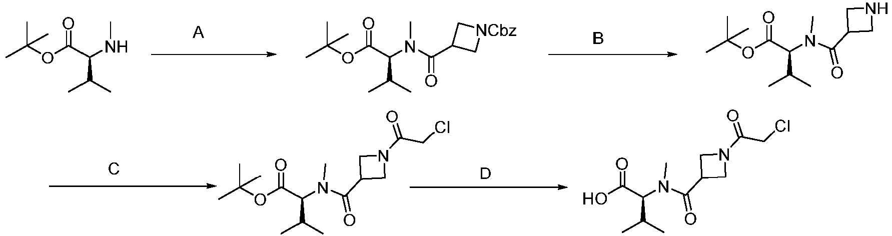

### Step A

To a stirred solution of *tert-butyl* (2*S*)-3-methyl-2-(methylamino)butanoate (1.5 g, 8.01 mmol, 1.0 equiv) and DIEA (2.1 g, 16.02 mmol, 2.0 equiv) in dichloromethane (15 mL) was added 1-[(benzyloxy)carbonyl]azetidine-3-carboxylic acid (1.9 g, 8.01 mmol, 1 equiv) and CIP (3.3 g, 12.01 mmol, 1.5 equiv) in portions at 0 °C. The resulting mixture was stirred for 2 hours at room temperature and the solution was then concentrated under reduced pressure. The residue was purified by prep-TLC (petroleum ether / ethyl acetate 5:1 w/ 0.1% TEA) to afford benzyl 3-[[(2S)-1-(tert-butoxy)-3-methyl-1-oxobutan-2-yl](methyl)carbamoyl] azetidine-1-carboxylate(1.2 g, 37%) as a colorless oil. ESI-MS *m*/*z* = 427.40 [M+Na]⁺.

### Step B

To a solution of benzyl 3-[[(2*S*)-1-(*tert*-butoxy)-3-methyl-1-oxobutan-2-yl](methyl)carbamoyl]azetidine-1-carboxylate (1.2 mg, 1 equiv) in acetonitrile (20 mL) was added Pd/C (10%, 120 mg) under a nitrogen atmosphere. The mixture was stirred under an atmosphere of hydrogen for 4 h, filtered through celite, and concentrated under reduced pressure. The crude product was used in the next step directly without further purification. ESI-MS *m*/*z* = 271.20 [M+H]⁺

### Step C

To a solution of tert-butyl N-(azetidine-3-carbonyl)-N-methyl-L-valinate (6 g, 22.19 mmol, 1.0 equiv) in dichloromethane (50 mL) at 0 °C was added triethylamine (3.4 g, 33.29 mmol, 1.5 equiv) followed by dropwise addition of 2-chloroacetyl chloride (2.8 g, 24.41 mmol, 1.10 equiv). The resulting solution was stirred for 1 hour at 0 °C and then the mixture was concentrated under vacuum. The residue was purified by reverse phase chromatography (10-50% MeCN in water with 0.1% FA) to give tert-butyl *N*-(1-(2-chloroacetyl)azetidine-3-carbonyl)-N-methyl-L-valinate (2.03 g, 26% yield) as a light brown oil. ¹H-NMR (300 MHz, DMSO-*d*₆) δ 4.57-4.51 (m, 1H), 4.42-4.37 (m, 1H), 4.35-4.24 (m, 1H), 5.30-4.12 (s, 3H), 3.96-3.81 (m, 2H), 2.81-2.76 (m, 3H), 2.17-2.08 (m, 1H), 1.42 (s, 9H), 0.95 (d, *J =* 6.6Hz, 3H), 0.79 (d, *J =* 4.4Hz, 3H).

### Step D

A solution of *tert-butyl N*-(1-(2-chloroacetyl)azetidine-3-carbonyl)-N-methyl-L-valinate (200 mg, 0.58 mmol, 1 equiv) in dichloromethane (4 mL), at 0 °C was treated with trifluoroacetic acid (2 mL). The resulting solution was stirred for 30 min at 0 °C and then 3 hours at room temperature. The resulting mixture was concentrated to give *N*-(1-(2-chloroacetyl)azetidine-3-carbonyl)-N-methyl-L-valine (220 mg) as a crude solid that was used without further purification. ESI-MS *m*/*z* = 291.1 [M+H]⁺.

The following compounds were synthesized according to the procedure described to make Intermediate CV using appropriate building blocks and modified reaction conditions (such as reagents, ratio of reagents, temperature, and reaction time) as needed.

| **Intermediate No.** | **Structure** | **Analytical Data** |
|---|---|---|
| CV-1 | 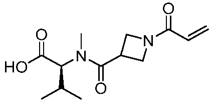 | ESI-MS m/z = 269.1 [M+H]+ |
| CV-2 | 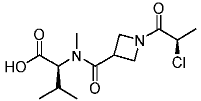 | ESI-MS *m*/*z* = 305.2 [M+H]+. |
| CV-3 | 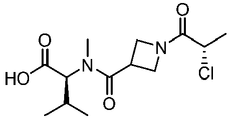 | ESI-MS *m*/*z* = 305.2 [M+H]+ |
| CV-4 | 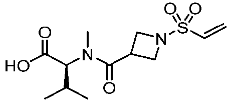 | ESI-MS *m*/*z* = 305.14 [M+H]+ |
| CV-5 | 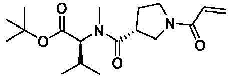 | ESI-MS *m*/*z* = 283.2 [M+H]+ |
| CV-6 | 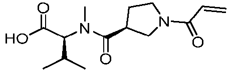 | ESI-MS *m*/*z* = 283.2 [M+H]+ |
| CV-7 | 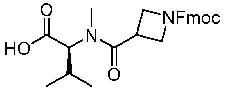 | ESI-MS *m*/*z* = 437.2 [M+H]+ |
| CV-8 | 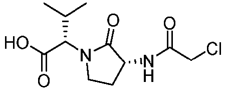 | ESI-MS *m*/*z* = 263.1 [M+H]+ |
| CV-9 | 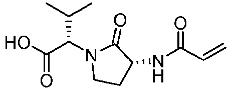 | |
| CV-10 | 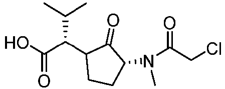 | ESI-MS *m*/*z* = 291.1 [M+H]+ |
| CV-11 | 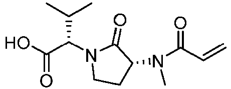 | ESI-MS *m*/*z* = 269.1 [M+H]+ |
| CV-12 | 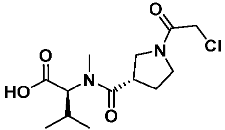 | ESI-MS *m*/*z* = 305.1 [M+Na]+ |
| CV-13 | 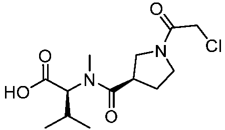 | ESI-MS *m*/*z* = 305.2 [M+H]+ |
| CV-14 | 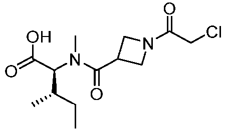 | ESI-MS *m*/*z* = 305.1 [M+H]+ |
| CV-15 | 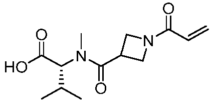 | ESI-MS *m*/*z* = 291.1 [M+H]+ |
| CV-16 | 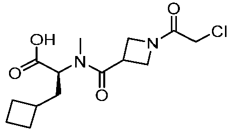 | ESI-MS *m*/*z* = 317.2 [M+H]+ |
| CV-17 | 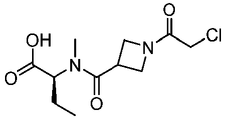 | ESI-MS *m*/*z* = 277.1 [M+H]+ |
| CV-18 | 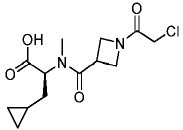 | ESI-MS *m*/*z* = 303.1[M+H]+ |
| CV-19 | 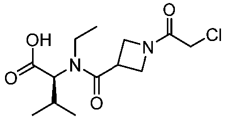 | ESI-MS *m*/*z* = 305.1 [M+H]+; ¹H NMR (300 MHz, DMSO-*d*₆) δ 13.1 (s, 0.5H), 12.5 (s, 0.5H), 4.39 - 4.28 (m, 2H), 4.24 - 4.01 (m, 3H), 3.94 - 3.79 (m, 2H), 3.64 - 3.61 (m, 1H), 3.28 - 3.22 (m, 2H), 2.25 - 2.15 (m, 1H), 1.12 - 0.96 (m, 6H), 0.81 - 0.76 (m, 3H). |
| CV-20 | 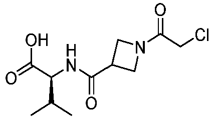 | ESI-MS *m*/*z* = 277.1 [M+H]+ |
| CV-21 | 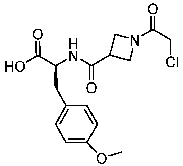 | ESI-MS *m*/*z* = 355.0 [M+H]+ |

### CW: N-(1-(but-2-ynoyl)azetidine-3-carbonyl)-N-methyl-L-valine

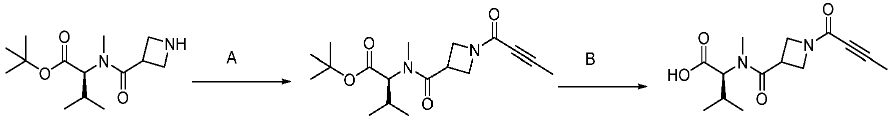

### Step A

A solution of tert-butyl N-(azetidine-3-carbonyl)-N-methyl-L-valinate (1 g, 3.70 mmol, 1 equiv), MeCN (10 mL), DIEA (1.4 g, 11.10 mmol, 3 equiv), but-2-ynoic acid (373.1 mg, 4.44 mmol, 1.2 equiv), and CIP (1.5 g, 5.55 mmol, 1.5 equiv) was stirred for 1 hour at 0 °C. The solvent was removed and the crude product was purified by silica gel chromatography (petroleum ether/ethyl aceate (2:3)), to give *tert-*butyl N-(1-(but-2-ynoyl)azetidine-3-carbonyl)-N-methyl-L-valinate (1.0 g , 80% yield) as a brown oil. ESI-MS *m*/*z* = 337.4 [M+H]⁺.

### Step B

N-(1-(but-2-ynoyl)azetidine-3-carbonyl)-N-methyl-L-valine was prepared from tert-butyl N-(1-(but-2-ynoyl)azetidine-3-carbonyl)-N-methyl-L-valinate using a procedure similar to the one described for the synthesis of N-(1-(2-chloroacetyl)azetidine-3-carbonyl)-N-methyl-L-valine. ESI-MS *m*/*z* = 281.2 [M+H]⁺.

The following intermediates were synthesized according to the procedure described to make Intermediate CW using appropriate building blocks and modified reaction conditions (such as reagents, ratio of reagents, temperature, and reaction time) as needed.

| **Intermediate No.** | **Structure** | **Analytical Data** |
|---|---|---|
| CW-1 | 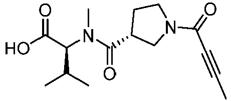 | ESI-MS *m*/*z* = 295.2 [M+H]+ |
| CW-2 | 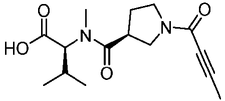 | ESI-MS *m*/*z* = 295.2 [M+H]+ |
| CW-3 | 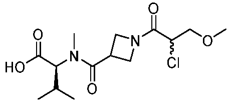 | ESI-MS *m*/*z* = 335.1 [M+H]+ |

### CX: N-(1-acryloyl-3-fluoroazetidine-3-carbonyl)-N-methyl-L-valine

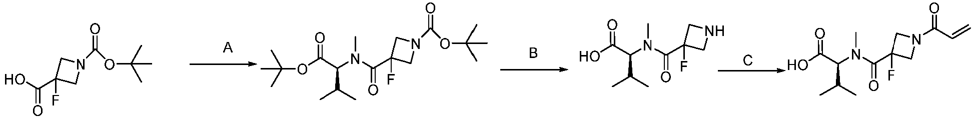

### Step A

To a solution of 1-[(tert-butoxy)carbonyl]-3-fluoroazetidine-3-carboxylic acid (220 mg, 1.004 mmol, 1.0 equiv), tert-butyl (2S)-3-methyl-2-(methylamino)butanoate (225.55 mg, 1.204 mmol, 1.2 equiv) and DIEA (389 mg, 3.01 mmol, 3 equiv) in acetonitrile (3.0 mL) at 0 °C was added HATU (763 mg, 2.01 mmol, 2 equiv). The resulting solution was stirred for 2 hours at room temperature. The solution was diluted with 100 mL of ethyl acetate. The layers were separated and the aqueous layer was washed with 2 x 50 ml of NH₄Cl and 2 x 50 mL of brine. The resulting mixture was concentrated under vacuum. The crude product was purified by Prep-HPLC (5-95% acetonitrile in water with 0.1% FA) to give *tert-butyl (S)-*3-((1-(*tert*-butoxy)-3-methyl-1-oxobutan-2-yl)(methyl)carbamoyl)-3-fluoroazetidine-1-carboxylate (360 mg 92% yield) as a brown oil. ESI-MS *m*/*z* = 411.2 [M+Na]⁺.

### Step B

A solution of *tert-butyl* (*S*)-3-((1-(*tert*-butoxy)-3-methyl-1-oxobutan-2-yl)(methyl)carbamoyl)-3-fluoroazetidine-1-carboxylat (360 mg, 0.927 mmol, 1.0 equiv) and TFA (2 mL) in dichloromethane (4 mL) was stirred at 0 °C for 2 h. The solid was concentrated *in vacuo* to give N-(3-fluoroazetidine-3-carbonyl)-N-methyl-L-valine (220 mg) (crude) as a brown oil. ESI-MS *m*/*z* = 233.4 [M+H]⁺.

### Step C

To a solution of *N*-(1-acryloyl-3-fluoroazetidine-3-carbonyl)-N-methyl-L-valine (220 mg, 0.947 mmol, 1.0 equiv) and DIEA (367.27 mg, 2.842 mmol, 3 equiv) in dichloromethane (4.0 mL) at 0 °C, was added prop-2-enoyl chloride (103 mg, 1.14 mmol, 1.2 equiv). The resulting solution was stirred for 1 hour at 0 °C. The mixture was concentrated under vacuum. The crude product was purified by Prep-HPLC (5-95% water in acetonitrile with 0.1% FA) to give N-(1-acryloyl-3-fluoroazetidine-3-carbonyl)-N-methyl-L-valine (37% yield) as a light yellow oil. ESI-MS *m*/*z* = 287.1 [M+H]⁺.

The following intermediate was synthesized according to the procedure described to make Intermediate CX using appropriate building blocks and modified reaction conditions (such as reagents, ratio of reagents, temperature, and reaction time) as needed.

| **Intermediate No.** | **Structure** | **Analytical Data** |
|---|---|---|
| CX-1 | 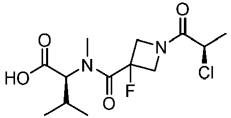 | ESI-MS *m*/*z* = 295.2 [M+H]+ |

### CY: N-((R)-1-acryloylazetidine-2-carbonyl)-N-methyl-L-valine

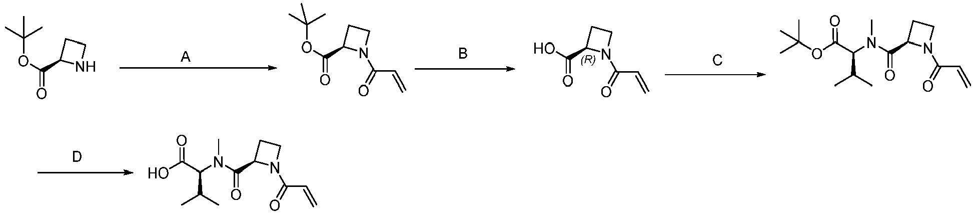

### Step A

To a solution of tert-butyl (R)-azetidine-2-carboxylate in ethyl acetate (30 mL) was added dropwise acryloyl chloride (2.2 g, 25 mmol, 1.0 equiv) at 0 °C. The reaction mixture was stirred for 10 minutes at 0 °C and then water was added. The layers were separated and the organic layer was washed with brine (10 mL), dried over anhydrous sodium sulfate, filtered, and the solvent was removed under reduced pressure. The residue was purified by column chromatography on silica gel (33-50% ethyl acetate in petroleum ether) to give the *tert-butyl* (*R*)-1-acryloylazetidine-2-carboxylate (3.5 g) as a white oil. ESI-MS *m*/*z* = 212.1 [M+H]⁺.

### Step B

To a solution of *tert-butyl* (*R*)-1-acryloylazetidine-2-carboxylate (3.5 g, 16.5 mmol, 1.0 equiv) in dichloromethane (16.0 mL) was added TFA (48.0 mL) at 20 °C. The resulting solution was stirred at 20 °C for 1 hour. The solvent was removed under reduced pressure to give (*R*)-1-acryloylazetidine-2-carboxylic acid (4.0 g) as a white solid. This crude product was used in the next step without further purification. ESI-MS *m*/*z* = 156.1 [M+H]⁺.

### Step C

To a stirred solution of (*R*)-1-acryloylazetidine-2-carboxylic acid (4.0 g crude, 13.2 mmol, 1.0 equiv) and tert-butyl methyl-L-valinate (5 g, 26.4 mmol, 2.0 equiv) in DMF (30 mL) was added DIEA (8 g, 66 mmol, 5.0 equiv) followed by HATU (7.4 mg, 19.8 mmol, 1.5 equiv) at 20 °C. The resulting solution was stirred for 1 hour. The solution was diluted with ethyl acetate (100 mL) and water (40 mL). Layer were separated and the organic layer was washed with water (3 × 30 mL), brine (30 mL), dried over anhydrous sodium sulfate, filtered, and the solvent was removed under reduced pressure. The residue was purified by reverse phase chromatography (0.1%formic acid in MeCN/Water) to give the tert-butyl N-((*R*)-1-acryloylazetidine-2-carbonyl)-N-methyl-L-valinate (1.2 g, P: 98%) as a white solid. ESI-MS *m*/*z* = 325.2 [M+H]⁺. ¹H NMR (400 MHz, DMSO-*d₆*) δ 6.33 (ddd, J= 17.0, 10.3, 2.9 Hz, 0.5H), 6.17 - 5.77 (m, 2H), 5.69 (dd, *J =* 10.3, 1.7 Hz, 0.5H), 5.62 - 5.42 (m, 1.5H), 5.33 - 5.06 (m, 0.5H), 4.42 (dd, *J* = 10.3, 3.7 Hz, 0.5H), 4.12 (dt, *J =* 11.3, 6.3 Hz, 1H), 3.96 - 3.75 (m, 1.5H), 3.69 (d, *J* = 10.4 Hz, 0.5H), 2.91 (d, *J* = 15.0 Hz, 0.5H), 2.83 (s, 1H), 2.75 (d, *J=* 13.1 Hz, 0.5H), 2.72 (s, 1.5H), 2.16 (ddd, *J=* 10.3, 8.7, 5.3 Hz, 1H), 2.10 - 1.89 (m, 1H), 1.41 (d, *J =* 4.6 Hz, 9H), 0.95 (t, *J =* 5.8 Hz, 3H), 0.86 (dd, *J =* 6.6, 3.4 Hz, 1.5H), 0.82 - 0.73 (m, 1.5H).

### Step D

A solution of *tert-butyl N*-((*R*)-1-acryloylazetidine-2-carbonyl)-*N*-methyl-L-valinate (38 mg, 0.116 mmol, 1.0 equiv) in dichloromethane (1.0 mL) was treated with TFA (0.5 mL) at 20 °C. The resulting solution was stirred at 20 °C for 1 hour. The solvent was removed under reduced pressure to give *N-((R)-*1-acryloylazetidine-2-carbonyl)-*N*-methyl-L-valine (40 mg) as a yellow oil. This crude product was used in the next step without further purification. ESI-MS *m*/*z* = 269.1 [M+H]⁺.

The following compounds were synthesized according to the procedure described to make Intermediate CY using appropriate building blocks and modified reaction conditions (such as reagents, ratio of reagents, temperature, and reaction time) as needed.

| **Intermediate No.** | **Structure** | **Analytical Data** |
|---|---|---|
| CY-1 | 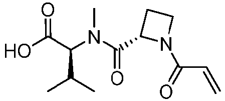 | ESI-MS *m*/*z* = 269.2 [M+H]+ |
| CY-2 | 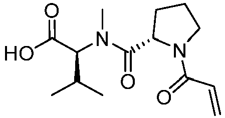 | ESI-MS *m*/*z* = 283.2 [M+H]+. |
| CY-3 | 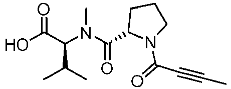 | ESI-MS *m*/*z* = 295.2 [M+H]+. |
| CY-4 | 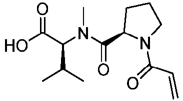 | |
| CY-5 | 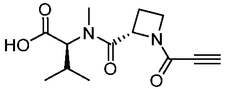 | ESI-MS *m*/*z* = 281.2 [M+H]+ |
| CY-6 | 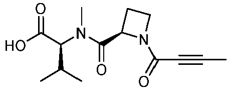 | ESI-MS *m*/*z* = 281.2 [M+H]+ |
| CY-7 | 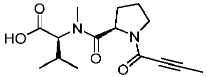 | ESI-MS *m*/*z :* 295.1 |

### CZ: (R)-3-methyl-2-(vinylsulfonamidomethyl)butanoic acid

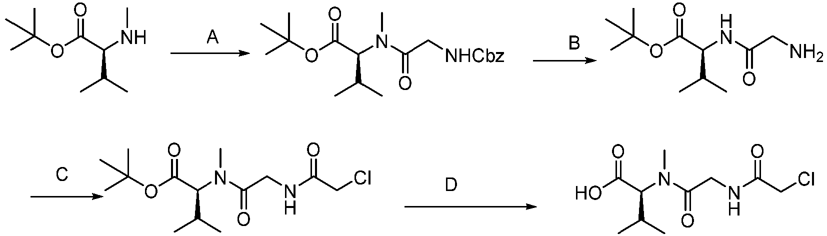

### Step A

To a stirred solution of tert-butyl methyl-L-valinate (350 mg, 1.86 mmol, 1.0 equiv) and [(benzyloxy)carbonyl]glycine (587 mg, 2.8 mmol, 1.5 equiv) in DMF (5 mL) at 0 °C was added DIEA (2400 mg, 18.7mmol, 10 equiv) dropwise at 0 °C. After 5 minutes, COMU (1600 mg, 3.7mmol, 2 equiv) was added in portions over the course of 5 min. The resulting mixture was stirred for 2 hours at 0 °C. The resulting mixture was diluted with water (50 mL) and extracted with ethyl acetate (3 x 20 mL). The combined organics were dried over anhydrous sodium sulfate. After filtration, the filtrate was concentrated under reduced pressure. The residue was purified by C18 reverse phase chromatography (0% MeCN to 100% MeCN in water with 0.05% FA) to give tert-butyl N-(((benzyloxy)carbonyl)glycyl)-N-methyl-L-valinate (469 mg, 66% yield) as an orange oil. ESI-MS *m*/*z* = 401.3 [M+Na]⁺.

### Step B

A solution of *tert*-butyl *N*-(((benzyloxy)carbonyl)glycyl)-N-methyl-L-valinate (459 mg, 1.21 mmol, 1.0 equiv) and Pd/C (150 mg, 1.41 mmol, 1.16 equiv) in ethyl acetate (5 mL) was stirred for 4 hours at room temperature under a hydrogen atmosphere. The resulting mixture was filtered, the filter cake was washed with ethyl acetate (3 x 20mL). The filtrate was concentrated under reduced pressure to afford *tert*-butyl glycyl-L-valinate (281 mg, 95% yield) as a yellow oil. The crude product was used in the next step directly without further purification. ESI-MS *m*/*z* = 231.3 [M+H]⁺.

### Step C

To a stirred solution of tert-butyl glycyl-L-valinate (120 mg, 0.49 mmol, 1.0 equiv) and TEA (149 mg, 1.47 mmol, 3.0 equiv) in dichloromethane (2.5 mL) at 0 °C was added 2-chloroacetyl chloride (83 mg, 0.73 mmol, 1.50 equiv) dropwise. The resulting mixture was stirred for 2 hours at 0 °C. After filtration, the mixture was concentrated under reduced pressure. The residue was purified by C18 reverse phase chromatography (0% MeCN to 100% MeCN with NH₄HCO₃, 0.5%) to give tert-butyl *N-((2-*chloroacetyl)glycyl)-N-methyl-L-valinate (104 mg, 66% yield) as a brown oil. ESI-MS *m*/*z* = 321.2 [M+H]⁺.

### Step D

To a stirred solution of tert-butyl N-((2-chloroacetyl)glycyl)-N-methyl-L-valinate (100 mg, 0.31 Mmol, 1.0 equiv) in dichloromethane (0.8 mL) was added TFA (0.5 mL) dropwise at 0 °C. After 2 hours, the resulting mixture was concentrated under reduced pressure. The residue was diluted with 5 mL toluene, and concentrated under vacuum again. The above procedure was repeated for another one additional time and (R)-3-methyl-2-(vinylsulfonamidomethyl)butanoic acid (148 mg) was obtained as a brown oil that was used in the next step directly without purification. ESI-MS *m*/*z* = 265.2 [M+H]⁺.

### DA: (S)-2-(2-acryloyl-5-oxo-2, 6-diazaspiro[3.4]octan-6-yl)-3-methylbutanoic acid

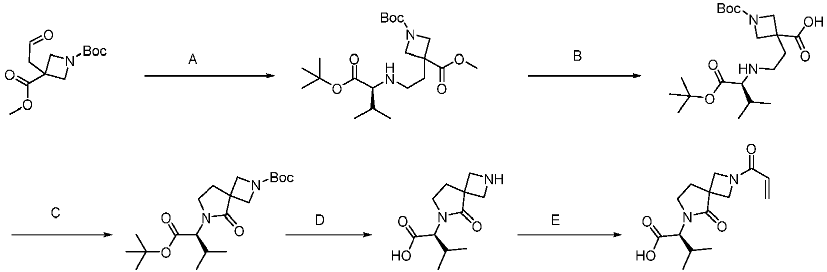

### Step A

To a stirred mixture of 1-tert-butyl 3-methyl 3-(2-oxoethyl)azetidine-1,3-dicarboxylate (1.4 g, 5.44 mmol, 1.0 equiv), *tert*-butyl (2*S*)-2-amino-3-methylbutanoate hydrochloride (1.37 g, 6.530 mmol, 1.2 equiv) and ZnCl₂ (0.74 g, 5.441 mmol, 1 equiv) in MeOH (30 mL) was added NaBH₃CN (0.34 g, 5.44 mmol, 1.0 equiv) at 0 °C. After stirring for 2 hours, the reaction was quenched with water (50 mL) at 0 °C and the methanol was removed under *in vacuo.* The resulting mixture was extracted with ethyl acetate (3 x 50 mL) and the combined organic layers were washed with brine (3 x 30 mL) and dried over anhydrous sodium sulfate. After filtration, the filtrate was concentrated under reduced pressure to give *1-tert-butyl* 3-methyl 3-(2-[[(2*S*)-1-(*tert*-butoxy)-3-methyl-1-oxobutan-2-yl]amino]ethyl)azetidine-1,3-dicarboxylate (1.2 g, 53% yield) as an oil. The crude product mixture was used in the next step directly without further purification. ¹ H NMR (300 MHz, CDCl₃) δ 4.32 - 4.07 (m, 4H), 3.76 (s, 4H), 2.88 (d, *J =* 5.6 Hz, 1H), 2.69 (ddd, *J =* 11.8, 7.5, 5.6 Hz, 1H), 2.55 - 2.40 (m, 1H), 2.26 - 2.06 (m, 2H), 1.91 (dq, *J* = 13.2, 6.7 Hz, 1H), 1.48 (s, 9H), 1.45 (s, 10H), 0.94 (d, *J =* 6.8 Hz, 6H).

### Step B

A solution of 1-*tert*-butyl 3-methyl 3-(2-[[(2*S*)-1-(*tert*-butoxy)-3-methyl-1-oxobutan-2-yl]amino]ethyl)azetidine-1,3-dicarboxylate (1.2 g, 2.89 mmol, 1.0 equiv) and LiOH-H₂O (607 mg, 14.5 mmol, 5.0 equiv) in MeOH/H2O (5/1) (40 mL) was stirred for 1 hour. The methanol was removed under reduced pressure and the aqueous was diluted with ethyl acetate (50 mL). The layers were separated and the organics were washed with 3 x 25 mL of brine and dried over anhydrous sodium sulfate. After filtration, the filtrate was concentrated under reduced pressure and the crude product *3-(2-[[(2S)-1-(tert-*butoxy)-3-methyl-1-oxobutan-2-yl]amino]ethyl)-1-[(*tert*-butoxy)carbonyl]azetidine-3-carboxylic acid was used in the next step directly without further purification. ¹H NMR (300 MHz, DMSO-*d*₆) δ = 4.01 (d, *J =* 8.4 Hz, 3H), 3.73 (d, *J =* 11.8 Hz, 4H), 2.85 (s, 2H), 2.25 (dd, *J =* 18.1, 6.8 Hz, 1H), 1.48 (s, 8H), 1.39 (d, *J* = 1.5 Hz, 13H), 1.04 (d, *J= 6.9* Hz, 3H), 0.94 (d, *J=* 6.8 Hz, 3H).

### Step C

A solution of 3-(2-[[(2*S*)-1-(*tert*-butoxy)-3-methyl-1-oxobutan-2-yl]amino]ethyl)-1-[(*tert-*butoxy)carbonyl]azetidine-3-carboxylic acid (1.2 g, 2.99 mmol, 1.0 equiv), COMU (2.56 g, 6.0 mmol, 2.0 equiv) and DIEA (1.94 g, 15 mmol, 5.0 equiv) in DMF (30 mL) was stirred for 1 hour at 0 °C. The mixture was diluted with water (80 mL) and the resulting mixture was extracted with ethyl acetate (3 x 50 mL). The combined organic layers were washed with brine (3 x 50 mL) and dried over anhydrous sodium sulfate. After filtration, the filtrate was concentrated under reduced pressure. The residue was purified by reverse phase chromatography with the following conditions (10% to 50% acetonitrile in water, 25 min gradient) to afford *tert-butyl 6-[(2S)-1-(tert-butoxy)-3-methyl-1-oxobutan-2-yl]-5-oxo-2,6-diazaspiro[3.4]octane-2-*carboxylate (800 mg, 70% yield) as a yellow solid. ¹H NMR (300 MHz, CDCl₃) δ 4.39 (d, *J =* 9.6 Hz, 1H), 4.22 (dd, *J =* 10.4, 8.3 Hz, 2H), 3.78 (dd, *J =* 8.3, 3.5 Hz, 2H), 3.62 (dt, *J =* 10.0, 6.6 Hz, 1H), 3.31 (dt, *J =* 10.0, 6.8 Hz, 1H), 2.39 - 2.23 (m, 2H), 2.18 (dp, *J =* 9.6, 6.7 Hz, 1H), 1.46 (d, *J =* 4.0 Hz, 17H), 1.01 (d, *J* = 6.7 Hz, 3H), 0.88 (d, *J =* 6.8 Hz, 3H).

### Step D

A solution of tert-butyl 6-[(2S)-3-methyl-1-oxo-1-(propan-2-yloxy)butan-2-yl]-5-oxo-2,6-diazaspiro[3.4]octane-2-carboxylate (200 mg, 0.543 mmol, 1.0 equiv) and TFA (2 mL) in dichloromethane (2 mL) was stirred for 1 hour. The resulting mixture was concentrated under vacuum to afford (2S)-3-methyl-2-[5-oxo-2,6-diazaspiro[3.4]octan-6-yl]butanoic acid (120 mg, 98% yield). The crude product was carried on without further purification. ¹H NMR (300 MHz, DMSO-*d*₆) δ 9.00 (s, 1H), 8.87 (s, 1H), 4.18 (d, *J=* 9.8 Hz, 1H), 3.99 (s, 1H), 3.50 - 3.37 (m, 1H), 3.31 (dt, *J* = 9.8, 6.8 Hz, 1H), 2.36 (t, *J* = 6.8 Hz, 2H), 2.21 - 2.03 (m, 1H), 0.94 (d, *J =* 6.6 Hz, 3H), 0.80 (d, *J =* 6.7 Hz, 3H).

### Step E

To a stirred solution of (2S)-3-methyl-2-[5-oxo-2,6-diazaspiro[3.4]octan-6-yl]butanoic acid (120 mg, 0.53 mmol, 1.0 equiv) and DIEA (342 mg, 2.65 mmol, 5.0 equiv) in dichloromethane (3 mL) was added prop-2-enoyl chloride (144 mg, 1.59 mmol, 3.0 equiv) at 0 °C and then maintatined for 3 hours at that temperature. The resulting mixture was concentrated under vacuum and purified by reverse phase chromatography (10% to 25% gradient in 30 min) to afford (2S)-3-methyl-2-[5-oxo-2-(prop-2-enoyl)-2,6-diazaspiro[3.4]octan-6-yl]butanoic acid (140 mg, 94% yield) as a white solid. ¹H NMR (300 MHz, DMSO-*d*₆) *δ* = 6.32 (ddd, *J =* 17.0, 10.2, 1.9 Hz, 1H), 6.11 (dd, *J* = 17.0, 2.3 Hz, 1H), 5.74 - 5.63 (m, 1H), 4.30 - 4.09 (m, 2H), 4.09 - 3.78 (m, 4H), 3.65 (s, 1H), 3.22 (d, *J =* 8.1 Hz, 1H), 2.20 (tq, *J =* 24.8, 9.4, 7.7 Hz, 2H), 0.93 (d, *J=* 6.5 Hz, 3H), 0.76 (d, *J =* 6.7 Hz, 3H).

The following intermediates were synthesized according to the procedure described to make Intermediate DA using appropriate building blocks and modified reaction conditions (such as reagents, ratio of reagents, temperature, and reaction time) as needed.

| **Intermediate No.** | **Structure** | **Analytical Data** |
|---|---|---|
| DA-1 | 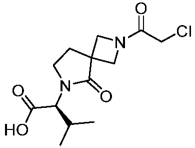 | ESI-MS *m*/*z* = 303.1 [M+H]+ |
| DA-2 | 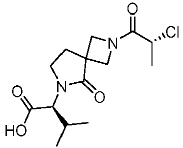 | ESI-MS *m*/*z* = 317.1 [M+H]+ |

### DB: (S)-2-(2-acryloyl-5-oxo-2,6-diazaspiro[3.4]octan-6-yl)-3-methylbutanoic aci

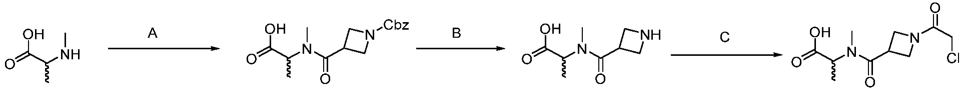

### Step A

To a stirred solution of (2S)-2-(methylamino)propanoic acid (600 mg, 5.82 mmol, 1.0 equiv) and DIEA (2.26 g, 17.46 mmol, 3.0 equiv) in dichloromethane (50 mL) was added benzyl 3-(carboxy)azetidine-1-carboxylate (1.77 g, 6.982 mmol, 1.2 equiv) dropwise at 0 °C. The resulting mixture was stirred for 2 hours at that temperature and then the mixture was diluted with dichloromethane (100 mL).The combined organic layers were washed with water (3 x 50 mL) and dried over anhydrous sodium sulfate. After filtration, the filtrate was concentrated under reduced pressure. The crude product was purified by reverse phase chromatography (0-100% MeCN in water with 0.05% FA) to afford *(2S)-2-(1-[1-*[(benzyloxy)carbonyl]azetidin-3-yl]-N-methylformamido)propanoic acid (700 mg, 22% yield) as a light yellow oil. ESI-MS *m*/*z* = 321.2 [M+H]⁺.

### Step B

A solution of (2*S*)-2-(1-[1-[(benzyloxy)carbonyl]azetidin-3-yl]-N-methylformamido)propanoic acid (700 mg, 2.185 mmol, 1 equiv) in methanol (50 mL) was treated wtih Pd/C (100 mg, 5% on carbon). Hydrogen gas was bubbled through the solution and then stirred for an additional 3 hours under an atmosphere of hydrogen. The resulting mixture was filtered and the filter cake was washed with MeOH (2 x 30 mL). The filtrate was concentrated under reduced pressure and the crude product was used in the next step directly without further purification. ESI-MS *m*/*z* = 187.1 [M+H]⁺.

### Step C

To a stirred solution of (2S)-2-[1-(azetidin-3-yl)-N-methylformamido]propanoic acid (400 mg, 1.074 mmol, 1 equiv) and DIEA (416 mg, 3.222 mmol, 3.0 equiv) in THF (30 mL) was added 2-chloroacetyl chloride (145 mg, 1.29 mmol, 1.2 equiv) dropwise at 0 °C. The resulting mixture was stirred for 2 hours at 20 °C and the resulting mixture was concentrated under vacuum. The residue was purified by reverse phase chromatography (0-100% MeCN in water with 0.05% FA) to afford (2S)-2-[1-[1-(2-chloroacetyl)azetidin-3-yl]-N-methylformamido]propanoic acid (100 mg, 24.81% yield) as a yellow oil. ESI-MS *m*/*z* = 263.2 [M+H]⁺.

### DC: N-(2-(3,4-dimethyl-2,5-dioxo-2,5-dihydro-1H-pyrrol-1-yl)acetyl)-N-methyl-L-valine

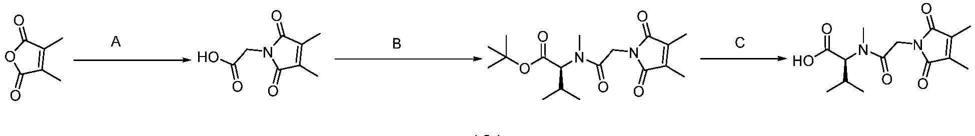

### Step A

A solution of dimethyl-2,5-dihydrofuran-2,5-dione (2.0 g, 15.86 mmol, 1.0 equiv), 2-aminoacetic acid (1.2 g, 15.86 mmol, 1.0 equiv) and acetic acid (20 mL) was irradiated with microwave radiation for 2 hours at 120 °C. The crude product was purified by C18 reverse phase chromatography (2-4% MeCN in water (10 mmol/L TFA)) to give 2-(3,4-dimethyl-2,5-dioxo-2,5-dihydro-1H-pyrrol-1-yl)acetic acid (1.06 g, 34%) as a light yellow solid. ESI-MS *m*/*z* = 182.0 [M-H]⁺.

### Step B

Into a 40-mL vial was placed 2-(3,4-dimethyl-2,5-dioxo-2,5-dihydro-1H-pyrrol-1-yl)acetic acid (200 mg, 1.09 mmol, 1.0 equiv), dichloromethane (2.0 mL), DIEA (0.9 mL, 6.98 mmol, 5 equiv), tert-butyl (2S)-3-methyl-2-(methylamino)butanoate (245 mg, 1.31 mmol, 1.2 equiv) and HATU (830 mg, 2.18 mmol, 2.0 equiv). The resulting solution was stirred for 2 hours at 0 °C and then the resulting mixture was concentrated. The residue was purified by silica gel chromatography with ethyl acetate/petroleum ether (1/1) to give *tert*-butyl (2S)-2-[2-(3,4-dimethyl-2,5-dioxo-2,5-dihydro-1H-pyrrol-1-yl)-*N*-methylacetamido]-3-methylbutanoate (260 mg, 68% yield) as a yellow oil. ESI-MS *m*/*z* = 353.3 [M+H]⁺.

### Step C

A solution of *tert*-butyl (2*S*)-2-[2-(3,4-dimethyl-2,5-dioxo-2,5-dihydro-1H-pyrrol-1-yl)-N-methylacetamido]-3-methylbutanoate (35 mg), dichloromethane (1.5 mL), and TFA (1 mL) was stirred for 2 hours at 0 °C. The resulting mixture was concentrated to give 30 mg of (2S)-2-[2-(3,4-dimethyl-2,5-dioxo-2,5-dihydro-1H-pyrrol-1-yl)-N-methylacetamido]-3-methylbutanoic acid as a yellow oil. ESI-MS *m*/*z* = 297.2 [M+H]⁺.

### Example 2 - Synthesis of (2S)-2-(3-acryloyl-2-oxoimidazolidin-1-yl)-N-((6³S,4S)-2⁵-hydroxy-1²,10,10-trimethyl-5,7-dioxo-6¹,6²,6³,6⁴,6⁵,6⁵-hexahydro-1¹H-8-oxa-1(6,1)-benzo[d]imidazola-6(1,3)-pyridazina-2(1,3)-benzenacycloundecaphane-4-yl)-3-methylbutanamide (Compound 1)

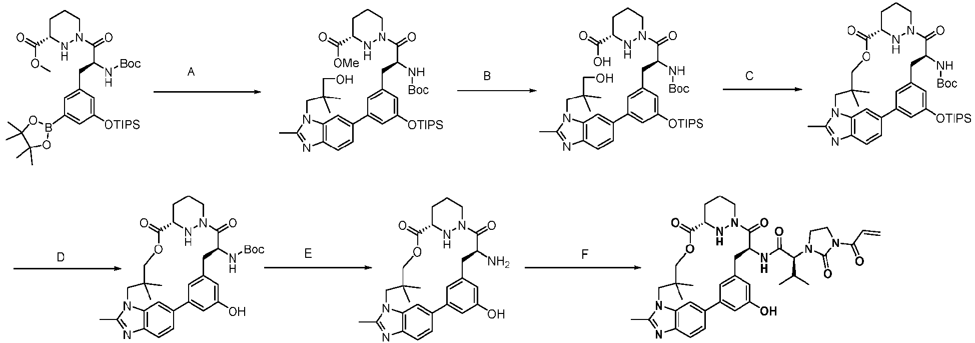

### Step A

A solution of methyl (*S*)-1-((*S*)-2-((tert-butoxycarbonyl)amino)-3-(3-(4,4,5,5-tetramethyl-1,3,2-dioxaborolan-2-yl)-5-((triisopropylsilyl)oxy)phenyl)propanoyl)hexahydropyridazine-3-carboxylate (1.0 g, 1.4 mmol, 1.0 eq), 3-(6-bromo-2-methyl-1H-benzo[d]imidazol-1-yl)-2,2-dimethylpropan-1-ol (430 mg, 1.4 mmol, 1.0 eq), Pd(dppf)Cl₂ (100 mg, 10 mol %), and K₂CO₃ (500 mg, 3.6 mmol, 2.5 eq) in dioxane (30 mL) was stirred at 75°C for 16 hours. After concentration, the residue was purified by silica gel column chromatography (petroleum ether/ethyl acetate (5/1 to 1/3)) to afford methyl *(S)-1-((S)-2-((tert-*butoxycarbonyl)amino)-3-(3-(1-(3-hydroxy-2,2-dimethylpropyl)-2-methyl-1H-benzo[d]imidazol-6-yl)-5-((triisopropylsilyl)oxy)phenyl)propanoyl)hexahydropyridazine-3-carboxylate (330 mg, 27%) as a light yellow solid. ESI-MS *m*/*z* = 780.5 [M+H]⁺.

### Step B

A solution of methyl (*S*)-1-((*S*)-2-((*tert*-butoxycarbonyl)amino)-3-(3-(1-(3-hydroxy-2,2-dimethylpropyl)-2-methyl-1H-benzo[d]imidazol-6-yl)-5-((triisopropylsilyl)oxy)phenyl)propanoyl)hexahydropyridazine-3-carboxylate (450 mg, 0.58 mmol, 1.0 eq in, dichloroethane (5.0 mL) was treated with trimethyltin hydroxide (522 mg, 2.88 mmol, 5.0 equiv). The resulting solution was stirred for 6 hours at 60 °C. After concentration, the crude product was diluted with ethyl acetate (20 mL), washed with water (10 mL) and brine (10 mL), dried over sodium sulfate, and concentrated to afford (*S*)-1-((*S*)-2-((*tert*-butoxycarbonyl)amino)-3-(3-(1-(3-hydroxy-2,2-dimethylpropyl)-2-methyl-1H-benzo[d]imidazol-6-yl)-5-((triisopᵣopylsilyl)oxy)phenyl)propanoyl)hexahydropyridazine-3-carboxylic acid (400 mg) as a gray foam, which was used directly for the next step without further purification. ESI-MS *m*/*z* = 766.4 [M+H]⁺.

### Step C

To a solution of crude (*S*)-1-((*S*)-2-((*tert*-butoxycarbonyl)amino)-3-(3-(1-(3-hydroxy-2,2-dimethylpropyl)-2-methyl-1H-benzo[d]imidazol-6-yl)-5-((triisopropylsilyl)oxy)phenyl)propanoyl)hexahydropyridazine-3-carboxylic acid (360 mg, 1.0 equiv) in toluene (55 mL) was added 1,2-di(pyridin-2-yl)disulfane (610 mg, 2.76 mmol, 6.0 equiv) and triphenylphosphine (610 mg, 2.32 mmol, 5.0 equiv). The solution was stirred at 85°C for 3 hours. After concentration, the crude product was purified by silica gel chromatography (100% ethyl acetate) to give *tert-butyl* ((6³*S*,4*S*)-1²,10,10-trimethyl-5,7-dioxo-2⁵-((triisopropylsilyl)oxy)-6¹,6²,6³,6⁴,6⁵,6⁶-hexahydro-1¹*H-*8-oxa-1(6,1)-benzo[d]imidazola-6(1,3)-pyridazina-2(1,3)-benzenacycloundecaphane-4-yl)carbamate (250 mg, 64% yield) as a white foam. ESI-MS *m*/*z* = 748.4 [M+H]⁺; ¹H NMR (400 MHz, MeOD) δ 7.80 (d, *J=* 11.8 Hz, 1H), 7.60 (q, *J =* 6.5 Hz, 1H), 7.53 (d, *J =* 8.5 Hz, 1H), 7.28 (s, 1H), 7.08 (s, 1H), 6.86 (s, 1H), 5.24 (d, *J* = 3.7 Hz, 1H), 4.51 - 4.36 (m, 2H), 4.08 (d, *J* = 7.1 Hz, 1H), 3.86 (d, *J* = 11.8 Hz, 1H), 3.74 (d, J = 10.8 Hz, 1H), 3.49 (d, *J =* 11.9 Hz, 1H), 2.91 - 2.69 (m, 3H), 2.65 (d, *J* = 4.1 Hz, 3H), 2.17 (d, *J* = 10.3 Hz, 1H), 1.92 (d, *J =* 12.6 Hz, 1H), 1.78 - 1.54 (m, 2H), 1.41 (d, *J =* 16.5 Hz, 9H), 1.31 (d, *J =* 7.2 Hz, 3H), 1.20 - 1.11 (m, 24H).

### Step D

To a solution of *tert-butyl* ((6³*S*,4*S*)-1²,10,10-trimethyl-5,7-dioxo-2⁵-((triisopropylsilyl)oxy)-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)carbamate (230 mg, 0.31 mmol, 1.0 equiv) in THF (5 mL) was added TBAF (1 M in THF, 0.31 mmol, 1.0 equiv). The solution was stirred at room temperature for 0.5 hours. After concentration, the crude product was diluted with ethyl acetate (20 mL) and washed with water (5 mL x 5). The organics were dried over anhydrous sodium sulfate, filtered, and the solvent was removed under reduce pressure to give the *tert*-butyl ((6³*S*,4*S*)-2⁵-hydroxy-1²,10,10-trimethyl-5,7-dioxo-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)carbamate (220 mg) as a white solid. ESI-MS *m*/*z* = 592.3[M+H]⁺.

### Step E

To a solution of *tert*-butyl ((6³*S*,4*S*)-2⁵-hydroxy-1²,10,10-trimethyl-5,7-dioxo-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)carbamate (200 mg, 0.34 mmol, 1.0 equiv) in dichloromethane (10 mL) was added trifluoroacetic acid (2.0 mL). The resulting solution was stirred for 2 hours and then the solvent was removed under reduced pressure to give the TFA salt of (6³S,4S)-4-amino-2⁵-hydroxy-1²,10,10-trimethyl-6¹,6²,6³,6⁴,6⁵,6⁶hexahydro-1¹*H*-8-oxa-1(6,1)-benzo[d]imidazola-6(1,3)-pyridazina-2(1,3)-benzenacycloundecaphane-5,7-dione (200 mg) as a white solid that was used without further purification. ESI-MS *m*/*z* = 492.1 [M+H]⁺.

### Step F

To a stirred solution of (S)-2-(3-acryloyl-2-oxoimidazolidin-1-yl)-3-methylbutanoic acid (48 mg, 0.20 mmol, 1.0 equiv) in DMF (6 mL) was added HATU (114 mg, 0.30 mmol, 1.5 equiv) and diethylisopropylamine (130 mg, 1.0 mmol, 5.0 equiv). After stirring for 10 minutes at room temperature, the TFA salt of (6³*S*,4*S*)-4-amino-2⁵-hydroxy-1²,10,10-trimethyl-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-5,7-dione (120 mg , 0.20 mmol, 1.0 equiv) in DMF (1 mL) was added. The resulting solution was stirred for 1 hour and then diluted with ethyl acetate (10 mL) and water (10 mL). The layers was separated and the organic layer was washed with water (3 × 10 mL), brine (10 mL), dried over anhydrous sodium sulfate, filtered, and the solvent was removed under reduced pressure. The residue was purified by reverse phase preparative HPLC(0.1%formic acid in MeCN/Water) to give (2*S*)-2-(3-acryloyl-2-oxoimidazolidin-1-yl)-N-((6³*S*,4*S*)-2⁵-hydroxy-1²,10,10-trimethyl-5,7-dioxo-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)-3-methylbutanamide (15.5 mg, 11% yield) as a white solid.

The synthesis of Compound 1 is a representative example of the use of appropriate intermediates of Example 1 and procedures similar those described in Method A to make compounds of the invention.

### Example 3 - Synthesis of (2S)-2-(3-acryloyl-2-oxoimidazolidin-1-yl)-N-((6³S,4S)-2⁶-hydroxy-5,7-dioxo-6¹,6²,6³,6⁴,6⁶,6⁶-hexahydro-8,11-dioxa-1(6,4)-quinolina-6(1,3)-pyridazina-2(1,3)-benzenacycloundecaphane-4-yl)-3-methylbutanamide (Compound 2)

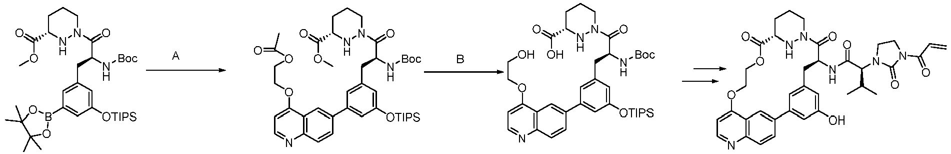

### Step A

A solution of methyl (3*S*)-1-[(2*S*)-2-[[(*tert*-butoxy)carbonyl]amino]-3-[3-(tetramethyl-1,3,2-dioxaborolan-2-yl)-5-[[tris(propan-2-yl)silyl]oxy]phenyl]propanoyl]-1,2-diazinane-3-carboxylate (500 mg, 0.72 mmol, 1 equiv) in 1,4-dioxane (5 mL) was treated with 2-[(6-bromoquinolin-4-yl)oxy]ethyl acetate (246 mg, 0.79 mmol, 1.1 equiv), K₂CO₃ (1.31 g, 9.50 mmol, 13.1 equiv) and Pd(dppf)Cl₂ (52.6 mg, 0.07 mmol, 0.1 equiv). The resulting solution was stirred for 6 hours at 65°C. The solids were filtered off and the solvent was removed *in vacuo.* The resulting residue was purified by silica gel chromatography (ethyl acetate/petroleum ether (3:1)) and purified to give methyl (3S)-1-[(2S)-3-(3-[4-[2-(acetyloxy)ethoxy]quinolin-6-yl]-5-[[tris(propan-2-yl)silyl]oxy]phenyl)-2-[[(*tert-*butoxy)carbonyl]amino]propanoyl]-1,2-diazinane-3-carboxylate (400 mg, 69% yield) as a yellow solid. ESI-MS *m*/*z* = 793.4 [M+H]⁺.

### Step B

A solution of methyl (3*S*)-1-[(2S)-3-(3-[4-[2-(acetyloxy)ethoxy]quinolin-6-yl]-5-[[tris(propan-2-yl)silyl]oxy]phenyl)-2-[[(*tert*-butoxy)carbonyl]amino] propanoyl]-1,2-diazinane-3-carboxylate (400 mg, 0.50 mmol, 1 equiv) in DCE (4 mL) was treated with Me₃SnOH (551 mg, 3.03 mmol, 6.0 equiv). The resulting solution was stirred overnight at 80 °C. and then the mixture was concentrated. The solution was diluted with 100 mL of ethyl acetate and then washed with 3 x 100 ml of 0.01 N aqueous KHSO₄ followed by 100 mL of brine. The organics were dried over anhydrous sodium sulfate and filtered to give *(3S)-1-[(2S)-2-*[[(tert-butoxy)carbonyl]amino]-3-[3-[4-(2-hydroxyethoxy)quinolin-6-yl]-5-[[tris(propan-2-yl)silyl]oxy] phenyl]propanoyl]-1,2-diazinane-3-carboxylic acid (370 mg 99% yield) as a yellow solid which was converted to (2*S*)-2-(3-acryloyl-2-oxoimidazolidin-1-yl)-*N*-((6³*S*,4*S*)-2⁵-hydroxy-5,7-dioxo-6¹,6²,6³,6⁴,6⁵,6⁶-hexahydro-8,11-dioxa-1 (6,4)-quinolina-6(1,3)-pyridazina-2(1,3)-benzenacycloundecaphane-4-yl)-3-methylbutanamide using the appropriate intermediates of Example 1 and procedures similar those described in Method A. The synthesis of Compound 2 is a representative example of the use of Method B to make compounds of the invention.

### Example 4 - Syntheis of 1-(2-chloroacetyl)-N-((2S)-1-(((6³S,4S)-1³-cyano-2⁵-hydroxy-10,10-dimethyl-5,7-dioxo-6¹,6²,6³,6⁴,6⁵,6⁶-hexahydro-1¹H-8-aza-1(6,1)-indola-6(1,3)-pyridazina-2(1,3)-benzenacycloundecaphane-4-yl)amino)-3-methyl-1-oxobutan-2-yl)-N-methylazetidine-3-carboxamide (Compound 3)

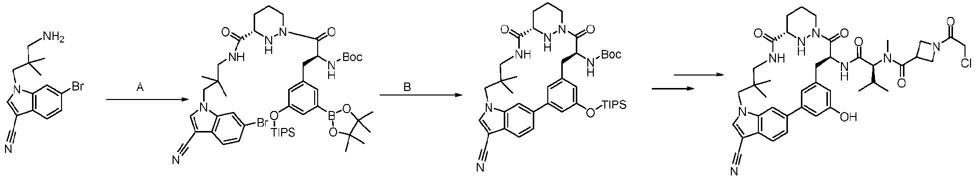

### Step A

HATU (684 mg, 1.8 mmol, 1.2 equiv) was added to the solution of *(S)-1-((S)-2-((tert-*butoxycarbonyl)amino)-3-(3-(4,4,5,5-tetramethyl-1,3,2-dioxaborolan-2-yl)-5-((triisopropylsilyl)oxy)phenyl)propanoyl)hexahydropyridazine-3-carboxylic acid (980 mg, 1.5 mmol, 1.0 equiv), 1-(3-amino-2,2-dimethylpropyl)-6-bromo-1H-indole-3-carbonitrile (549 mg, 1.8 mmol, 1.2 equiv) and DIPEA (580 mg, 4.5 mmol, 3.0 equiv) in DMF (20 mL) at 0 °C. The mixture was stirred at 0 °C to 5 °C for 1 hour and then diluted with ethyl acetate (200 mL) and washed with water (150 mL x 2) and brine (150 mL). The organic phase was collected, dried over sodium sulfate, filtered and concentrated to give a residue that was purified by silica gel chromatography (ethyl acetate/petroleum ether (2:1)) to give *tert*-butyl ((*S*)-1-((*S*)-3-((3-(6-bromo-3-cyano-1*H*-indol-1-yl)-2,2-dimethylpropyl)carbamoyl)tetrahydropyridazin-1(2*H*)-yl)-1-oxo-3-(3-(4,4,5,5-tetramethyl-1,3,2-dioxaborolan-2-yl)-5-((triisopropylsilyl)oxy)phenyl)propan-2-yl)carbamate (530 mg, 36% yield) as an off-white solid. ESI-MS *m*/*z* = 963.3 [M+H]⁺.

### Step B

A mixture of *tert-butyl* ((*S*)-1-((*S*)-3-((3-(6-bromo-3-cyano-1H-indol-1-yl)-2,2-dimethylpropyl)carbamoyl)tetrahydropyridazin-1(2H)-yl)-1-oxo-3-(3-(4,4,5,5-tetramethyl-1,3,2-dioxaborolan-2-yl)-5-((triisopropylsilyl)oxy)phenyl)propan-2-yl)carbamate (530 mg, 0.55 mmol, 1.0 equiv), K₂CO₃ (190 mg, 1.375 mmol, 2.5 equiv) and Pd(dppf)Cl₂CH₂Cl₂ (45 mg, 0.055 mmol, 0.1 equiv) in dioxane (20 mL) and H₂O (4 mL) was stirred at 80 °C for 2 hours. The mixture was diluted with ethyl acetate (100 mL) and then washed with water (50 mL x 2) and brine (80 mL). The organic phase was collected, dried over sodium sulfate, filtered and concentrated to give a residue. The residue was purified by chromatography (ethyl acetate) to give *tert*-butyl ((6³*S*,4*S*)-1³-cyano-10,10-dimethyl-5,7-dioxo-2⁵-((triisopropylsilyl)oxy)-6¹,6²,6³,6⁴,6⁵,6⁵-hexahydro-1¹*H*-8-aza-1(6,1)-indola-6(1,3)-pyridazina-2(1,3)-benzenacycloundecaphane-4-yl)carbamate (220 mg, 53% yield) as a light yellow solid. ESI-MS *m*/*z* = 757.5 [M+H]⁺.

1-(2-Chloroacetyl)-*N*-((2*S*)-1-(((6³*S*,4*S*)-1³-cyano-2⁵-hydroxy-10,10-dimethyl-5,7-dioxo-6¹,6²,6³,6⁴,6⁵,6⁶-hexahydro-1¹*H*-8-aza-1(6,1)-indola-6(1,3)-pyridazina-2(1,3)-benzenacycloundecaphane-4-yl)amino)-3-methyl-1-oxobutan-2-yl)-N-methylazetidine-3-carboxamide was synthesized from tert-butyl ((6³*S*,4*S*)-1³-cyano-10,10-dimethyl-5,7-dioxo-2⁵-((triisopropylsilyl)oxy)-6¹,6²,6³,6⁴,6⁵,6⁶-hexahydro-1¹*H-8-*aza-1(6,1)-indola-6(1,3)-pyridazina-2(1,3)-benzenacycloundecaphane-4-yl)carbamate using the appropriate intermediates of Example 1 and procedures similar those described in Method A. The synthesis of Compound 3 is a representative example of the use of Method C to make compounds of the invention.

### Example 5 - Synthesis of (2S)-2-(3-acryloyl-2-oxoimidazolidin-1-yl)-N-((6³S,4S)-12-ethyl-5,7-dioxo-6¹,6²,6³,6⁴,6⁵,6⁶-hexahydro-1¹H-8-oxa-1(6,1)-benzo[d]imidazola-6(1,3)-pyridazina-2(3,5)-pyridinacycloundecaphane-4-yl)-3-methylbutanamide (Compound 4)

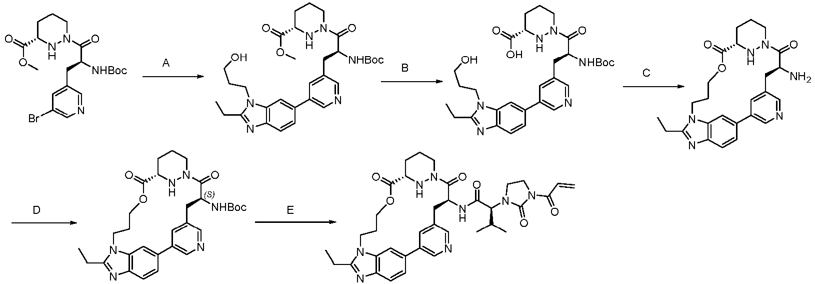

### Step A

A solution of methyl (*S*)-1-((*S*)-3-(5-bromopyridin-3-yl)-2-((*tert-*butoxycarbonyl)amino)propanoyl)hexahydropyridazine-3-carboxylate (1.41 g, 3.0 mmol, 1.0 equiv), Pd(dppf)Cl₂ (245 mg, 0.3 mmol, 0.1 eq), 3-(2-ethyl-6-(4,4,5,5-tetramethyl-1,3,2-dioxaborolan-2-yl)-1H-benzo[d]imidazol-1-yl)propan-1-ol (990 mg, 3.0 mmol, 1.0 eq) and K₂CO₃ (1.24 g, 9.0 mmol, 3.0 eq) in dioxane (30 mL) and water (50 mL) was stirred at 90 °C for 5 hours. The reaction was quenched by addition of ice water (100 mL) and extracted with ethyl acetate (3 x 100 mL). The combined organic layers were dried over anhydrous sodium sulfate and concentrated under reduced pressure to give a crude product, which was purified by silica gel chromatography (dichloromethane to dichloromethane/MeOH=20:1) to give methyl (*S*)-1-((*S*)-2-((*tert*-butoxycarbonyl)amino)-3-(5-(2-ethyl-1-(3-hydroxypropyl)-1H-benzo[d]imidazol-6-yl)pyridin-3-yl)propanoyl)hexahydropyridazine-3-carboxylate (940 mg, 53% yield) as a yellow solid. ESI-MS *m*/*z=* 595.3 [M+H]⁺.

### Step B

To a solution of methyl (*S*)-1-((*S*)-2-((*tert*-butoxycarbonyl)amino)-3-(5-(2-ethyl-1-(3-hydroxypropyl)-1H-benzo[d]imidazol-6-yl)pyridin-3-yl)propanoyl)hexahydropyridazine-3-carboxylate (594 mg, 1.0 mmol, 1.0 equiv) in MeOH (10 mL) was added LiOH (120 mg, 5.0 mmol, 5.0 equiv) in H₂O (2 mL) at 0 °C. The mixture was stirred at 0 °C for 2 hours. The mixture was acidified to about pH 5 with 1 M HCl and extracted with ethyl acetate (100 mL x 2). The organic phase was washed with brine (100 mL x 3), dried over anhydrous sodium sulfate, filtered, and concentrated to give a residue. The crude product was used in the next step directly without further purification (580 mg crude). ESI-MS *m*/*z* = 582.3 [M+H]⁺.

### Step C

A stirred solution of (*S*)-1-((*S*)-3-(3-(((benzyloxy)carbonyl)amino)-5-(1-(3-hydroxypropyl)-1H-benzo[d]imidazol-6-yl)phenyl)-2-((*tert*-butoxycarbonyl)amino)propanoyl)hexahydropyridazine-3-carboxylic acid (580 mg, 1.0 mmol, 1.0 equiv), PySSPy (2.2 g, 10.0 mmol, 10.0 equiv), PPh₃ (2.62 g, 10.0 mmol, 10.0 equiv) in toluene (120 mL) was stirred at 80 °C for 15 hours. The reaction was quenched by the addition of ice water (100 mL) and was extracted with ethyl acetate (3 x 100 mL). The combined organic layer was dried over anhydrous sodium sulfate and concentrated under reduced pressure to give a crude product that was purified by silica gel chromatography (dichloromethane to dichloromethane/MeOH (20:1)) to give tert-butyl ((6³*S*,4*S*)-12-ethyl-5,7-dioxo-6¹,6²,6³,6⁴,6⁵,6⁶-hexahydro-1¹*H*-8-oxa-1(6,1)-benzo[d]imidazola-6(1,3)-pyridazina-2(3,5)-pyridinacycloundecaphane-4-yl)carbamate (260 mg, 46% yield) as a yellow solid. ESI-MS *m*/*z=* 564.3 [M+H]⁺.

### Step D

To a solution of *tert-butyl* ((6³*S*,4*S*)-12-ethyl-5,7-dioxo-6¹,6²,6³,6⁴,6⁵,6⁶-hexahydro-1¹*H*-8-oxa-1(6,1)-benzo[d]imidazola-6(1,3)-pyridazina-2(3,5)-pyridinacycloundecaphane-4-yl)carbamate (56 mg, 0.1 mmol, 1.0 eq) in dichloromethane (30 mL) was added TFA (1 mL) dropwise at 0 °C. The mixture was stirred at 0 °C for 2 hours. The mixture was concentrated to give the crude product (6³*S*,4*S*)-4-amino-12-ethyl-6¹,6²,6³,6⁴,6⁵,6⁶-hexahydro-1¹*H*-8-oxa-1(6,1)-benzo[d]imidazola-6(1 ,3)-pyridazina-2(3,5)-pyridinacycloundecaphane-5,7-dione (46.3 mg) as a yellow oil that was used in the next step without further purification. ESI-MS *m*/*z* = 463.3 [M+H]⁺.

### Step E

A solution of (6³*S*,4*S*)-4-amino-12-ethyl-6¹,6²,6³,6⁴,6⁵,6⁶-hexahydro-1¹*H*-8-oxa-1(6,1)-benzo[d]imidazola-6(1,3)-pyridazina-2(3,5)-pyridinacycloundecaphane-5,7-dione (46 mg, 0.1 mmol, 1.0 eq) , (S)-2-(3-acryloyl-2-oxoimidazolidin-1-yl)-3-methylbutanoic acid (24.1 g, 0.1 mmol, 1.0 eq), HATU (41.8 mg, 0.11 mmol, 1.1 eq), and DIEA (64.5 mg, 0.5 mmol, 5.0 eq) in DMF (5 mL) was stirred at 0 °C for 2 hours. The mixture was poured into water and extracted with ethyl acetate (20 mL) and washed with brine (20 mL x 2). The organic layer was dried over anhydrous sodium sulfate and concentrated to give a residue that was purified by prep-HPLC (formic acid in MeCN/Water) to afford (2S)-2-(3-acryloyl-2-oxoimidazolidin-1-yl)-N-((6³*S*,4*S*)-12-ethyl-5,7-dioxo-6¹,6²,6³,6⁴,6⁵,6⁶-hexahydro-1¹*H*-8-oxa-1(6,1)-benzo[d]imidazola-6(1,3)-pyridazina-2(3,5)-pyridinacycloundecaphane-4-yl)-3-methylbutanamide (6.2 mg, 9.0 % yield) as a white solid. The synthesis of Compound 4 is a representative example of the use of Method D to make compounds of the invention.

### Example 6 - Synthesis of 1-(2-chloroacetyl)-N-((2S)-1-(((6³S,4S,10S)-1³-(2-cyanophenyl)-2⁵-hydroxy-10-methyl-5,7-dioxo-6¹,6²,6³,6⁴,6⁵,6⁶-hexahydro-1¹H-8-oxa-1(6,1)-indola-6(1,3)-pyridazina-2(1,3)-benzenacycloundecaphane-4-yl)amino)-3-methyl-1-oxobutan-2-yl)-N-methylazetidine-3-carboxamide (Compound 5)

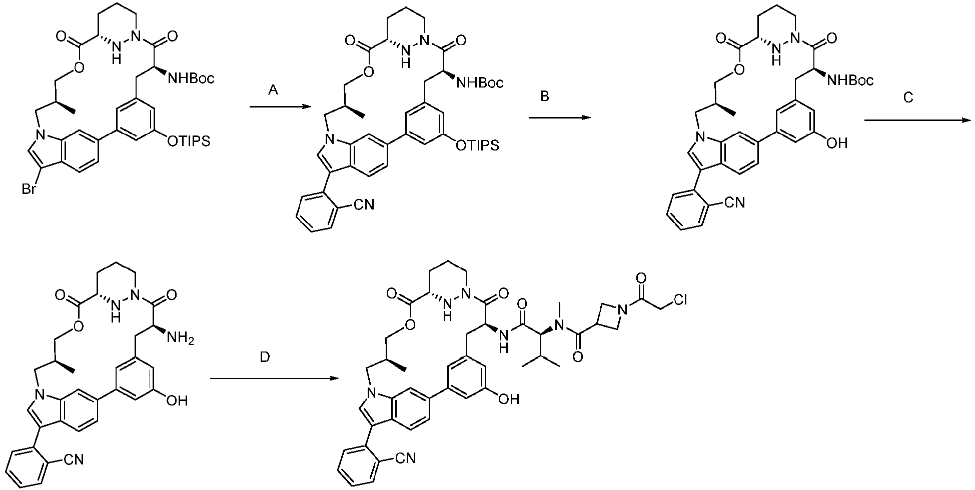

### Step A

To a stirred solution of tert-butyl ((6³*S*,4*S*,10*S*)-1³-bromo-10-methyl-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (250 mg, 0.31 mmol, 1.0 equiv) and 2-(4,4,5,5-tetramethyl-1,3,2-dioxaborolan-2-yl)benzonitrile (215.34 mg, 0.94 mmol, 3.0 equiv) in dioxane (2.5 mL) and H₂O (0.5 mL) was added Pd(DTBPf)Cl₂ (41 mg, 0.063 mmol, 0.2 equiv) and K₂CO₃ (108 mg, 0.783 mmol, 2.5 equiv) portionwise. After stirring for 4 hours at 80 °C, the solution was concentrated and the residue was purified by silica gel chromatography, eluting with petroleum ether/ethyl acetate (5:1) to afford tert-butyl *(*(6³*S*,4*S*,10*S*)-1³-(2-cyanophenyl)-10-methyl-5,7-dioxo-2⁵-((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (200 mg, 77% yield) as a white solid. ESI-MS *m*/*z* = 820.4 [M+H]⁺.

### Step B

To a solution of *tert-butyl* ((6³*S*,4*S*,10*S*)-1³-(2-cyanophenyl)-10-methyl-5,7-dioxo-2⁵. ((triisopropylsilyl)oxy)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (200 mg, 0.244 mmol, 1.0 equiv) in THF (2.0 mL) at 0 °C was added TBAF (64 mg, 0.244 mmol, 1.00 equiv, 1 M in THF). After stirring for 1 hour, the solution was concentrated and the residue was purified by silica gel column chromatography, eluting with petroleum ether/ethyl acetate (4:1) to afford *tert-butyl* ((6³*S*,4*S*,10*S*)-1³-(2-cyanophenyl)-2⁵-hydroxy-10-methyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (190 mg, 94% yield) as an off-white solid. ESI-MS *m*/*z* =664.3 [M+H]⁺.

### Step C

To a stirred solution of tert-butyl ((6³*S*,4*S*,10*S*)-1³-(2-cyanophenyl)-2⁵-hydroxy-10-methyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate (190 mg, 0.29 mmol, 1.0 equiv) in dichloromethane (2.0 mL) at 0 °C was added TFA (1.00 mL). After stirring for 3 hours, the solution was concentrated and the residue was purified by silica gel chromatography, eluting with petroleum ether/ethyl acetate (4:1) afford 2-((6³*S*,4*S*,10*S*)-4-amino-2⁵-hydroxy-10-methyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-1³-yl)benzonitrile (150 mg, 84%) as a white solid. ESI-MS *m*/*z* =564.2 [M+H]⁺.

### Step D

To a stirred solution of 2-((6³*S*,4*S*,10*S*)-4-amino-2⁵-hydroxy-10-methyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-1³-yl)benzonitrile (60 mg, 0.11 mmol, 1.0 equiv) and (2S)-2-[1-[1-(2-chloroacetyl)azetidin-3-yl]-N-methylformamido]-3-methylbutanoic acid (34 mg, 0.12 mmol, 1.1 equiv) in DMF (1 mL) was added DIEA (27 mg, 0.213 mmol, 2 equiv) and COMU (68 mg, 0.160 mmol, 1.5 equiv) portionwise at 0 °C. After stirring for 2 hours, the solution was concentrated and the resulting residue was purified by reverse phase chromatography (10 to 50% MeCN in water) to afford 1-(2-chloroacetyl)-N-((2S)-1-(((6³*S*,4S,10*S*)-1³-(2-cyanophenyl)-2⁵-hydroxy-10-methyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)amino)-3-methyl-1-oxobutan-2-yl)-N-methylazetidine-3-carboxamide (9.5 mg, 11% yield) as a white solid. The synthesis of Compound 5 is a representative example of the use of Method E to make compounds of the invention.

### Example 7 - Synthesis of (2S)-2-(3-acryloyl-2-oxoimidazolidin-1-yl)-N-((6³S,4S)-2⁶-hydroxy-5,7-dioxo-1³-(tetrahydro-2H-pyran-4-yl)-6¹,6²,6³,6⁴,6⁵,6⁶-hexahydro-1¹H-8-oxa-1(6,1)-indola-6(1,3)-pyridazina-2(1,3)-benzenacycloundecaphane-4-yl)-3-methylbutanamide (Compound 229)

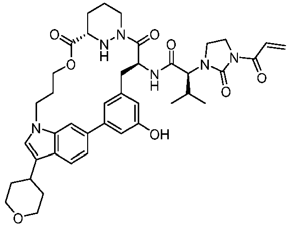

(2*S*)-2-(3-Acryloyl-2-oxoimidazolidin-1-yl)-N-((6³*S*,4*S*)-2⁵-hydroxy-5,7-dioxo-1³-(tetrahydro-2*H-*pyran-4-yl)-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)-3-methylbutanamide was synthesized from methyl (*S*)-1-((*S*)-3-(3-(1-(3-acetoxypropyl)-3-(tetrahydro-2H-pyran-4-yl)-1H-indol-6-yl)-5-((triisopropylsilyl)oxy)phenyl)-2-((tert-butoxycarbonyl)amino)propanoyl)hexahydropyridazine-3-carboxylate using procedures similar to those described in Method B.

### Example 8 - Synthesis of N-((2S)-1-(((6³S,4S)-2⁵-amino-1³-cyano-10,10-dimethyl-5,7-dioxo-6¹,6²,6³,6⁴,6⁵,6⁶-hexahydro-1¹H-8-oxa-1(6,1)-indola-6(1,3)-pyridazina-2(1,3)-benzenacycloundecaphane-4-yl)amino)-3-methyl-1-oxobutan-2-yl)-1-(2-chloroacetyl)-N-methylazetidine-3-carboxamide (Compound 230)

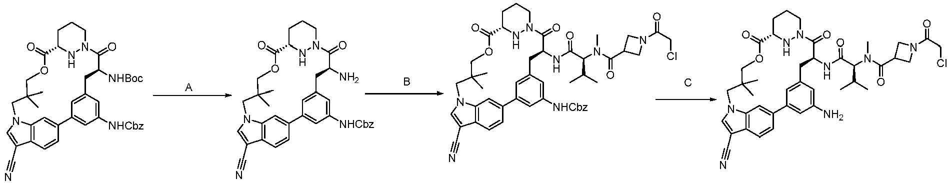

### Step A

Benzyl *tert-butyl* ((6³*S*,4*S*)-1³-cyano-10,10-dimethyl-5,7-dioxo-6¹,6²,6³,6⁴,6⁵,6⁶-hexahydro-1¹*H*-8-oxa-1 (6,1)-indola-6(1 ,3)-pyridazina-2(1 ,3)-benzenacycloundecaphane-25,4-diyl)dicarbamate was synthesized from 1-(3-hydroxy-2,2-dimethylpropyl)-6-(4,4,5,5-tetramethyl-1,3,2-dioxaborolan-2-yl)-1H-indole-3-carbonitrile and methyl 1-(3-(3-(((benzyloxy)carbonyl)amino)-5-bromophenyl)-2-((*tert-*butoxycarbonyl)amino)propanoyl)hexahydropyridazine-3-carboxylate using the appropriate intermediates of Example 1 and procedures similar those described in Method A.

TFA (0.3 mL) was added to a solution of benzyl *tert-butyl* ((6³*S*,4*S*)-1³-cyano-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-2⁵,4-diyl)dicarbamate (100 mg, 0.14 mmol, 1.0 equiv) in dichloromethane (1.5 mL) at 0 °C and then stirred for 2 hours at 0 °C. The mixture was concentrated to give crude ((6³*S*,4*S*)-4-amino-1³-cyano-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-2⁵-yl)carbamate and the residue was used to next step without further purification. ESI-MS *m*/*z* = 635.3 [M+H]⁺

### Step B

HATU (53 mg, 0.14 mmol, 1.0 equiv) was added to a solution of ((6³*S*,4*S*)-4-amino-1³-cyano-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-2⁵-yl)carbamate (89 mg, 0.14 mmol, 1.0 equiv), N-(1-(2-chloroacetyl)azetidine-3-carbonyl)-N-methyl-L-valine (41 mg, 0.14 mmol, 1.0 equiv) and DIPEA (54 mg, 0.42 mmol, 3.0 equiv) in DMF (2 mL) at 0 °C. The mixture was maintained at that temperature for 1 hour. The mixture was diluted with ethyl acetate (20 mL) and then washed with water (15 mL x 2) and brine (10 mL). The organic phase was collected, dried over sodium sulfate, filtered and concentrated to give a residue. The residue was purified by prep-TLC (dichloromethane /MeOH = 20/1) to give benzyl ((6³*S*,4*S*)-4-((*S*)-2-(1-(2-chloroacetyl)-N-methylazetidine-3-carboxamido)-3-methylbutanamido)-1³-cyano-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-2⁵-yl)carbamate (85.0 mg, 67% yield) as an off-white solid. ESI-MS *m*/*z* = 907.1 [M+H]⁺

### Step C

Benzyl ((6³*S*,4*S*)-4-((*S*)-2-(1-(2-chloroacetyl)-N-methylazetidine-3-carboxamido)-3-methylbutanamido)-1³-cyano-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-2⁵-yl)carbamate (70 mg, 0.077 mmol, 1.0 equiv) was added to a solution of BCl₃ (2 mL, 1 M in dichloromethane) at 0 °C. The mixture was then stirred at room temperature for 4 hours. MeOH (2 mL) was added and the reaction solution was diluted with with ethyl acetate (20 mL) and washed with water (15 mL x 2) and brine (10 mL). The organic phase was collected, dried over sodium sulfate, filtered and concentrated to give a crude residue. The residue was purified by prep-TLC (EA/MeOH = 8/1) to give N-((2*S*)-1-(((6³*S*,4*S*)-2⁵-amino-1³-cyano-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)amino)-3-methyl-1-oxobutan-2-yl)-1-(2-chloroacetyl)-N-methylazetidine-3-carboxamide (13.2 mg, 10% yield) as an off-white solid.

### Example 9 - Synthesis of 1-(2-chloroacetyl)-N-((2S)-1-(((6³S,4S)-1³-cyano-2⁵-methoxy-10,10-dimethyl-5,7-dioxo-6¹,6²,6³,6⁴,6⁵,6⁶-hexahydro-1¹H-8-oxa-1(6,1)-indola-6(1,3)-pyridazina-2(1,3)-benzenacycloundecaphane-4-yl)amino)-3-methyl-1-oxobutan-2-yl)-N-methylazetidine-3-carboxamide (Compound 231)

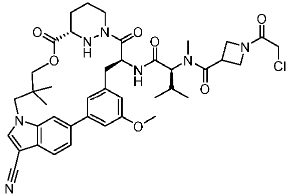

The title compound was synthesized from tert-butyl ((6³*S*,4*S*)-1³-cyano-2⁵-methoxy-10,10-dimethyl-5,7-dioxo-6¹,6²,6³,6⁴,6⁵,6⁶-hexahydro-1¹*H*-8-oxa-1(6,1)-indola-6(1,3)-pyridazina-2(1,3)-benzenacycloundecaphane-4-yl)carbamate using the appropriate intermediates of Example 1 and procedures similar those described in Method A.

### Example 10 - 1-acryloyl-N-((2S)-1-(((6³S,4S)-2⁵-hydroxy-10,10-dimethyl-1³-(1-methylpiperidin-4-yl)-5,7-dioxo-6¹,6²,6³,6⁴,6⁵,6⁶-hexahydro-1¹H-8-oxa-1(6,1)-indazola-6(1,3)-pyridazina-2(1,3)-benzenacycloundecaphane-4-yl)amino)-3-methyl-1-oxobutan-2-yl)-N-methylazetidine-3-carboxamide (Compound 232)

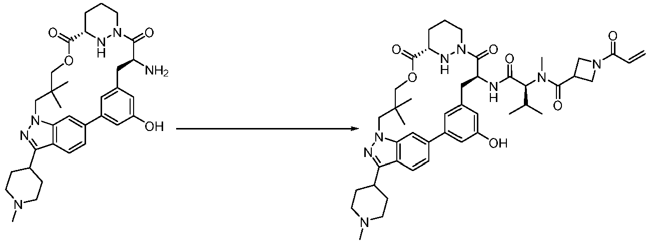

(6³*S*,4*S*)-4-amino-2⁵-hydroxy-10,10-dimethyl-1³-(1-methylpiperidin-4-yl)-6¹,6²,6³,6⁴,6⁵,6⁶-hexahydro-1¹*H*-8-oxa-1(6,1)-indazola-6(1 ,3)-pyridazina-2(1 ,3)-benzenacycloundecaphane-5,7-dione was synthesized from tert-butyl ((6³*S*,4*S*)-2⁵-hydroxy-10,10-dimethyl-1³-(1-methylpiperidin-4-yl)-5,7-dioxo-6¹,6²,6³,6⁴,6⁵,6⁶-hexahydro-1¹*H*-8-oxa-1(6,1)-indazola-6(1,3)-pyridazina-2(1,3)-benzenacycloundecaphane-4-yl)carbamate using the appropriate intermediates of Example 1 and procedures similar those described in Method A.

The title compound was synthesized from (6³*S*,4*S*)-4-amino-2⁵-hydroxy-10,10-dimethyl-1³-(1-methylpiperidin-4-yl)-6¹,6²,6³,6⁴,6⁵,6⁶-hexahydro-1¹*H*-8-oxa-1(6,1)-indazola-6(1,3)-pyridazina-2(1,3)-benzenacycloundecaphane-5,7-dione according to a procedure similar to the one described in Method A.

### Example 11 - 1-(2-chloroacetyl)-N-((2S)-1-(((6³S,4S)-1³-ethynyl-2⁵-hydroxy-10,10-dimethyl-5,7-dioxo-6¹,6²,6³,6⁴,6⁵,6⁶-hexahydro-1¹H-8-oxa-1(6,1)-indazola-6(1,3)-pyridazina-2(1,3)-benzenacycloundecaphane-4-yl)amino)-3-methyl-1-oxobutan-2-yl)-N-methylazetidine-3-carboxamide (Compound 233)

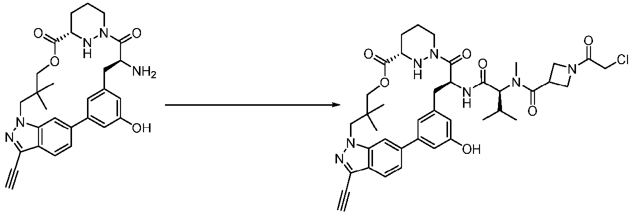

(6³*S*,4*S*)-4-amino-1³-ethynyl-2⁵-hydroxy-10,10-dimethyl-6¹,6²,6³,6⁴,6⁵,6⁶-hexahydro-1¹*H*-8-oxa-1(6,1)-indazola-6(1,3)-pyridazina-2(1,3)-benzenacycloundecaphane-5,7-dione was synthesized from *tert-*butyl ((6³*S*,4*S*)-1³-ethynyl-2⁵-hydroxy-10,10-dimethyl-5,7-dioxo-6¹,6²,6³,6⁴,6⁵,6⁶-hexahydro-1¹*H*-8-oxa-1(6,1)-indazola-6(1,3)-pyridazina-2(1,3)-benzenacycloundecaphane-4-yl)carbamate using a procedure similar to the one described in Method A.

The title compound was synthesized from (6³*S*,4*S*)-4-amino-1³-ethynyl-2⁵-hydroxy-10,10-dimethyl-6¹,6²,6³,6⁴,6⁵,6⁶-hexahydro-1¹*H*-8-oxa-1(6,1)-indazola-6(1,3)-pyridazina-2(1,3)-benzenacycloundecaphane-5,7-dione according to a procedure similar to the one described in Method A.

### Example 12 - Synthesis of 1-(2-chloroacetyl)-N-((2S)-1-(((6³S,4S)-2⁶-hydroxy-5,7-dioxo-6¹,6²,6³,6⁴,6⁵,6⁶-hexahydro-1¹H-8-oxa-1(6,1)-benzo[d]imidazola-6(1,3)-pyridazina-2(1,3)-benzenacycloundecaphane-4-yl)amino)-3-methyl-1-oxobutan-2-yl)-N-methylazetidine-3-carboxamide (Compound 234)

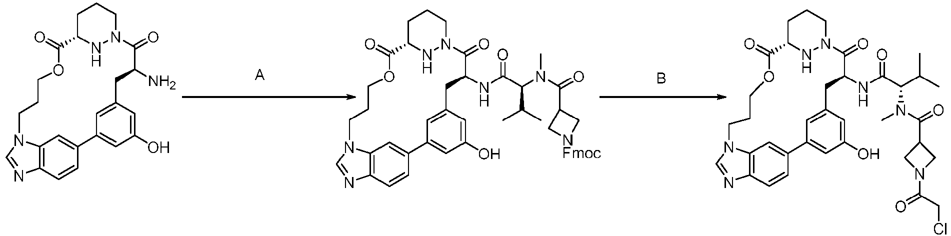

### Step A

(6³*S*,4*S*)-4-amino-2⁵-hydroxy-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-5,7-dione was synthesized from 3-(6-bromo-1H-benzo[d]imidazol-1-yl)propan-1-ol using the appropriate intermediates of Example 1 and procedures similar those described in Method A.

To a stirred solution of (6³*S*,4*S*)-4-amino-2⁵-hydroxy-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-5,7-dione (45 mg, 0.10 mmol, 1.0 equiv), 1-(((9H-fluoren-9-yl)methoxy)carbonyl)azetidine-3-carboxylic acid (43.7 mg, 0.10 mmol, 1.0 equiv) and DIEA (65 mg, 0.50 mmol, 5 equiv) in DMF (2 mL) was added COMU (64 mg, 0.15 mmol, 1.5 equiv) at 0 °C. The resulting mixture was stirred for 2h at 0 °C. After aqueous workup the residue was purified by Prep-TLC (petroleum ether/ethyl acetate (1:1)) to afford (9H-fluoren-9-yl)methyl 3-(((2*S*)-1-(((6³*S*,4*S*)-2⁵-hydroxy-5,7-dioxo-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)amino)-3-methyl-1-oxobutan-2-yl)(methyl)carbamoyl)azetidine-1-carboxylate (60 mg, 69% yield) as a yellow oil. ESI-MS *m*/*z* = 868.3 [M+H]⁺

### Step B

To a stirred solution of (9H-fluoren-9-yl)methyl 3-(((2*S*)-1-(((6³*S*,4*S*)-2⁵-hydroxy-5,7-dioxo-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)amino)-3-methyl-1-oxobutan-2-yl)(methyl)carbamoyl)azetidine-1-carboxylate (35 mg, 0.04 mmol, 1 equiv) in MeCN (0.3 mL) was added TEA (0.3 mL) in portions at 0 °C. The resulting mixture was stirred for 2 hours at room temperature and then HATU (18.4 mg, 0.05 mmol, 1.20 equiv) and 2-chloroacetic acid (4.6 mg, 0.05 mmol, 1.21 equiv) was added in portions at 0 °C. The resulting mixture was stirred for 2 hours at 0 °C. The solvent was removed *in vacuo* and the crude product was purified by Prep-HPLC with the following conditions (5% to 39% MeCN in water with 0.1% FA in 10.5 min)) to afford 1-(2-chloroacetyl)-N-((2S)-1-(((6³*S*,4*S*)-2⁵-hydroxy-5,7-dioxo-6¹,6²,6³,6⁴,6⁵,6⁶-hexahydro-1¹*H*-8-oxa-1(6,1)-benzo[d]imidazola-6(1,3)-pyridazina-2(1,3)-benzenacycloundecaphane-4-yl)amino)-3-methyl-1-oxobutan-2-yl)-N-methylazetidine-3-carboxamide (3.8 mg, 13% yield) as a white solid.

### Example 13 - Determination of CYPA-Compound Binary Complex Formation by SPR

In order to determine the affinity of compounds of the invention for cyclophilin A (CYPA), we employed surface plasmon resonance biniding analysis using the following reagents, instruments and protocol as supplied by the instrument manufacturer.

### Reagents and Instrument

1. Instrument: Biacore S200 (GE Healthcare Life Sciences)
2. Running buffer: 1xHBS-P+ (1X HBS supplemented with 0.05% Tween20), pH7.4, and 2% DMSO.
3. Ligand: CypA-Avi
4. Analyte: Serial dilution of WDB compounds from 50 µM to 0 µM, 2-fold dilution, 10 points (10 mM stock concentration).
5. Sensor kit: Biotin CAPture Kit (BR28-9202-34, GE Healthcare Life Sciences)
6. Regeneration buffer: 1 volume of 1 M NaOH and 3 volumes of 8 M guanidine hydrochloride (supplied with CAPture Kit, BR28-9202-34, GE Healthcare Life Sciences)

### Experimental Procedure

1. Capture of CAPreagent: 2 µl/min for 60 seconds.
2. Immobilization of ligand CypA: Use Fc1 as the reference cell; immobilize CypA at 4 µg/ml using 5 µl/min flowrate on Fc2 (60s), Fc3 (80s) and Fc4 (80s), respectively.
3. Binding of analytes: Inject WDB compound dilutions sequentially at 50 µl/min for association of 60 seconds. And then let it dissociates in buffer at 50 µl/min for 60 seconds.
4. Regeneration: 2 injections of regeneration buffer at 30 µl/min for 60 seconds to regenerate the chip surface.
5. Solvent correction: Run solvent correction at beginning and the end of the run. If run more than 6 compounds, add additional solvent correction every 6 compounds.
6. Data fitting with built-in Biacore Evaluation Software: Steady-state fitting.

### Example 14 - Determination of KRAS-BRAF Complex Disruption through Competition with CYPA-Compound by TR-FRET

In this example, TR-FRET was used to measure the compound-facilitated disruption of a KRAS-BRAF complex. A mixture of tagless Cyclophilin A, His6-KRAS G12C-GMP-PNP, and GST-BRAF RAS binding domain was added to a 384-well assay plate containing compounds of the invention and incubated for 3 hours. A mixture of Anti-His Eu-W1024 and anti-GST allophycocyanin was then added and the reaction was incubated for an additional 1.5 hours. TR-FRET signal was read on an EnVision microplate reader (Perkin Elmer, Ex 320 nm, Em 665/615 nm). Compounds that facilitate disruption of a KRAS_BRAF complex were identified as those eliciting a decrease in the TR-FRET ratio relative to DMSO control wells. The results are showin in Table 4, below. To determine the presenter-dependence of the compound-mediated KRAS-BRAF complex disruption, Cyclophilin A was left out of the initial incubation (see Table 5). To determine G12C specificity of the compound-mediated KRAS-BRAF complex disruption, wild-type KRAS was used instead of G12C KRAS (see Table 5).

### Reagents and Instrument

▪ Tagless CYPA; 519 µM in PBS buffer, pH 7.4
▪ GST BRAF; 110 µM in PBS buffer, pH 7.4
▪ His6-KRAS_{G12C-GMP-PNP}; 50 µM in PBS buffer, pH 7.4
▪ His6-KRAS_{WT-GMP-PNP}; 40 µM in PBS buffer, pH 7.4
▪ Anti-His Eu-W1024 (LANCE^{®} Eu-W1024; Perkin Elmer)
▪ Anti-GST allophycocyanin (Anti-GST IgG conjugated to SureLight^{®}-Allophycocyanin; Perkin Elmer; Product Number AD0059G)
▪ Test compounds, 10 mM in 100% DMSO
▪ EnVision (Perkin Elmer)
▪ Combi MultiDrop liquid dispenser with 8-channel small volume cassette
▪ 384-well ProxiPlate (black)

### Experimental Protocol

1. Use Mosquito to dispense 100 nL/well of compounds (varying concentration in DMSO-*d₆*) into 384-well black ProxiPlate to make assay-ready-plate (ARP).
2. Make assay buffer containing 25 mM Hepes pH 7.3, 100 mM NaCl, 5 mM MgCl₂, 0.05% BSA, and 0.002% Tween-20.
3. Make PRE-MIX A: delivers final concentrations of 50 nM of His6-KRas G12C-GTP (1-169) and 500 nM of tagless CypA (1-165) in assay buffer.
   a. For presenter dependence experiments, omit addition of tagless CypA
   b. For G12C/wt specificity experiments, substitute His6-KRas WT-GTP (1-169) for His6-KRas G12C-GTP (1-169)
4. Use MultiDrop Combi to dispense PRE-MIX A into ARP, 7 µl/well. Incubate 3 hr at room temperature.
5. Make PRE-MIX B: delivers final concentrations of 10 nM of anti-His Eu-W1024 and 50 nM of anti-GST APC.
6. Use MultiDrop Combi to dispense PRE-MIX B into ARP, 3 µl/well. Shake briefly on Combi and incubate 1.5 hr at room temperature.
7. Read on EnVision (Ex: 320 nm; Em1: 615 nm; Em2: 665 nm).
8. Data is processed using Dotmatics. Curves are fit using a 4-parameter non-linear fit to determine the EC50 value for formation of the ternary complex.

### Example 15 - Determination of pERK inhibition in H358 cells

H358 cells (5500 cells) derived from a human lung cancer were plated in a 96 well plate in media (100 uL, RPMI with 10% FBS). After 24 hours, cells were treated for 4 hours with compound or DMSO. Cells were washed twice with room temperature TBS (200 uL) and fixed for 20 minutes with 4% paraformaldehyde diluted with TBS (150uL). Cells were washed four times for five minutes with 0.1% TritonX/TBS (150 uL) to permeabilize the membrane. Cells were incubated with TBS blocking buffer (100 uL) at room temperature for 60 minutes. Primary antibody (Phospho-p44/42 MAPK (Erk1/2) (Thr202/Tyr204) (D13.14.4E) XP^{®} Rabbit mAb #4370, Cell Signaling Technology; 1:200) was added, and the cells were incubated overnight at 4C. The cells were washed four times for five minutes with 0.1% Tween 20/TBS (150 uL). Secondary antibody (IRDye^{®} 800CW Goat-Anti-Rabbit IgG, Li-Cor Biosciences; 1:1000) and DRAQ5^{™} (Invitrogen; 1:2000) was added, and the cells were incubated for 1 hour at room temperature. The cells were washed four times for five minutes with 0.1% Tween 20/TBS (150 uL) and scanned using the LICOR (700 and 800 nm).

The SPR CypA K_{D}, biochemical BRAF-KRAS G12C-GTP disruption assay EC₅₀, and cellular inhibition of pERK IC₅₀ of compounds described herein are shown in Table 4. For CYPA Binding Affinity: A, K_{D} ≤ 5.0 µM; B, 5.0 µM < K_{D} ≤ 15 µM; C, K_{D} > 15 µM. For BRAF-GTP-KRAS-G12C Disruption: A, EC₅₀ ≤ 0.5 µM; B, 0.5 µM < EC₅₀ ≤ 5.0 µM; C, EC₅₀ > 5.0 µM. For Cellular pERK Inhibition: A, IC₅₀≤ 1.0 µM; B, 1.0 µM < IC₅₀ ≤ 10 µM; C, IC₅₀ > 10.0 µM. Blanks in the table represent that the compound was not tested in the indicated assay.

**Table 4.**

| # | CYPA Binding Affinity | BRAF-GTP-KRAS-G12C Disruption | Cellular pERK Inhibition |
|---|---|---|---|
| 1 | B | A | C |
| 2 | A | C | |
| 3 | A | A | C |
| 4 | B | C | |
| 5 | A | A | A |
| 6 | A | B | |
| 7 | A | A | |
| 8 | A | B | |
| 9 | C | A | A |
| 10 | B | B | |
| 11 | A | A | C |
| 12 | A | C | |
| 13 | C | C | C |
| 14 | C | C | C |
| 15 | B | A | A |
| 16 | B | B | C |
| 17 | A | B | C |
| 18 | C | B | C |
| 19 | A | A | B |
| 20 | C | C | C |
| 21 | C | B | C |
| 22 | B | A | C |
| 23 | C | A | C |
| 24 | B | A | A |
| 25 | B | A | C |
| 26 | B | B | C |
| 27 | B | A | B |
| 28 | A | B | C |
| 29 | A | C | C |
| 30 | A | B | B |
| 31 | C | B | C |
| 32 | A | C | C |
| 33 | B | A | A |
| 34 | B | A | B |
| 35 | B | C | C |
| 36 | B | A | B |
| 37 | B | A | B |
| 38 | B | A | B |
| 39 | B | A | C |
| 40 | B | A | B |
| 41 | A | A | B |
| 42 | B | B | B |
| 43 | B | A | B |
| 44 | B | A | B |
| 45 | C | C | C |
| 46 | B | A | A |
| 47 | B | A | B |
| 48 | B | A | B |
| 49 | C | A | B |
| 50 | B | A | B |
| 51 | | A | B |
| 52 | B | A | A |
| 53 | C | B | C |
| 54 | C | B | C |
| 55 | C | B | C |
| 56 | C | A | C |
| 57 | C | A | B |
| 58 | C | B | C |
| 59 | C | C | C |
| 60 | B | C | C |
| 61 | B | B | B |
| 62 | B | A | A |
| 63 | B | A | A |
| 64 | B | A | B |
| 65 | C | A | B |
| 66 | C | A | C |
| 67 | C | A | B |
| 68 | A | A | B |
| 69 | A | A | B |
| 70 | | A | C |
| 71 | C | A | B |
| 72 | A | A | B |
| 73 | A | A | B |
| 74 | A | A | B |
| 75 | A | A | A |
| 76 | A | A | A |
| 77 | A | A | A |
| 78 | B | A | B |
| 79 | C | A | A |
| 80 | C | A | A |
| 81 | B | A | A |
| 82 | B | A | B |
| 83 | B | A | B |
| 84 | | A | C |
| 85 | A | A | A |
| 86 | C | A | A |
| 87 | B | A | B |
| 88 | C | B | |
| 89 | B | B | B |
| 90 | B | B | A |
| 91 | B | B | B |
| 92 | A | B | C |
| 93 | A | C | |
| 94 | A | B | |
| 95 | A | B | |
| 96 | A | B | |
| 97 | C | A | C |
| 98 | B | A | |
| 99 | C | A | A |
| 100 | C | A | A |
| 101 | A | B | |
| 102 | B | A | C |
| 103 | C | A | B |
| 104 | C | A | A |
| 105 | B | B | |
| 106 | B | A | B |
| 107 | A | B | C |
| 108 | B | A | C |
| 109 | C | A | C |
| 110 | A | A | B |
| 111 | B | A | C |
| 112 | C | C | C |
| 113 | A | A | B |
| 114 | A | A | B |
| 115 | A | A | C |
| 116 | B | A | B |
| 117 | B | A | A |
| 118 | C | A | B |
| 119 | C | A | B |
| 120 | C | A | C |
| 121 | A | A | B |
| 122 | B | A | A |
| 123 | C | A | A |
| 124 | C | A | A |
| 125 | C | A | B |
| 126 | B | A | C |
| 127 | B | A | B |
| 128 | B | A | A |
| 129 | B | A | C |
| 130 | B | A | C |
| 131 | B | B | |
| 132 | A | C | |
| 133 | A | C | |
| 134 | A | B | C |
| 135 | B | C | |
| 136 | B | B | |
| 137 | A | C | |
| 138 | | C | |
| 139 | B | A | C |
| 140 | C | B | |
| 141 | A | B | |
| 142 | A | C | |
| 143 | B | A | C |
| 144 | B | C | |
| 145 | B | B | |
| 146 | B | B | |
| 147 | A | A | C |
| 148 | C | B | |
| 149 | A | B | |
| 150 | B | B | |
| 151 | B | A | C |
| 152 | B | A | B |
| 153 | B | C | |
| 154 | B | B | |
| 155 | A | B | C |
| 156 | C | A | C |
| 157 | A | C | C |
| 158 | A | C | C |
| 159 | C | C | C |
| 160 | B | B | C |
| 161 | C | C | C |
| 162 | C | C | C |
| 163 | B | C | C |
| | | | |
| 164 | A | C | |
| 165 | A | C | |
| 166 | A | A | |
| 167 | A | B | |
| 168 | A | A | |
| 169 | A | B | |
| 170 | B | B | |
| 171 | B | B | |
| 172 | B | B | C |
| 173 | A | C | |
| 174 | A | C | |
| 175 | B | A | C |
| 176 | B | A | B |
| 177 | B | A | B |
| 178 | B | B | C |
| 179 | A | A | B |
| 180 | C | A | B |
| 181 | A | | |
| 182 | A | C | |
| 183 | B | B | B |
| 184 | A | B | C |
| 185 | A | B | C |
| 186 | B | C | C |
| 187 | B | C | C |
| 188 | A | C | C |
| 189 | A | A | A |
| 190 | A | B | C |
| 191 | B | A | A |
| 192 | B | A | B |
| 193 | B | B | C |
| 194 | A | B | C |
| 195 | B | A | C |
| 196 | C | A | B |
| 197 | A | A | B |
| 198 | A | A | C |
| 199 | B | B | C |
| 200 | C | A | C |
| 201 | C | A | A |
| 202 | A | A | B |
| 203 | C | C | C |
| 204 | B | C | B |
| 205 | C | C | C |
| 206 | A | C | C |
| 207 | C | A | A |
| 208 | B | A | B |
| 209 | B | A | A |
| 210 | C | A | A |
| 211 | B | A | A |
| 212 | C | A | B |
| 213 | C | A | B |
| 214 | C | B | |
| 215 | B | A | B |
| 216 | B | A | C |
| 217 | B | A | A |
| 218 | A | A | A |
| | | | |
| 219 | A | A | B |
| 220 | B | A | A |
| 221 | A | A | A |
| 222 | A | A | A |
| 223 | A | A | B |
| 224 | A | A | B |
| 225 | A | A | B |
| 226 | A | A | B |
| 227 | B | | C |
| 228 | A | | B |
| 229 | | B | C |
| 230 | B | B | C |
| 231 | B | C | C |
| 232 | A | B | C |
| 233 | C | A | |
| 234 | A | B | |
| 235 | | C | C |
| 236 | B | A | B |
| 237 | A | B | C |
| 238 | C | A | C |
| 239 | A | A | B |
| 240 | B | A | B |
| 241 | C | A | B |
| 242 | C | A | B |
| 243 | A | B | C |
| 244 | C | B | C |
| 245 | B | B | C |
| 246 | B | A | B |
| 247 | C | A | C |
| 248 | A | B | B |
| 249 | A | B | C |
| 250 | C | A | A |
| 251 | A | B | C |
| 252 | A | B | B |
| 253 | C | A | B |
| 254 | C | C | C |
| 255 | B | B | C |
| 256 | A | A | A |
| 257 | B | A | B |
| 258 | A | C | C |
| 259 | B | B | C |
| 260 | A | C | B |
| 261 | C | B | B |
| 262 | A | A | A |
| 263 | B | B | B |
| 264 | C | A | A |
| 265 | A | A | A |
| 266 | A | A | A |
| 267 | C | A | B |
| 268 | B | A | A |
| 269 | C | A | B |
| 270 | B | A | A |
| 271 | | A | A |
| 272 | B | C | C |
| 273 | C | A | C |
| | | | |
| 274 | B | A | A |
| 275 | C | A | B |
| 276 | B | B | C |
| 277 | B | C | C |
| 278 | C | A | B |
| 279 | A | A | B |
| 280 | C | B | C |
| 281 | B | B | B |
| 282 | B | B | C |
| 283 | B | A | A |
| 284 | B | C | C |
| 285 | B | A | B |
| 286 | C | B | C |
| 287 | B | B | B |
| 288 | B | C | C |
| 289 | B | B | C |
| 290 | B | C | C |
| 291 | A | C | C |
| 292 | A | B | C |
| 293 | B | A | B |
| 294 | B | A | A |
| 295 | C | B | B |
| 296 | C | A | B |
| 297 | C | A | C |
| 298 | A | C | C |
| 299 | B | B | B |
| 300 | B | A | A |
| 301 | B | A | A |
| 302 | B | A | B |
| 303 | A | A | C |
| 304 | A | A | B |
| 305 | C | A | B |
| 306 | C | A | B |
| 307 | B | A | A |
| 308 | B | B | B |
| 309 | A | C | C |
| 310 | | C | B |
| 311 | | C | C |
| 312 | B | | C |
| 313 | A | | A |
| 314 | A | | C |
| 315 | A | | C |
| 316 | B | | A |
| 317 | B | | B |
| 318 | B | | C |
| 319 | B | | C |
| 320 | A | | C |
| 321 | A | | C |
| 322 | A | | C |
| 323 | | | C |
| 324 | B | A | A |
| 325 | C | C | C |
| 326 | A | C | C |
| 327 | A | C | C |
| 328 | A | B | C |
| | | | |
| 329 | B | C | B |
| 330 | A | A | A |
| 331 | A | C | C |
| 332 | B | C | C |
| 333 | A | C | C |
| 334 | A | C | C |
| 335 | | C | C |
| 336 | | B | C |
| 337 | A | B | A |
| 338 | A | C | C |
| 339 | A | C | C |
| 340 | A | B | B |
| 341 | C | C | C |
| 342 | C | C | C |
| 343 | A | B | B |
| 344 | B | B | B |
| 345 | A | A | B |
| 346 | A | B | B |
| 347 | | C | C |
| 348 | | B | B |
| 349 | A | B | C |
| 350 | A | B | C |
| 351 | A | A | A |
| 352 | A | A | A |
| 353 | B | A | A |
| 354 | B | B | B |
| 355 | A | C | C |
| 356 | B | B | C |
| 357 | B | B | B |
| 358 | B | B | A |
| 359 | | B | B |
| 360 | | B | B |
| 361 | C | A | A |
| 362 | B | B | B |
| 363 | B | C | C |
| 364 | A | B | A |
| 365 | C | B | C |
| 366 | C | C | C |
| 367 | C | B | B |
| 368 | C | C | C |
| 369 | C | B | B |
| 370 | C | A | B |
| 371 | | C | C |
| 372 | | B | A |
| 373 | | C | B |
| 374 | | C | C |
| 375 | | C | C |
| 376 | A | A | A |
| 377 | A | B | A |
| 378 | A | B | B |
| 379 | A | B | A |
| 380 | C | C | C |
| 381 | A | A | A |
| 382 | B | B | A |
| 383 | | B | C |
| 384 | B | B | B |
| 385 | | B | B |
| 386 | B | B | A |
| 387 | B | C | C |
| 388 | B | B | B |
| 389 | B | B | B |
| 390 | | C | C |
| 391 | A | C | C |
| 392 | B | A | A |
| 393 | B | B | B |
| 394 | B | B | C |
| 395 | A | B | A |
| 396 | | C | B |
| 397 | A | A | A |
| 398 | A | B | A |
| 399 | B | B | B |
| 400 | B | A | B |
| 401 | | B | C |
| 402 | | A | A |
| 403 | C | A | |
| 404 | C | A | B |
| 405 | B | B | A |
| 406 | C | B | A |
| 407 | B | B | B |
| 408 | C | B | B |
| 409 | C | B | B |
| 410 | C | B | C |
| 411 | B | B | B |
| 412 | B | B | C |
| 413 | B | A | B |
| 414 | B | C | |
| 415 | A | C | |
| 416 | B | B | |
| 417 | C | C | |
| 418 | C | A | C |

The BRAF- KRAS disruption assay results of exemplary compounds in the absence of cyclophilin A or using wild-type (WT) KRAS instead of KRAS-G12C are shown in Table 5. These results demonstrate that the tested compounds: (1) require the presence of CYPA to cause disruption of the BRAF-GTP-KRAS-G12C complex; and (2) are incapable of disrupting a BRAF-GTP-KRAS complex when wild-type KRAS is used.

**Table 5.**

| **Compound Number** | **BRAF-GTP-KRAS-G12C complex disruption without CYPA (IC₅₀, µM)** | **BRAF-GTP-KRASWT complex disruption with CYPA ( IC₅₀, µM)** |
|---|---|---|
| 110 | >30 | >30 |
| 169 | >30 | >30 |
| 192 | >30 | >30 |
| 221 | >30 | >30 |
| 226 | >30 | >30 |

### Example 16 - Determination of percentage of crosslinking to KRAS-G12C in the presence of BME Materials and Reagents

**1.** 10 x Incubation buffer: 125 mM HEPES pH 7.4, 750 mM NaCl, 10 mM MgCl₂
**2.** 50 µM Protein A stock (CypA) in incubation buffer
**3.** 5 µM Protein B (G12C-GMPNP) stock in incubation buffer
**4.** 20 µM compound stock in 1 x Incubation buffer with 10% DMSO
**5.** 25 mM BME stock solution (prepared by diluting the BME stock in Milli Q water)

### Procedure:

CypA 1-165 (final concentration 5 µM), test compound (final concentration 2 µM) and G12C-GMPNP (final concentration 0.5 µM) were incubated in the incubation buffer (125 mM HEPES pH 7.4, 750 mM NaCl, 10 mM MgCl₂) for the requisite amount of time at room temperature. The samples were quenched with formic acid, final concentration 0.5%. 10 µL aliquots were injected to TOF-MS.

### Sequence of addition for a final incubation volume of 50 µL

**1.** 5 µL 10X incubation buffer
**2.** 28 ul H₂O
**3.** 2 µL from 25 mM BME stock
**4.** 5 µL CypA at 50 uM
**5.** 5 µL from 5 µM G12C-GMPNP
**6.** 5 µL compound from 20 µM compound stock

### TOF-MS Analysis:

LC-MS is performed on an Agilent 6230 TOF-LC mass spectrometer equipped with an electrospray probe operated in positive ionization mode. 10 µL samples are injected on a Sepax Bio-C4, *300* Å, 2.1×100mm column. The mobile phase is 0.1 % (vol/vol) formic acid and 0.1 % 1 mM ammonium formate in 95 % water, 4.8 % acetonitrile (A) and 0.1 % (vol/vol) formic acid and 0.1 % 1 mM ammonium formate in 95 % acetonitrile and 4.8 % water (B). The separation is performed by a 9 min total gradient consisting of 5 min linear gradient from 25 % to 50 % B, and a wash at 100 % B for 1.25 min, all at a flow rate of 0.6 mL/min (see attached timetable below). Mass spectrometer source conditions were capillary voltage, 4,000 V; cone voltage, 120 V; source temperature, 275 °C; scan range, 100-2,000 a.m.u. with a cycle time of 1 s.

### Calculations:

The observed mass is generated by averaging the major peak in the total ion current (TIC). The charge-state series of the species are deconvoluted using Agilent MassHunter Bioconfirm using maximum entropy setting (range is set to 17000-23000 Da). Integration of deconvoluted protein peaks (bound and unbound species) enables % bound calculation using equation: % *bound to Protein B= peak height of bound species*/ *[peak height of bound species + peak height of unbound] X 100.*

The biochemical crosslinking assay results of compounds described herein are shown in Table 6.

**Table 6.**

| **Compound Number** | **% Crosslinking with KRAS-G12C-GTP in presence of CYPA, 5 min incubation** |
|---|---|
| 110 | 83 |
| 169 | 39* |
| 192 | 62 |
| 221 | 28 |
| 226 | 27 |

| | |
|---|---|
| * 60 minute incubation | |

These results show that the exemplary compounds are capable of cross-linking to KRAS-G12C in the presence of CYPA and therefore should form a covalent bond to the cysteine at amino acid 12 in KRAS-G12C *in vivo.*

## Claims

1. A compound of formula I: 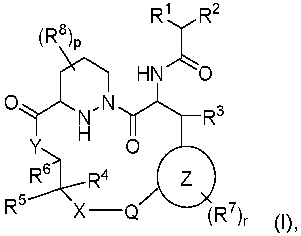 a pharmaceutically acceptable salt, an enantiomer, a stereoisomer, or a tautomer thereof, wherein:
Q is a bicyclic arylene, a bicyclic heteroarylene, or a bicyclic heterocyclylene, wherein a first ring in Q is bonded to X, and a second ring in Q is bonded to Z, and wherein Q is optionally substituted;
X is a bond; a straight chain C₁-C₃ alkylene optionally substituted with 1 to 3 substituents independently selected from fluoro, -CN, -C₁-C₃ alkyl, and -O-C₁-C₃ alkyl; -O-; -S(O)₀-₂-; *-CH₂-O-; *-CH₂-S(O)₀-₂-; *-O-CH₂-; or *-CH₂-S(O)₀-₂-, wherein "*" represents a portion of X bound to -C(R⁴)(R⁵)-;
Y is -O-, -NH- or -N(C₁-C₃ alkyl)-;
ring Z is phenyl or a 6-membered heteroaryl;
R¹ is optionally substituted C₁-C₆ alkyl, -(CH₂)₀-₁-(C₃-C₆ optionally substituted cycloalkyl), -(CH₂)₀-₁-(optionally substituted aryl), or optionally substituted heterocyclyl;
R² is: 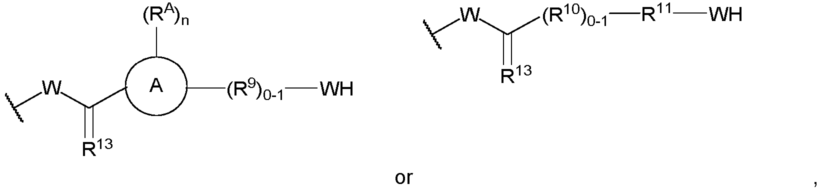 wherein:
ring A is a 4-8 membered cycloalkyl or a 4-8 membered heterocyclyl;
W is -N(R¹²)-, -O-, or -C(R^{12a})(R^{12b})-;
each R^{A} is each independently fluoro; chloro; -CN; -OH; -NH₂; -C₁-C₃ alkyl optionally substituted with CN, OH, NH₂ or -O-C₁-C₃ alkyl; -O-C₁-C₃ alkyl; or -NH-C₁-C₃ alkyl;
R⁹, if present, is -N(C₀-C₅ alkylene-H)-, -N(C(O)-(C₀-C₅ alkylene-H)-, -C(C₀-C₃ alkylene-H)(C₀-C₅ alkylene-H)-, or -C(C₀-C₃ alkylene-H)(C(O)-C₀-C₅ alkylene-H)-, wherein each alkylene portion of R⁹ is optionally substituted with one or more substituent, wherein each substituent is, independently, selected from halo, -CN, -OH, -C₁-C₃ alkyl, and -O-C₁-C₃ alkyl;
R¹⁰, if present, is C₁-C₄ alkylene optionally substituted with one or more substituent, wherein each substituent is, independently, selected from halo, -CN, -OH, -C₁-C₃ alkyl,
and -O-C₁-C₃ alkyl;
R¹¹ is -N(C₀-C₅ alkylene-H)-, -N(C(O)-(C₀-C₅ alkylene-H)-, -C(C₀-C₃ alkylene-H)(C₀-C₅ alkylene-H)-, -C(C₀-C₃ alkylene-H)(C(O)-C₀-C₅ alkylene-H)-, or a saturated, nitrogen-containing heterocyclyl, where each alkylene portion of R¹¹ is optionally substituted with one or more substituent, wherein each substituent is, independently, selected from halo, -CN, -OH, -C₁-C₃ alkyl, and -O-C₁-C₃ alkyl;
R¹² is hydrogen, or -C₁-C₃ alkyl, or
R¹² is taken together with one R^{A}, the atoms to which they are respectively attached and any intervening atoms to form an optionally substituted, 5-8 membered heterocyclyl that is fused or spiro-fused to ring A, or
R¹² is taken together with any methylene unit in R¹⁰, or any methylene unit in R¹¹, the atoms to which they are respectively attached and any intervening atoms to form an optionally substituted, 5-8 membered heterocyclyl;
each of R^{12a} and R^{12b} are independently hydrogen, or -C₁-C₃ alkyl, or R^{12a} and R^{12b} are taken together with the carbon atom to which they are bound to form a 3-6 membered cycloalkyl ring;
R¹³ is O, S, N-CN, or N-O-C₁-C₃ alkyl; and
WH is 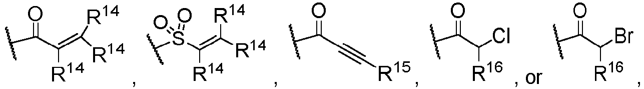
each R¹⁴ is independently hydrogen, -CN, or -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or an optionally substituted 4-7 membered saturated heterocyclyl;
R¹⁵ is -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or an optionally substituted 4-7 membered saturated heterocyclyl;
R¹⁶ is hydrogen, -C₁-C₃ alkyl optionally substituted with one or more substituents independently selected from -OH, -O-C₁-C₃ alkyl, -NH₂, -NH(C₁-C₃ alkyl), -N(C₁-C₃ alkyl)₂, or an optionally substituted 4-7 membered saturated heterocyclyl; or
R¹⁴ is taken together with either of R⁹ or R¹¹, the atoms to which they are attached and any intervening atoms to form an optionally substituted 5-8 membered ring system; or
R¹⁶ is taken together with either of R⁹ or R¹¹, the atoms to which they are attached and any intervening atoms to form an optionally substituted 5-8 membered ring system;
R³ is hydrogen, halogen, C₁-C₃ alkyl, or C₁-C₃ hydroxyalkyl;
R⁴ is hydrogen, halogen, or optionally substituted C₁-C₃ alkyl;
R⁵ is hydrogen, halogen, -OH, -CN, -O-(optionally substituted C₁-C₃ alkyl), optionally substituted C₁-C₃ alkyl, optionally substituted C₂-C₆ alkenyl, optionally substituted C₂-C₆ alkynyl, -(CH₂)₀-₁-aryl, -(CH₂)₀-₁-heteroaryl, -(CH₂)₀-₁-cycloalkyl, or -(CH₂)₀-₁-heterocyclyl; or
R⁴ and R⁵ are taken together to form =CH₂, an optionally substituted C₃-C₆ cycloalkyl, or a 3-7 membered saturated heterocyclyl; or
R⁵ is taken together with a ring atom in Q, the carbon atom to which R⁴ is bound and X to form a 4-9 membered saturated or unsaturated heterocyclyl that is fused to Q;
R⁶ is hydrogen or -CH₃;
each R⁷ is independently halo, C₁-C₃ alkyl, C₁-C₃ haloalkyl, C₁-C₃ hydroxyalkyl, -OH, -O-C₁-C₃ alkyl, -O-C₁-C₃ haloalkyl, -NRⁿ¹Rⁿ², -NRⁿ¹ORⁿ², -ONRⁿ¹Rⁿ², or -NRⁿ¹NRⁿ²Rⁿ³;
Rⁿ¹ is H, C₁-C₃ alkyl, C₁-C₃ heteroalkyl, C₁-C₃ haloalkyl, -C₁-C₃ hydroxyalkyl, or C₁-C₃ aminoalkyl, wherein one methylene unit of Rⁿ¹ is optionally substituted with 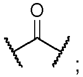
Rⁿ² is H, C₁-C₃ alkyl, C₁-C₃ heteroalkyl, C₁-C₃ haloalkyl, C₁-C₃ hydroxyalkyl, or C₁-C₃ aminoalkyl, wherein one methylene unit of Rⁿ² is optionally substituted with 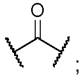
Rⁿ³ is H, C₁-C₃ alkyl, C₁-C₃ heteroalkyl, C₁-C₃ haloalkyl, C₁-C₃ hydroxyalkyl, or C₁-C₃ aminoalkyl, wherein one methylene unit of Rⁿ³ is optionally substituted with 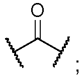
each R⁸ is independently halo, C₁-C₃ alkyl, or C₁-C₃ haloalkyl;
n is 0, 1, 2, 3, 4, 5, or 6;
p is 0, 1, 2, or 3; and
r is 0, 1, 2, 3, or 4.

2. The compound of claim 1, wherein said compound has the structure of formula (la): 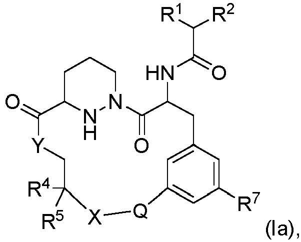 or a pharmaceutically acceptable salt, an enantiomer, a stereoisomer, or a tautomer thereof,
wherein:
X is a bond, -O-, -CH₂-, -CH(CH₃)-, *-CH₂-O-, or -CH₂-CH₂-, wherein "*" represents a portion of X bound to C(R⁴)(R⁵);
Y is -O- or -NH-;
R¹ is -C₁-C₄ alkyl, -(CH₂)₀-₁-(C₃-C₆ cycloalkyl), or -C₄-C₆ cycloalkyl;
R² is: 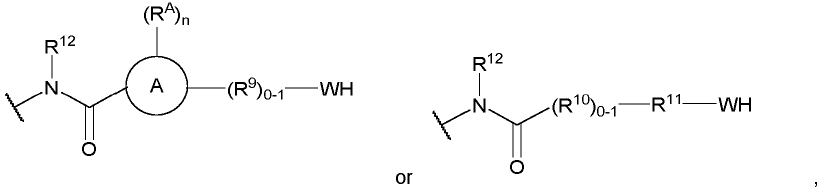 wherein:
ring A is a 4-8 membered cycloalkyl or a 4-8 membered saturated heterocyclyl;
each R^{A} is each independently fluoro; chloro; -CN; -OH; -NH₂; -C₁-C₃ alkyl optionally substituted with CN, OH, NH₂ or -O-C₁-C₃ alkyl; -O-C₁-C₃ alkyl; or -NH-C₁-C₃ alkyl;
n is 0, 1, 2, 3, 4, 5, or 6;
R⁹, if present, is -N(C₀-C₅ alkylene-H)-, -N(C(O)-(C₀-C₅ alkylene-H)-, -C(C₀-C₃ alkylene-H)(C₀-C₅ alkylene-H)-, or -C(C₀-C₃ alkylene-H)(C(O)-C₀-C₅ alkylene-H)-, wherein each alkylene portion of R⁹ is optionally substituted with one or more substituent independently selected from halo, -CN, -OH, -C₁-C₃ alkyl, and -O-C₁-C₃ alkyl;
R¹⁰, if present, is C₁-C₄ alkylene optionally substituted with one or more substituent independently selected from halo, -CN, -OH, -C₁-C₃ alkyl, and -O-C₁-C₃ alkyl;
R¹¹ is -N(C₀-C₅ alkylene-H)-, -N(C(O)-(C₀-C₅ alkylene-H)-, -C(C₀-C₃ alkylene-H)(C₀-C₅ alkylene-H)-, or -C(C₀-C₃ alkylene-H)(C(O)-C₀-C₅ alkylene-H)-, wherein each alkylene portion of R¹¹ is optionally substituted with one or more substituent independently selected from halo, -CN, -OH, -C₁-C₃ alkyl, and -O-C₁-C₃ alkyl;
R¹² is hydrogen, or -C₁-C₃ alkyl, or
R¹² is taken together with one R^{A}, the atoms to which they are respectively attached and any intervening atoms to form an optionally substituted, 5-8 membered heterocyclyl that is fused to ring A, or
R¹² is taken together with any methylene unit in R¹⁰, or any methylene unit in R¹¹, the atoms to which they are respectively attached and any intervening atoms to form an optionally substituted, 5-8 membered heterocyclyl;
WH is 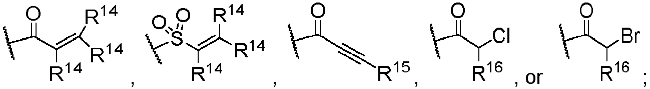
each R¹⁴ is independently hydrogen, -CN, -C₁-C₃ alkyl, -C₁-C₃ hydroxyalkyl, -O-C₁-C₃ alkyl;
R¹⁵ is -C₁-C₃ alkyl, -C₁-C₃ hydroxyalkyl, or -C₁-C₃ alkylene-O-C₁-C₃ alkyl;
R¹⁶ is hydrogen, -C₁-C₃ alkyl, -C₁-C₃ hydroxyalkyl, or -C₁-C₃ alkylene-O-C₁-C₃ alkyl; or
R¹⁴ is taken together with either of R⁹ or R¹¹, the atoms to which they are attached and any intervening atoms to form an optionally substituted 5-8 membered ring system, or
R¹⁶ is taken together with either of R⁹ or R¹¹, the atoms to which they are attached and any intervening atoms to form an optionally substituted 5-8 membered ring system;
R⁴ is hydrogen, halo, or C₁-C₃ alkyl;
R⁵ is hydrogen, halo, -OH, C₁- C₃ alkyl, C₁-C₃ hydroxyalkyl, C₁-C₃ alkylene-O-C₁-C₃ alkyl, C₁-C₃ haloalkyl, -(CH₂)₀-₁-C₃-C₆ cycloalkyl, C₁-C₃ cyanoalkyl, or -(CH₂)₀-₁-aryl (benzyl), or
R⁴ and R⁵ are taken together to form =CH₂, or a C₃-C₆ cycloalkyl, or
R⁵ is taken together with a ring atom of Q, the carbon atom to which it is bound and X to form a 5-7 membered saturated heterocyclyl;
R⁷ is -OH, -NH₂, or C₁-C₃ haloalkyl;
Q is a bicyclic arylene, a bicyclic heteroarylene, or a bicyclic heterocyclylene, wherein:
a first ring in Q is bonded to X, and a second ring in Q is bonded Z; and
Q is optionally substituted with one or more independently selected substituents selected from =O; -CN; -C₁-C₅ alkyl optionally substituted with one or more independently selected halo, CN, OH, -O-(C₁-C₃ alkyl), -C(O)-(C₁-C₃ alkyl), -O-(C₂-C₃ alkynyl), -(C₃-C₆ cycloalkyl), or a 4-7 membered saturated heterocyclyl; -O-(C₁-C₃ alkyl) optionally substituted with one or more independently selected halo; C₂-C₅ alkenyl optionally substituted with one or more independently selected -CN, or -OH; C₂-C₃ alkynyl; -S(O)₂-C₁-C₃ alkyl; -(CH₂)₀-₁-C₃-C₆ cycloalkyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heteroaryl optionally substituted with one or more independently selected halo, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-heterocyclyl optionally substituted with one or more independently selected halo, =O, -CN, C₁-C₃ alkyl optionally substituted with -CN or -O-C₁-C₃ alkyl, -C(O)-saturated heterocyclyl, -O-saturated heterocyclyl, O-cycloalkyl, or -O-aryl; -(CH₂)₀-₁-aryl optionally substituted with one or more independently selected halo, -CN, -C₁-C₃ alkyl optionally substituted with -CN or -O-C₁-C₃ alkyl, -C(O)-saturated heterocyclyl, -O-saturated heterocyclyl, O-cycloalkyl, or -O-aryl; -C(O)-NH-(C₁-C₃ alkyl); -C(O)-N(C₁-C₃ alkyl)₂; C₂-C₃ alkenylene=N-O-(C₁-C₃ alkyl) optionally substituted with C₃-C₆ cycloalkyl; or
two substituents on the same or adjacent ring atoms of Q are taken together to form a 5-7 membered monocyclic ring or a 6-12 membered bicyclic ring optionally substituted with one or more independently selected halo, =O, -CN, C₁-C₃ alkyl, or -O-C₁-C₃ alkyl; and fused to Q.

3. The compound of claim 2, wherein said compound has the structure of formula (Ib): 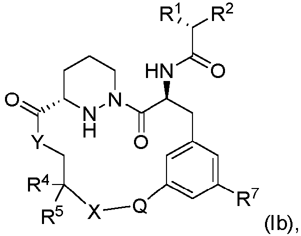 or a pharmaceutically acceptable salt, an enantiomer, a stereoisomer, or a tautomer thereof or
said compound has the structure of formula (Ic): 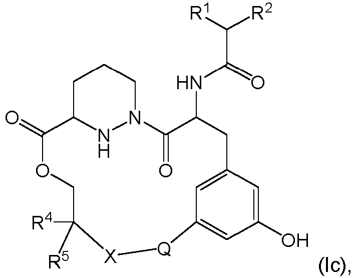 or a pharmaceutically acceptable salt, an enantiomer, a stereoisomer, or a tautomer thereof.

4. The compound of any one of claims 1-3, or a pharmaceutically acceptable salt, an enantiomer, a stereoisomer, or a tautomer thereof, wherein Q is a 5,6 bicyclic heteroarylene, a 5,6 bicyclic heterocyclylene, a 6,6 bicyclic heteroarylene, or a 6,6 bicyclic heterocyclylene; and wherein Q is optionally substituted;
optionally wherein Q is selected from the group consisting of: 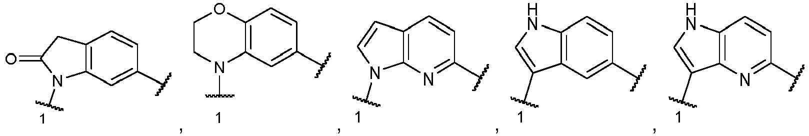 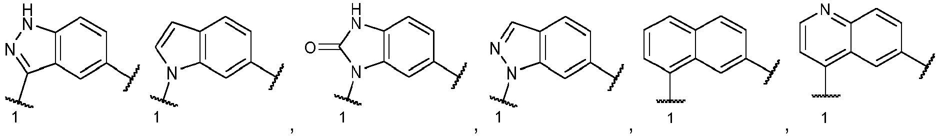 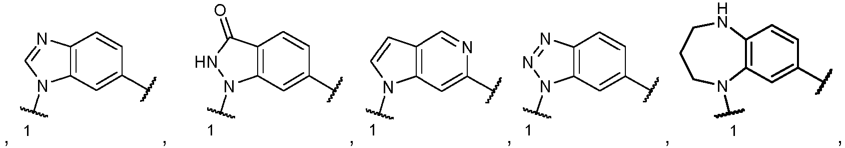 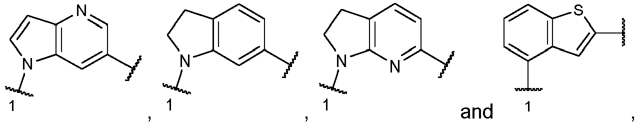 wherein:
"1" indicates a portion of Q bound to X; and
Q is further optionally substituted.

5. The compound of any one of claims 1-4, or a pharmaceutically acceptable salt, an enantiomer, a stereoisomer, or a tautomer thereof, wherein:
(i) R¹ is -CH₃, -CH₂CH₃, -(CH₂)₂CH₃, -CH(CH₃)₂, -CH(CH₃)CH₂CH₃, cyclopropylmethyl, cyclobutylmethyl, cyclopentylmethyl, cyclohexylmethyl, cyclopropyl, cyclobutyl, cyclopentyl, cyclohexyl, phenyl, 4-methoxybenzyl, or tetrahydropyran-4-yl; and/or
(ii) R⁹ is absent and ring A is a saturated, nitrogen-containing heterocyclyl; and/or
(iii) the portion of R² represented by: 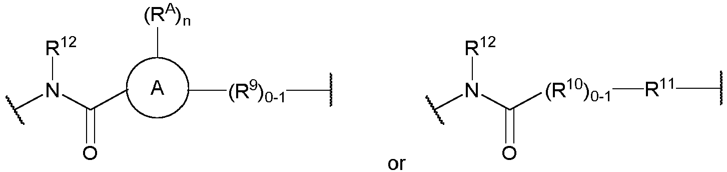 is selected from the group consisting of: 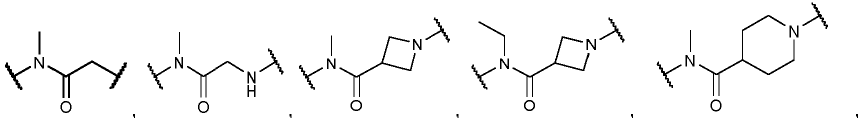 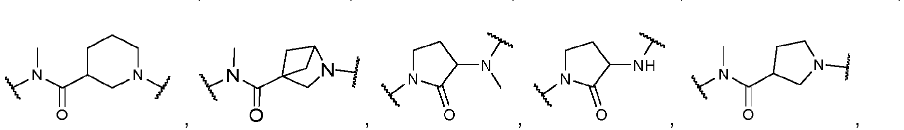 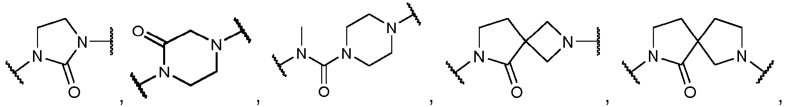 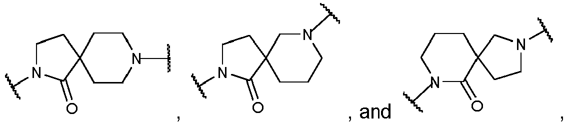 wherein each ring system in R² is optionally substituted with up to 4 substituents independently selected from fluoro;
chloro; -CN; -OH; -NH₂; -C₁-C₃ alkyl optionally substituted with CN, OH, NH₂ or -O-C₁-C₃ alkyl; -O-C₁-C₃ alkyl; and -NH-C₁-C₃ alkyl; and/or
(iv) the portion of R² represented by WH is -C(O)-C≡C-CH₃, -C(O)-CH=CH₂, -S(O)₂-CH=CH₂, -C(O)-CH₂Cl, -C(O)-CH(CH₃)Cl, or -C(O)-CH(Cl)-CH₂-O-CH₃, or
the portion of R² represented by -R¹¹-WH, when R¹¹ is taken together with one R¹⁴ is 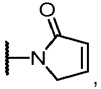 or

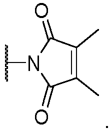

6. The compound of any one of claims 1-5, or a pharmaceutically acceptable salt, an enantiomer, a stereoisomer, or a tautomer thereof, wherein:
R⁴ is hydrogen, fluoro, or -CH₃; and
R⁵ is hydrogen, fluoro,
chloro, -OH, -CH₃, -CH₂CH₃, -CH(CH₃)₂, -CH₂OH, -CH₂OCH₃, -CH₂F, -CHF₂, CH₂CN, -CH₂-cyclopropyl, cyclopropyl, pyridyl, phenyl, or -CH₂-phenyl, wherein any phenyl portion of R⁵ is optionally substituted with up to 4 substituents independently selected from halo, -CN, and -O-C₁-C₃ alkyl; or
R⁴ and R⁵ are taken together to form =CH₂ or cyclopropyl, or cyclobutyl, or cyclopentyl, or cyclohexyl; or
R⁵ is taken together with the carbon atom to which it is bound, a ring atom of Q, and X to form oxazepane.

7. The compound of any one of claims 1-6, or a pharmaceutically acceptable salt, an enantiomer, a stereoisomer, or a tautomer thereof, wherein R⁷ is -OH, -NH₂, or -CHF₂.

8. A compound according to claim 1, or a pharmaceutically acceptable salt, an enantiomer, a stereoisomer, or a tautomer thereof, selected from:

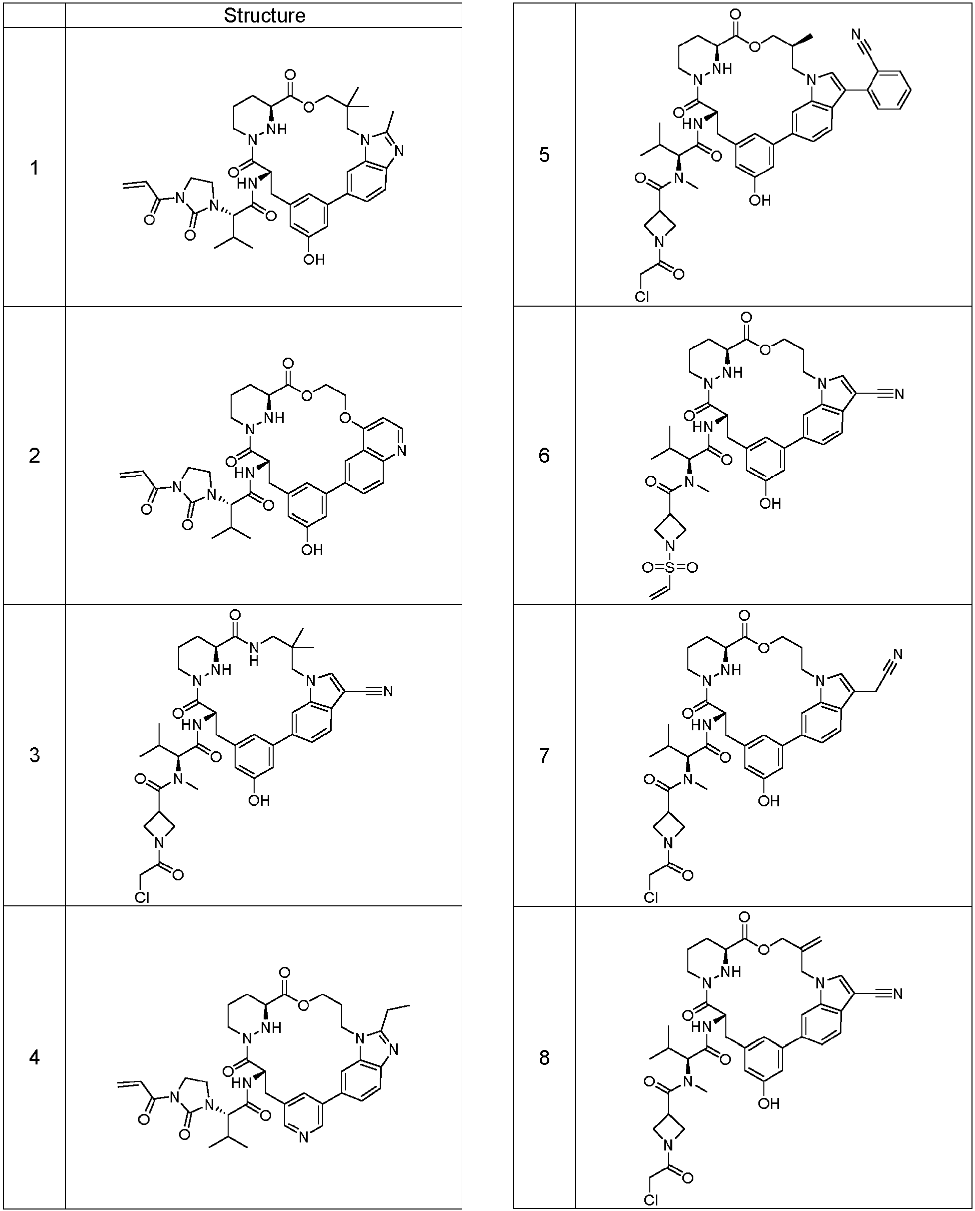

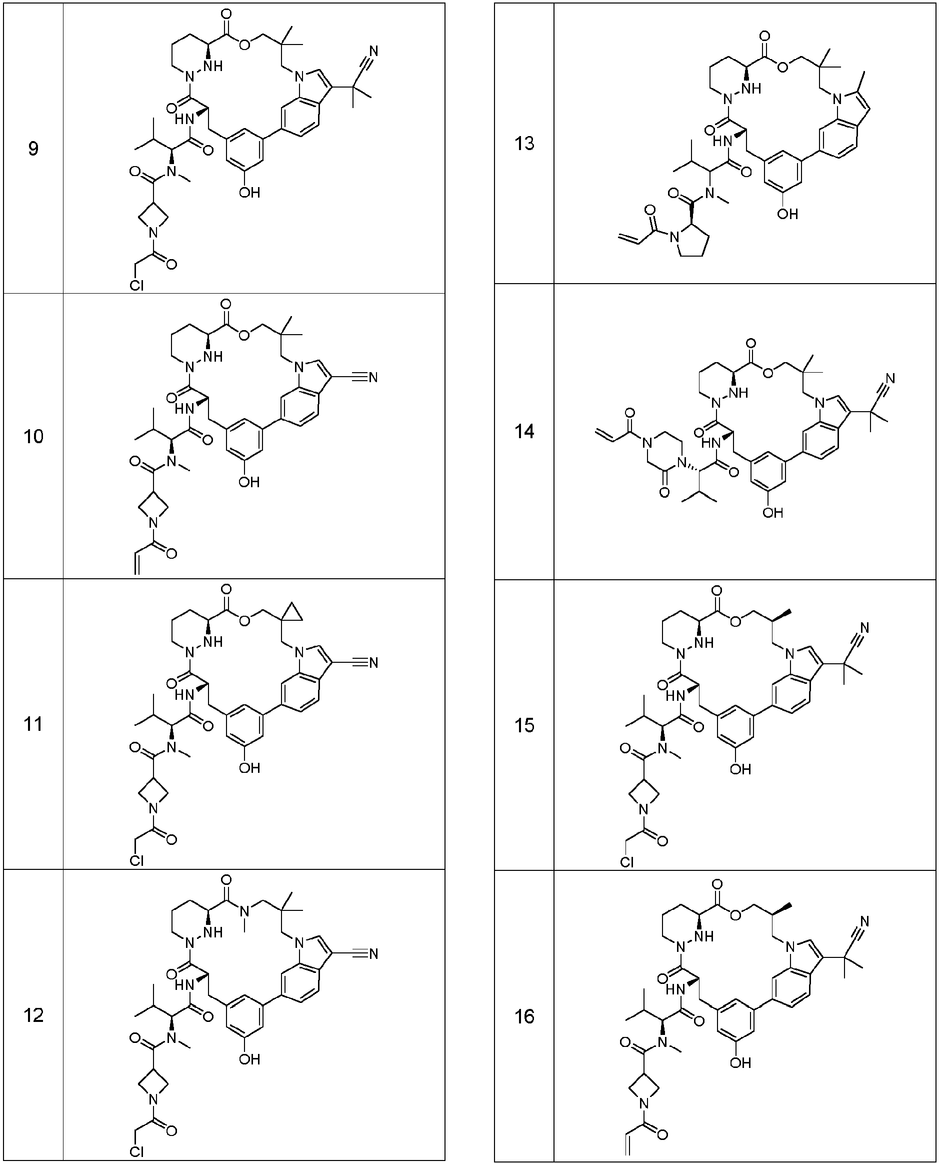

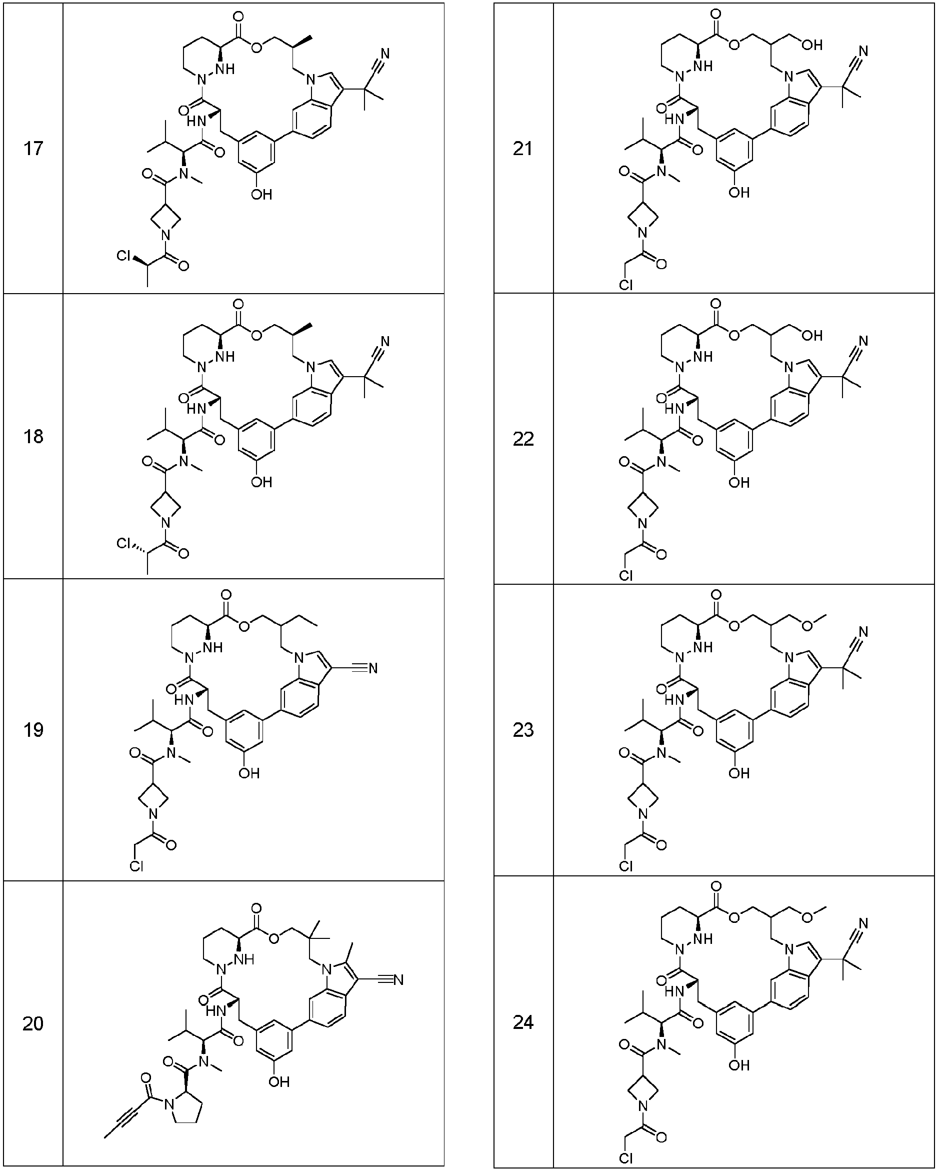

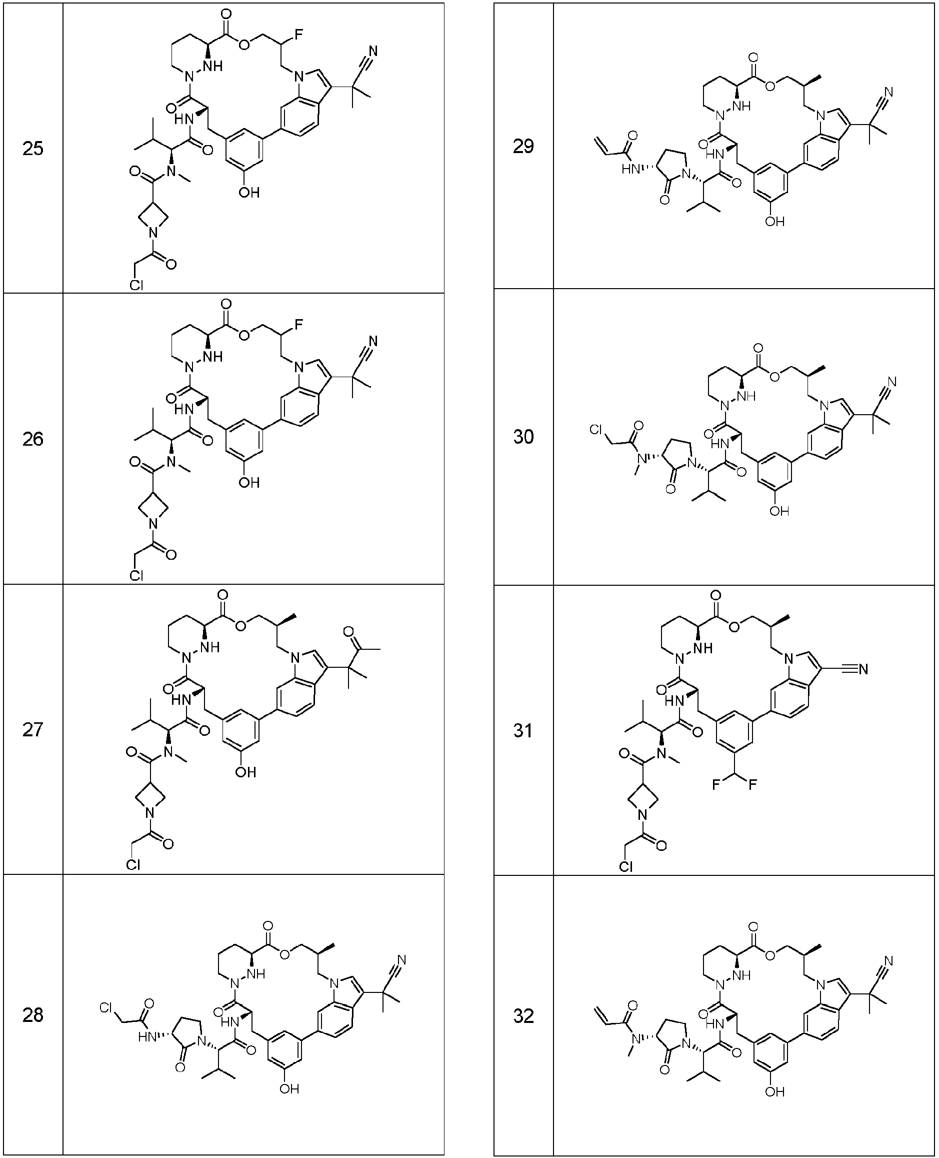

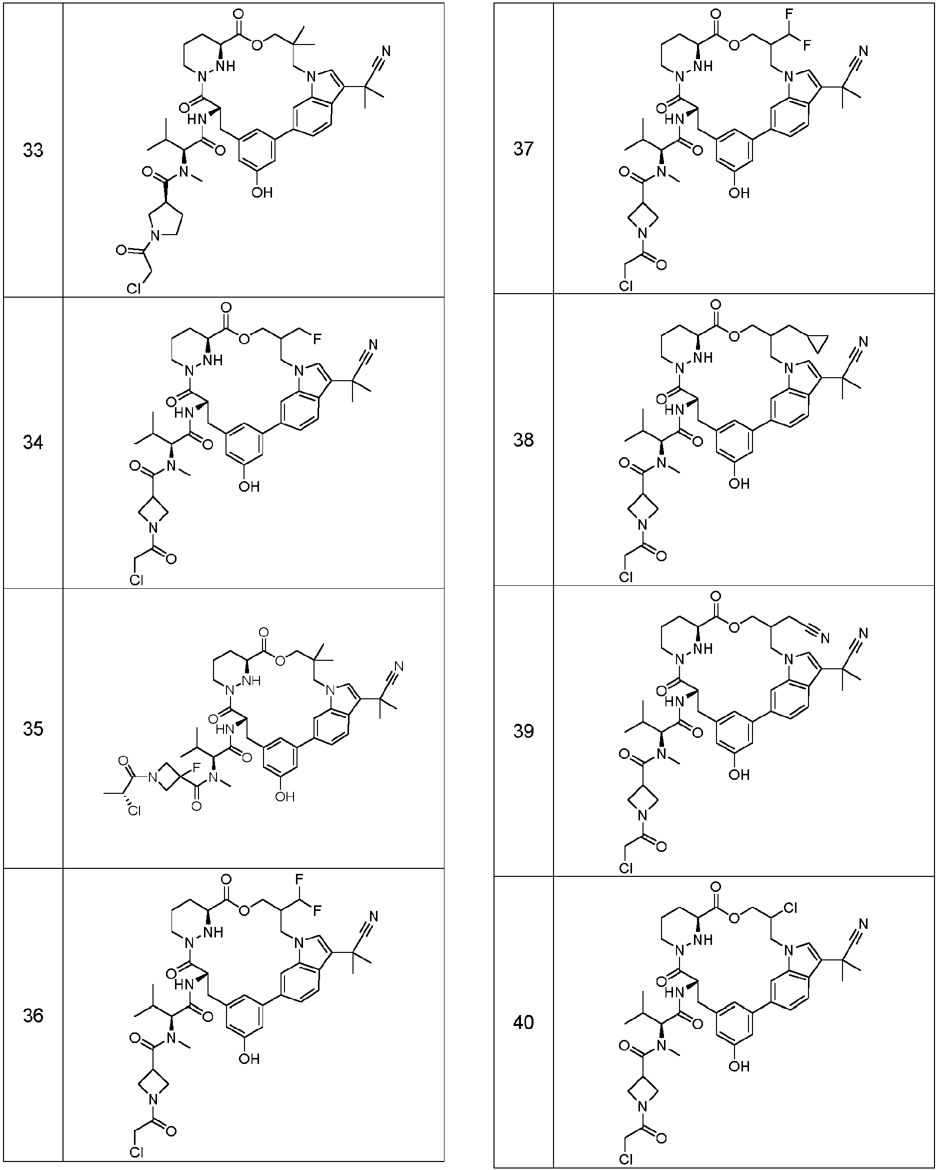

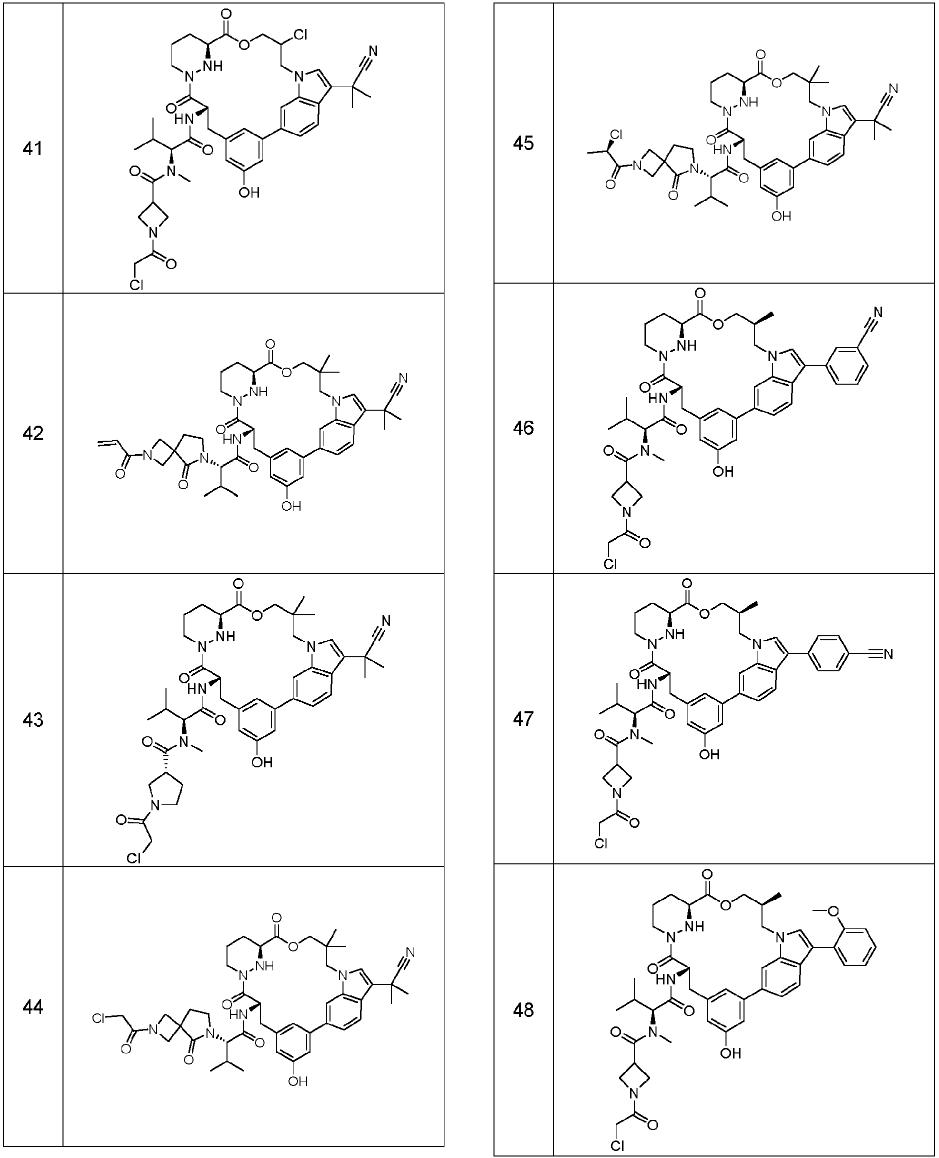

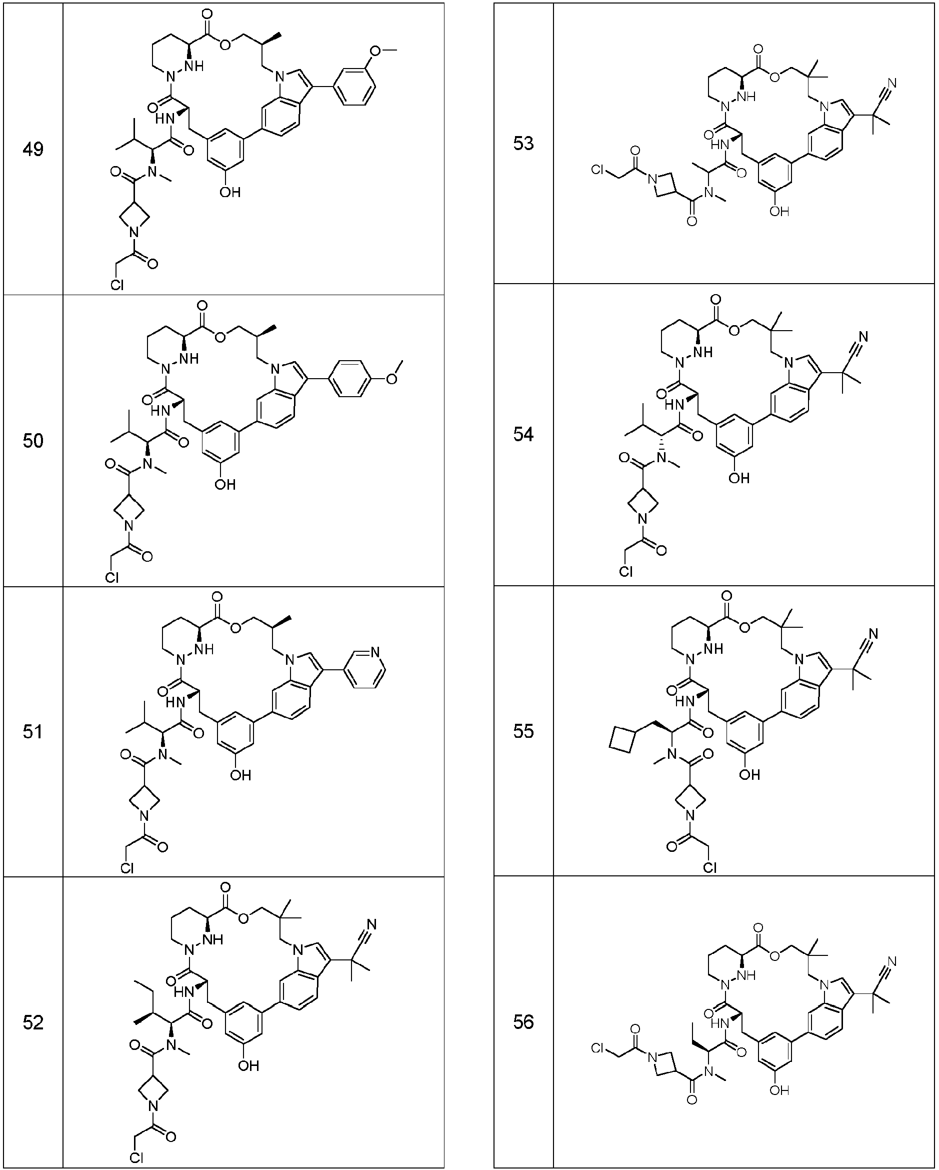

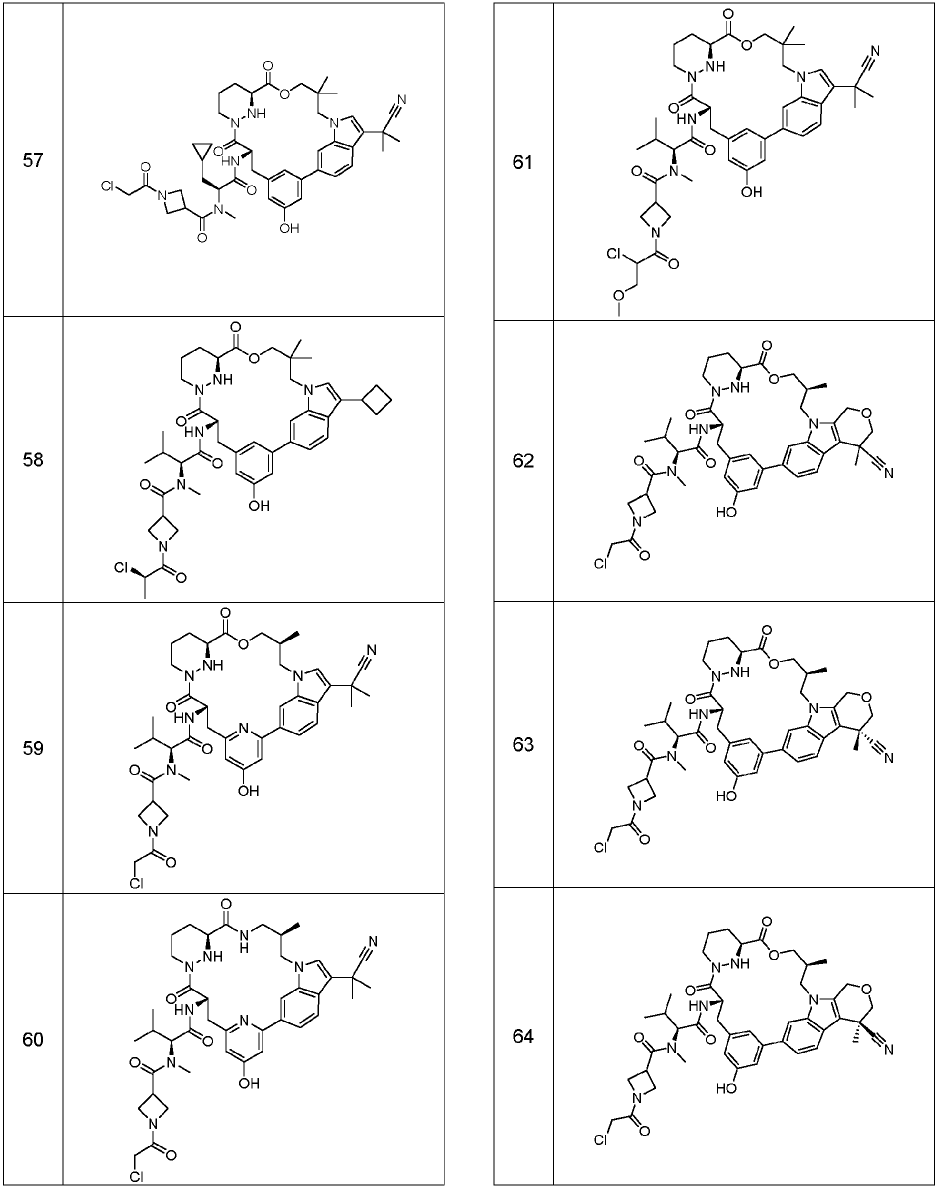

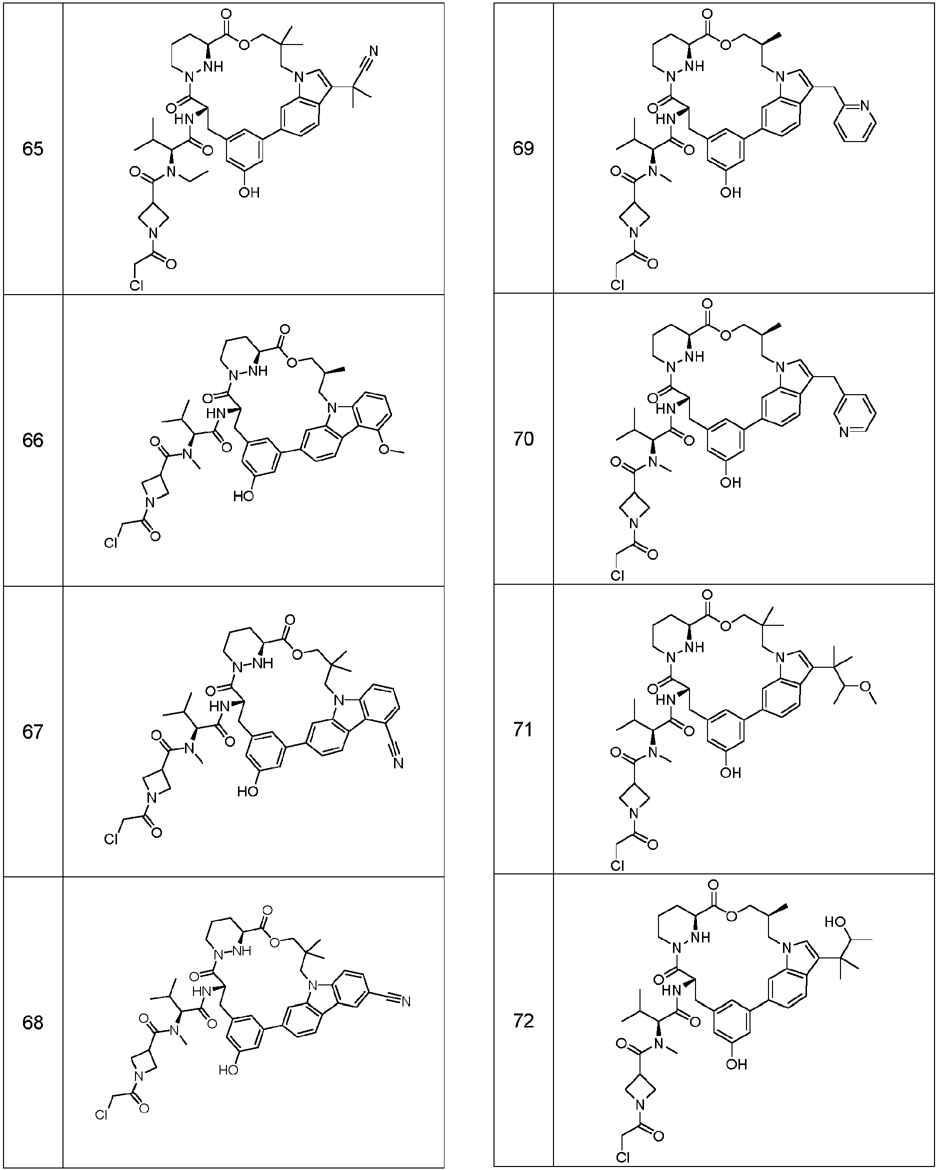

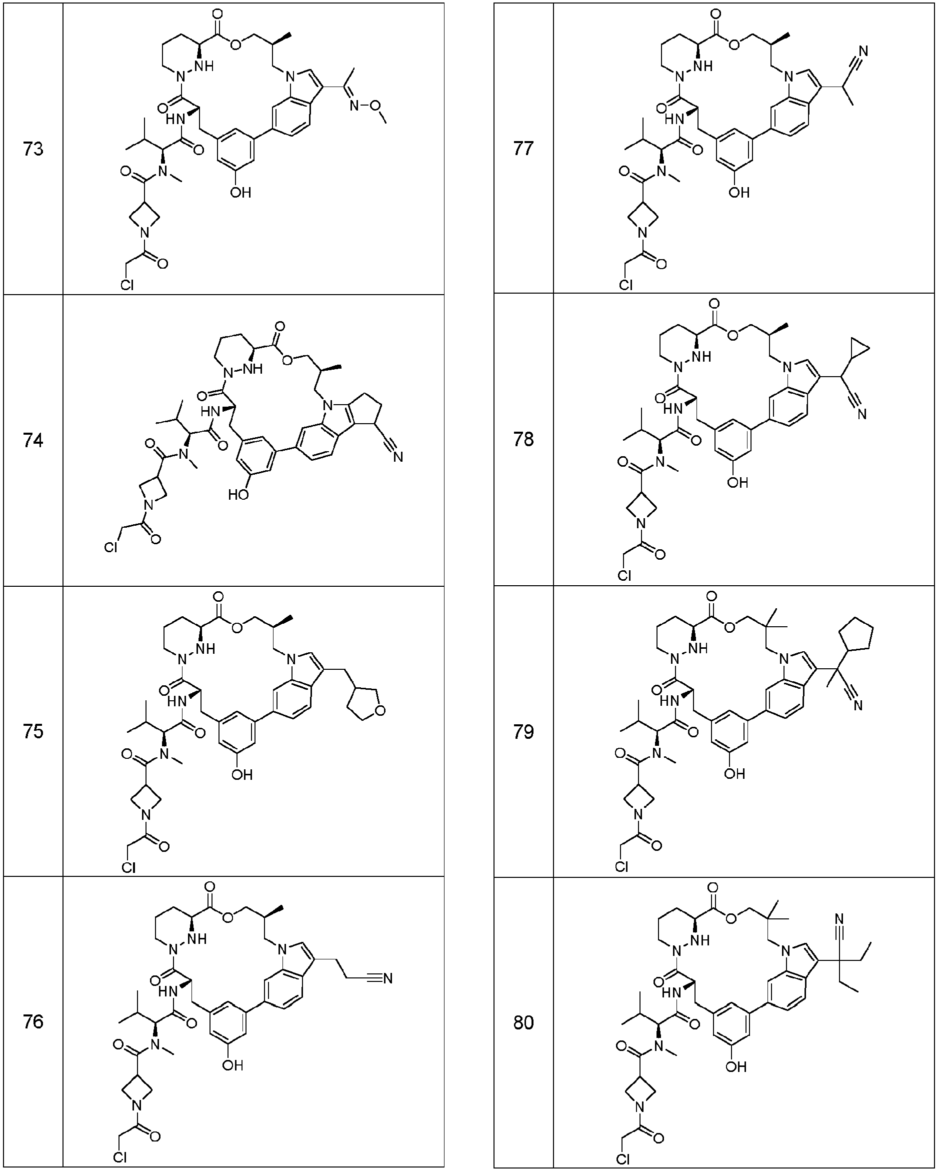

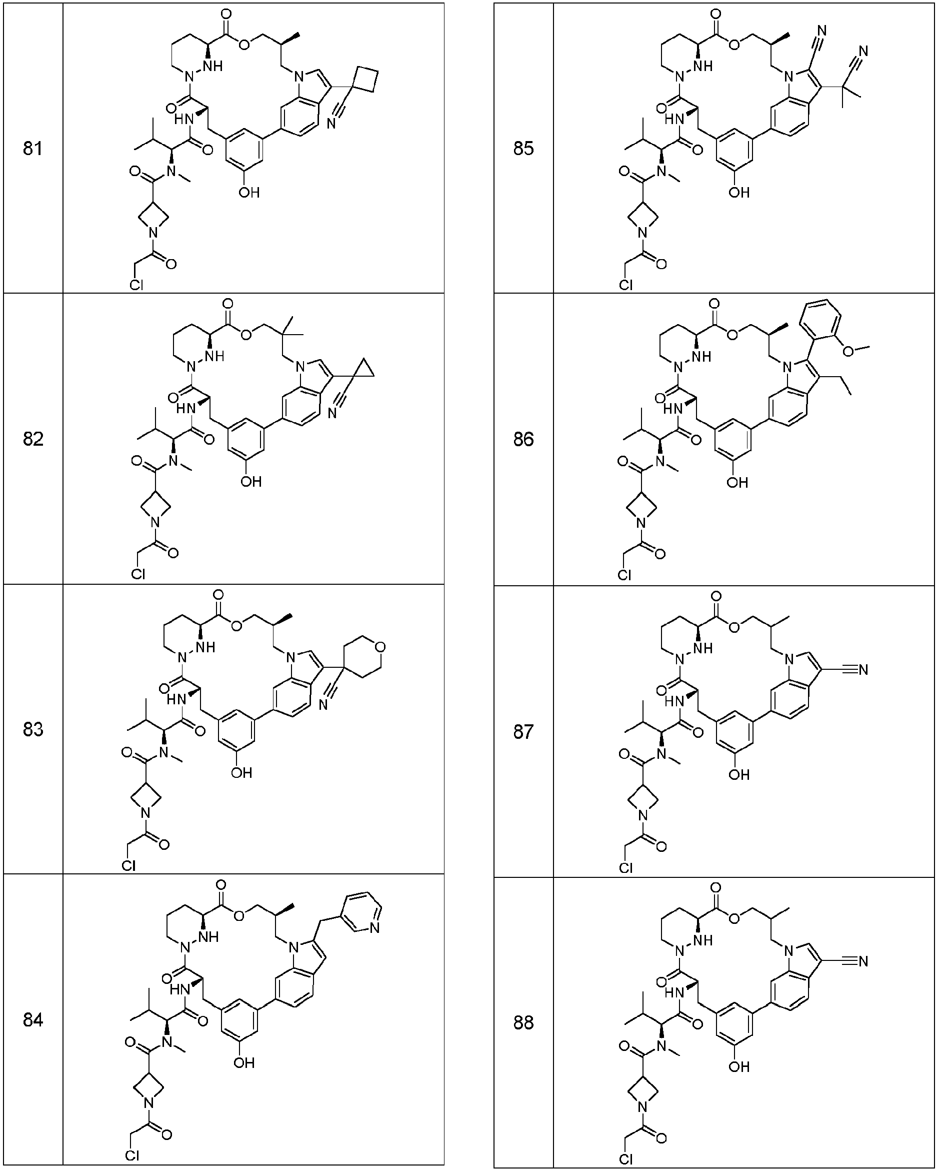

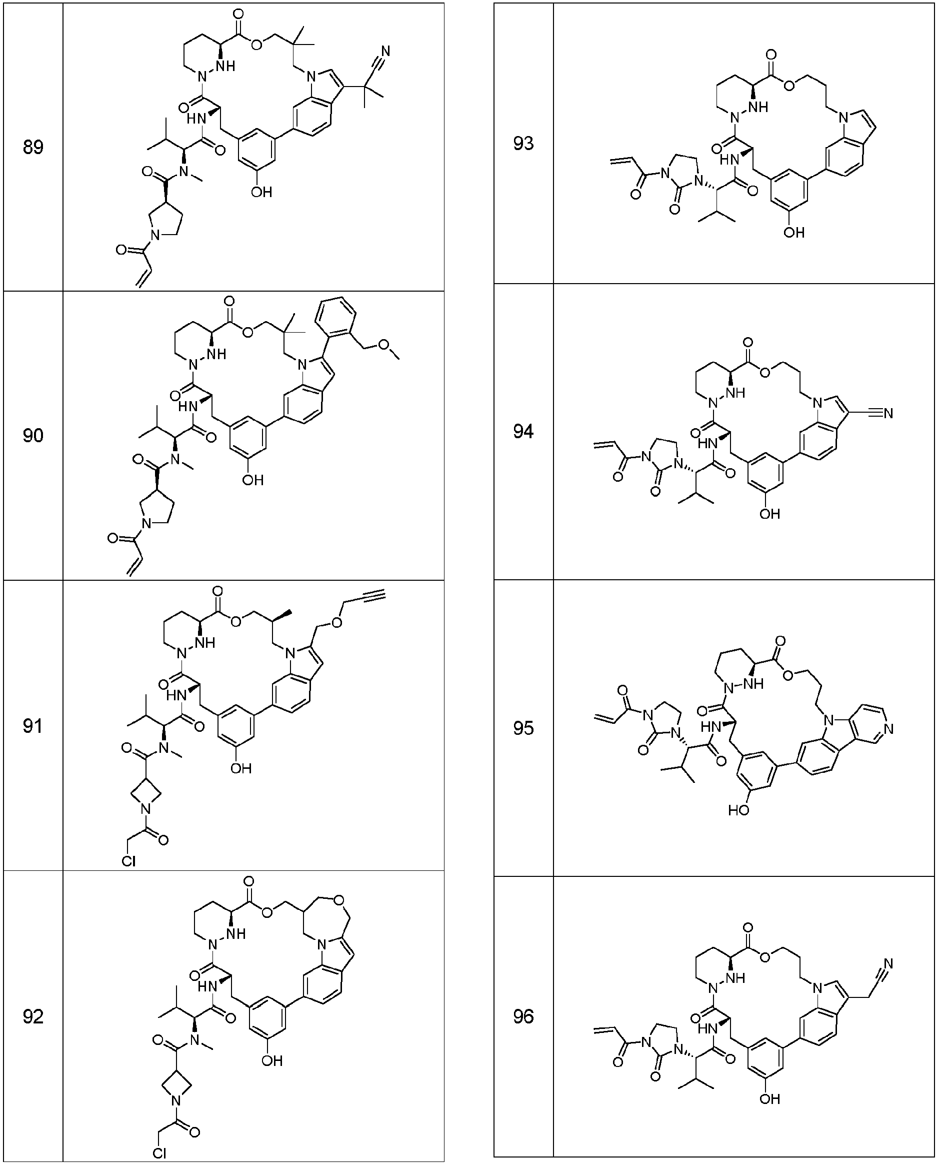

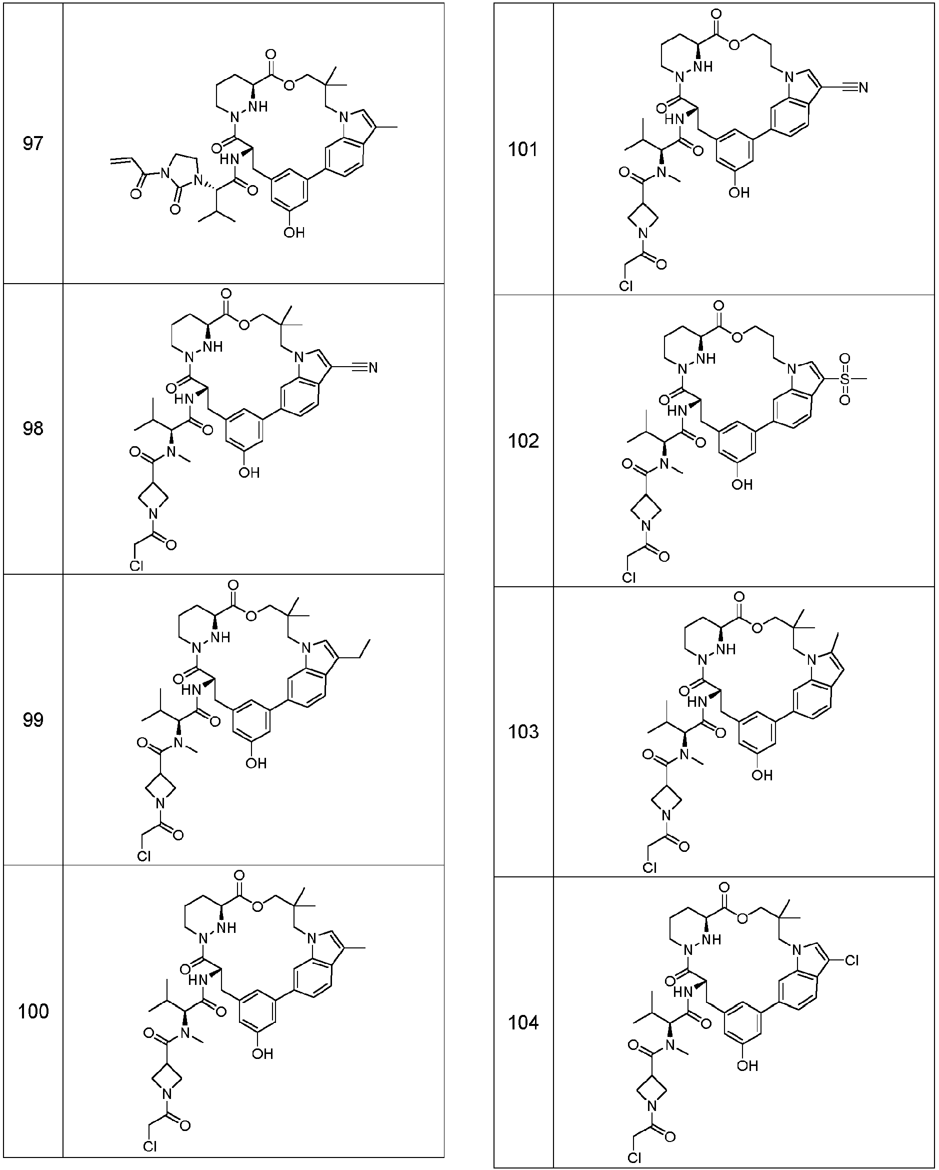

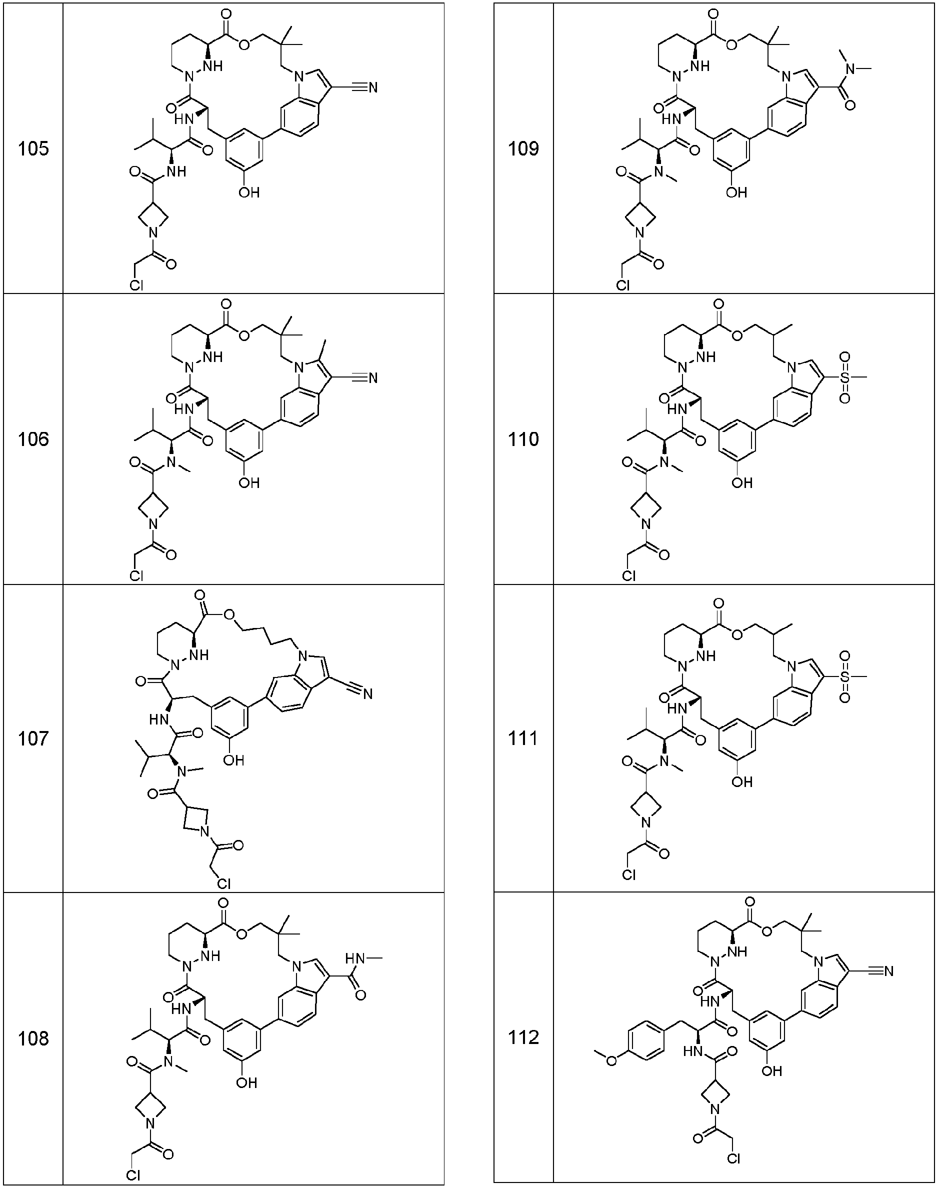

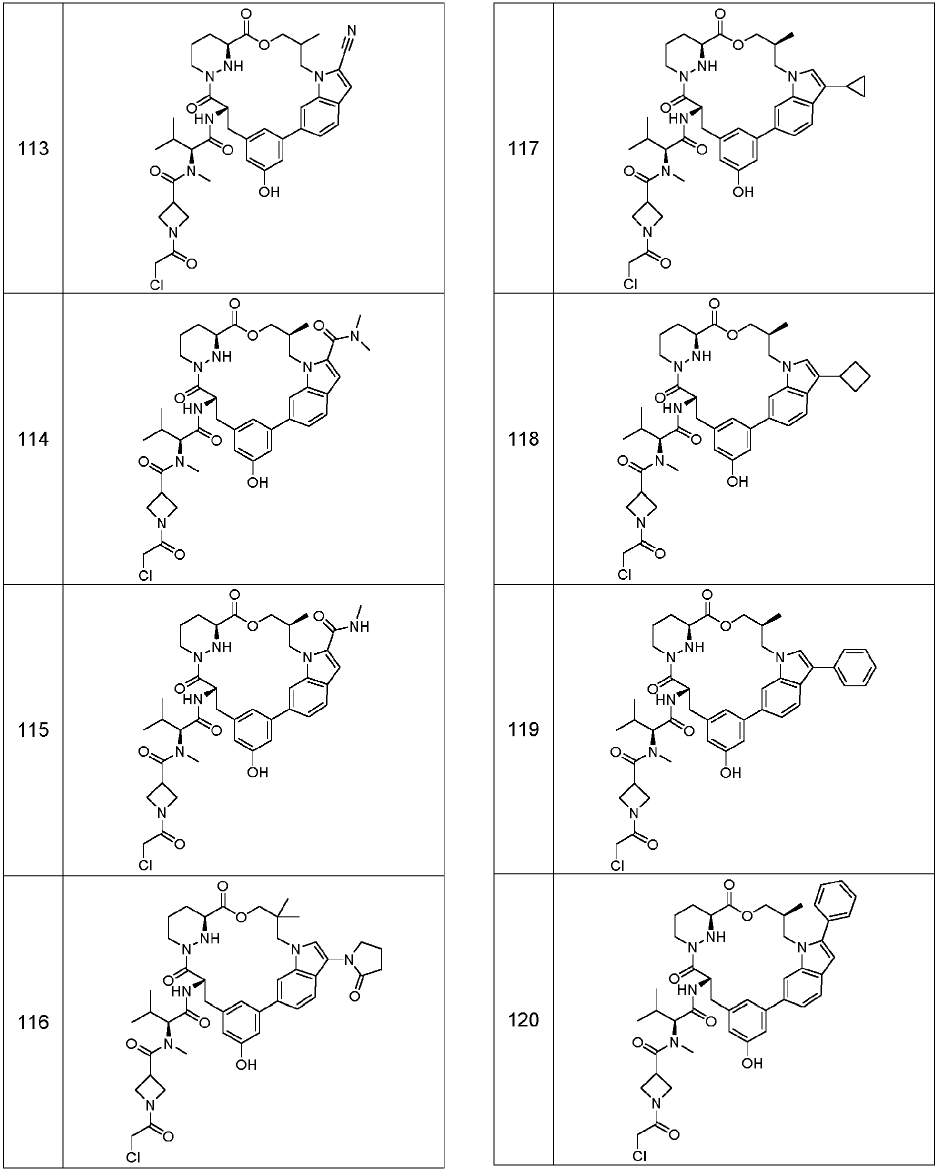

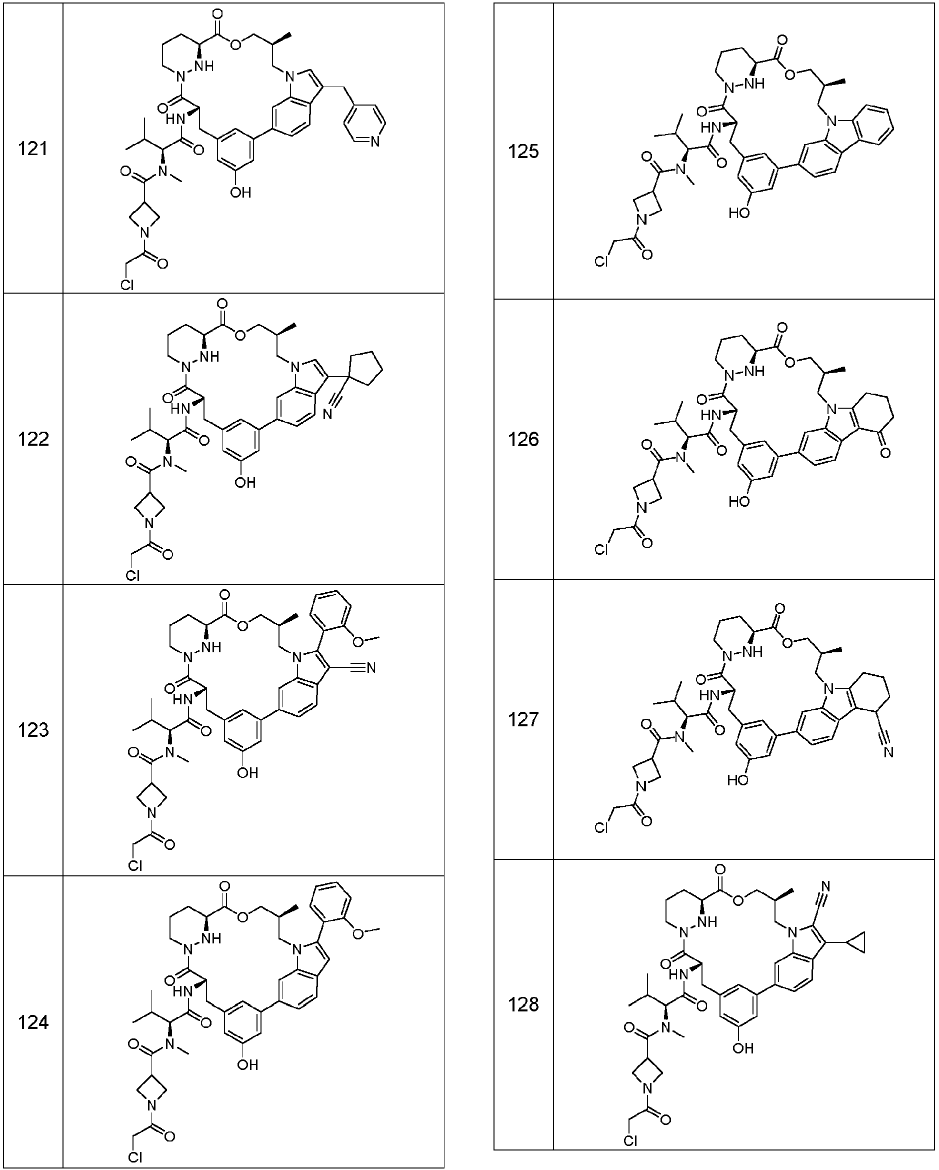

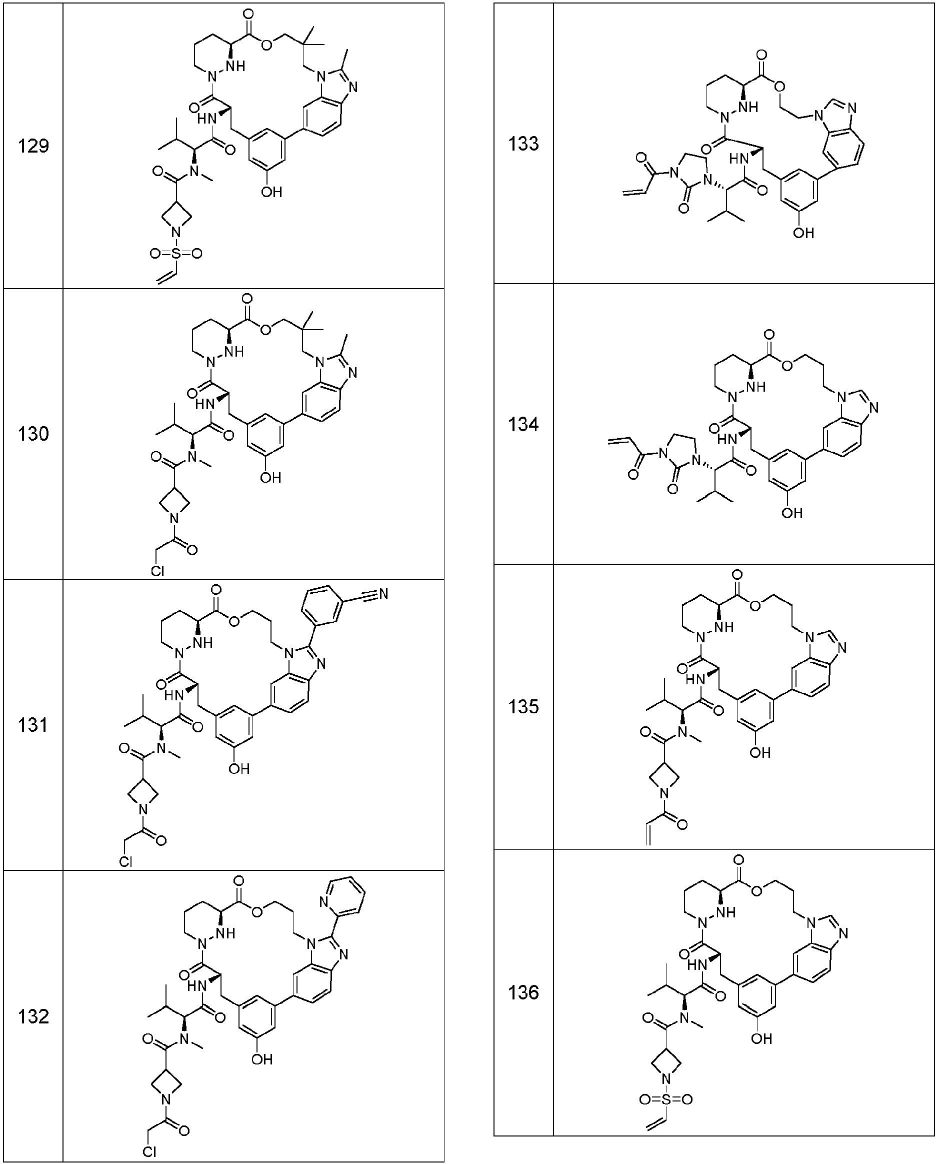

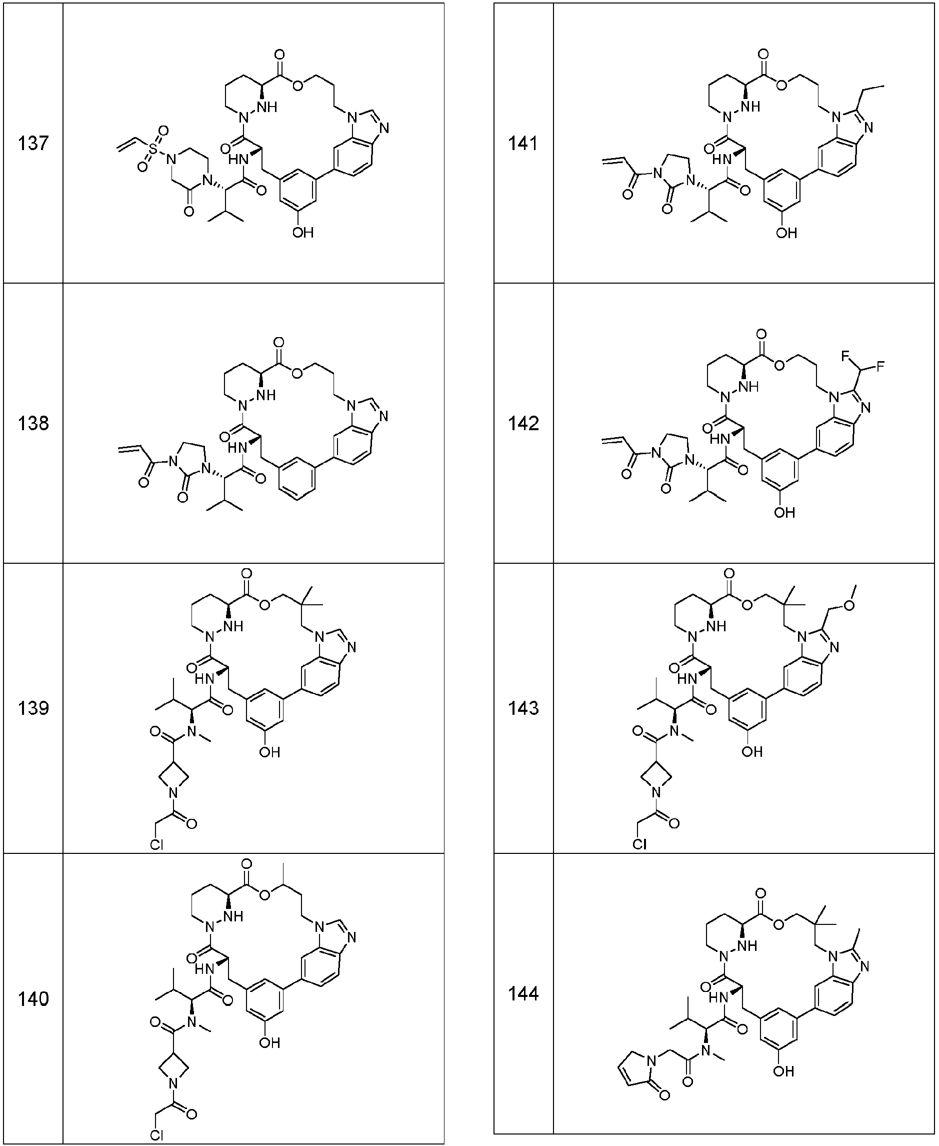

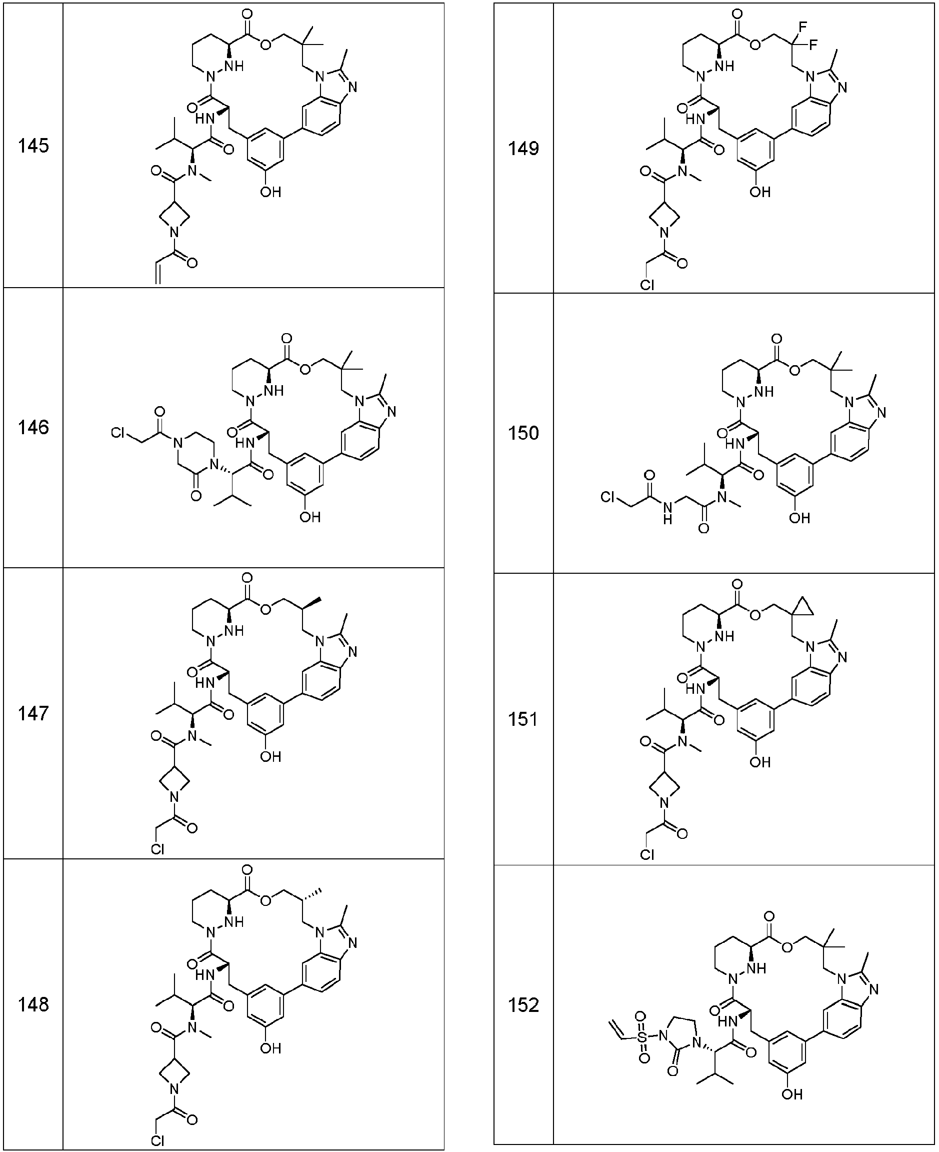

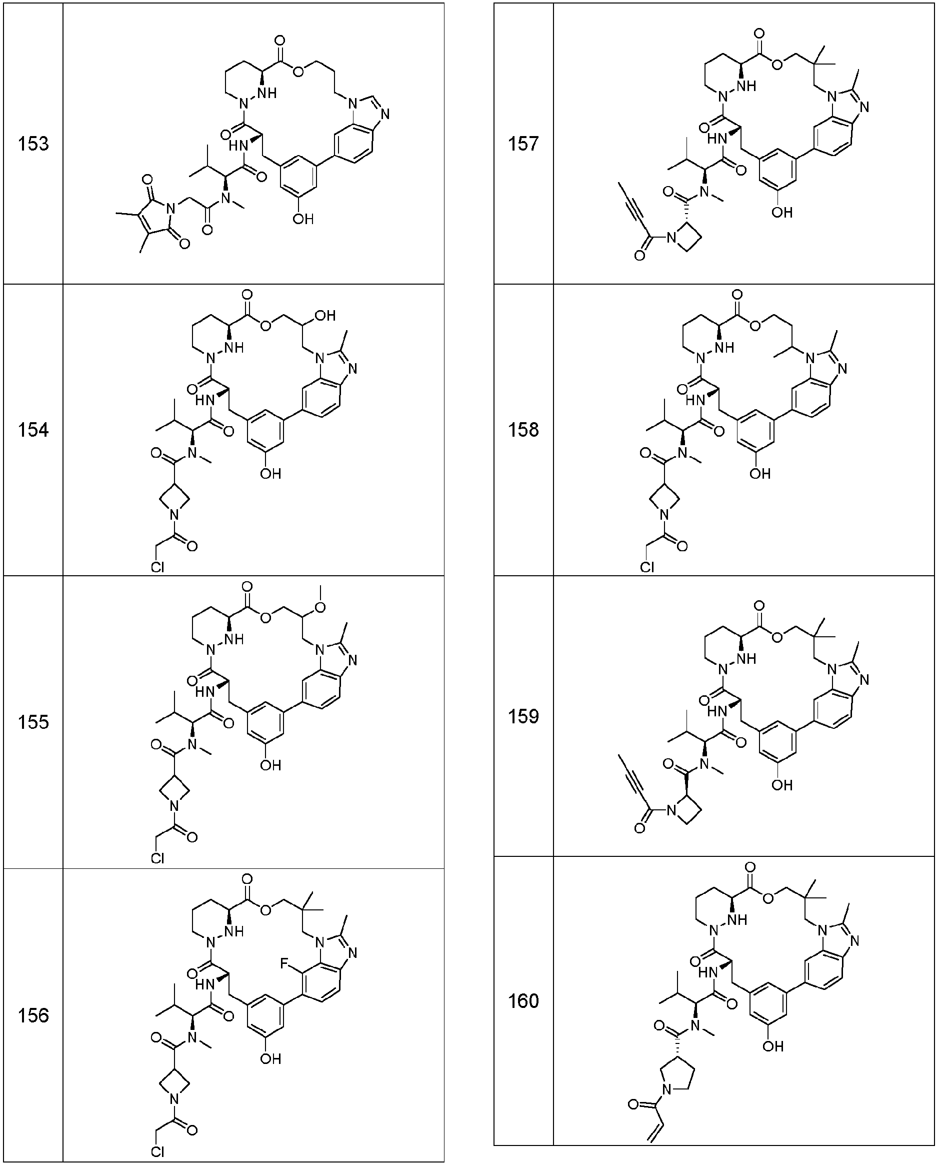

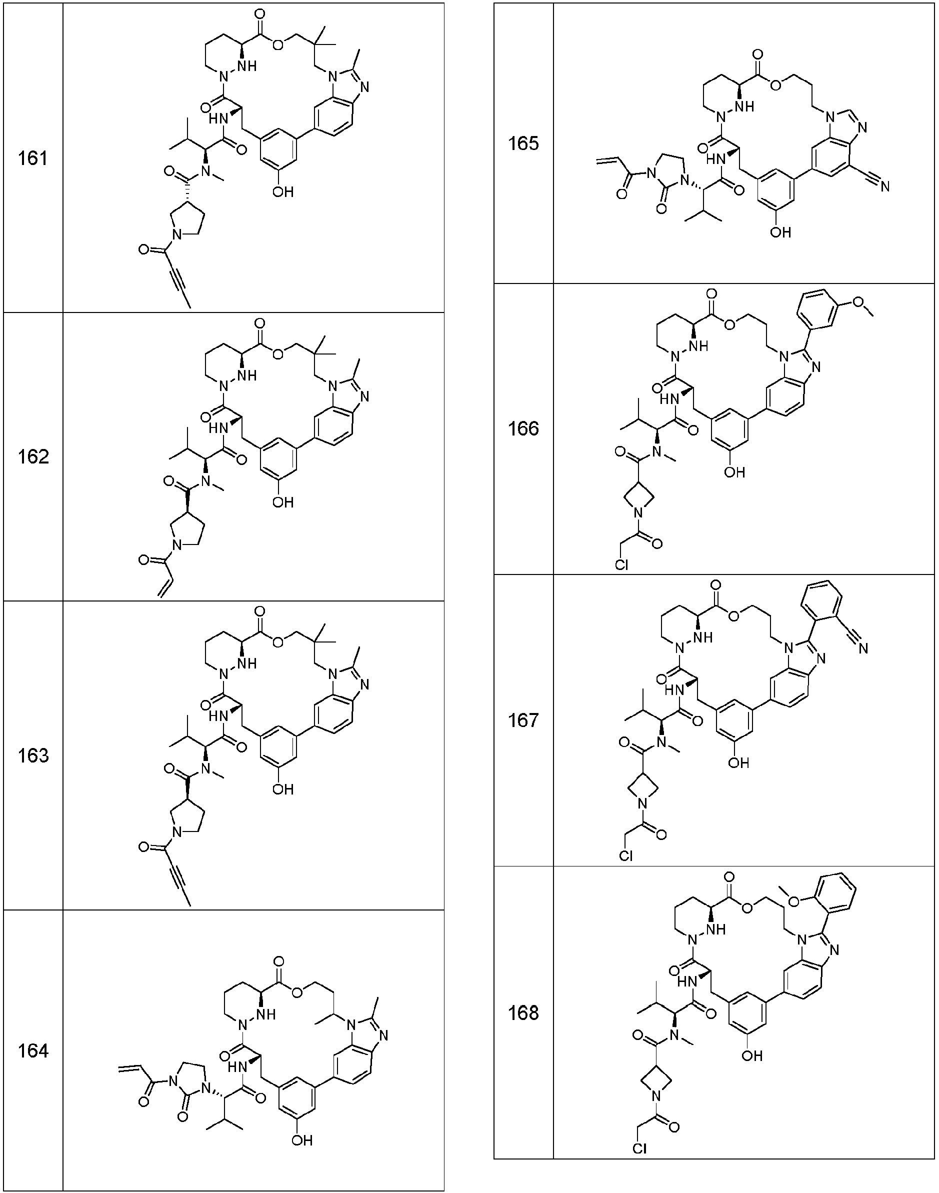

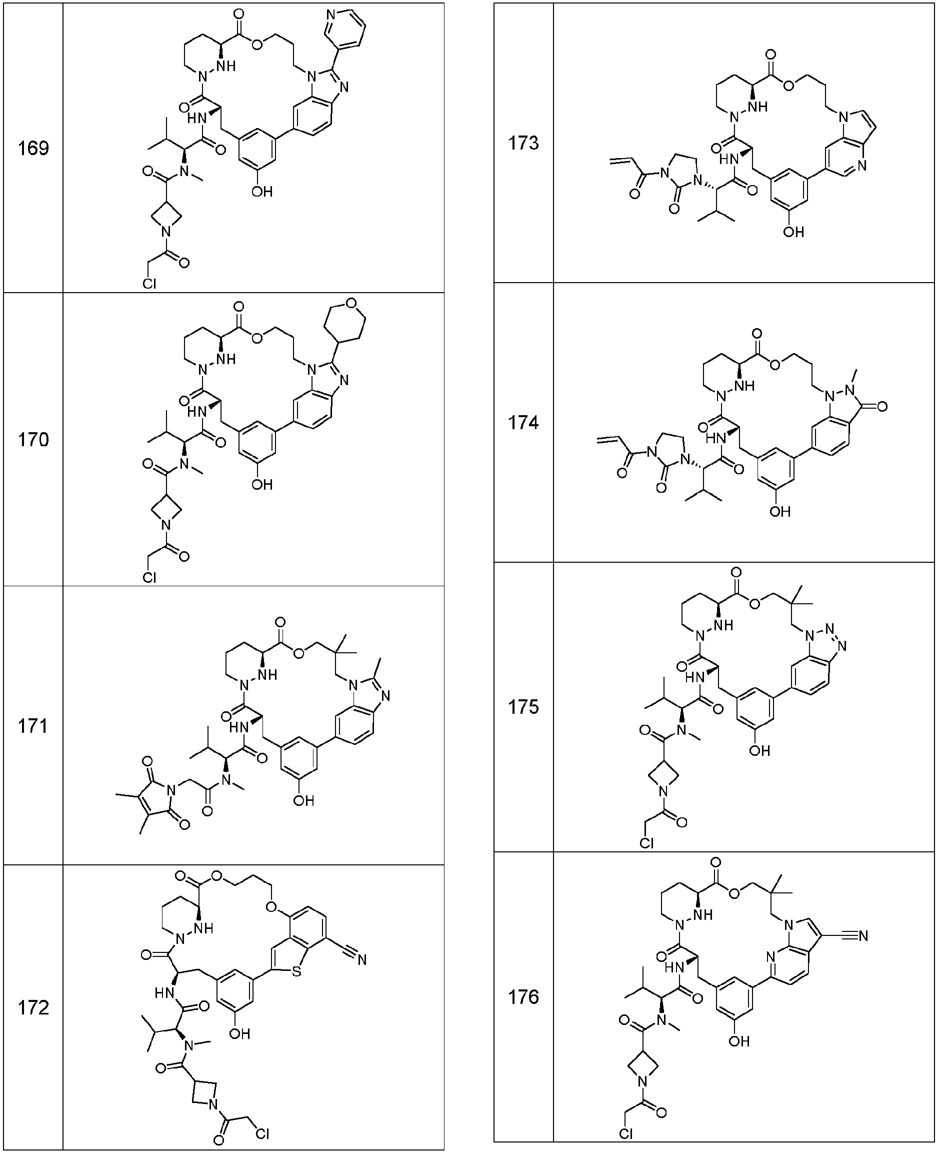

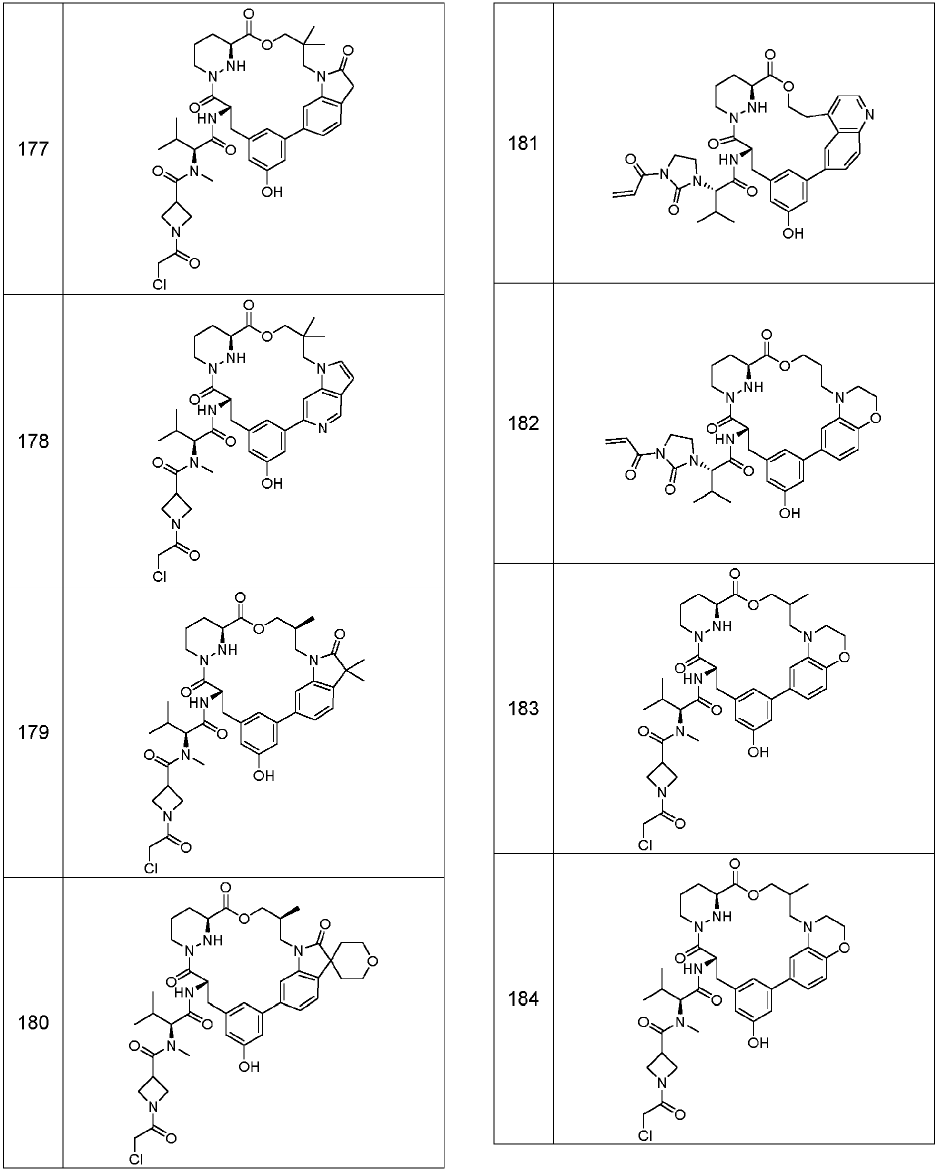

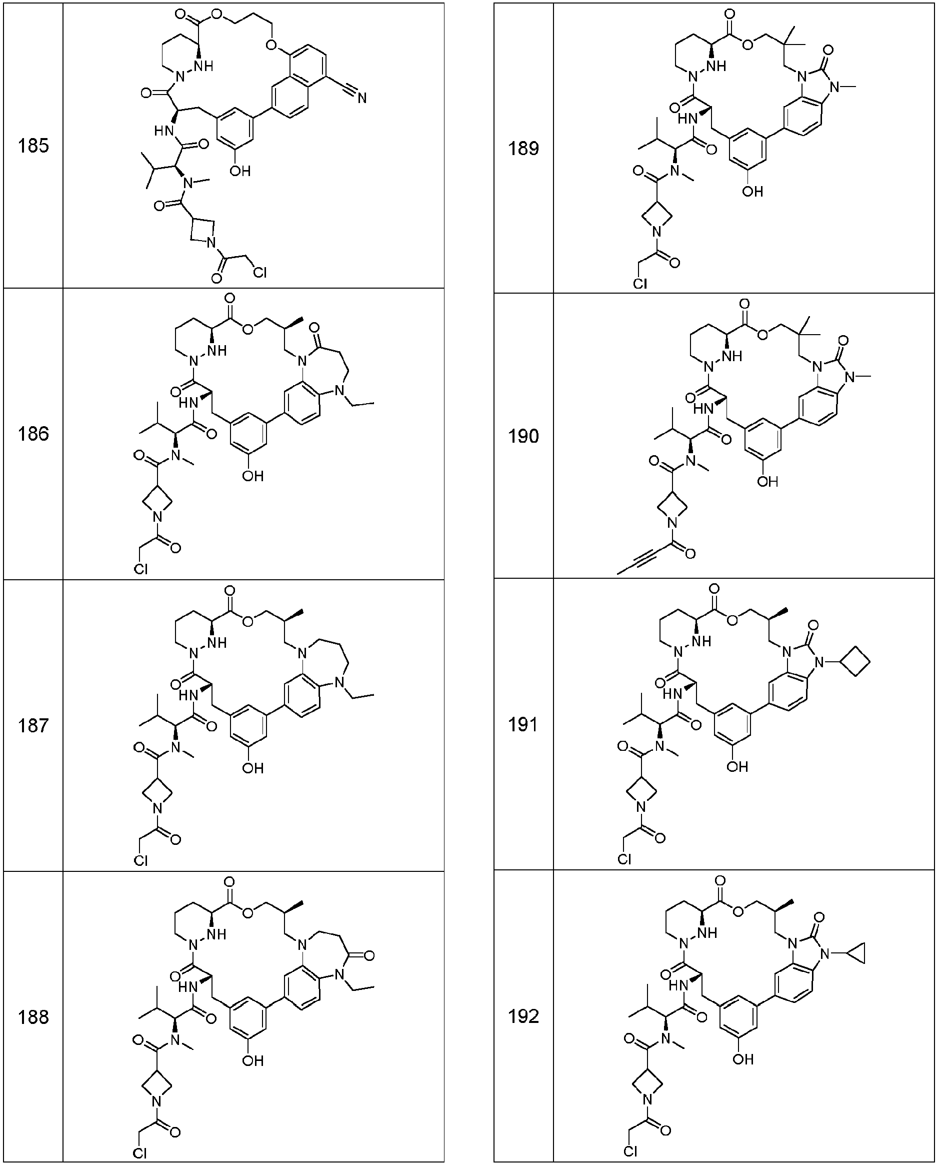

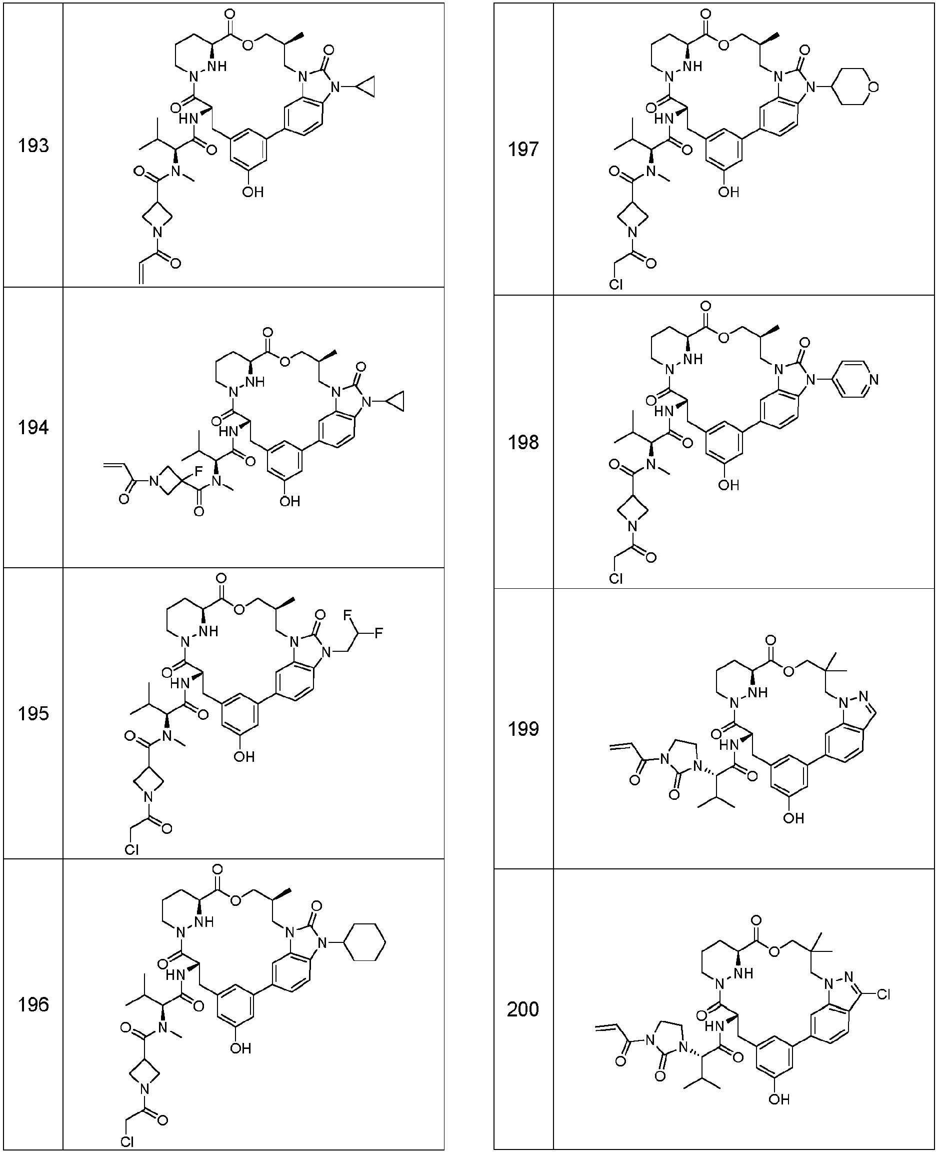

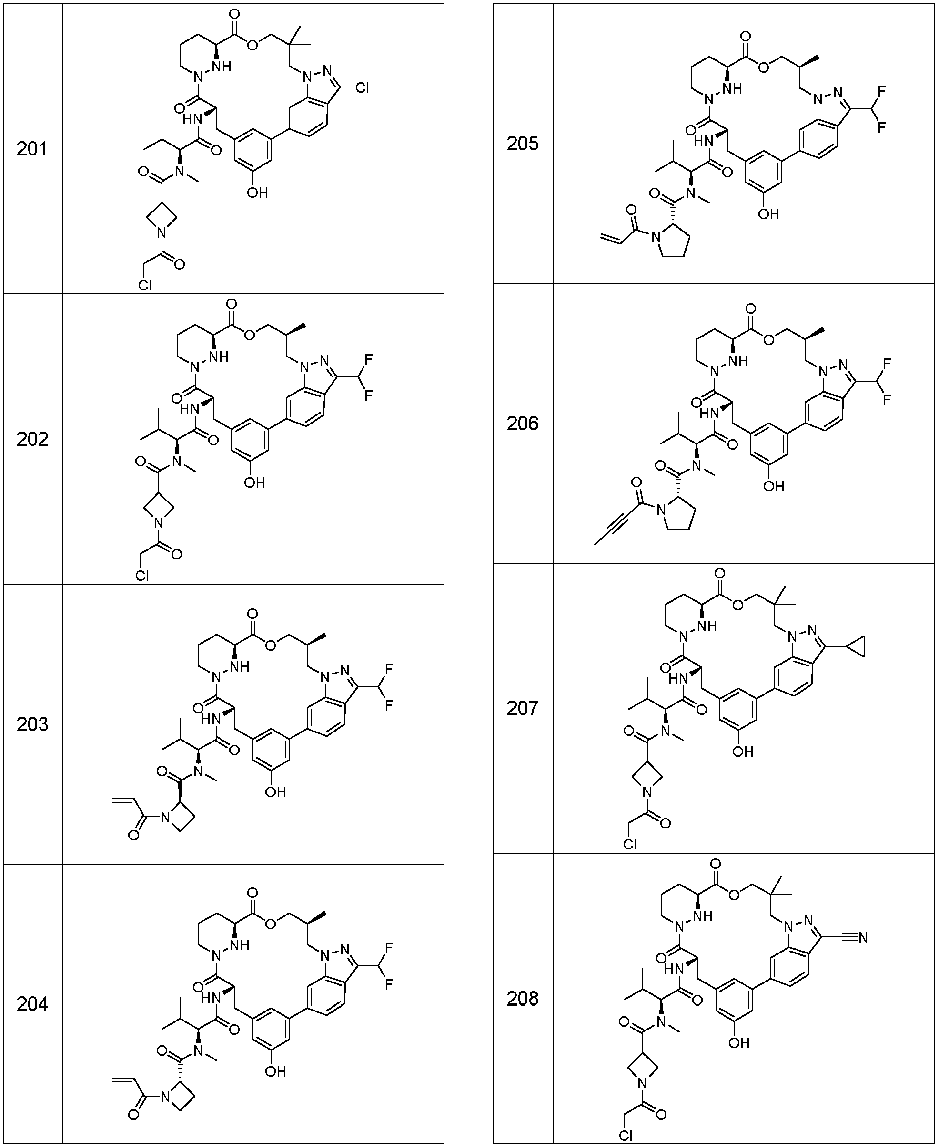

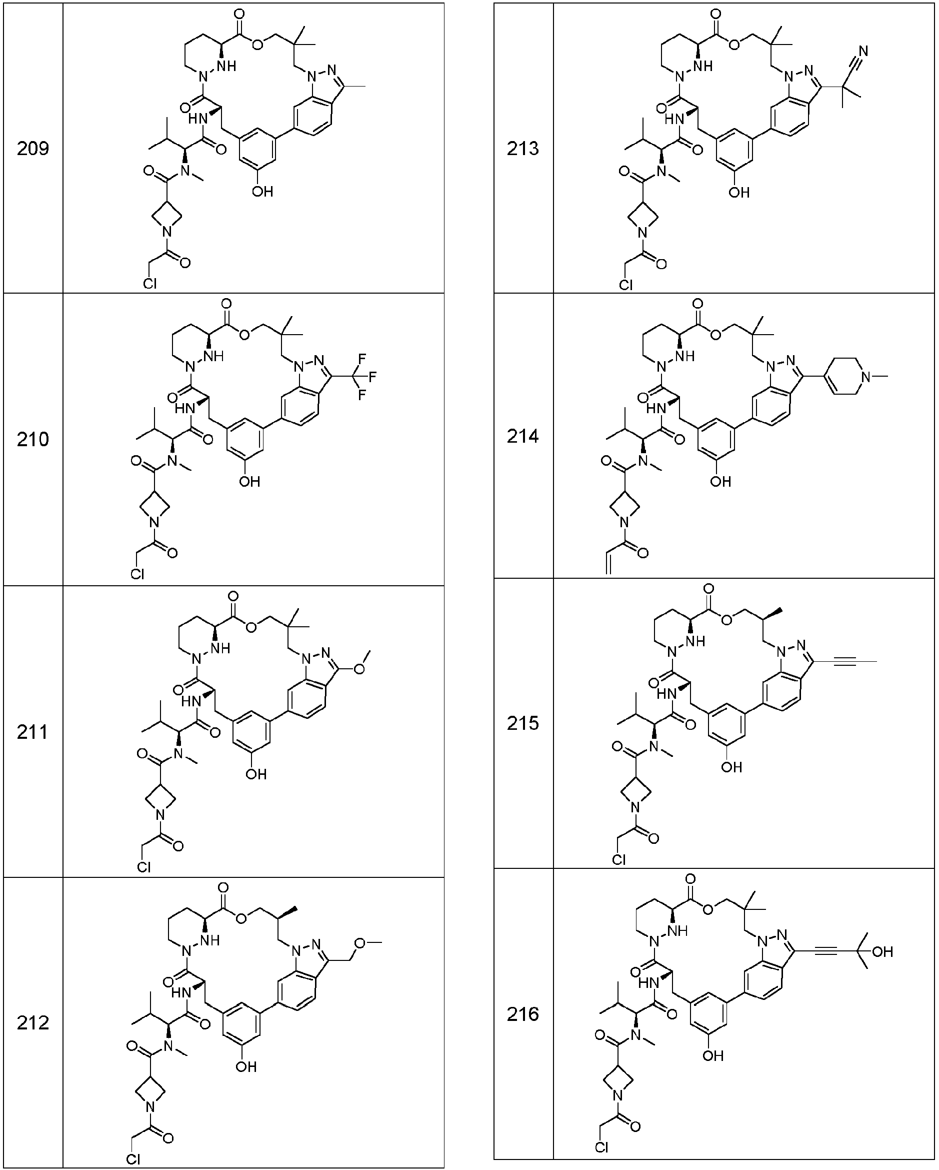

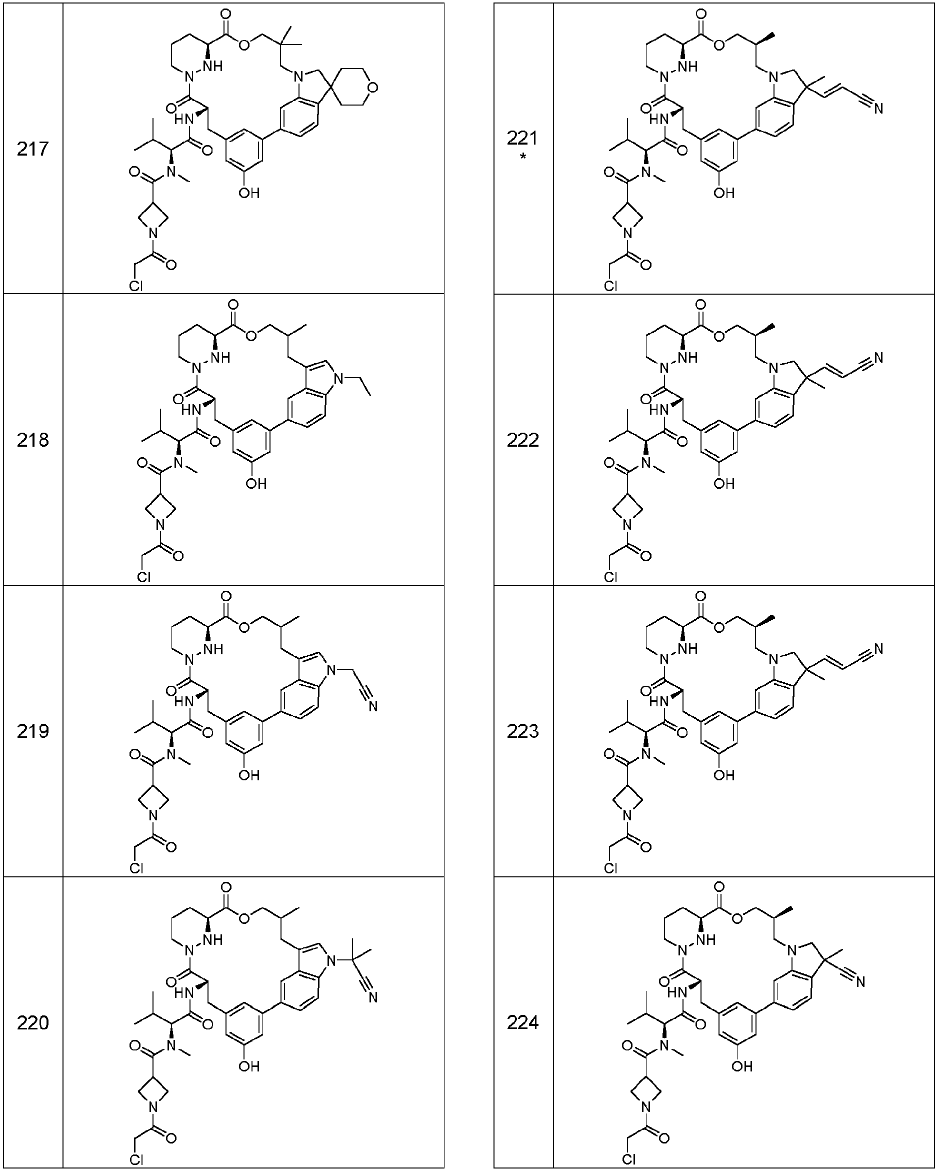

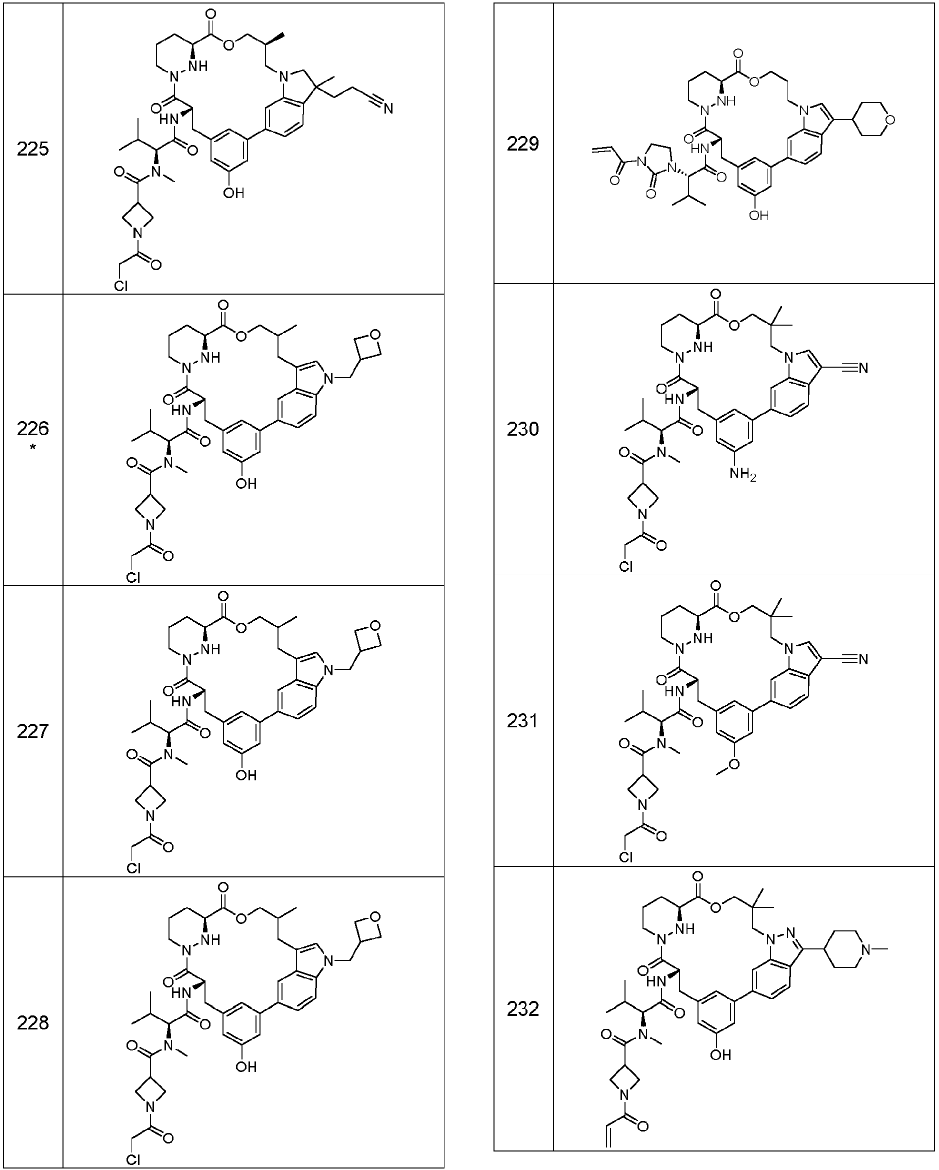

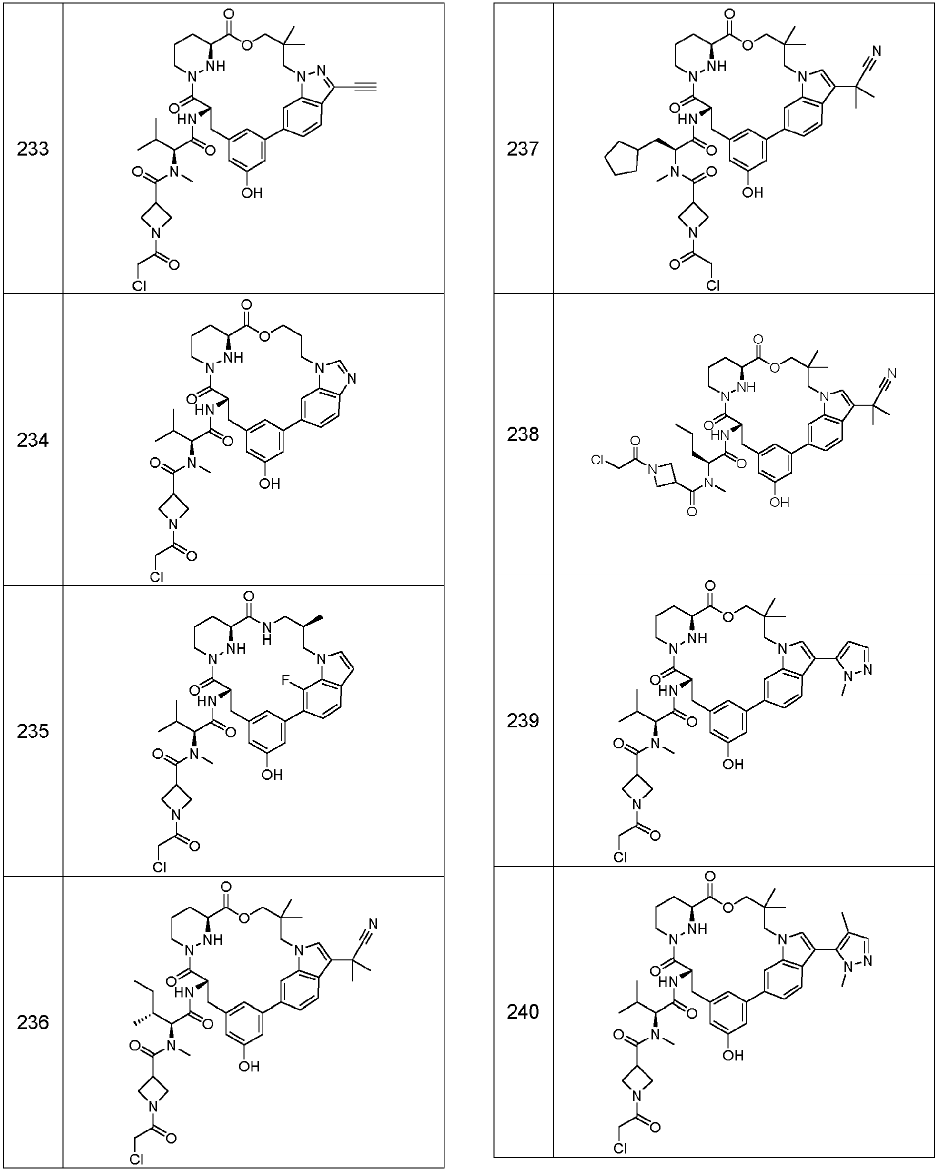

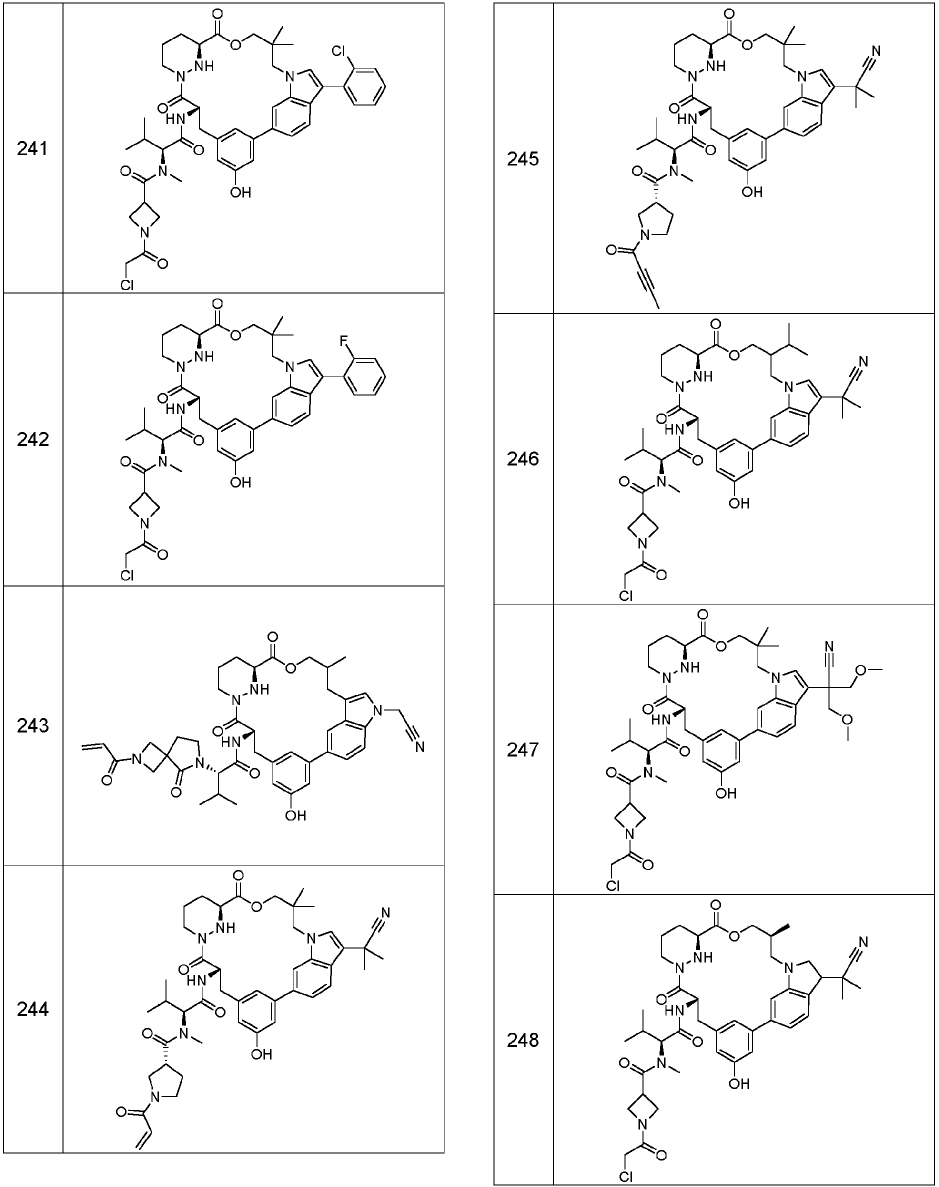

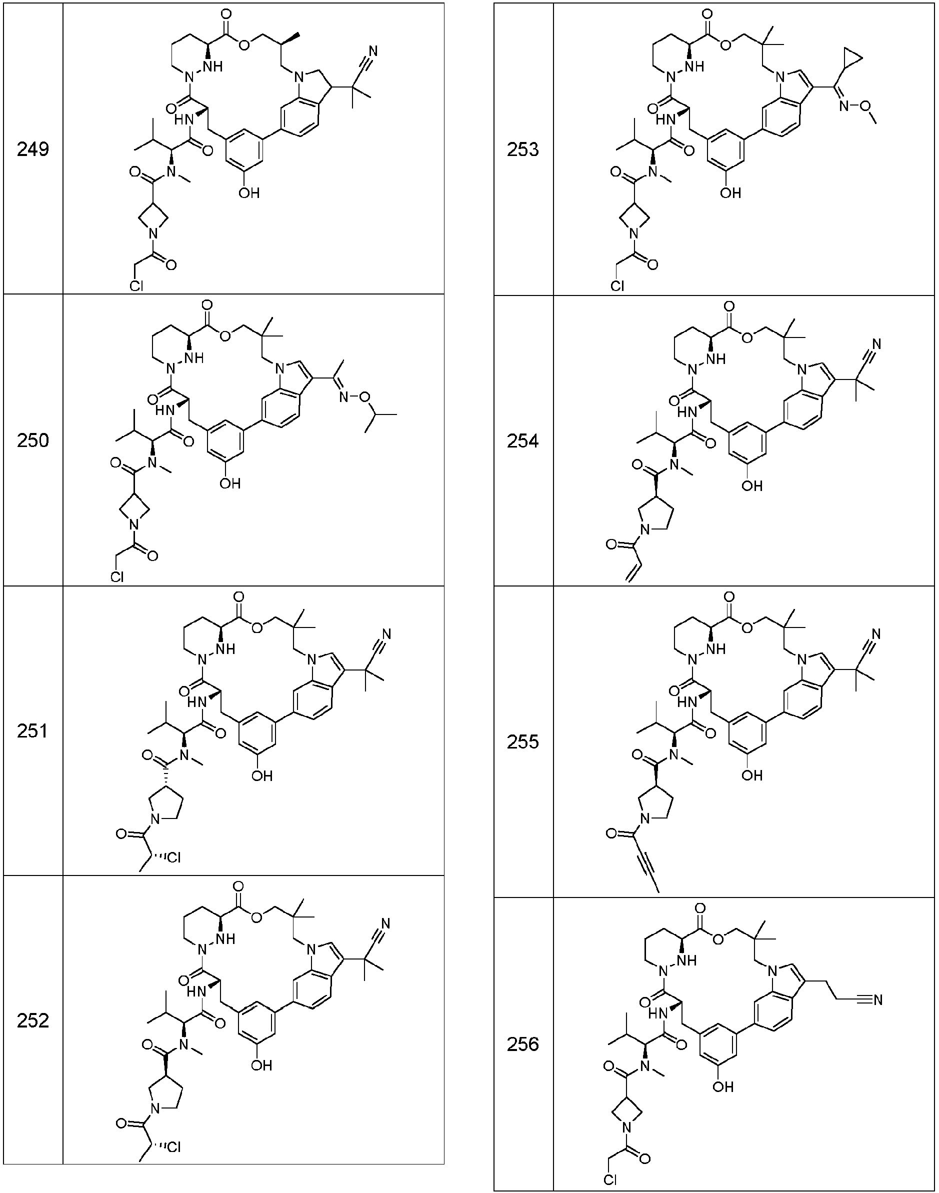

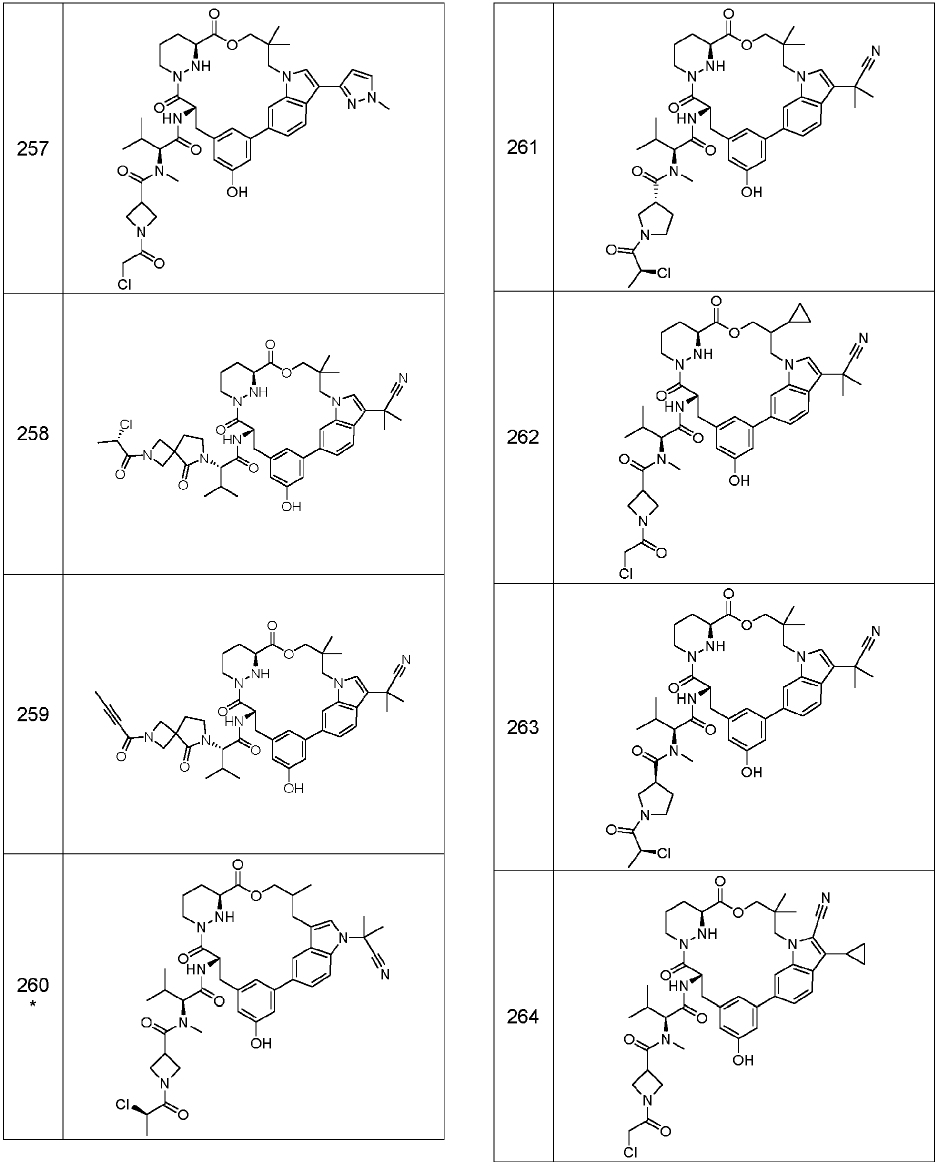

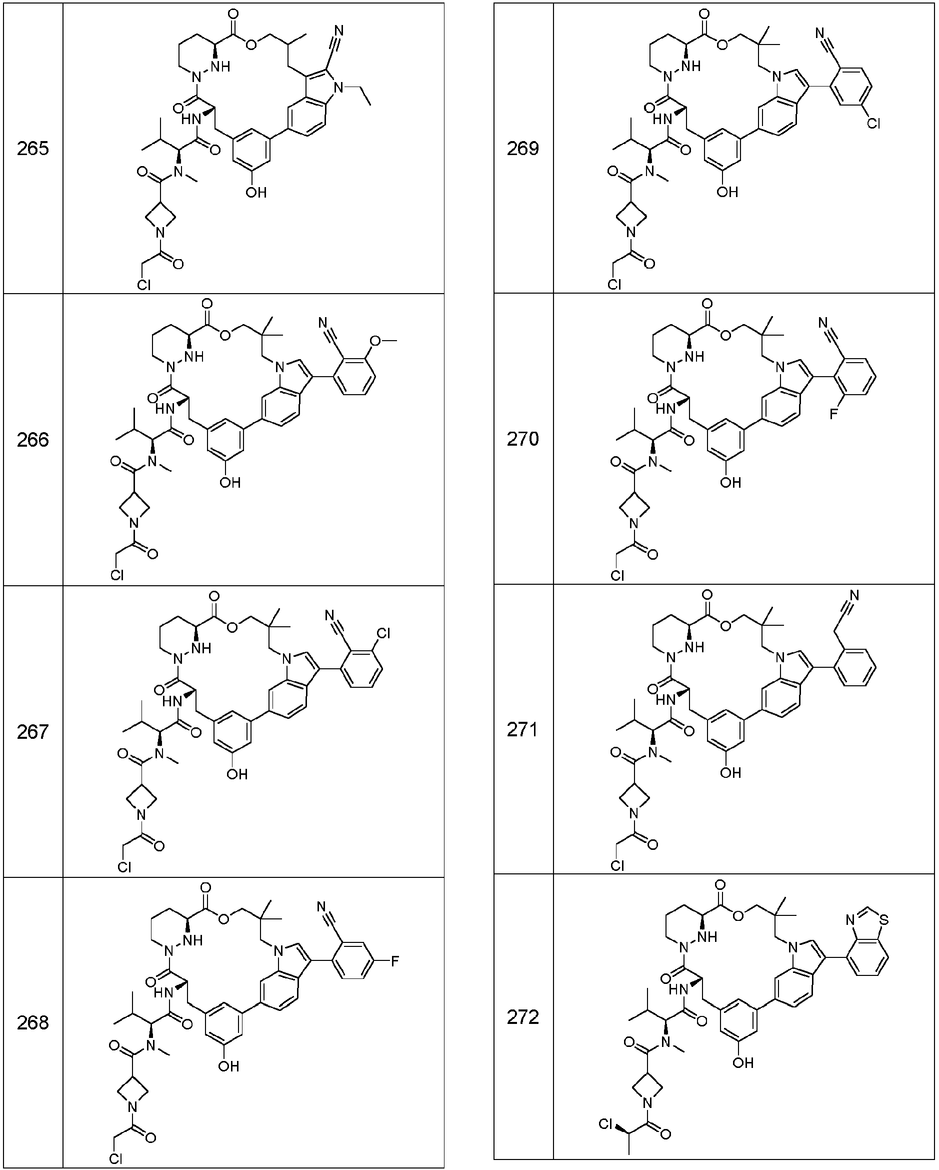

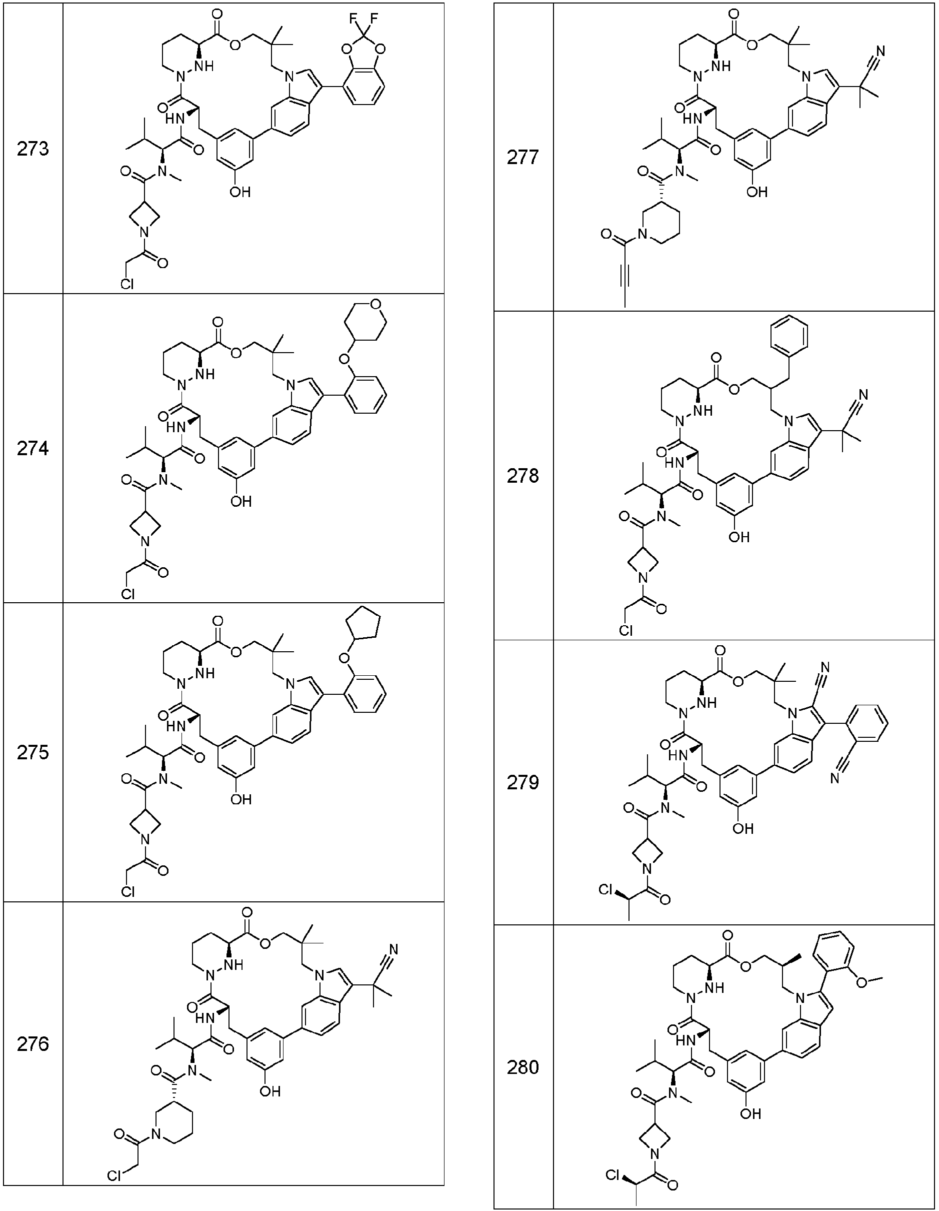

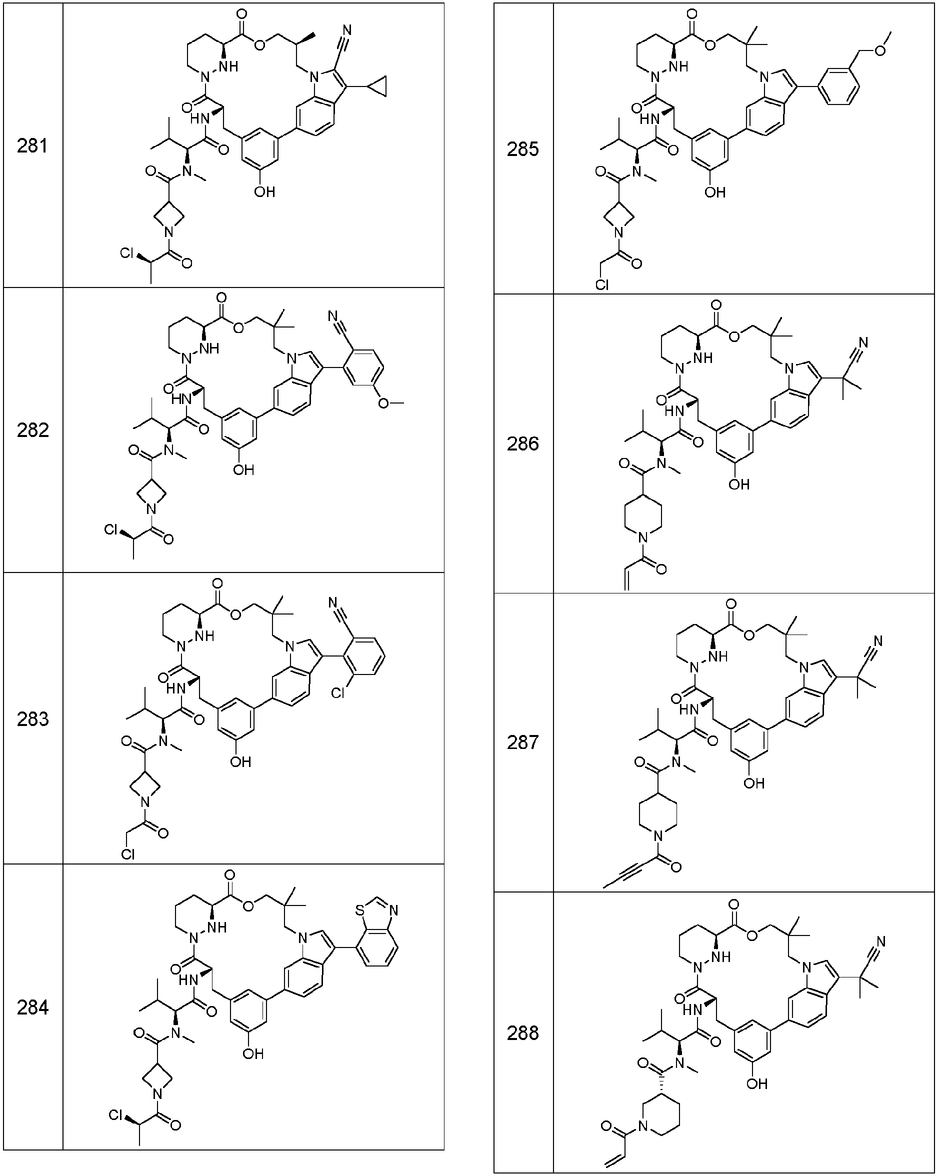

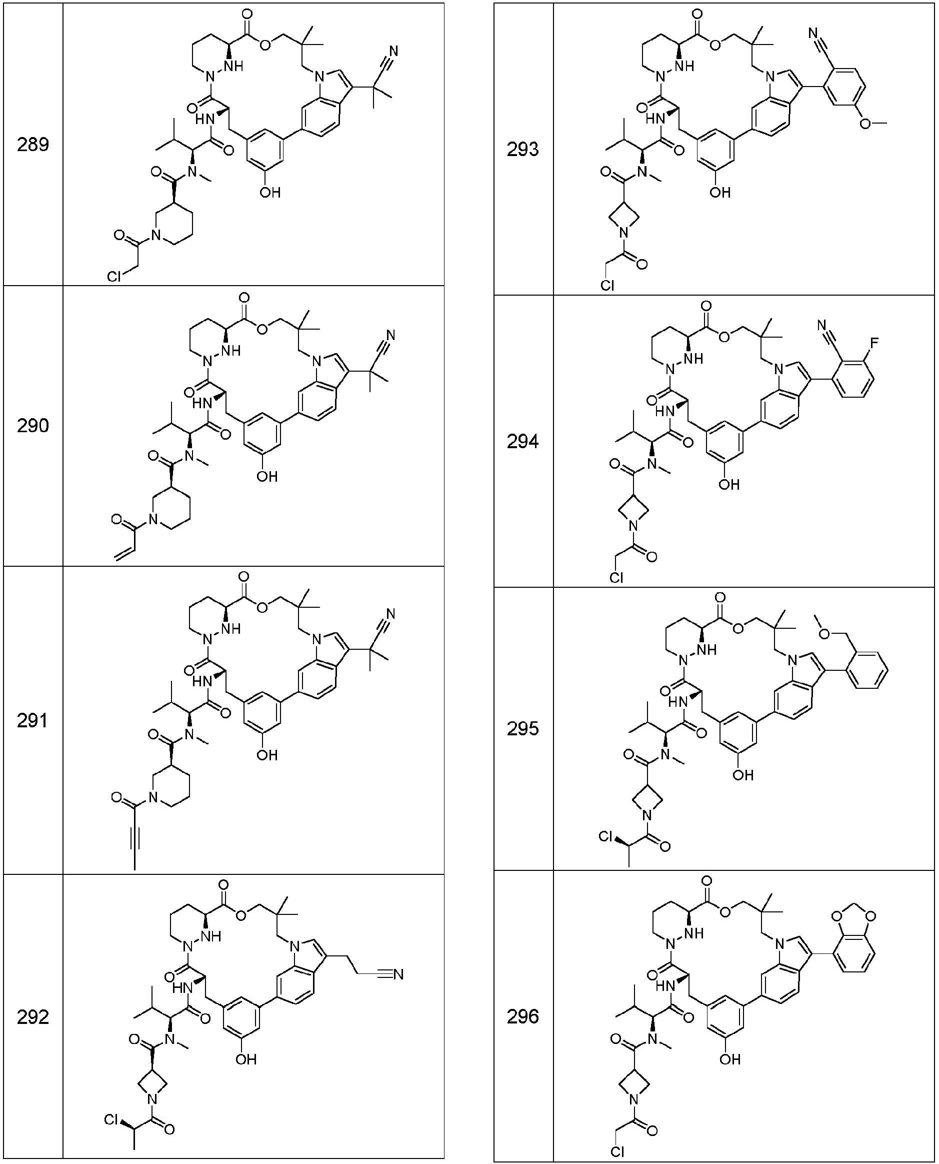

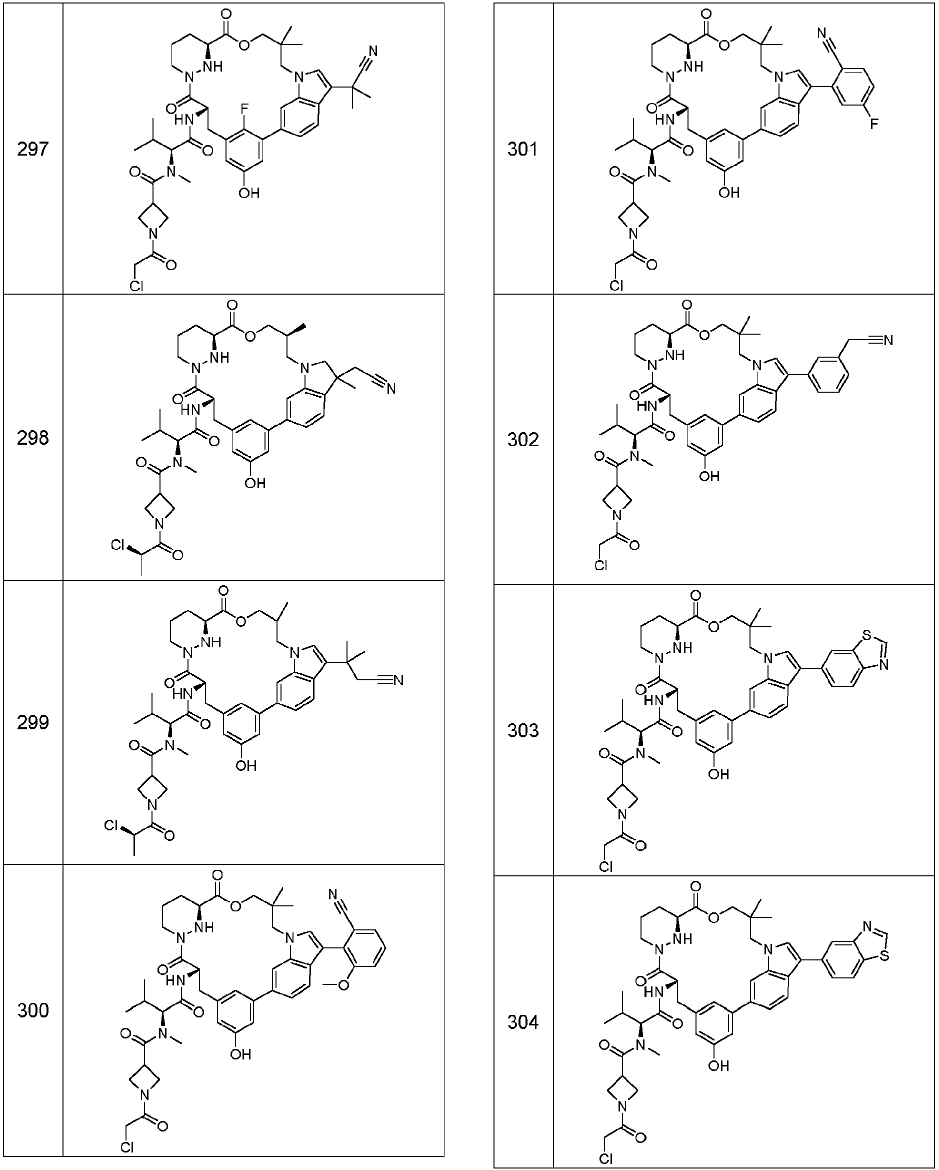

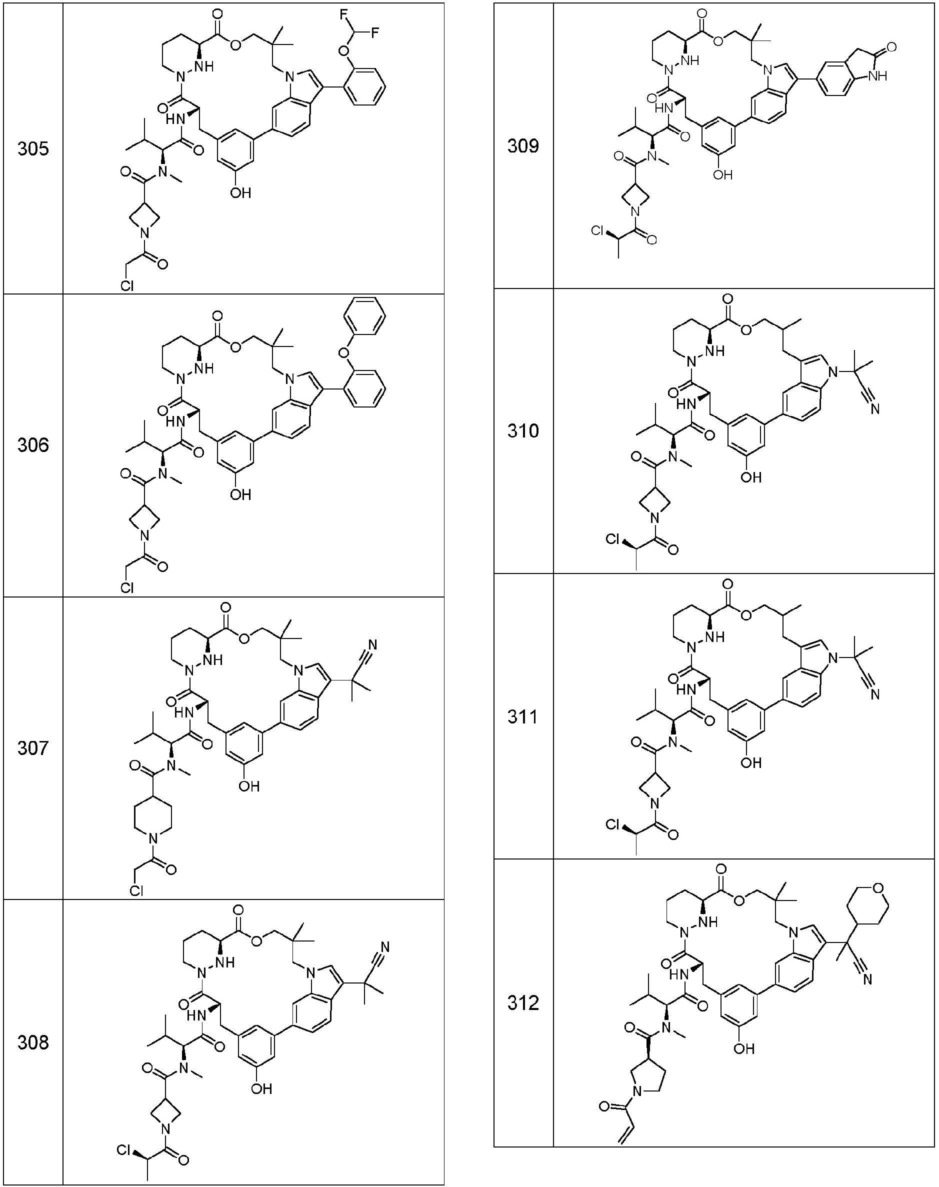

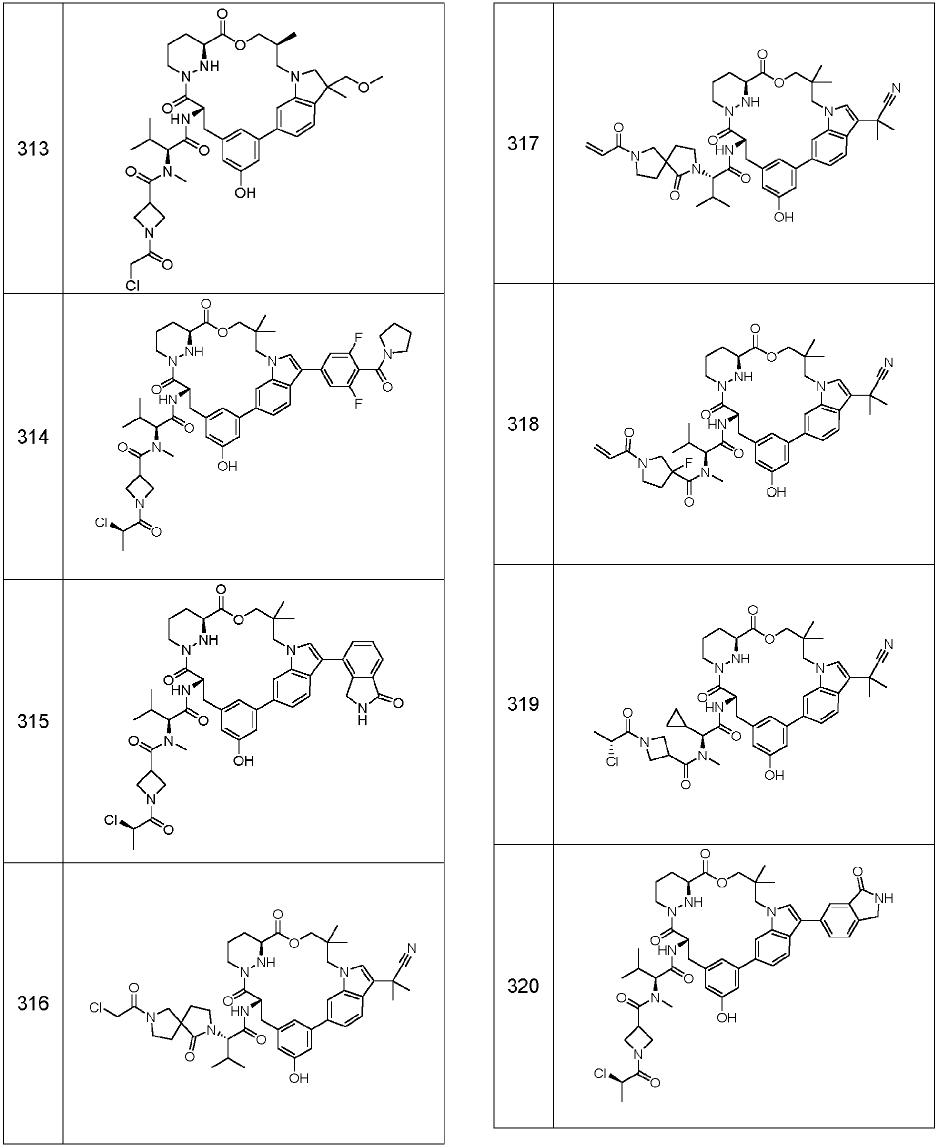

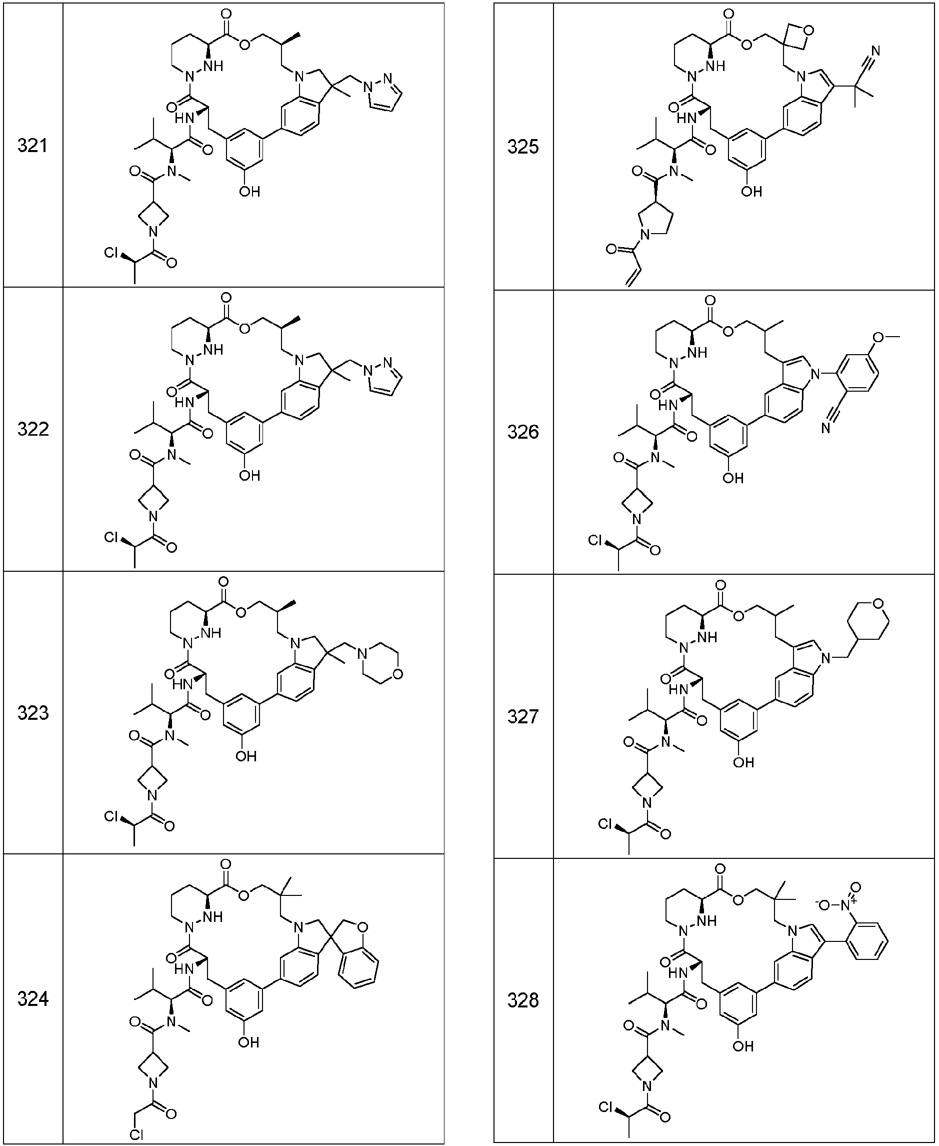

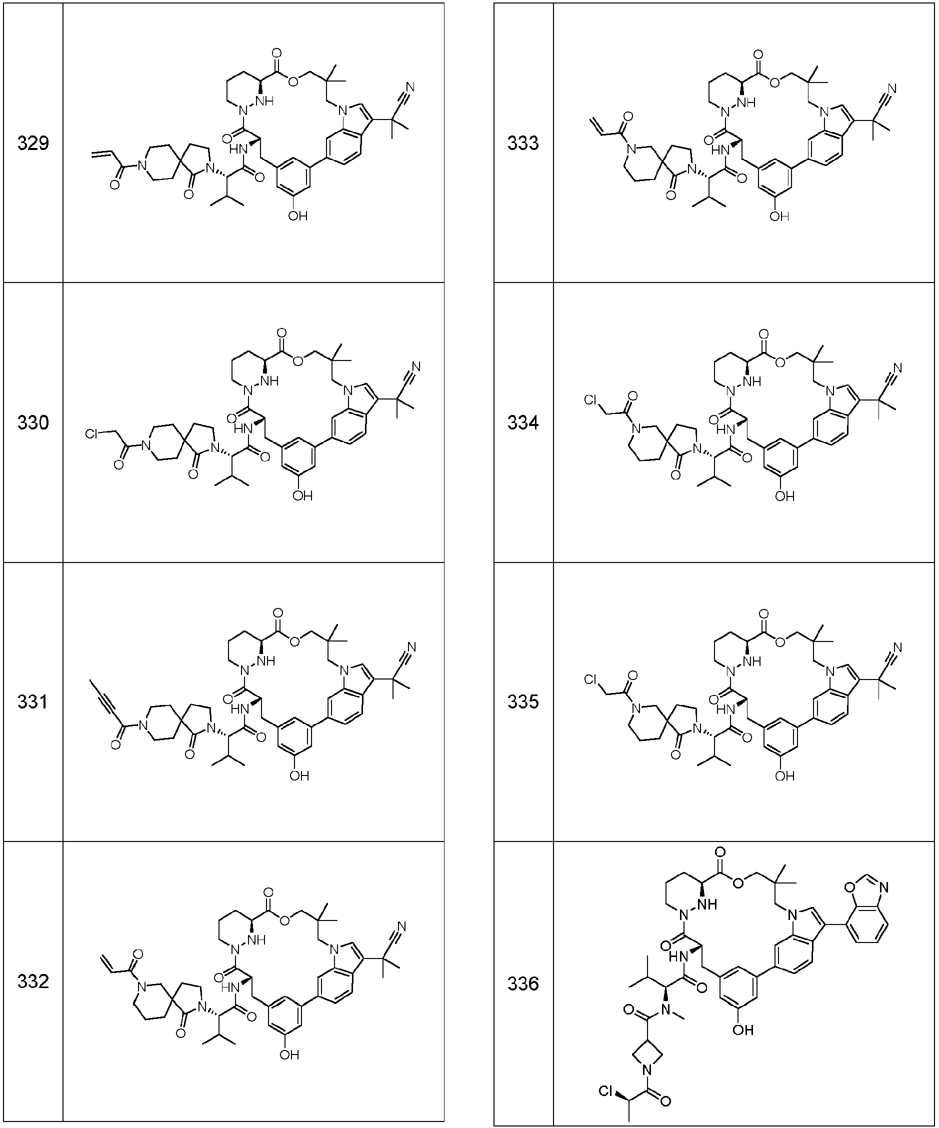

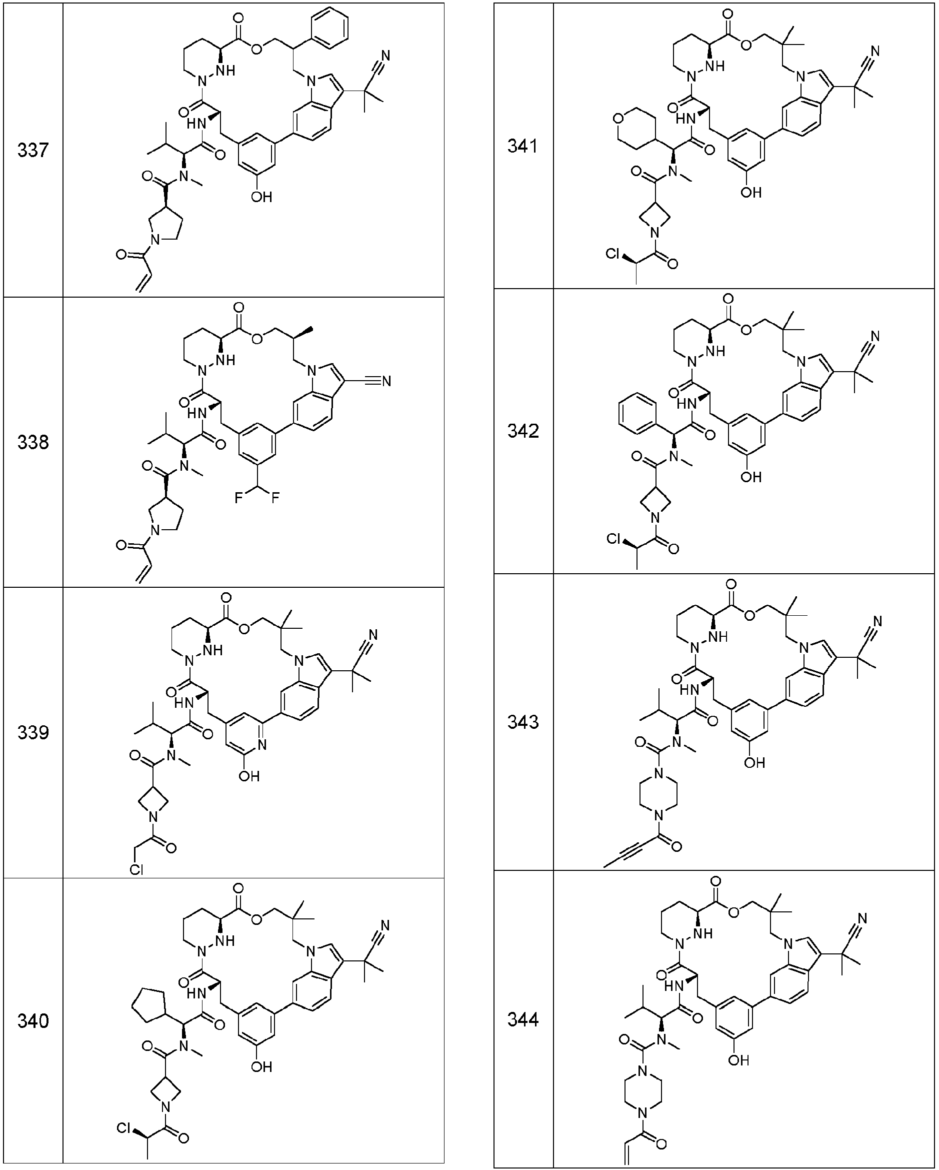

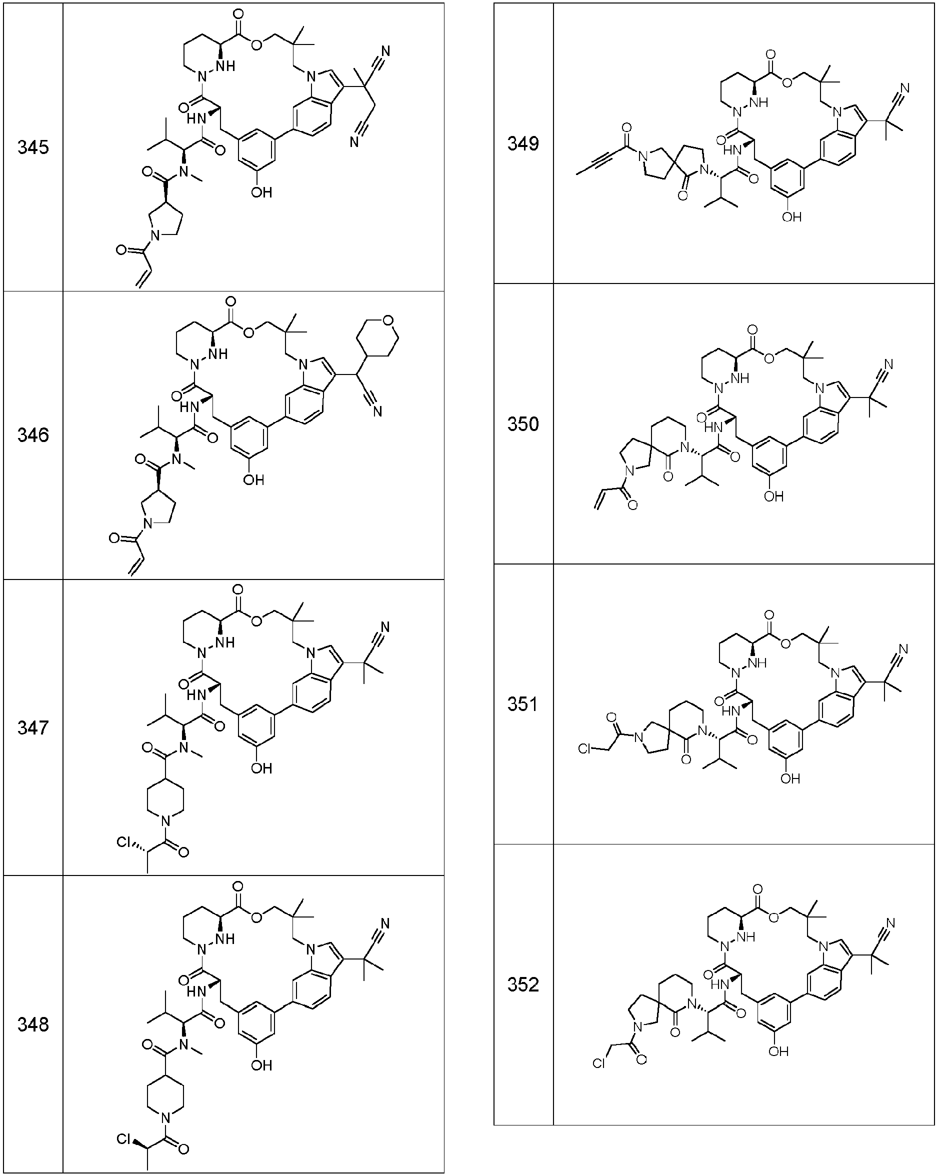

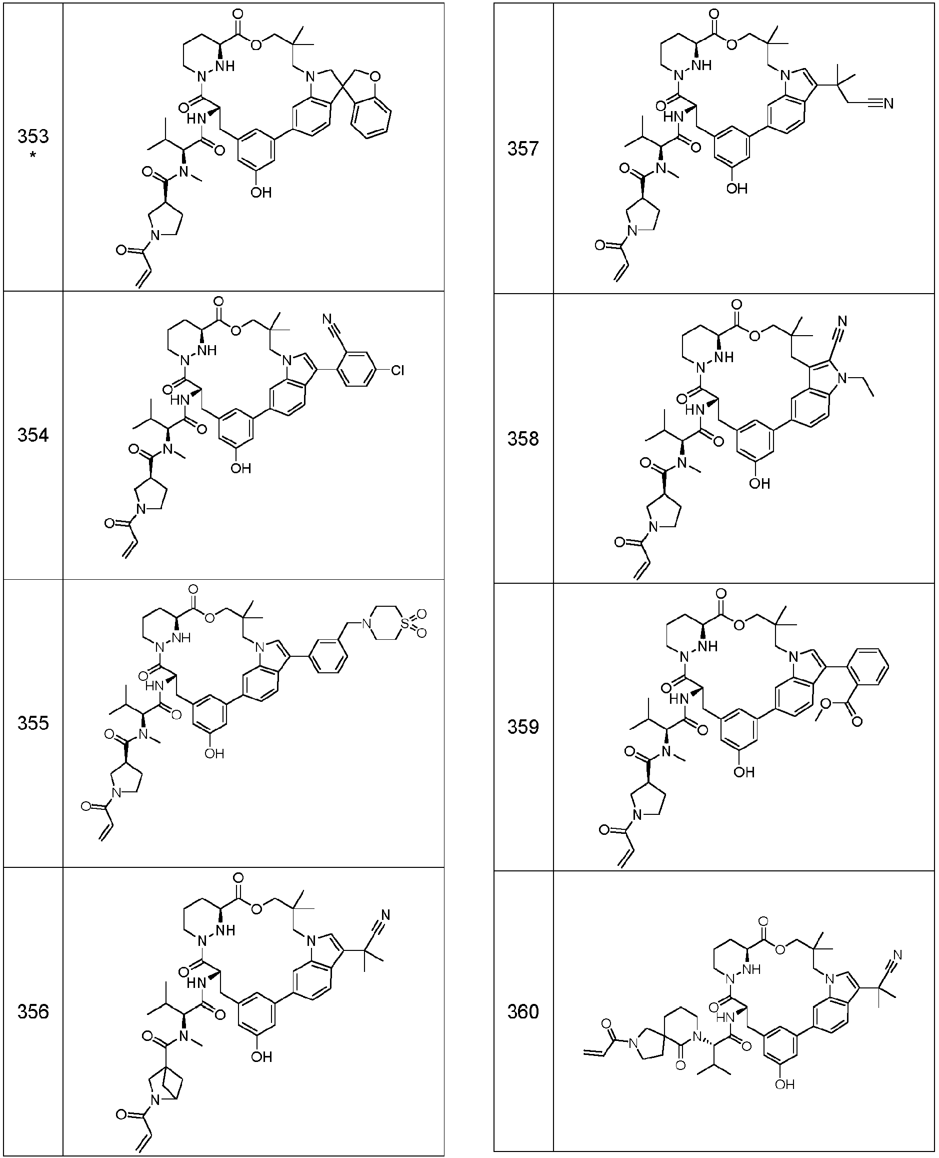

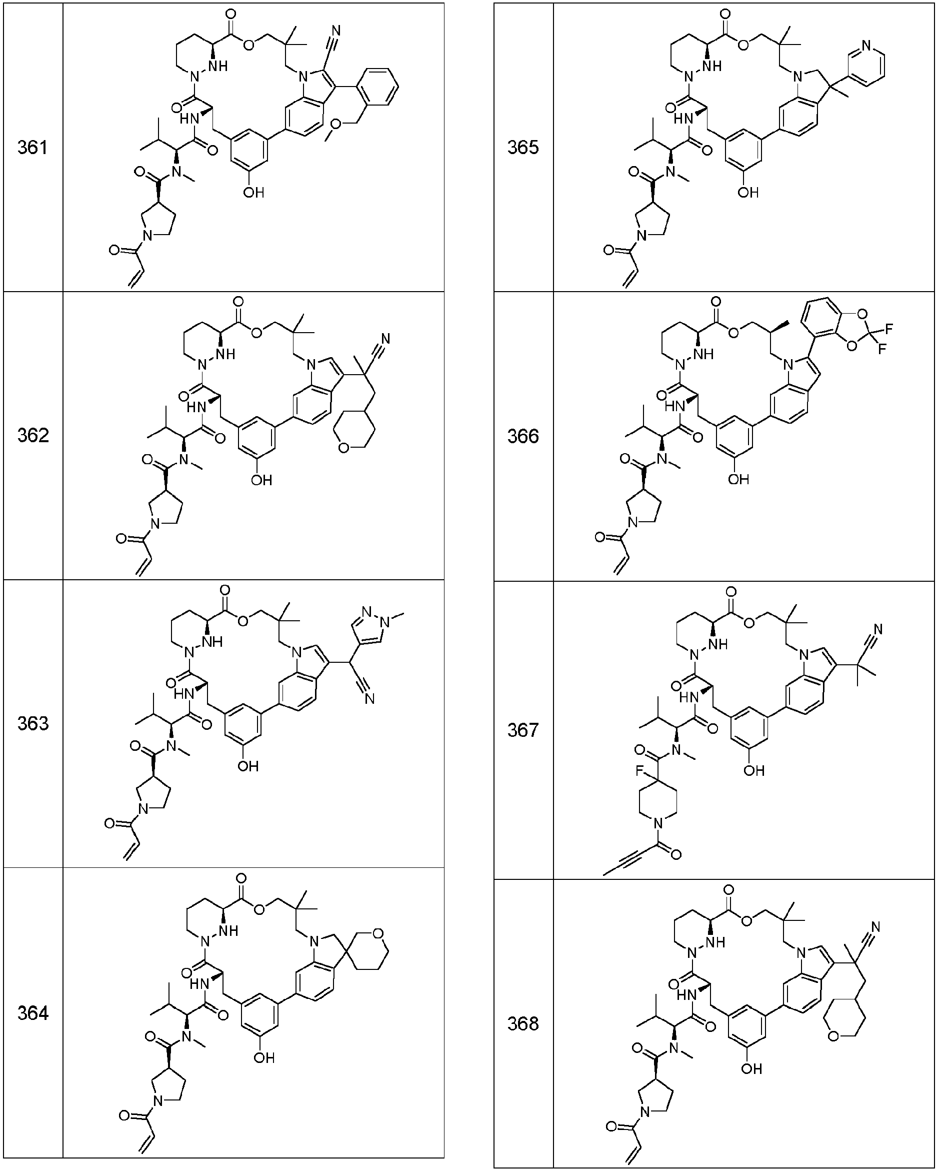

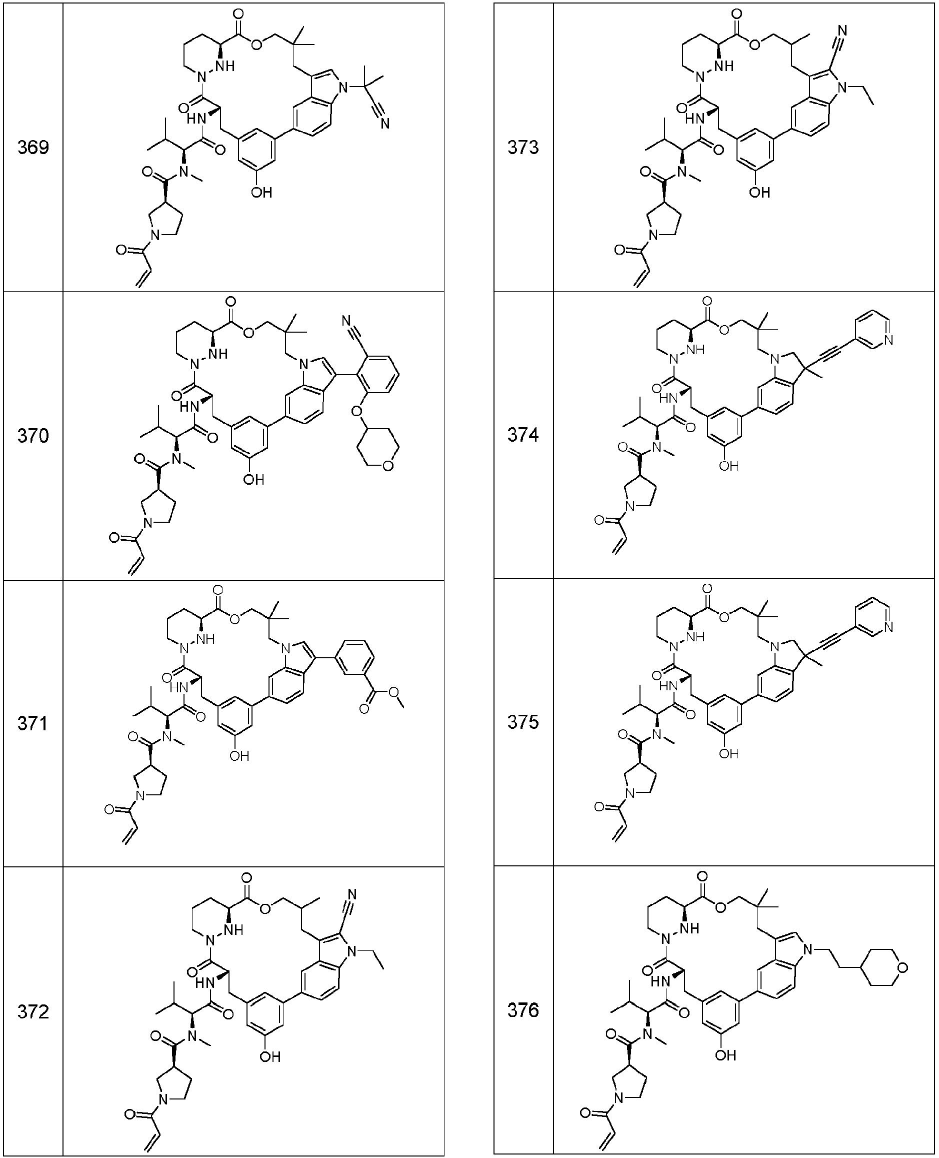

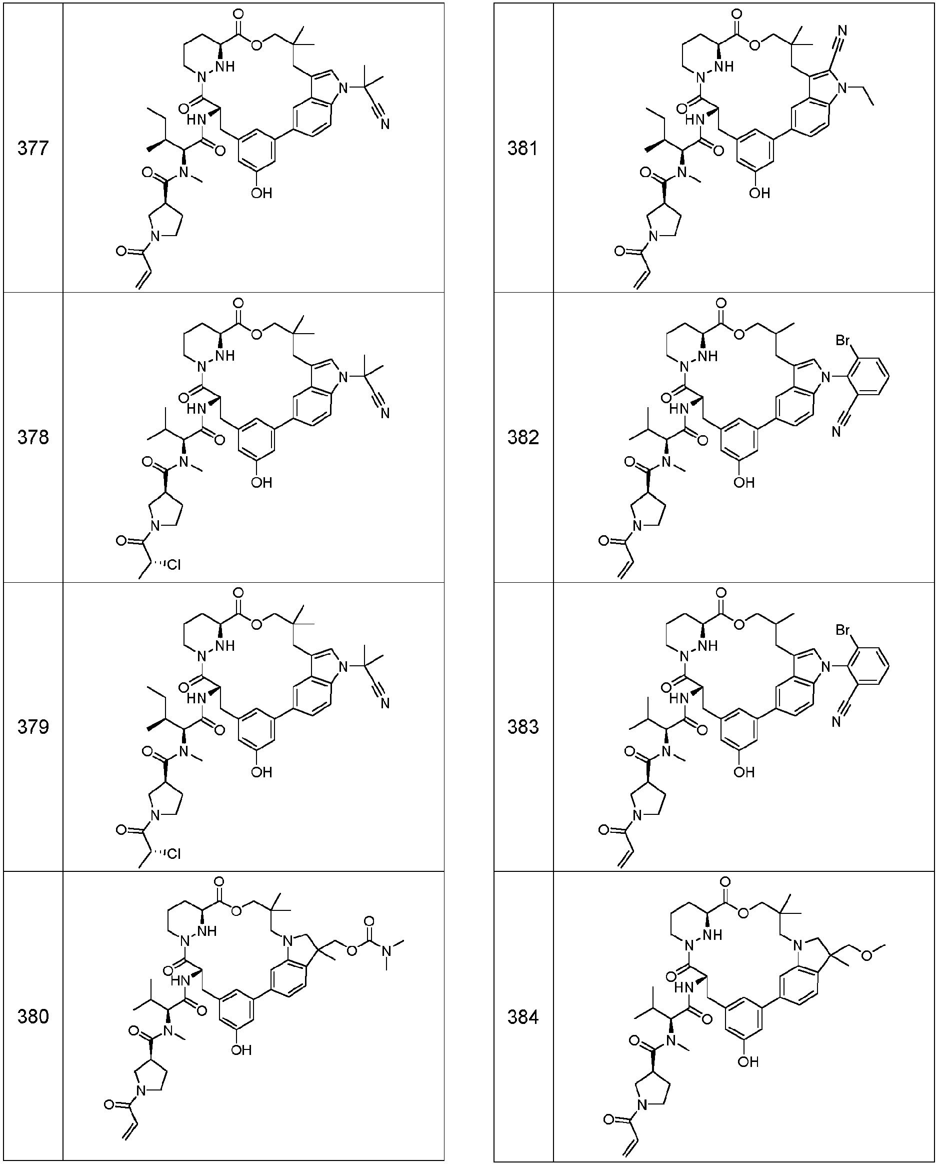

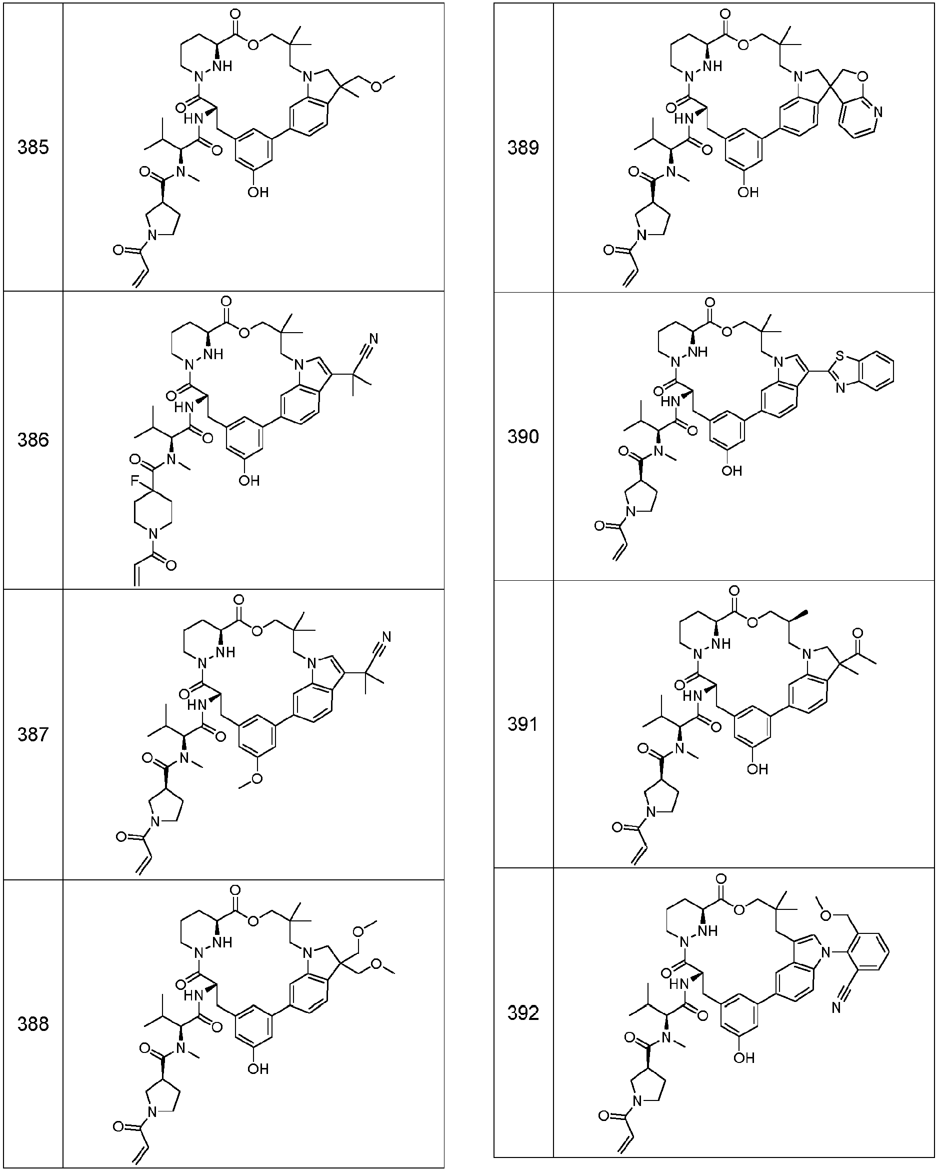

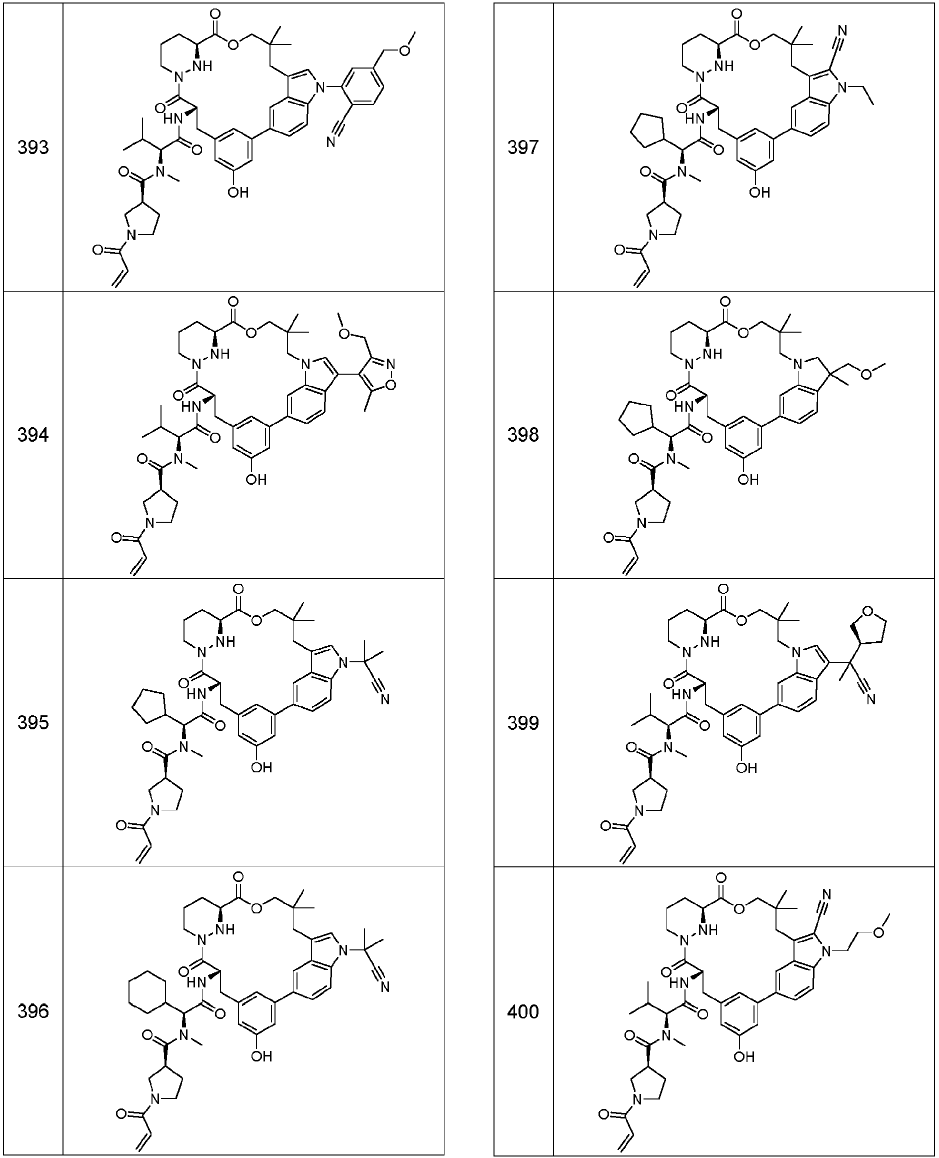

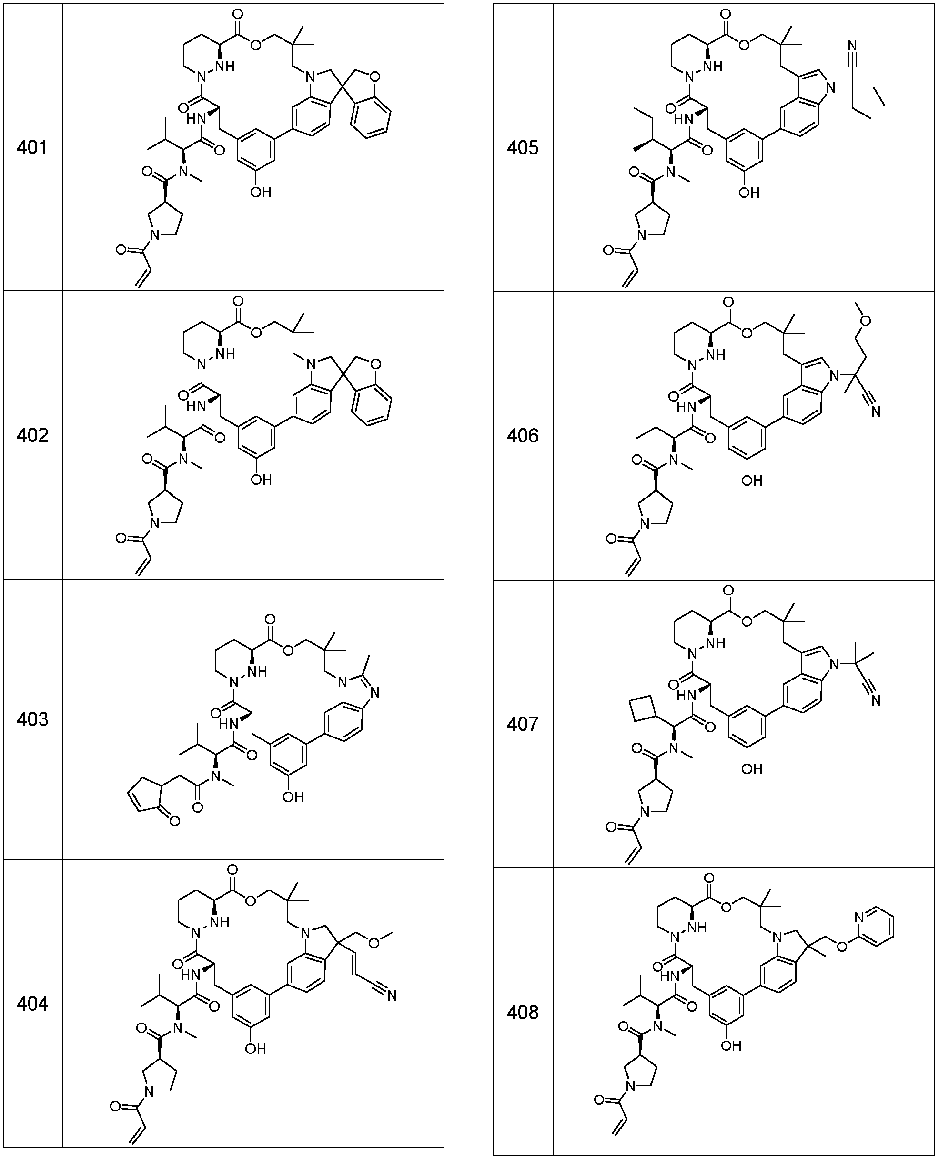

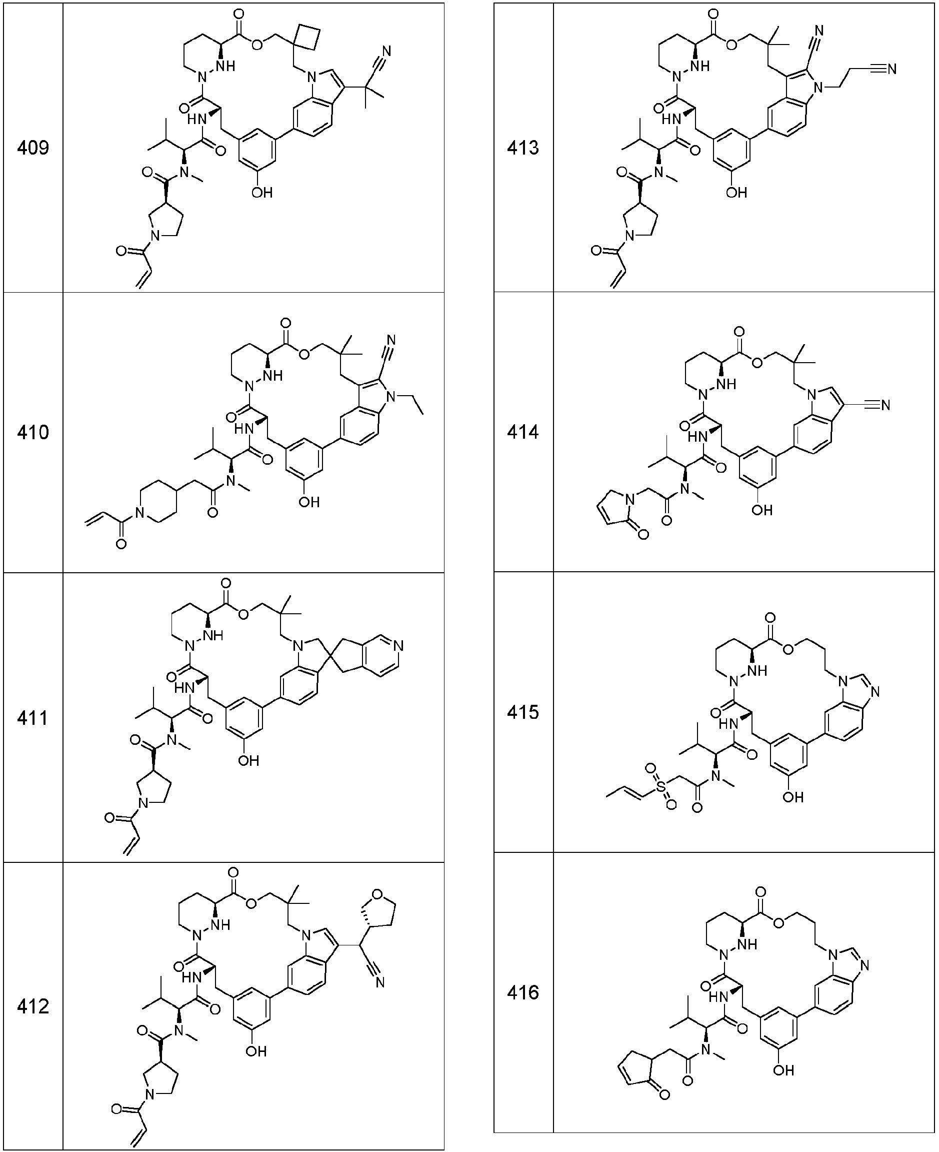

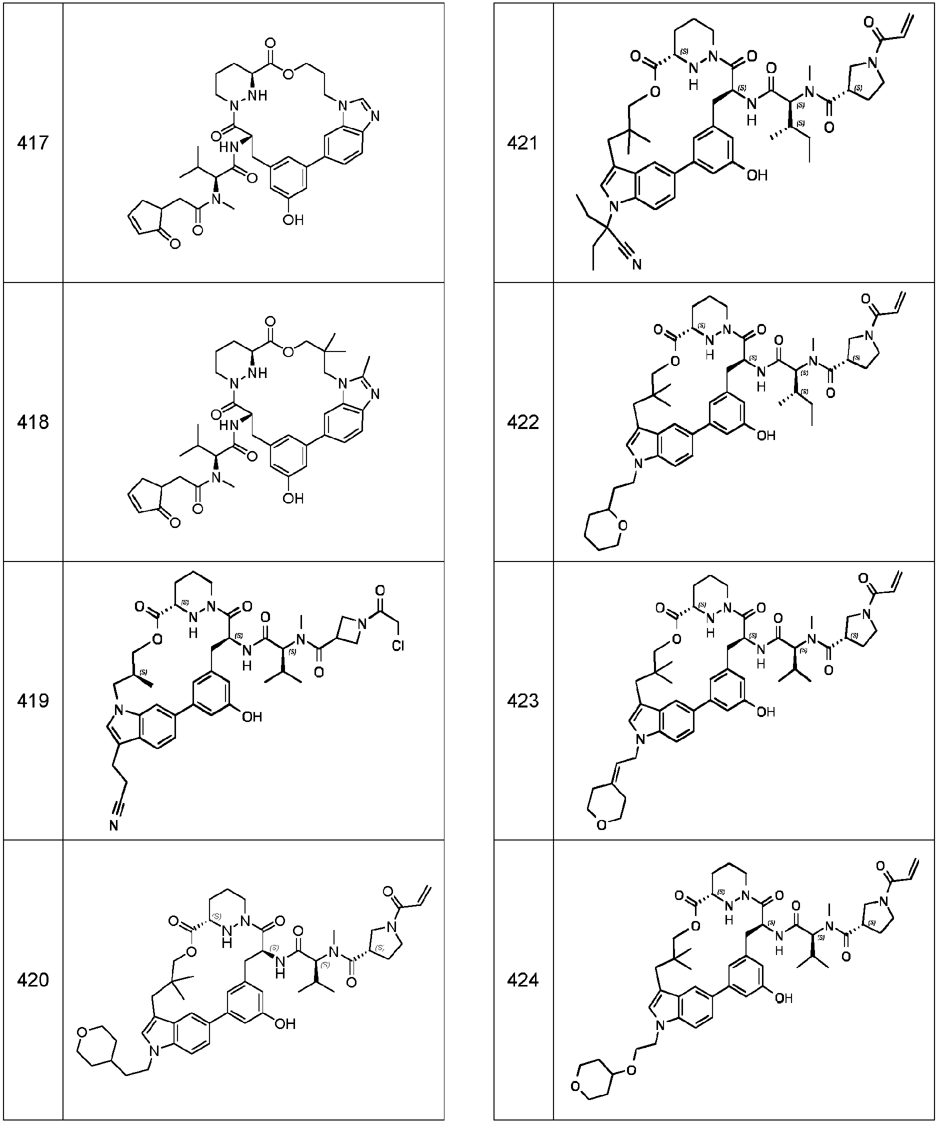

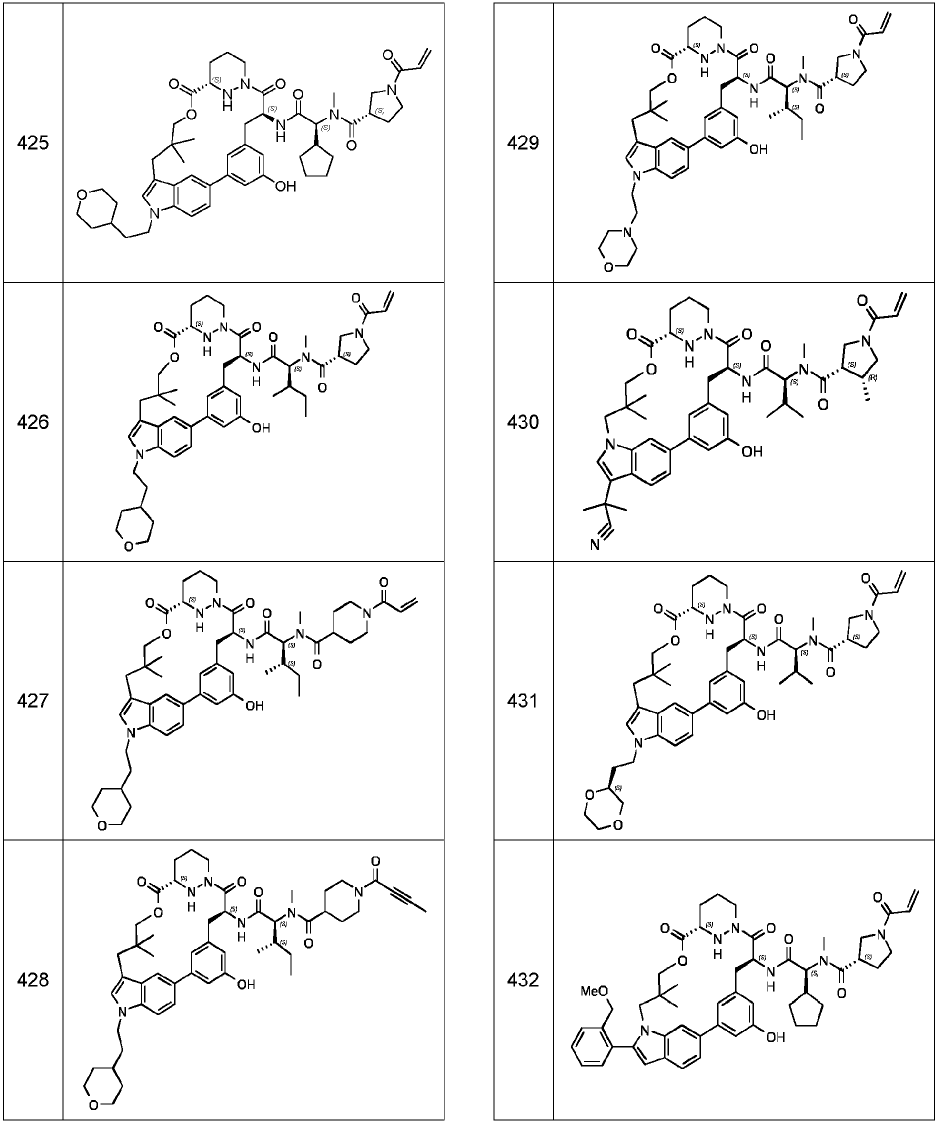

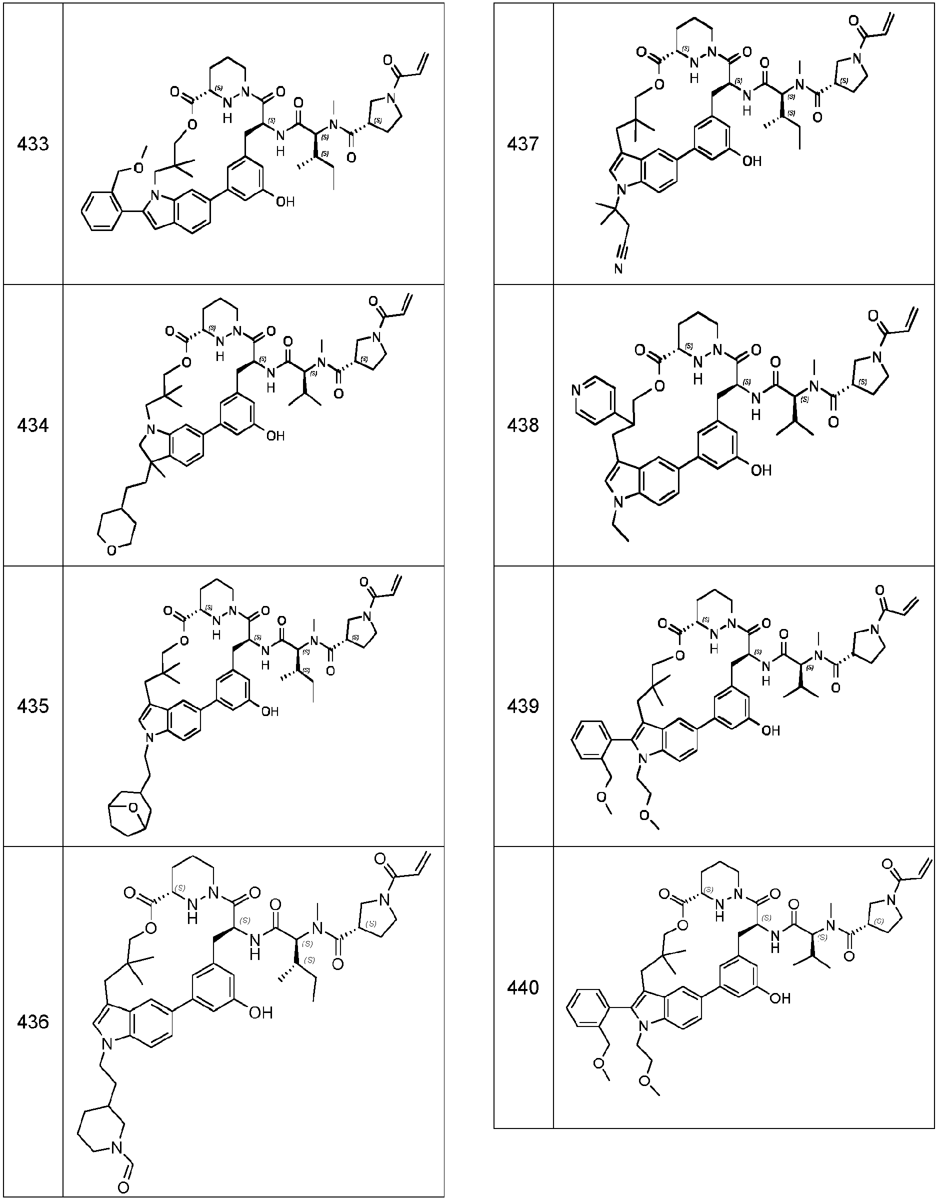

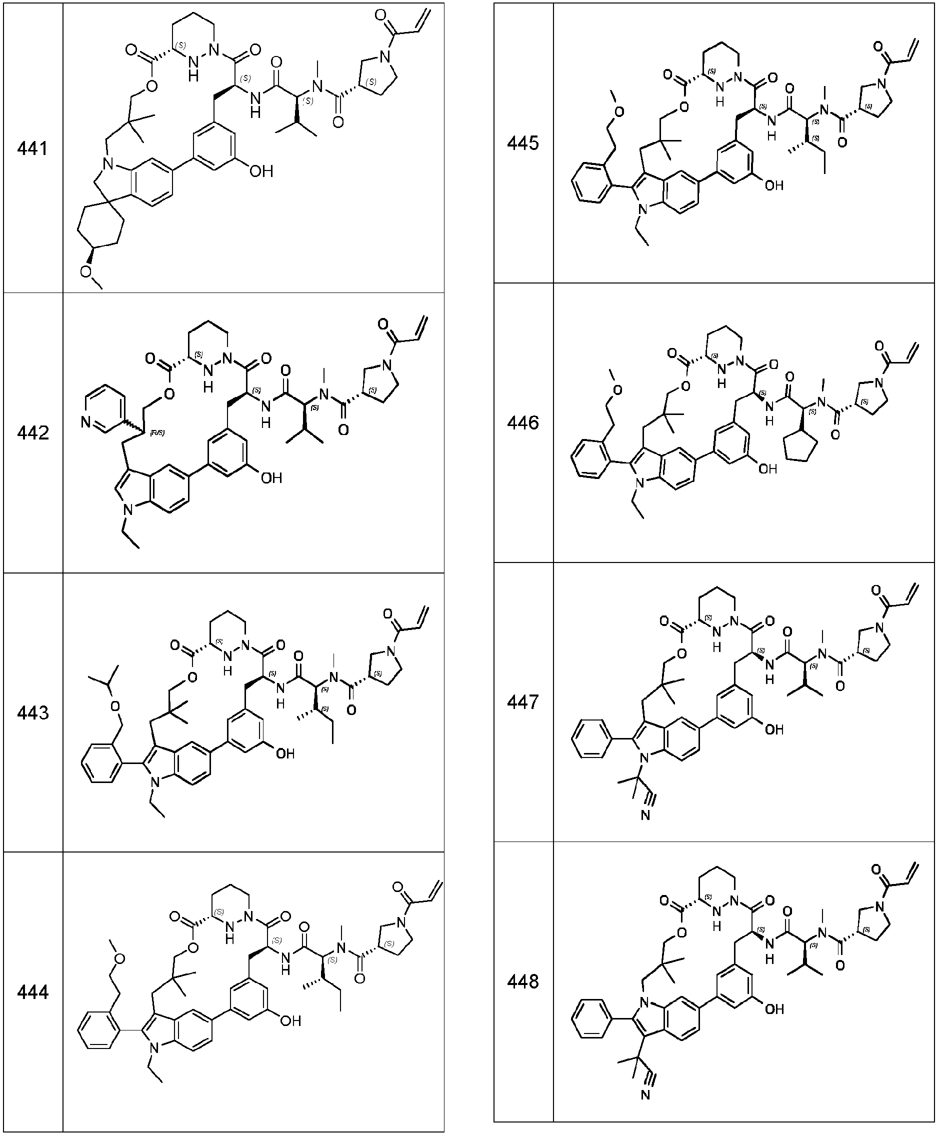

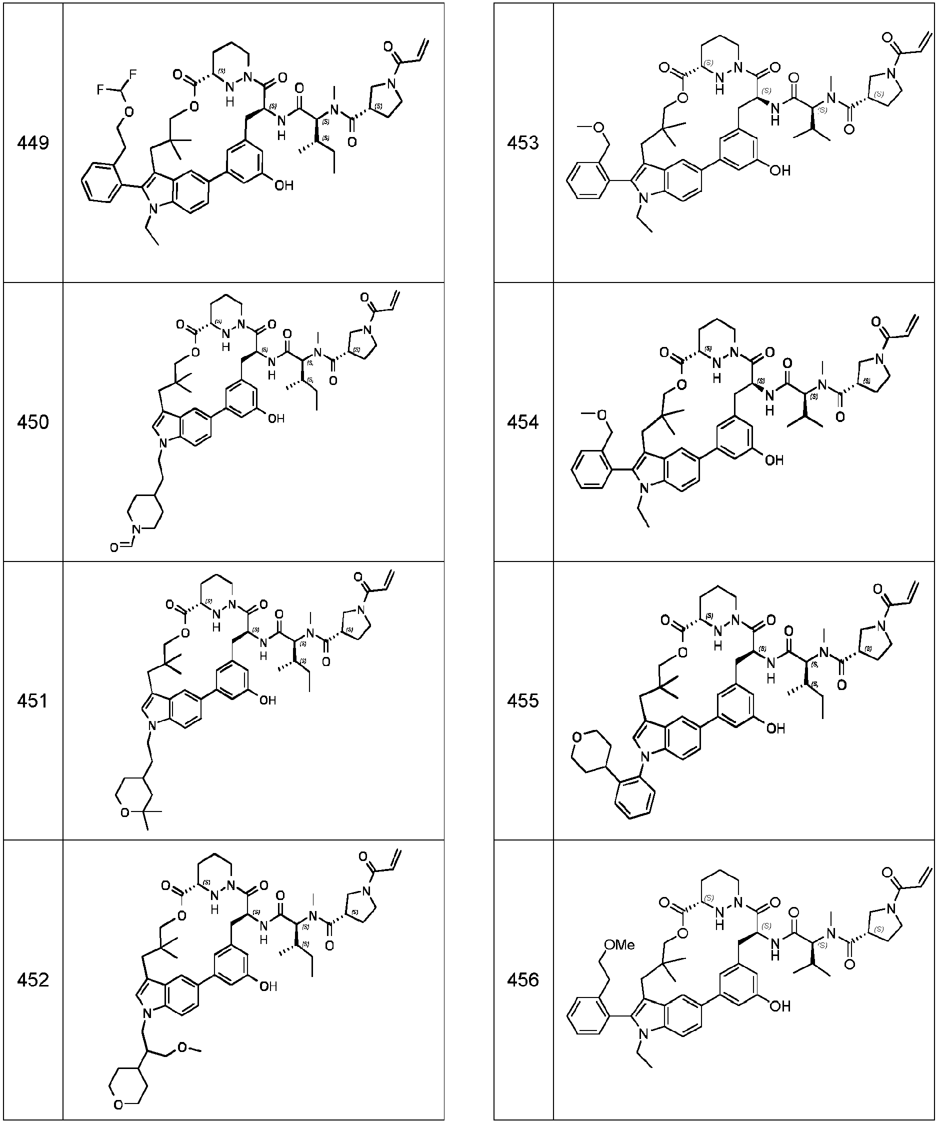

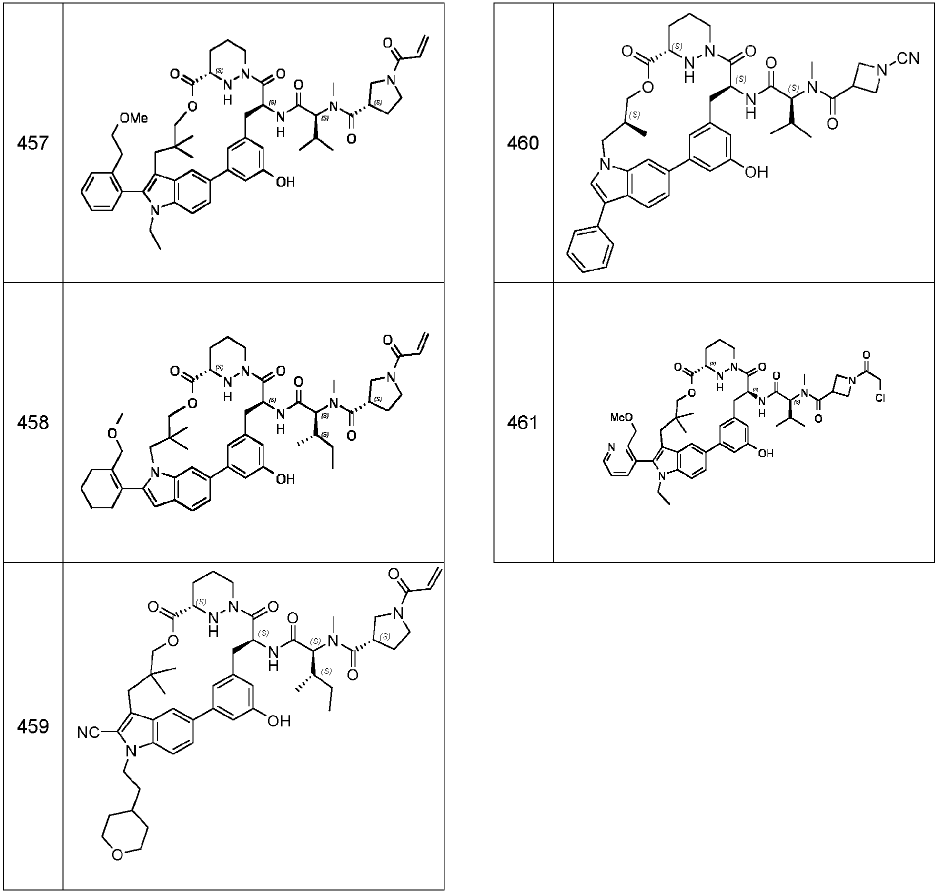

9. A pharmaceutical composition comprising a compound of any one of claims 1-8, or a pharmaceutically acceptable salt, an enantiomer, a stereoisomer, or a tautomer thereof, and a pharmaceutically acceptable carrier.

10. A complex comprising a presenter protein, a RAS protein, and a compound of any one of claims 1-8, or a pharmaceutically acceptable salt thereof, or a pharmaceutical composition of claim 9.

11. The compound of any one of claims 1-8, or a pharmaceutically acceptable salt, an enantiomer, a stereoisomer, or a tautomer thereof, for use in a method of treating cancer in a subject in need thereof.

12. The compound of any one of claims 1-8, or a pharmaceutically acceptable salt, an enantiomer, a stereoisomer, or a tautomer thereof, for use in a method of treating a KRAS G12C protein-related disorder in a subject in need thereof.

13. The compound for use of claim 11 or 12, wherein the method further comprises administering an additional therapeutic agent.

14. The compound for use of claim 13, wherein the additional therapeutic agent is a HER2 inhibitor, an EGFR inhibitor, a second Ras inhibitor, a SHP2 inhibitor, a SOS1 inhibitor, a Raf inhibitor, a MEK inhibitor, an ERK inhibitor, a PI3K inhibitor, a PTEN inhibitor, an AKT inhibitor, an mTORC1 inhibitor, a BRAF inhibitor, a PD-L1 inhibitor, a PD-1 inhibitor, a CDK 4/6 inhibitor, or a combination thereof.

## Patentansprüche

1. Verbindung der Formel I: 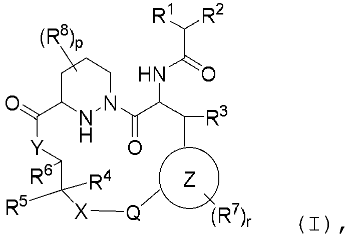 ein pharmazeutisch annehmbares Salz, ein Enantiomer, ein Stereoisomer oder ein Tautomer davon, worin
Q ein bicyclisches Arylen, ein bicyclisches Heteroarylen oder ein bicyclisches Heterocyclylen ist, worin ein erster Ring in Q an X gebunden ist und ein zweiter Ring in Q an Z gebunden ist und worin Q optional substituiert ist,
X eine Bindung, ein geradkettiges C₁-C₃-Alkylen, das optional mit 1 bis 3 Substituenten, unabhängig ausgewählt aus Fluor, -CN, -C₁-C₃-Alkyl und -O-C₁-C₃-Alkyl, -O-, -S(O)₀-₂-, *-CH₂-O-, *-CH₂-S(O)₀-₂-, *-O-CH₂- oder *-CH₂-S(O)₀₋₂- substituiert ist, worin "*" einen Teil von X darstellt, der an -C(R⁴)(R⁵)- gebunden ist,
Y -O-, -NH- oder -N(C₁-C₃-Alkyl)- ist,
Ring Z Phenyl oder ein 6-gliedriges Heteroaryl ist,
R¹ optional substituiertes C₁-C₆-Alkyl, -(CH₂)₀-₁-(C₃-C₆-optional substituiertes Cycloalkyl), -(CH₂)₀₋₁-(optional substituiertes Aryl) oder optional substituiertes Heterocyclyl ist,
R² 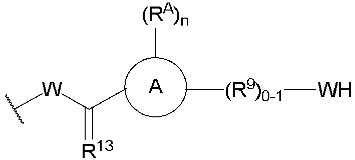 oder 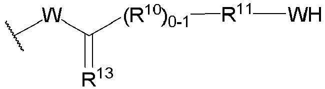 ist, worin
Ring A ein 4-8-gliedriges Cycloalkyl oder ein 4-8-gliedriges Heterocyclyl ist,
W -N(R¹²)-, -O- oder -C(R^{12a})(R^{12b})- ist,
jedes R^{A} unabhängig Fluor, Chlor, -CN, -OH, -NH₂, -C₁-C₃-Alkyl, das optional mit CN, OH, NH₂ oder -O-C₁-C₃-Alkyl substituiert ist, -O-C₁-C₃-Alkyl oder -NH-C₁-C₃-Alkyl ist,
R⁹, falls vorhanden, -N(C₀-C₃-Alkylen-H)-, -N(C(O)-(C₀-C₅-Alkylen-H)-, -C(C₀-C₃-Alkylen-H)(C₀-C₅-alkylen-H)- oder -C(C₀-C₃-Alkylen-H)(C(O)-C₀-C₃-alkylen-H)- ist, worin jeder Alkylenteil von R⁹ optional mit einem oder mehreren Substituenten substituiert ist, wobei jeder Substituent unabhängig aus Halogen, -CN, -OH, -C₁-C₃-Alkyl und -O-C₁-C₃-Alkyl ausgewählt ist,
R¹⁰, falls vorhanden, C₁-C₄-Alkylen ist, das optional mit einem oder mehreren Substituenten substituiert ist, wobei jeder Substituent unabhängig aus Halogen, -CN, -OH, -C₁-C₃-Alkyl und -O-C₁-C₃-Alkyl ausgewählt ist,
R¹¹ -N(C₀-C₅-Alkylen-H)-, -N(C(O)-(C₀-C₅-Alkylen-H)-, -C(C₀-C₃-Alkylen-H)(C₀-C₅-Alkylen-H)-, -C(C₀-C₃-Alkylen-H) (C(O)-C₀-C₅-alkylen-H)- oder ein gesättigtes, stickstoffhaltiges Heterocyclyl ist, worin jeder Alkylenteil von R¹¹ optional mit einem oder mehreren Substituenten substituiert ist, wobei jeder Substituent unabhängig aus Halogen, -CN, -OH, -C₁-C₃-Alkyl und -O-C₁-C₃-Alkyl ausgewählt ist,
R¹² Wasserstoff oder -C₁-C₃-Alkyl ist oder
R¹² zusammen mit einem R^{A}, den Atomen, an denen diese jeweils gebunden sind, und jeglichen dazwischenliegenden Atomen genommen wird, um ein optional substituiertes, 5-8-gliedriges Heterocyclyl zu bilden, das mit Ring A kondensiert oder spiro-kondensiert ist, oder
R¹² zusammen mit irgendeiner Methyleneinheit in R¹⁰ oder irgendeiner Methyleneinheit in R¹¹, den Atomen, an denen diese jeweils gebunden sind, und jeglichen dazwischenliegenden Atomen zusammengenommen wird, um ein optional substituiertes, 5-8-gliedriges Heterocyclyl zu bilden,
jedes von R^{12a} und R^{12b} unabhängig Wasserstoff oder -C₁-C₃-Alkyl sind oder R^{12a} und R^{12b} zusammen mit dem Kohlenstoffatom, an das sie gebunden sind, genommen werden, um einen 3-6-gliedrigen Cycloalkylring zu bilden,
R¹³ O, S, N-CN oder N-O-C₁-C₃-Alkyl ist und
WH 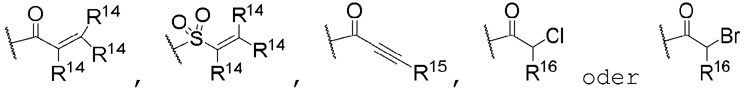 ist,
jedes R¹⁴ unabhängig Wasserstoff, -CN oder -C₁-C₃-Alkyl ist, das optional mit einem oder mehreren Substituenten, unabhängig ausgewählt aus -OH, -O-C₁-C₃-Alkyl, -NH₂, -NH(C₁-C₃-Alkyl), -N(C₁-C₃-Alkyl)₂ oder einem optional substituierten, 4-7-gliedrigen gesättigten Heterocyclyl, substituiert ist,
R¹⁵ -C₁-C₃-Alkyl ist, das optional mit einem oder mehreren Substituenten, unabhängig ausgewählt aus -OH, -O-C₁-C₃-Alkyl, -NH₂, -NH(C₁-C₃-Alkyl), -N(C₁-C₃-Alkyl)₂ oder einem optional substituierten 4-7-gliedrigen gesättigten Heterocyclyl, substituiert ist,
R¹⁶ Wasserstoff, -C₁-C₃-Alkyl ist, das optional mit einem oder mehreren Substituenten, ausgewählt aus -OH, -O-C₁-C₃-Alkyl, -NH₂, -NH(C₁-C₃-Alkyl), -N(C₁-C₃-Alkyl)₂ oder einem optional substituierten 4-7-gliedrigen gesättigten Heterocyclyl, substituiert ist, oder
R¹⁴ zusammen mit einem von R⁹ oder R¹¹, den Atomen, an denen diese gebunden sind, und jeglichen dazwischenliegenden Atomen genommen wird, um ein optional substituiertes 5-8-gliedriges Ringsystem zu bilden, oder
R¹⁶ zusammen mit einem von R⁹ oder R¹¹, den Atomen, an denen diese gebunden sind, und jeglichen dazwischenliegenden Atomen genommen wird, um ein optional substituiertes 5-8-gliedriges Ringsystem zu bilden,
R³ Wasserstoff, Halogen, C₁-C₃-Alkyl oder C₁-C₃-Hydroxyalkyl ist,
R⁴ Wasserstoff, Halogen oder optional substituiertes C₁-C₃-Alkyl ist,
R⁵ Wasserstoff, Halogen, -OH, -CN, -O-(optional substituiertes C₁-C₃-Alkyl), optional substituiertes C₁-C₃-Alkyl, optional substituiertes C₂-C₆-Alkenyl, optional substituiertes C₂-C₆-Alkinyl, -(CH₂)₀-₁-Aryl, -(CH₂)₀-₁-Heteroaryl, -(CH₂)₀₋₁-Cycloalkyl oder -(CH₂)₀₋₁-Heterocyclyl ist oder
R⁴ und R⁵ zusammengenommen werden, um =CH₂, ein optional substituiertes C₃-C₆-Cycloalkyl oder ein 3-7-gliedriges gesättigtes Heterocyclyl zu bilden, oder
R⁵ zusammen mit einem Ringatom in Q, dem Kohlenstoffatom, an das R⁴ gebunden ist, und X genommen wird, um ein 4-9-gliedriges gesättigtes oder ungesättigtes Heterocyclyl zu bilden, das mit Q kondensiert ist,
R⁶ Wasserstoff oder -CH₃ ist,
jedes R⁷ unabhängig Halogen, C₁-C₃-Alkyl, C₁-C₃-Halogenalkyl, C₁-C₃-Hydroxyalkyl, -OH, -O-C₁-C₃-Alkyl, -O-C₁-C₃-Halogenalkyl, -NRⁿ¹Rⁿ², -NRⁿ¹ORⁿ², -ONRⁿ¹Rⁿ² oder -NRⁿ¹NRⁿ²Rⁿ³ ist,
Rⁿ¹ H, C₁-C₃-Alkyl, C₁-C₃-Heteroalkyl, C₁-C₃-Halogenalkyl, -C₁-C₃-Hydroxyalkyl oder C₁-C₃-Aminoalkyl ist,
worin eine Methyleneinheit von Rⁿ¹ optional mit 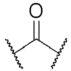 substituiert ist,
Rⁿ² H, C₁-C₃-Alkyl, C₁-C₃-Heteroalkyl, C₁-C₃-Halogenalkyl, C₁-C₃-Hydroxyalkyl oder C₁-C₃-Aminoalkyl ist,
worin eine Methyleneinheit von Rⁿ² optional mit 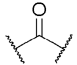 substituiert ist,
Rⁿ³ H, C₁-C₃-Alkyl, C₁-C₃-Heteroalkyl, C₁-C₃-Halogenalkyl, C₁-C₃-Hydroxyalkyl oder C₁-C₃-Aminoalkyl ist,
worin eine Methyleneinheit von Rⁿ³ optional mit 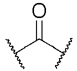 substituiert ist,
jedes R⁸ unabhängig Halogen, C₁-C₃-Alkyl oder C₁-C₃-Halogenalkyl ist,
n 0, 1, 2, 3, 4, 5 oder 6 ist,
p 0, 1, 2 oder 3 ist und
r 0, 1, 2, 3 oder 4 ist.

2. Verbindung nach Anspruch 1, wobei die Verbindung die Struktur der Formel (Ia) aufweist: 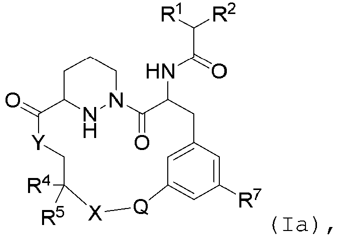 oder ein pharmazeutisch annehmbares Salz, ein Enantiomer, ein Stereoisomer oder ein Tautomer davon,
worin
X ein Bindung, -O-, -CH₂-, -CH(CH₃)-, *-CH₂-O- oder -CH₂-CH₂- ist, worin "*" einen Teil von X dargestellt, der an C(R⁴)(R⁵) gebunden ist,
Y -O- oder -NH- ist,
R¹ -C₁-C₄-Alkyl, -(CH₂)₀₋₁-(C₃-C₆-Cycloalkyl) oder -C₄-C₆-Cycloalkyl ist,
R² 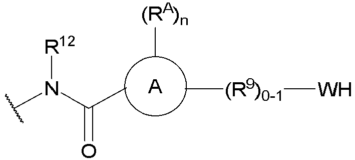 oder 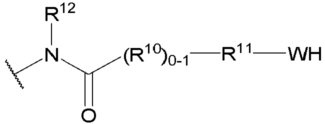 ist, worin
Ring A ein 4-8-gliedriges Cycloalkyl oder ein 4-8-gliedriges gesättigtes Heterocyclyl ist,
jedes R^{A} jeweils unabhängig Fluor, Chlor, -CN, -OH, -NH₂, -C₁-C₃-Alkyl, das optional mit CN, OH, NH₂ oder -O-C₁-C₃-Alkyl substituiert ist, -O-C₁-C₃-Alkyl oder -NH-C₁-C₃-Alkyl ist,
n 0, 1, 2, 3, 4, 5 oder 6 ist,
R⁹, falls vorhanden, -N(C₀-C₅-Alkylen-H)-, -N(C(O)-(C₀-C₅-Alkylen-H)-, -C(C₀-C₃-Alkylen-H) (C₀-C₅-alkylen-H)- oder -C(C₀-C₃-Alkylen-H) (C(O)-C₀-C₅-alkylen-H)- ist, worin jeder Alkylenteil von R⁹ optional mit einem oder mehreren Substituenten, unabhängig ausgewählt aus Halogen, -CN, -OH, -C₁-C₃-Alkyl und -O-C₁-C₃-Alkyl, substituiert ist,
R¹⁰, falls vorhanden, C₁-C₄-Alkylen ist, das optional mit einem oder mehreren Substituenten, unabhängig ausgewählt aus Halogen, -CN, -OH, -C₁-C₃-Alkyl und -O-C₁-C₃-Alkyl, substituiert ist,
R¹¹ -N(C₀-C₅-Alkylen-H)-, -N(C(O)-(C₀-C₅-Alkylen-H)-, -C(C₀-C₃-Alkylen-H)(C₀-C₅-alkylen-H)- oder -C(C₀-C₃-Alkylen-H) (C(O)-C₀-C₅-alkylen-H)- ist, worin jeder Alkylenteil von R¹¹ optional mit einem oder mehreren Substituenten, unabhängig ausgewählt aus Halogen, -CN, -OH, -C₁-C₃-Alkyl und -O-C₁-C₃-Alkyl, substituiert ist,
R¹² Wasserstoff oder -C₁-C₃-Alkyl ist oder
R¹² zusammen mit einem R^{A}, den Atomen, an denen diese jeweils gebunden sind, und jeglichen dazwischenliegenden Atomen genommen wird, um ein optional substituiertes, 5-8-gliedriges Heterocyclyl zu bilden, das mit Ring A kondensiert ist, oder
R¹² zusammen mit irgendeiner Methyleneinheit in R¹⁰ oder irgendeiner Methyleneinheit in R¹¹, den Atomen, an denen diese jeweils gebunden sind, und jeglichen dazwischenliegenden Atomen genommen wird, um ein optional substituiertes, 5-8-gliedriges Heterocyclyl zu bilden,
WH 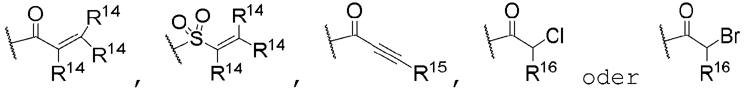 ist,
jedes R¹⁴ unabhängig Wasserstoff, -CN, -C₁-C₃-Alkyl, -C₁-C₃-Hydroxyalkyl, -O-C₁-C₃-Alkyl ist,
R¹⁵ -C₁-C₃-Alkyl, -C₁-C₃-Hydroxyalkyl oder -C₁-C₃-Alkylen-O-C₁-C₃-alkyl ist,
R¹⁶ Wasserstoff, -C₁-C₃-Alkyl, -C₁-C₃-Hydroxyalkyl oder -C₁-C₃-Alkylen-O-C₁-C₃-alkyl ist oder
R¹⁴ zusammen mit einem von R⁹ oder R¹¹, den Atomen, an denen diese gebunden sind, und jeglichen dazwischenliegenden Atomen genommen wird, um ein optional substituiertes 5-8-gliedriges Ringsystem zu bilden, oder
R¹⁶ zusammen mit einem von R⁹ oder R¹¹, den Atomen, an denen diese gebunden sind, und jeglichen dazwischenliegenden Atomen genommen wird, um ein optional substituiertes 5-8-gliedriges Ringsystem zu bilden,
R⁴ Wasserstoff, Halogen oder C₁-C₃-Alkyl ist,
R⁵ Wasserstoff, Halogen, -OH, C₁- C₃-Alkyl, C₁-C₃-Hydroxyalkyl, C₁-C₃-Alkylen-O-C₁-C₃-alkyl, C₁-C₃-Halogenalkyl, -(CH₂)₀₋₁-C₃-C₆-Cycloalkyl, C₁-C₃-Cyanoalkyl oder -(CH₂)₀₋₁-Aryl (Benzyl) ist oder
R⁴ und R⁵ zusammengenommen werden, um =CH₂ oder ein C₃-C₆-Cycloalkyl zu bilden, oder
R⁵ zusammen mit einem Ringatom von Q, dem Kohlenstoffatom, an das es gebunden ist, und X genommen wird, um ein 5-7-gliedriges gesättigtes Heterocyclyl zu bilden,
R⁷ -OH, -NH₂ oder C₁-C₃-Halogenalkyl ist,
Q ein bicyclisches Arylen, ein bicyclisches Heteroarylen oder ein bicyclisches Heterocyclylen ist,
worin
ein erster Ring in Q an X gebunden ist und ein zweiter Ring in Q an Z gebunden ist und
Q optional mit einem oder mehreren unabhängig ausgewählten Substituenten substituiert ist, ausgewählt aus =O, -CN, -C₁-C₅-Alkyl, optional substituiert mit einem oder mehreren, unabhängig ausgewählt aus Halogen, CN, OH, -O-(C₁-C₃-Alkyl), -C(O)-(C₁-C₃-Alkyl), -O-(C₂-C₃-Alkinyl), -(C₃-C₆-Cycloalkyl) oder einem 4-7-gliedrigen gesättigten Heterocyclyl, -O-(C₁-C₃-Alkyl), optional substituiert mit einem oder mehreren, unabhängig ausgewählt aus Halogen, C₂-C₅-Alkenyl, optional substituiert mit einem oder mehreren, unabhängig ausgewählt aus -CN oder -OH, C₂-C₃-Alkinyl, -S(O)₂-C₁-C₃-Alkyl, -(CH₂)₀₋₁-C₃-C₆-Cycloalkyl, optional substituiert mit einem oder mehreren, unabhängig ausgewählt aus Halogen, =O, -CN, C₁-C₃-Alkyl, optional substituiert mit -CN oder -O-C₁-C₃-Alkyl, -C(O)-gesättigtem Heterocyclyl, -O-gesättigtem Heterocyclyl, O-Cycloalkyl oder -O-Aryl, -(CH₂)₀₋₁-Heteroaryl, optional substituiert mit einem oder mehreren, unabhängig ausgewählt aus Halogen, -CN, C₁-C₃-Alkyl, optional substituiert mit -CN oder -O-C₁-C₃-Alkyl, -C(O)-gesättigtem Heterocyclyl, -O-gesättigtem Heterocyclyl, O-Cycloalkyl oder -O-Aryl, -(CH₂)₀-₁-Heterocyclyl, optional substituiert mit einem oder mehreren, unabhängig ausgewählt aus Halogen, =O, -CN, C₁-C₃-Alkyl, optional substituiert mit -CN oder -O-C₁-C₃-Alkyl, -C(O)-gesättigtem Heterocyclyl, -O-gesättigtem Heterocyclyl, O-Cycloalkyl oder -O-Aryl, -(CH₂)₀₋₁-Aryl, optional substituiert mit einem oder mehreren, unabhängig ausgewählt aus Halogen, -CN, -C₁-C₃-Alkyl, optional substituiert mit -CN oder -O-C₁-C₃-Alkyl, -C(O)-gesättigtem Heterocyclyl, -O-gesättigtem Heterocyclyl, O-Cycloalkyl oder -O-Aryl, -C(O)-NH-(C₁-C₃-Alkyl), -C(O)-N(C₁-C₃-Alkyl)₂, C₂-C₃-Alkenylen=N-O-(C₁-C₃-alkyl), optional substituiert mit C₃-C₆-Cycloalkyl, oder
zwei Substituenten an denselben oder benachbarten Ringatomen von Q zusammengenommen werden, um einen 5-7-gliedrigen monocyclischen Ring oder einen 6-12-gliedrigen bicyclischen Ring, der optional mit einem oder mehreren, unabhängig ausgewählt aus Halogen, =O, -CN, C₁-C₃-Alkyl oder -O-C₁-C₃-Alkyl, substituiert ist und mit Q kondensiert ist.

3. Verbindung nach Anspruch 2, wobei die Verbindung die Struktur der Formel (Ib) aufweist: 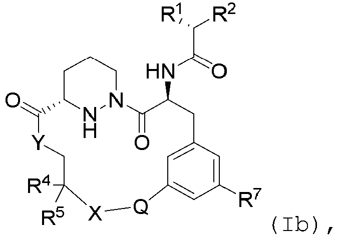 oder ein pharmazeutisch annehmbares Salz, ein Enantiomer, ein Stereoisomer oder ein Tautomer davon, oder die Verbindung die Struktur der Formel (Ic) aufweist: 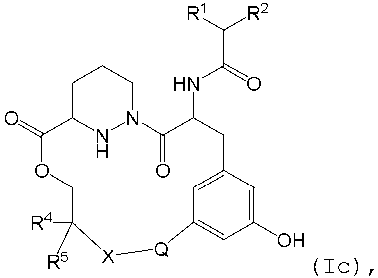 oder ein pharmazeutisch annehmbares Salz, ein Enantiomer, ein Stereoisomer oder ein Tautomer davon.

4. Verbindung nach einem der Ansprüche 1-3 oder ein pharmazeutisch annehmbares Salz, ein Enantiomer, ein Stereoisomer oder ein Tautomer davon, worin Q ein 5,6-bicyclisches Heteroarylen, ein 5,6-bicyclisches Heterocyclylen, ein 6,6-bicyclisches Heteroarylen oder ein 6,6-bicyclisches Heterocyclylen ist und worin Q optional substituiert ist,
wobei Q optional aus der Gruppe, bestehend aus Folgenden, ausgewählt ist: 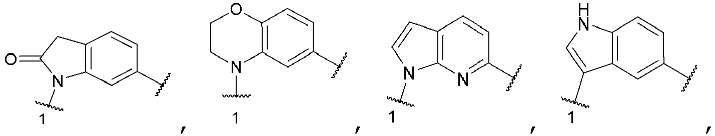 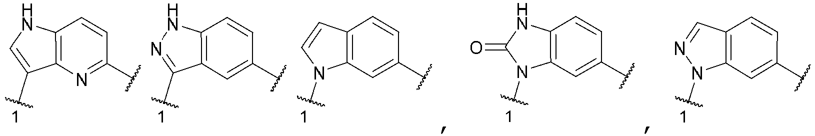 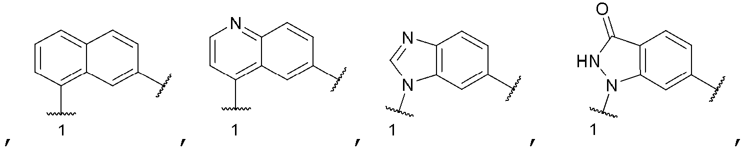 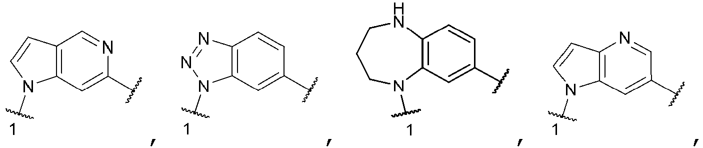 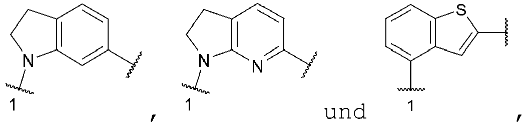 worin
"1" einen Teil von Q angibt, der an X gebunden ist, und Q ferner optional substituiert ist.

5. Verbindung nach einem der Ansprüche 1-4 oder ein pharmazeutisch annehmbares Salz, ein Enantiomer, ein Stereoisomer oder ein Tautomer davon, worin
(i) R¹ -CH₃, -CH₂CH₃, -(CH₂)₂CH₃, -CH(CH₃)₂, -CH(CH₃)CH₂CH₃, Cyclopropylmethyl, Cyclobutylmethyl, Cyclopentylmethyl, Cyclohexylmethyl, Cyclopropyl, Cyclobutyl, Cyclopentyl, Cyclohexyl, Phenyl, 4-Methoxybenzyl oder Tetrahydropyran-4-yl ist und/oder
(ii) R⁹ abwesend ist und Ring A ein gesättigtes, stickstoffhaltiges Heterocyclyl ist und/oder
(iii) der durch 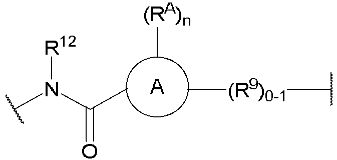 oder 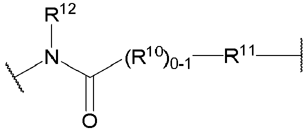 dargestellte Teil von R² aus der Gruppe ausgewählt ist, bestehend aus: 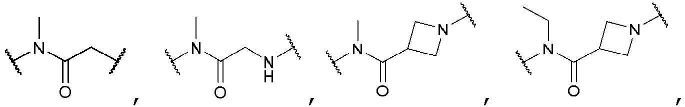 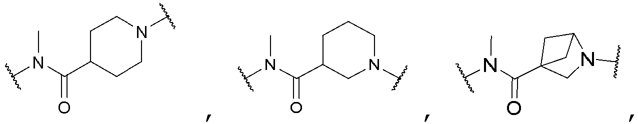 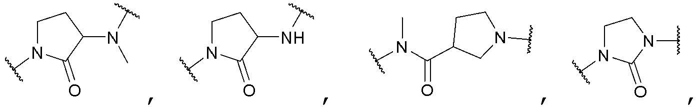 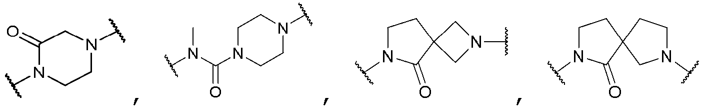 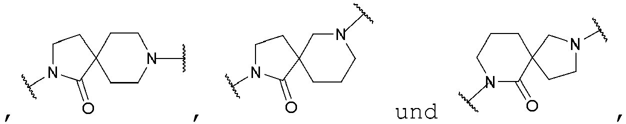 worin jedes Ringsystem in R² optional mit bis zu 4 Substituenten, unabhängig ausgewählt aus Fluor, Chlor, -CN, -OH, -NH₂, -C₁-C₃-Alkyl, optional substituiert mit CN, OH, NH₂ oder -O-C₁-C₃-Alkyl, -O-C₁-C₃-Alkyl und -NH-C₁-C₃-Alkyl, substituiert ist, und/oder
(iv) der durch WH dargestellte Teil von R² -C(O)-C≡C-CH₃, -C(O)-CH=CH₂, -S(O)₂-CH=CH₂, -C(O)-CH₂Cl, -C(O)-CH(CH₃)Cl, oder -C(O)-CH(Cl)-CH₂-O-CH₃ ist oder
der durch -R¹¹-WH dargestellte Teil von R², wenn R¹¹ zusammen mit einem R¹⁴ genommen wird, 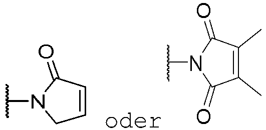 ist.

6. Verbindung nach einem der Ansprüche 1-5 oder ein pharmazeutisch annehmbares Salz, ein Enantiomer, ein Stereoisomer oder ein Tautomer davon, worin
R⁴ Wasserstoff, Fluor oder -CH₃ ist und
R⁵ Wasserstoff, Fluor, Chlor, -OH, -CH₃, -CH₂CH₃, -CH(CH₃)₂, -CH₂OH, -CH₂OCH₃, -CH₂F, -CHF₂, CH₂CN, -CH₂-Cyclopropyl, Cyclopropyl, Pyridyl, Phenyl oder -CH₂-Phenyl ist, worin jeglicher Phenylteil von R⁵ optional mit bis zu 4 Substituenten, unabhängig ausgewählt aus Halogen, -CN und -O-C₁-C₃-Alkyl, substituiert ist, oder
R⁴ und R⁵ zusammengenommen werden, um =CH₂ oder Cyclopropyl oder Cyclobutyl oder Cyclopentyl oder Cyclohexyl zu bilden, oder
R⁵ zusammen mit dem Kohlenstoffatom, an das es gebunden ist, einem Ringatom von Q und X genommen wird, um Oxazepan zu bilden.

7. Verbindung nach einem der Ansprüche 1-6 oder ein pharmazeutisch annehmbares Salz, ein Enantiomer, ein Stereoisomer oder ein Tautomer davon, worin R⁷ -OH, -NH₂ oder -CHF₂ ist.

8. Verbindung gemäß Anspruch 1 oder ein pharmazeutisch annehmbares Salz, ein Enantiomer, ein Stereoisomer oder ein Tautomer davon, ausgewählt aus:

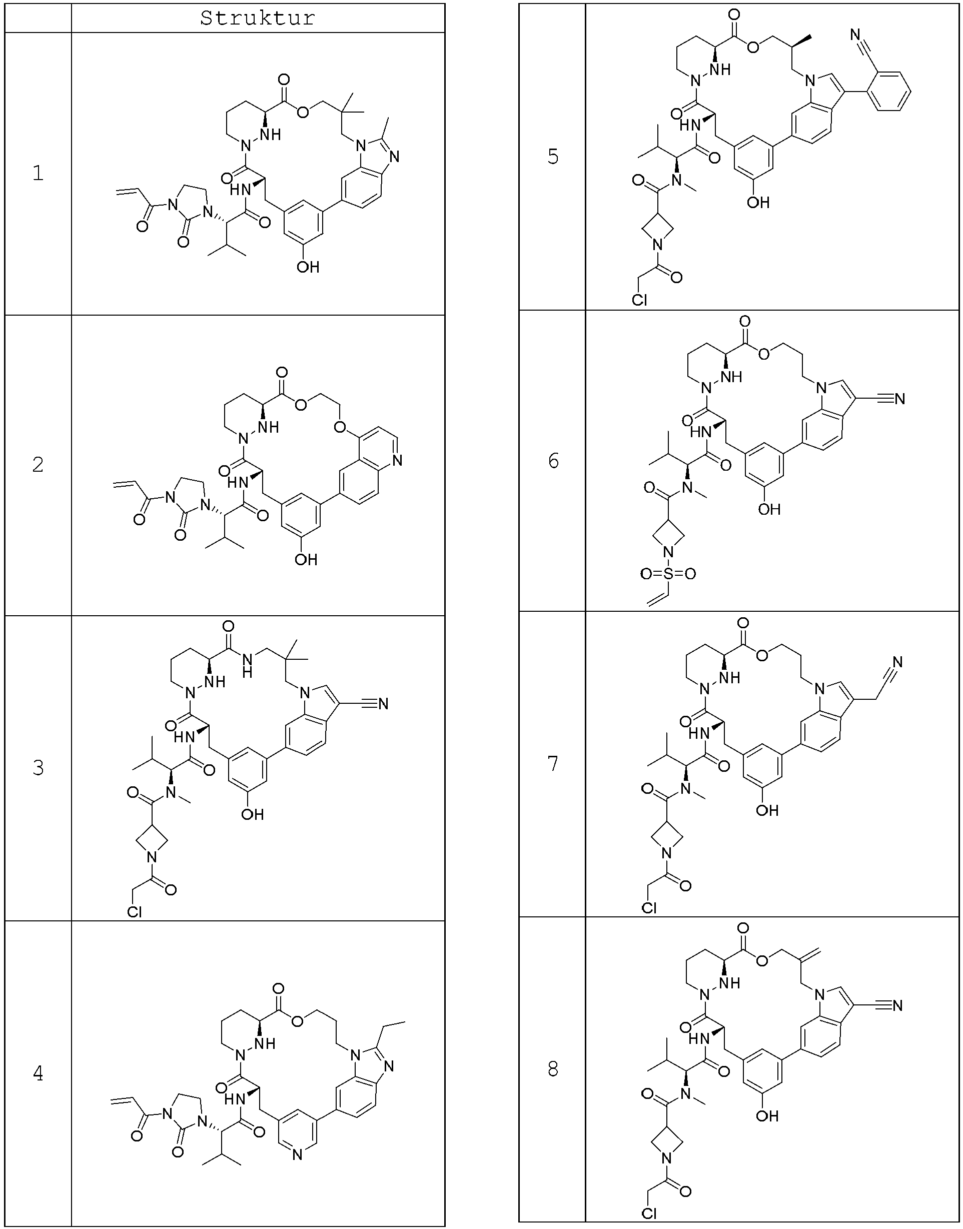

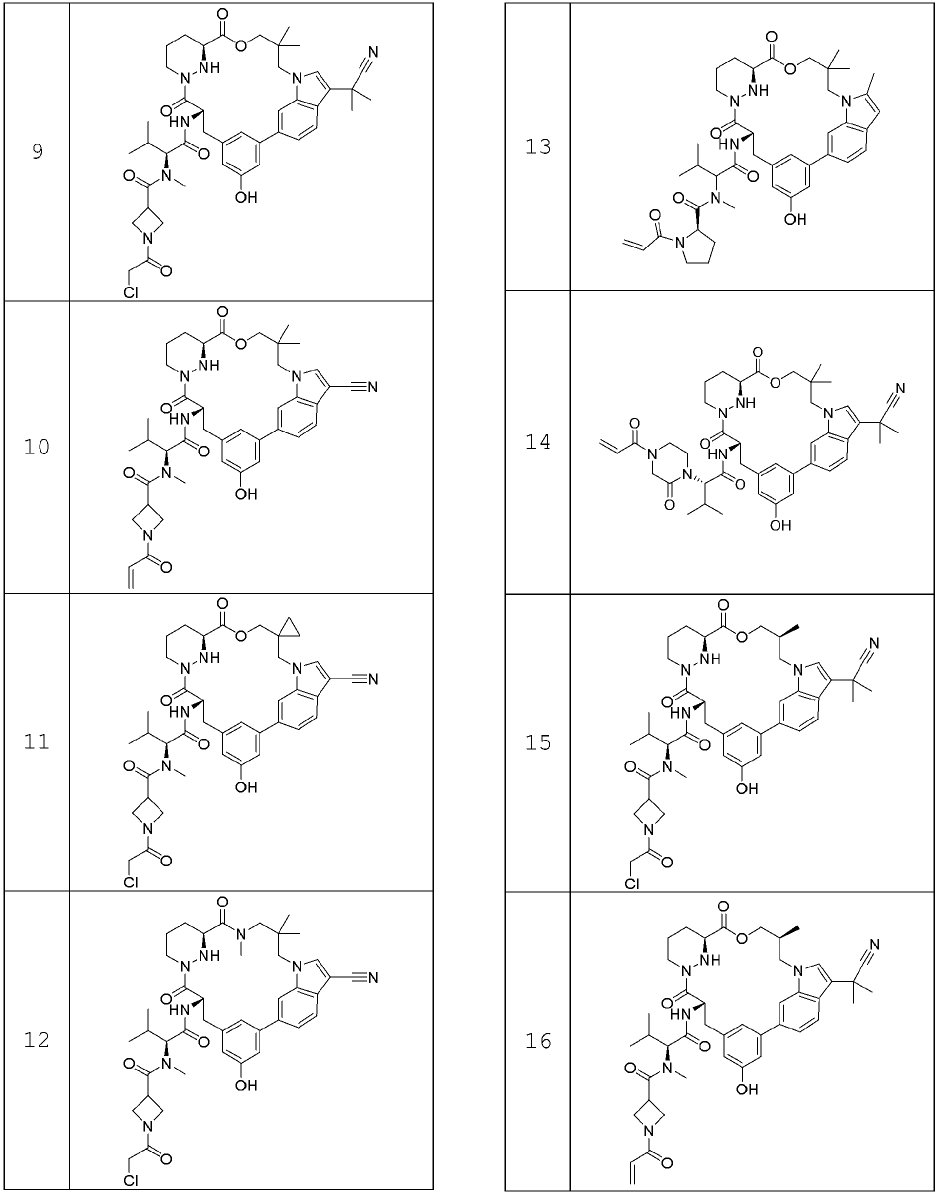

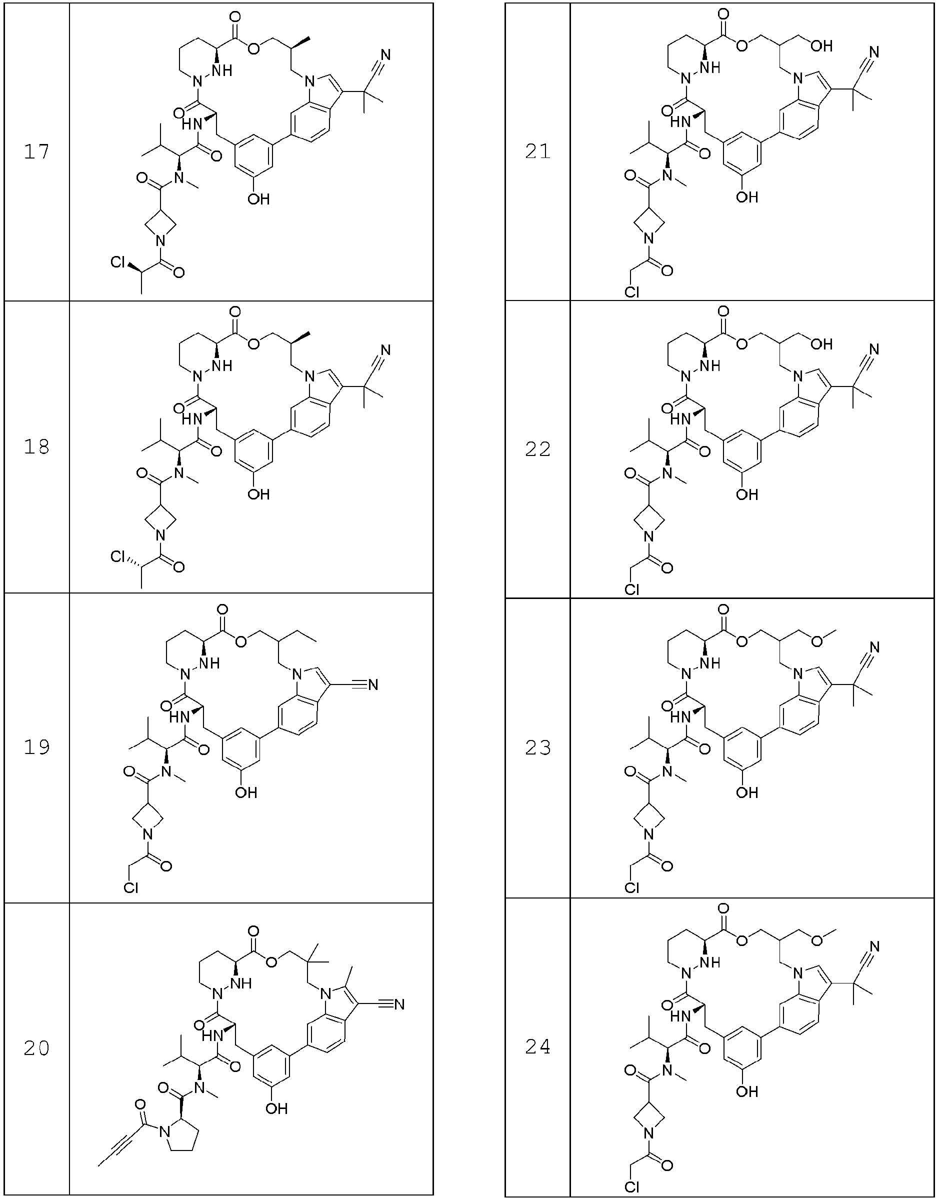

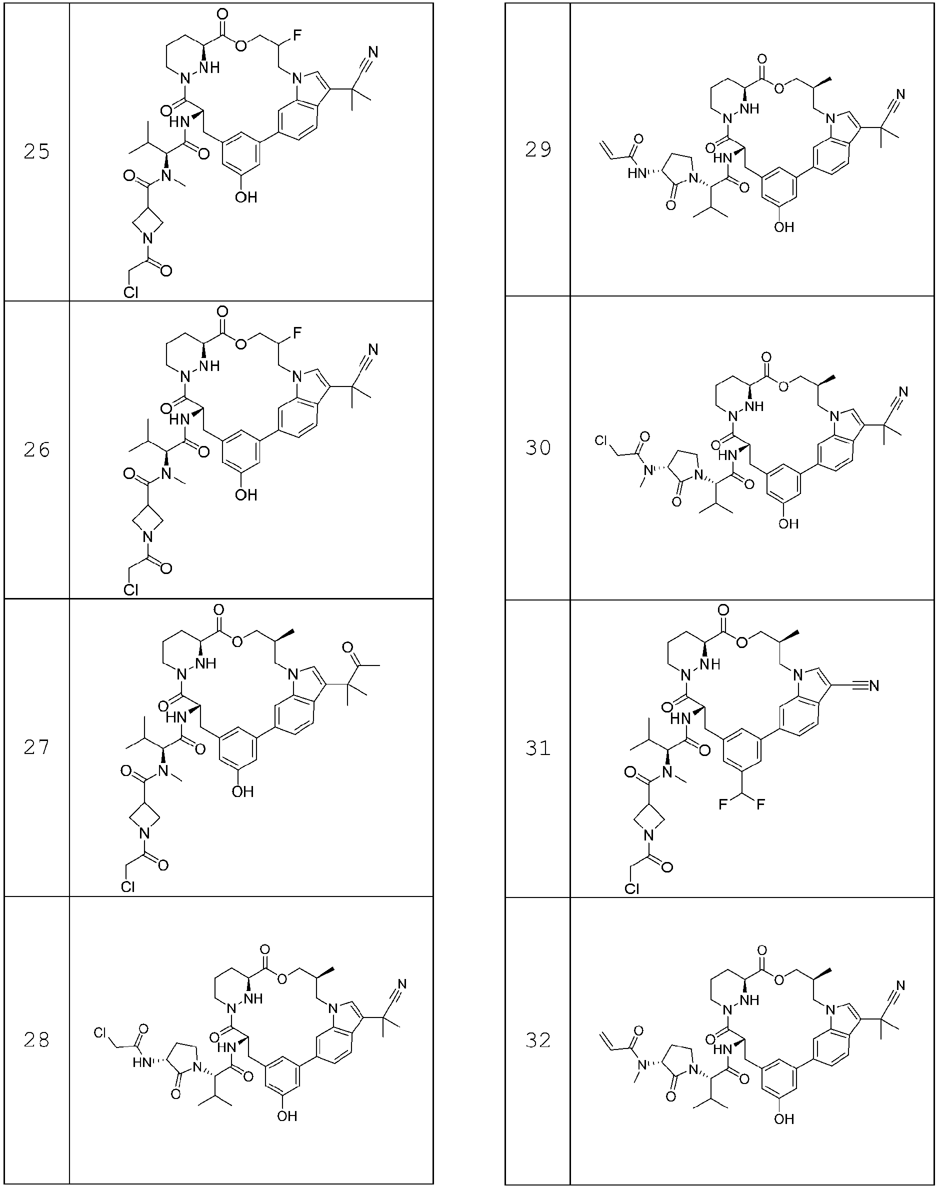

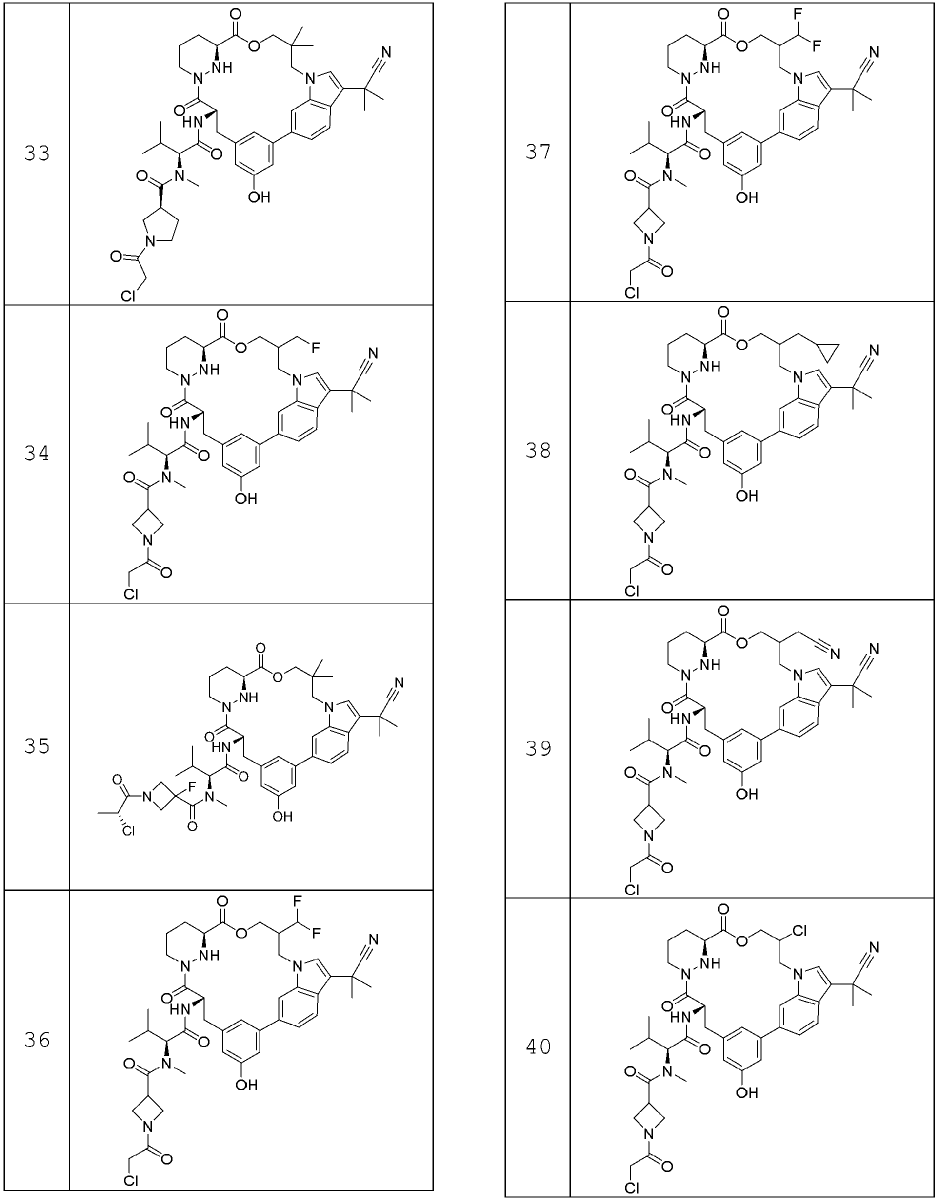

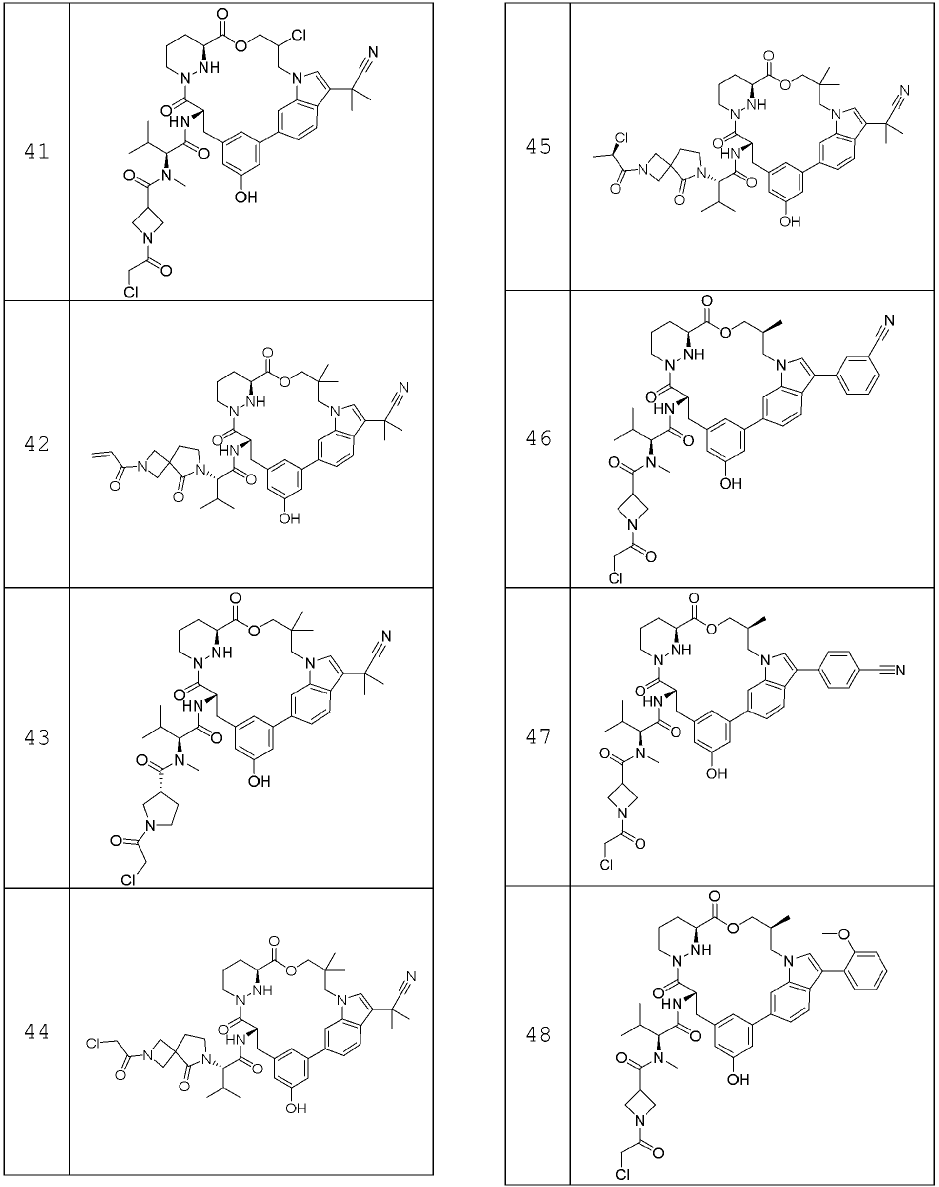

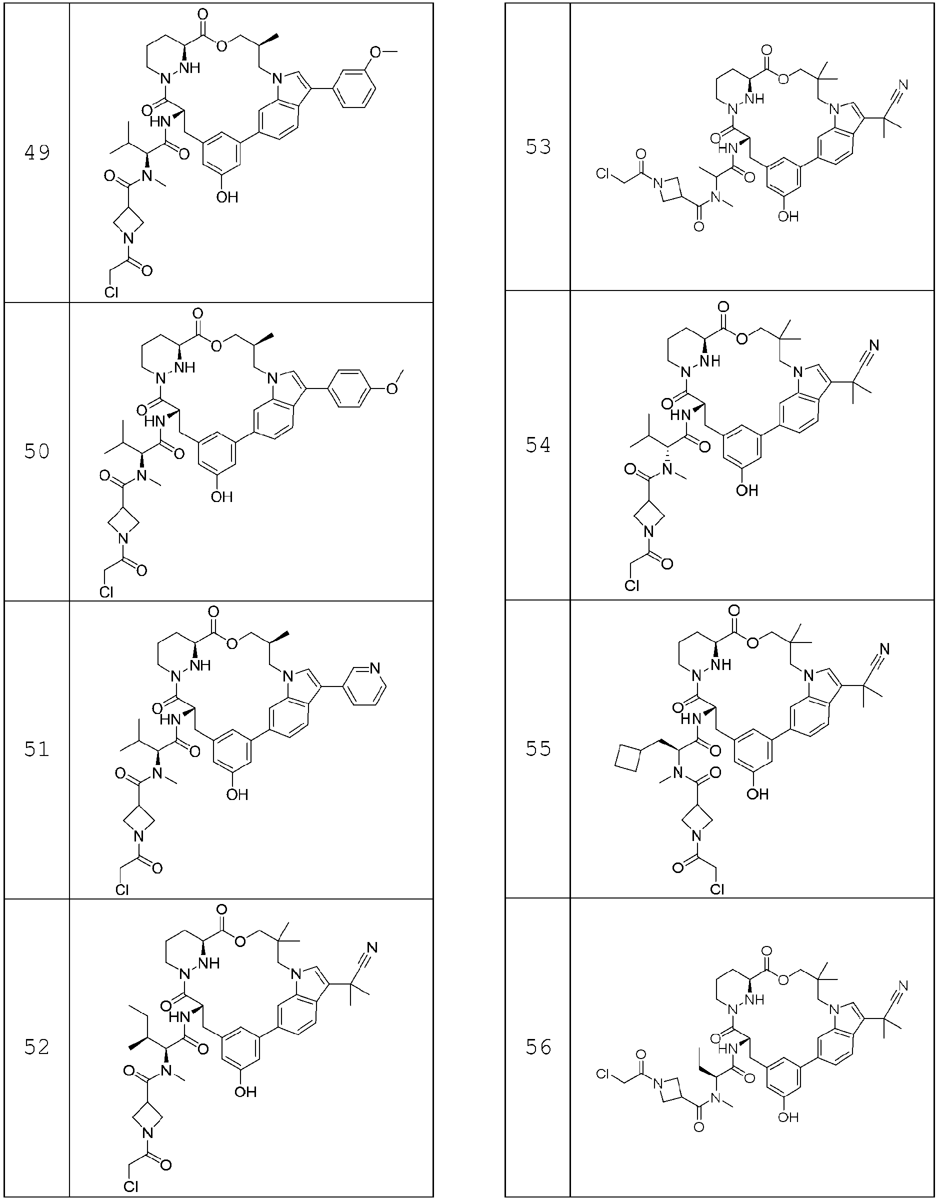

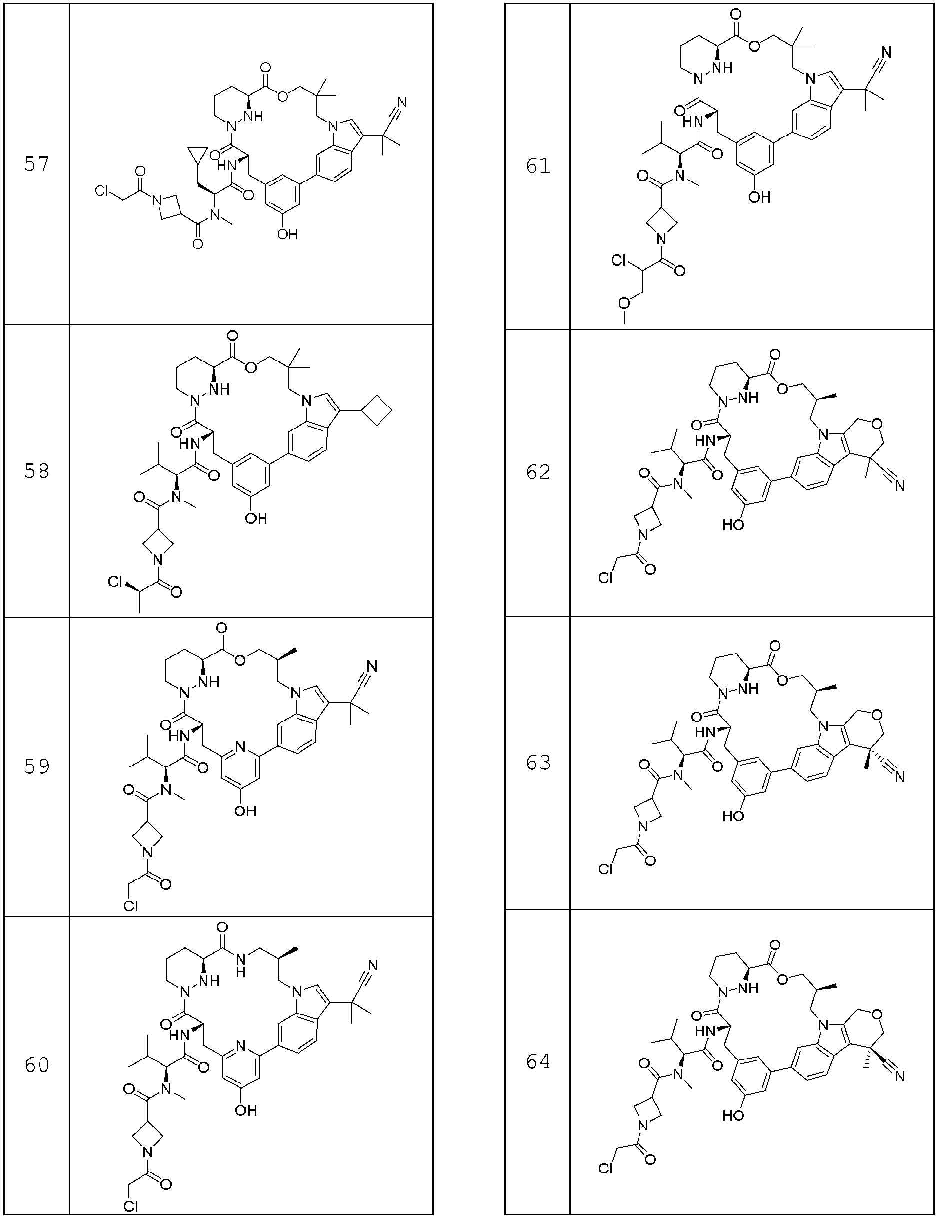

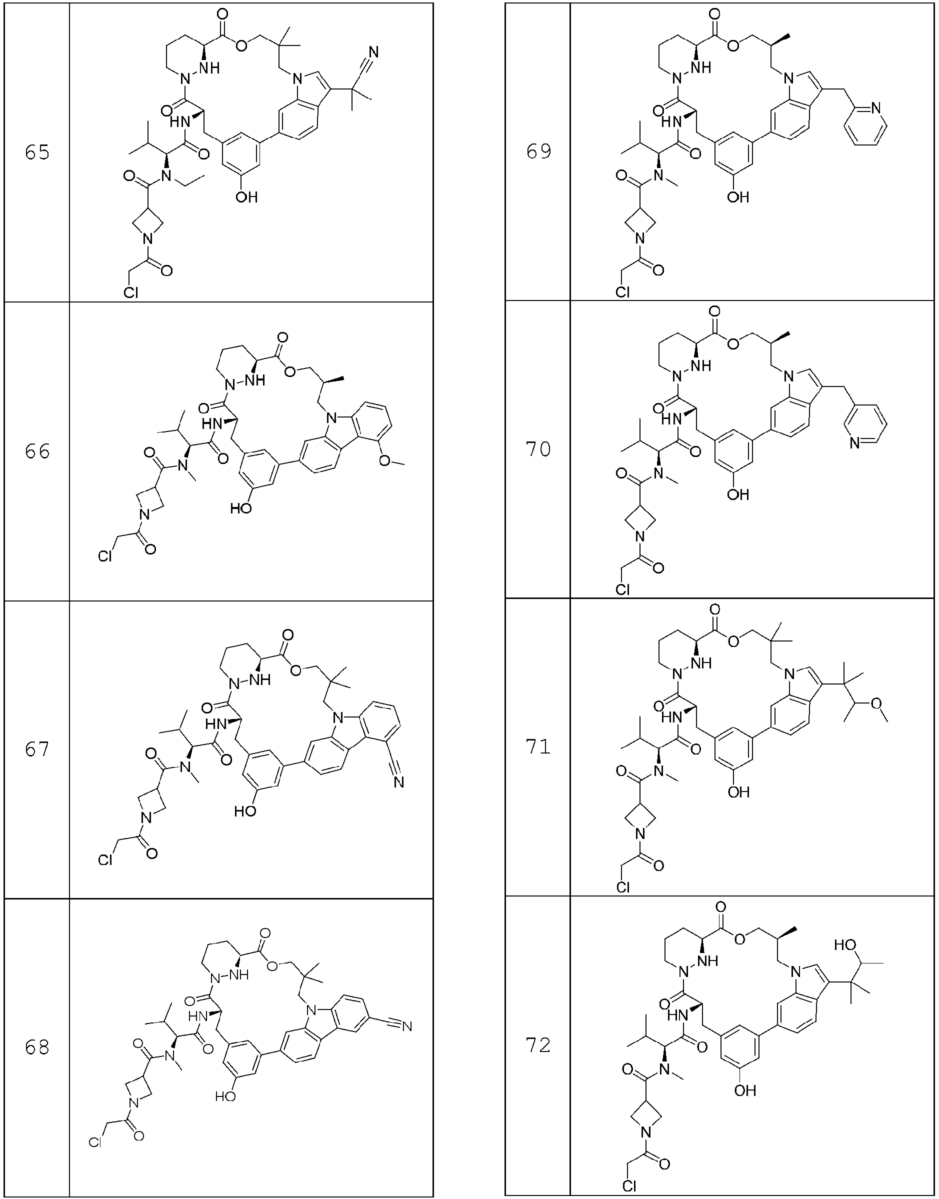

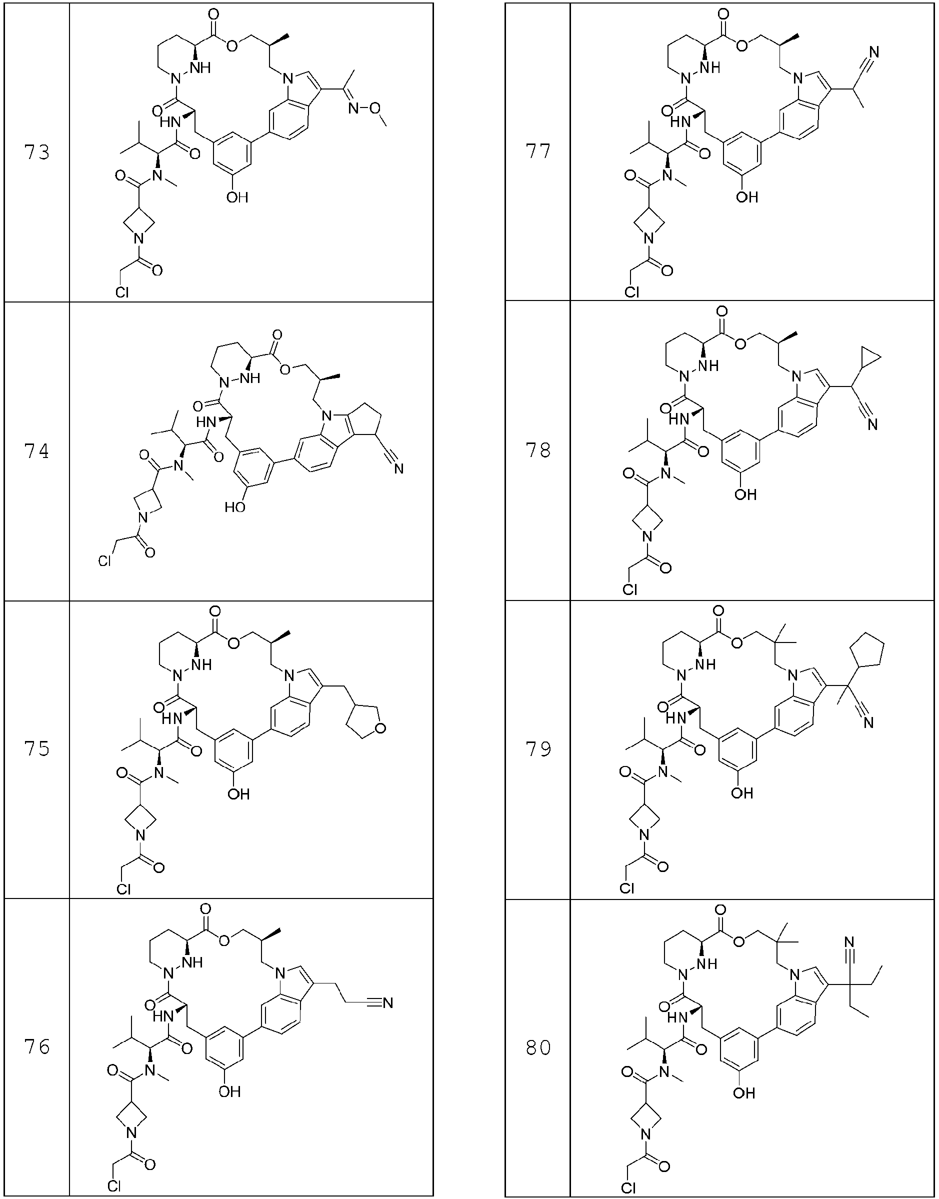

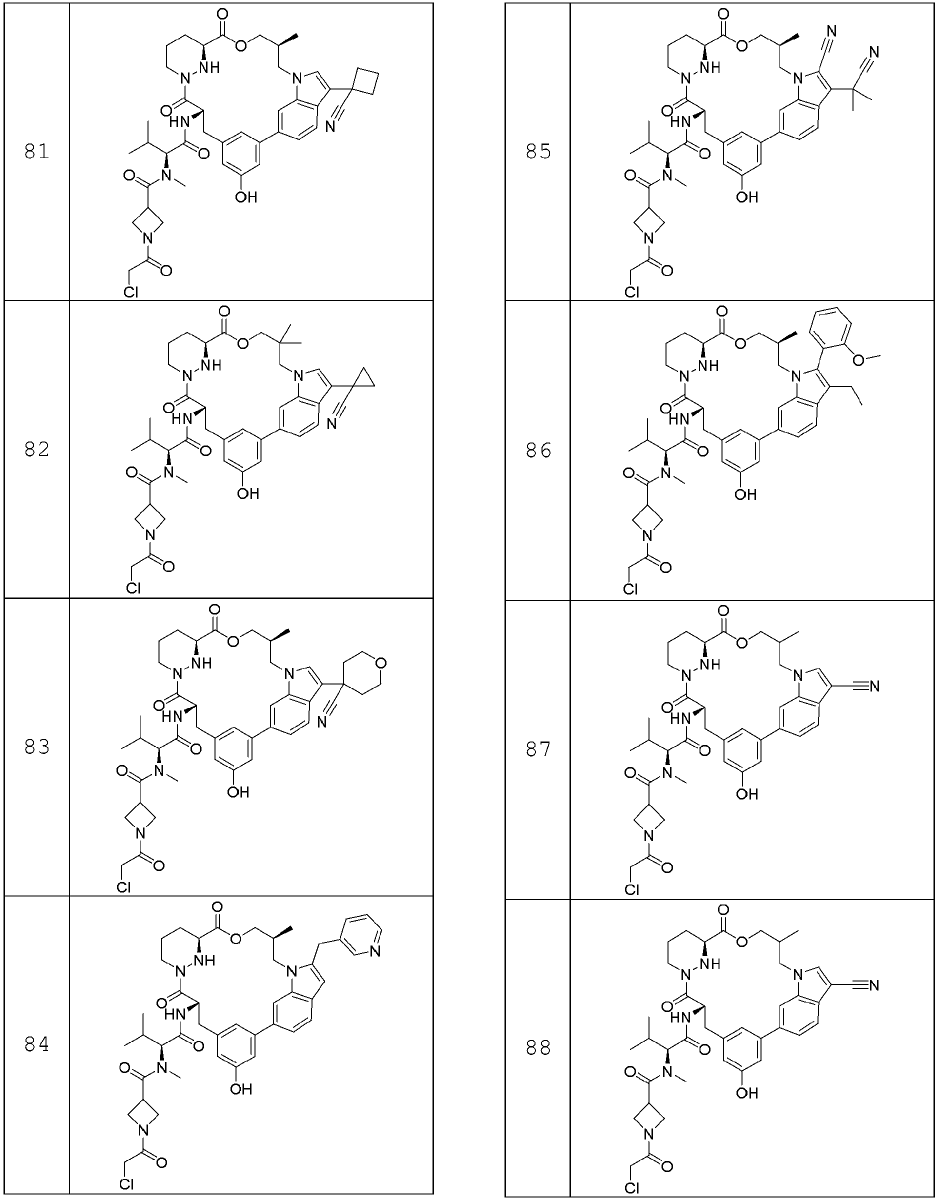

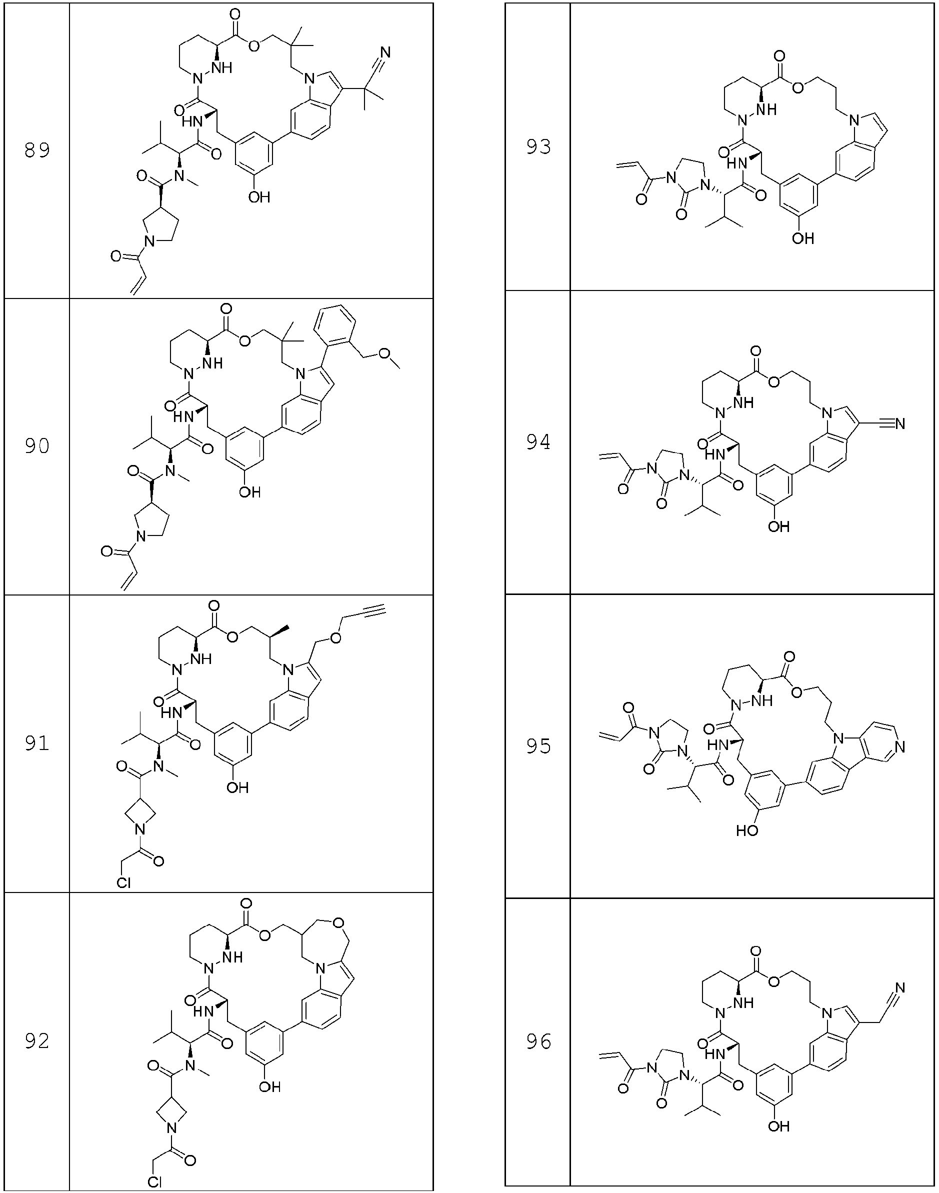

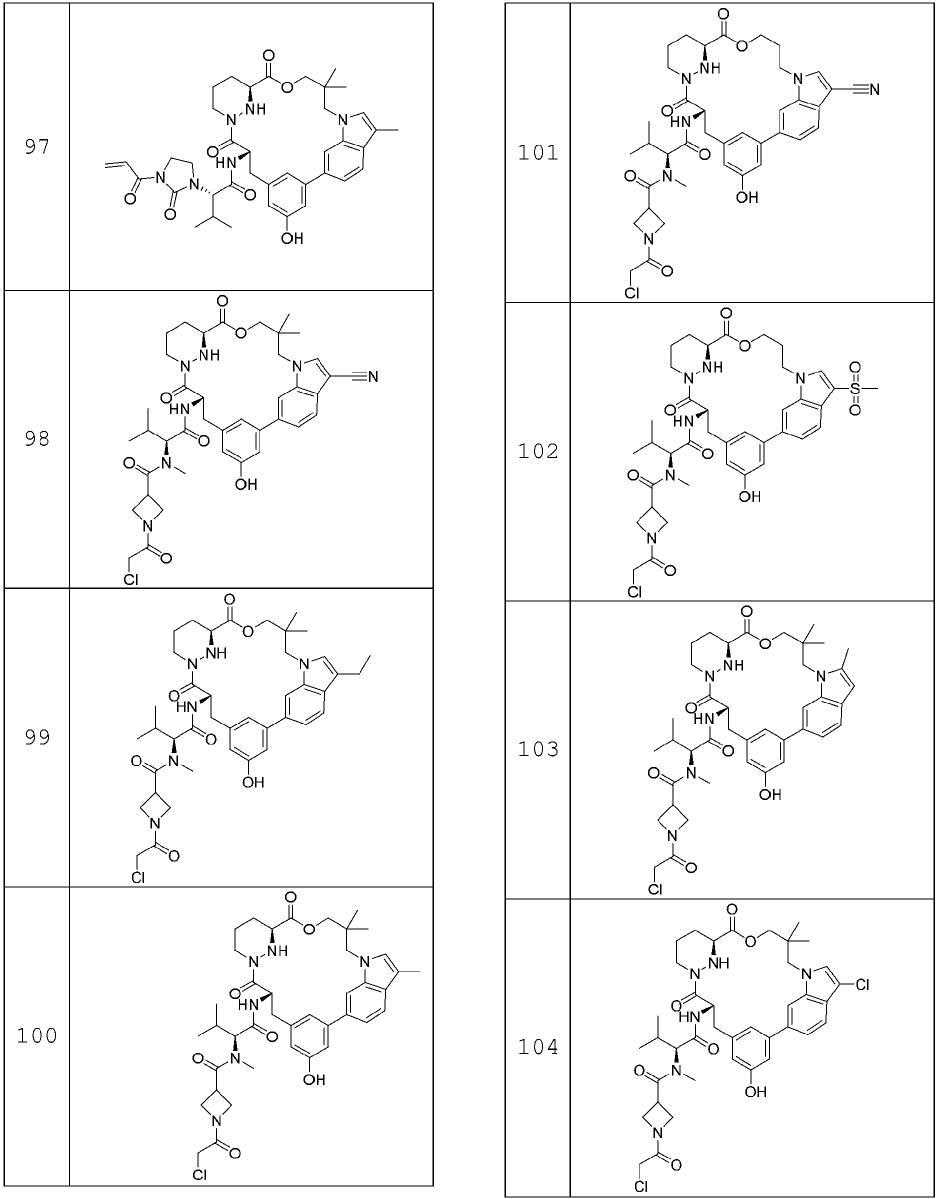

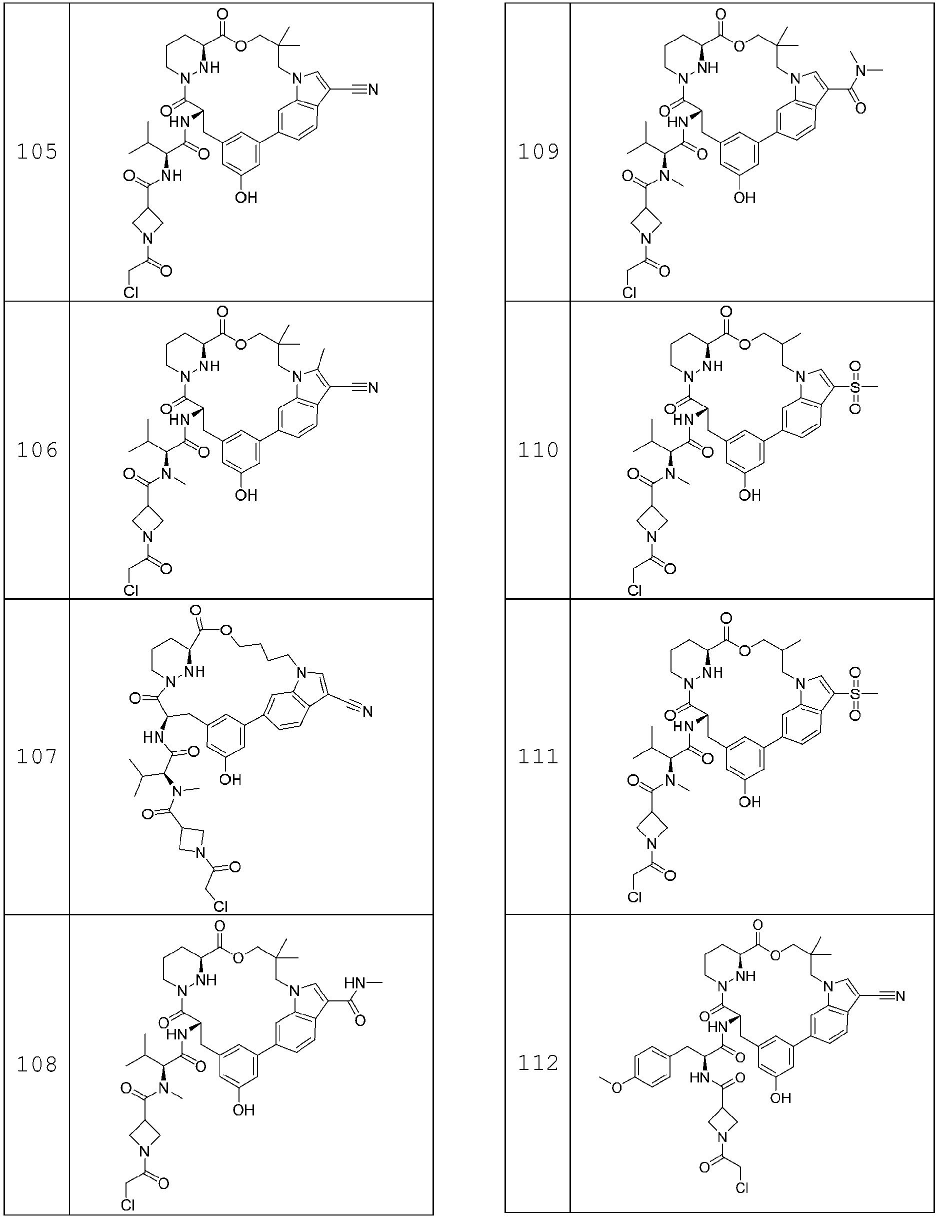

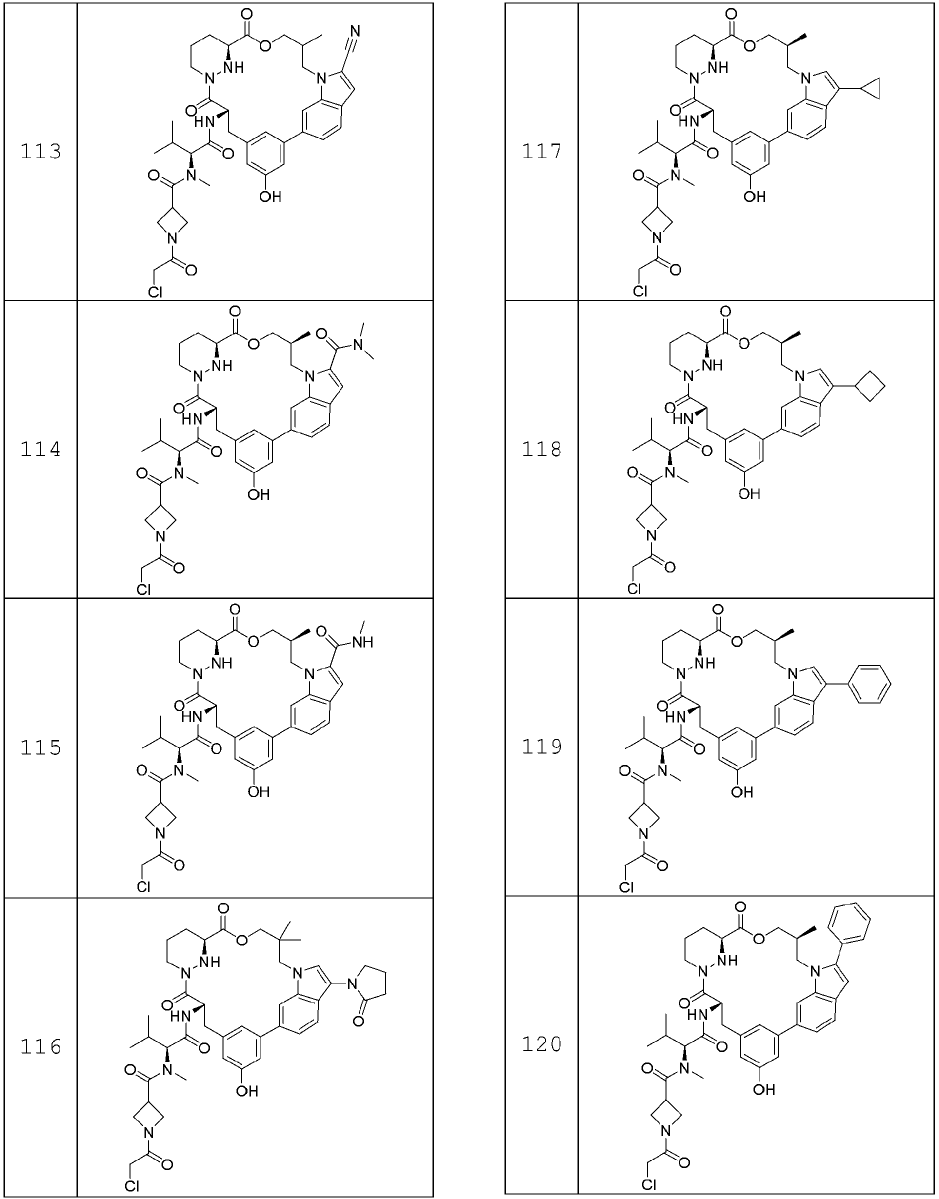

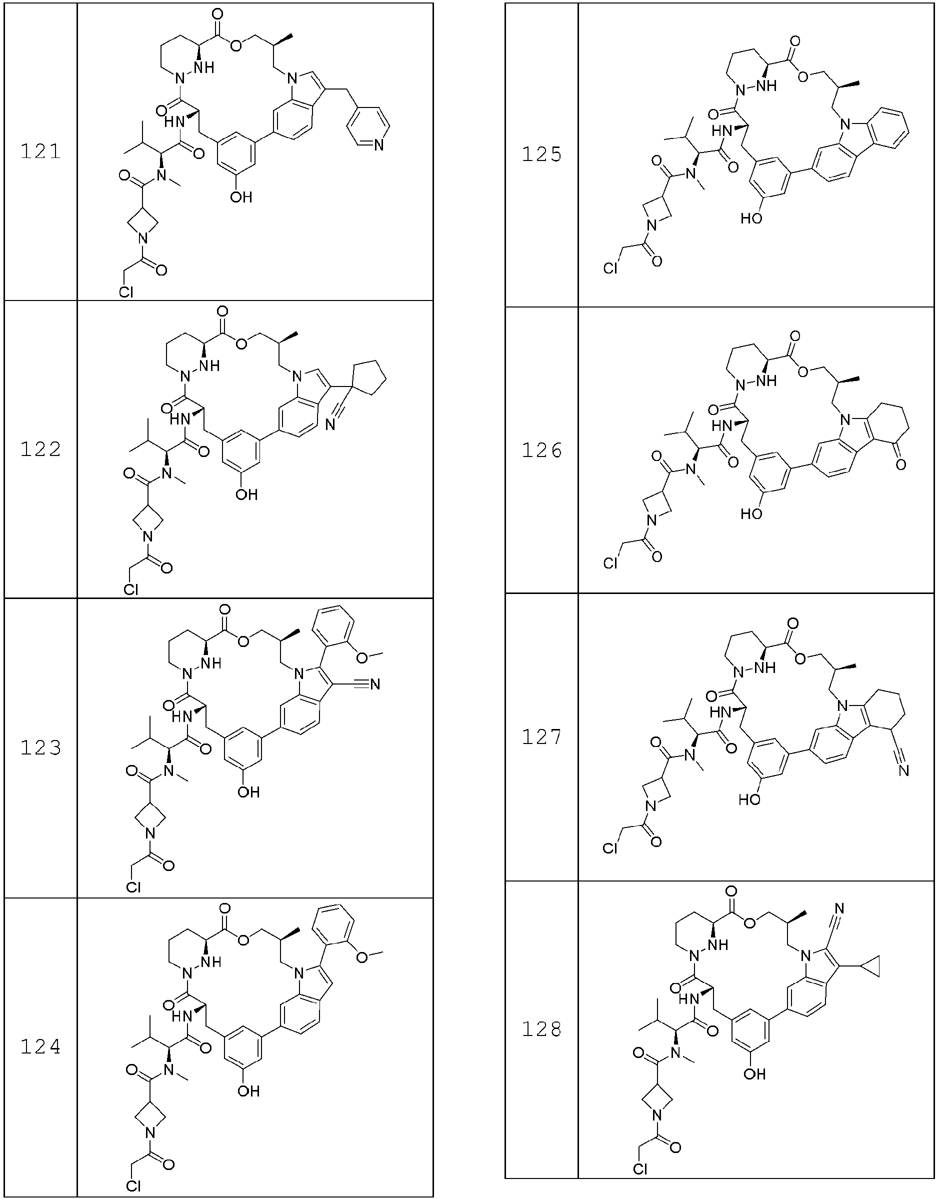

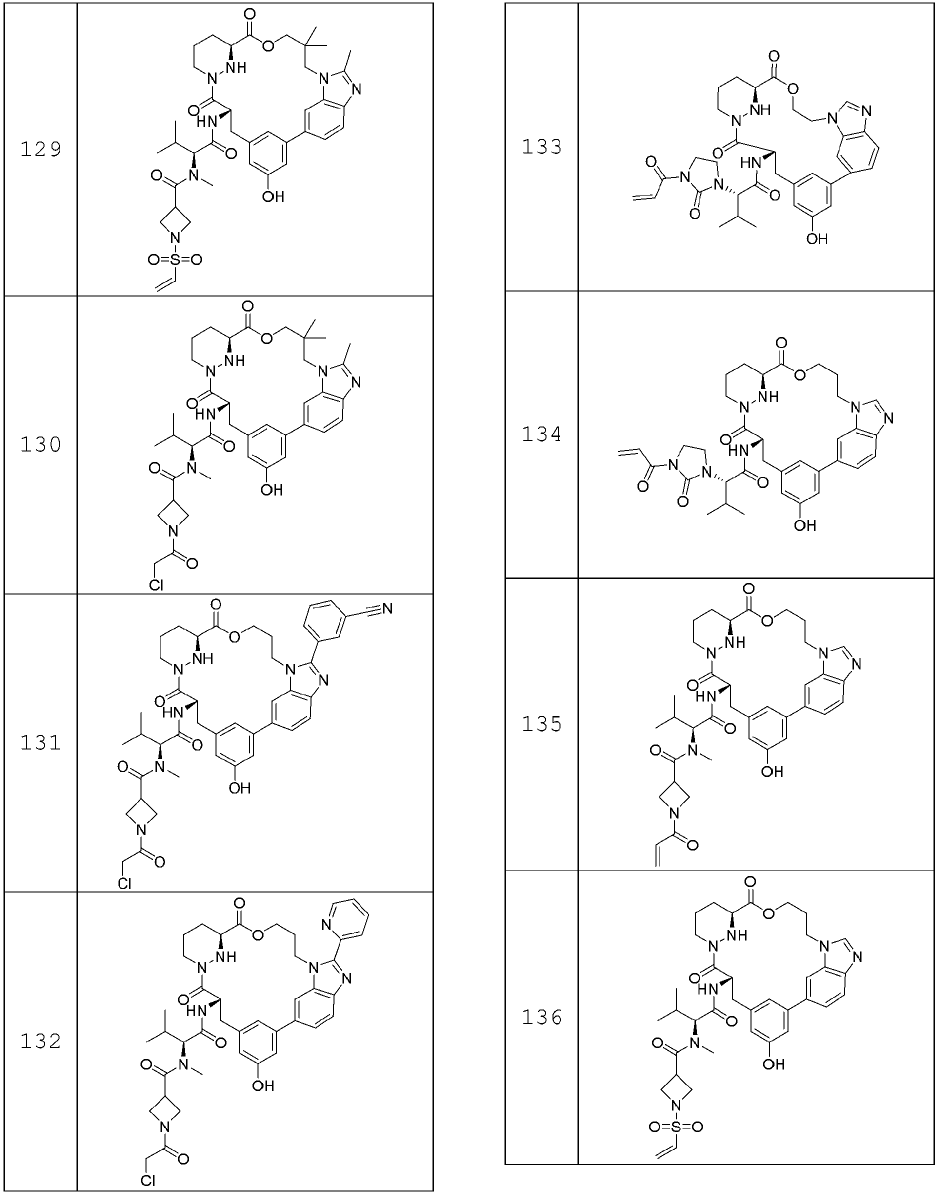

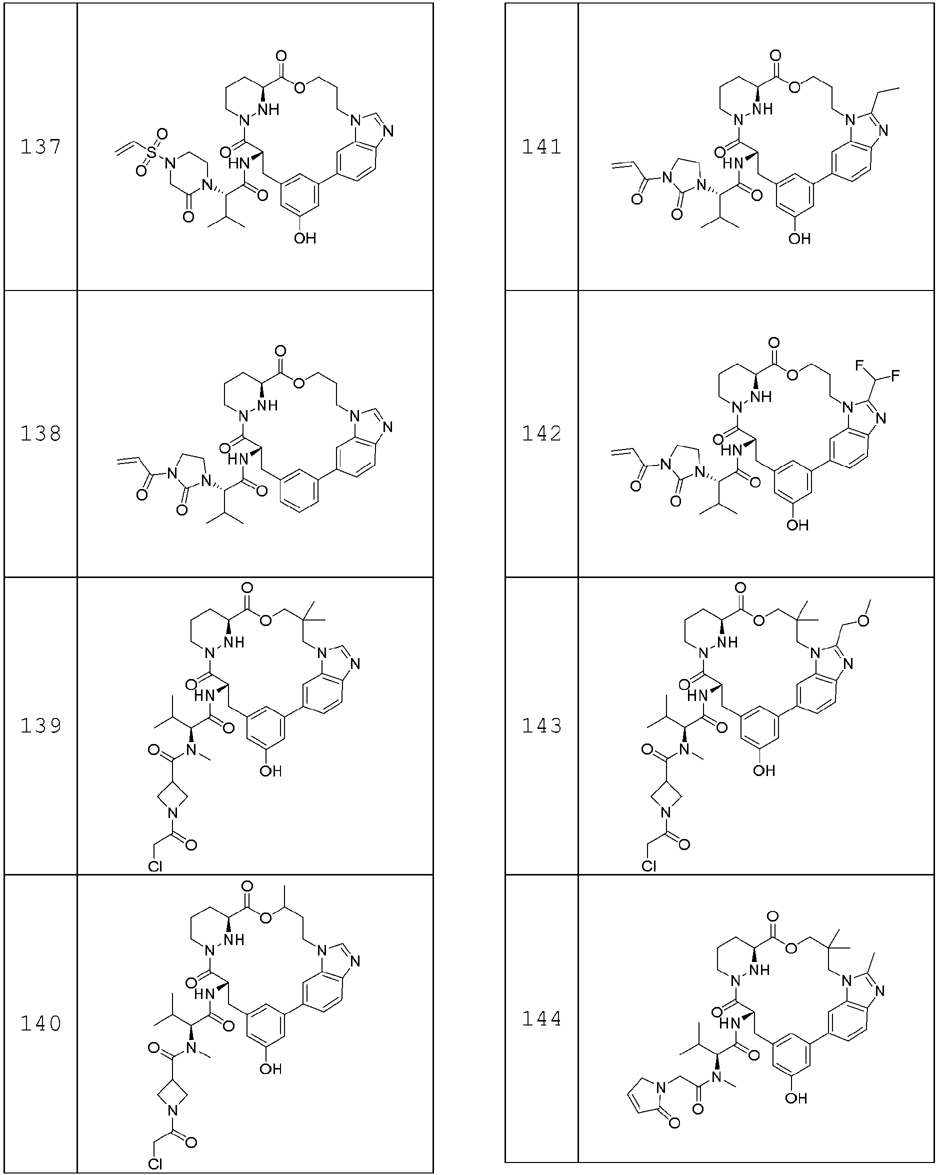

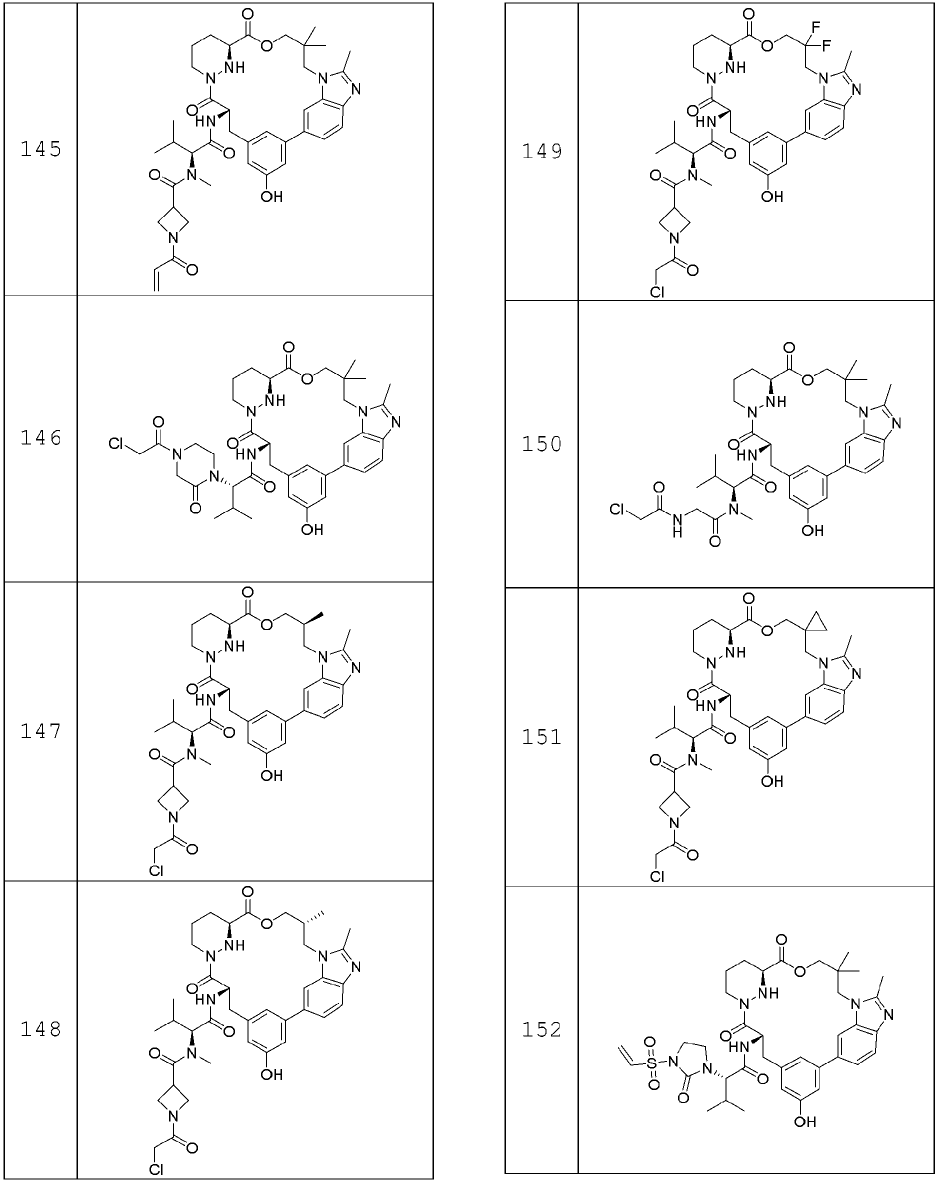

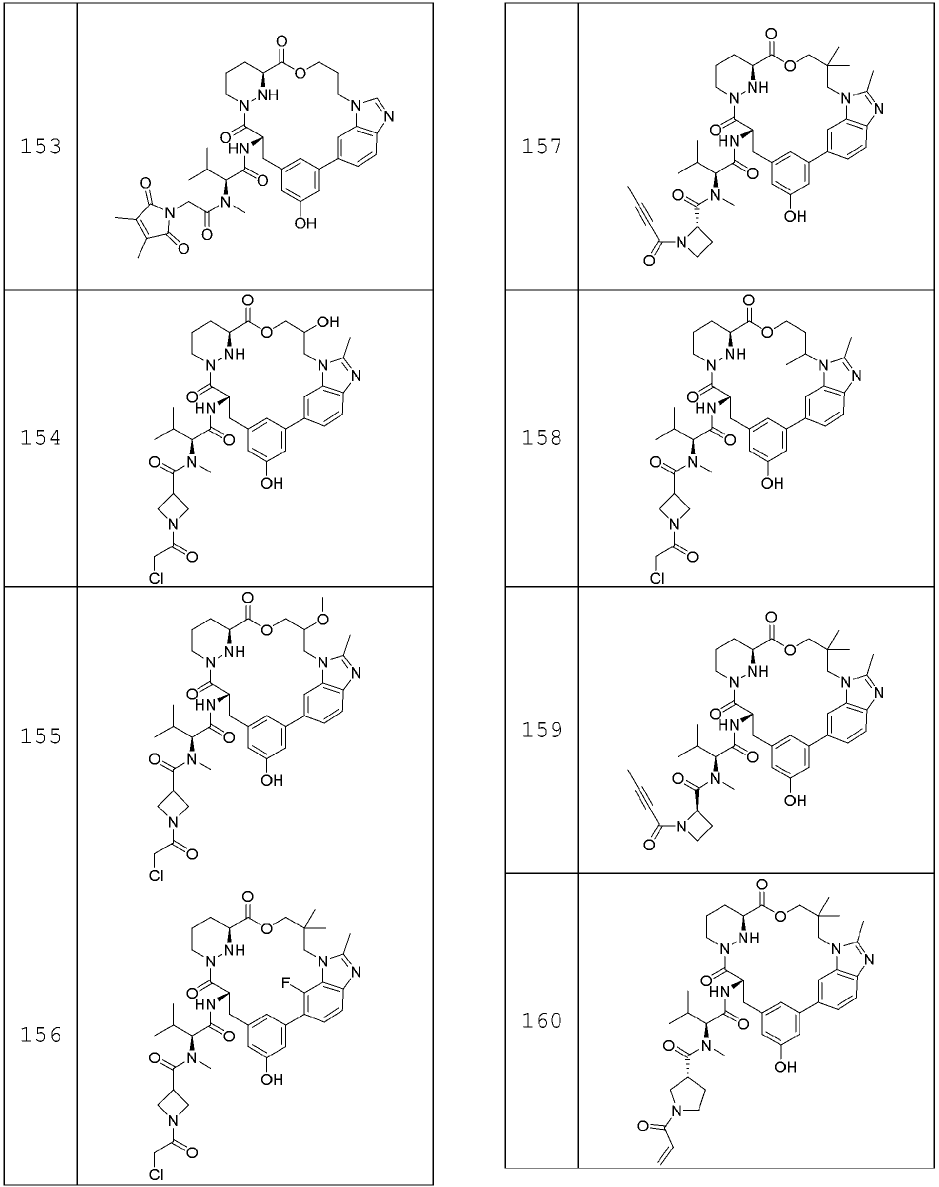

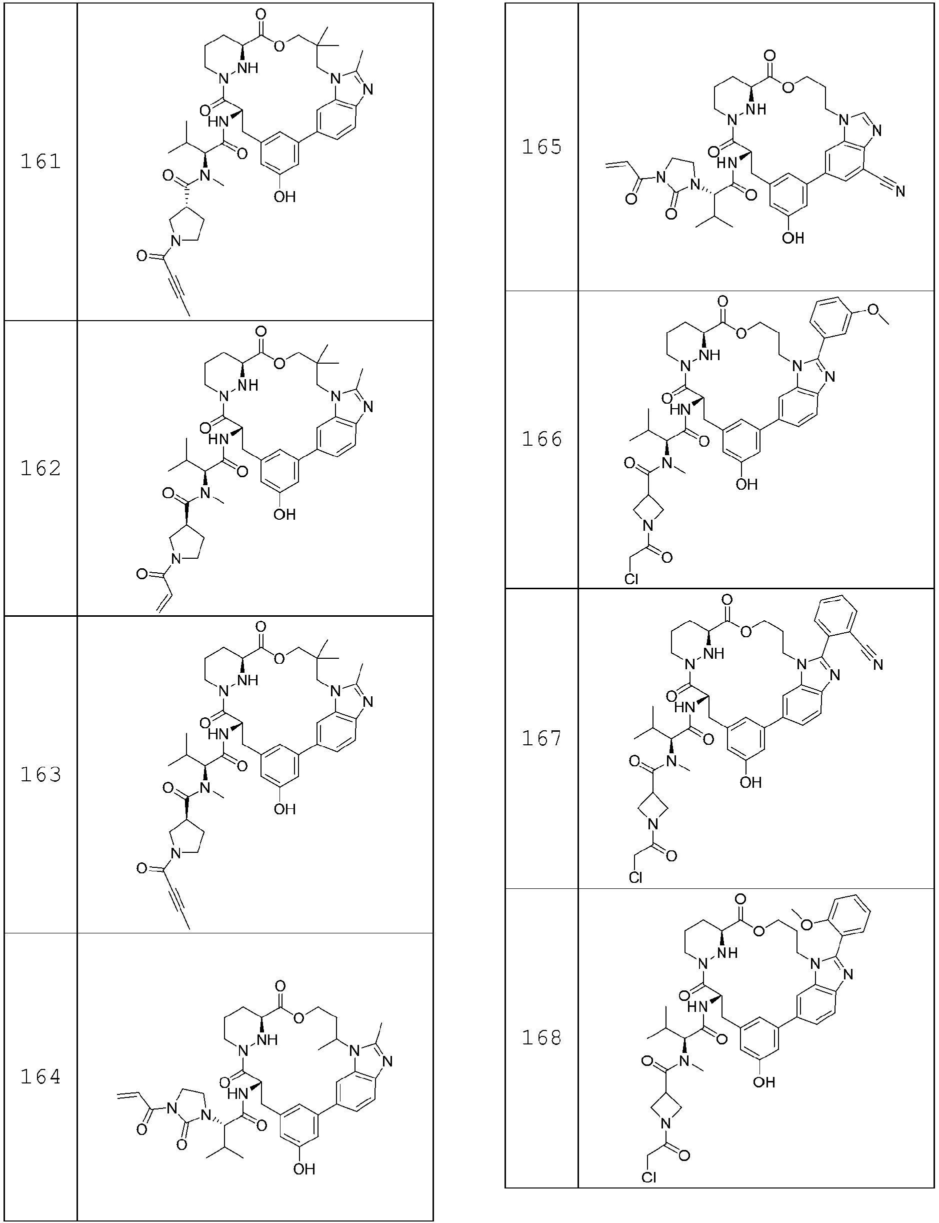

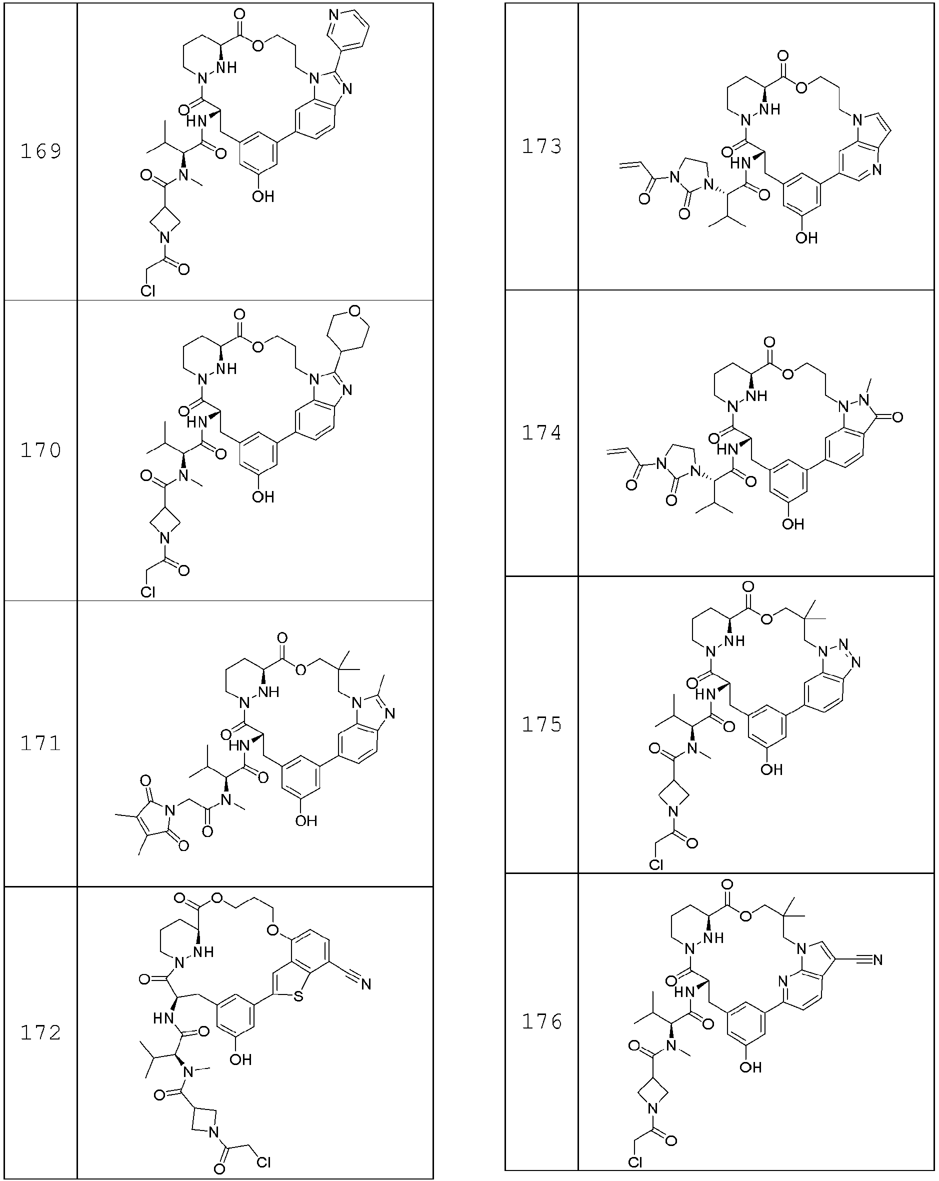

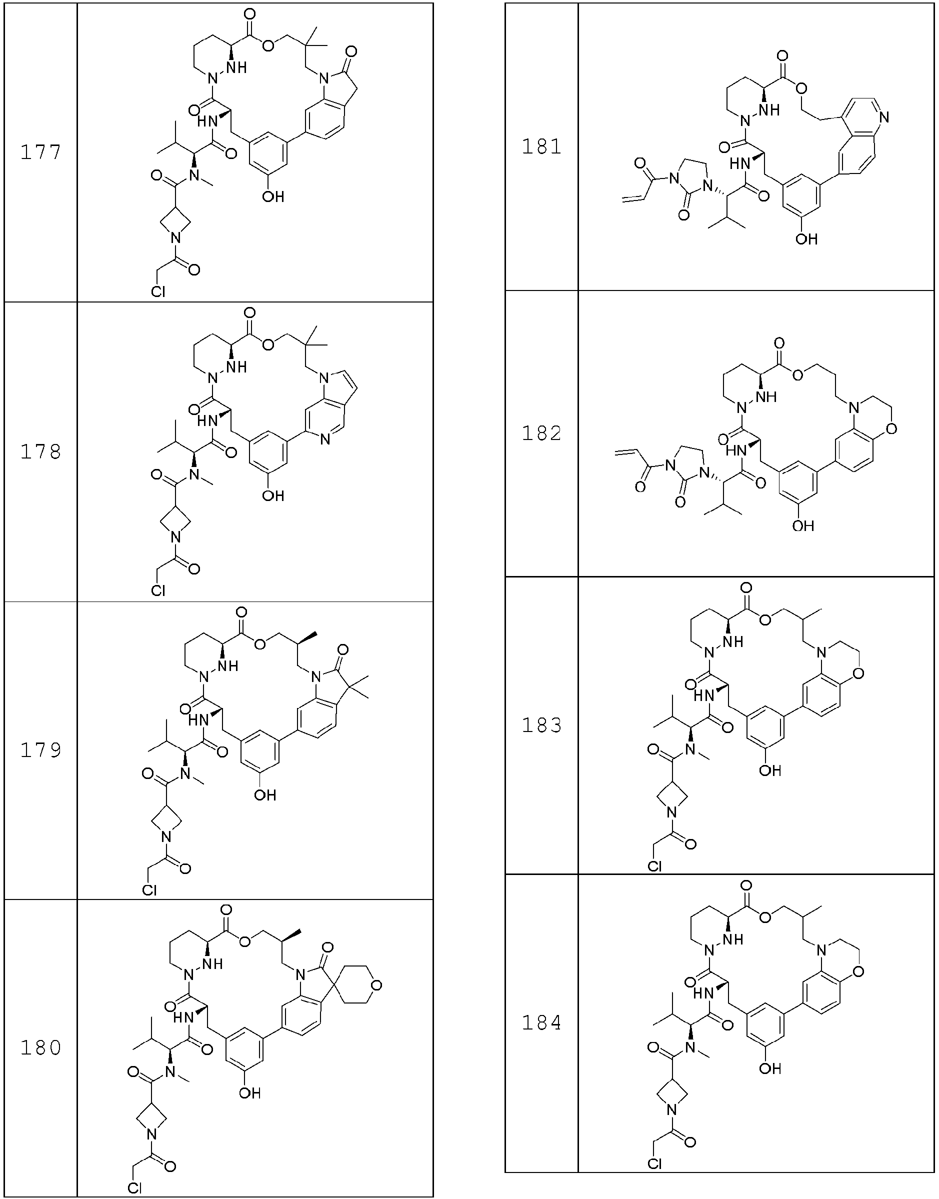

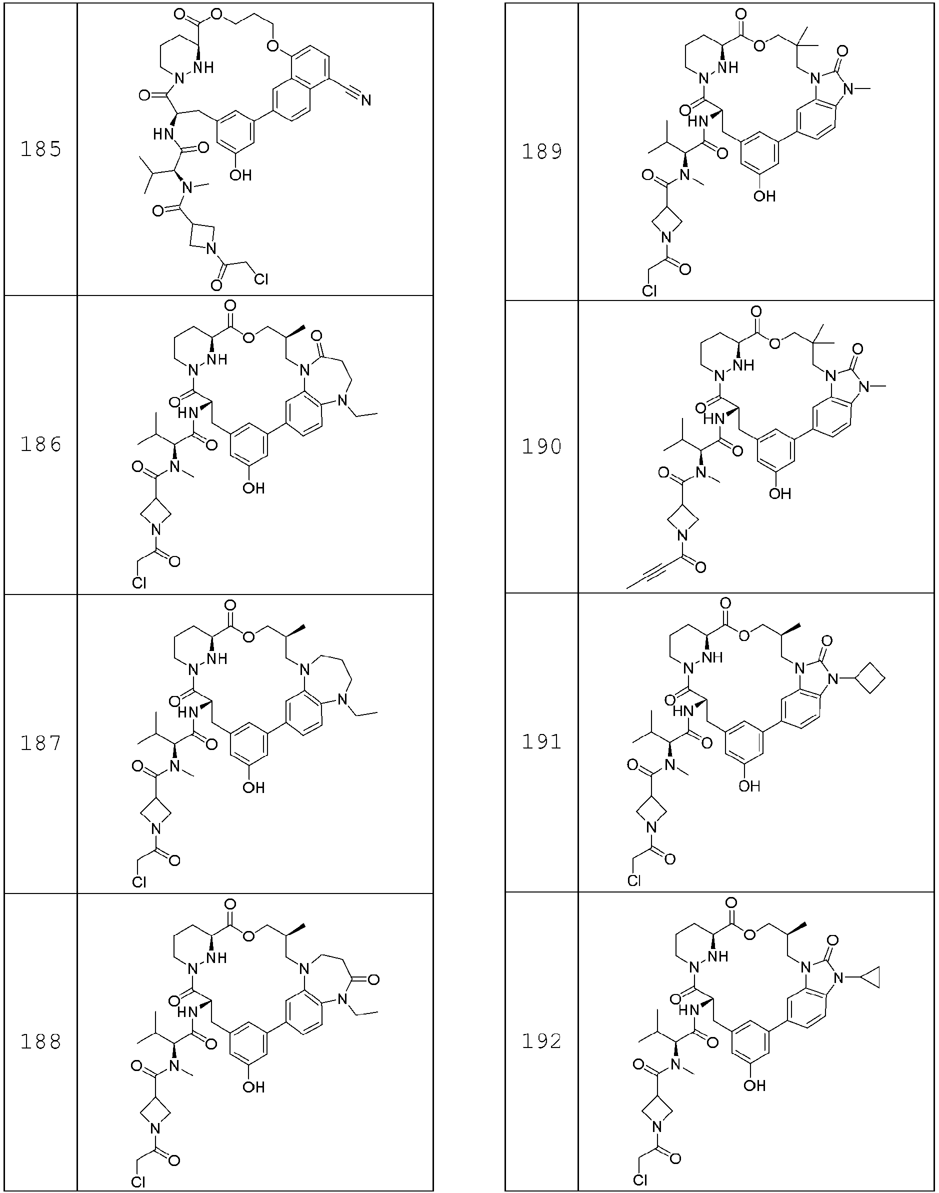

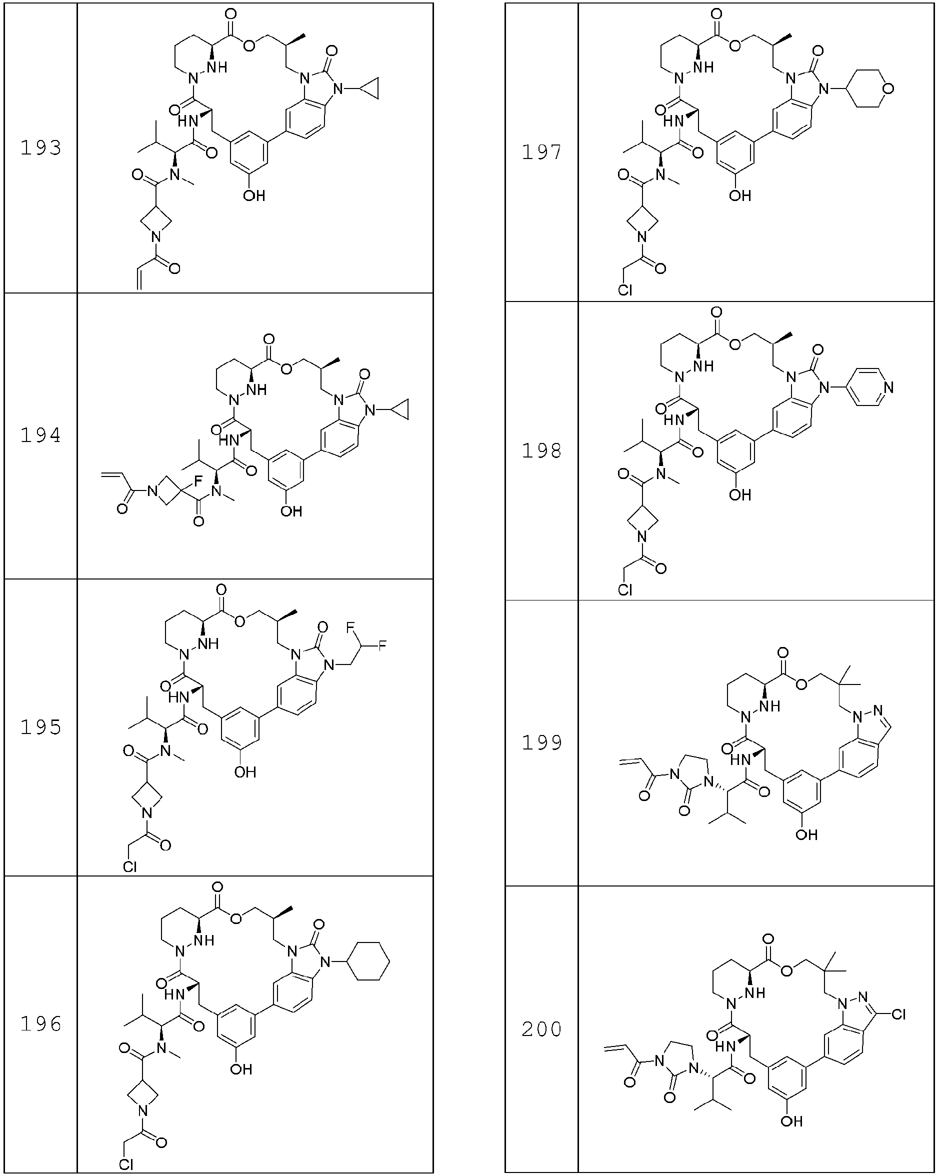

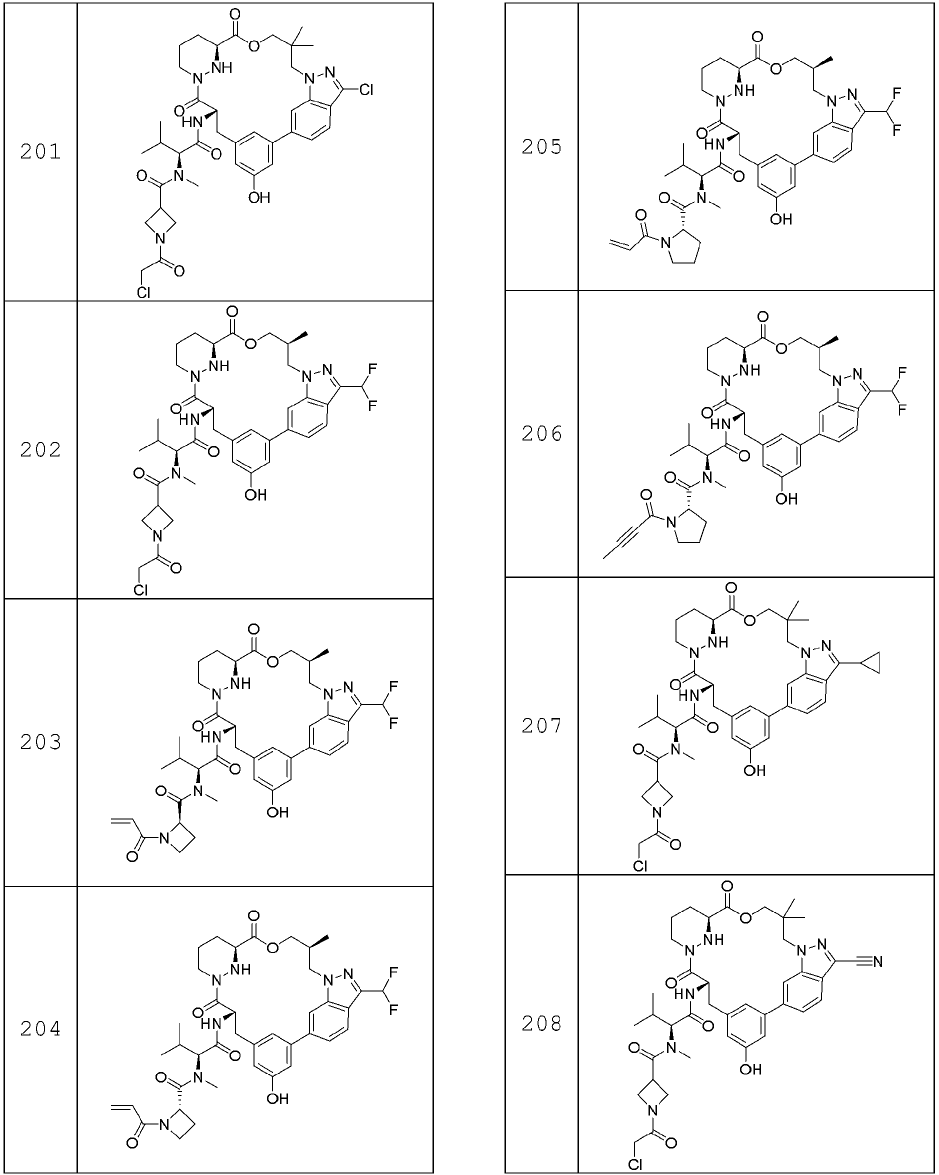

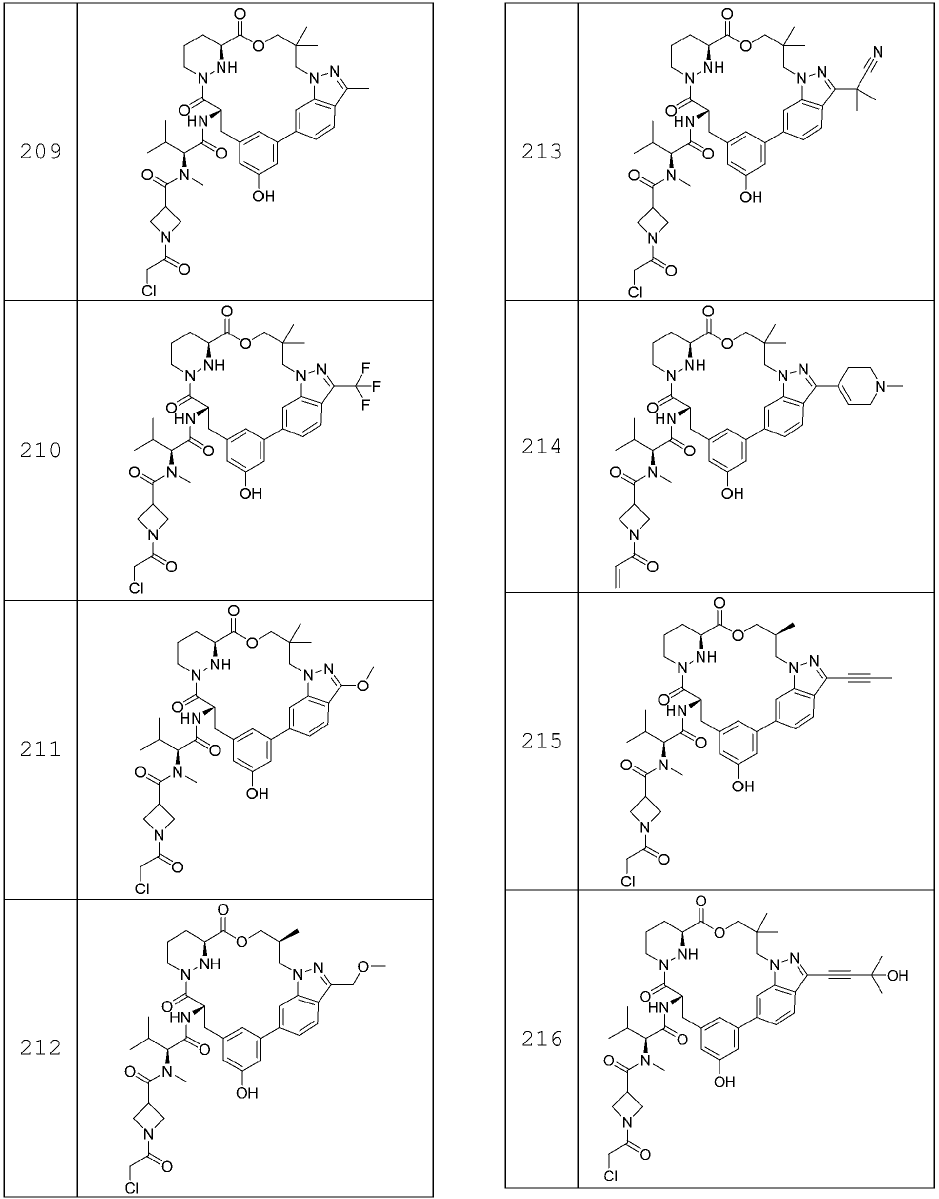

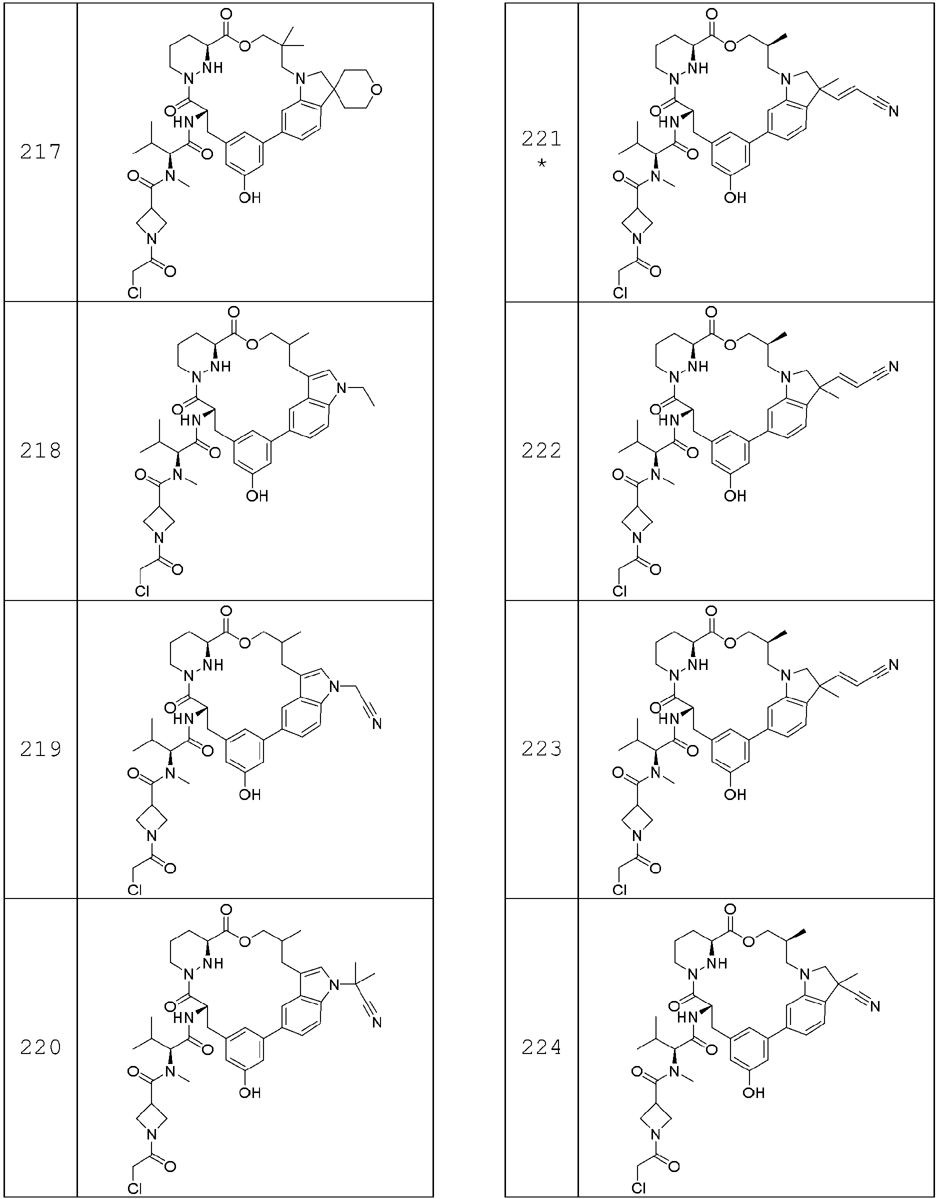

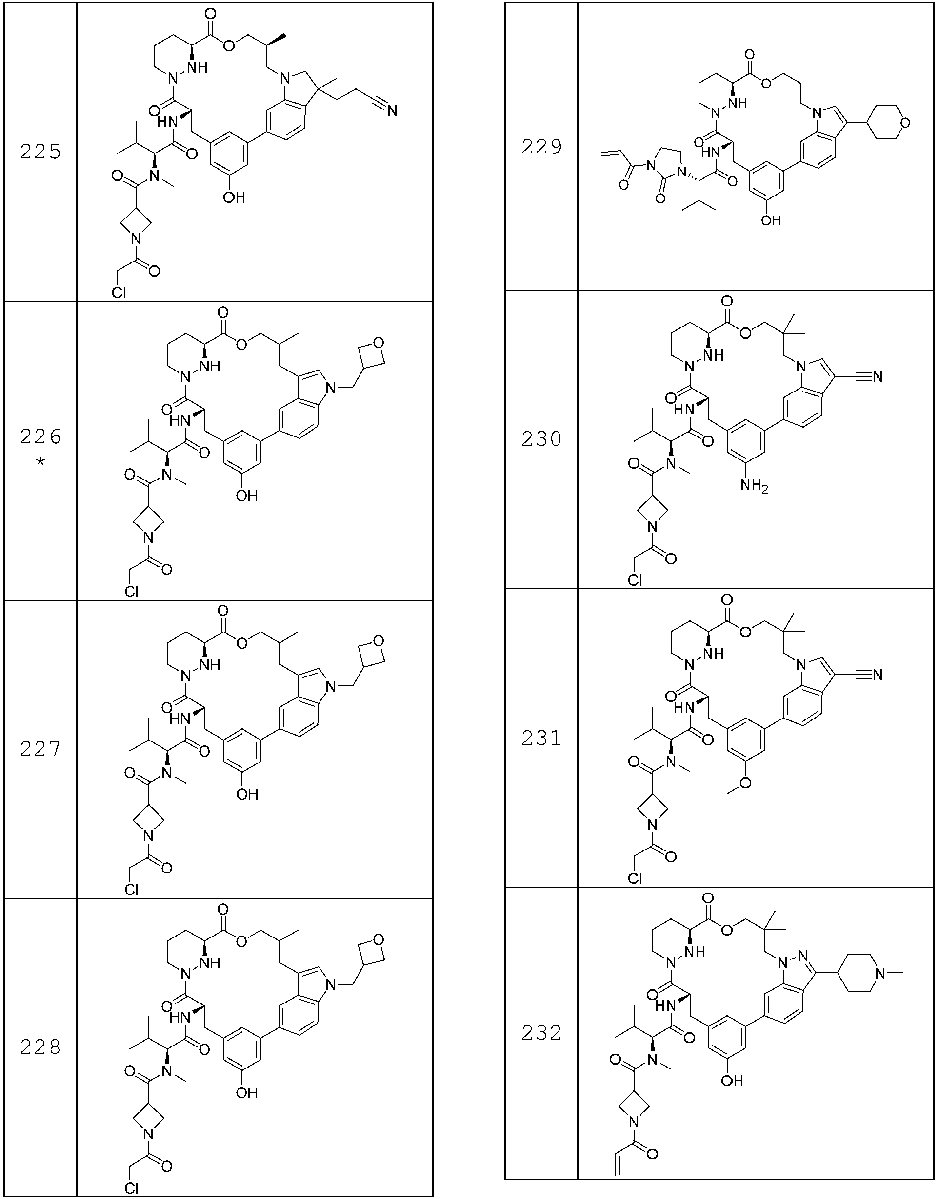

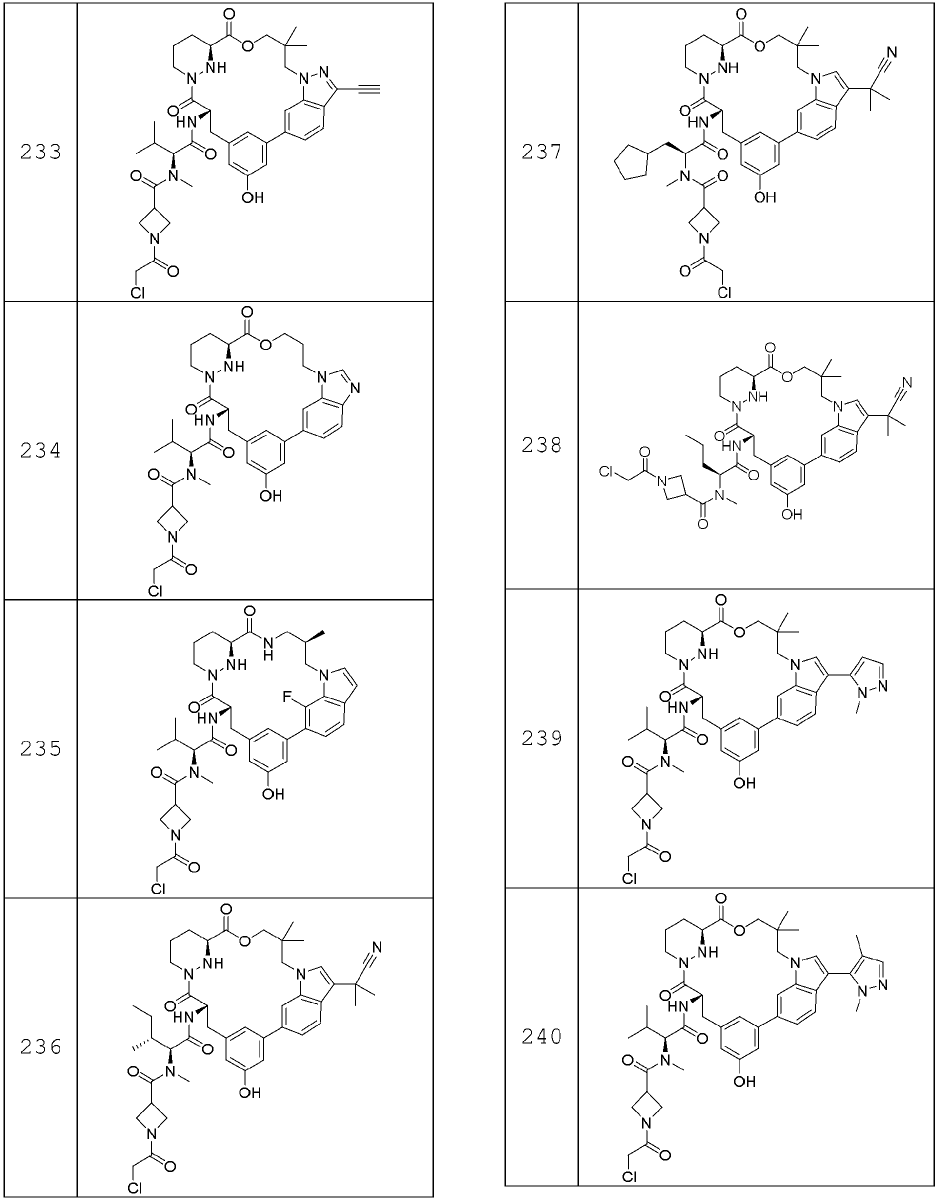

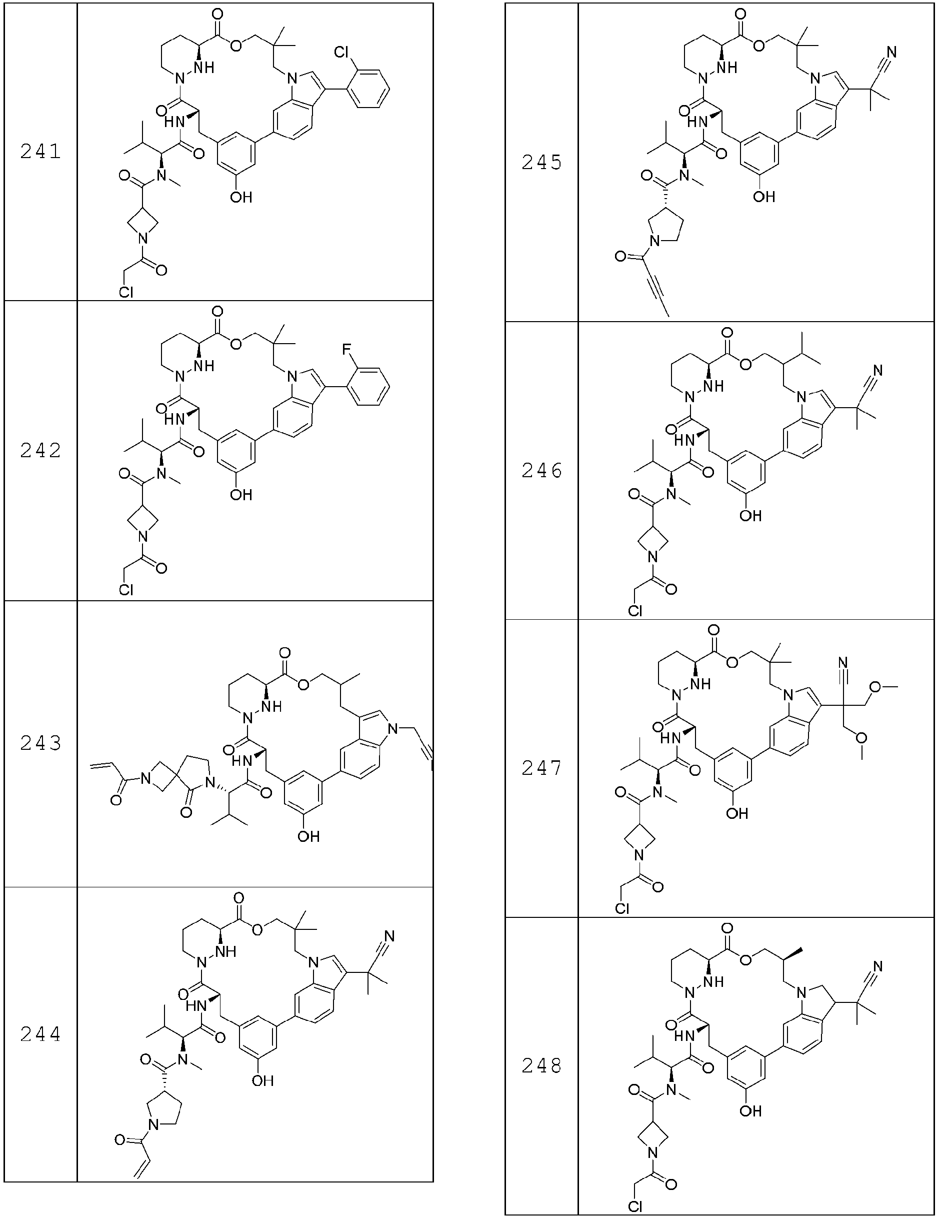

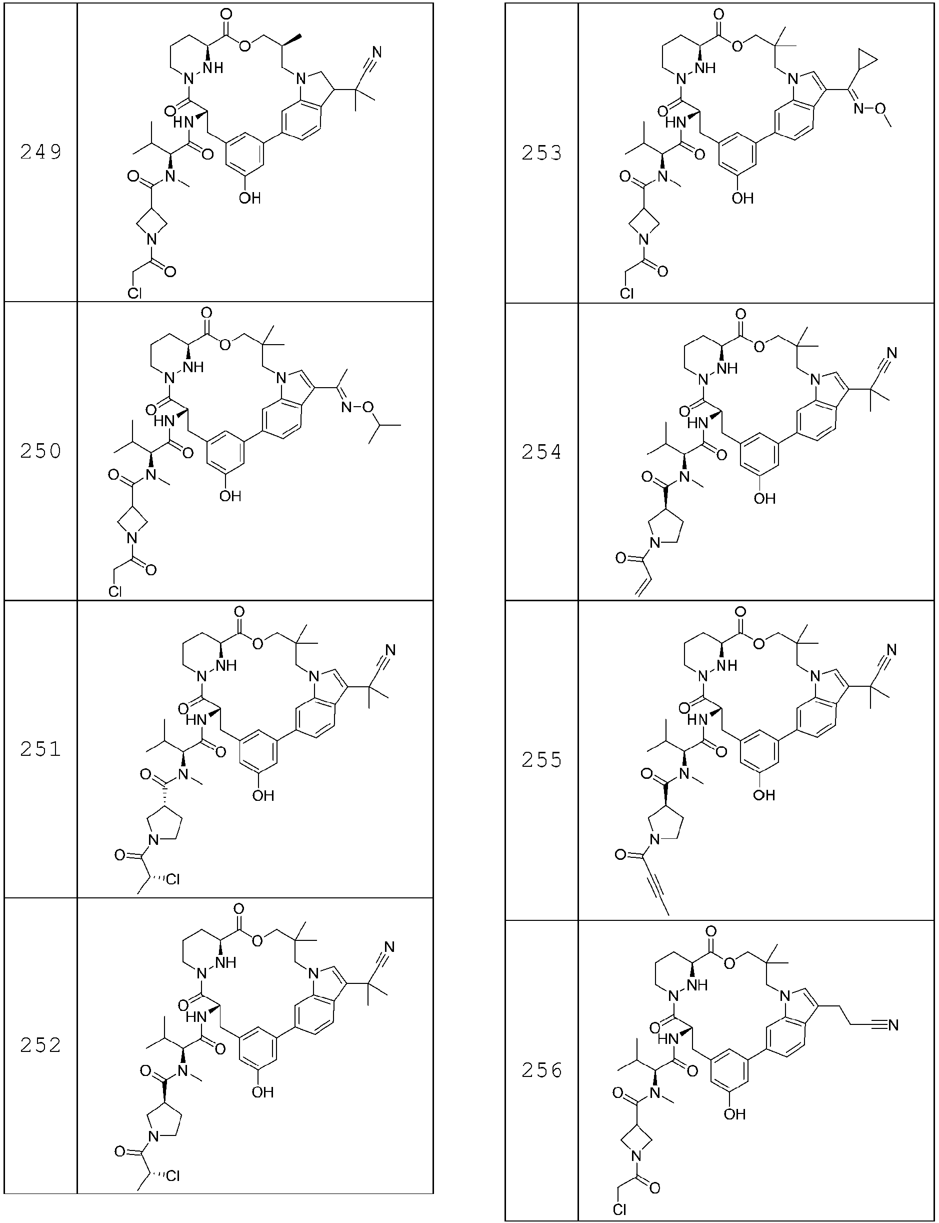

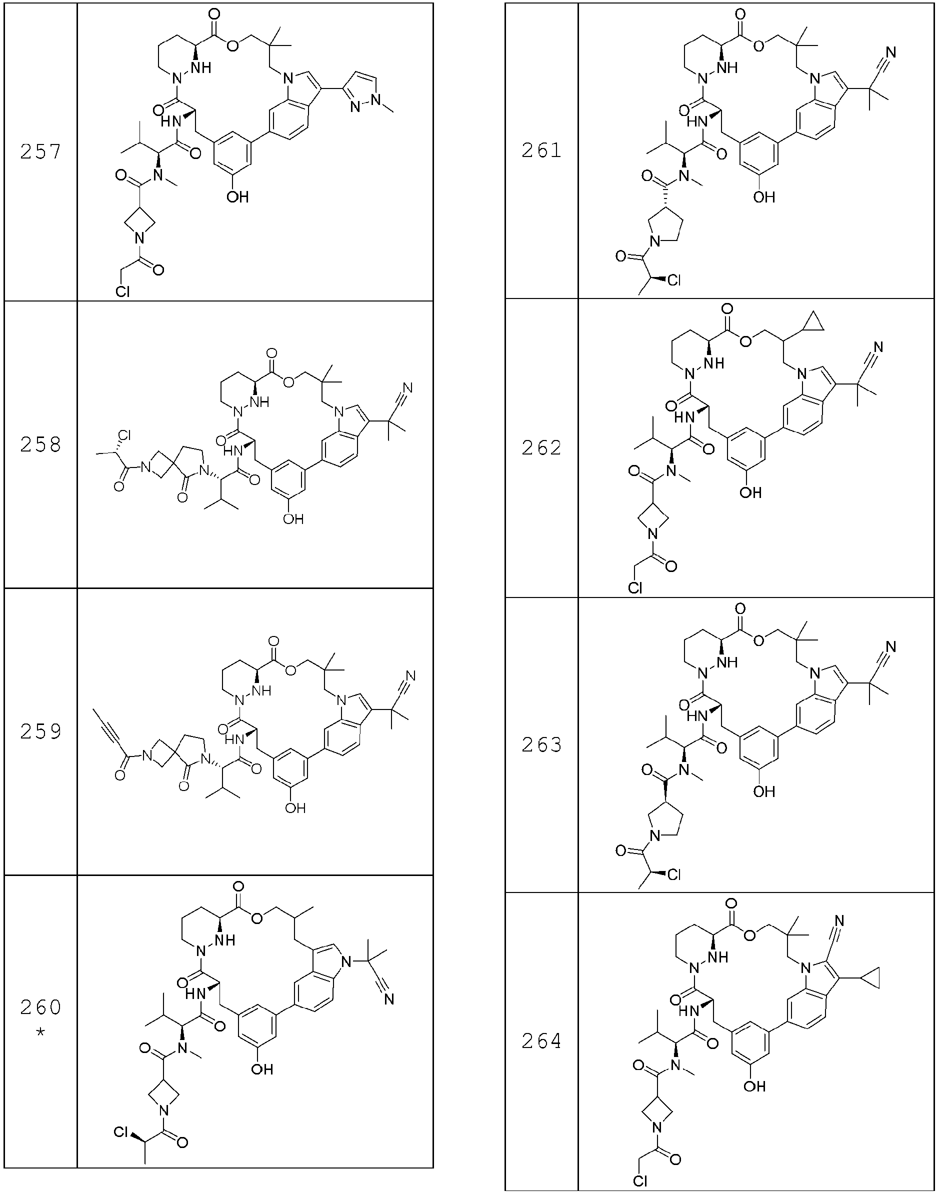

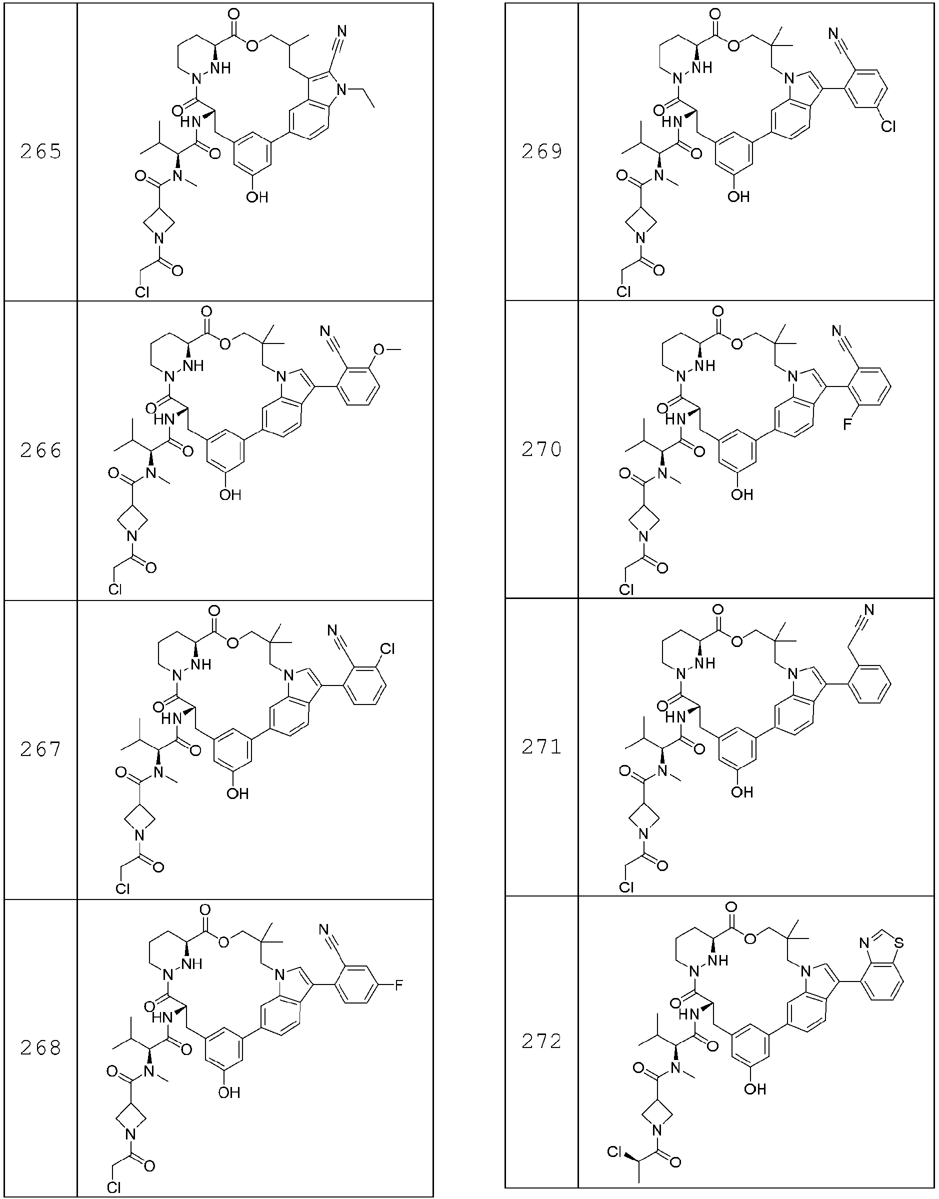

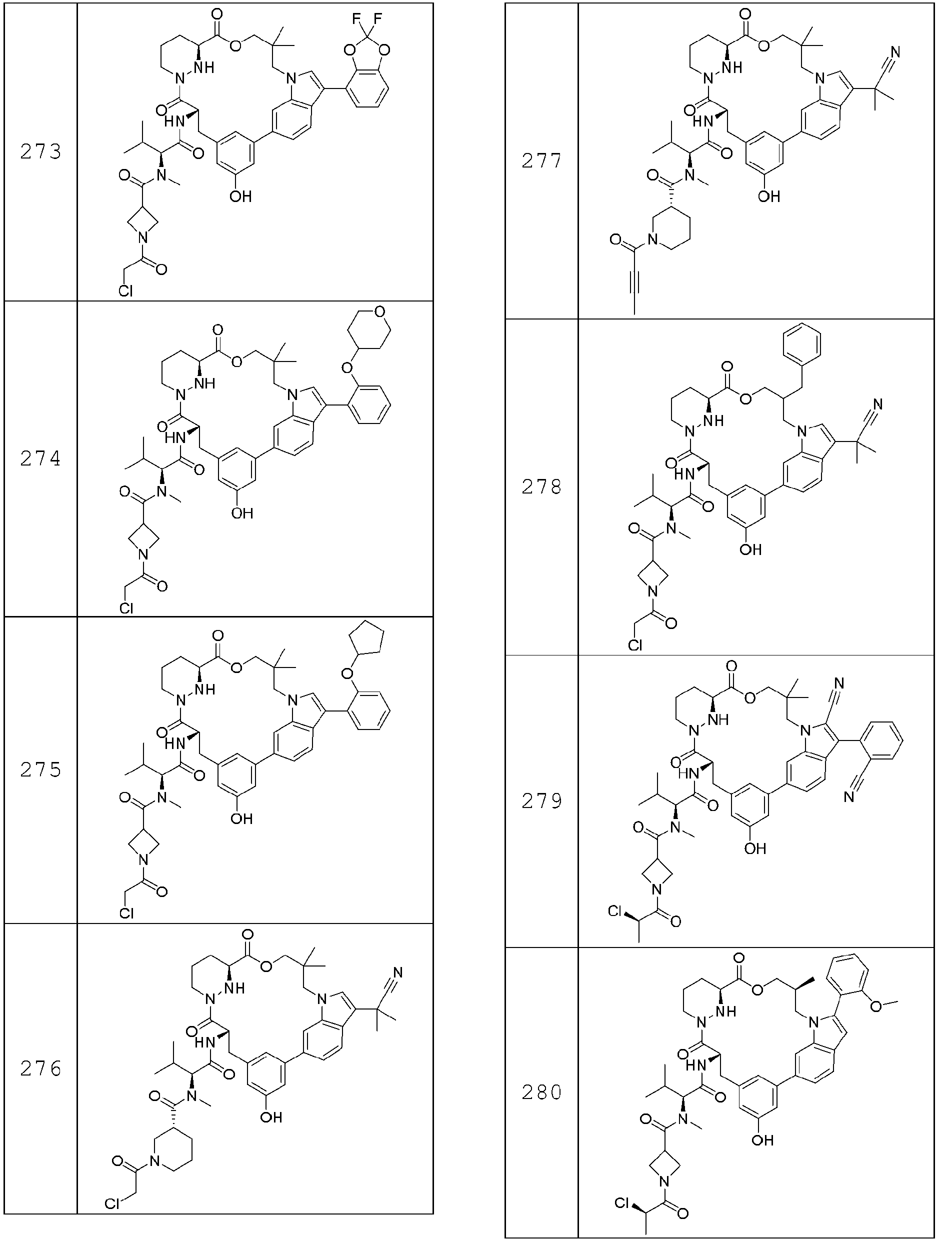

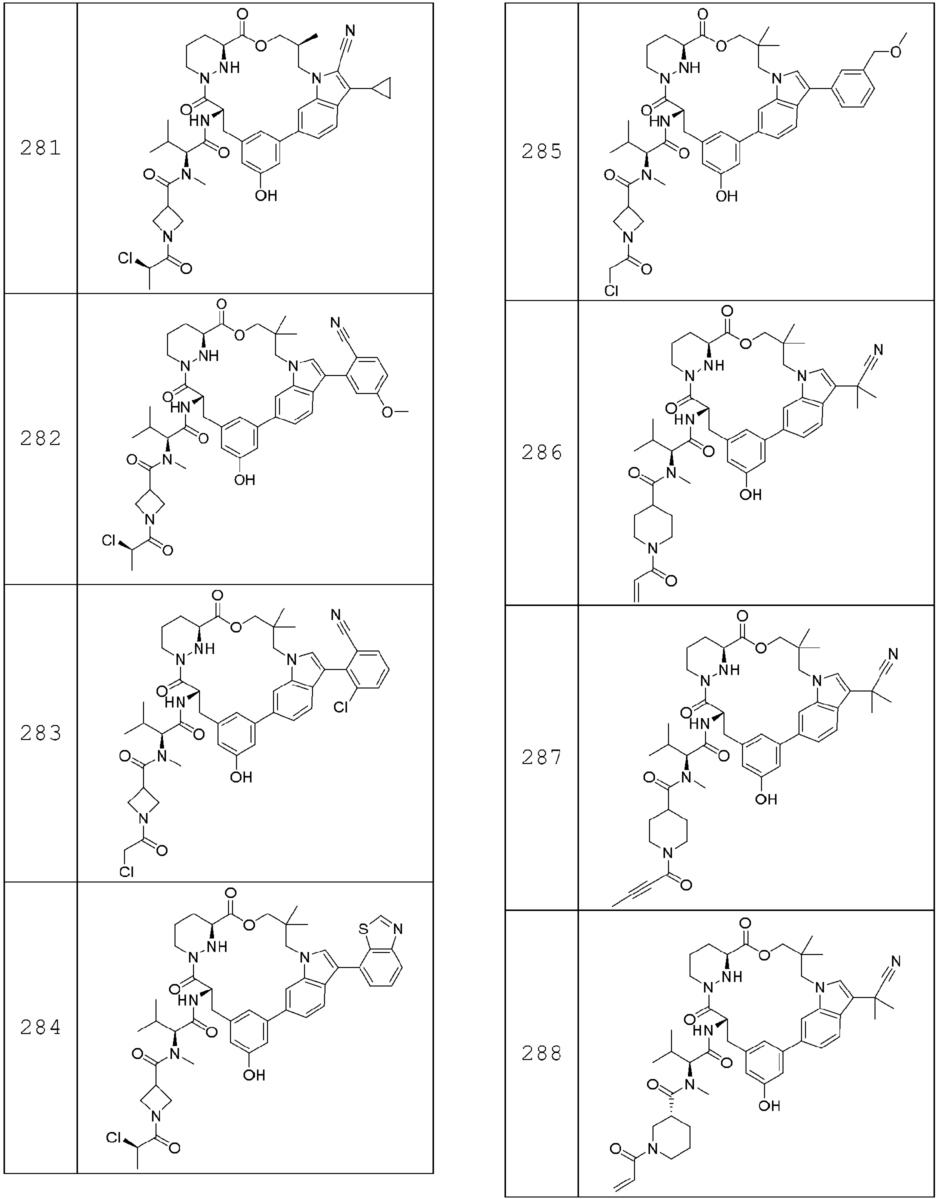

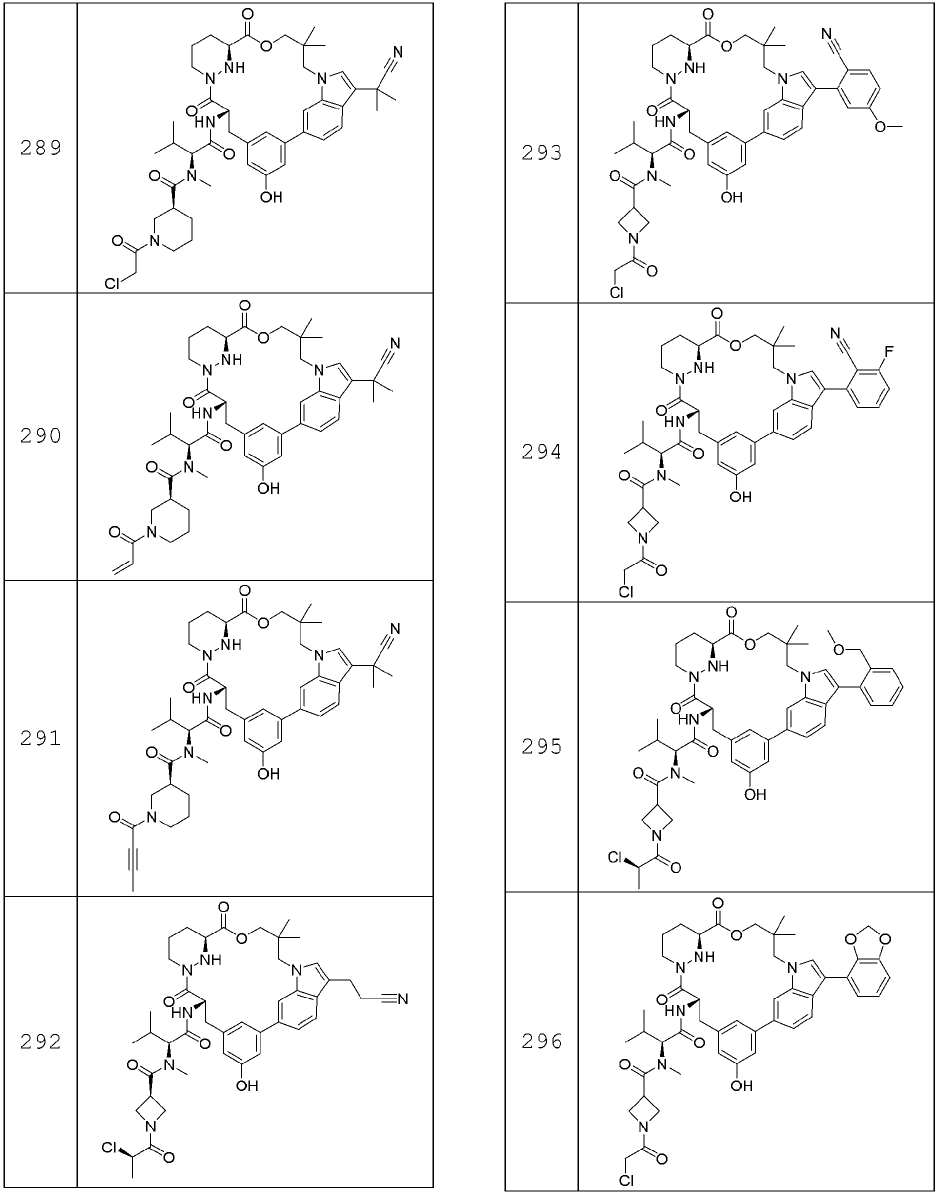

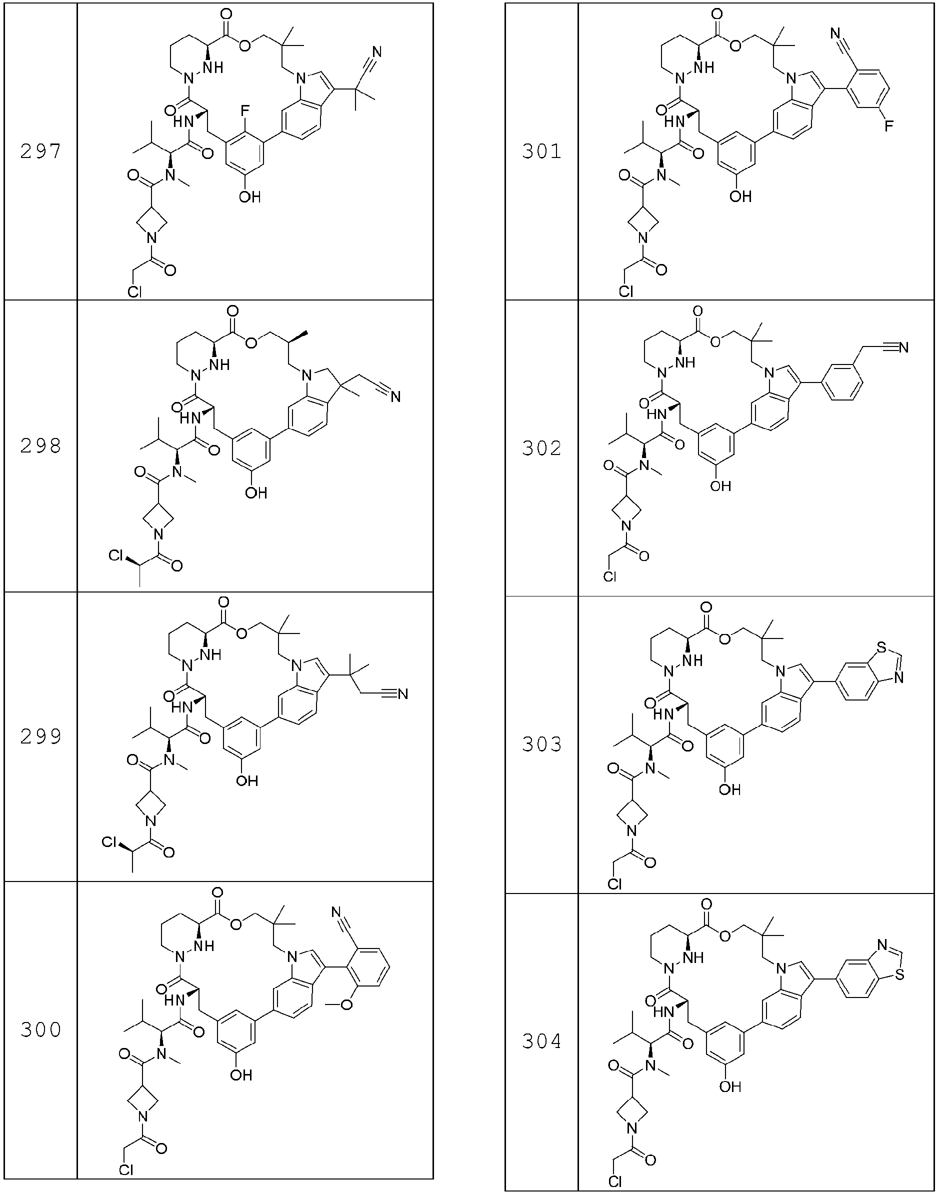

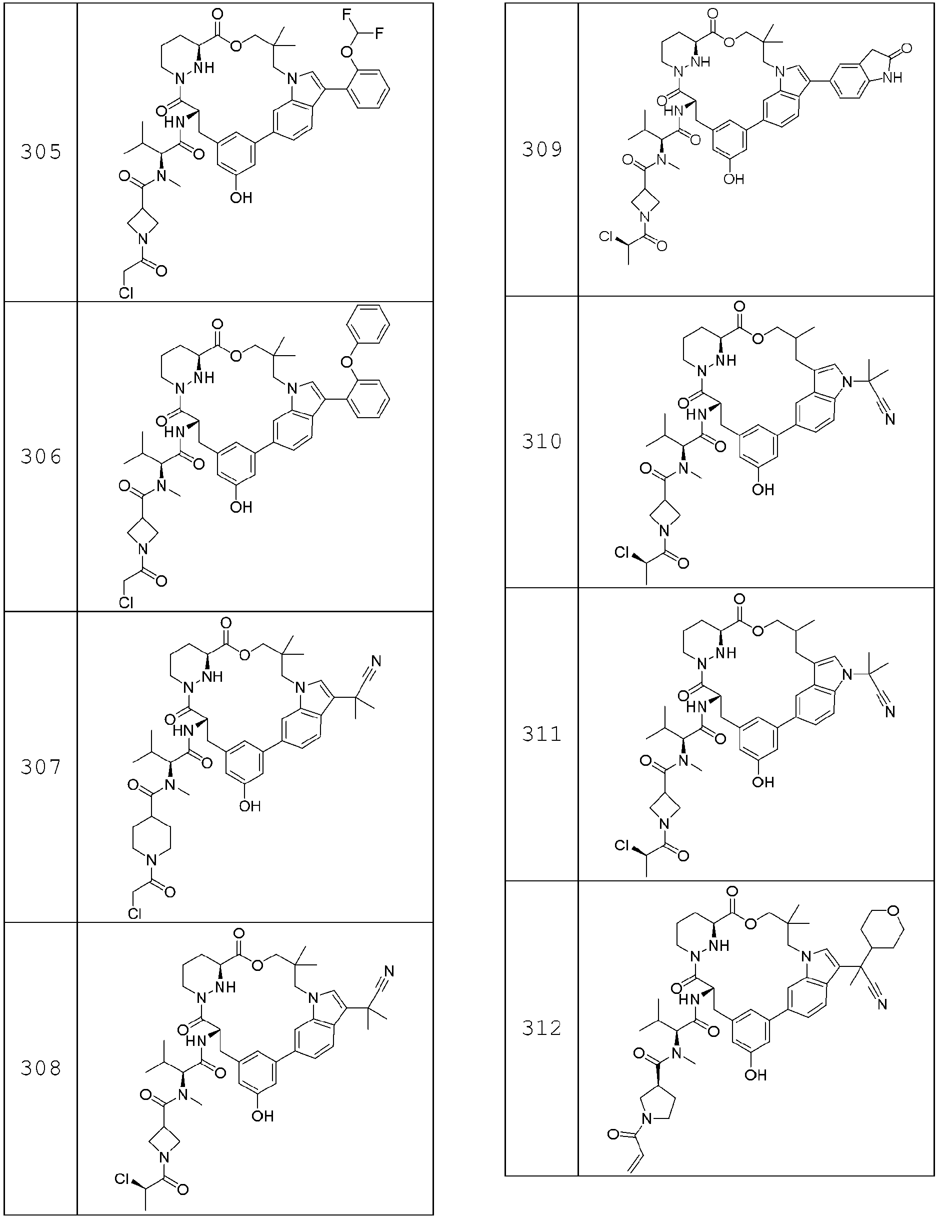

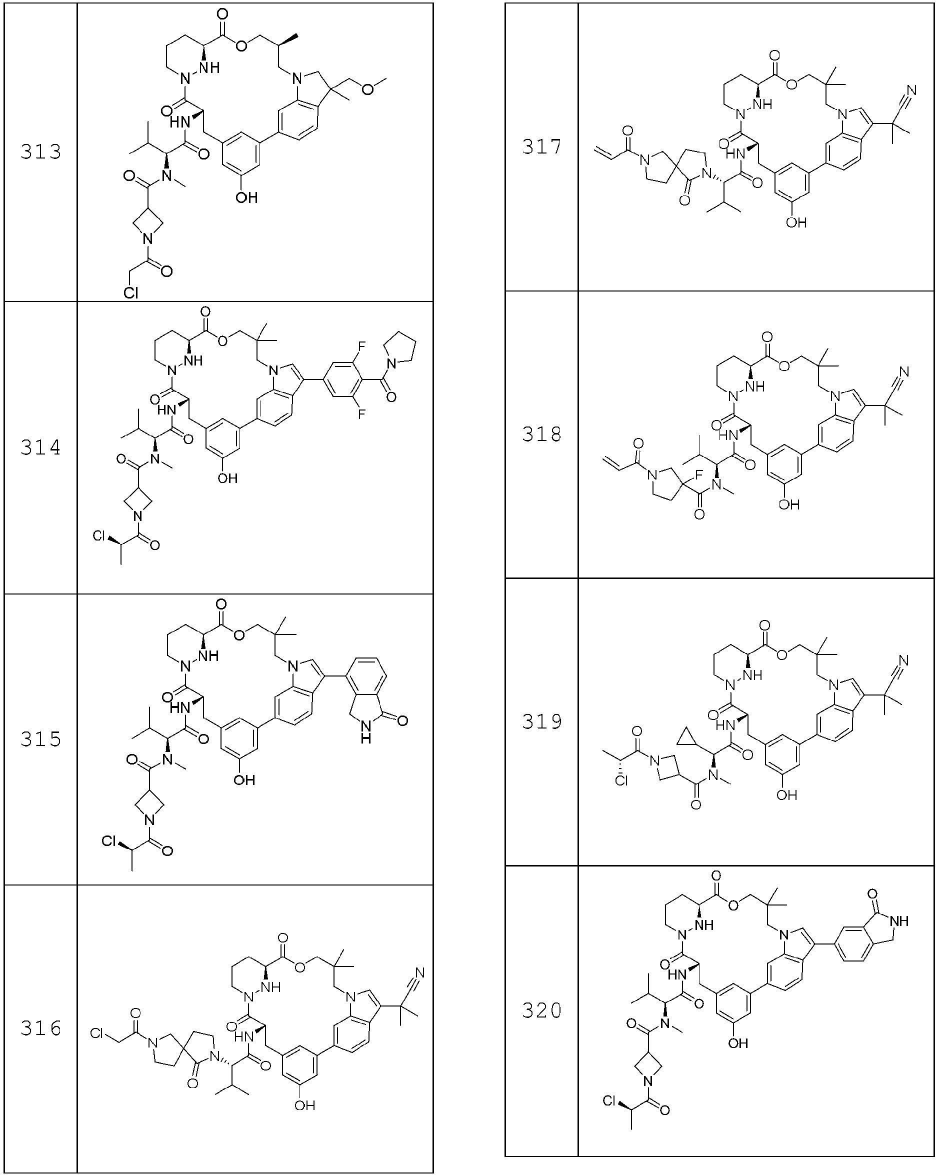

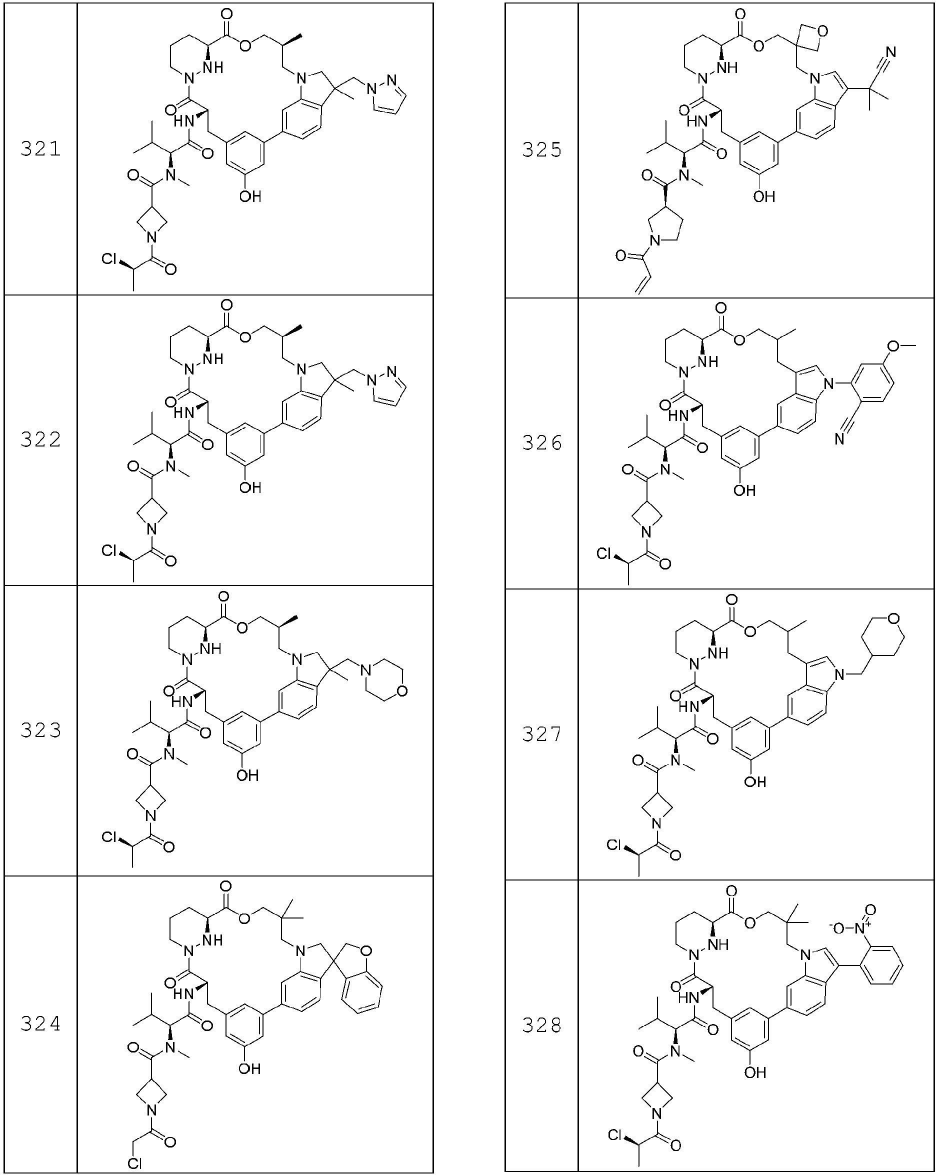

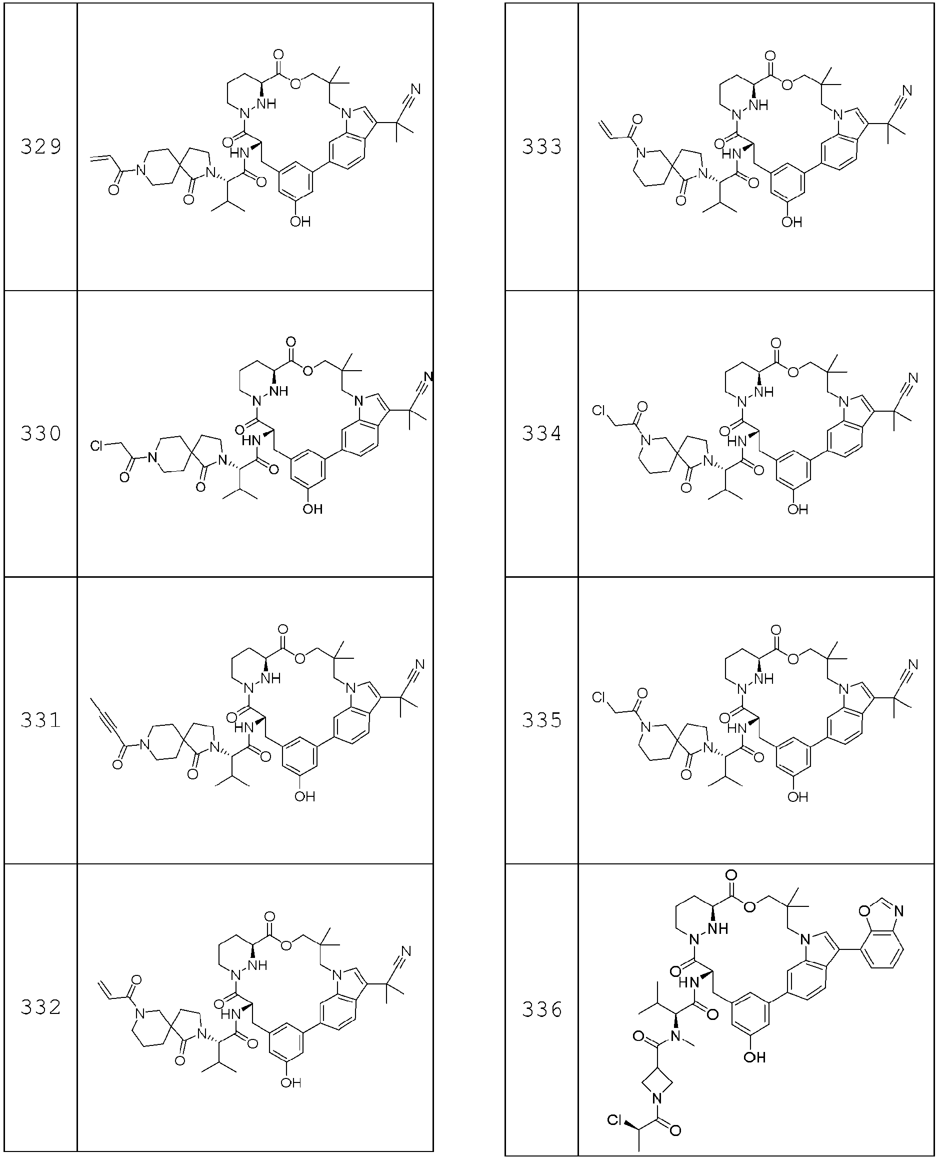

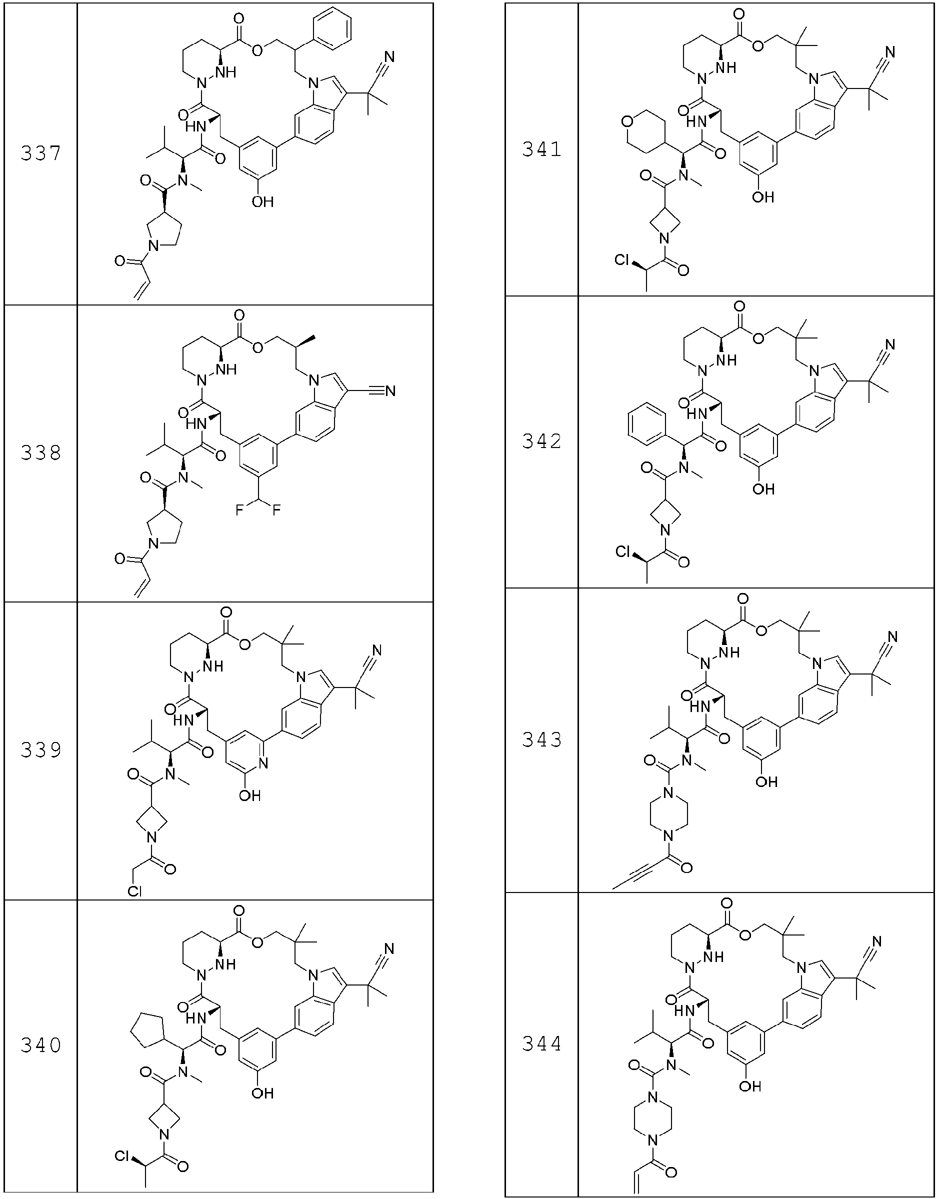

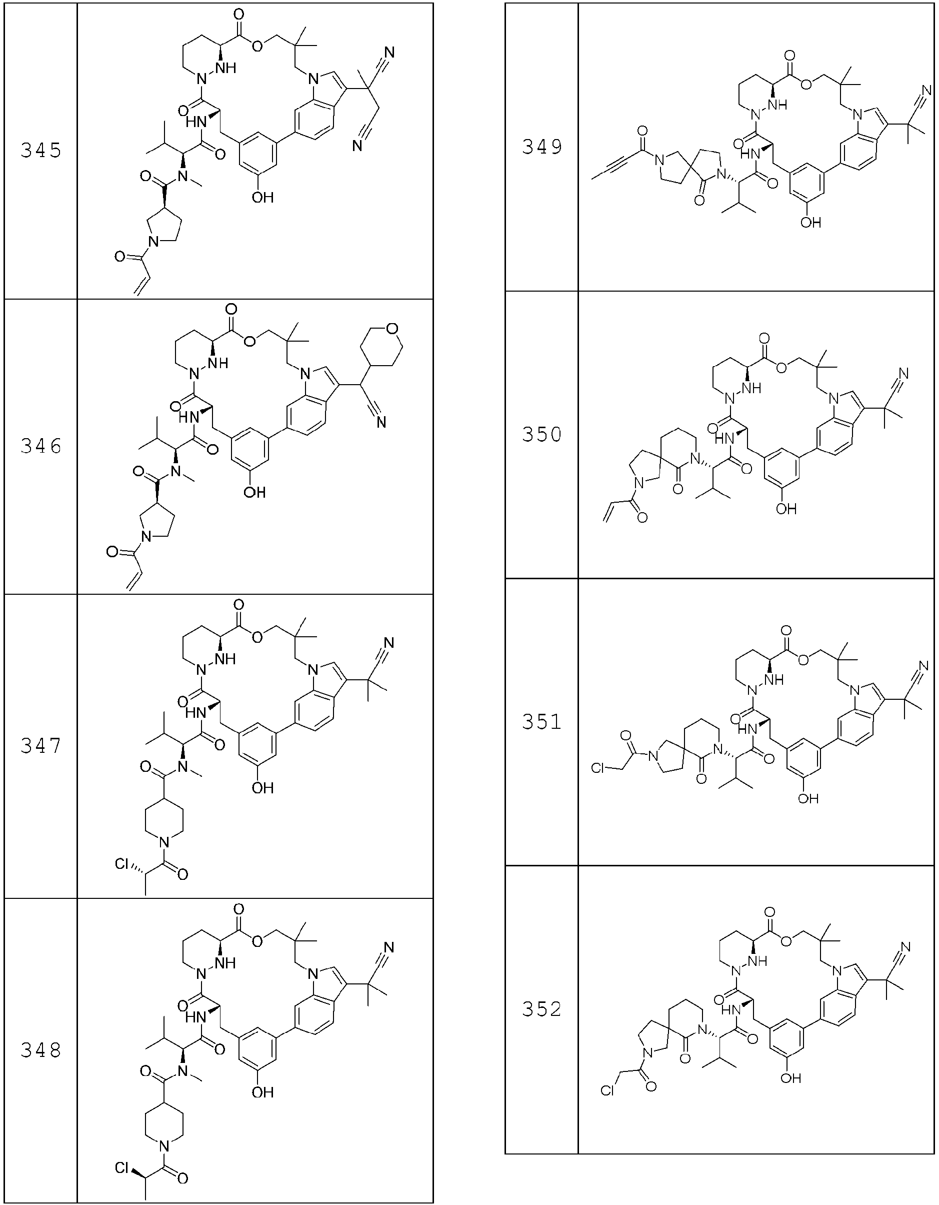

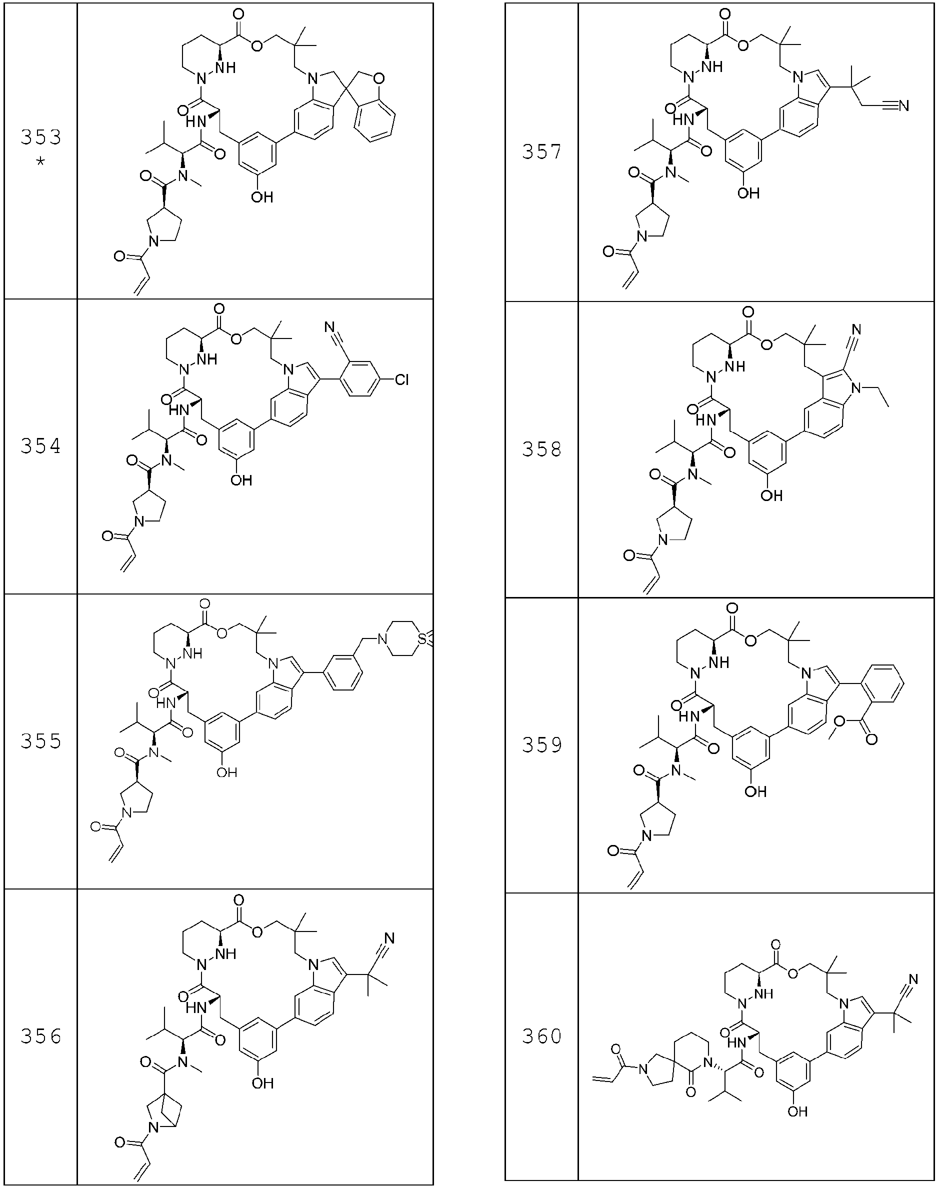

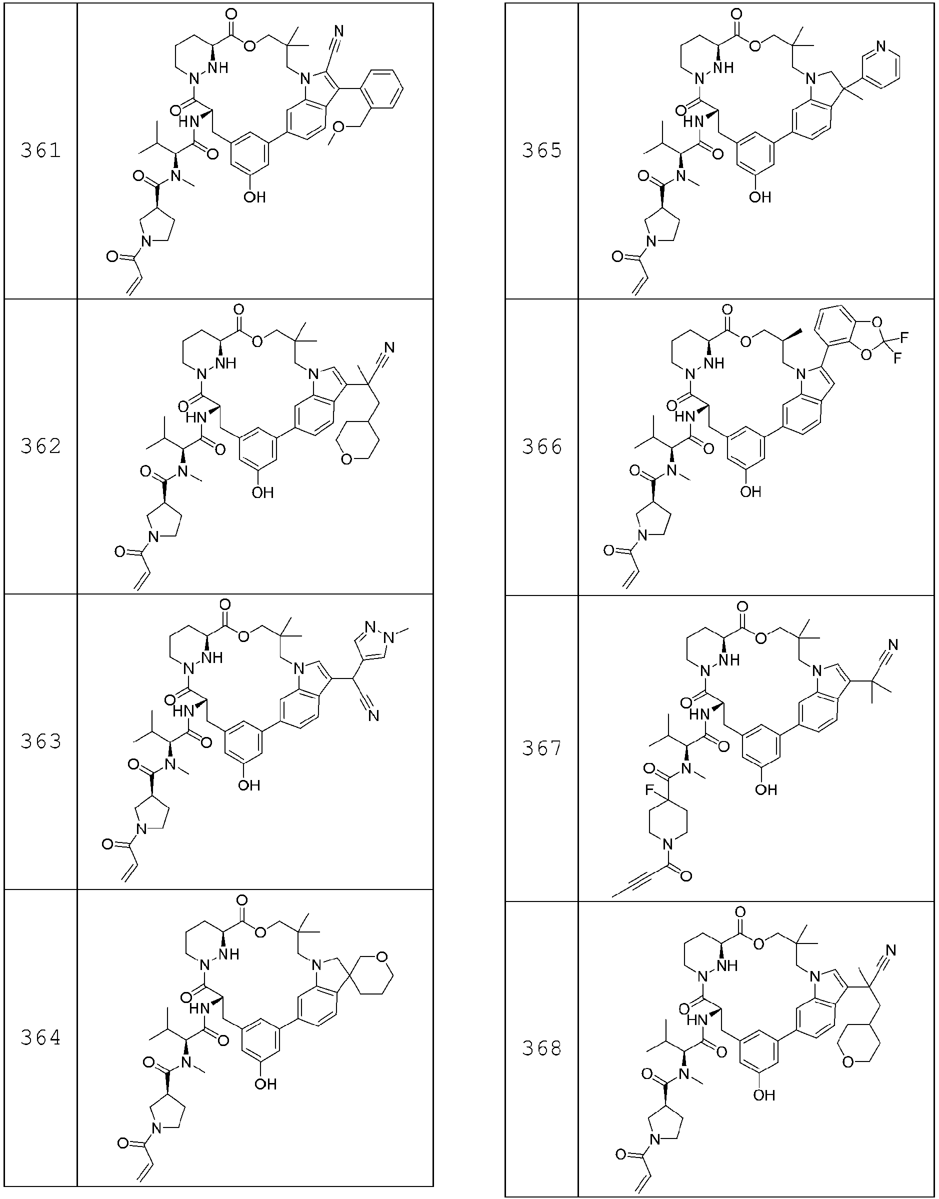

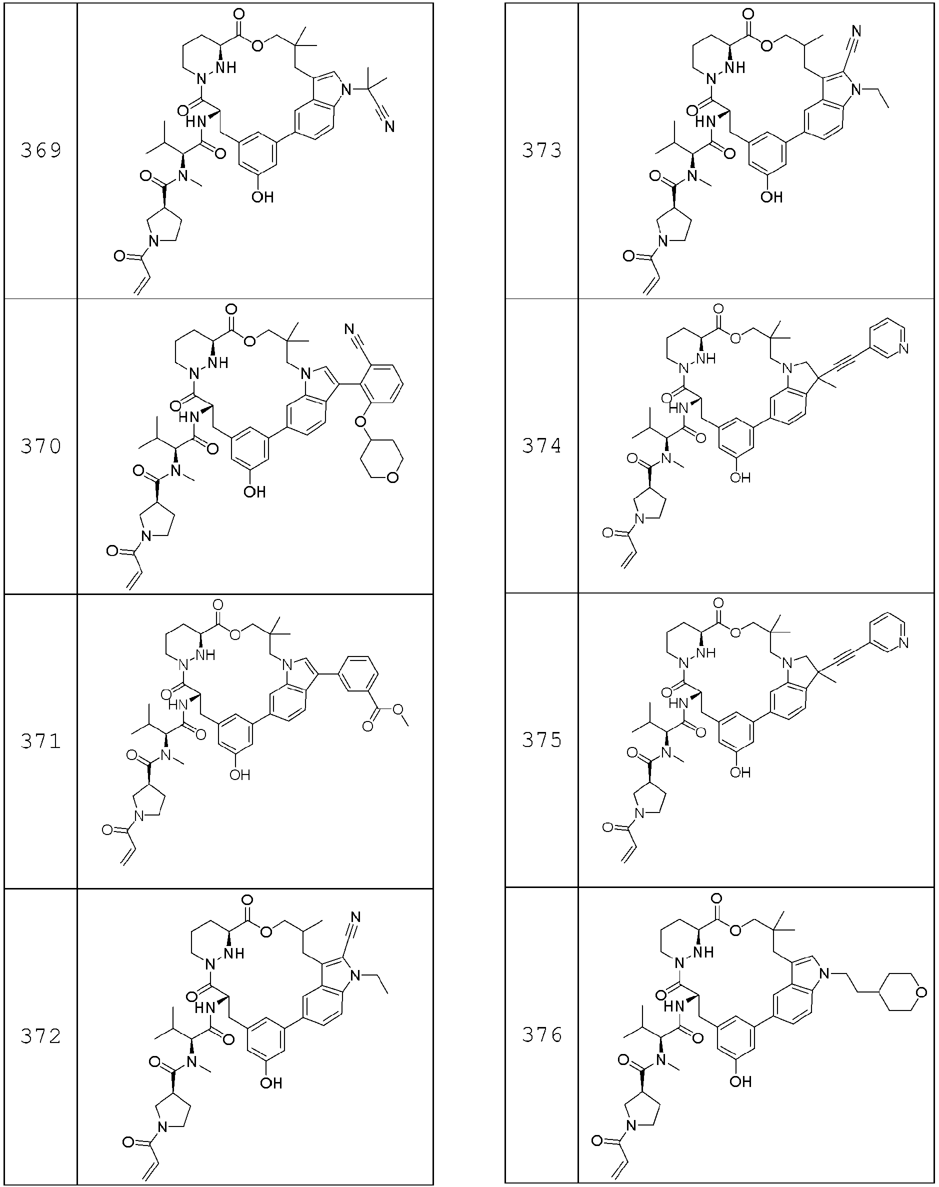

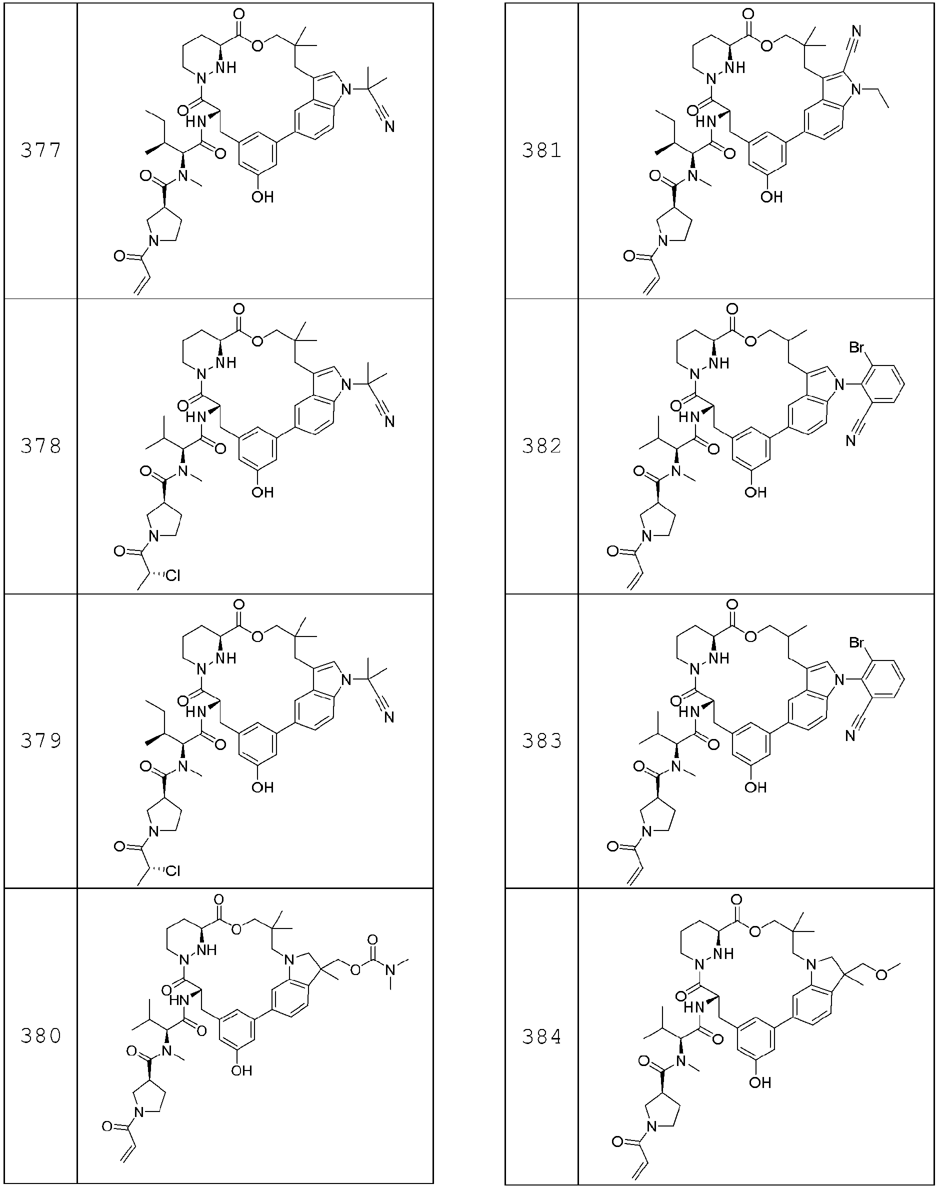

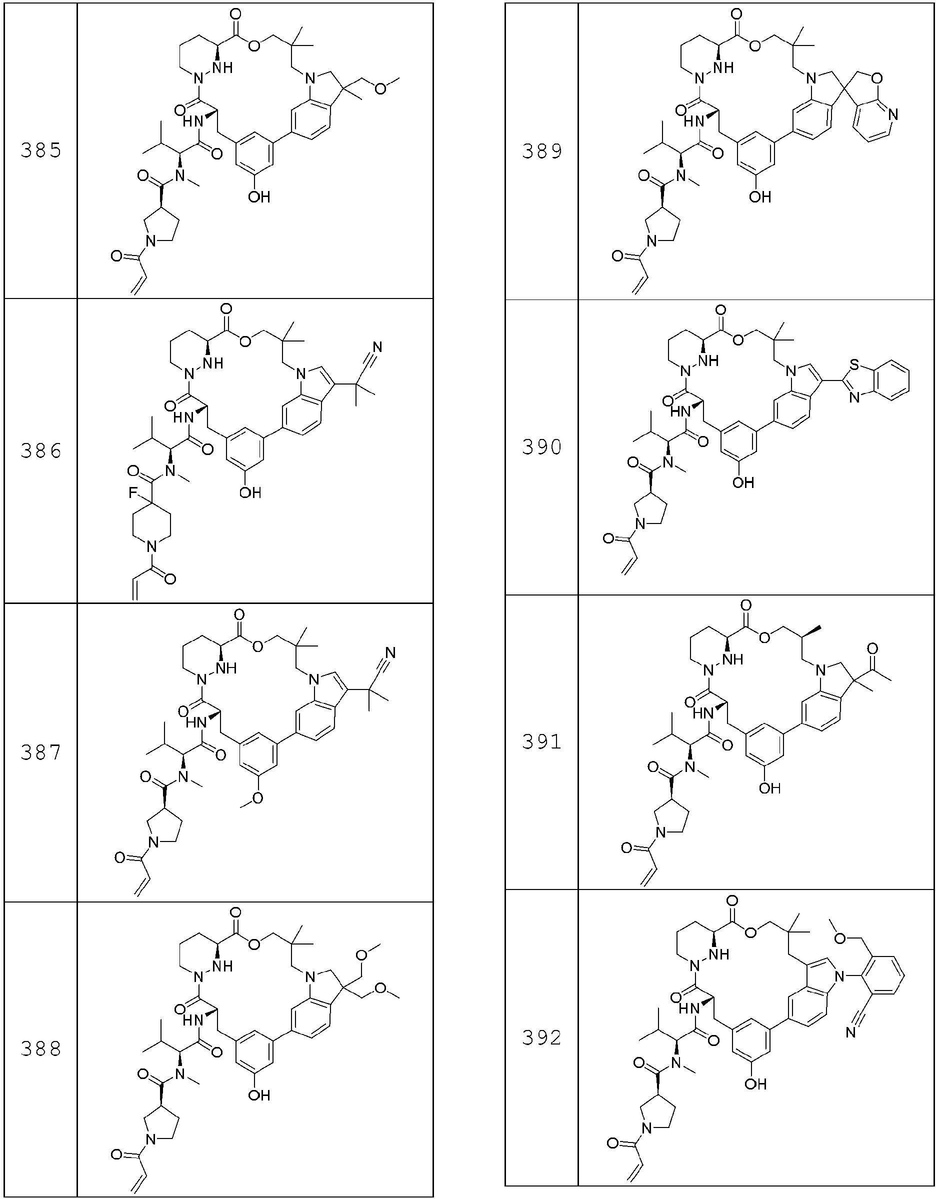

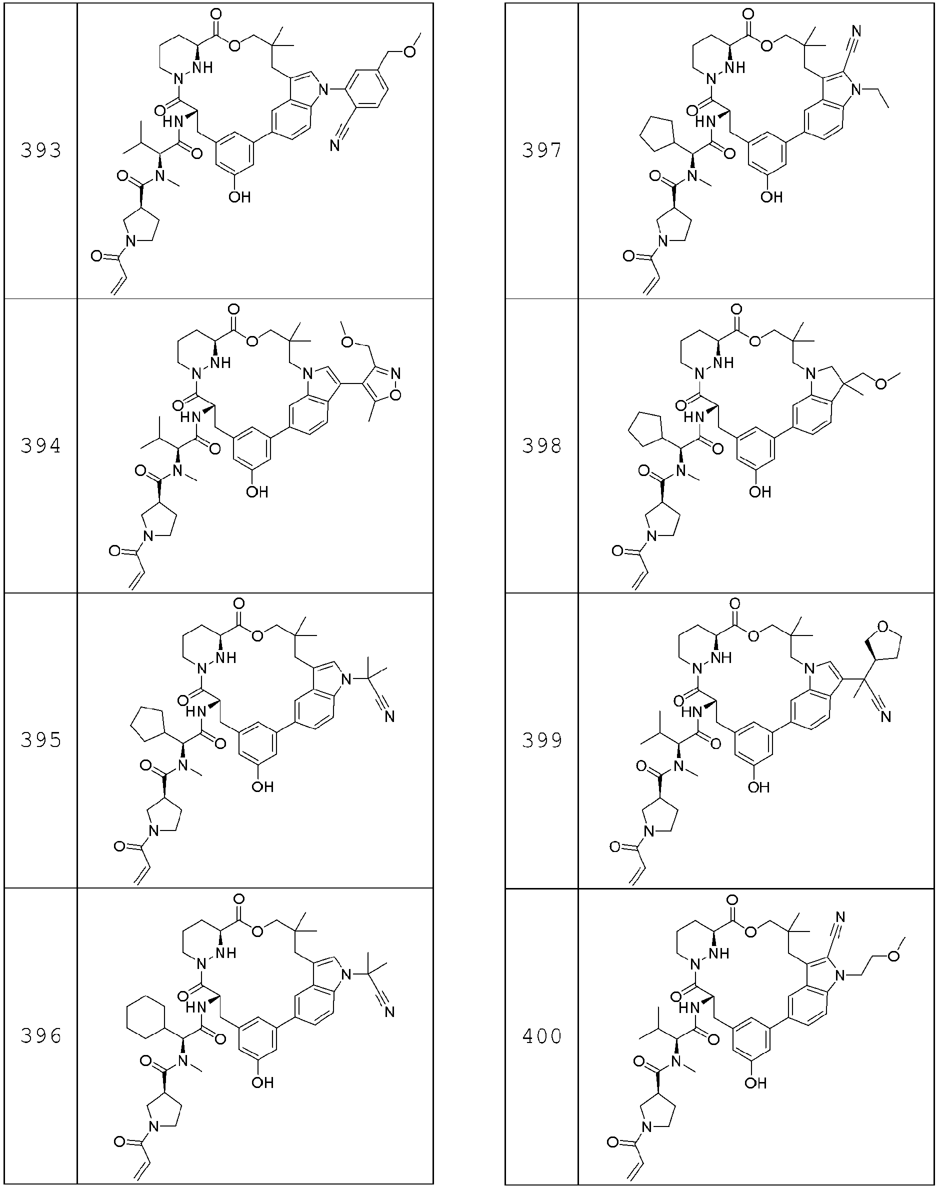

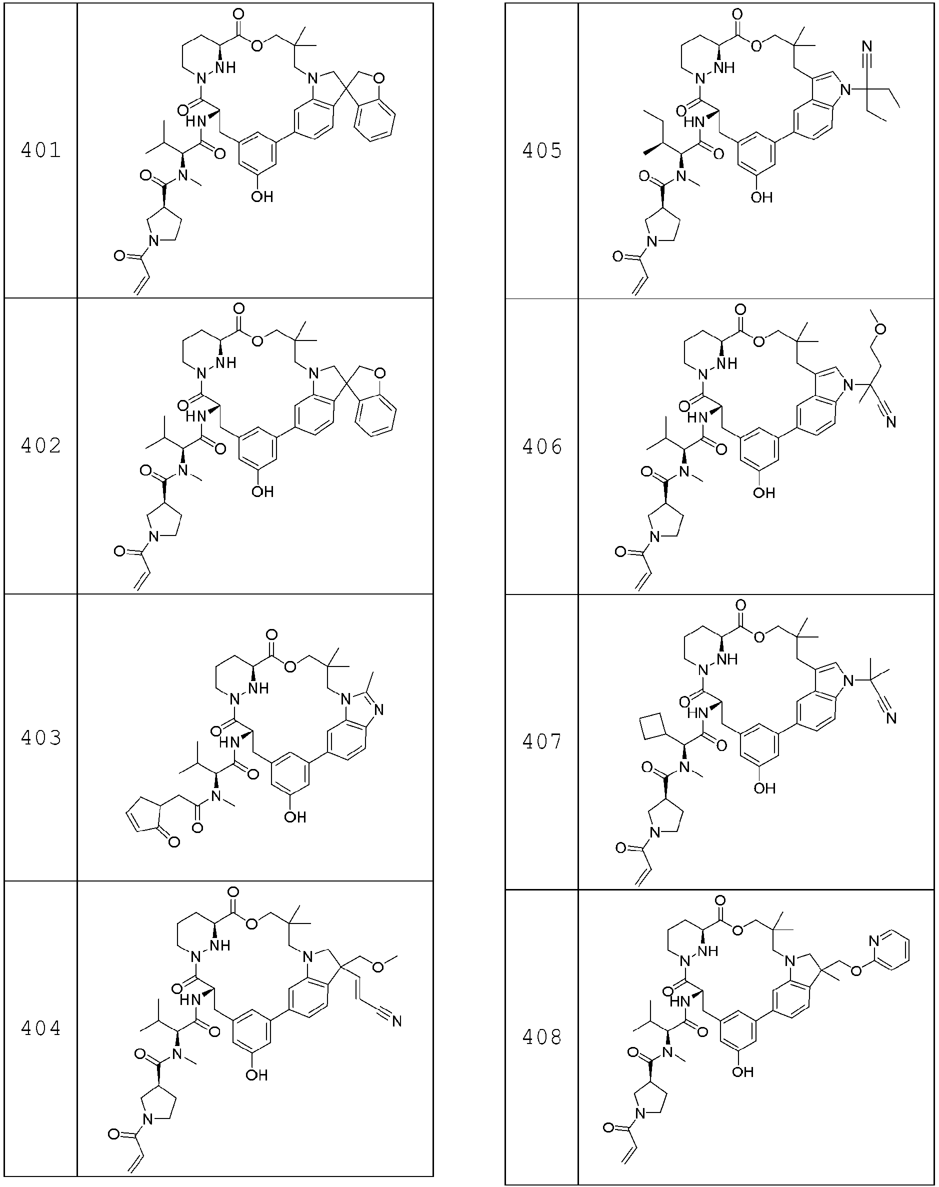

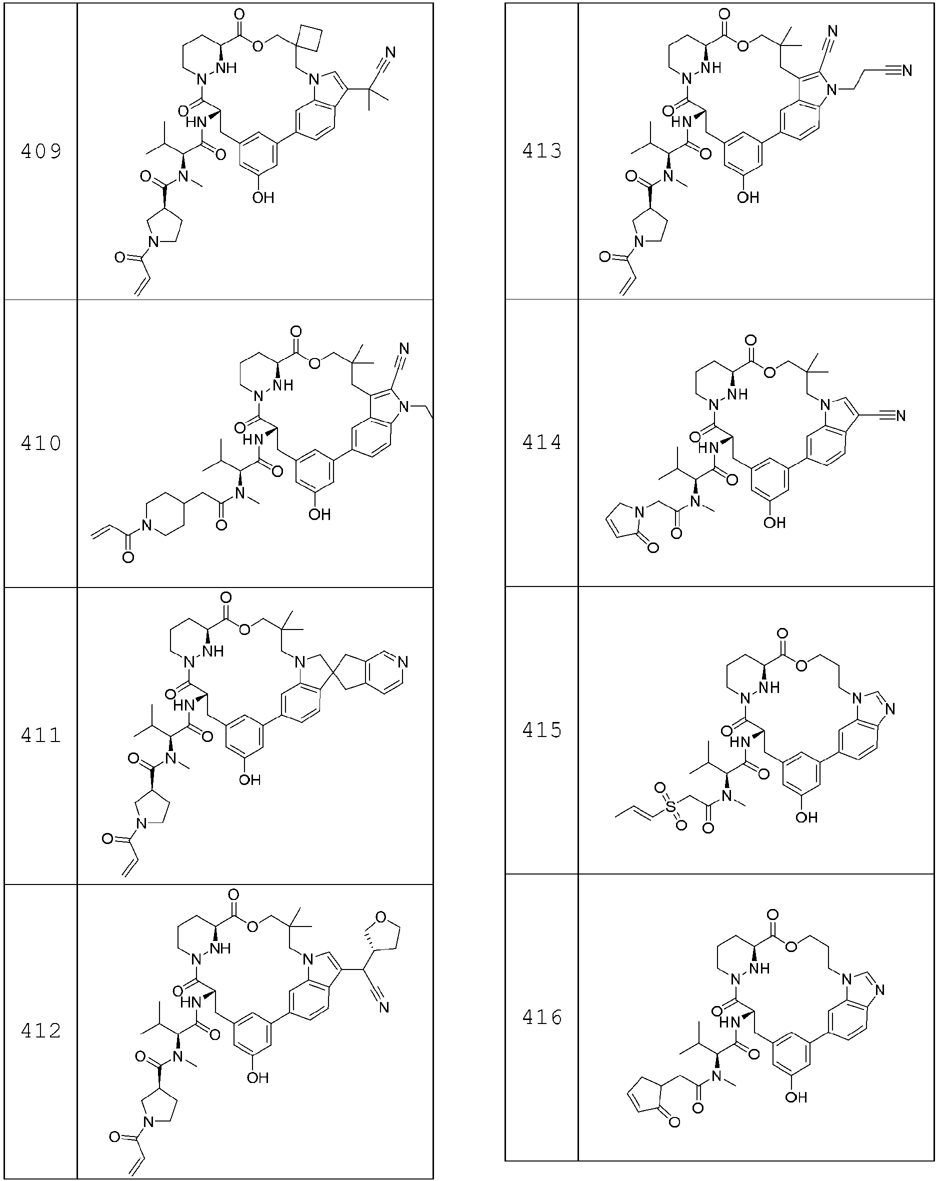

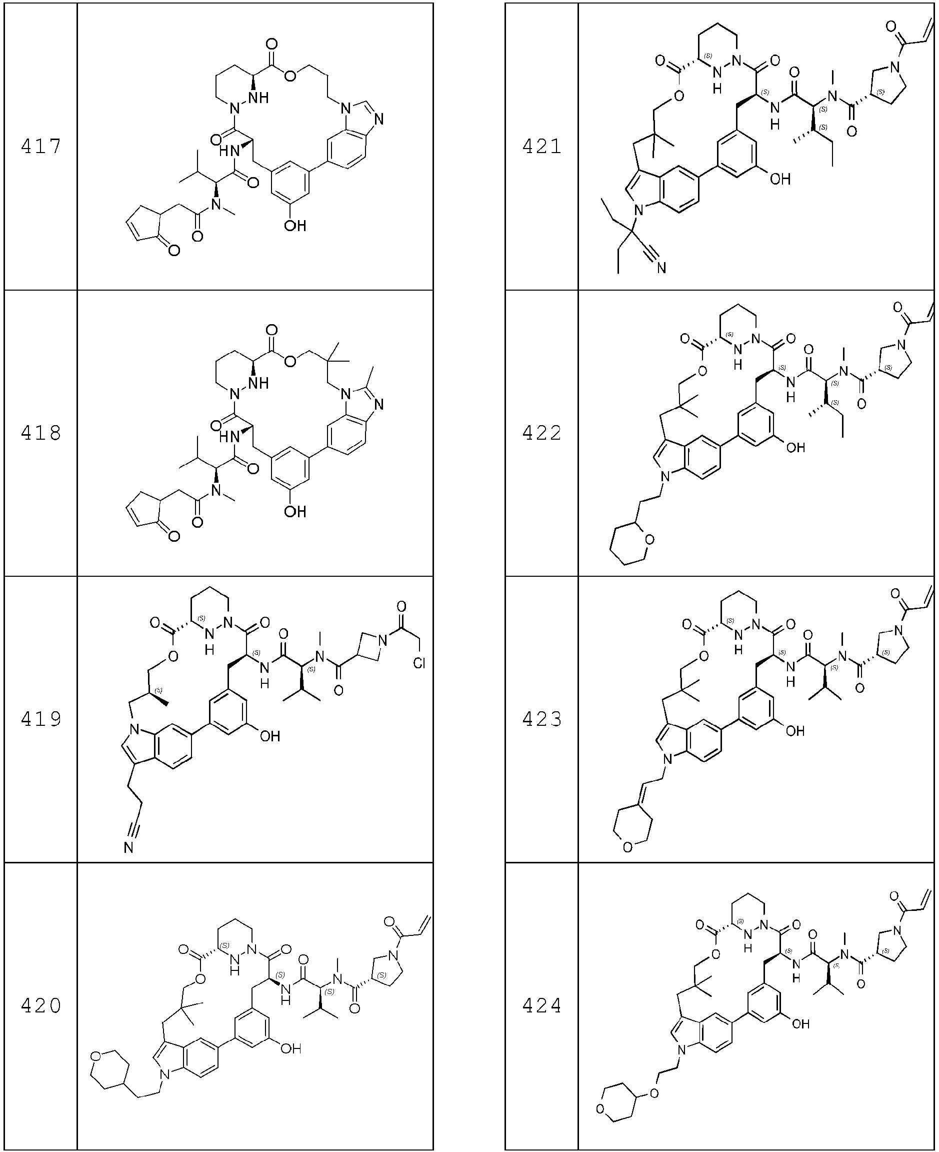

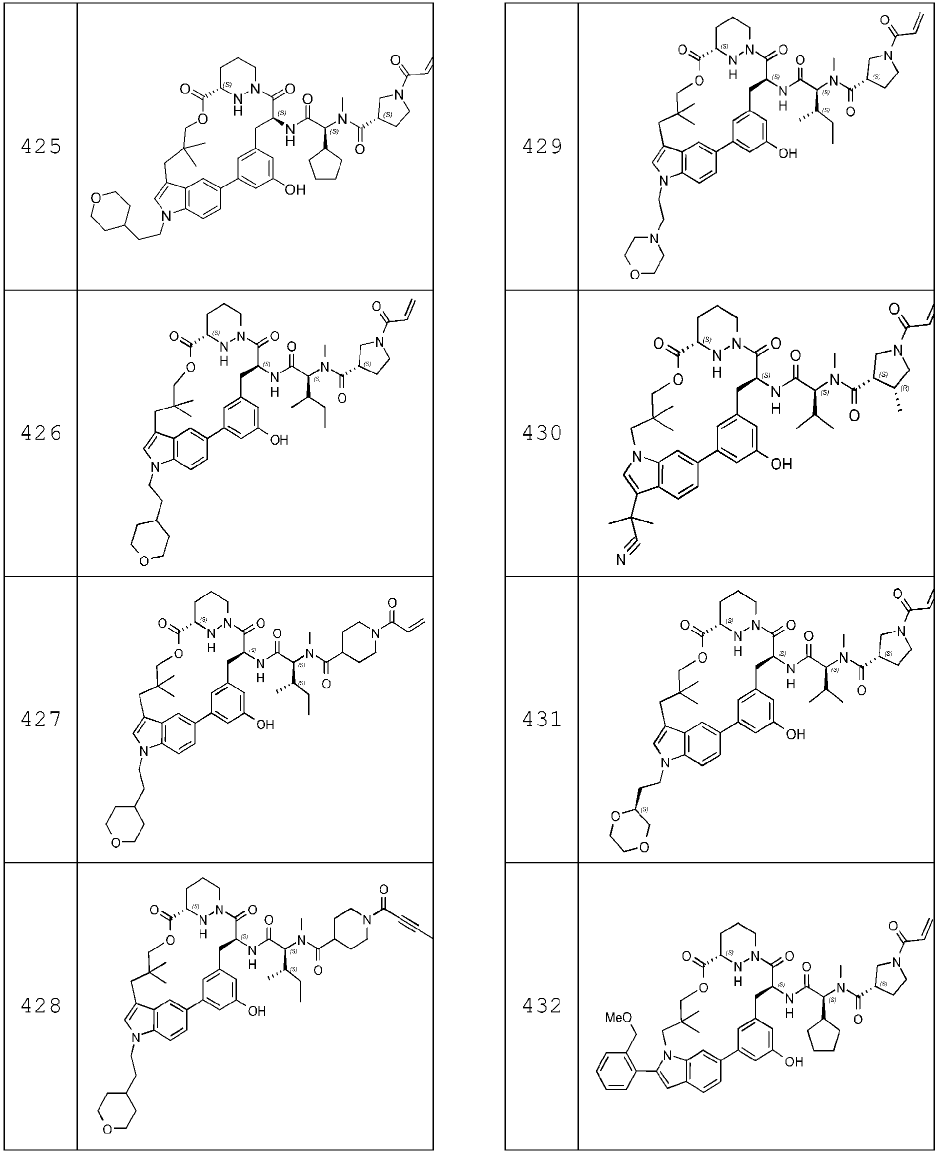

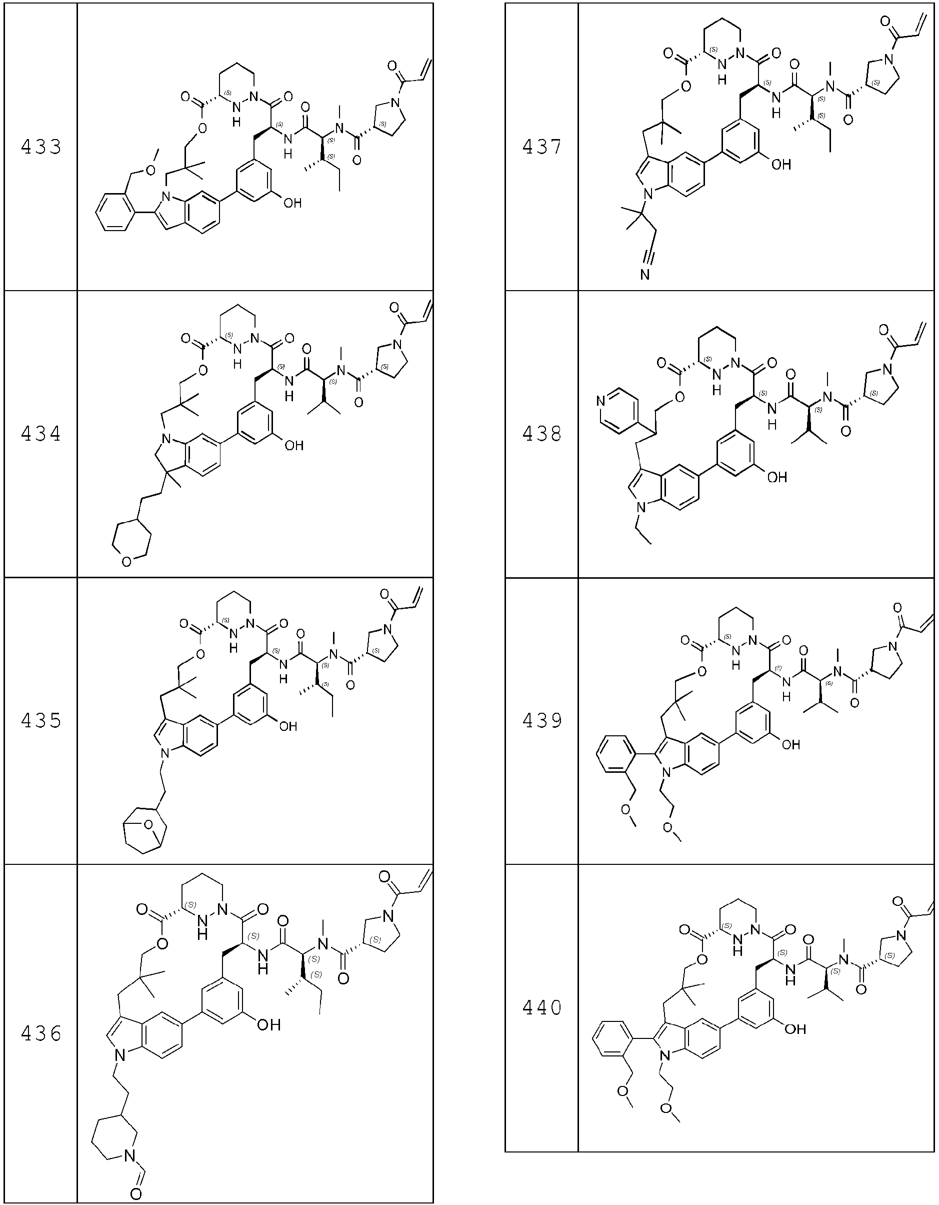

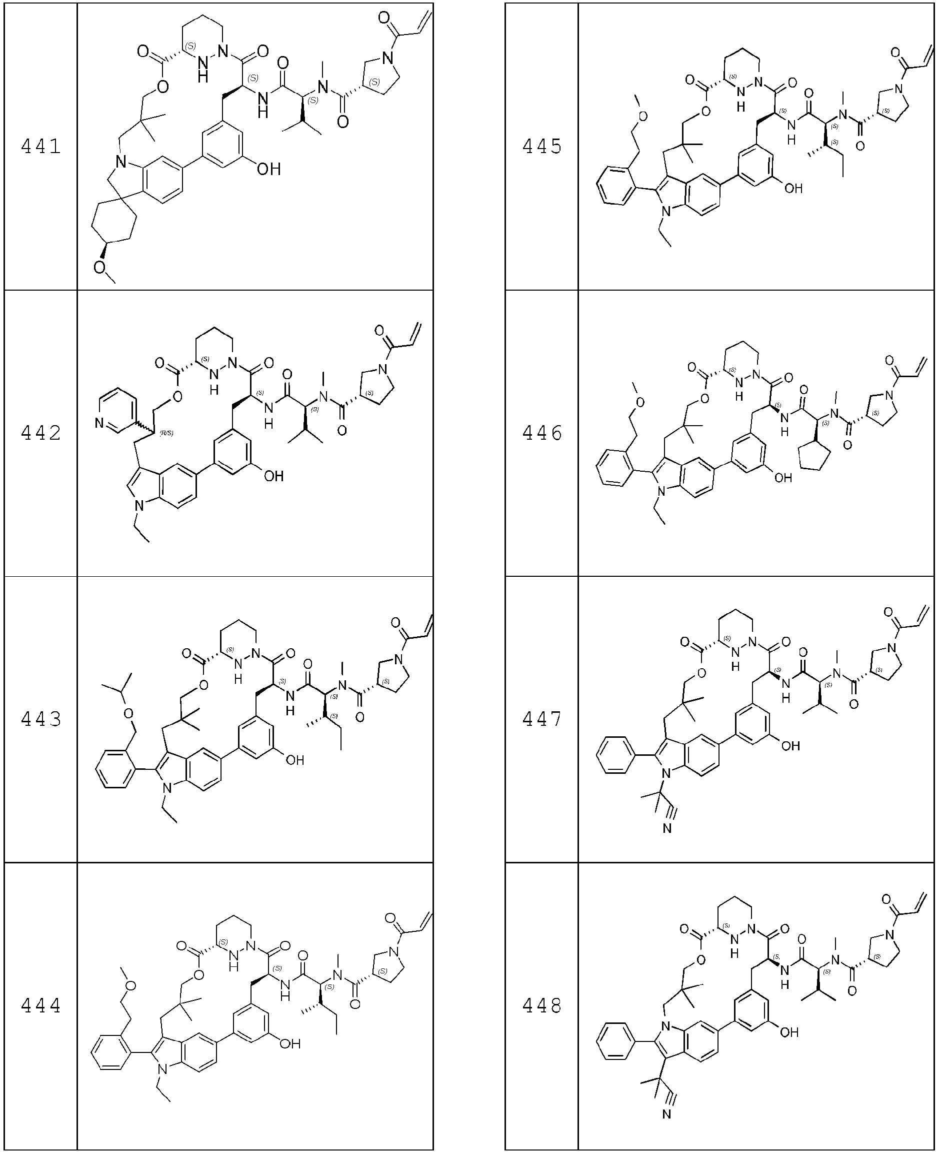

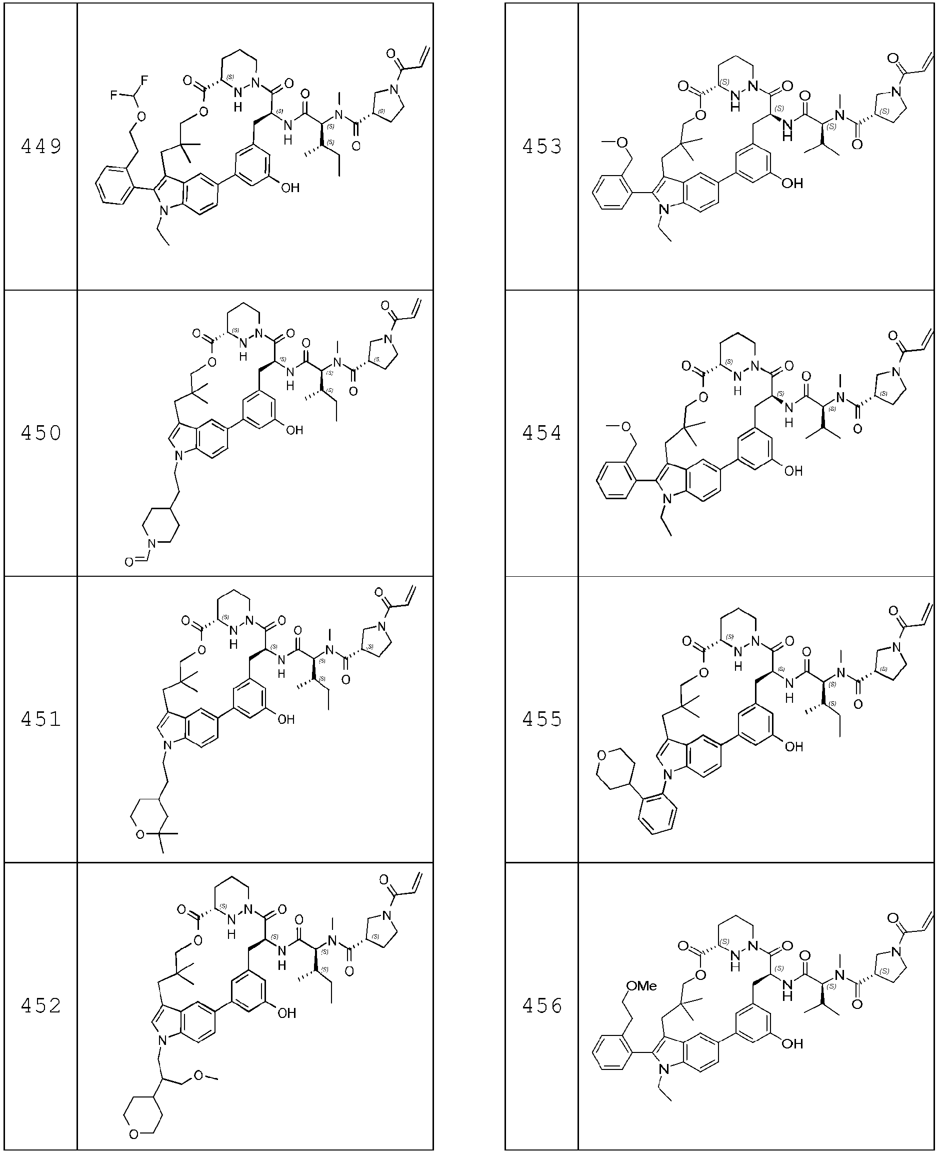

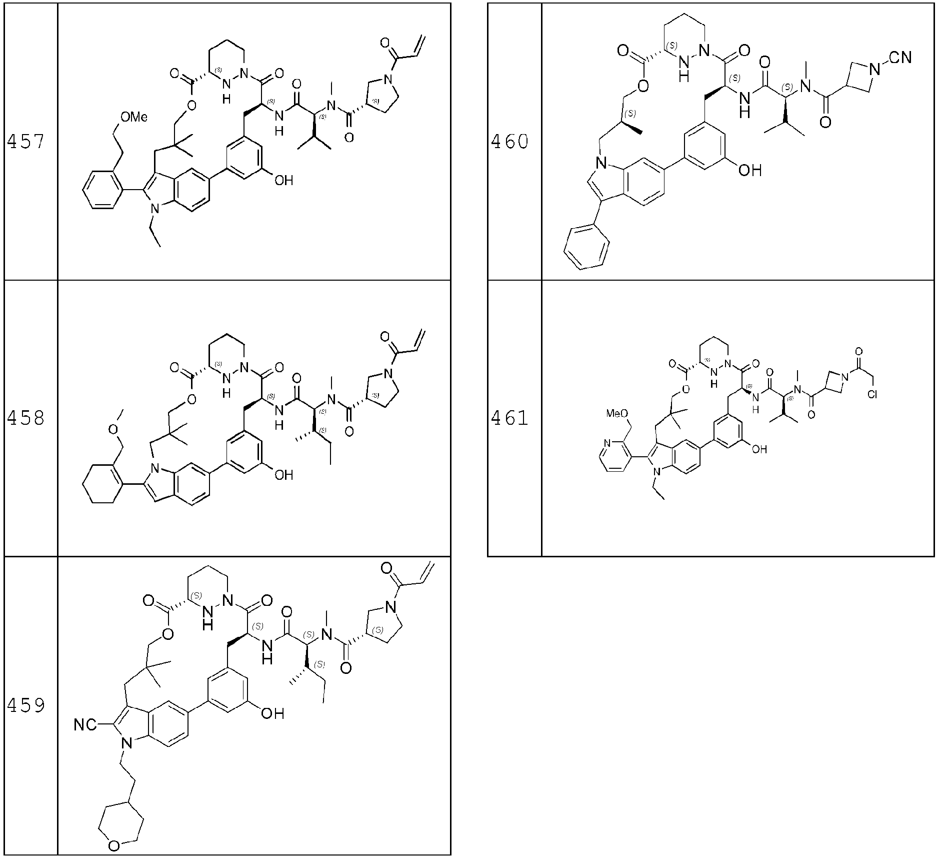

9. Pharmazeutische Zusammensetzung, umfassend eine Verbindung nach einem der Ansprüche 1-8 oder ein pharmazeutisch annehmbares Salz, ein Enantiomer, ein Stereoisomer oder ein Tautomer davon und einen pharmazeutisch annehmbaren Träger.

10. Komplex, umfassend ein Presenter-Protein, ein RAS-Protein und eine Verbindung nach einem der Ansprüche 1-8 oder ein pharmazeutisch annehmbares Salz davon oder eine pharmazeutische Zusammensetzung nach Anspruche 9.

11. Verbindung nach einem der Ansprüche 1-8 oder ein pharmazeutisch annehmbares Salz, ein Enantiomer, ein Stereoisomer oder ein Tautomer davon zur Verwendung in einem Verfahren zur Behandlung von Krebs bei einem Subjekt mit Bedarf daran.

12. Verbindung nach einem der Ansprüche 1-8 oder ein pharmazeutisch annehmbares Salz, ein Enantiomer, ein Stereoisomer oder ein Tautomer davon zur Verwendung in einem Verfahren zur Behandlung einer KRAS G12C-Protein-bedingten Störung bei einem Subjekt mit Bedarf daran.

13. Verbindung zur Verwendung nach Anspruch 11 oder 12, wobei das Verfahren ferner das Verabreichen eines zusätzlichen therapeutischen Mittels umfasst.

14. Verbindung zur Verwendung nach Anspruch 13, wobei das zusätzliche therapeutische Mittel ein HER2-Inhibitor, an EGFR-Inhibitor, ein zweiter Ras-Inhibitor, ein SHP2-Inhibitor, ein SOS1-Inhibitor, ein Raf-Inhibitor, ein MEK-Inhibitor, ein ERK-Inhibitor, ein PI3K-Inhibitor, ein PTEN-Inhibitor, ein AKT-Inhibitor, ein mTORC1-Inhibitor, ein BRAF-Inhibitor, ein PD-L1-Inhibitor, ein PD-1-Inhibitor, ein CDK 4/6-Inhibitor oder eine Kombination davon ist.

## Revendications

1. Composé de formule I : 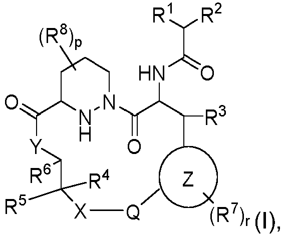 un sel pharmaceutiquement acceptable, un énantiomère, un stéréoisomère, ou un tautomère de celui-ci, dans lequel :
Q est un arylène bicyclique, un hétéroarylène bicyclique, ou un hétérocyclène bicyclique, dans lequel un premier cycle dans Q est en liaison avec X, et un deuxième cycle dans Q est en liaison avec Z, et dans lequel Q est facultativement substitué ;
X est une liaison ; un alkylène à chaîne linéaire en C₁-C₃ facultativement substitué par 1 à 3 substituants sélectionnés indépendamment parmi fluoro, -CN, -C₁-C₃ alkyle, et - O-C₁-C₃ alkyle ; -O- ; -S(O)₀₋₂₋ ; *-CH₂-O- ; *-CH₂-S(O)₀₋₂₋ ; *-O-CH₂- ; ou *-CH₂-S(O)₀-₂-, dans lequel « * » représente une portion de X liée à -C(R⁴)(R⁵)- ;
Y est -O-, -NH- ou -N(C₁-C₃ alkyle)- ;
le cycle Z est un phényle ou un hétéroaryle à 6 chaînons ;
R¹ est un alkyle en C₁-C₆ facultativement substitué, -(CH₂)₀₋₁₋(cycloalkyle en C₃-C₆ facultativement substitué),
-(CH₂)₀₋₁₋(aryle facultativement substitué), ou un groupe hétérocyclyle facultativement substitué ;
R² est : 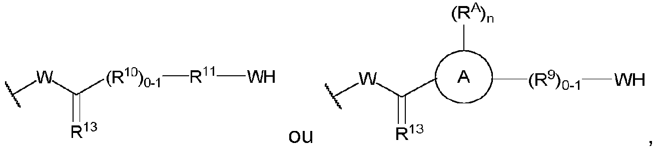 dans lequel :
le cycle A est un cycloalkyle à 4 à 8 chaînons ou un groupe hétérocyclyle à 4 à 8 chaînons ;
W est -N(R¹²)-, -O-, ou -C(R^{12a})(R^{12b})- ;
chaque R^{A} est chacun indépendamment fluoro ; chloro ; -CN ; -OH ; -NH₂ ; - C₁-C₃ alkyle facultativement substitué par CN, OH, NH₂ ou -O-C₁-C₃ alkyle ; -O-C₁-C₃ alkyle ; ou -NH-C₁-C₃ alkyle ;
R⁹, s'il est présent, est -N(C₀-C₅ alkylène-H)-, -N(C(O)-(C₀-C₅ alkylène-H)-, - C(C₀-C₃ alkylène-H)(C₀-C₅ alkylène-H)-, ou -C(C₀-C₃ alkylène-H)(C(O)-C₀-C₅ alkylène-H)-, dans lequel chaque portion alkylène de R⁹ est facultativement substituée par un ou plusieurs substituants, dans lequel chaque substituant est, indépendamment, sélectionné parmi halo, -CN, -OH, -C₁-C₃ alkyle, et -O-C₁-C₃ alkyle ;
R¹⁰, s'il est présent, est un alkylène en C₁-C₄ facultativement substitué par un ou plusieurs substituants, dans lequel chaque substituant est, indépendamment, sélectionné parmi halo, -CN, -OH, -C₁-C₃ alkyle, et -O-C₁-C₃ alkyle ;
R¹¹ est -N(C₀-C₅ alkylène-H)-, -N(C(O)-(C₀-C₅ alkylène-H)-, -C(C₀-C₃ alkylène-H)(C₀-C₅ alkylène-H)-, -C(C₀-C₃ alkylène-H)(C(O)-C₀-C₅ alkylène-H)-, ou un groupe hétérocyclyle saturé contenant de l'azote, dans lequel chaque portion alkylène de R¹¹ est facultativement substituée par un ou plusieurs substituants, dans lequel chaque substituant est, indépendamment, sélectionné parmi halo, -CN, -OH, -C₁-C₃ alkyle, et -O-C₁-C₃ alkyle ;
R¹² est hydrogène, ou -C₁-C₃ alkyle, ou
R¹² est pris conjointement avec un R^{A}, les atomes auxquels ils sont respectivement attachés et tous atomes intermédiaires pour former un groupe hétérocyclyle facultativement substitué à 5 à 8 chaînons qui est fusionné ou spiro-fusionné au cycle A, ou
R¹² est pris conjointement avec toute unité méthylène dans R¹⁰, ou toute unité méthylène dans R¹¹, les atomes auxquels ils sont respectivement attachés et tous atomes intermédiaires pour former un groupe hétérocyclyle facultativement substitué à 5 à 8 chaînons ;
chacun de R^{12a} et R^{12b} est indépendamment hydrogène, ou -C₁-C₃ alkyle, ou R^{12a} et R^{12b} sont pris conjointement avec l'atome de carbone auquel ils sont liés pour former un cycle cycloalkyle à 3 à 6 chaînons ;
R¹³ est O, S, N-CN, ou N-O-C₁-C₃ alkyle ; et
WH est 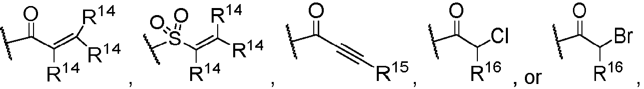 chaque R¹⁴ est indépendamment hydrogène, -CN, ou -C₁-C₃ alkyle facultativement substitué par un ou plusieurs substituants sélectionnés indépendamment parmi -OH, -O-C₁-C₃ alkyle, -NH₂, -NH(C₁-C₃ alkyle), -N(C₁-C₃ alkyle)₂, ou un groupe hétérocyclyle saturé à 4 à 7 chaînons facultativement substitué ;
R¹⁵ est -C₁-C₃ alkyle facultativement substitué par un ou plusieurs substituants sélectionnés indépendamment parmi -OH, -O-C₁-C₃ alkyle, -NH₂, - NH(C₁-C₃ alkyle), -N(C₁-C₃ alkyle)₂, ou un groupe hétérocyclyle saturé à 4 à 7 chaînons facultativement substitué ;
R¹⁶ est hydrogène, -C₁-C₃ alkyle facultativement substitué par un ou plusieurs substituants sélectionnés indépendamment parmi -OH, -O-C₁-C₃ alkyle, - NH₂, -NH(C₁-C₃ alkyle), -N(C₁-C₃ alkyle)₂, ou un groupe hétérocyclyle saturé à 4 à 7 chaînons facultativement substitué ; ou
R¹⁴ est pris conjointement avec soit R⁹ soit R¹¹, les atomes auxquels ils sont attachés et tous atomes intermédiaires pour former un système cyclique facultativement substitué à 5 à 8 chaînons ; ou
R¹⁶ est pris conjointement avec soit R⁹ soit R¹¹, les atomes auxquels ils sont attachés et tous atomes intermédiaires pour former un système cyclique facultativement substitué à 5 à 8 chaînons ;
R³ est hydrogène, halogène, alkyle en C₁-C₃, ou hydroxyalkyle en C₁-C₃ ;
R⁴ est hydrogène, halogène, ou alkyle en C₁-C₃ facultativement substitué ;
R⁵ est hydrogène, halogène, -OH, -CN, -O-(alkyle en C₁-C₃ facultativement substitué), alkyle en C₁-C₃ facultativement substitué, alcényle en C₂-C₆ facultativement substitué, alcynyle en C₂-C₆ facultativement substitué, -(CH₂)₀₋₁₋aryle, -(CH₂)₀₋₁₋hétéroaryle, -(CH₂)₀₋₁₋cycloalkyle, ou -(CH₂)₀₋₁₋groupe hétérocyclyle ; ou
R⁴ et R⁵ sont pris conjointement pour former =CH₂, un cycloalkyle en C₃-C₆ facultativement substitué, ou un groupe hétérocyclyle saturé à 3 à 7 chaînons ; ou
R⁵ est pris conjointement avec un atome du cycle dans Q, l'atome de carbone auquel R⁴ est lié et X pour former un groupe hétérocyclyle saturé ou insaturé à 4 à 9 chaînons qui est fusionné à Q ;
R⁶ est hydrogène ou -CH₃ ;
chaque R⁷ est indépendamment halo, alkyle en C₁-C₃, haloalkyle en C₁-C₃, hydroxyalkyle en C₁-C₃, -OH, -O-C₁-C₃ alkyle, -O-C₁-C₃ haloalkyle, -NRⁿ¹Rⁿ², -NRⁿ¹ORⁿ², - ONRⁿ¹Rⁿ², ou -NRⁿ¹NRⁿ²Rⁿ³ ;
Rⁿ¹ est H, alkyle en C₁-C₃, hétéroalkyle en C₁-C₃, haloalkyle en C₁-C₃, -C₁-C₃ hydroxyalkyle, ou aminoalkyle en C₁-C₃,
dans lequel une unité méthylène de Rⁿ¹ est facultativement substituée par 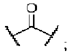
Rⁿ² est H, alkyle en C₁-C₃, hétéroalkyle en C₁-C₃, haloalkyle en C₁-C₃, hydroxyalkyle en C₁-C₃, ou aminoalkyle en C₁-C₃,
dans lequel une unité méthylène de Rⁿ² est facultativement substituée par 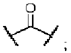
Rⁿ³ est H, alkyle en C₁-C₃, hétéroalkyle en C₁-C₃, haloalkyle en C₁-C₃, hydroxyalkyle en C₁-C₃, ou aminoalkyle en C₁-C₃,
dans lequel une unité méthylène de Rⁿ³ est facultativement substituée par 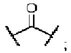
chaque R⁸ est indépendamment halo, alkyle en C₁-C₃, ou haloalkyle en C₁-C₃ ;
n est 0, 1, 2, 3, 4, 5, ou 6 ;
p est 0, 1, 2, ou 3 ; et
r est 0, 1,2, 3, ou 4.

2. Composé selon la revendication 1, dans lequel ledit composé présente la structure de formule (la) : 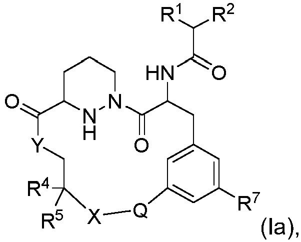 ou un sel pharmaceutiquement acceptable, un énantiomère, un stéréoisomère, ou un tautomère de celui-ci,
dans lequel :
X est une liaison, -O-, -CH₂-, -CH(CH₃)-, *-CH₂-O-, ou -CH₂-CH₂-, dans lequel « * » représente une portion de X liée à C(R⁴)(R⁵) ;
Y est -O- ou -NH- ;
R¹ est -C₁-C₄ alkyle, -(CH₂)₀₋₁₋(cycloalkyle en C₃-C₆), ou -C₄-C₆ cycloalkyle ;
R² est : 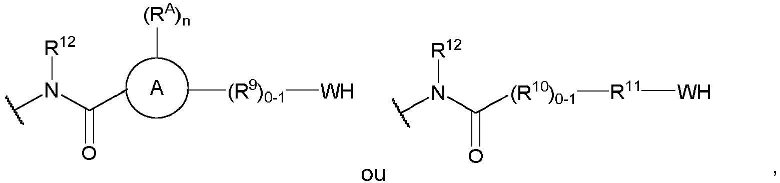 dans lequel :
le cycle A est un cycloalkyle à 4 à 8 chaînons ou un groupe hétérocyclyle saturé à 4 à 8 chaînons ;
chaque R^{A} est chacun indépendamment fluoro ; chloro ; -CN ; -OH ; -NH₂ ; - C₁-C₃ alkyle facultativement substitué par CN, OH, NH₂ ou -O-C₁-C₃ alkyle ; -O-C₁-C₃ alkyle ; ou -NH-C₁-C₃ alkyle ;
n est 0, 1, 2, 3, 4, 5, ou 6 ;
R⁹, s'il est présent, est -N(C₀-C₅ alkylène-H)-, -N(C(O)-(C₀-C₅ alkylène-H)-, - C(C₀-C₃ alkylène-H)(C₀-C₅ alkylène-H)-, ou -C(C₀-C₃ alkylène-H)(C(O)-C₀-C₅ alkylène-H)-, dans lequel chaque portion alkylène de R⁹ est facultativement substituée par un ou plusieurs substituants sélectionnés indépendamment parmi halo, -CN, -OH, -C₁-C₃ alkyle, et -O-C₁-C₃ alkyle ;
R¹⁰, s'il est présent, est un alkylène en C₁-C₄ facultativement substitué par un ou plusieurs substituants sélectionnés indépendamment parmi halo, -CN, -OH, - C₁-C₃ alkyle, et -O-C₁-C₃ alkyle ;
R¹¹ est -N(C₀-C₅ alkylène-H)-, -N(C(O)-(C₀-C₅ alkylène-H)-, -C(C₀-C₃ alkylène-H)(C₀-C₅ alkylène-H)-, ou -C(C₀-C₃ alkylène-H)(C(O)-C₀-C₅ alkylène-H)-, dans lequel chaque portion alkylène de R¹¹ est facultativement substituée par un ou plusieurs substituants sélectionnés indépendamment parmi halo, -CN, -OH, -C₁-C₃ alkyle, et -O-C₁-C₃ alkyle ;
R¹² est hydrogène, ou -C₁-C₃ alkyle, ou
R¹² est pris conjointement avec un R^{A}, les atomes auxquels ils sont respectivement attachés et tous atomes intermédiaires pour former un groupe hétérocyclyle facultativement substitué à 5 à 8 chaînons qui est fusionné au cycle A, ou
R¹² est pris conjointement avec toute unité méthylène dans R¹⁰, ou toute unité méthylène dans R¹¹, les atomes auxquels ils sont respectivement attachés et tous atomes intermédiaires pour former un groupe hétérocyclyle facultativement substitué à 5 à 8 chaînons ;
WH est 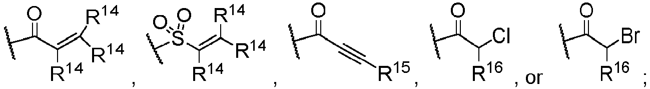
chaque R¹⁴ est indépendamment hydrogène, -CN, -C₁-C₃ alkyle, -C₁-C₃ hydroxyalkyle, -O-C₁-C₃ alkyle ;
R¹⁵ est -C₁-C₃ alkyle, -C₁-C₃ hydroxyalkyle, ou -C₁-C₃ alkylène-O-C₁-C₃ alkyle ;
R¹⁶ est hydrogène, -C₁-C₃ alkyle, -C₁-C₃ hydroxyalkyle, ou -C₁-C₃ alkylène-O-C₁-C₃ alkyle ; ou
R¹⁴ est pris conjointement avec soit R⁹ soit R¹¹, les atomes auxquels ils sont attachés et tous atomes intermédiaires pour former un système cyclique facultativement substitué à 5 à 8 chaînons, ou
R¹⁶ est pris conjointement avec soit R⁹ soit R¹¹, les atomes auxquels ils sont attachés et tous atomes intermédiaires pour former un système cyclique facultativement substitué à 5 à 8 chaînons ;
R⁴ est hydrogène, halo, ou alkyle en C₁-C₃ ;
R⁵ est hydrogène, halo, -OH, alkyle en C₁-C₃, hydroxyalkyle en C₁-C₃, alkylène en C₁-C₃-O-C₁-C₃ alkyle, haloalkyle en C₁-C₃, cycloalkyle en -(CH₂)₀₋₁₋C₃-C₆, cyanoalkyle en C₁-C₃, ou -(CH₂)₀₋₁₋aryle (benzyle), ou
R⁴ et R⁵ sont pris conjointement pour former =CH₂, ou un cycloalkyle en C₃-C₆, ou
R⁵ est pris conjointement avec un atome du cycle de Q, l'atome de carbone auquel il est lié et X pour former un groupe hétérocyclyle saturé à 5 à 7 chaînons ;
R⁷ est -OH, -NH₂, ou haloalkyle en C₁-C₃ ;
Q est un arylène bicyclique, un hétéroarylène bicyclique, ou un hétérocyclène bicyclique, dans lequel :
un premier cycle dans Q est lié à X, et un deuxième cycle dans Q est lié à Z ; et
Q est facultativement substitué par un ou plusieurs substituants sélectionnés indépendamment parmi =O ; -ON ; -C₁-C₅ alkyle facultativement substitué par un ou plusieurs halo, CN, OH, -O-(C₁-C₃ alkyle), -C(O)-(C₁-C₄ alkyle), -O-(C₂-C₃ alcynyle), -(C₃-C₆ cycloalkyle), ou un groupe hétérocyclyle saturé à 4 à 7 chaînons sélectionnés indépendamment ; -O-(C₁-C₃ alkyle) facultativement substitué par un ou plusieurs halo sélectionnés indépendamment ; alcényle en C₂-C₅ facultativement substitué par un ou plusieurs -CN, ou -OH sélectionnés indépendamment ; alcynyle en C₂-C₃ ; -S(O)₂-C₁-C₃ alkyle ; cycloalkyle en -(CH₂)₀₋₁₋ C₃-C₆ facultativement substitué par un ou plusieurs halo sélectionnés indépendamment, =O, -CN, C₁-C₃ alkyle facultativement substitué par -CN ou -O-C₁-C₃ alkyle, -C(O)-groupe hétérocyclyle saturé, -O-groupe hétérocyclyle saturé, O-cycloalkyle, ou -O-aryle sélectionnés indépendamment ; -(CH₂)₀₋₁₋hétéroaryle facultativement substitué par un ou plusieurs halo, -CN, C₁-C₃ alkyle facultativement substitué par -CN ou -O-C₁-C₃ alkyle, -C(O)-groupe hétérocyclyle saturé, -O-groupe hétérocyclyle saturé, O-cycloalkyle, ou -O-aryle sélectionnés indépendamment ; -(CH₂)₀₋₁₋groupe hétérocyclyle facultativement substitué par un ou plusieurs halo, =O, -CN, C₁-C₃ alkyle facultativement substitué par -CN ou -O-C₁-C₃ alkyle, -C(O)-groupe hétérocyclyle saturé, -O-groupe hétérocyclyle saturé, O-cycloalkyle, ou -O-aryle sélectionnés indépendamment ; -(CH₂)₀₋₁₋aryle facultativement substitué par un ou plusieurs halo, -CN, -C₁-C₃ alkyle facultativement substitué par -CN ou -O-C₁-C₃ alkyle, -C(O)-groupe hétérocyclyle saturé, -O-groupe hétérocyclyle saturé, O-cycloalkyle, ou -O-aryle sélectionnés indépendamment ; -C(O)-NH-(C₁-C₃ alkyle) ; - C(O)-N(C₁-C₃ alkyle)₂ ; C₂-C₃ alcénylène=N-O-(C₁-C₃ alkyle) facultativement substitué par un cycloalkyle en C₃-C₆ ; ou
deux substituants sur les mêmes atomes du cycle ou sur des atomes adjacents du cycle de Q sont pris conjointement pour former un cycle monocyclique à 5 à 7 chaînons ou un cycle bicyclique à 6 à 12 chaînons facultativement substitué par un ou plusieurs halo, =O, -ON, C₁-C₃ alkyle, ou -O-C₁-C₃ alkyle sélectionnés indépendamment ; et fusionné à Q.

3. Composé selon la revendication 2, dans lequel ledit composé présente la structure de formule (Ib) : 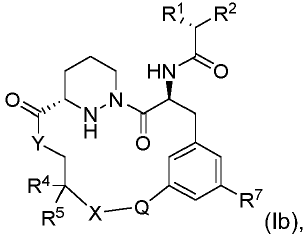 ou un sel pharmaceutiquement acceptable, un énantiomère, un stéréoisomère, ou un tautomère de celui-ci ou
ledit composé présente la structure de formule (Ic) : 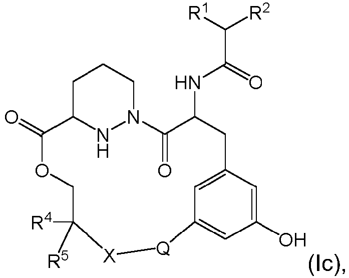 ou un sel pharmaceutiquement acceptable, un énantiomère, un stéréoisomère, ou un tautomère de celui-ci.

4. Composé selon l'une quelconque des revendications 1 à 3, ou un sel pharmaceutiquement acceptable, un énantiomère, un stéréoisomère, ou un tautomère de celui-ci, dans lequel Q est un hétéroarylène bicyclique 5,6, un hétérocyclène bicyclique 5,6, un hétéroarylène bicyclique 6,6, ou un hétérocyclène bicyclique 6,6 ; et dans lequel Q est facultativement substitué ;
facultativement dans lequel Q est sélectionné parmi le groupe constitué de : 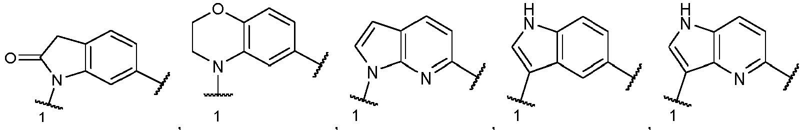 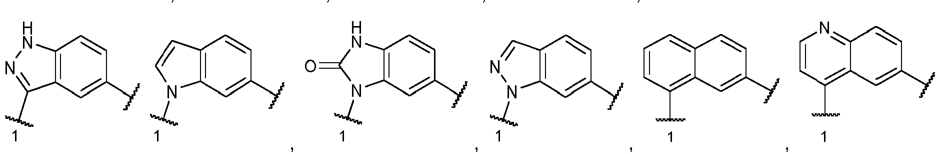 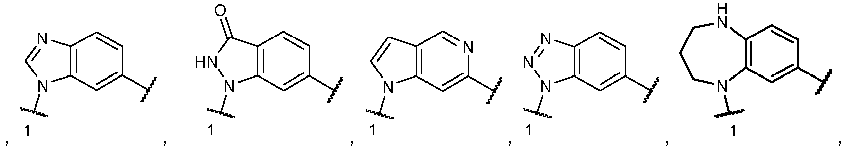 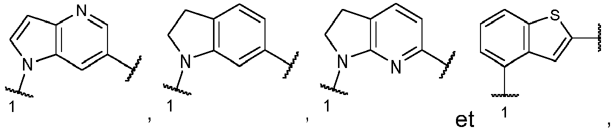 dans lequel :
« 1 » indique une portion de Q liée à X ; et
Q est en outre facultativement substitué.

5. Composé selon l'une quelconque des revendications 1 à 4, ou un sel pharmaceutiquement acceptable, un énantiomère, un stéréoisomère, ou un tautomère de celui-ci, dans lequel :
(i) R¹ est -CH₃, -CH₂CH₃, -(CH₂)₂CH₃, -CH(CH₃)₂, -CH(CH₃)CH₂CH₃, cyclopropylméthyle, cyclobutylméthyle, cyclopentylméthyle, cyclohexylméthyle, cyclopropyle, cyclobutyle, cyclopentyle, cyclohexyle, phényle, 4-méthoxybenzyle, ou tétrahydropyran-4-yle ; et/ou
(ii) R⁹ est absent et le cycle A est un groupe hétérocyclyle saturé contenant de l'azote ; et/ou
(iii) la portion de R² représentée par : 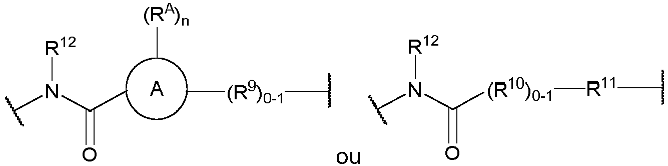 est sélectionné parmi le groupe consistant en : 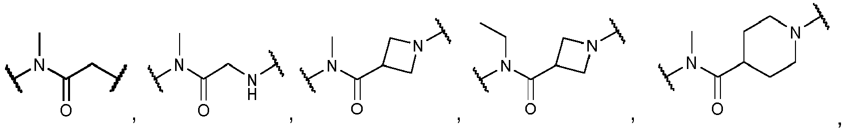 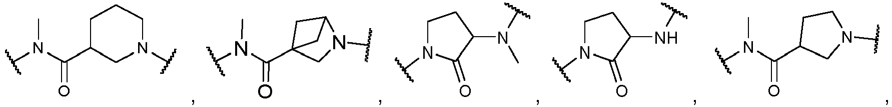 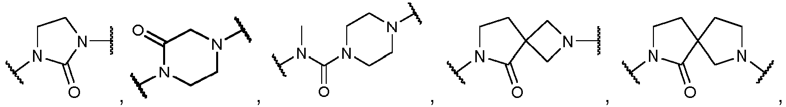 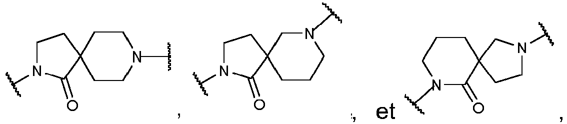 dans lequel chaque système cyclique dans R² est facultativement substitué par jusqu'à 4 substituants sélectionnés indépendamment parmi fluoro ;
chloro ; -CN ; -OH ; -NH₂ ; -C₁-C₃ alkyle facultativement substitué par CN, OH, NH₂ ou -OC₁-C₃ alkyle ; -O-C₁-C₃ alkyle ; et -NH-C₁-C₃ alkyle ; et/ou
(iv) la portion de R² représentée par WH est -C(O)-C=C-CH₃, -C(O)-CH=CH₂, - S(O)₂-CH=CH₂, -C(O)-CH₂Cl, -C(O)-CH(CH₃)Cl, ou -C(O)-CH(Cl)-CH₂-O-CH₃, ou
la portion de R² représentée par -R¹¹-WH, lorsque R¹¹ est pris conjointement avec un R¹⁴ est

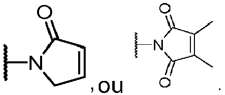

6. Composé selon l'une quelconque des revendications 1 à 5, ou un sel pharmaceutiquement acceptable, un énantiomère, un stéréoisomère, ou un tautomère de celui-ci, dans lequel :
R⁴ est hydrogène, fluoro, ou -CH₃ ; et
R⁵ est hydrogène, fluoro,
chloro, -OH, -CH₃, -CH₂CH₃, -CH(CH₃)₂, -CH₂OH, -CH₂OCH₃, -CH₂F, -CHF₂, CH₂CN, -CH₂-cyclopropyle, cyclopropyle, pyridyle, phényle, ou -CH₂-phényle, dans lequel toute portion phényle de R⁵ est facultativement substituée par jusqu'à 4 substituants sélectionnés indépendamment parmi halo, -CN, et -O-C₁-C₃ alkyle ; ou
R⁴ et R⁵ sont pris conjointement pour former =CH₂ ou cyclopropyle, ou cyclobutyle, ou cyclopentyle, ou cyclohexyle ; ou
R⁵ est pris conjointement avec l'atome de carbone auquel il est lié, un atome du cycle de Q, et X pour former l'oxazépane.

7. Composé selon l'une quelconque des revendications 1 à 6, ou un sel pharmaceutiquement acceptable, un énantiomère, un stéréoisomère, ou un tautomère de celui-ci, dans lequel R⁷ est -OH, -NH₂, ou -CHF₂.

8. Composé selon la revendication 1, ou un sel pharmaceutiquement acceptable, un énantiomère, un stéréoisomère, ou un tautomère de celui-ci, sélectionné parmi :

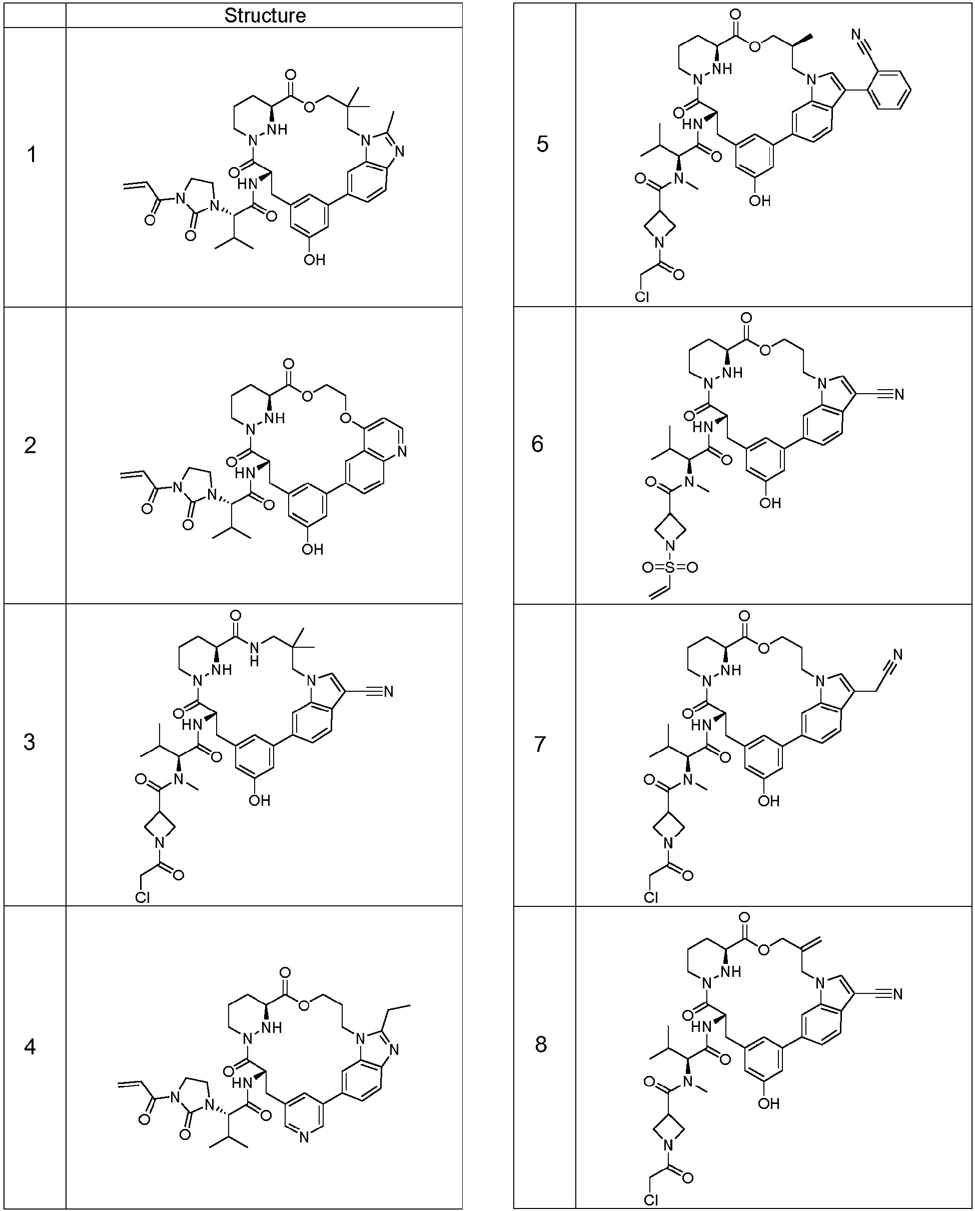

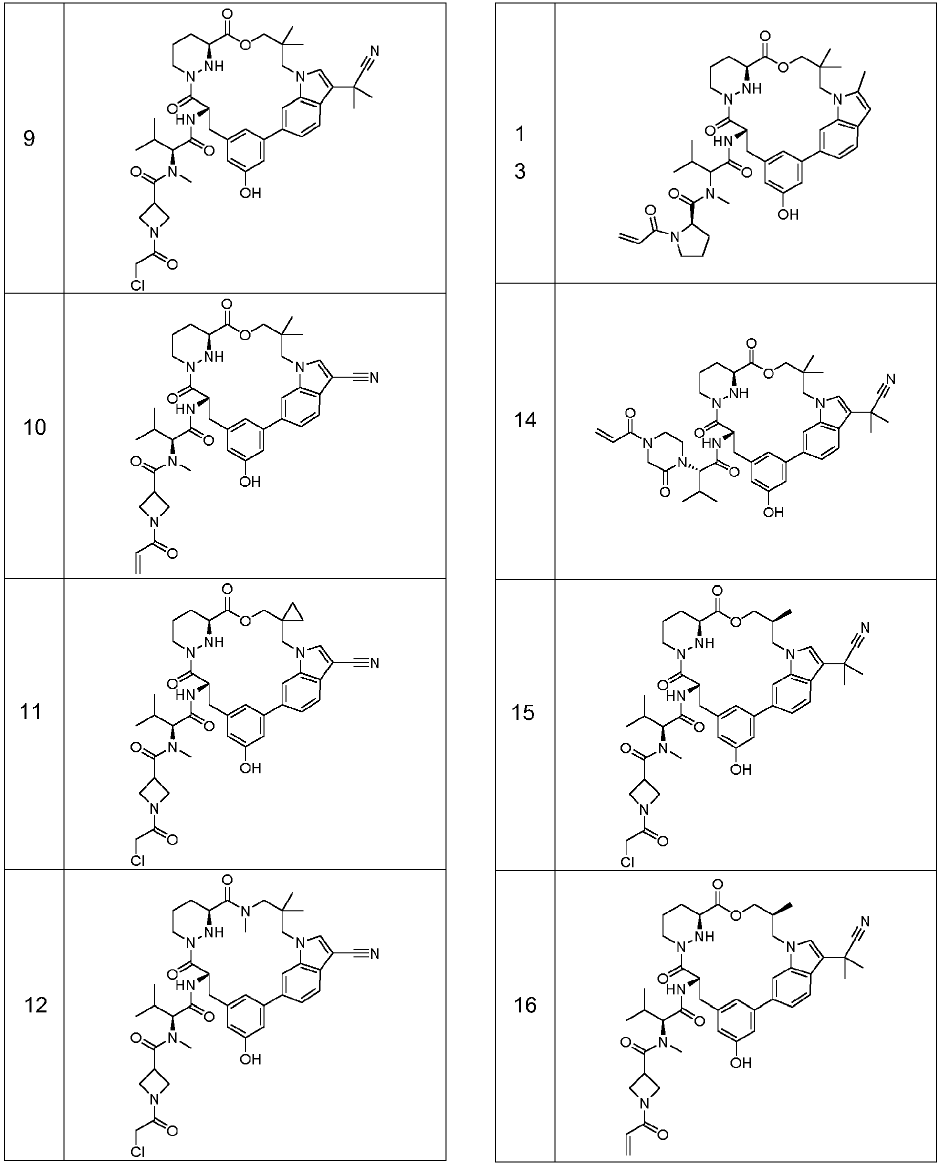

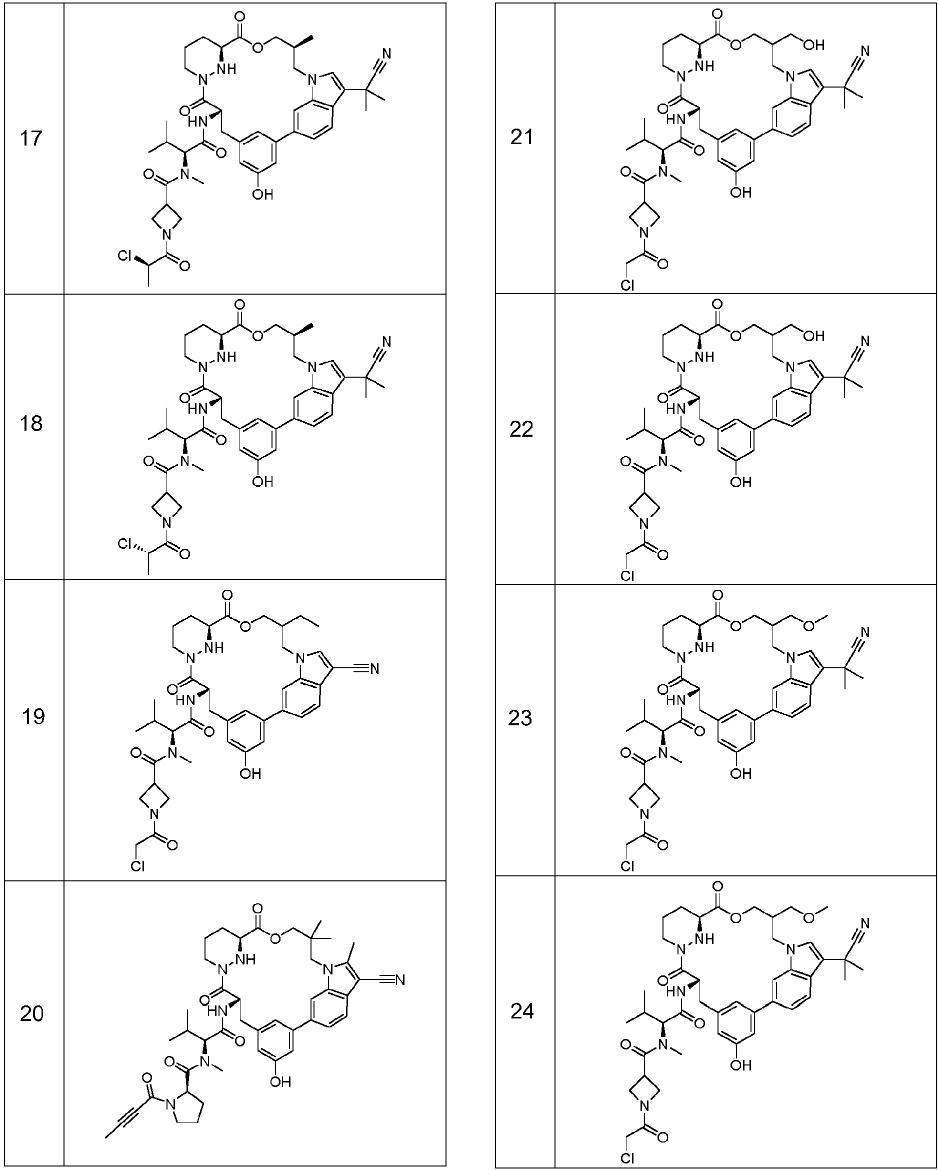

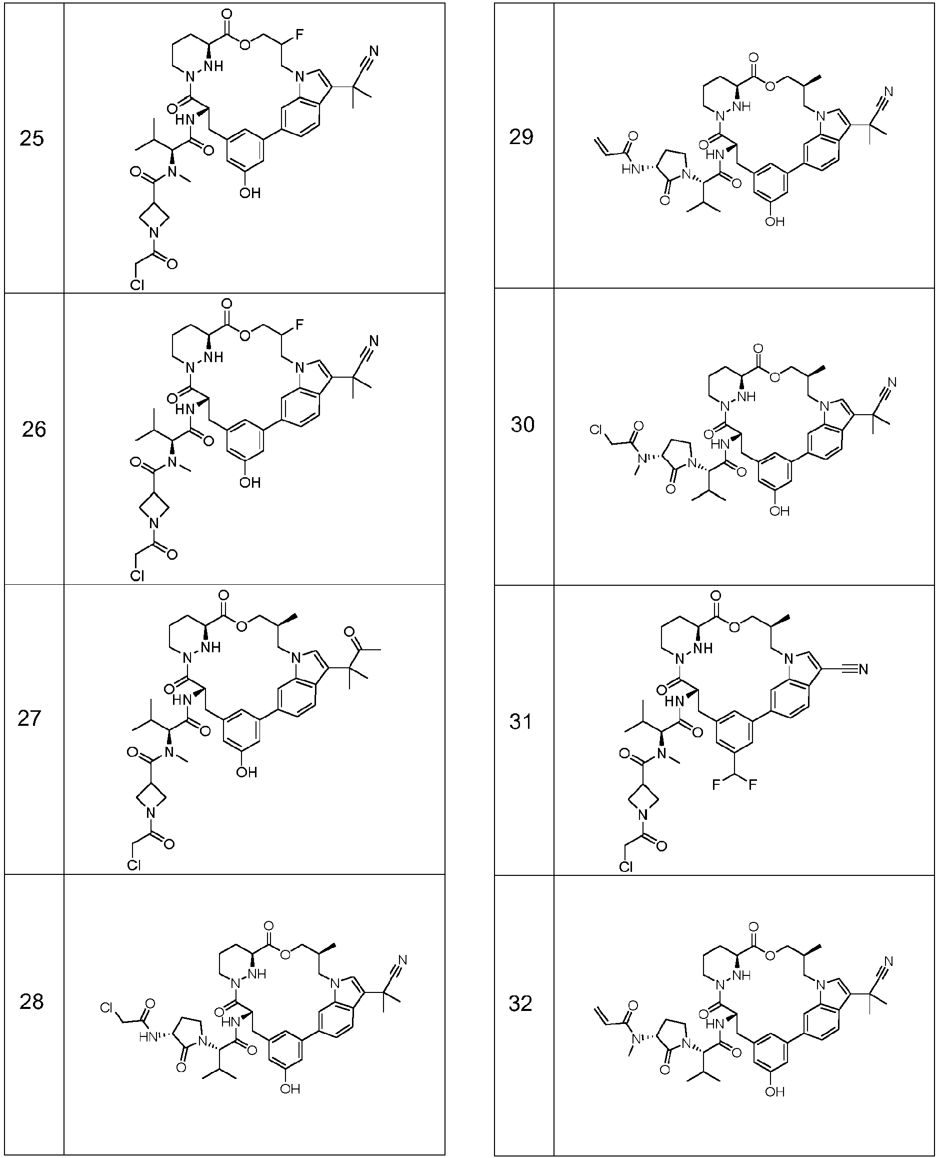

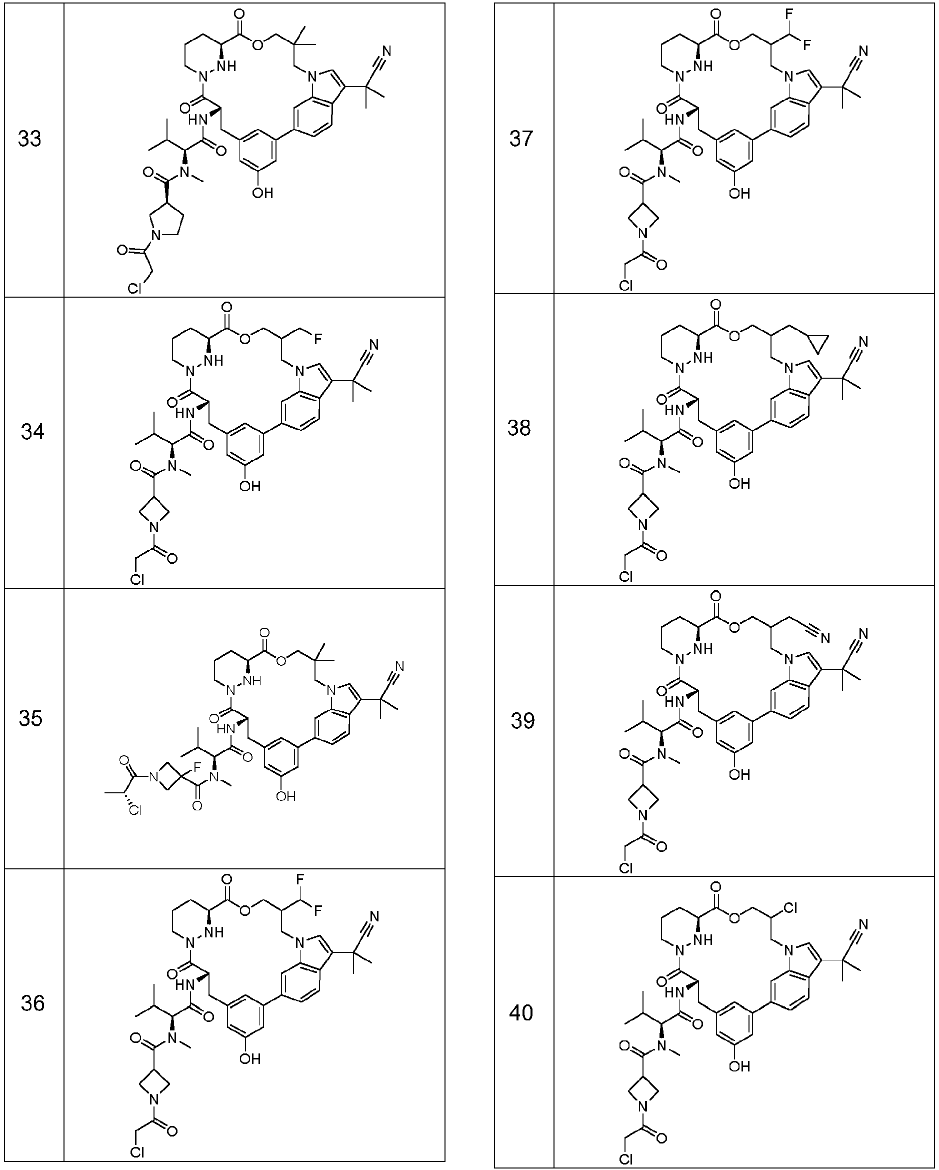

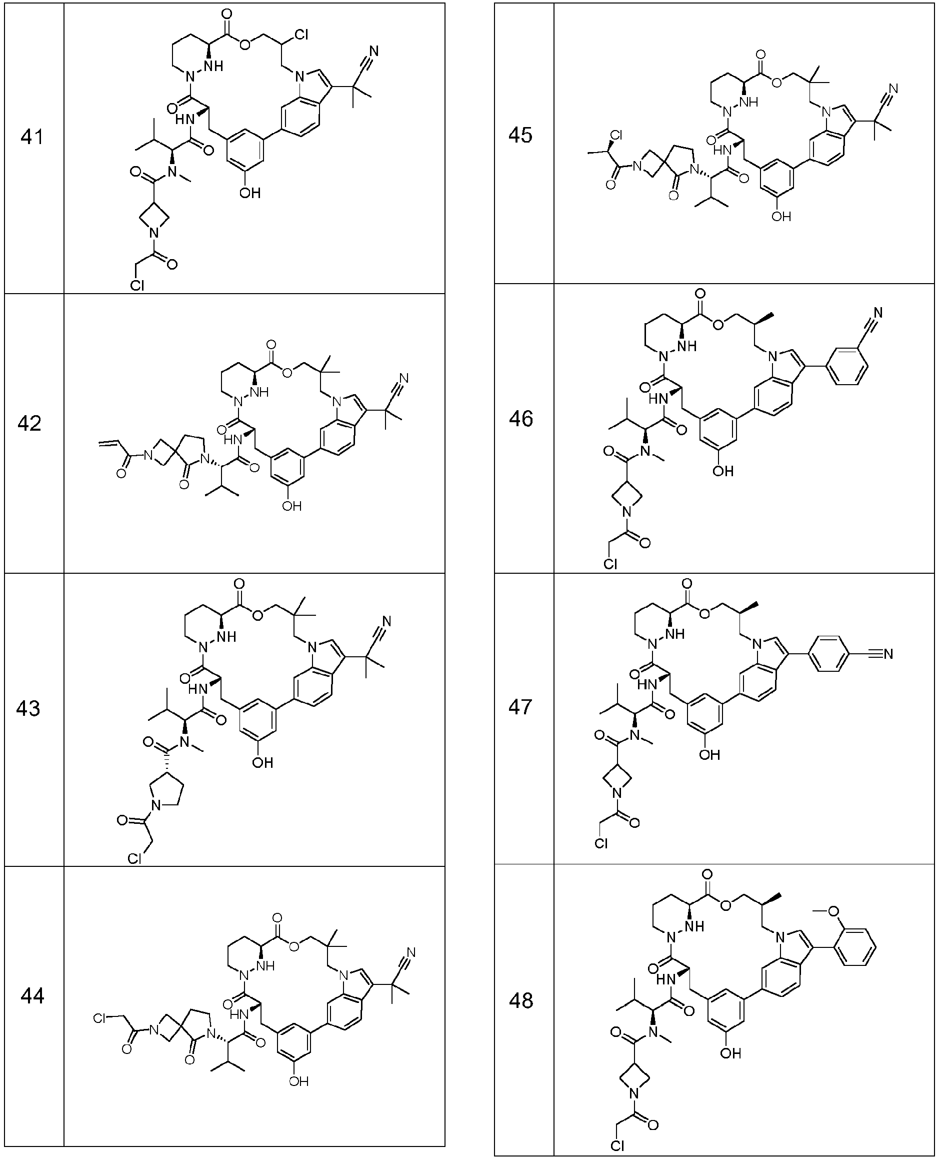

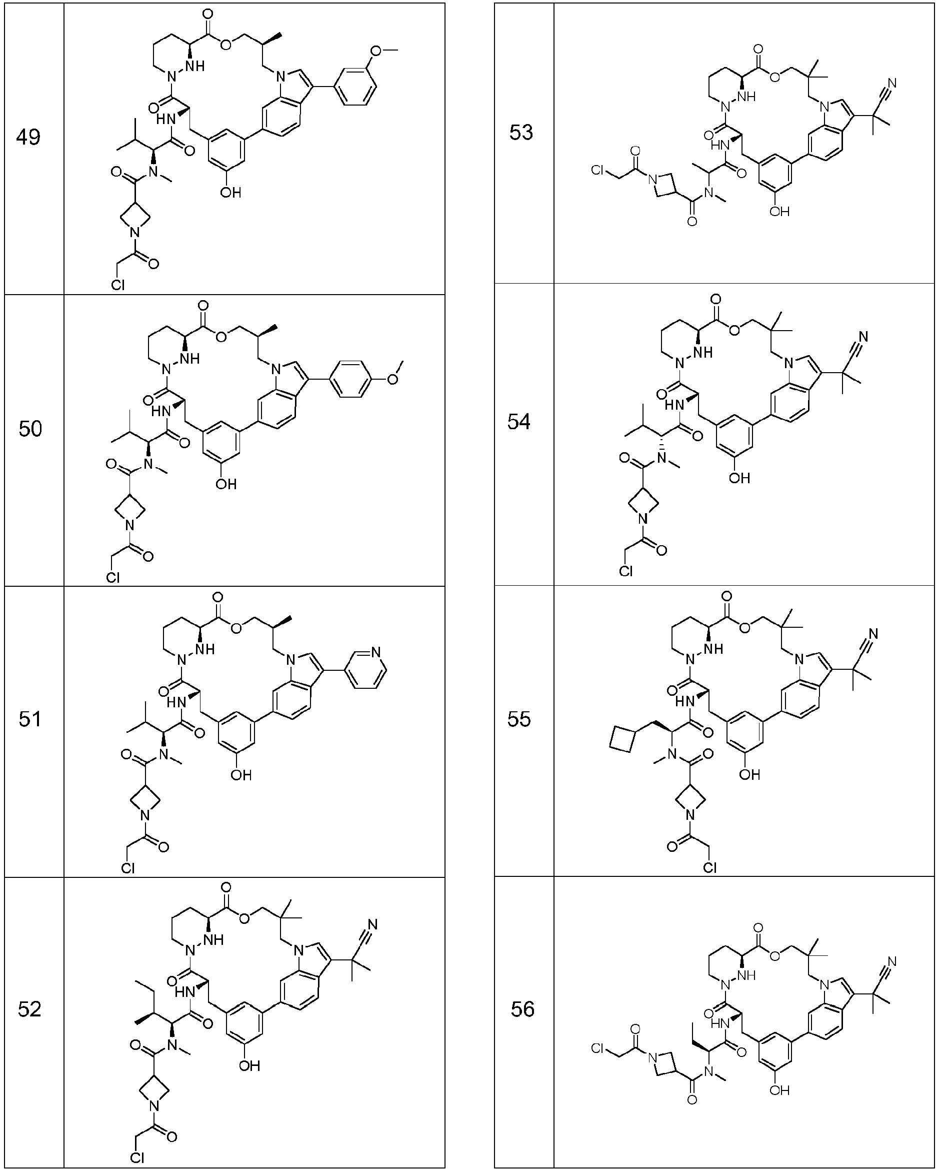

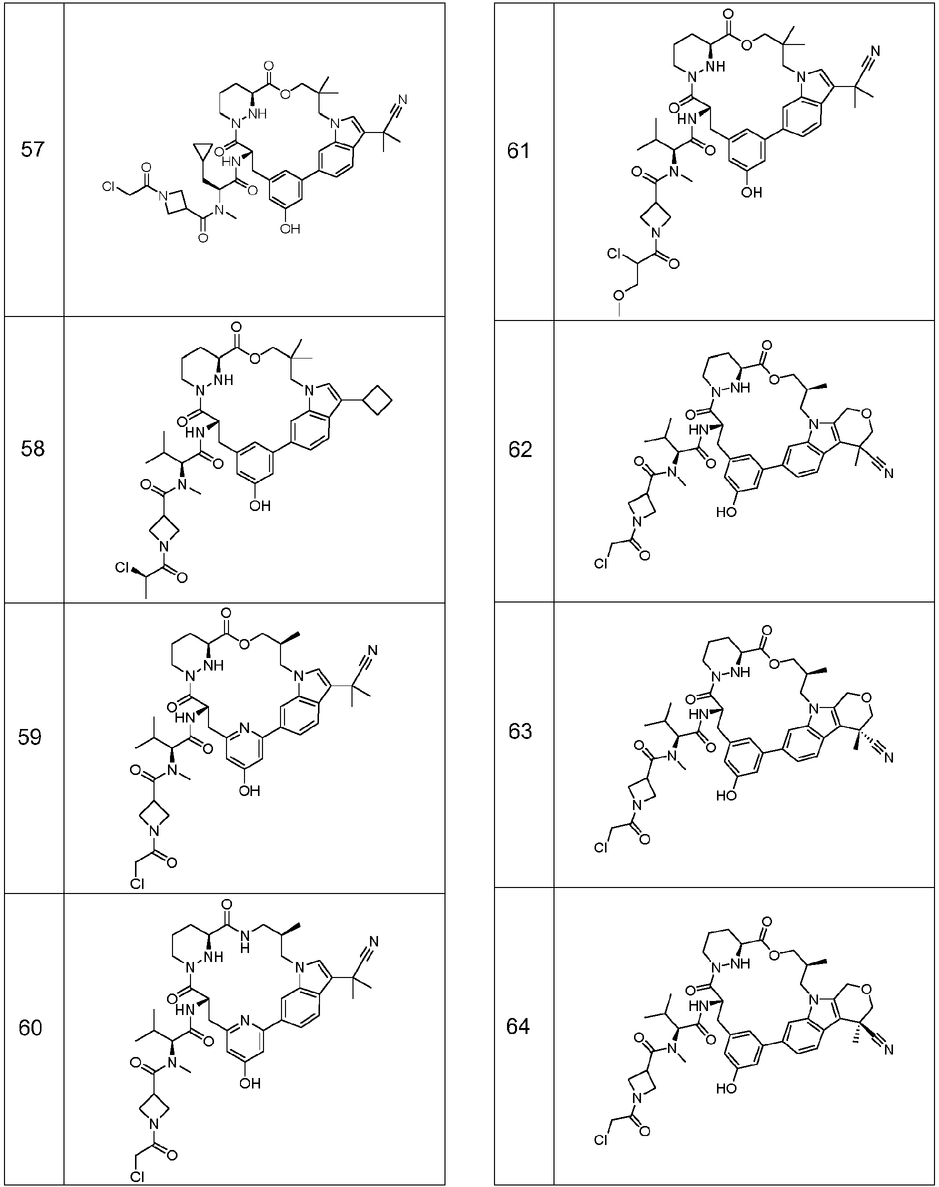

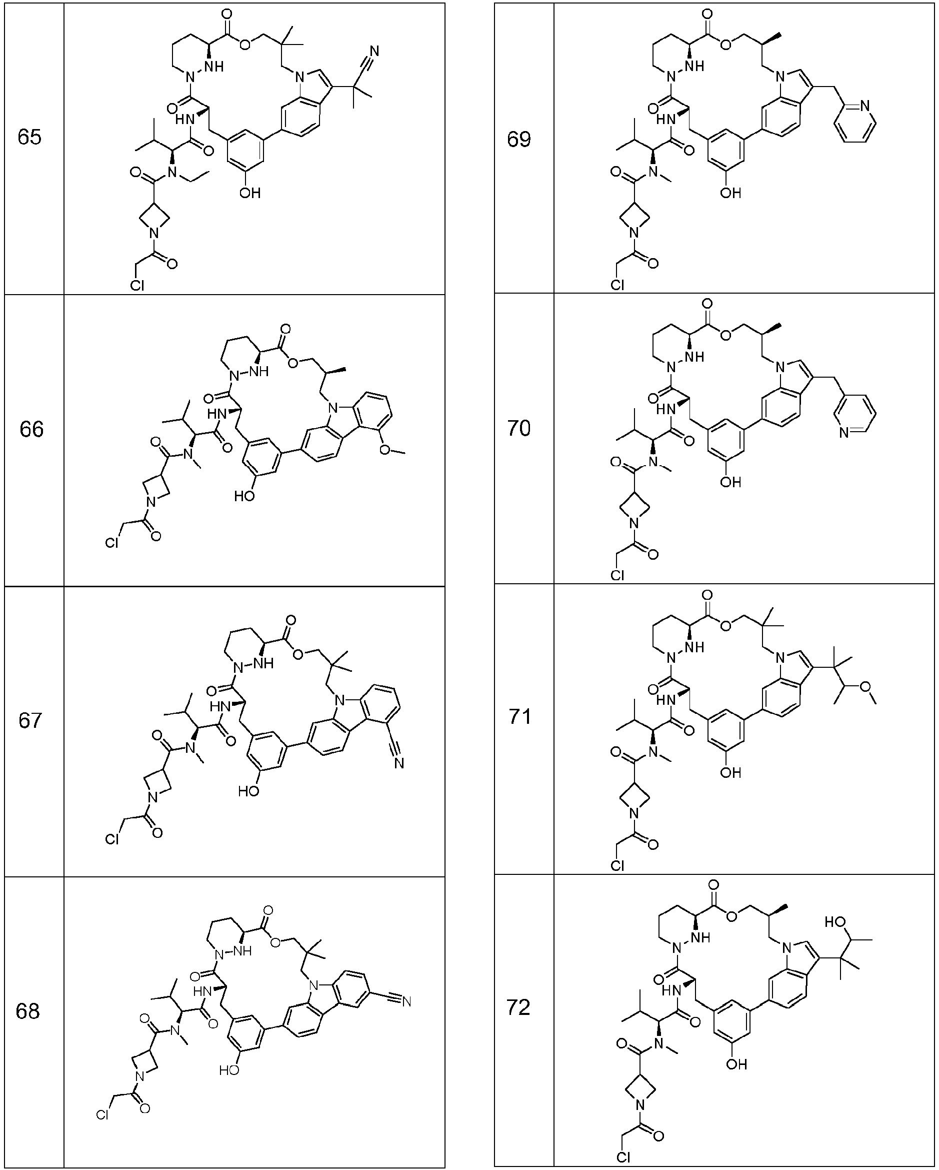

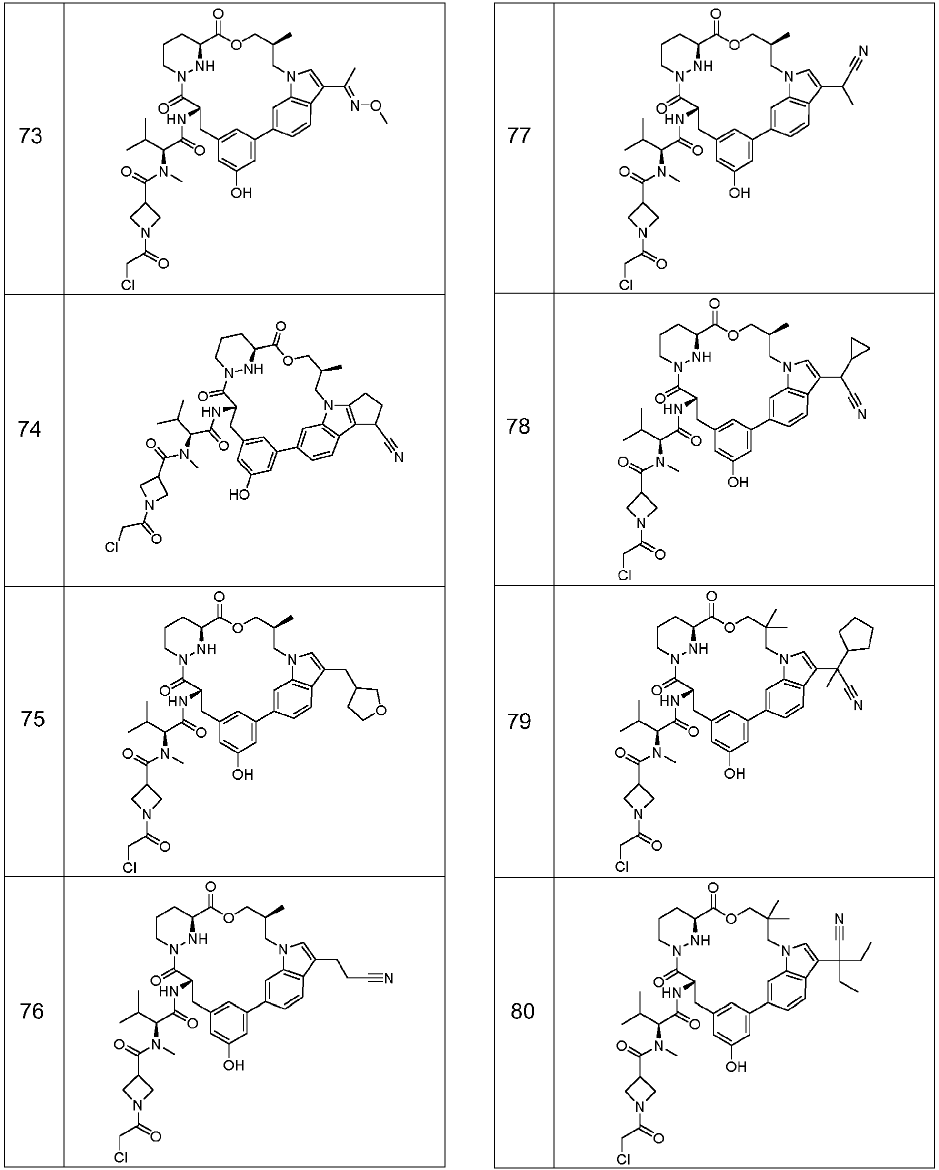

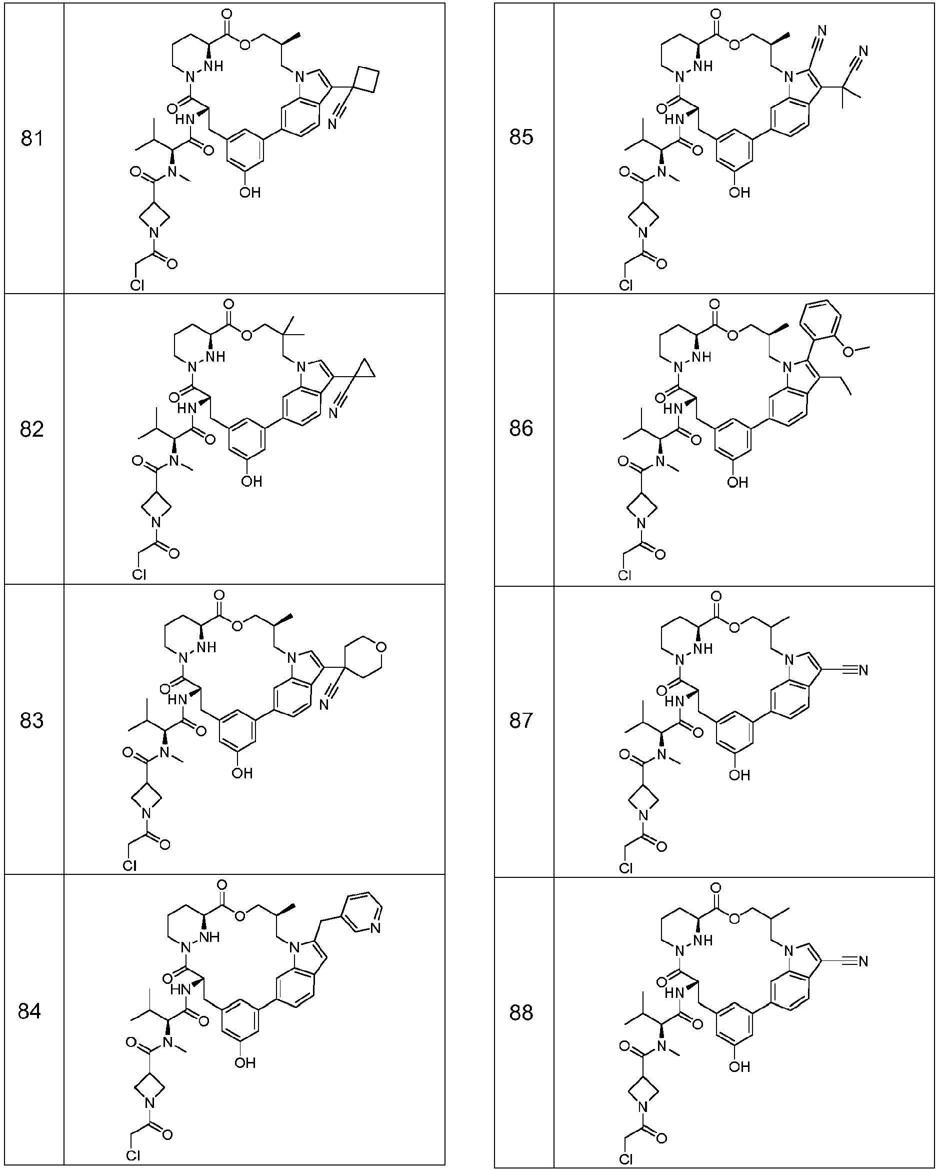

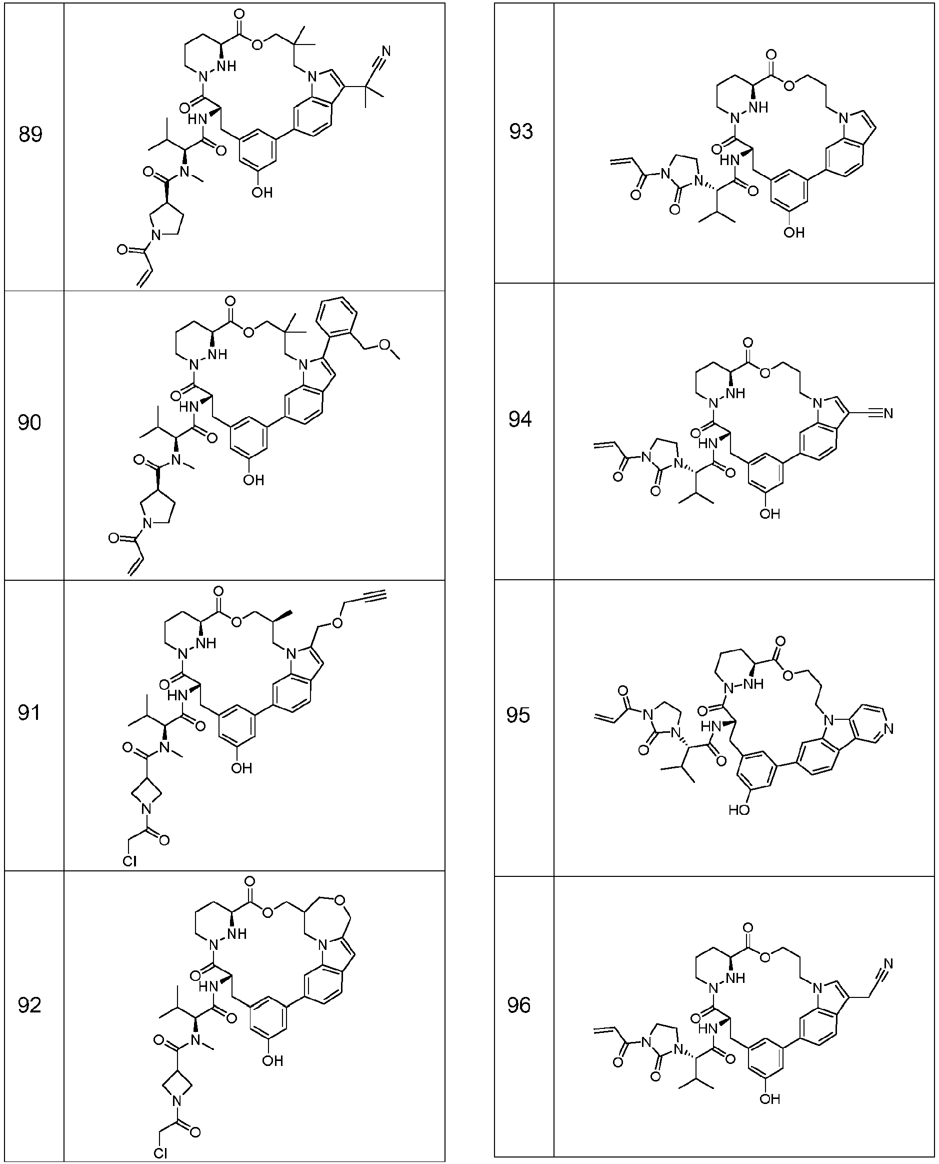

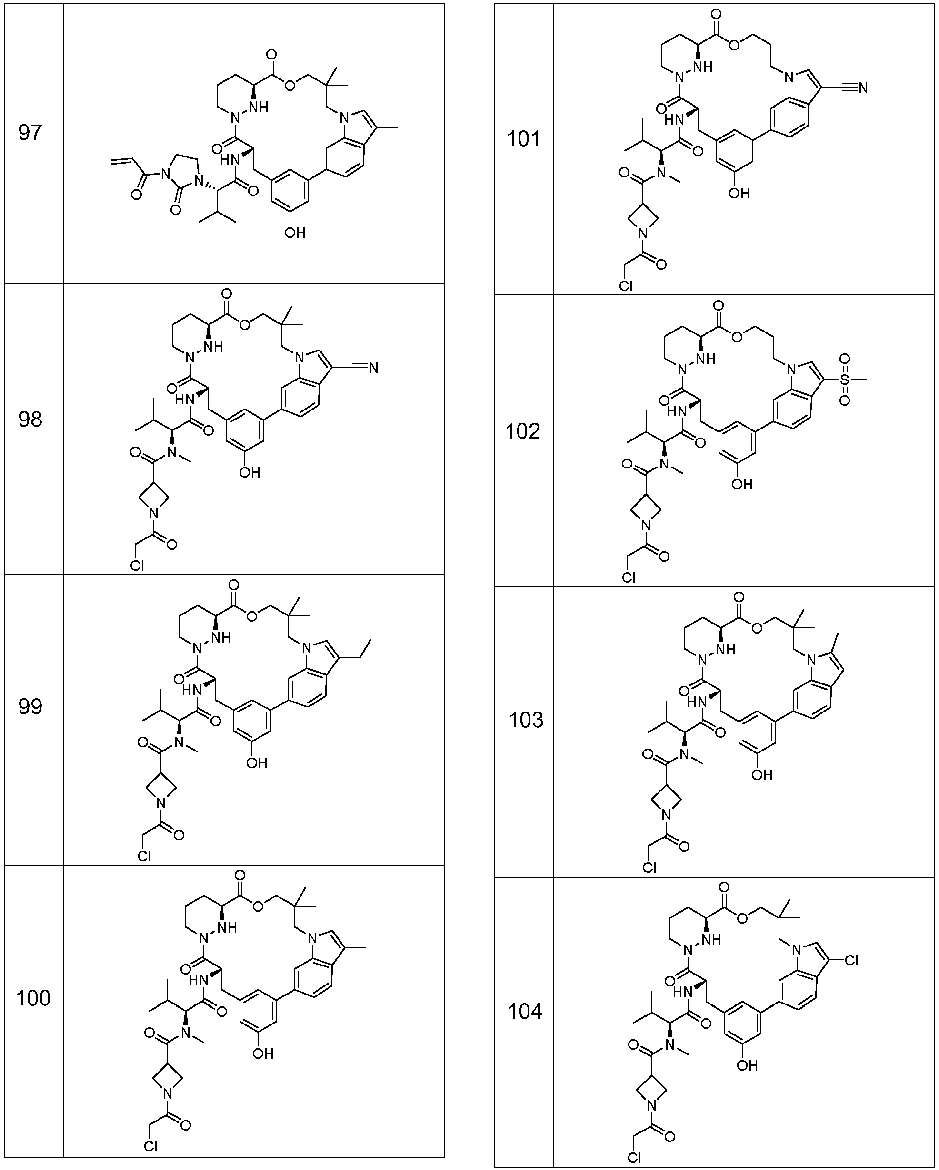

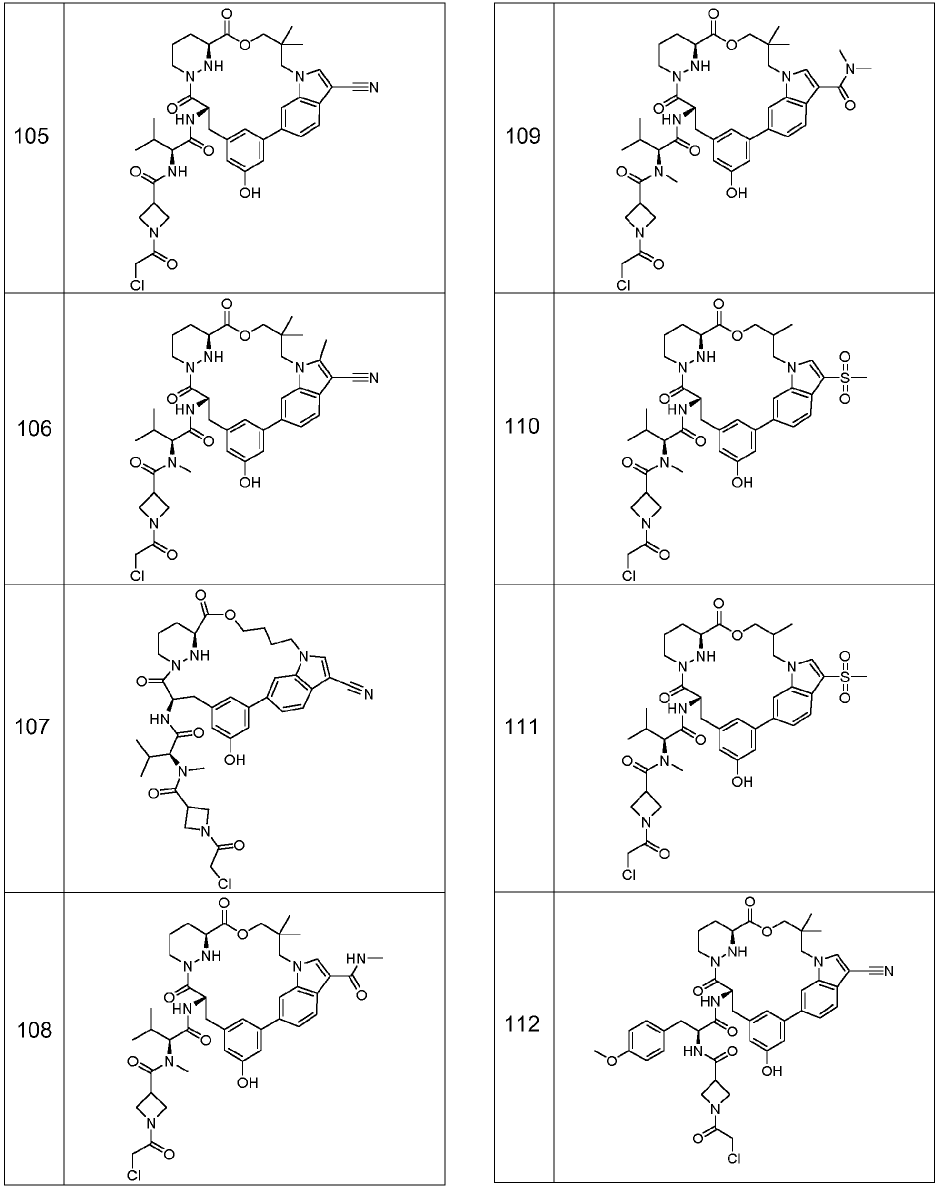

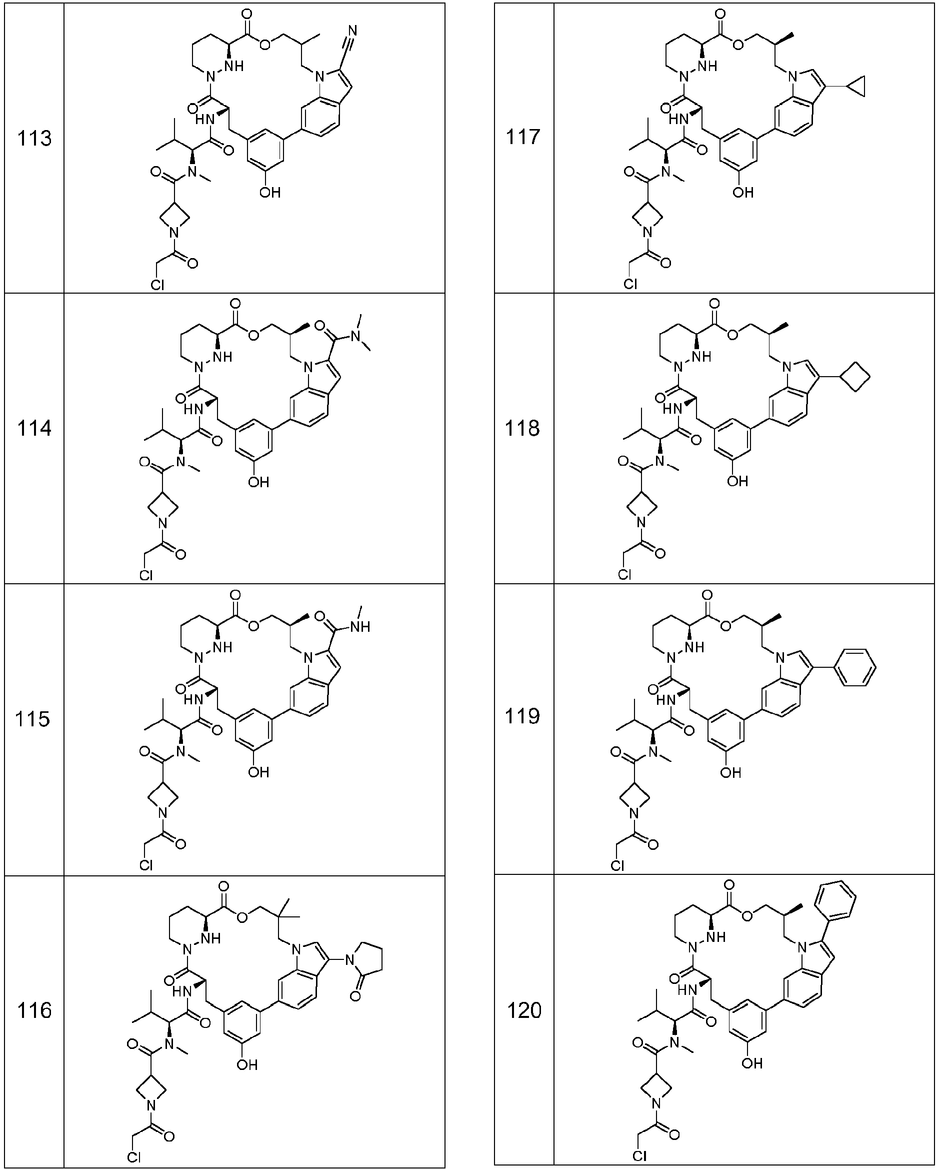

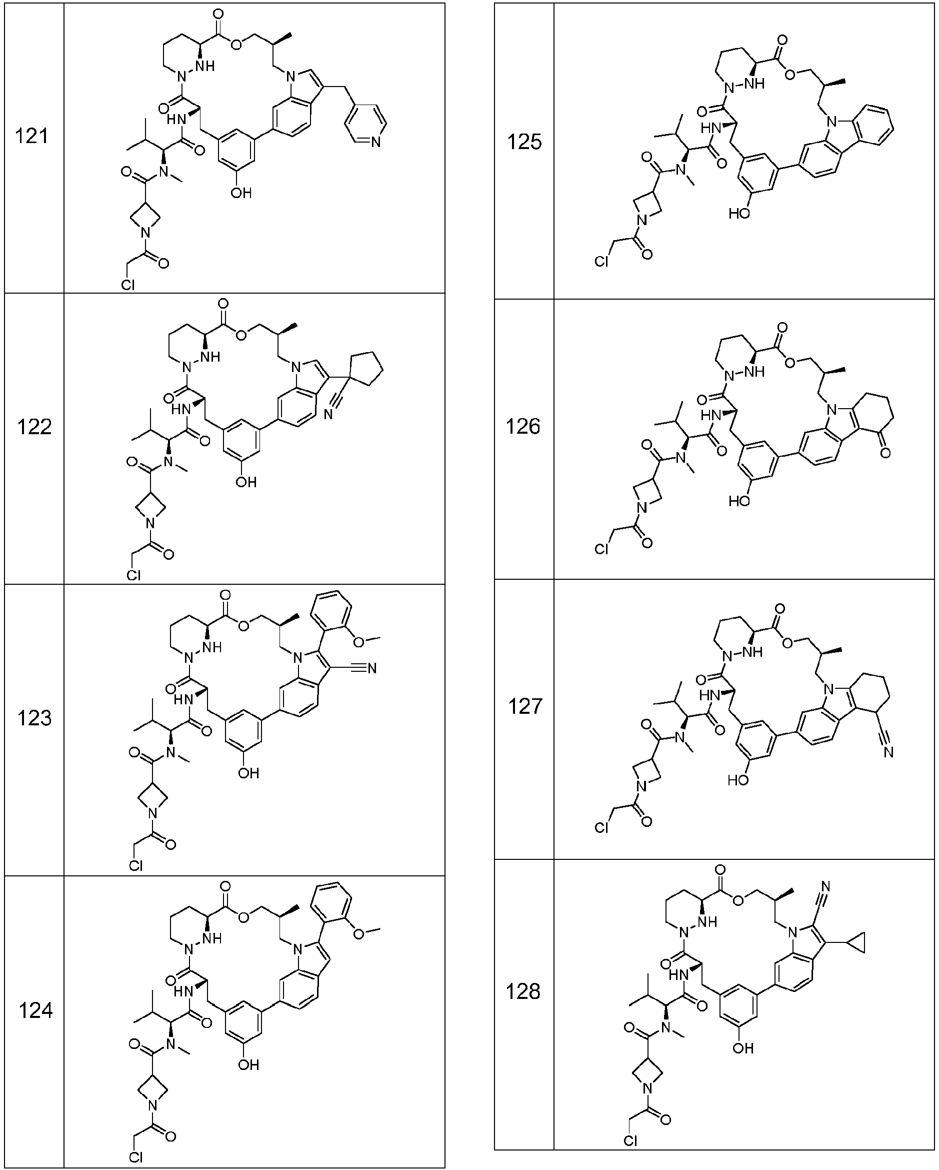

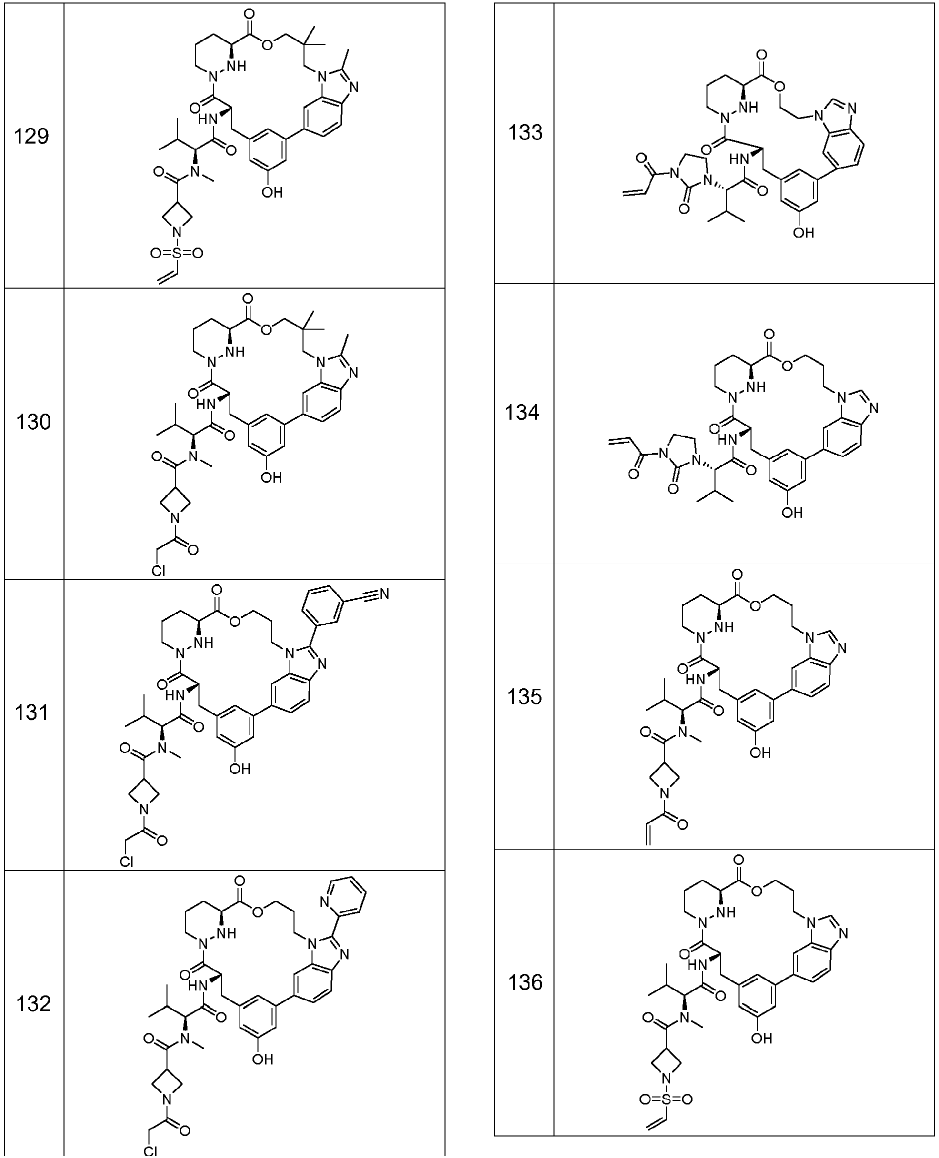

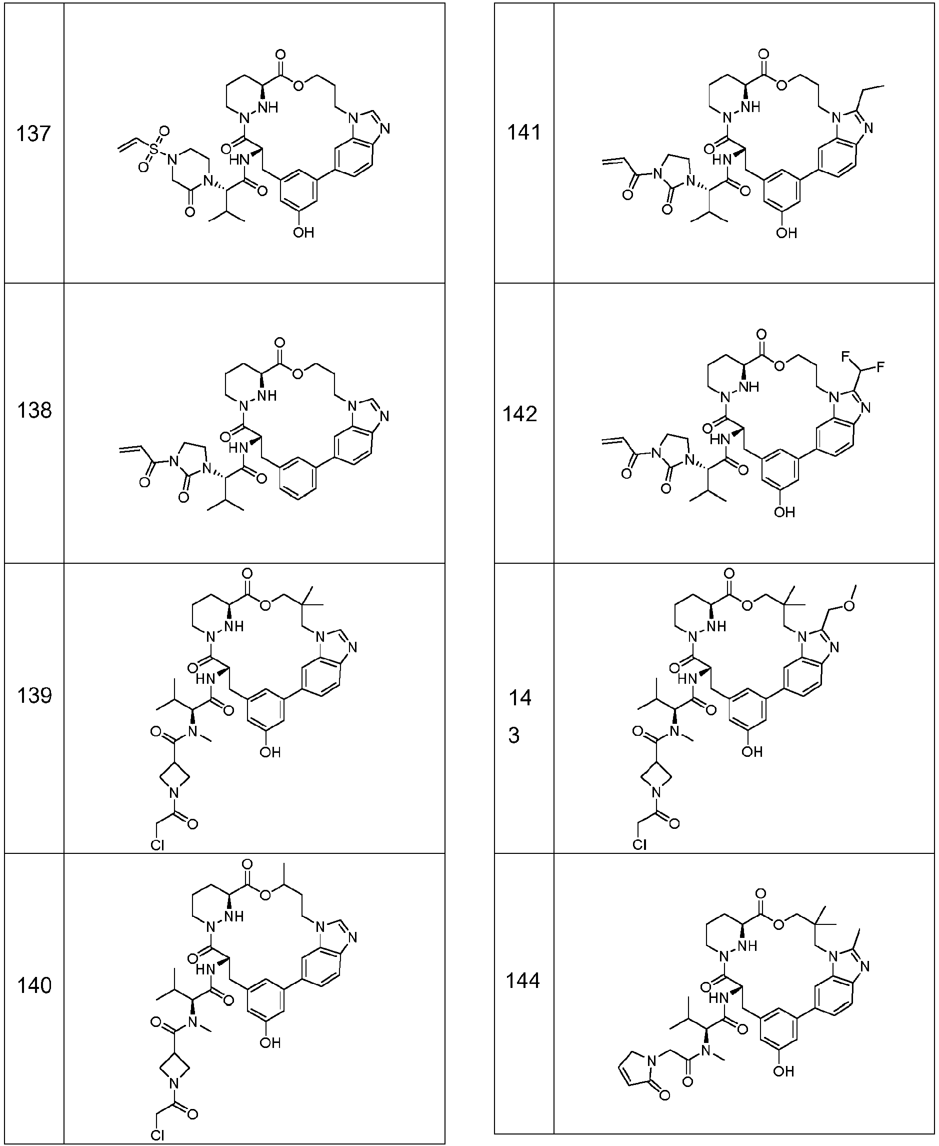

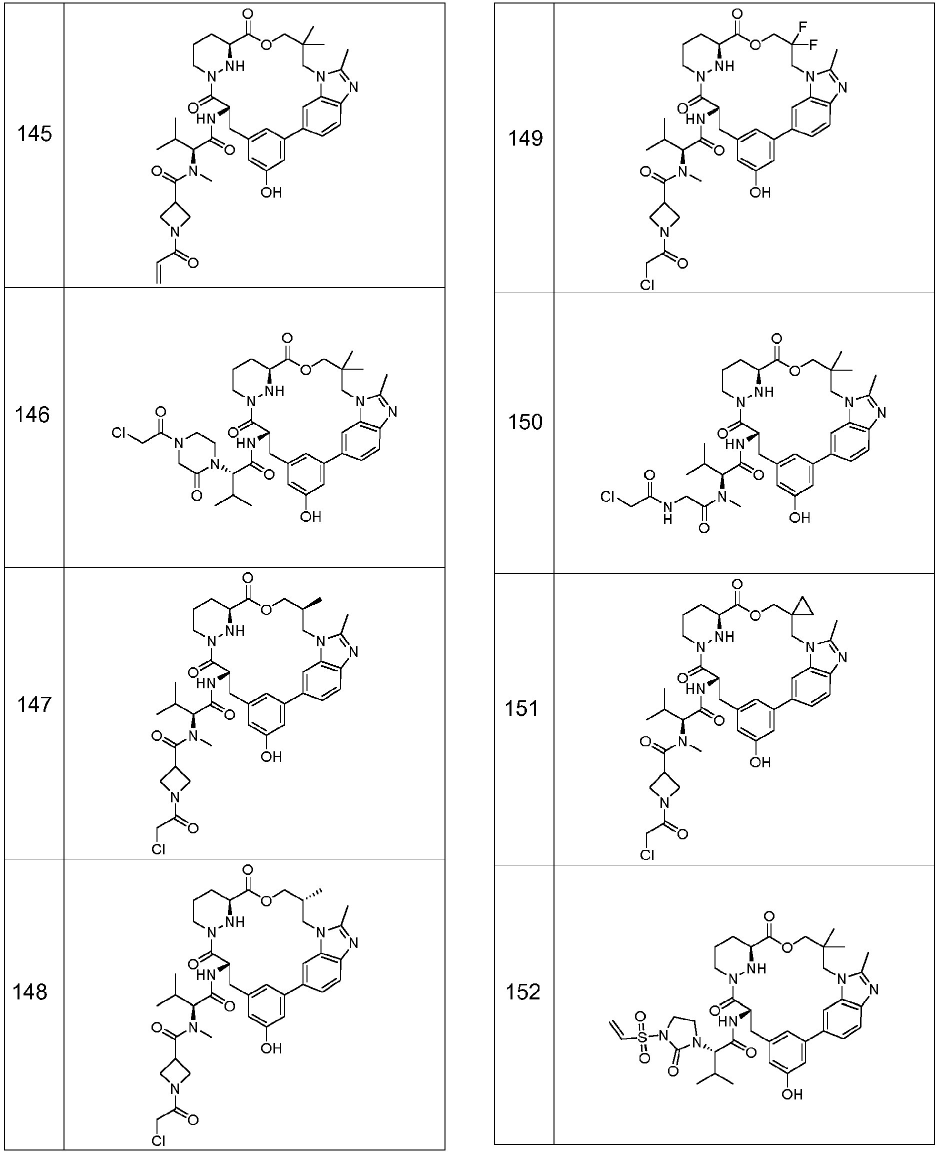

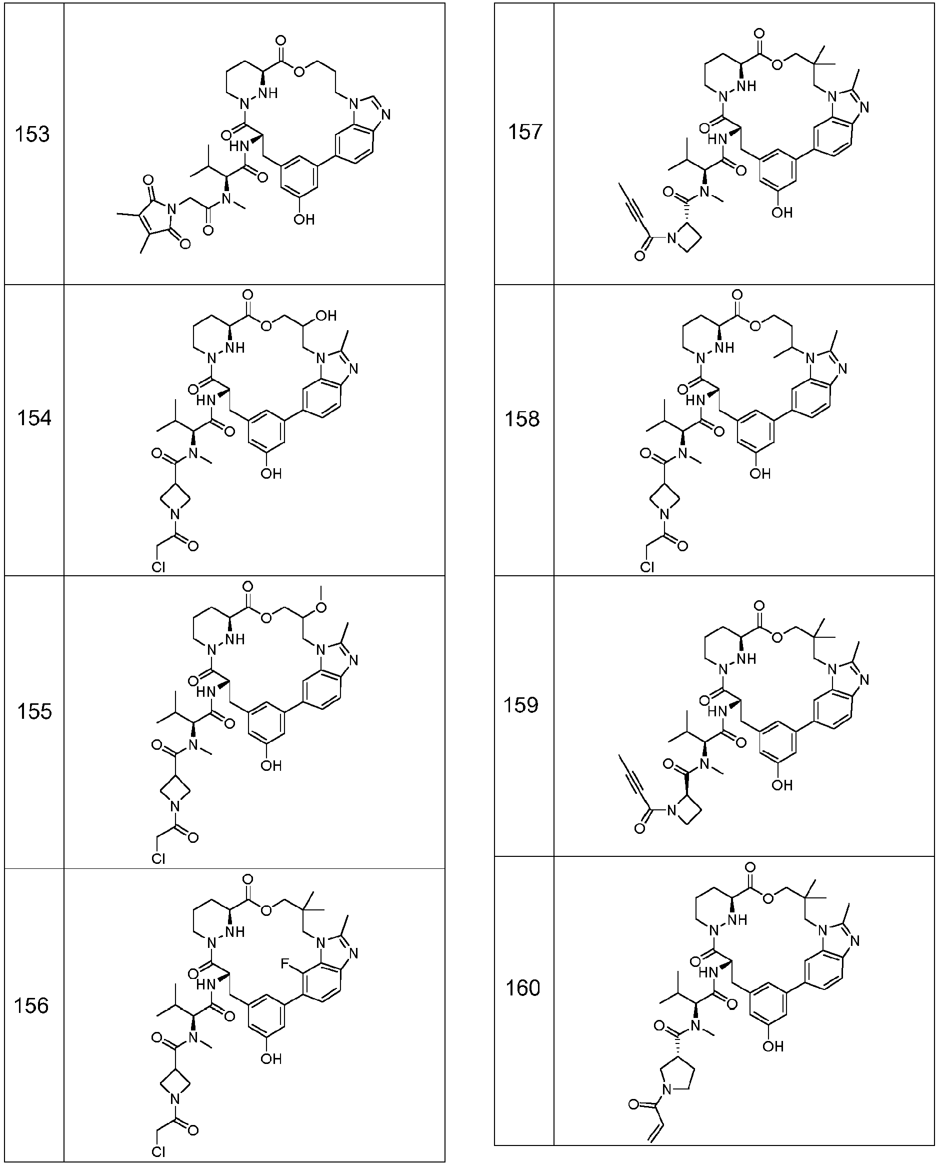

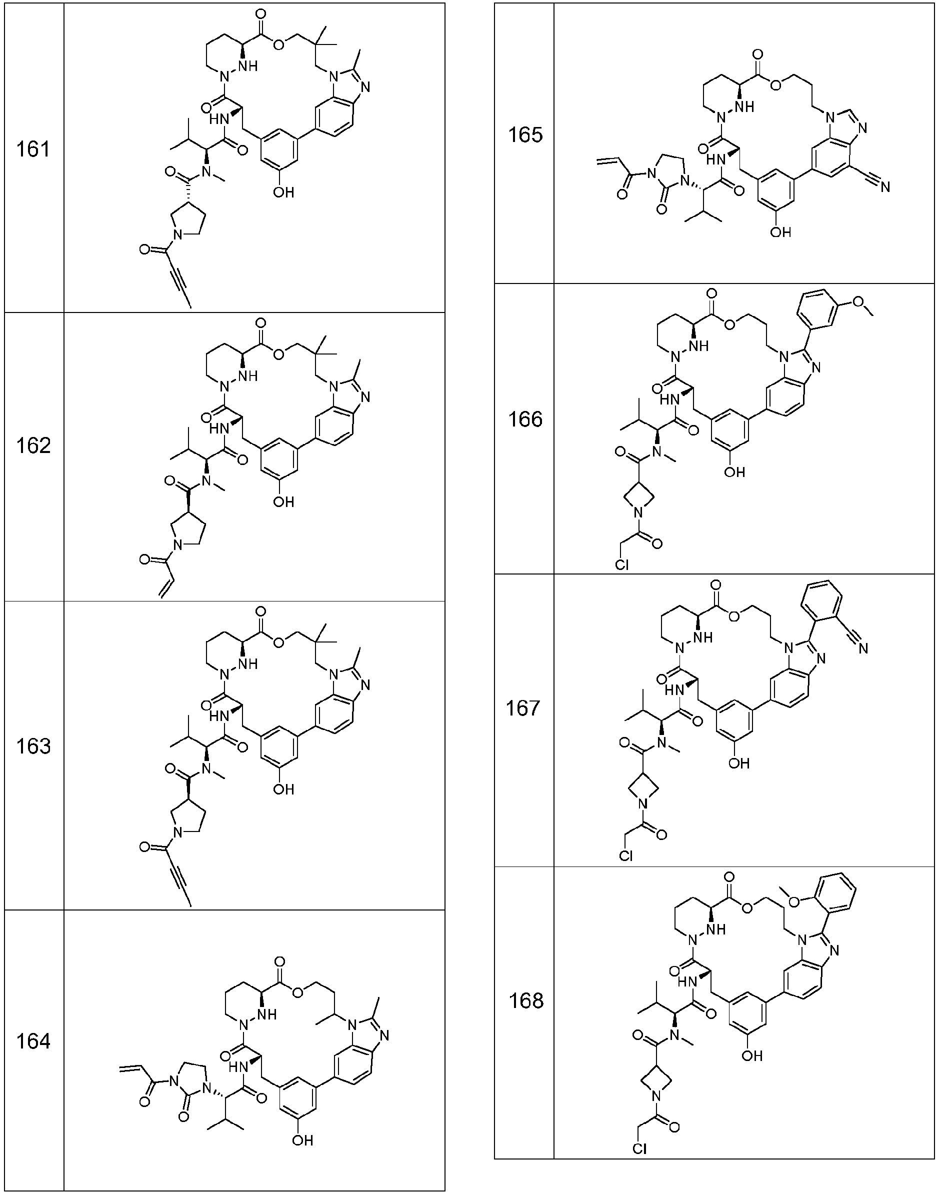

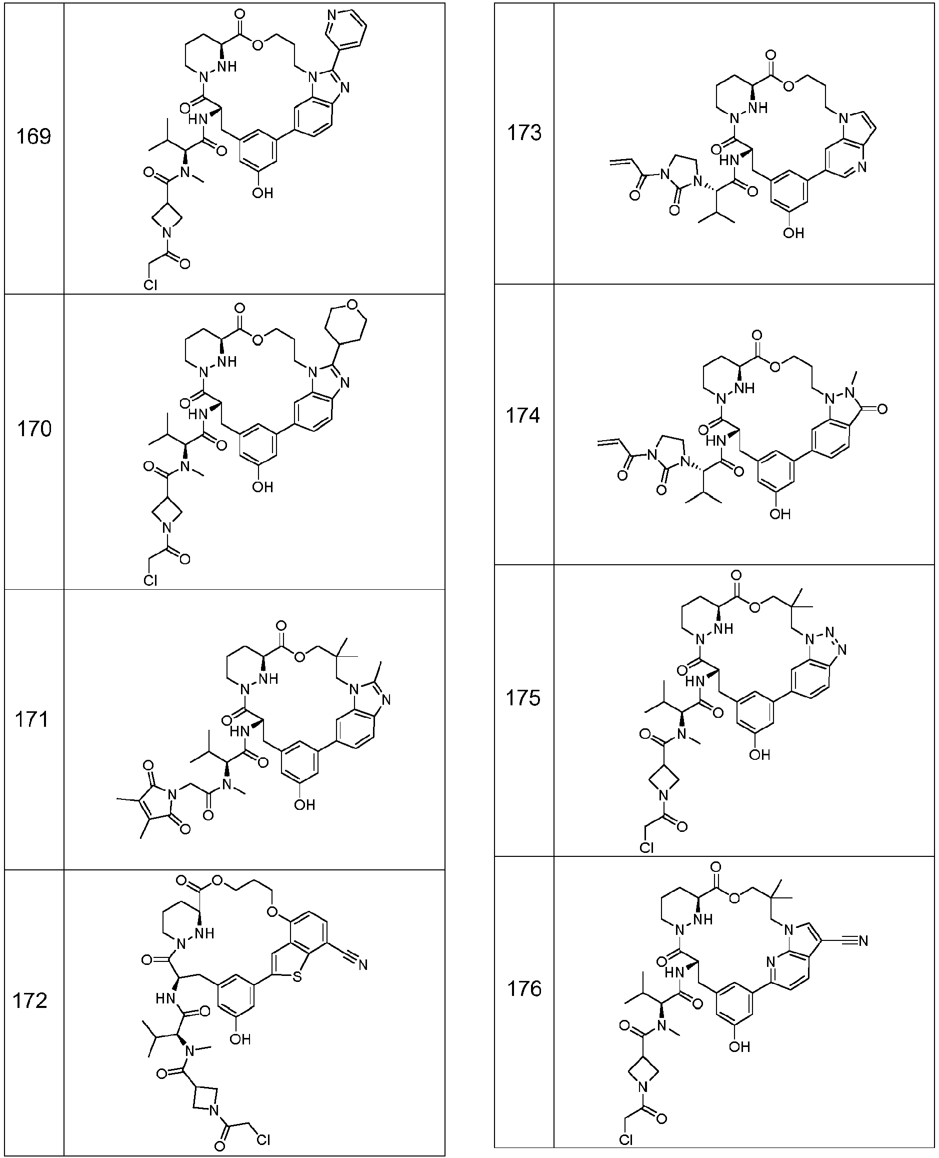

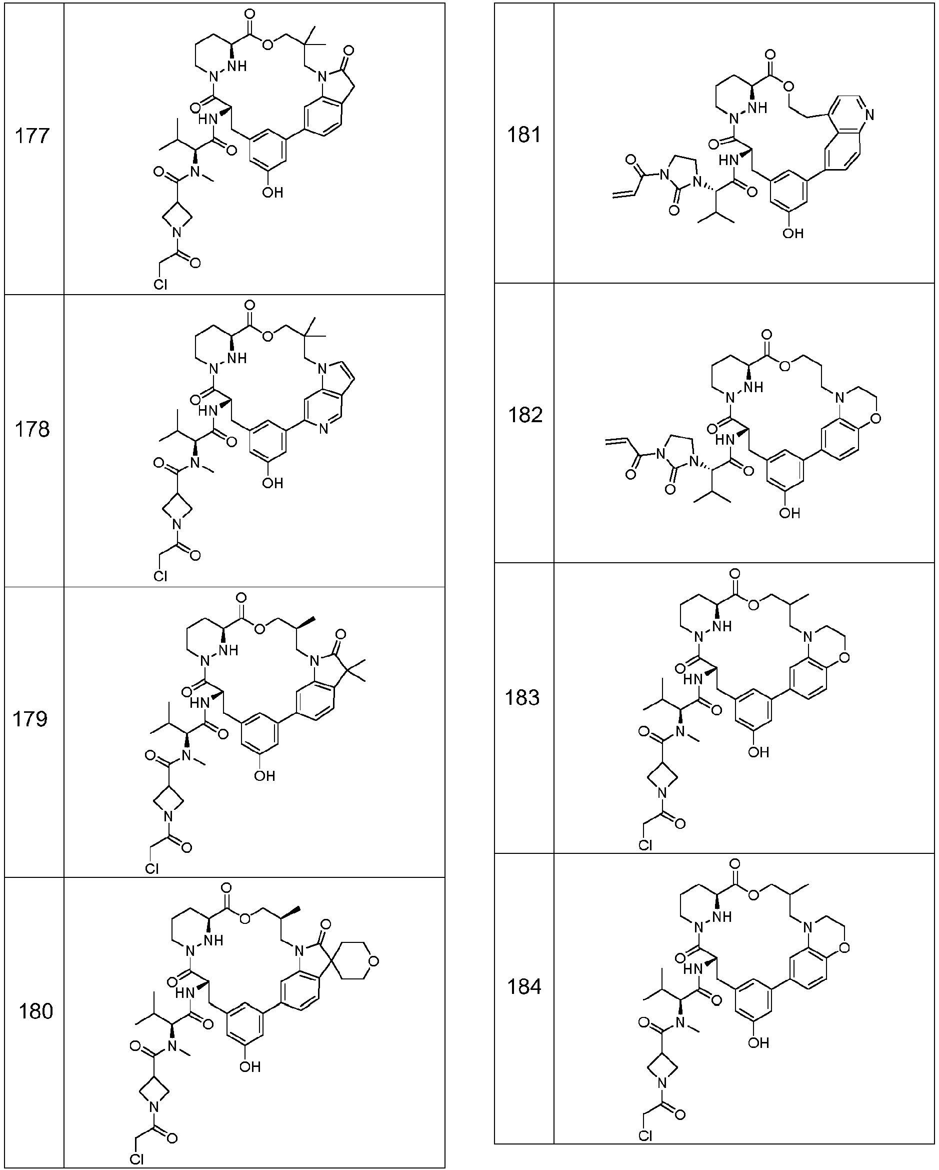

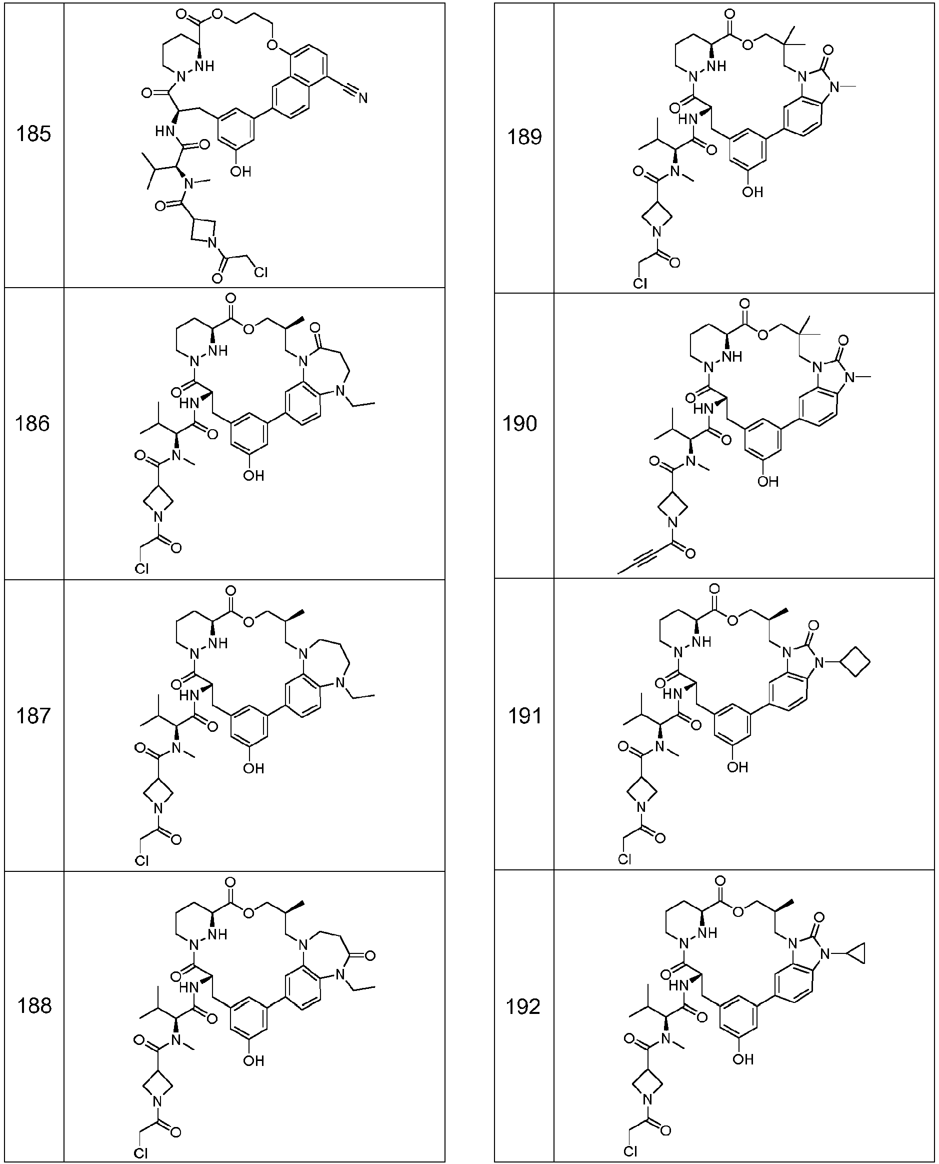

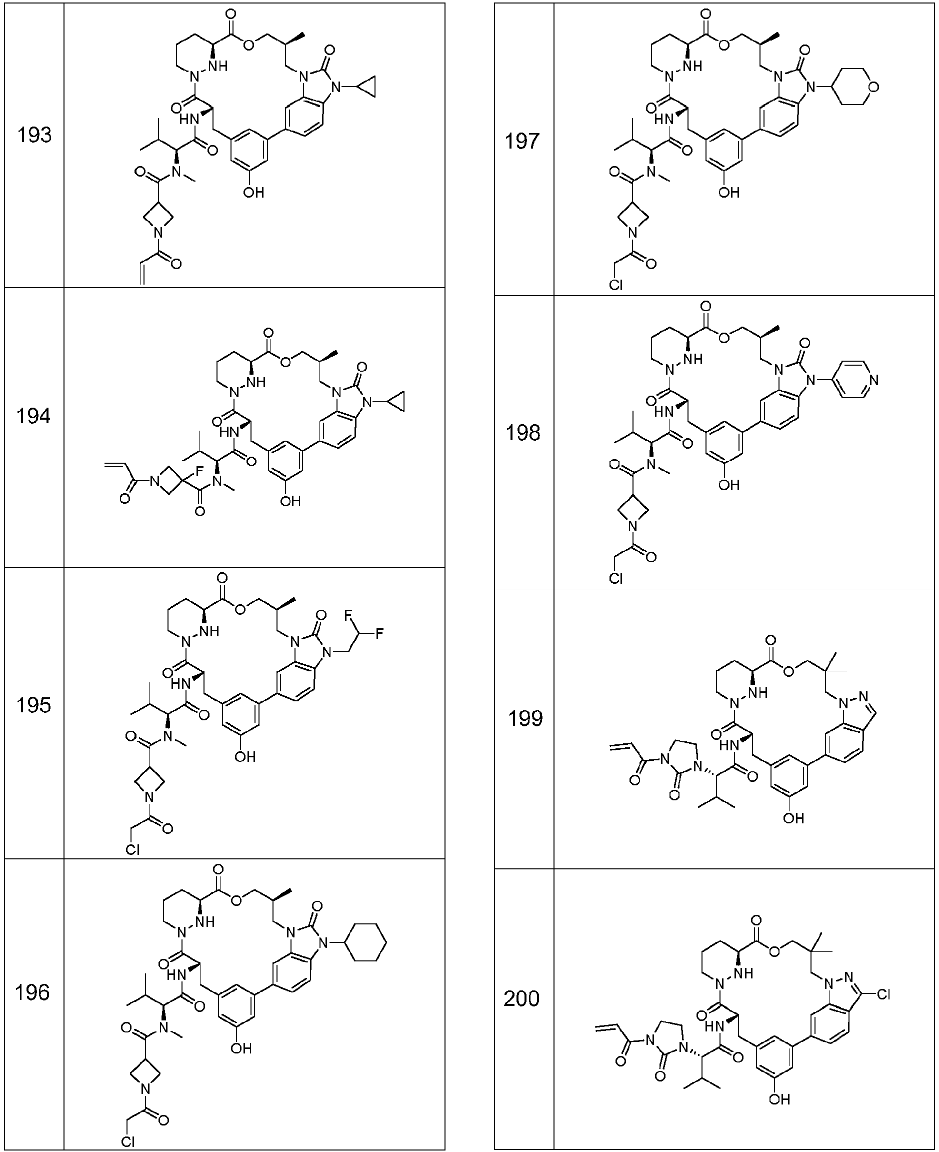

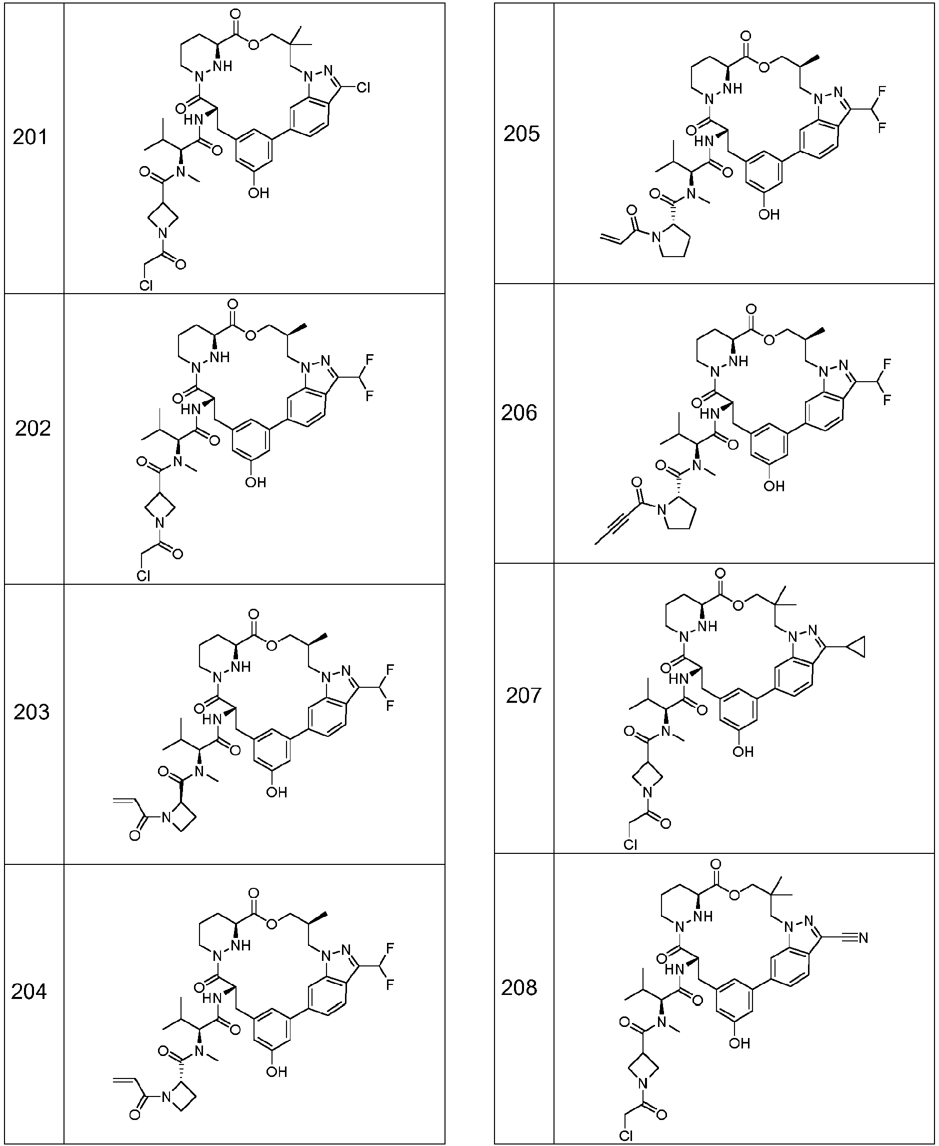

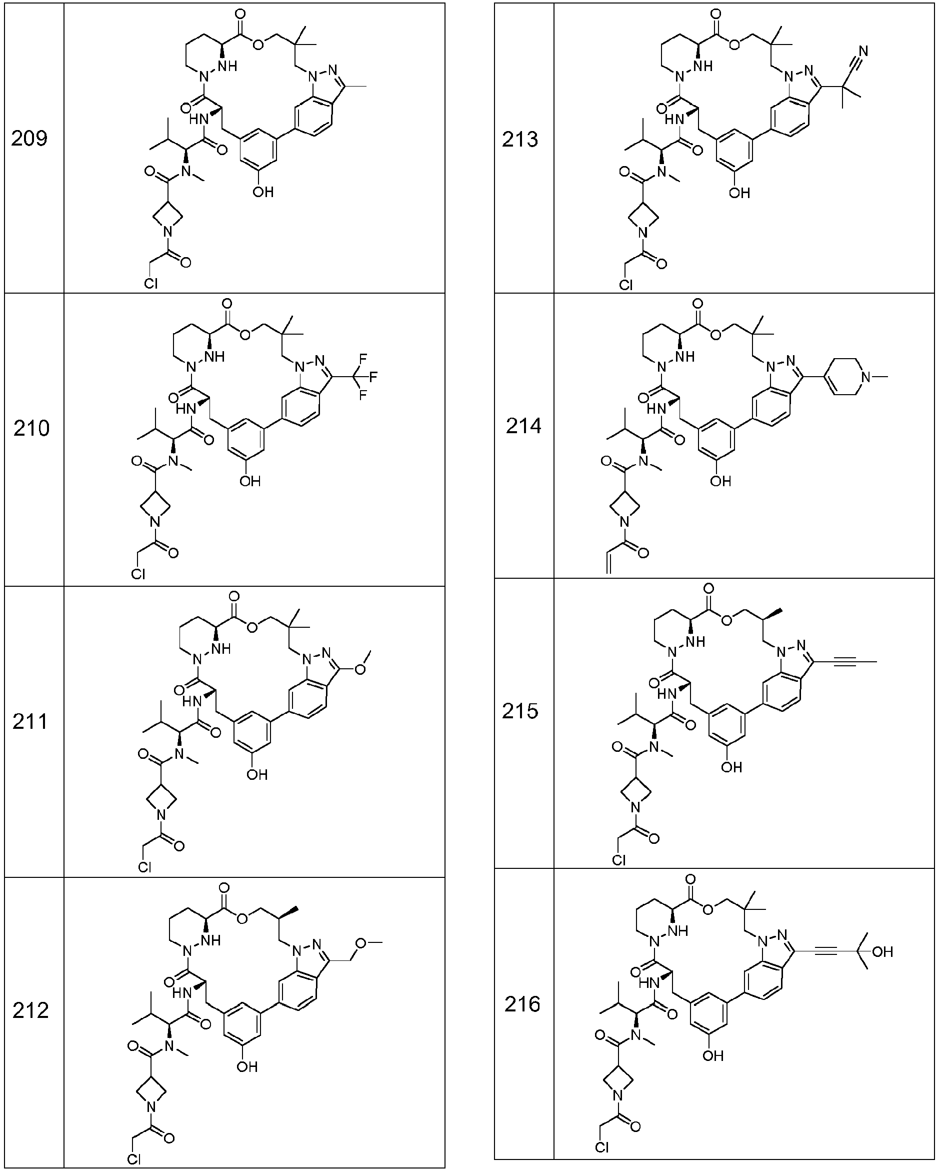

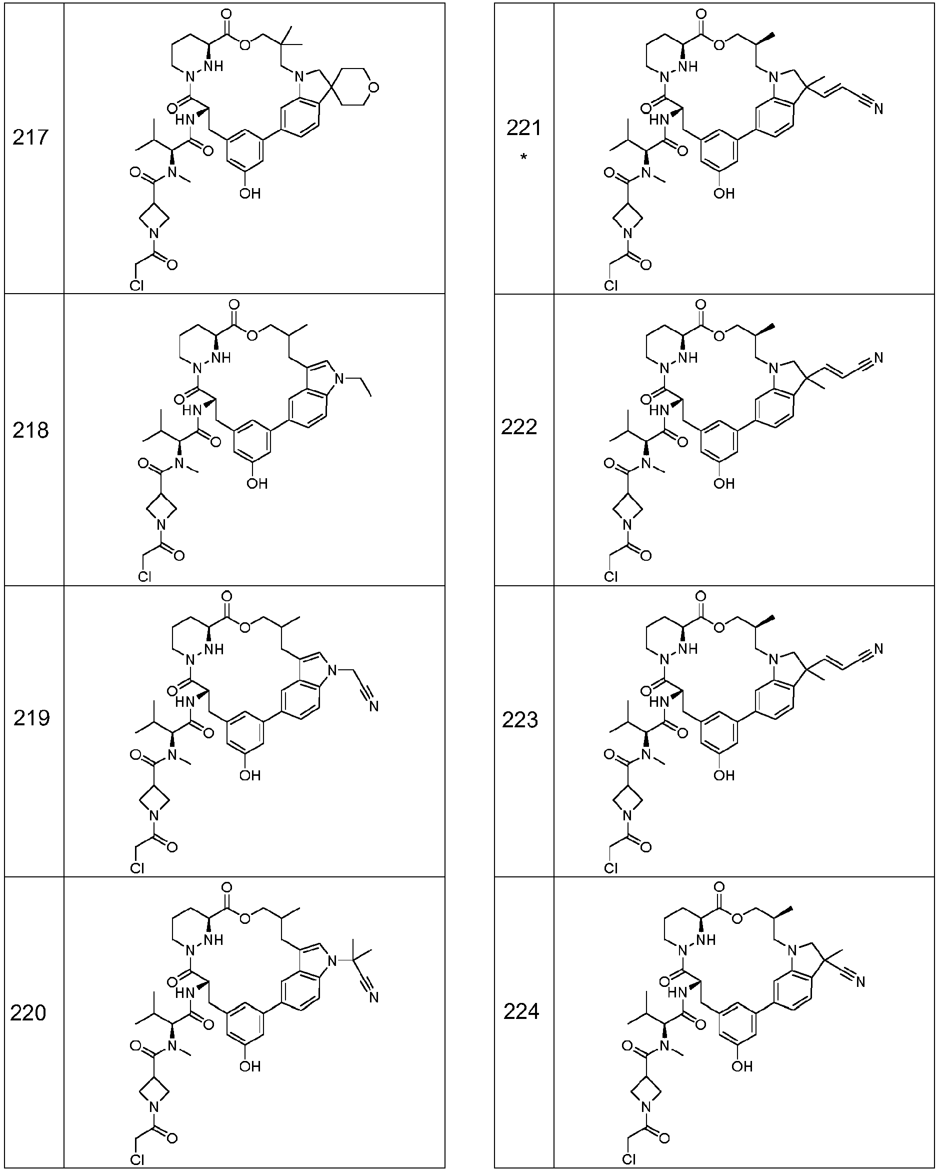

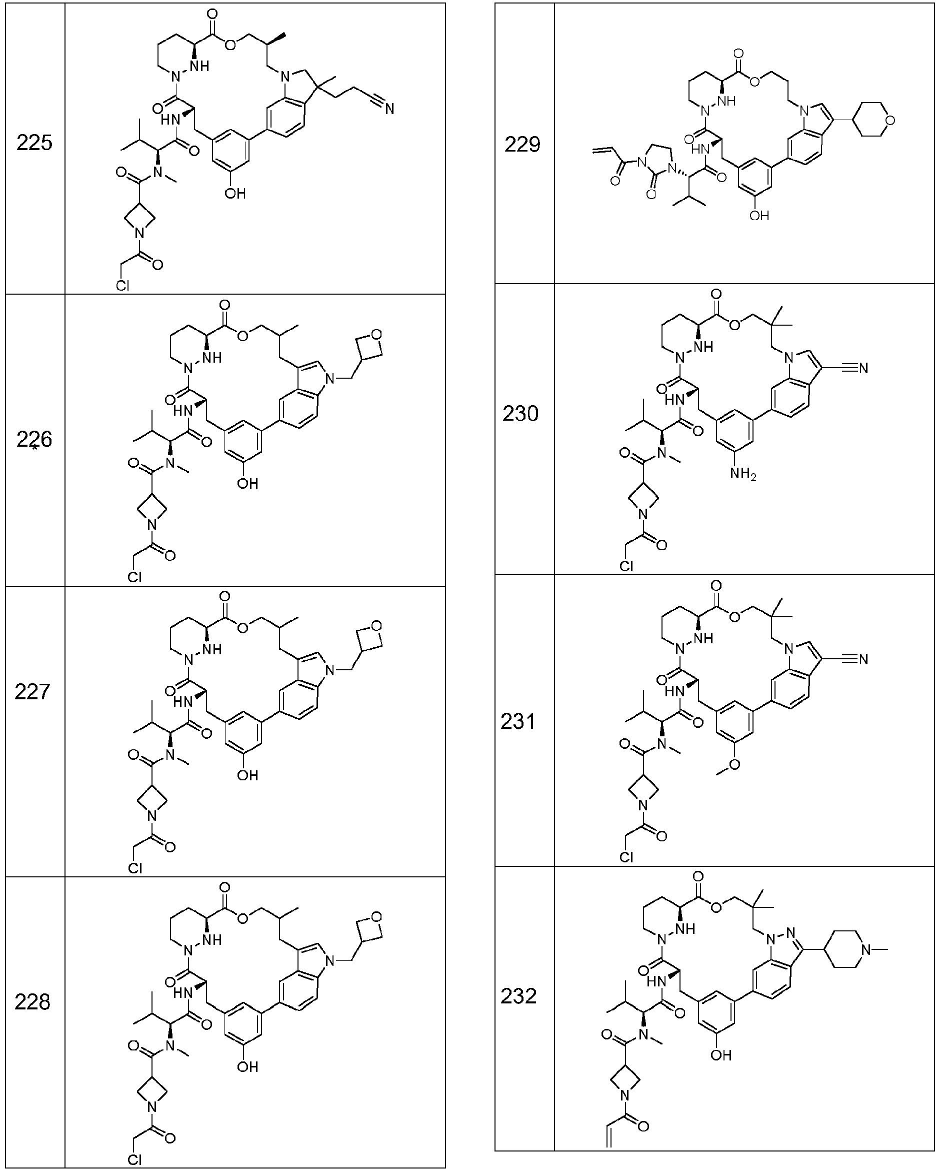

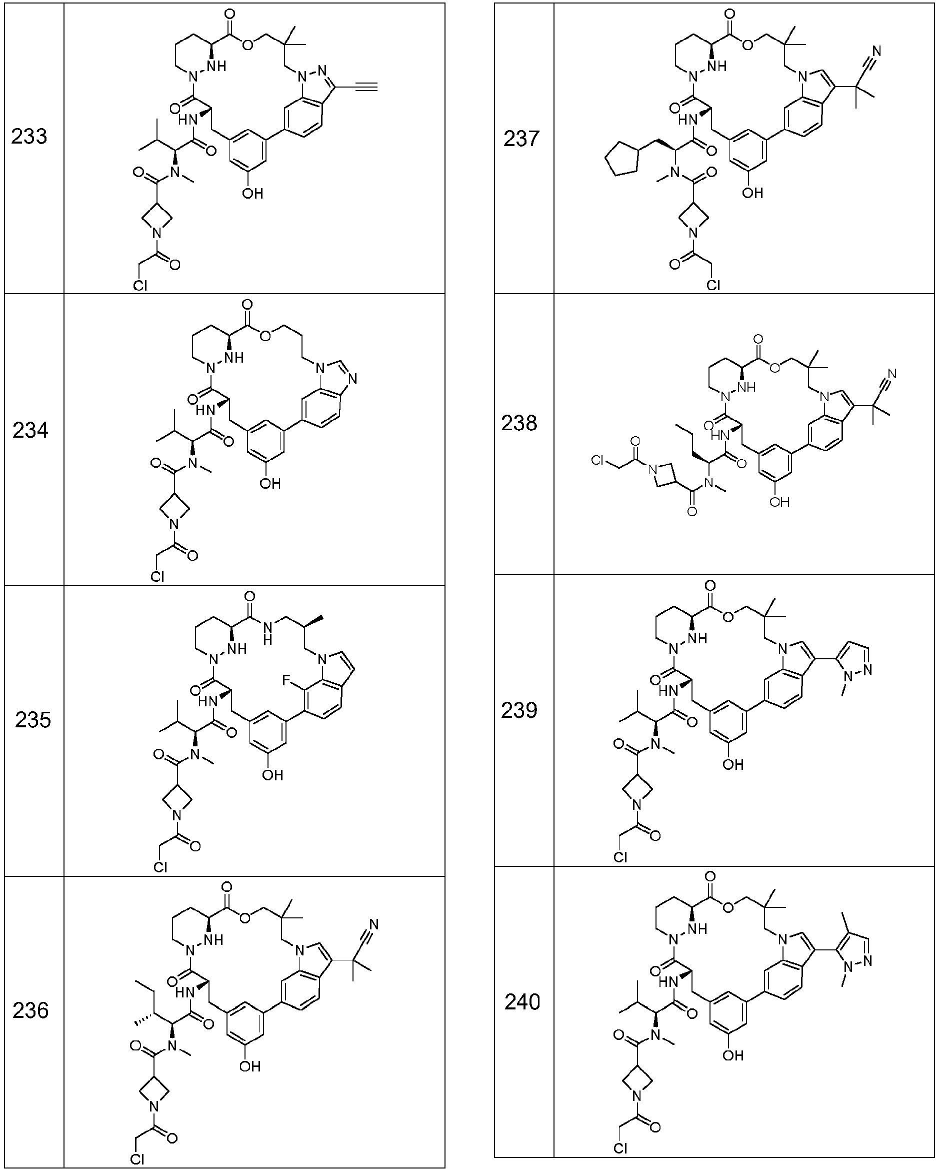

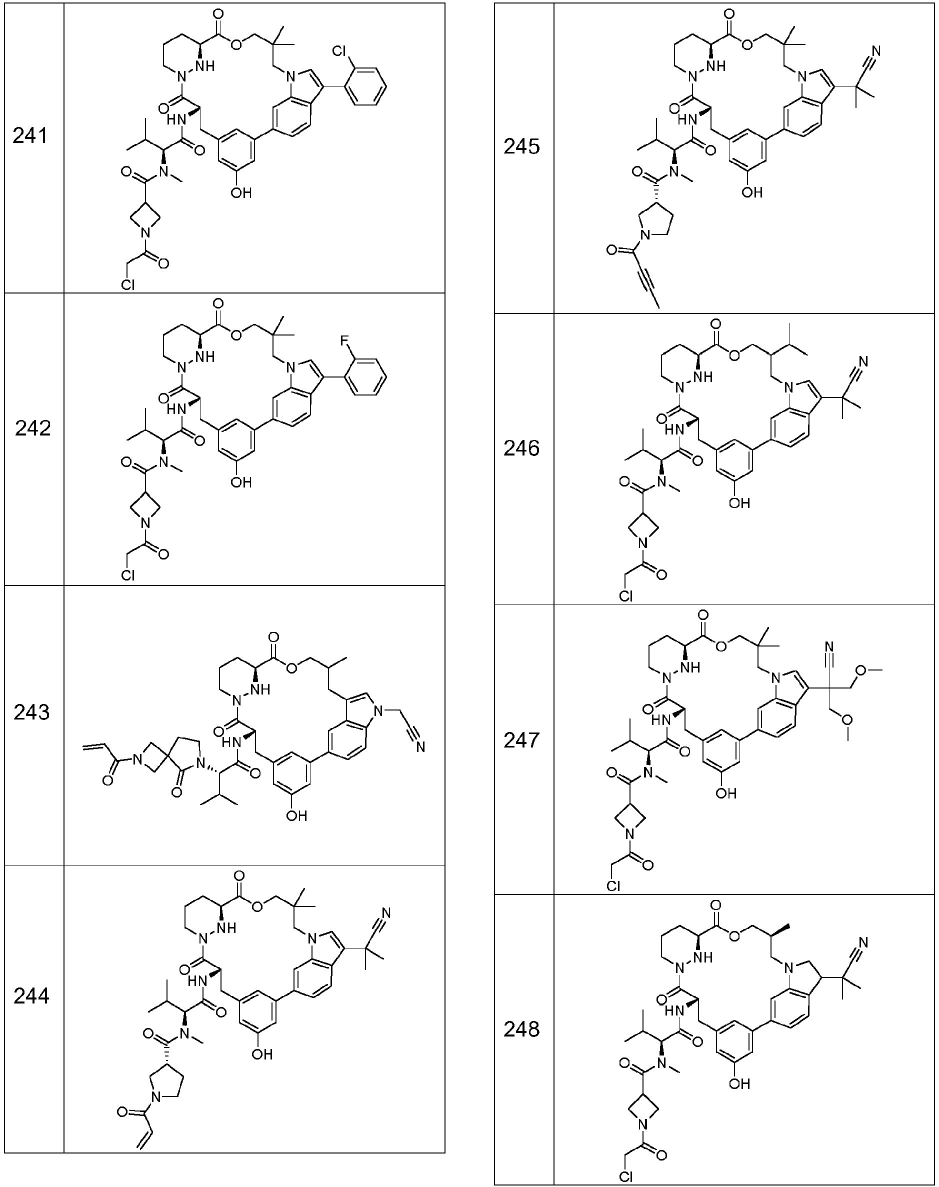

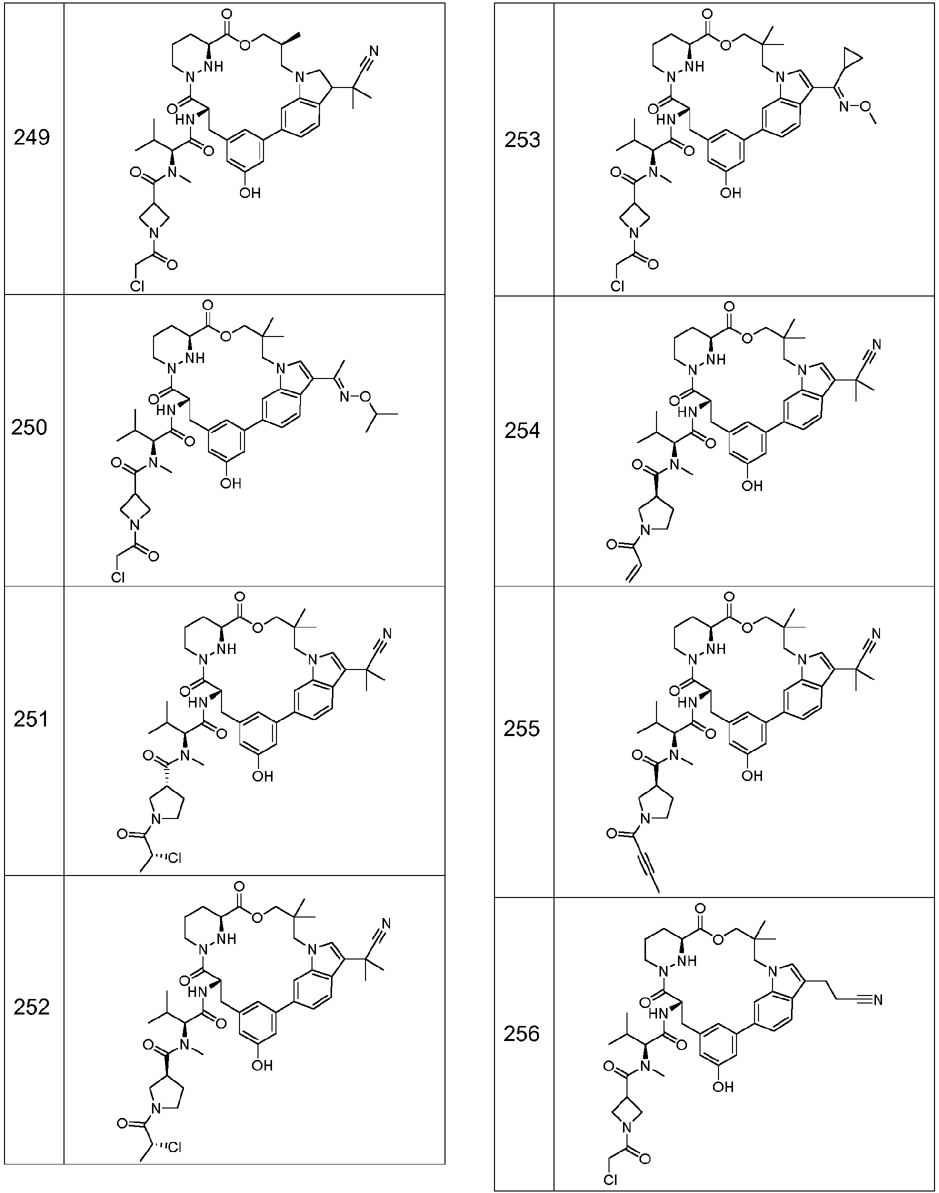

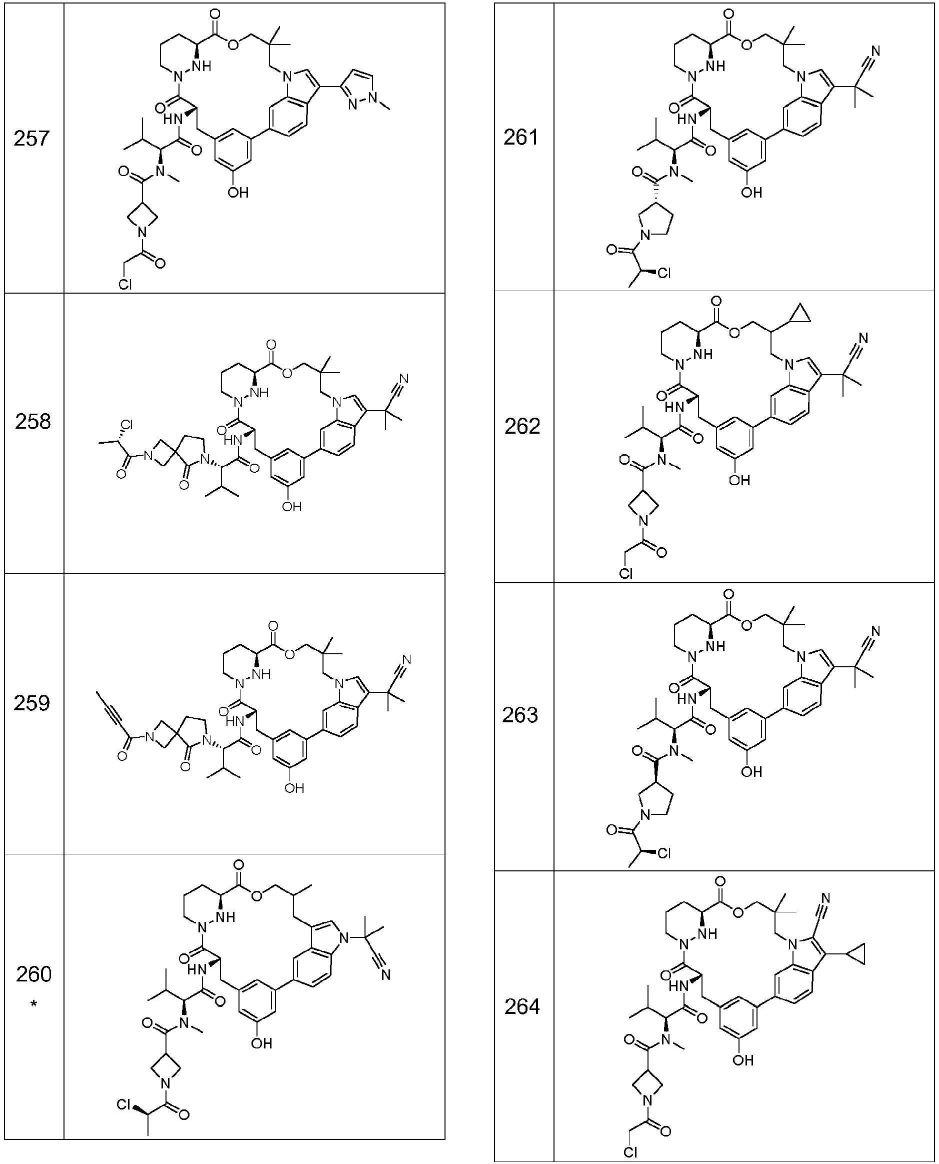

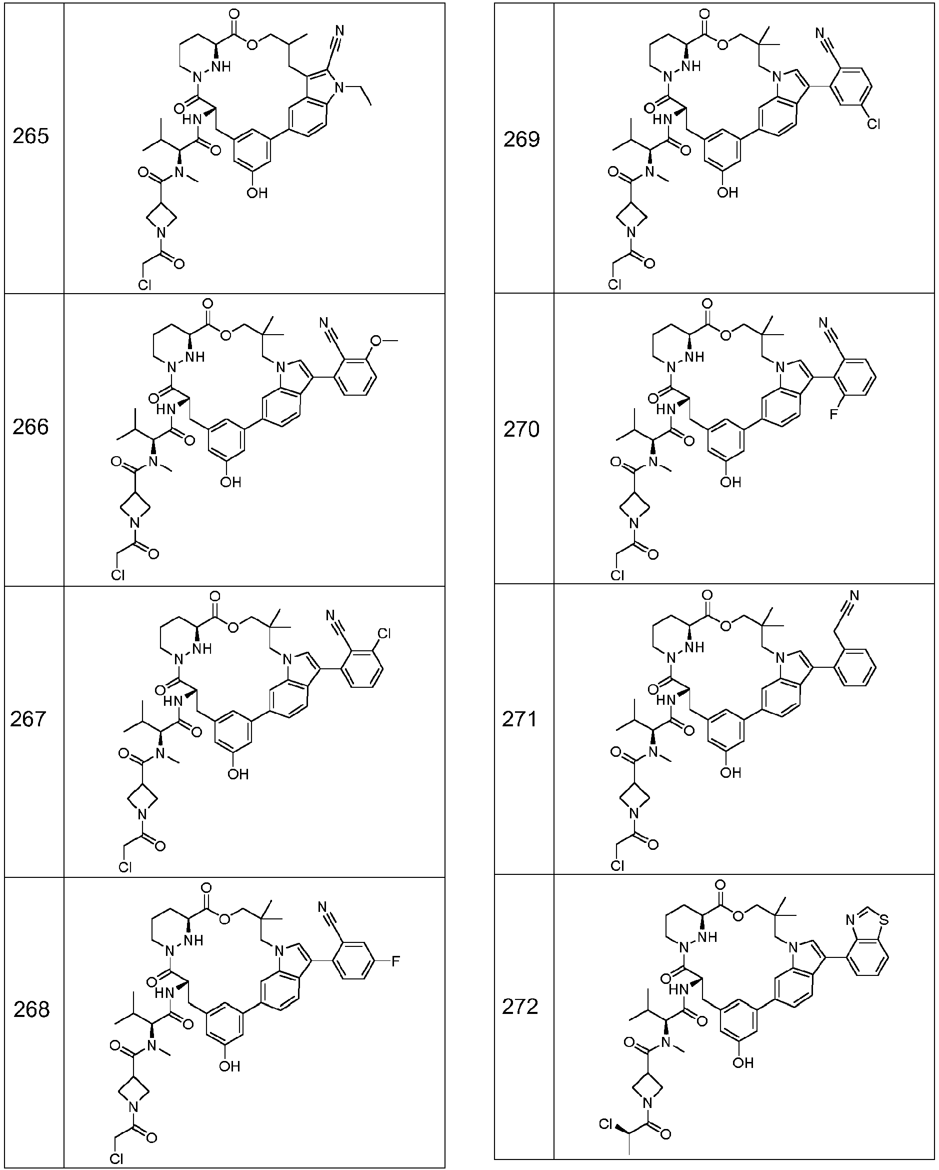

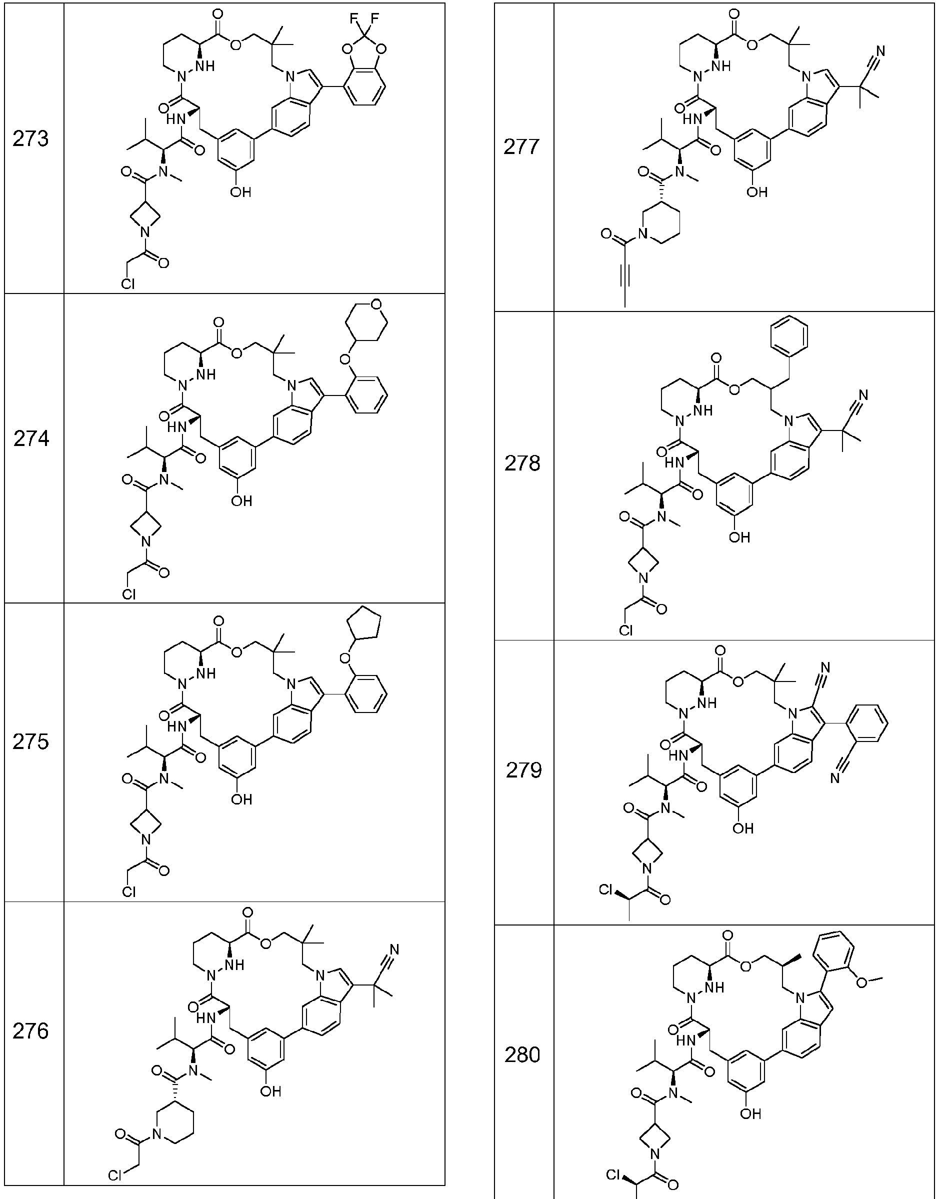

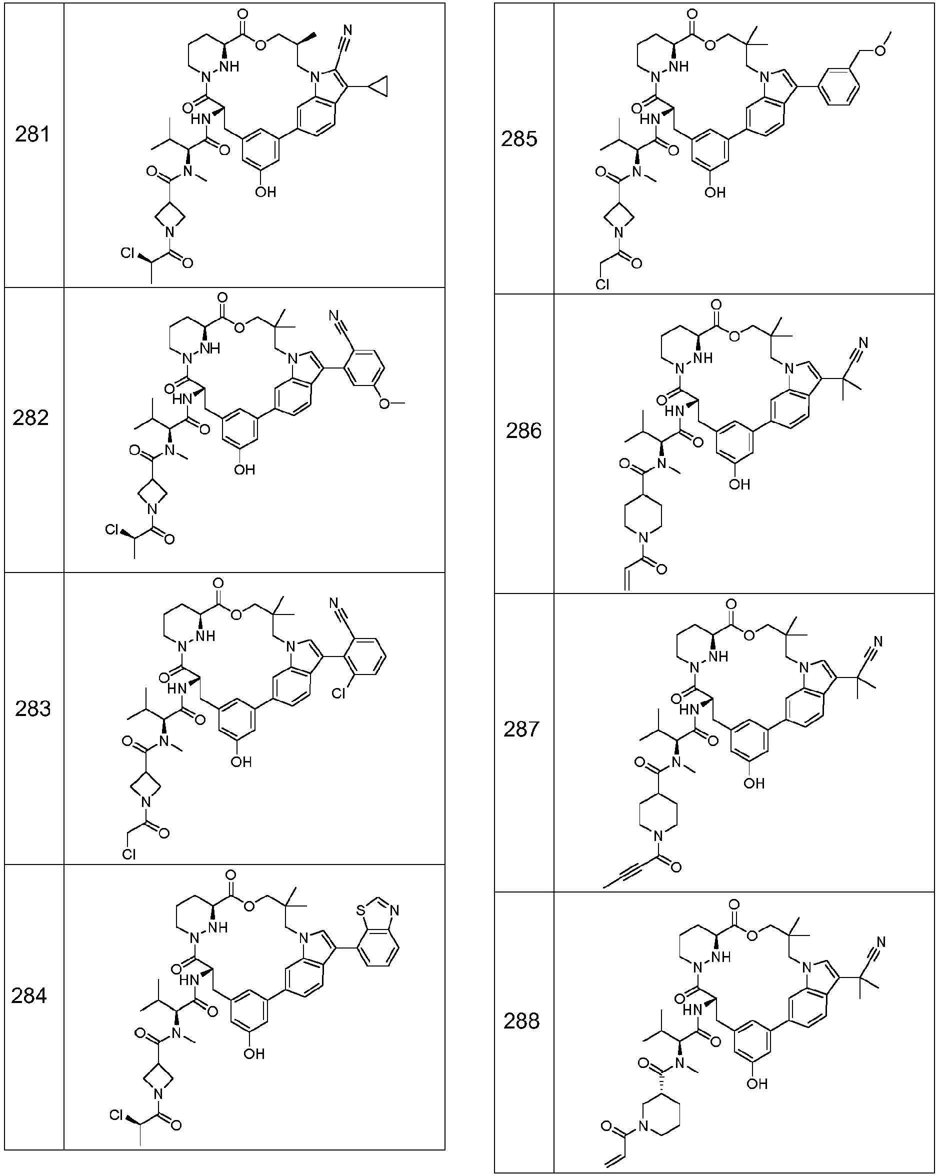

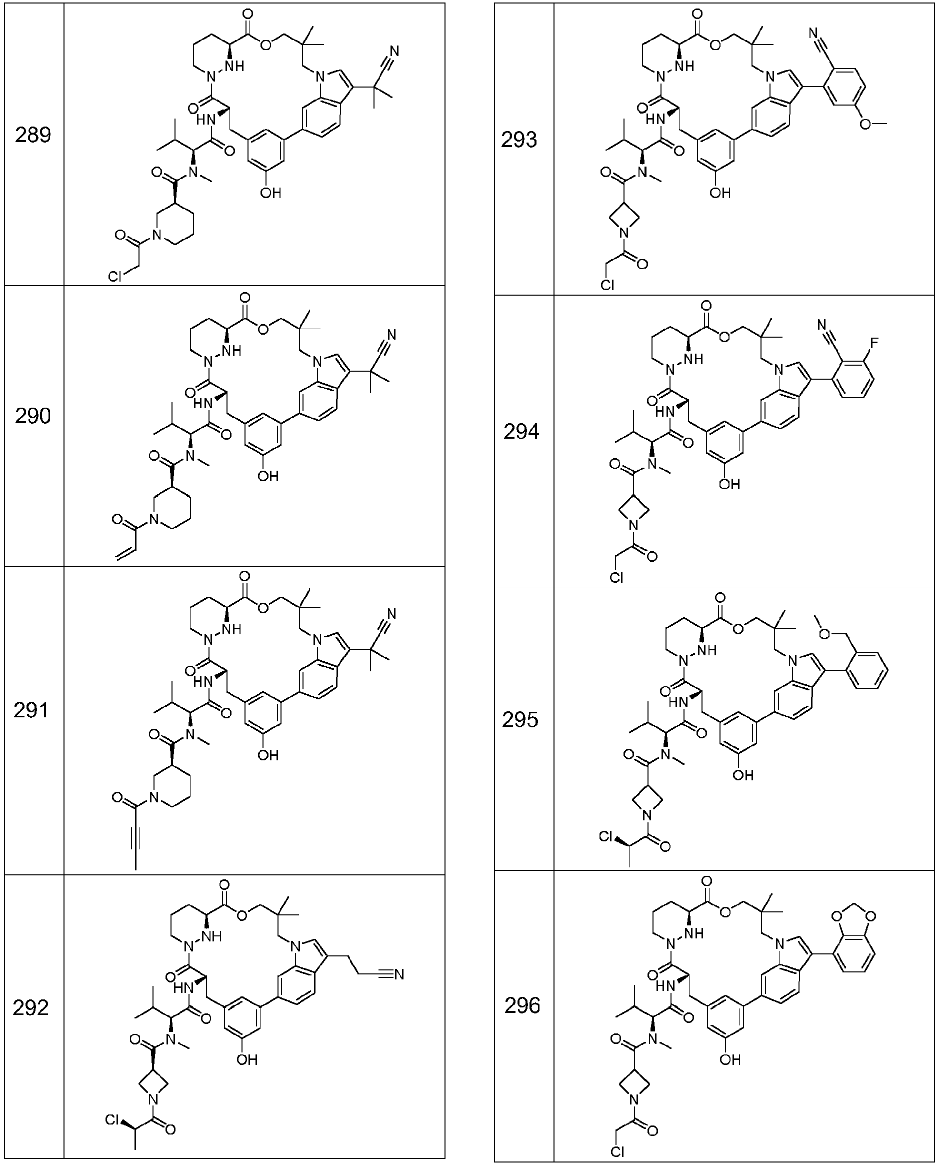

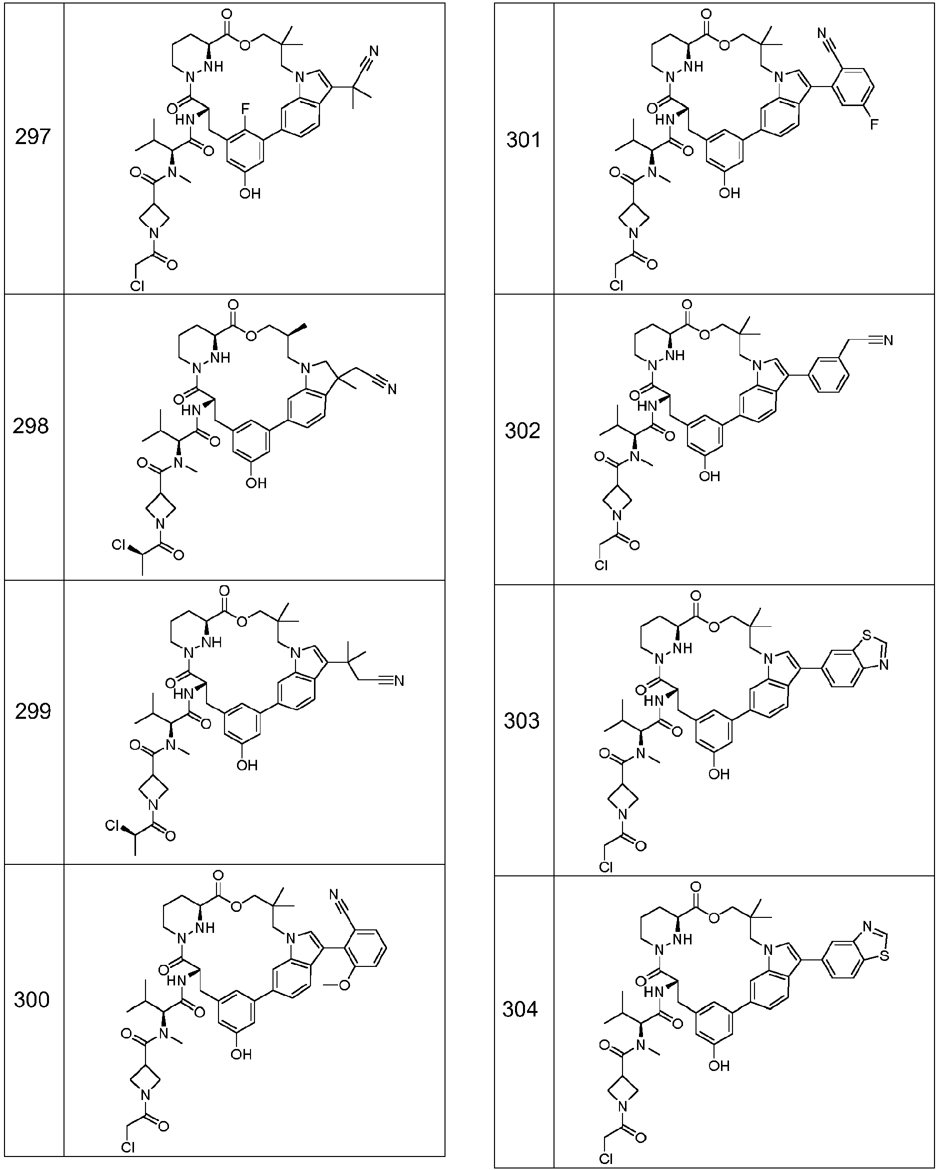

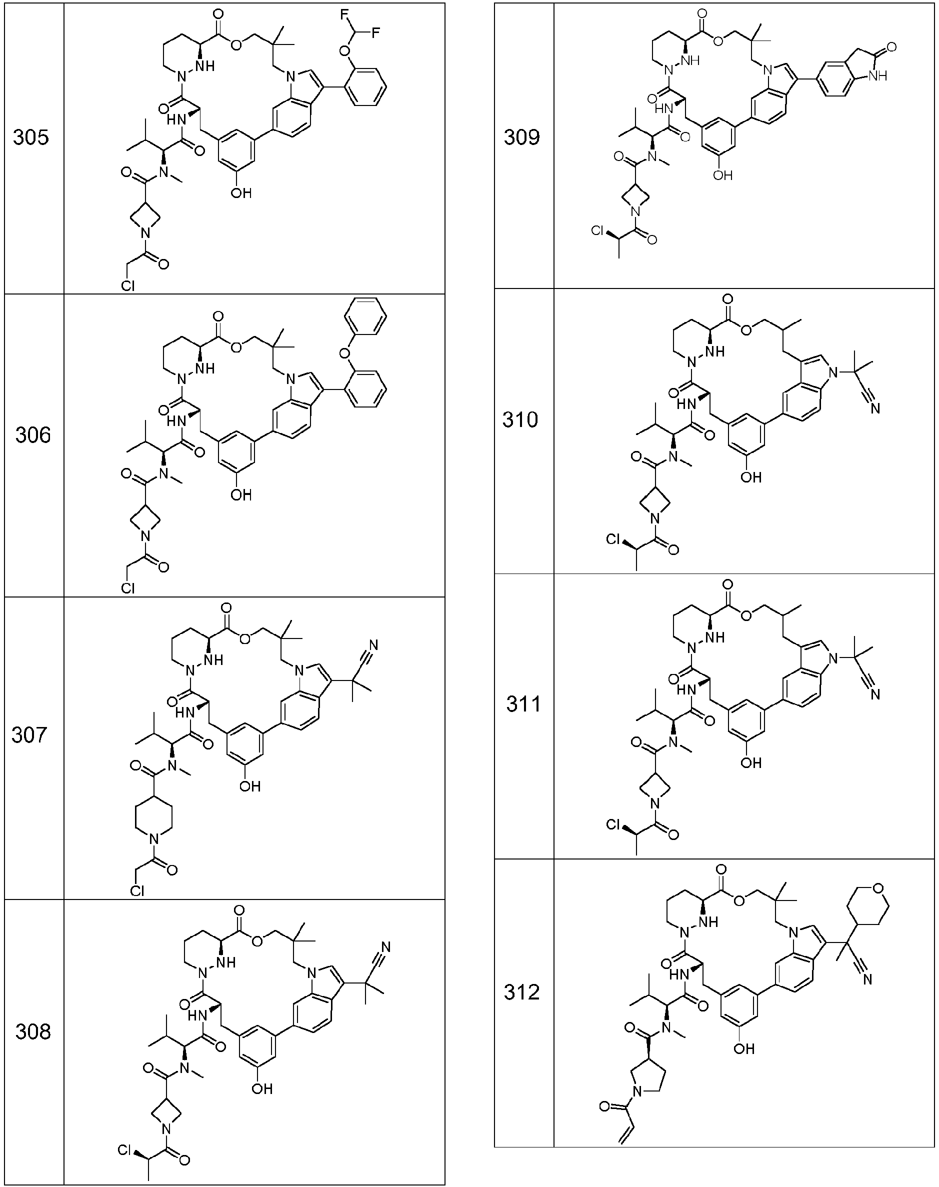

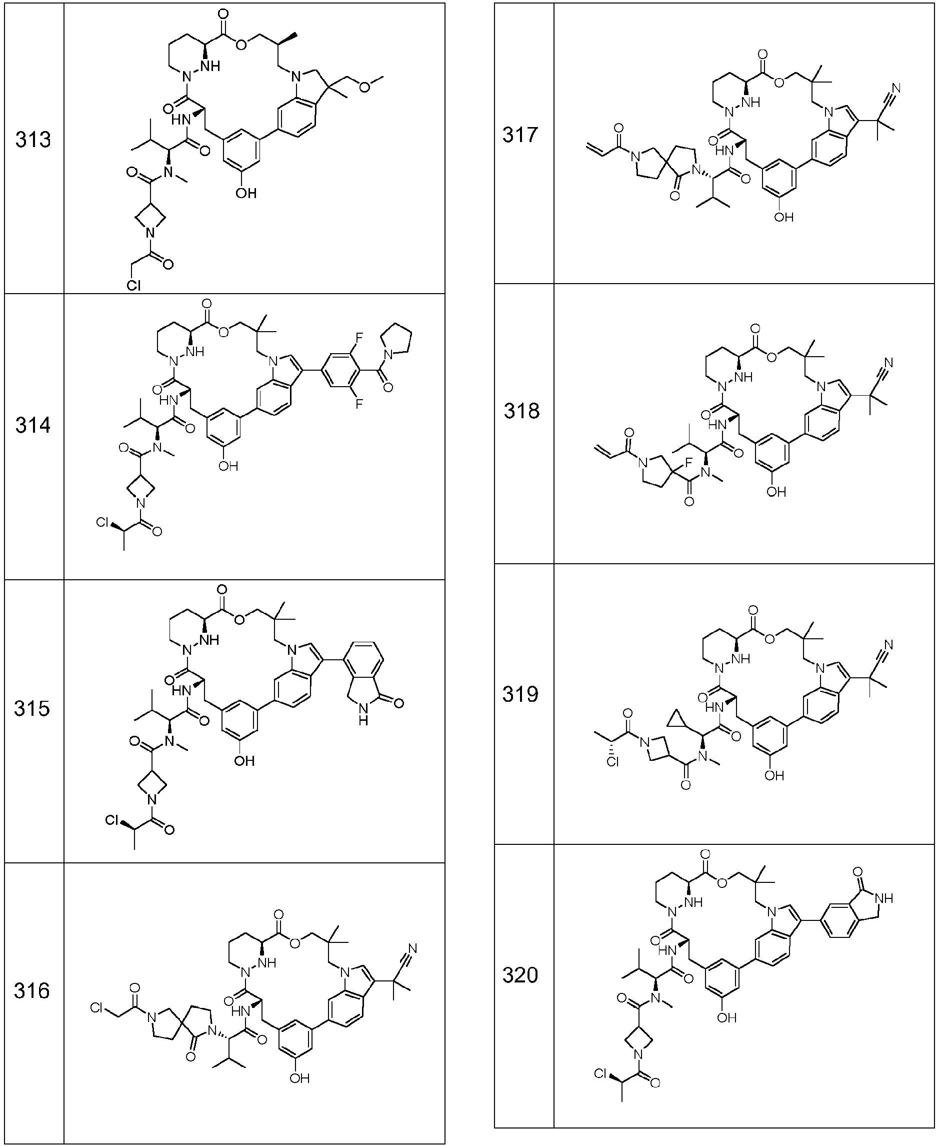

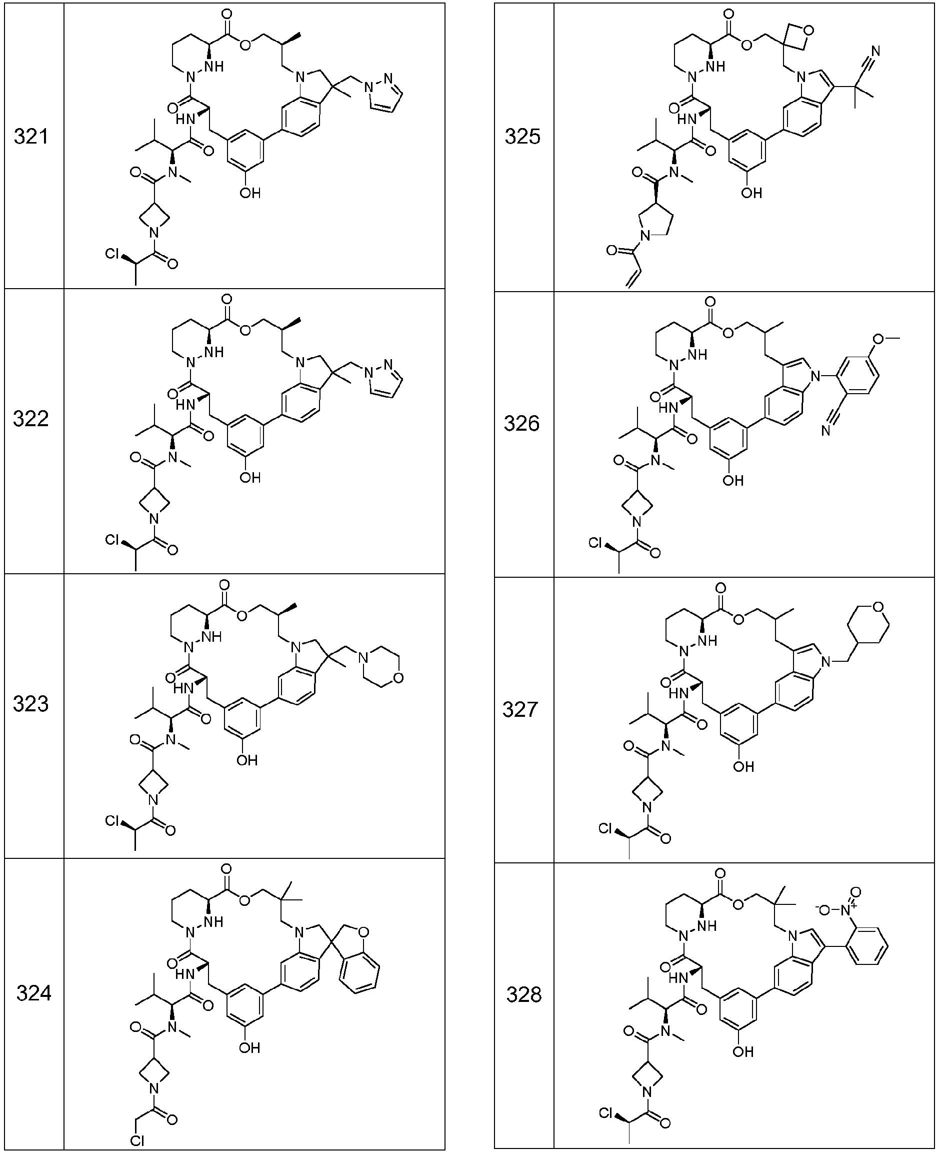

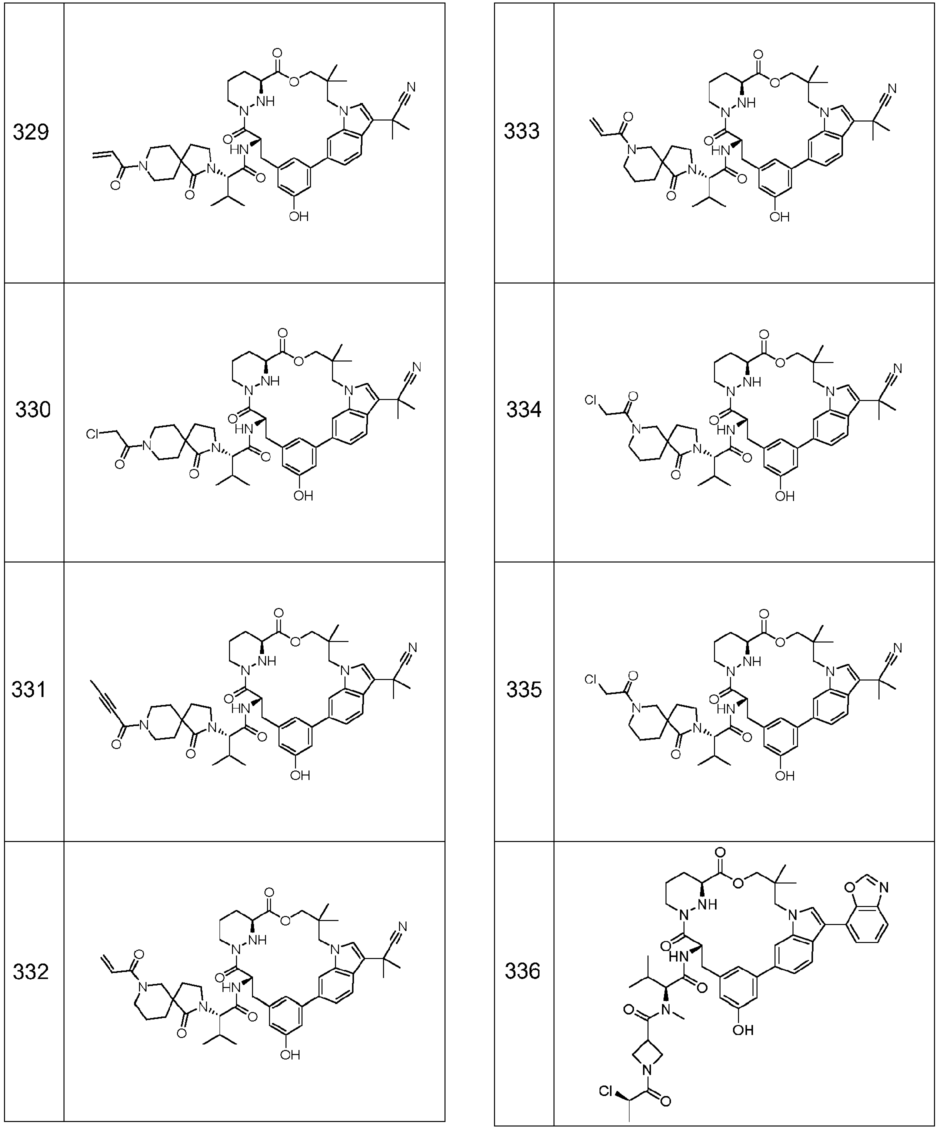

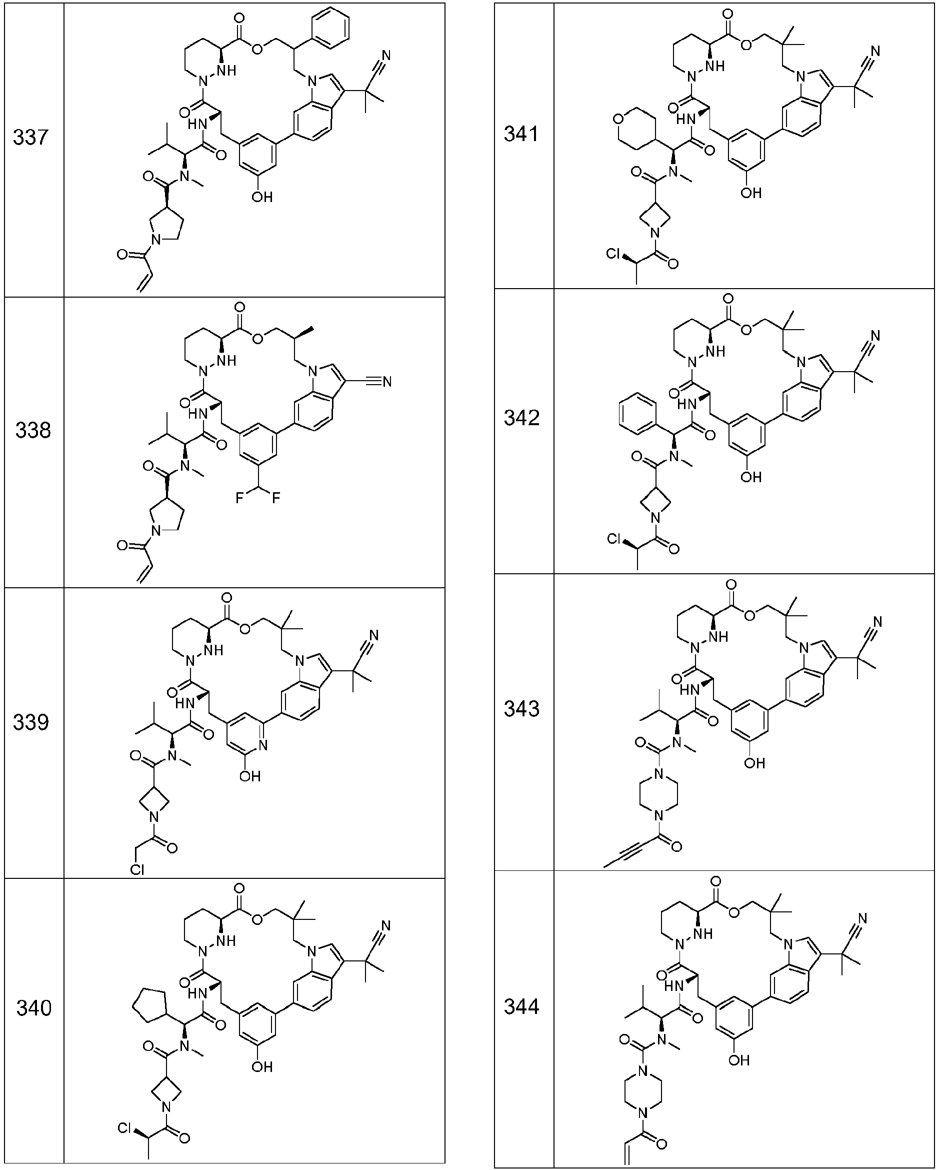

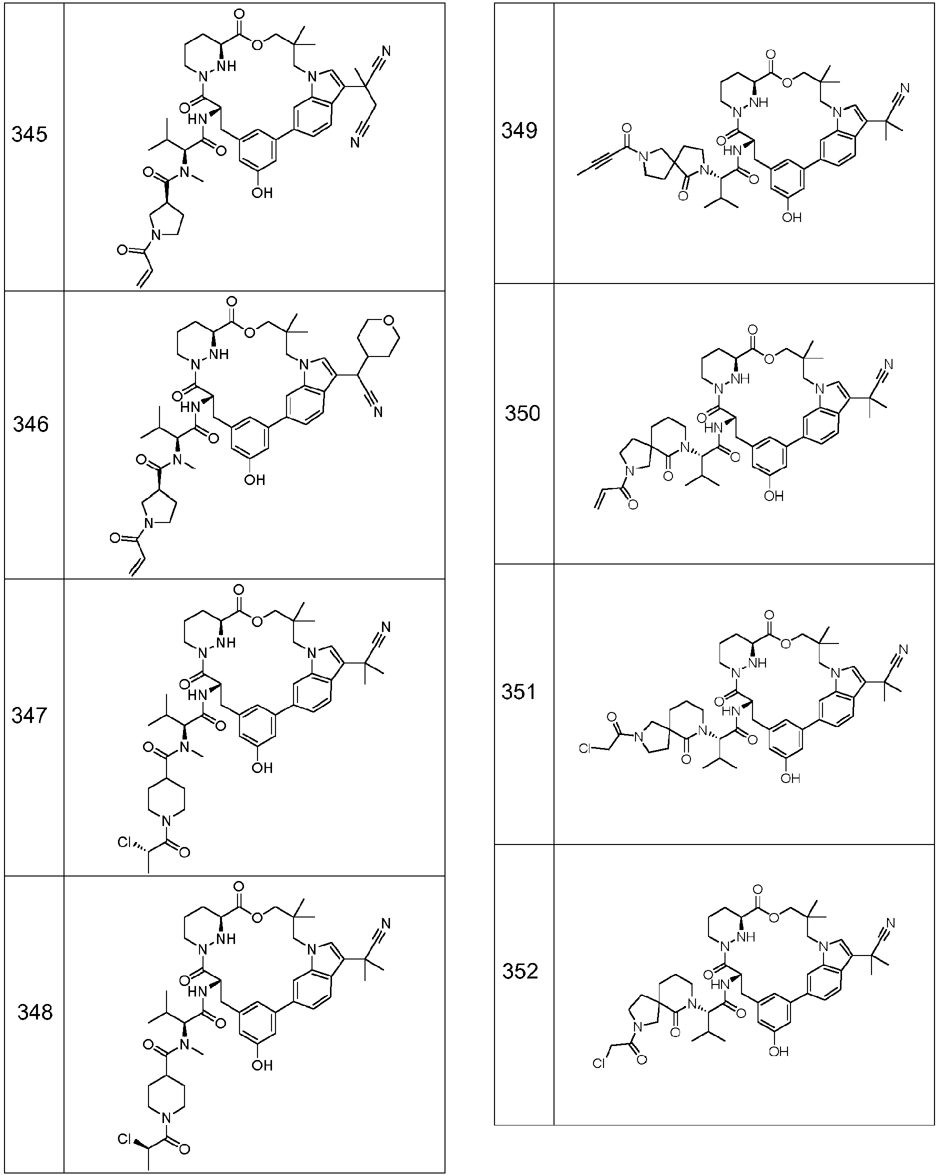

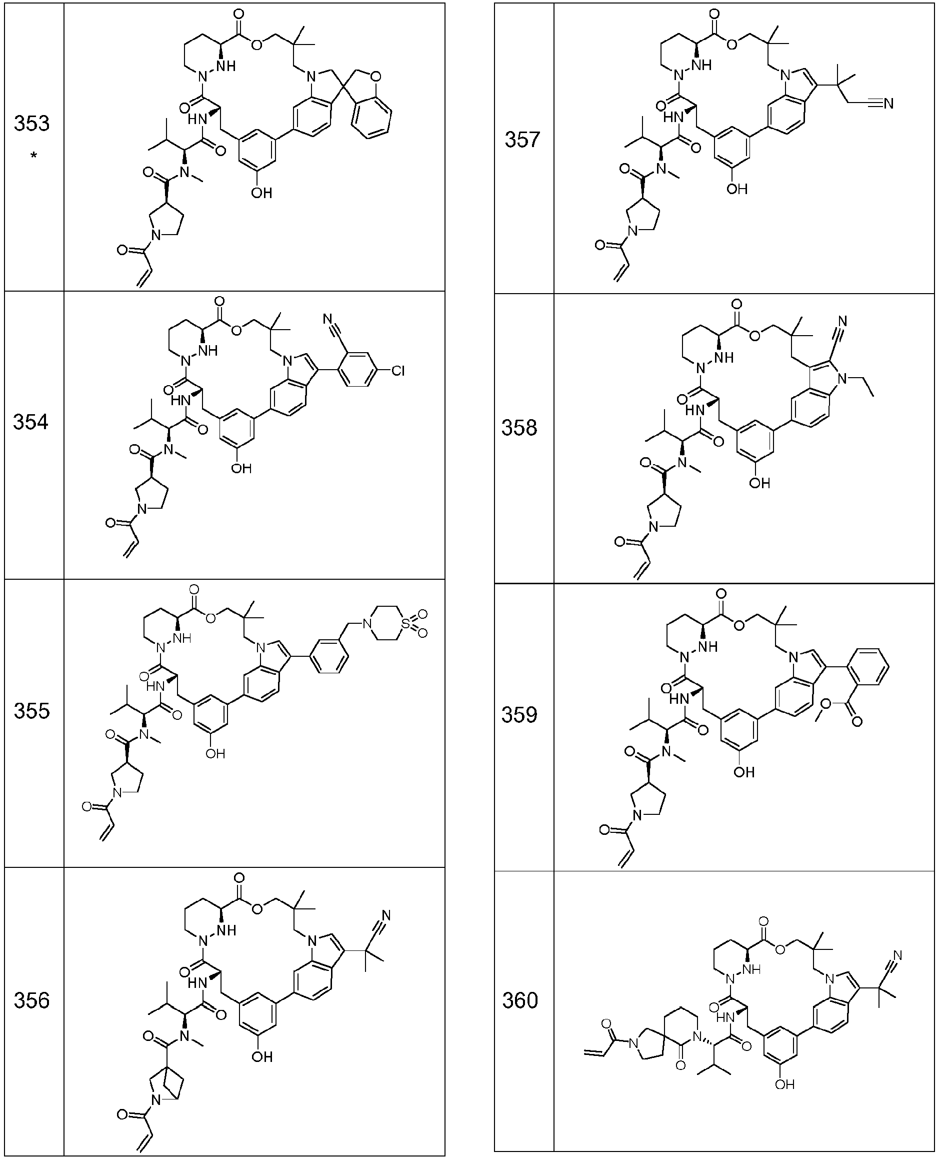

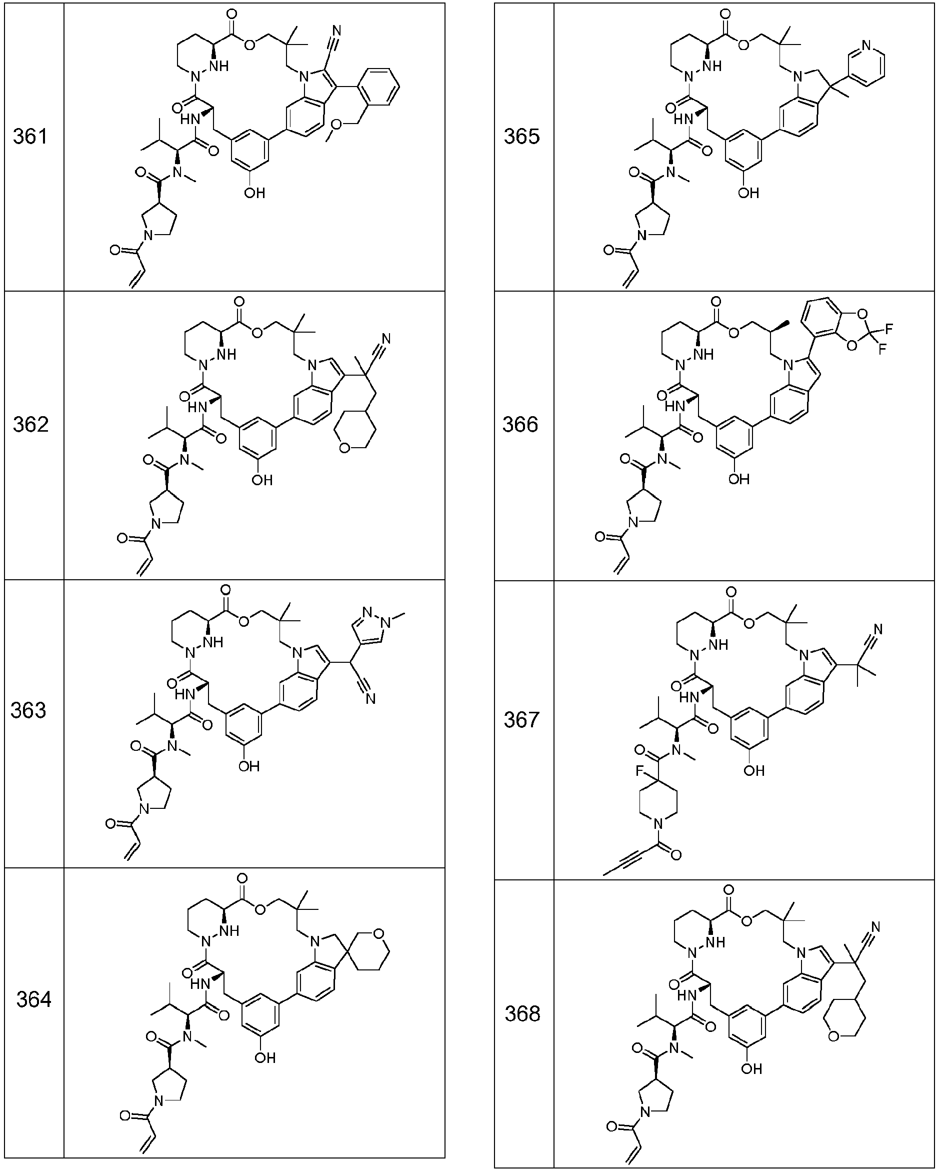

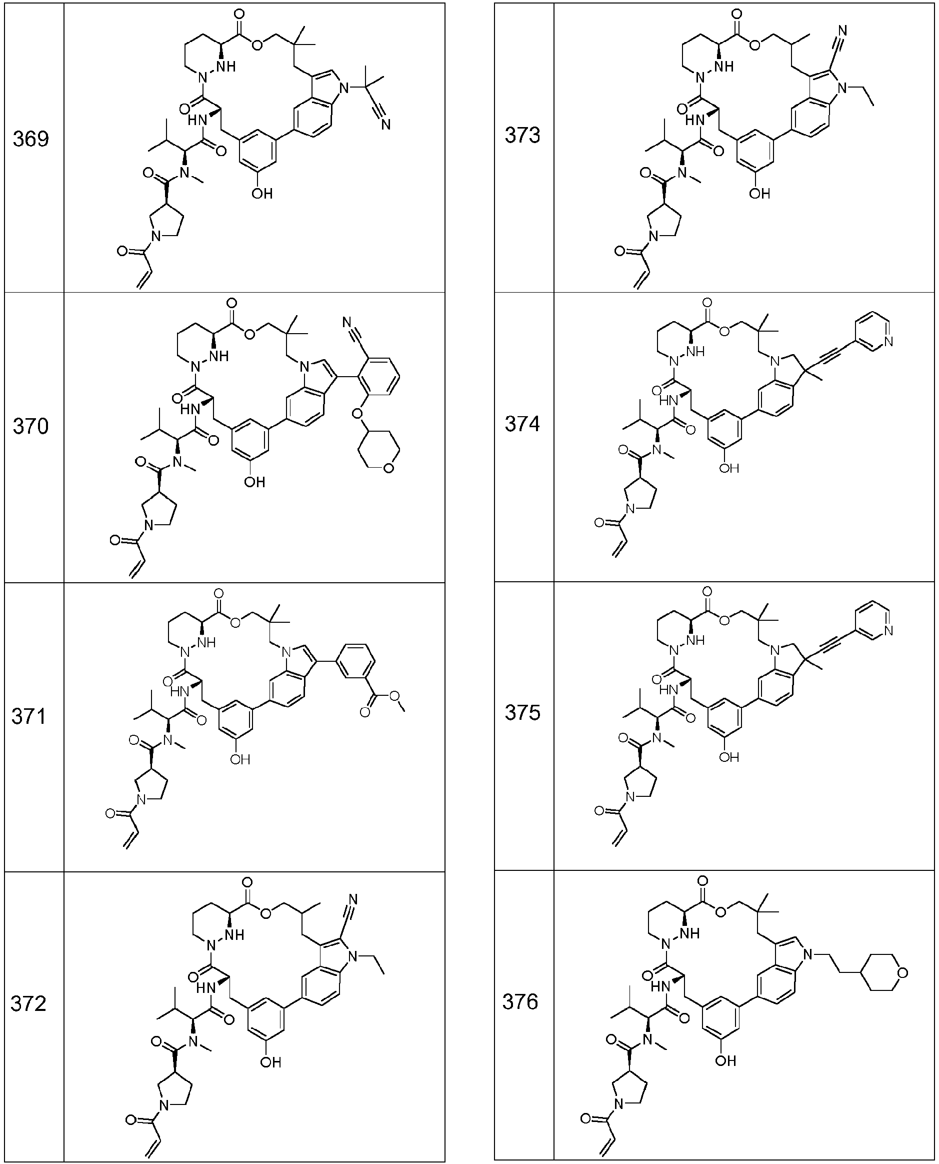

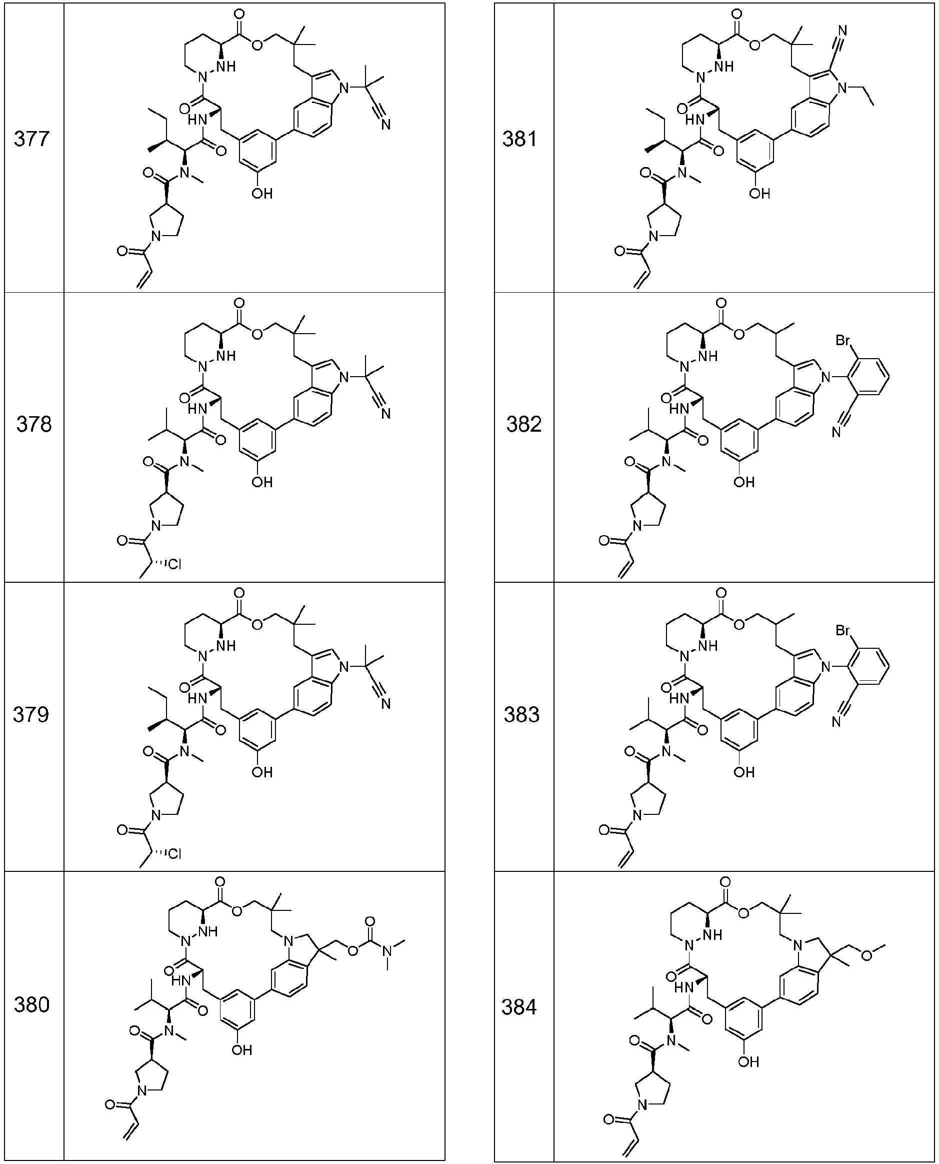

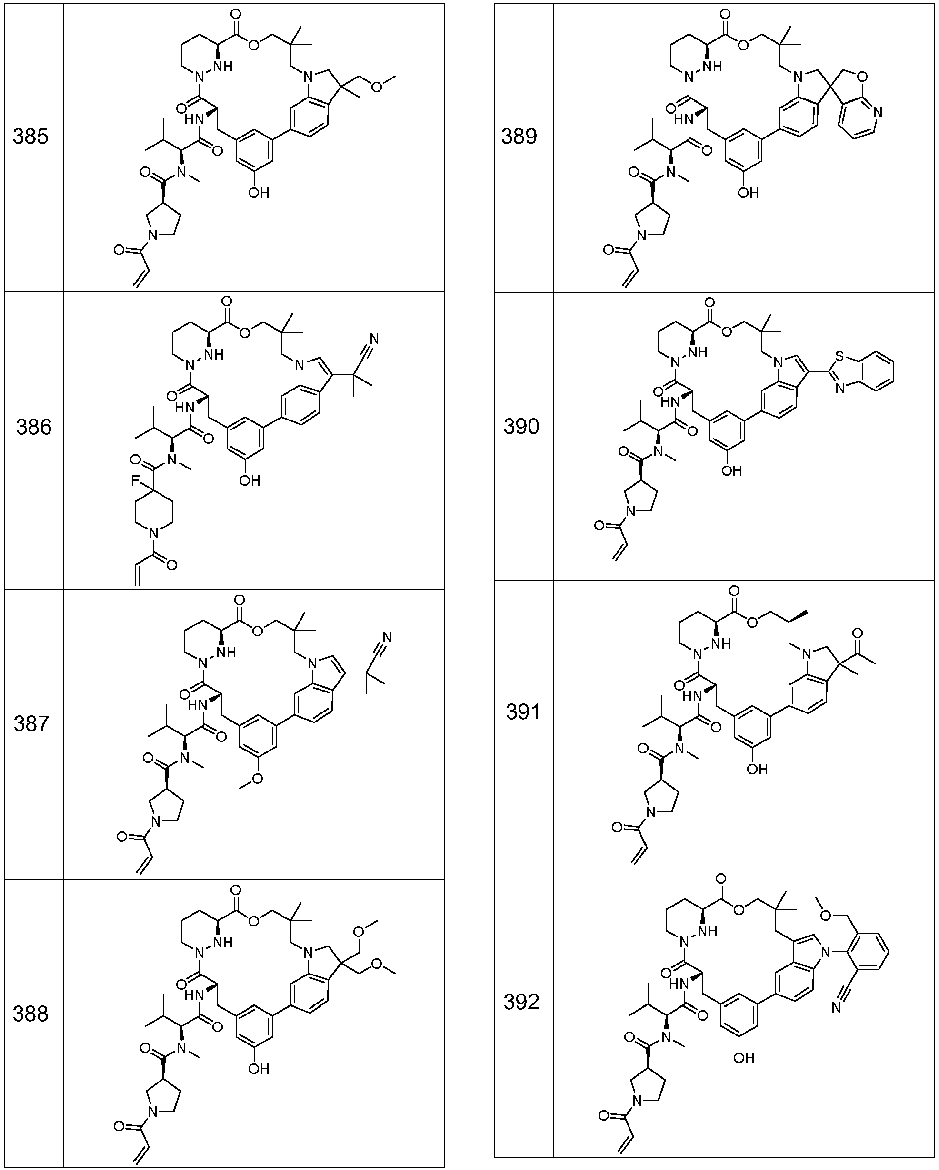

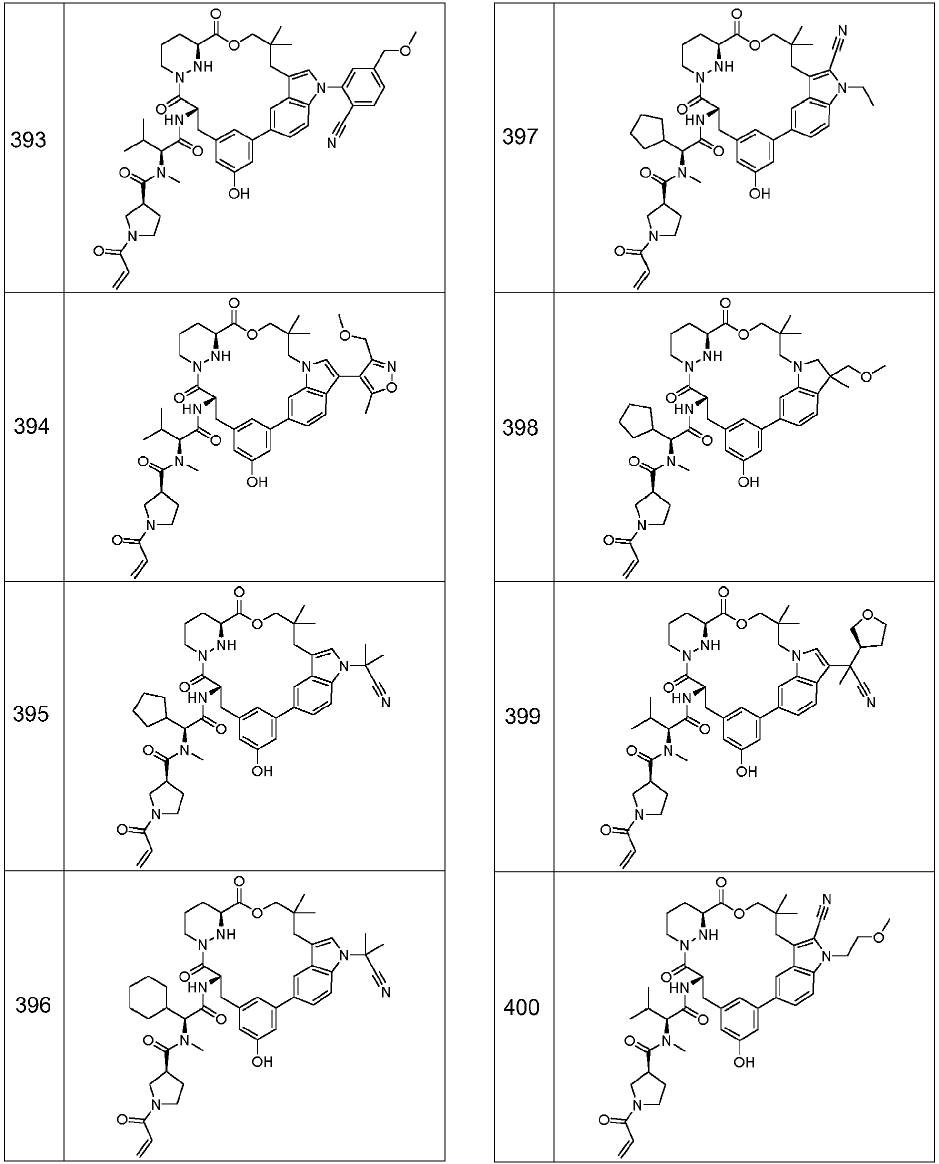

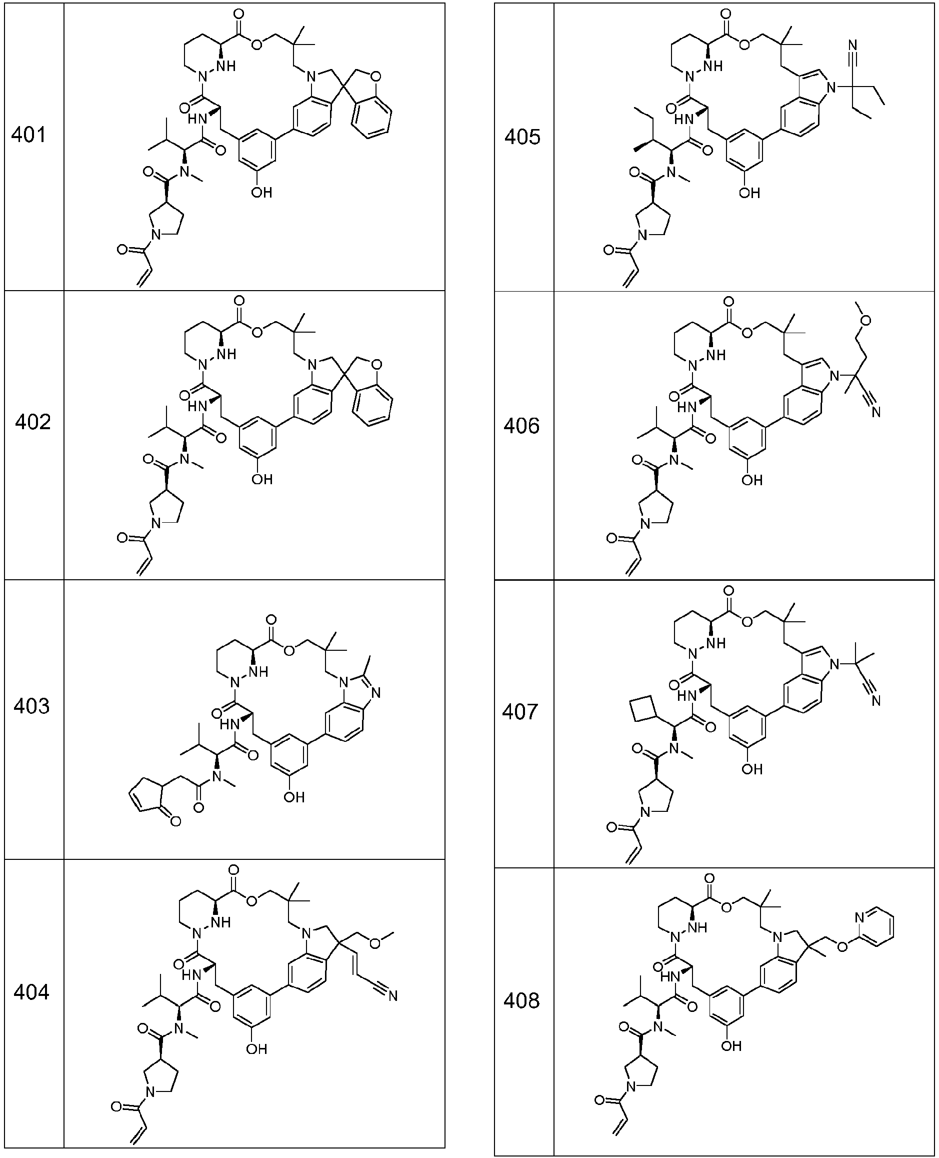

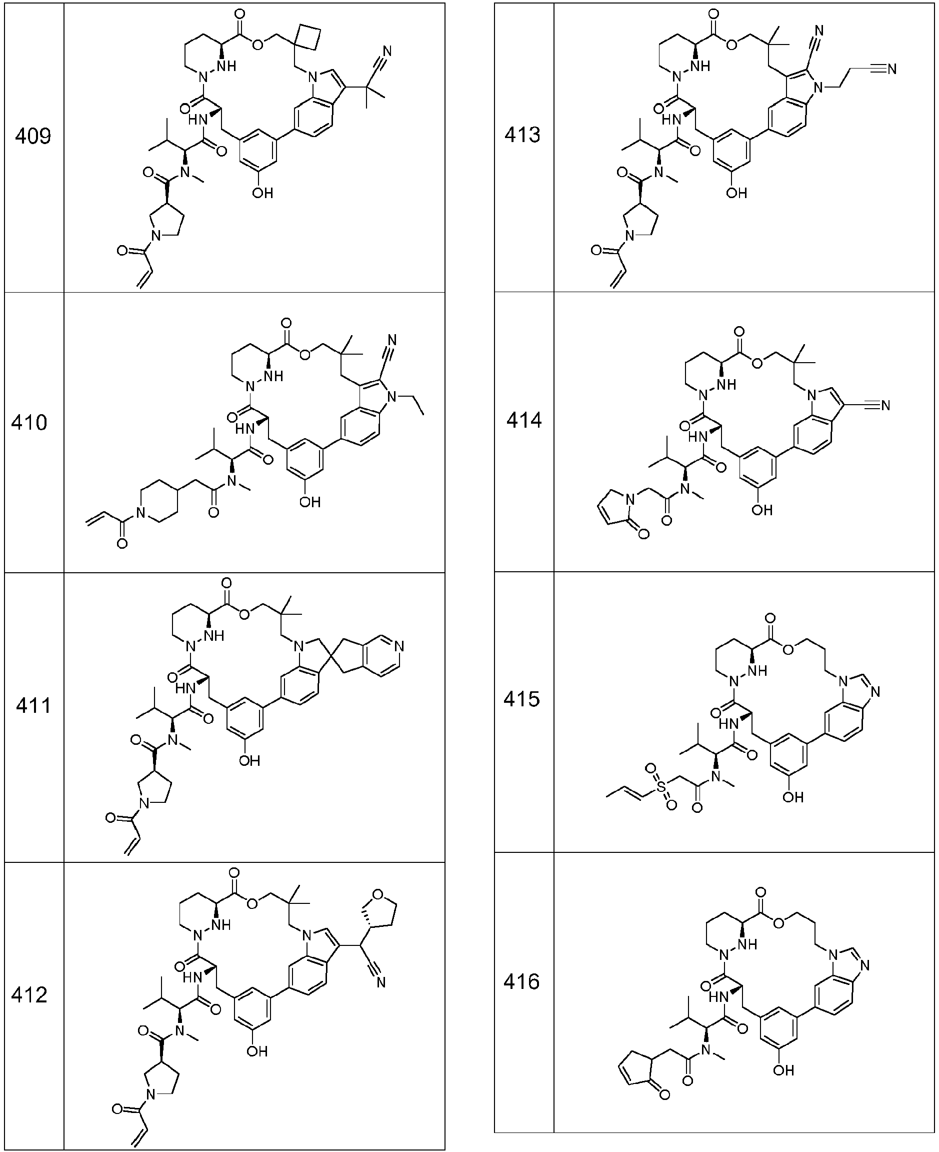

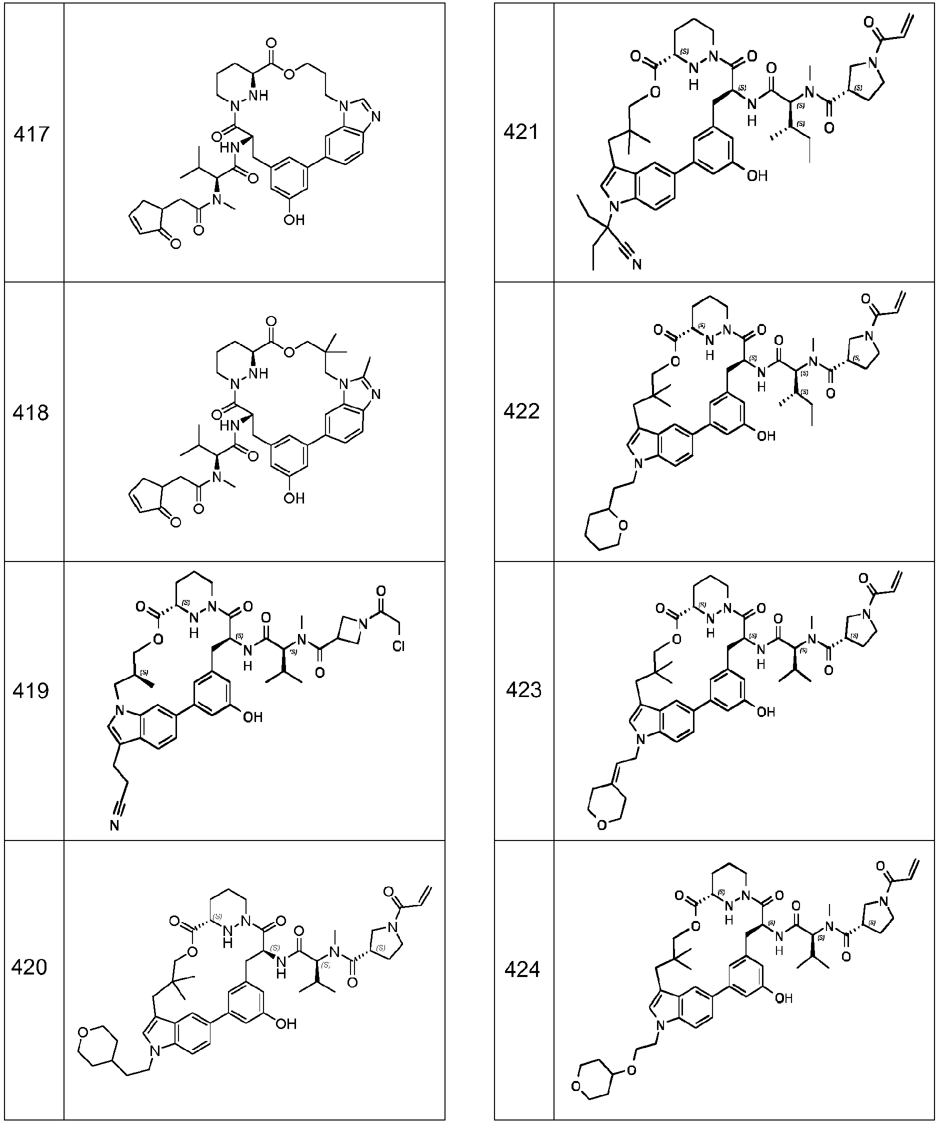

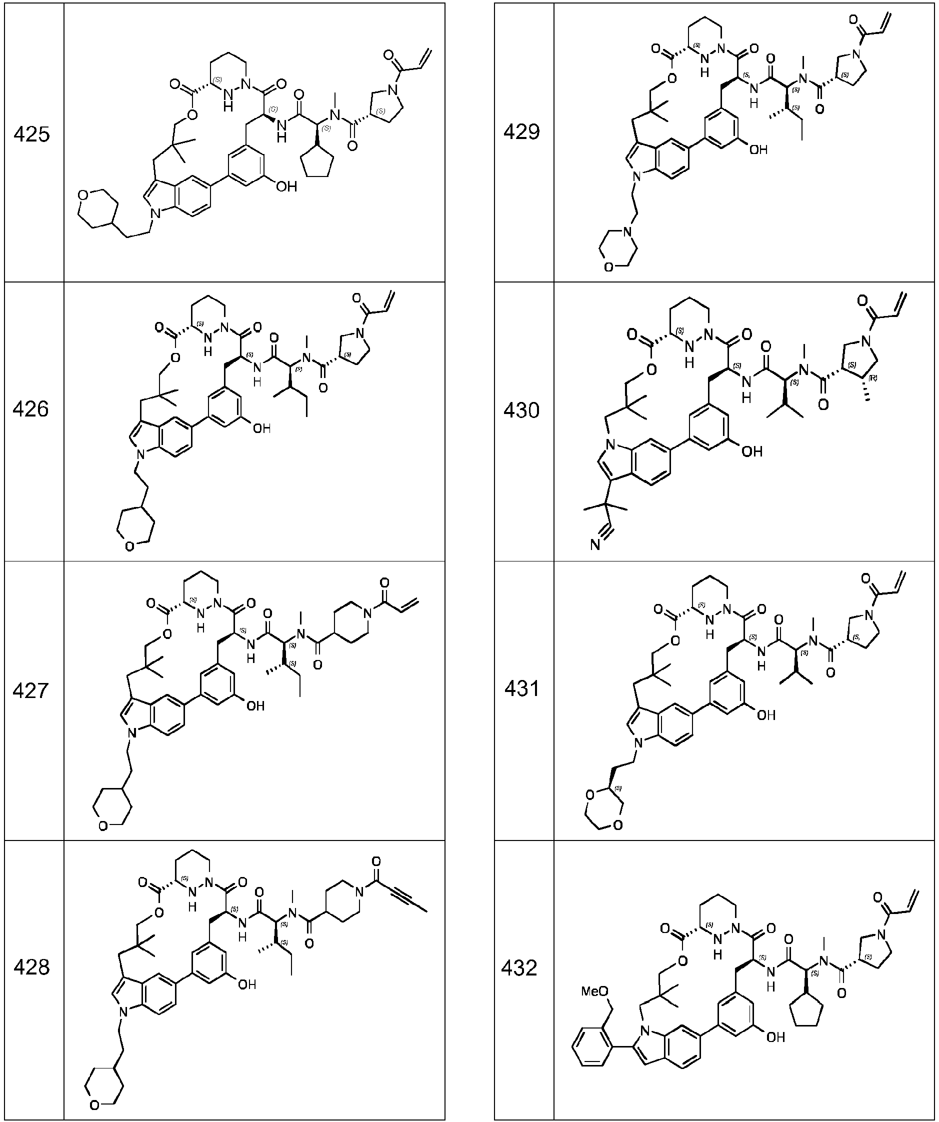

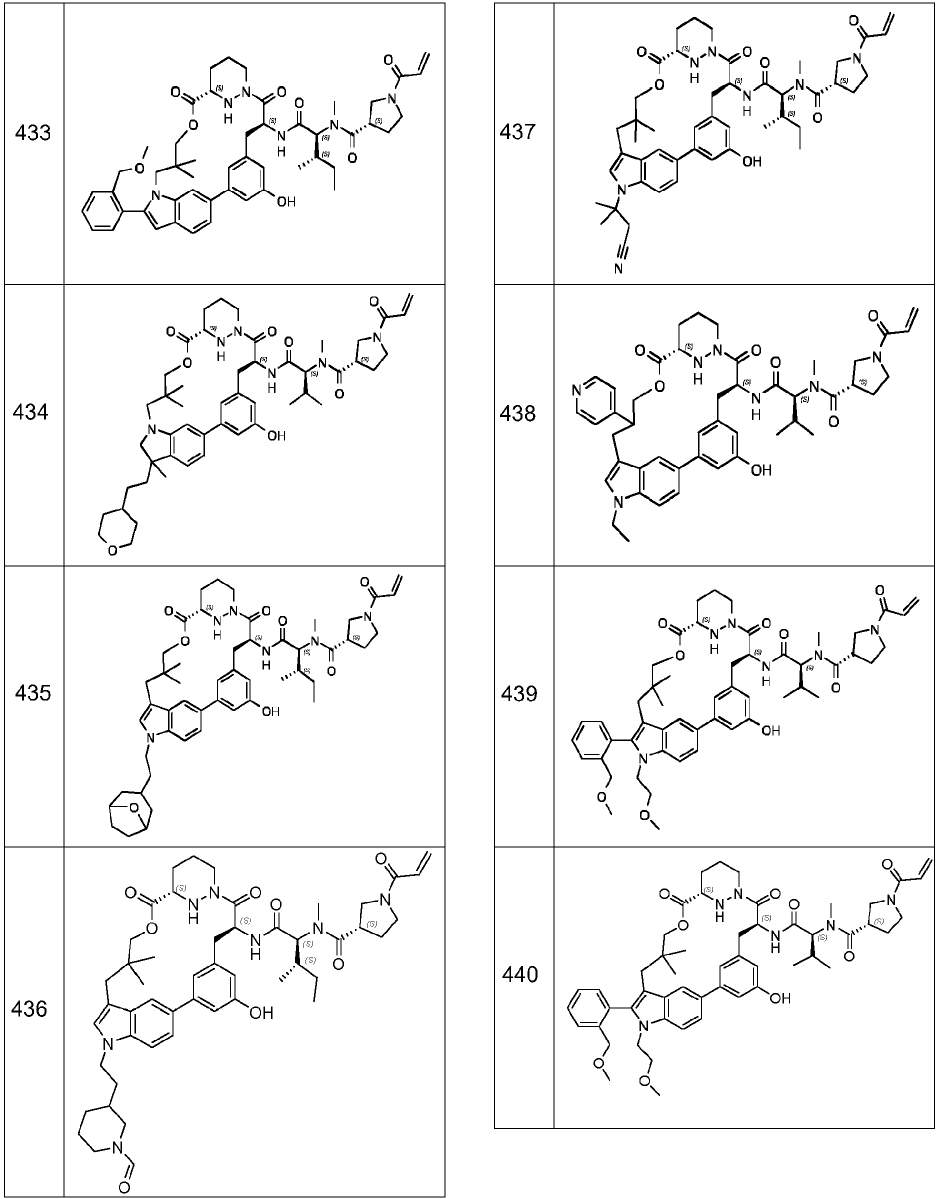

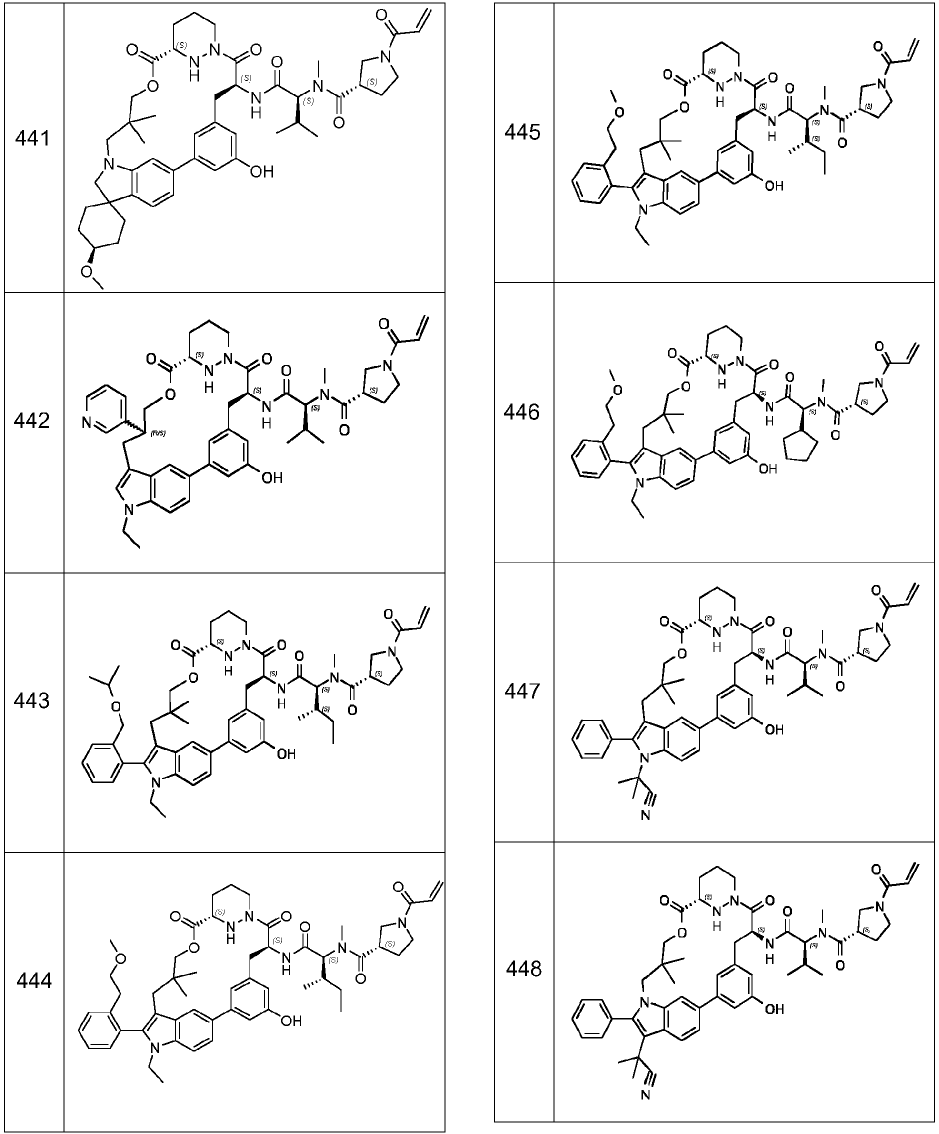

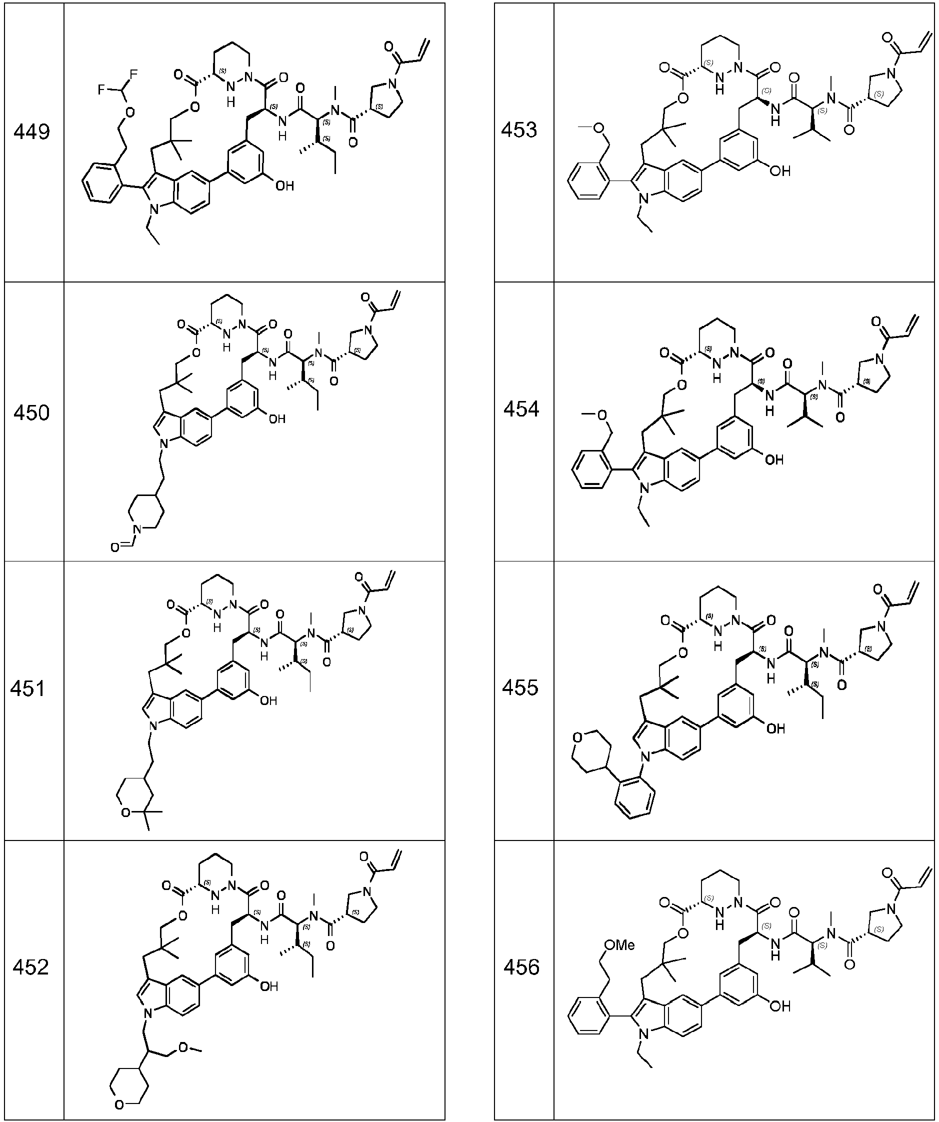

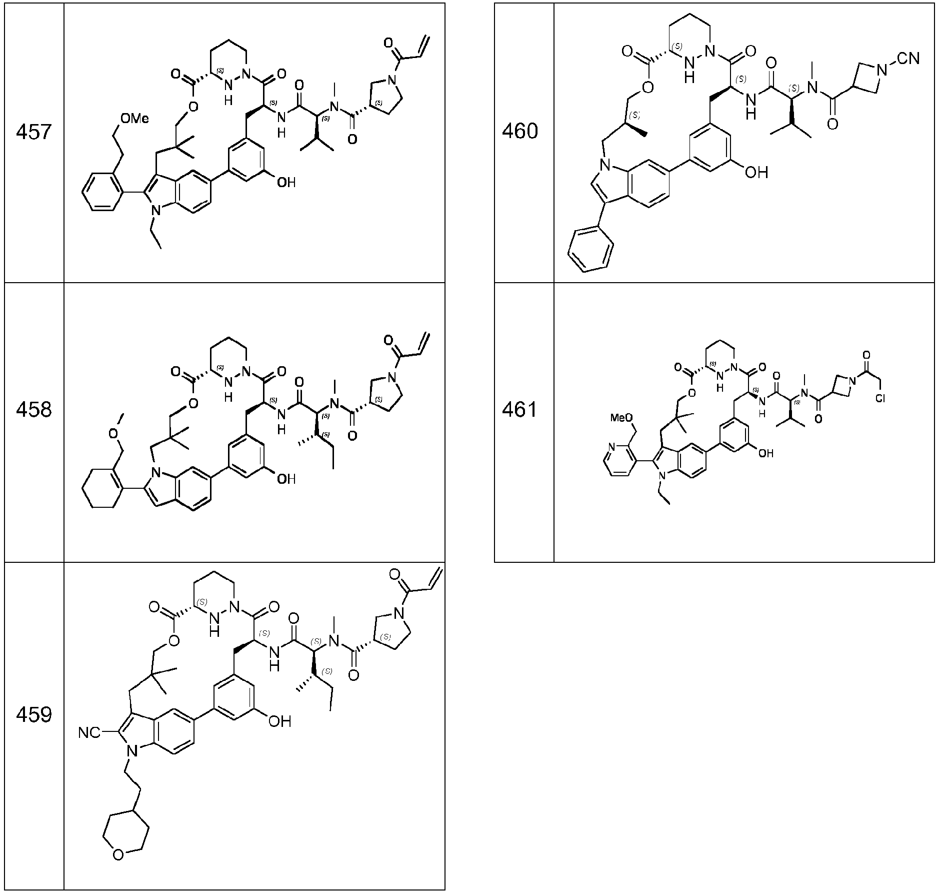

9. Composition pharmaceutique comprenant un composé selon l'une quelconque des revendications 1 à 8, ou un sel pharmaceutiquement acceptable, un énantiomère, un stéréoisomère, ou un tautomère de celui-ci, et un excipient pharmaceutiquement acceptable.

10. Complexe comprenant une protéine présentatrice, une protéine RAS, et un composé selon l'une quelconque des revendications 1 à 8, ou un sel pharmaceutiquement acceptable de celui-ci, ou une composition pharmaceutique selon la revendication 9.

11. Composé selon l'une quelconque des revendications 1 à 8, ou un sel pharmaceutiquement acceptable, un énantiomère, un stéréoisomère, ou un tautomère de celui-ci, pour son utilisation dans un procédé de traitement du cancer chez un sujet qui en a besoin.

12. Composé selon l'une quelconque des revendications 1 à 8, ou un sel pharmaceutiquement acceptable, un énantiomère, un stéréoisomère, ou un tautomère de celui-ci, pour son utilisation dans un procédé de traitement d'un trouble lié à la protéine KRAS G12C chez un sujet qui en a besoin.

13. Composé pour utilisation selon la revendication 11 ou la revendication 12, dans lequel le procédé comprend en outre l'administration d'un agent thérapeutique supplémentaire.

14. Composé pour utilisation selon la revendication 13, dans lequel l'agent thérapeutique supplémentaire est un inhibiteur de HER2, un inhibiteur de EGFR, un deuxième inhibiteur de Ras, un inhibiteur de SHP2, un inhibiteur de SOS1, un inhibiteur de Raf, un inhibiteur de MEK, un inhibiteur de ERK, un inhibiteur de PI3K, un inhibiteur de PTEN, un inhibiteur de AKT, un inhibiteur de mTORC1, un inhibiteur de BRAF, un inhibiteur de PD-L1, un inhibiteur de PD-1, un inhibiteur de CDK 4/6, ou une combinaison de ceux-ci.
